(19) Europäisches Patentamt
European Patent Office
Office européen des brevets

(11) **EP 4 361 150 A2**

(12) # EUROPEAN PATENT APPLICATION

(43) Date of publication:
**01.05.2024 Bulletin 2024/18**

(21) Application number: **24159602.2**

(22) Date of filing: **16.12.2021**

(51) International Patent Classification (IPC):
***C07D 413/14*** *(2006.01)*

(52) Cooperative Patent Classification (CPC):
(C-Sets available)
**C07D 413/10; A01N 43/82; A01N 43/90; A01N 53/00; A01P 3/00; C07D 413/14** (Cont.)

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **17.12.2020 US 202063127068 P**

(62) Document number(s) of the earlier application(s) in accordance with Art. 76 EPC:
**21835114.6 / 4 262 396**

(71) Applicant: **FMC Corporation**
**Philadelphia, Pennsylvania 19104 (US)**

(72) Inventors:
- **PASTERIS, Robert, James**
  **Philadelphia (US)**
- **CHITTABOINA, Srinivas**
  **Philadelphia (US)**
- **MCMAHON, Travis Chandler**
  **Philadelphia (US)**
- **KAMIREDDY, Balreddy**
  **Philadelphia (US)**
- **REDDY, Ravisekhara P.**
  **Philadelphia (US)**
- **VEGA-JIMENEZ, Byron**
  **Philadelphia (US)**
- **UPPALAPATI, Srinivasa Rao**
  **Philadelphia (US)**
- **GAMBHIR, Nikita**
  **Philadelphia (US)**

(74) Representative: **Dehns**
**St. Bride's House**
**10 Salisbury Square**
**London EC4Y 8JD (GB)**

Remarks:
This application was filed on 26-02-2024 as a divisional application to the application mentioned under INID code 62.

(54) **FUNGICIDAL OXADIAZOLES AND THEIR MIXTURES**

(57) Disclosed is a fungicidal compound of Formula **1,** including all geometric and stereoisomers, tautomers, *N*-oxides, and salts thereof,

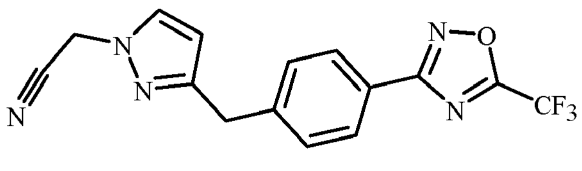

**1** ,

and compositions comprising said compound and optionally at least one additional fungicidal compound.

Also disclosed is a method for controlling plant diseases caused by fungal plant pathogens comprising applying to the plant or portion thereof, or to the plant seed, a fungicidally effective amount of a compound of Formula **1,** an *N*-oxide, or salt thereof (e.g., as a component in the aforesaid composition). Also disclosed is a composition comprising: the compound of Formula **1** described above, an *N*-oxide, or a salt thereof, and at least one invertebrate pest control compound or agent.

EP 4 361 150 A2

(52) Cooperative Patent Classification (CPC): (Cont.)

C-Sets
**A01N 43/82, A01N 37/34, A01N 37/50, A01N 43/40, A01N 43/54, A01N 43/56, A01N 43/653, A01N 43/84, A01N 45/02, A01N 47/14, A01N 59/16, A01N 59/20;**
**A01N 43/90, A01N 2300/00;**
**A01N 53/00, A01N 2300/00**

## Description

FIELD OF THE INVENTION

This invention relates to certain oxadiazoles, their N-oxides and salts, and to mixtures and compositions comprising such oxadiazoles and methods for using such derivatives and their mixtures and compositions as fungicides.

BACKGROUND OF THE INVENTION

The control of plant diseases caused by fungal plant pathogens is extremely important in achieving high crop efficiency. Plant disease damage to ornamental, vegetable, field, cereal and fruit crops can cause significant reduction in productivity and thereby result in increased costs to the consumer. In addition to often being highly destructive, plant diseases can be difficult to control and may develop resistance to commercial fungicides. Many products are commercially available for these purposes, but the need continues for new fungicidal compounds which are more effective, less costly, less toxic, environmentally safer or have different sites of action. Besides introduction of new fungicides, combinations of fungicides are often used to facilitate disease control, to broaden spectrum of control and to retard resistance development. Furthermore, certain rare combinations of fungicides demonstrate a greater-than-additive effect (i.e. synergistic) to provide commercially important levels of plant disease control. The advantages of particular fungicide combinations are recognized in the art to vary, depending on such factors as the particular plant species and plant disease to be treated, and whether the plants are treated before or after infection with the fungal plant pathogen. Accordingly, new advantageous combinations are needed to provide a variety of options to best satisfy particular plant disease control needs. Such combinations have now been discovered.

SUMMARY OF THE INVENTION

This invention relates to a fungicidal composition (i.e. combination, mixture) comprising

(a) at least one compound selected from the compounds of Formula 1 (including all stereoisomers), tautomers, N-oxides, hydrates (and solvates thereof), and salts thereof,

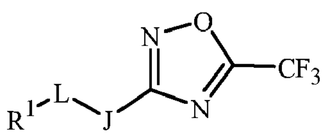

**1**

wherein

$R^1$ is a phenyl ring optionally substituted with up to 3 substituents independently selected from $R^2$; or
$R^1$ is a 5- to 6-membered heteroaromatic ring, each ring containing ring members selected from carbon atoms and 1 to 4 heteroatoms independently selected from up to 2 O, up to 2 S and up to 4 N atoms, wherein up to 2 ring members are independently selected from C(=O), C(=S), S(=O) and $S(=O)_2$, each ring optionally substituted with up to 3 substituents independently selected from $R^2$; or
$R^1$ is a 3- to 7-membered nonaromatic ring or an 8- to 11-membered bicyclic ring system, each ring or ring system containing ring members selected from carbon atoms and optionally up to 4 heteroatoms independently selected from up to 2 O, up to 2 S and up to 4 N atoms, wherein up to 2 ring members are independently selected from C(=O), C(=S), S(=O) and $S(=O)_2$, each ring or ring system optionally substituted with up to 3 substituents independently selected from $R^2$;
L is O, $NR^3$, $NR^3CH_2$, $CH_2NR^3$, $NR^3CH_2CH_2$, $CH_2CH_2NR^3$, $(CR^{4a}R^{4b})_n$, $OCH_2$, $CH_2O$, $OCH_2CH_2$, $CH_2CH_2O$ or $CH_2OCH_2$, wherein the atom to the left is connected to $R^1$, and the atom to the right is connected to J, each carbon atom is optionally substituted with up to 2 substituents independently selected from halogen, cyano, hydroxy, nitro, $C_1$-$C_3$ alkyl, $C_1$-$C_3$ haloalkyl, $C_1$-$C_2$ alkoxy and $C_1$-$C_2$ haloalkoxy;
J is a phenyl ring or a naphthalenyl ring system, each optionally substituted with up to 2 substituents independently selected from $R^5$; or a 3- to 7-membered carbocyclic ring, wherein up to 3 ring members are independently selected from C(=O) and C(=S), each ring optionally substituted with up to 2 substituents independently selected from $R^5$; or
J is a 5- to 6-membered heterocyclic ring, each ring containing ring members selected from carbon atoms and

1 to 4 heteroatoms independently selected from up to 2 O, up to 2 S and up to 4 N atoms, wherein up to 2 ring members are independently selected from C(=O), C(=S), S(=O) and $S(=O)_2$, each ring optionally substituted with up to 2 substituents independently selected from $R^5$;

each $R^2$ is independently halogen, cyano, hydroxy, nitro, thioyl, $SF_5$, CH(=O), C(=O)OH, $-NR^{3a}R^{3b}$, $C(=O)NR^{3a}R^{3b}$, $C(=O)C(=O)NR^{3a}R^{3b}$, $C(=S)NR^{3a}R^{3b}$, $C(R^6)=NR^7$, $N=CR^8NR^{9a}R^{9b}$ or -U-V-Q; or $C_1$-$C_6$ alkyl, $C_2$-$C_6$ alkenyl, $C_2$-$C_6$ alkynyl, $C_3$-$C_7$ cycloalkyl, $C_3$-$C_7$ cycloalkenyl, $C_1$-$C_6$ alkoxy, $C_2$-$C_6$ alkenyloxy, $C_2$-$C_6$ alkynyloxy, $C_3$-$C_7$ cycloalkoxy, $C_1$-$C_6$ alkylthio, $C_1$-$C_6$ alkylsulfinyl, $C_1$-$C_6$ alkylsulfonyl, $C_1$-$C_6$ alkylaminosulfinyl, $C_2$-$C_6$ dialkylaminosulfinyl, $C_1$-$C_6$ alkylsulfonyloxy, $C_1$-$C_6$ alkylsulfonylamino, $C_2$-$C_6$ alkylcarbonyl, $C_4$-$C_7$ cycloalkylcarbonyl, $C_2$-$C_6$ alkoxycarbonyl, $C_3$-$C_6$ alkenyloxycarbonyl, $C_3$-$C_6$ alkynyloxycarbonyl, $C_4$-$C_7$ cycloalkoxycarbonyl, $C_3$-$C_6$ alkyloxycarbonylcarbonyl, $C_2$-$C_6$ alkylcarbonyloxy, $C_4$-$C_7$ cycloalkylcarbonyloxy, $C_2$-$C_6$ alkoxycarbonyloxy, $C_4$-$C_7$ cycloalkoxycarbonyloxy, $C_2$-$C_6$ alkylaminocarbonyloxy, $C_4$-$C_7$ cycloalkylaminocarbonyloxy, $C_2$-$C_6$ alkylcarbonylamino, $C_4$-$C_7$ cycloalkylcarbonylamino, $C_2$-$C_6$ alkoxycarbonylamino, $C_4$-$C_7$ cycloalkoxycarbonylamino, $C_2$-$C_6$ alkylaminocarbonylamino, $C_4$-$C_7$ cycloalkylaminocarbonylamino or $C_2$-$C_6$ dialkoxyphosphinyl, each optionally substituted with up to 3 substituents independently selected from $R^{10}$;

each $R^3$ and $R^{3a}$ is independently H, cyano, hydroxy, $C_1$-$C_4$ alkyl, $C_1$-$C_4$ haloalkyl, $C_2$-$C_4$ alkenyl, $C_2$-$C_4$ haloalkenyl, $C_2$-$C_4$ alkynyl, $C_2$-$C_4$ haloalkynyl, $C_1$-$C_5$ alkoxy, $C_2$-$C_4$ alkoxyalkyl, $C_1$-$C_4$ alkylsulfonyl, $C_1$-$C_4$ haloalkylsulfonyl, $C_2$-$C_4$ alkylthioalkyl, $C_2$-$C_4$ alkylsulfinylalkyl, $C_2$-$C_4$ alkylsulfonylalkyl, $C_2$-$C_4$ alkylcarbonyl, $C_2$-$C_4$ haloalkylcarbonyl, $C_4$-$C_7$ cycloalkylcarbonyl, $C_2$-$C_5$ alkoxycarbonyl, $C_3$-$C_5$ alkoxycarbonylalkyl, $C_2$-$C_5$ alkylaminocarbonyl or $C_3$-$C_5$ dialkylaminocarbonyl;

each $R^{3b}$ is independently H, $C_1$-$C_6$ alkyl, $C_1$-$C_6$ haloalkyl, $C_2$-$C_6$ alkenyl, $C_2$-$C_6$ haloalkenyl, $C_2$-$C_6$ alkynyl, $C_2$-$C_6$ haloalkynyl, $C_3$-$C_6$ hydroxyalkyl, $C_2$-$C_6$ cyanoalkyl, $C_3$-$C_8$ cycloalkyl, $C_3$-$C_8$ halocycloalkyl, $C_3$-$C_8$ cycloalkenyl, $C_3$-$C_8$ halocycloalkenyl, $C_4$-$C_{10}$ alkylcycloalkyl, $C_4$-$C_{10}$ cycloalkylalkyl, $C_4$-$C_{10}$ halocycloalkylalkyl, $C_6$-$C_{14}$ cycloalkylcycloalkyl, $C_5$-$C_{10}$ alkylcycloalkylalkyl, $C_2$-$C_6$ alkoxyalkyl, $C_2$-$C_6$ haloalkoxyalkyl, $C_4$-$C_{10}$ cycloalkoxyalkyl, $C_3$-$C_8$ alkoxyalkoxyalkyl, $C_2$-$C_6$ alkylthioalkyl, $C_2$-$C_6$ alkylsulfinylalkyl, $C_2$-$C_6$ alkylsulfonylalkyl, $C_2$-$C_6$ alkylaminoalkyl, $C_2$-$C_6$ haloalkylaminoalkyl, $C_3$-$C_8$ dialkylaminoalkyl or $C_4$-$C_{10}$ cycloalkylaminoalkyl, each optionally substituted with up to 1 substituent selected from cyano, hydroxy, nitro, $C_2$-$C_4$ alkylcarbonyl, $C_2$-$C_4$ alkoxycarbonyl, $C_3$-$C_{15}$ trialkylsilyl and $C_3$-$C_{15}$ halotrialkylsilyl; or

a pair of $R^{3a}$ and $R^{3b}$ substituents are taken together with the nitrogen atom to which they are attached to form a 4- to 6-membered fully saturated heterocyclic ring, each ring containing ring members, in addition to the connecting nitrogen atom, selected from carbon atoms and up to 2 heteroatoms independently selected from up to 2 O, up to 2 S and up to 2 N atoms, each ring optionally substituted with up to 3 substituents independently selected from halogen and $C_1$-$C_3$ alkyl;

each $R^{4a}$ and $R^{4b}$ is independently H, halogen, cyano, hydroxy, nitro, $C_1$-$C_3$ alkyl, $C_1$-$C_3$ haloalkyl, $C_1$-$C_2$ alkoxy or $C_1$-$C_2$ haloalkoxy; or

a pair of $R^{4a}$ and $R^{4b}$ substituents attached to the same carbon atom are taken together to form a $C_3$-$C_5$ cycloalkyl ring optionally substituted with up to 2 substituents independently selected from halogen, methyl, methoxy and methylthio;

each $R^5$ is independently hydroxy, cyano, nitro, halogen, $C_1$-$C_4$ alkyl, $C_1$-$C_4$ haloalkyl, $C_2$-$C_4$ alkenyl or $C_1$-$C_4$ alkoxy;

each $R^6$ is independently H, cyano, halogen, methyl, methoxy, methylthio or methoxycarbonyl;

each $R^7$ is independently hydroxy or $NR^{11a}R^{11b}$; or $C_1$-$C_4$ alkoxy, $C_2$-$C_4$ alkenyloxy, $C_2$-$C_4$ alkynyloxy, $C_2$-$C_4$ alkylcarbonyloxy, $C_2$-$C_5$ alkoxycarbonyloxy, $C_2$-$C_5$ alkylaminocarbonyloxy or $C_3$-$C_5$ dialkylaminocarbonyloxy, each optionally substituted with up to 1 substituent selected from halogen, cyano, hydroxy and -C(=O)OH;

each $R^8$ is independently H, methyl, methoxy or methylthio;

each $R^{9a}$ and $R^{9b}$ is independently H or $C_1$-$C_4$ alkyl; or

a pair of $R^{9a}$ and $R^{9b}$ substituents are taken together with the nitrogen atom to which they are attached to form a 5- to 6-membered fully saturated heterocyclic ring, each ring containing ring members, in addition to the connecting nitrogen atom, selected from carbon atoms and up to 2 heteroatoms independently selected from up to 2 O, up to 2 S and up to 2 N atoms, each ring optionally substituted with up to 2 methyl;

each $R^{10}$ is independently halogen, amino, cyano, hydroxy, nitro, thioyl, $C_1$-$C_4$ alkyl, $C_1$-$C_4$ haloalkyl, $C_3$-$C_6$ cycloalkyl, $C_3$-$C_6$ halocycloalkyl, $C_1$-$C_4$ alkoxy, $C_1$-$C_4$ haloalkoxy, $C_2$-$C_4$ alkoxyalkoxy, $C_1$-$C_4$ alkylthio, $C_1$-$C_4$ alkylsulfinyl, $C_1$-$C_4$ alkylsulfonyl, $C_1$-$C_4$ haloalkylsulfonyl, $C_2$-$C_4$ alkylcarbonyl, $C_2$-$C_4$ haloalkylcarbonyl, $C_2$-$C_5$ alkoxycarbonyl, $C_1$-$C_6$ alkylamino, $C_2$-$C_6$ dialkylamino, $C_2$-$C_5$ alkylaminocarbonyl, $C_3$-$C_5$ dialkylaminocarbonyl, $C_3$-$C_5$ alkylthioalkylcarbonyl, $C_3$-$C_{15}$ trialkylsily, $C_3$-$C_{15}$ halotrialkylsilyl, $C(R^{13})=NOR^{14}$ or $C(R^{15})=NR^{16}$;

each U is independently a direct bond, C(=O)O, $C(=O)NR^{17}$ or $C(=S)NR^{18}$, wherein the atom to the left is connected to $R^1$, and the atom to the right is connected to V;

each V is independently a direct bond; or $C_1$-$C_6$ alkylene, $C_2$-$C_6$ alkenylene, $C_3$-$C_6$ alkynylene, $C_3$-$C_6$ cycloalkylene or $C_3$-$C_6$ cycloalkenylene, each optionally substituted with up to 3 substituents independently selected from halogen, cyano, nitro, hydroxy, $C_1$-$C_2$ alkyl, $C_1$-$C_2$ haloalkyl, $C_1$-$C_2$ alkoxy and $C_1$-$C_2$ haloalkoxy;
each Q is independently phenyl or phenoxy, each optionally substituted with up to 2 substituents independently selected from $R^{12}$; or
each Q is independently a 5- to 6-membered heteroaromatic ring, each ring containing ring members selected from carbon atoms and 1 to 4 heteroatoms independently selected from up to 2 O, up to 2 S and up to 4 N atoms, wherein up to 2 ring members are independently selected from C(=O), C(=S), S(=O) and $S(=O)_2$, each ring optionally substituted with up to 2 substituents independently selected from $R^{12}$; or
each Q is independently a 3- to 7-membered nonaromatic heterocyclic ring, each ring containing ring members selected from carbon atoms and 1 to 4 heteroatoms independently selected from up to 2 O, up to 2 S and up to 4 N atoms, wherein up to 2 ring members are independently selected from C(=O), C(=S), S(=O) and $S(=O)_2$, each ring optionally substituted with up to 2 substituents independently selected from $R^{12}$;
each $R^{11a}$ is independently H, $C_1$-$C_4$ alkyl or $C_2$-$C_4$ alkylcarbonyl;
each $R^{11b}$ is independently H, cyano, $C_1$-$C_5$ alkyl, $C_2$-$C_5$ alkylcarbonyl, $C_2$-$C_5$ haloalkylcarbonyl, $C_4$-$C_7$ cycloalkylcarbonyl, $C_2$-$C_5$ alkoxycarbonyl, $C_3$-$C_5$ alkoxycarbonylalkyl, $C_2$-$C_5$ alkylaminocarbonyl or $C_3$-$C_5$ dialkylaminocarbonyl; or
a pair of $R^{11a}$ and $R^{11b}$ substituents are taken together with the nitrogen atom to which they are attached to form a 5- to 6-membered fully saturated heterocyclic ring, each ring containing ring members, in addition to the connecting nitrogen atom, selected from carbon atoms and up to 2 heteroatoms independently selected from up to 2 O, up to 2 S and up to 2 N atoms, each ring optionally substituted with up to 2 methyl;
each $R^{12}$ is independently halogen, cyano, hydroxy, nitro, $C_1$-$C_4$ alkyl, $C_1$-$C_4$ haloalkyl, $C_2$-$C_4$ alkenyl, $C_1$-$C_4$ alkoxy, $C_2$-$C_4$ alkylcarbonyl or $C_2$-$C_4$ alkoxycarbonyl;
each $R^{13}$ and $R^{15}$ is independently H, cyano, halogen, $C_1$-$C_3$ alkyl, $C_1$-$C_3$ haloalkyl, $C_3$-$C_6$ cycloalkyl or $C_1$-$C_3$ alkoxy; or a phenyl ring optionally substituted with up to 2 substituents independently selected from halogen and $C_1$-$C_3$ alkyl;
each $R^{14}$ is independently H, $C_1$-$C_5$ alkyl, $C_1$-$C_5$ haloalkyl, $C_2$-$C_5$ alkenyl, $C_2$-$C_5$ haloalkenyl, $C_2$-$C_5$ alkynyl, $C_3$-$C_6$ cycloalkyl, $C_3$-$C_6$ halocycloalkyl, $C_2$-$C_5$ alkylcarbonyl or $C_2$-$C_5$ alkoxycarbonyl; or
each $R^{14}$ is a phenyl ring optionally substituted with up to 2 substituents independently selected from halogen and $C_4$-$C_3$ alkyl; or a 5- to 6-membered fully saturated heterocyclic ring, each ring containing ring members selected from carbon atoms and up to 2 heteroatoms independently selected from up to 2 O, up to 2 S and up to 2 N atoms, each ring optionally substituted with up to 2 substituents independently selected from halogen and $C_4$-$C_3$ alkyl;
each $R^{16}$ is independently H, cyano, $C_1$-$C_3$ alkyl, $C_1$-$C_3$ haloalkyl, $C_1$-$C_4$ alkoxy, $C_2$-$C_4$ alkylcarbonyl or $C_2$-$C_4$ alkoxycarbonyl;
each $R^{17}$ and $R^{18}$ is independently H, cyano, hydroxy, $C_1$-$C_4$ alkyl, $C_1$-$C_4$ haloalkyl, $C_2$-$C_4$ alkylcarbonyl, $C_2$-$C_4$ haloalkylcarbonyl, $C_2$-$C_4$ alkoxycarbonyl or $C_2$-$C_4$ haloalkoxycarbonyl; and
n is 1, 2 or 3; and

(b) at least one additional fungicidal compound.

This invention also relates to a composition comprising: (a) at least one compound selected from the compounds of Formula **1** described above, *N*-oxides, and salts thereof; and at least one invertebrate pest control compound or agent.

This invention also relates to a composition comprising one of the aforesaid compositions comprising component (a) and at least one additional component selected from the group consisting of surfactants, solid diluents and liquid diluents.

This invention also relates to a method for controlling plant diseases caused by fungal plant pathogens comprising applying to the plant or portion thereof, or to the plant seed, a fungicidally effective amount of one of the aforesaid compositions.

The aforedescribed method can also be described as a method for protecting a plant or plant seed from diseases caused by fungal pathogens comprising applying a fungicidally effective amount of one of the aforesaid compositions to the plant (or portion thereof) or plant seed (directly or through the environment (e.g., growing medium) of the plant or plant seed).

This invention also relates to a compound of Formula **1** described above, a tautomer, an *N*-oxide or salt thereof.

## DETAILS OF THE INVENTION

As used herein, the terms "comprises," "comprising," "includes," "including," "has," "having," "contains," "containing," "characterized by" or any other variation thereof, are intended to cover a non-exclusive inclusion, subject to any limitation explicitly indicated. For example, a composition, mixture, process, method, article, or apparatus that

comprises a list of elements is not necessarily limited to only those elements but may include other elements not expressly listed or inherent to such composition, mixture, process, method, article, or apparatus.

The transitional phrase "consisting of" excludes any element, step, or ingredient not specified. If in the claim, such would close the claim to the inclusion of materials other than those recited except for impurities ordinarily associated therewith. When the phrase "consisting of" appears in a clause of the body of a claim, rather than immediately following the preamble, it limits only the element set forth in that clause; other elements are not excluded from the claim as a whole.

The transitional phrase "consisting essentially of' is used to define a composition, method or apparatus that includes materials, steps, features, components, or elements, in addition to those literally disclosed, provided that these additional materials, steps, features, components, or elements do not materially affect the basic and novel characteristic(s) of the claimed invention. The term "consisting essentially of" occupies a middle ground between "comprising" and "consisting of".

Where applicants have defined an invention or a portion thereof with an open-ended term such as "comprising," it should be readily understood that (unless otherwise stated) the description should be interpreted to also describe such an invention using the terms "consisting essentially of' or "consisting of."

Further, unless expressly stated to the contrary, "or" refers to an inclusive or and not to an exclusive or. For example, a condition A or B is satisfied by any one of the following: A is true (or present) and B is false (or not present), A is false (or not present) and B is true (or present), and both A and B are true (or present).

Also, the indefinite articles "a" and "an" preceding an element or component of the invention are intended to be nonrestrictive regarding the number of instances (i.e. occurrences) of the element or component. Therefore "a" or "an" should be read to include one or at least one, and the singular word form of the element or component also includes the plural unless the number is obviously meant to be singular.

The term "agronomic" refers to the production of field crops such as for food and fiber and includes the growth of maize or corn, soybeans and other legumes, rice, cereal (e.g., wheat, oats, barley, rye and rice), leafy vegetables (e.g., lettuce, cabbage, and other cole crops), fruiting vegetables (e.g., tomatoes, pepper, eggplant, crucifers and cucurbits), potatoes, sweet potatoes, grapes, cotton, tree fruits (e.g., pome, stone and citrus), small fruit (e.g., berries and cherries) and other specialty crops (e.g., canola, sunflower and olives).

The term "nonagronomic" refers to other than field crops, such as horticultural crops (e.g., greenhouse, nursery or ornamental plants not grown in a field), residential, agricultural, commercial and industrial structures, turf (e.g., sod farm, pasture, golf course, lawn, sports field, etc.), wood products, stored product, agro-forestry and vegetation management, public health (i.e. human) and animal health (e.g., domesticated animals such as pets, livestock and poultry, undomesticated animals such as wildlife) applications.

The term "crop vigor" refers to rate of growth or biomass accumulation of a crop plant. An "increase in vigor" refers to an increase in growth or biomass accumulation in a crop plant relative to an untreated control crop plant. The term "crop yield" refers to the return on crop material, in terms of both quantity and quality, obtained after harvesting a crop plant. An "increase in crop yield" refers to an increase in crop yield relative to an untreated control crop plant.

The term "biologically effective amount" refers to the amount of a biologically active compound (e.g., a compound of Formula **1** or a mixture with at least one other fungicidal compound) sufficient to produce the desired biological effect when applied to (i.e. contacted with) a fungus to be controlled or its environment, or to a plant, the seed from which the plant is grown, or the locus of the plant (e.g., growth medium) to protect the plant from injury by the fungal disease or for other desired effect (e.g., increasing plant vigor).

As referred to in the present disclosure and claims, "plant" includes members of Kingdom Plantae, particularly seed plants (Spermatopsida), at all life stages, including young plants (e.g., germinating seeds developing into seedlings) and mature, reproductive stages (e.g., plants producing flowers and seeds). Portions of plants include geotropic members typically growing beneath the surface of the growing medium (e.g., soil), such as roots, tubers, bulbs and corms, and also members growing above the growing medium, such as foliage (including stems and leaves), flowers, fruits and seeds.

As referred to herein, the term "seedling", used either alone or in a combination of words means a young plant developing from the embryo of a seed.

As referred to herein, the term "broadleaf' used either alone or in words such as "broadleaf crop" means dicot or dicotyledon, a term used to describe a group of angiosperms characterized by embryos having two cotyledons.

As referred to in this disclosure, the terms "fungal pathogen" and "fungal plant pathogen" include pathogens in the Ascomycota, Basidiomycota and Zygomycota phyla, and the fungal-like Oomycota class that are the causal agents of a broad spectrum of plant diseases of economic importance, affecting ornamental, turf, vegetable, field, cereal and fruit crops. In the context of this disclosure, "protecting a plant from disease" or "control of a plant disease" includes preventative action (interruption of the fungal cycle of infection, colonization, symptom development and spore production) and/or curative action (inhibition of colonization of plant host tissues).

As used herein, the term "mode of action" (MOA) is as define by the Fungicide Resistance Action Committee (FRAC), and is used to distinguish fungicides according to their biochemical mode of action in the biosynthetic pathways of plant pathogens, and their resistance risk. FRAC-defined modes of actions include (A) nucleic acids metabolism, (B)

cytoskeleton and motor protein, (C) respiration, (D) amino acids and protein synthesis, (E) signal transduction, (F) lipid synthesis or transport and membrane integrity or function, (G) sterol biosynthesis in membranes, (H) cell wall biosynthesis, (I) melanin synthesis in cell wall, (P) host plant defense induction, (U) unknown mode of action, (M) chemicals with multi-site activity and (BM) biologicals with multiple modes of action. Each mode of action (i.e. letters A through BM) contain one or more subgroups (e.g., A includes subgroups A1, A2, A3 and A4) based either on individual validated target sites of action, or in cases where the precise target site is unknown, based on cross resistance profiles within a group or in relation to other groups. Each of these subgroups (e.g., A1, A2, A3 and A4) is assigned a FRAC code which is a number and/or letter. For example, the FRAC code for subgroup A1 is 4. Additional information on target sites and FRAC codes can be obtained from publicly available databases maintained, for example, by FRAC.

As used herein, the term "cross resistance" refers to the phenomenon that occurs when a pathogen develops resistance to one fungicide and simultaneously becomes resistant to one or more other fungicides. These other fungicides are typically, but not always, in the same chemical class or have the same target site of action, or can be detoxified by the same mechanism.

In the above recitations, the term "alkyl", used either alone or in compound words such as "alkylthio" or "haloalkyl" includes straight-chain and branched alkyl, such as, methyl, ethyl, n-propyl, i-propyl, and the different butyl, pentyl and hexyl isomers. "Alkenyl" includes straight-chain and branched alkenes such as ethenyl, 1-propenyl, 2-propenyl, and the different butenyl, pentenyl and hexenyl isomers. "Alkenyl" also includes polyenes such as 1,2-propadienyl and 2,4-hexadienyl. "Alkynyl" includes straight-chain and branched alkynes such as ethynyl, 1-propynyl, 2-propynyl, and the different butynyl, pentynyl and hexynyl isomers. "Alkynyl" can also include moieties comprised of multiple triple bonds such as 2,5-hexadiynyl. "Alkylene" denotes a straight-chain or branched alkanediyl. Examples of "alkylene" include $CH_2$, $CH_2CH_2$, $CH(CH_3)$, $CH_2CH_2CH_2$, $CH_2CH(CH_3)$, and the different butylene isomers. "Alkenylene" denotes a straight-chain or branched alkenediyl containing one olefinic bond. Examples of "alkenylene" include CH=CH, $CH_2CH$=CH, CH=$C(CH_3)$ and the different butenylene isomers. The term "cycloalkylene" denotes a cycloalkanediyl ring. Examples of "cycloalkylene" include cyclopropylene, cyclobutylene, cyclopentylene and cyclohexylene. The term "cycloalkenylene" denotes a cycloalkenediyl ring containing one olefinic bond. Examples of "cycloalkenylene" include cylopropenediyl and cyclpentenediyl.

"Alkoxy" includes, for example, methoxy, ethoxy, *n*-propyloxy, *i*-propyloxy, and the different butoxy, pentoxy and hexyloxy isomers. "Alkenyloxy" includes straight-chain and branched alkenyl attached to and linked through an oxygen atom. Examples of "alkenyloxy" include $H_2C{=}CHCH_2O$ and $CH_3CH{=}CHCH_2O$. "Alkynyloxy" includes straight-chain and branched alkynyloxy moieties. Examples of "alkynyloxy" include $HC{\equiv}CCH_2O$ and $CH_3C{\equiv}CCH_2O$.

The term "alkylthio" includes straight-chain and branched alkylthio moieties such as methylthio, ethylthio, and the different propylthio and butylthio isomers. "Alkylsulfinyl" includes both enantiomers of an alkylsulfinyl group. Examples of "alkylsulfinyl" include $CH_3S(=O)$, $CH_3CH_2S(=O)$, $CH_3CH_2CH_2S(=O)$, $(CH_3)_2CHS(=O)$, and the different butylsulfinyl isomers. Examples of "alkylsulfonyl" include $CH_3S(=O)_2$, $CH_3CH_2S(=O)_2$, $CH_3CH_2CH_2S(=O)_2$, $(CH_3)_2CHS(=O)_2$, and the different butylsulfonyl isomers. "Alkylthioalkyl" denotes alkylthio substitution on alkyl. Examples of "alkylthioalkyl" include $CH_3SCH_2$, $CH_3SCH_2CH_2$, $CH_3CH_2SCH_2$, $CH_3CH_2CH_2SCH_2$ and $CH_3CH_2SCH_2CH_2$; "alkylsulfinylalkyl" and "alkylsulfonylalkyl" include the corresponding sulfoxides and sulfones, respectively.

"Alkylamino" includes an NH radical substituted with a straight-chain or branched alkyl group. Examples of "alkylamino" include $CH_3CH_2NH$, $CH_3CH_2CH_2NH$, and $(CH_3)_2CHCH_2NH$. Examples of "dialkylamino" include $(CH_3)_2N$, $(CH_3CH_2CH_2)_2N$ and $CH_3CH_2(CH_3)N$. "Alkylaminoalkyl" denotes alkylamino substitution on alkyl. Examples of "alkylaminoalkyl" include $CH_3NHCH_2$, $CH_3NHCH_2CH_2$, $CH_3CH_2NHCH_2$, $CH_3CH_2CH_2CH_2NHCH_2$ and $CH_3CH_2NHCH_2CH_2$.

"Alkylcarbonyl" denotes a straight-chain or branched alkyl group bonded to a C(=O) moiety. Examples of "alkylcarbonyl" include $CH_3C(=O)$, $CH_3CH_2CH_2C(=O)$ and $(CH_3)_2CHC(=O)$. Examples of "alkoxycarbonyl" include $CH_3OC(=O)$, $CH_3CH_2OC(=O)$, $CH_3CH_2CH_2OC(=O)$, $(CH_3)_2CHOC(=O)$, and the different butoxy- and pentoxycarbonyl isomers. Examples of "alkylaminocarbonyl" include $CH_3NHC(=O)$, $CH_3CH_2NHC(=O)$, $CH_3CH_2CH_2NHC(=O)$, $(CH_3)_2CHNHC(=O)$, and the different butylamino- and pentylaminocarbonyl isomers. Examples of "dialkylaminocarbonyl" include $(CH_3)_2NC(=O)$, $(CH_3CH_2)_2NC(=O)$, $CH_3CH_2(CH_3)NC(=O)$, $(CH_3)_2CH(CH_3)NC(=O)$ and $CH_3CH_2CH_2(CH_3)NC(=O)$.

The term "alkylcarbonylamino" denotes alkyl bonded to a C(=O)NH moiety. Examples of "alkylcarbonylamino" include $CH_3CH_2C(=O)NH$ and $CH_3CH_2CH_2C(=O)NH$. The term "alkoxycarbonylamino" denotes alkoxy bonded to a C(=O)NH moiety. Examples of "alkoxycarbonylamino" include $CH_3OC(=O)NH$ and $CH_3CH_2OC(=O)NH$.

"Alkylsulfonylamino" denotes an NH radical substituted with alkylsulfonyl. Examples of "alkylsulfonylamino" include $CH_3CH_2S(=O)_2NH$ and $(CH_3)_2CHS(=O)_2NH$. The term "alkylsulfonyloxy" denotes an alkylsulfonyl group bonded to an oxygen atom. Examples of "alkylsulfonyloxy" include $CH_3S(=O)_2O$, $CH_3CH_2S(=O)_2O$, $CH_3CH_2CH_2S(=O)_2O$, $(CH_3)_2CHS(=O)_2O$, and the different butylsulfonyloxy, pentylsulfonyloxy and hexylsulfonyloxy isomers.

"Alkoxyalkyl" denotes alkoxy substitution on alkyl. Examples of "alkoxyalkyl" include $CH_3OCH_2$, $CH_3OCH_2CH_2$, $CH_3CH_2OCH_2$, $CH_3CH_2CH_2OCH_2$ and $CH_3CH_2OCH_2CH_2$. "Alkoxyalkoxy" denotes alkoxy substitution on another alkoxy moiety. "Alkoxyalkoxyalkyl" denotes alkoxyalkoxy substitution on alkyl. Examples of "alkoxyalkoxyalkyl" include

$CH_3OCH_2OCH_2$ $CH_3OCH_2OCH_2CH_2$ and $CH_3CH_2OCH_2OCH_2$.

The term "alkylcarbonyloxy" denotes a straight-chain or branched alkyl bonded to a C(=O)O moiety. Examples of "alkylcarbonyloxy" include $CH_3CH_2C(=O)O$ and $(CH_3)_2CHC(=O)O$. Examples of "alkoxycarbonyloxy" include $CH_3CH_2CH_2OC(=O)O$ and $(CH_3)_2CHOC(=O)O$. The term "alkoxycarbonylalkyl" denotes alkoxycarbonyl substitution on alkyl. Examples of "alkoxycarbonylalkyl" include $CH_3CH_2OC(=O)CH_2$, $(CH_3)_2CHOC(=O)CH_2$ and $CH_3OC(=O)CH_2CH_2$. The term "alkylaminocarbonyloxy" denotes a straight-chain or branched alkylaminocarbonyl attached to and linked through an oxygen atom. Examples of "alkylaminocarbonyloxy" include $(CH_3)_2CHCH_2NHC(=O)O$ and $CH_3CH_2NHC(=O)O$.

"Cycloalkyl" includes, for example, cyclopropyl, cyclobutyl, cyclopentyl and cyclohexyl. The term "cycloalkylalkyl" denotes cycloalkyl substitution on an alkyl moiety. Examples of "cycloalkylalkyl" include cyclopropylmethyl, cyclopentylethyl, and other cycloalkyl moieties bonded to a straight-chain or branched alkyl group. The term "alkylcycloalkyl" denotes alkyl substitution on a cycloalkyl moiety and includes, for example, ethylcyclopropyl, *i*-propylcyclobutyl, methylcyclopentyl and methylcyclohexyl. "Alkylcycloalkylalkyl" denotes an alkyl group substituted with alkylcycloalkyl. Examples of "alkylcycloalkylalkyl" include methylcyclohexylmethyl and ethylcycloproylmethyl. "Cycloalkenyl" includes groups such as cyclopentenyl and cyclohexenyl as well as groups with more than one double bond such as 1,3- or 1,4-cyclohexadienyl. The term "cycloalkylcycloalkyl" denotes cycloalkyl substitution on another cycloalkyl ring, wherein each cycloalkyl ring independently has from 3 to 7 carbon atom ring members. Examples of cycloalkylcycloalkyl include cyclopropylcyclopropyl (such as 1,1'-bicyclopropyl-1-yl, 1,1'-bicyclopropyl-2-yl), cyclohexylcyclopentyl (such as 4-cyclopentylcyclohexyl) and cyclohexylcyclohexyl (such as 1,1'-bicyclohexyl-1-yl), and the different *cis*- and *trans*-cycloalkylcycloalkyl isomers, (such as (1*R*,2*S*)-1,1'-bicyclopropyl-2-yl and (1*R*,2*R*)-1,1'-bicyclopropyl-2-yl).

The term "cycloalkoxy" denotes cycloalkyl attached to and linked through an oxygen atom including, for example, cyclopentyloxy and cyclohexyloxy. The term "cycloalkoxyalkyl" denotes cycloalkoxy substitution on an alkyl moiety. Examples of "cycloalkoxyalkyl" include cyclopropyloxymethyl, cyclopentyloxyethyl, and other cycloalkoxy groups bonded to a straight-chain or branched alkyl moiety.

The term "cycloalkylaminoalkyl" denotes cycloalkylamino substitution on an alkyl group. Examples of "cycloalkylaminoalkyl" include cyclopropylaminomethyl, cyclopentylaminoethyl, and other cycloalkylamino moieties bonded to a straight-chain or branched alkyl group.

"Cycloalkylcarbonyl" denotes cycloalkyl bonded to a C(=O) group including, for example, cyclopropylcarbonyl and cyclopentylcarbonyl. Cycloalkylcarbonyloxy" denotes cycloalkylcarbonyl attached to and linked through an oxygen atom. Examples of "cycloalkylcarbonyloxy" include cyclohexylcarbonyloxy and cyclopentylcarbonyloxy. The term "cycloalkoxycarbonyl" means cycloalkoxy bonded to a C(=O) group, for example, cyclopropyloxycarbonyl and cyclopentyloxycarbonyl. "Cycloalkylaminocarbonylamino" denotes cycloalkylamino bonded to a C(=O)NH group, for example, cyclopentylaminocarbonylamino and cyclohexylaminocarbonylamino.

The term "halogen", either alone or in compound words such as "haloalkyl", or when used in descriptions such as "alkyl substituted with halogen" includes fluorine, chlorine, bromine or iodine. Further, when used in compound words such as "haloalkyl", or when used in descriptions such as "alkyl substituted with halogen" said alkyl may be partially or fully substituted with halogen atoms which may be the same or different. Examples of "haloalkyl" or "alkyl substituted with halogen" include $F_3C$, $ClCH_2$, $CF_3CH_2$ and $CF_3CCl_2$. The terms "haloalkenyl", "haloalkynyl" "haloalkoxy", "haloalkylsulfonyl", "halocycloalkyl", and the like, are defined analogously to the term "haloalkyl". Examples of "haloalkenyl" include $Cl_2C{=}CHCH_2$ and $CF_3CH_2CH{=}CHCH_2$. Examples of "haloalkynyl" include $HC{\equiv}CCHCl$, $CF_3C{\equiv}C$, $CCl_3C{\equiv}C$ and $FCH_2C{\equiv}CCH_2$. Examples of "haloalkoxy" include $CF_3O$, $CCl_3CH_2O$, $F_2CHCH_2CH_2O$ and $CF_3CH_2O$. Examples of "haloalkylsulfonyl" include $CF_3S(=O)_2$, $CCl_3S(=O)_2$, $CF_3CH_2S(=O)_2$ and $CF_3CF_2S(=O)_2$. Examples of "halocycloalkyl" include 2-chlorocyclopropyl, 2-fluorocyclobutyl, 3-bromocyclopentyl and 4-chorocyclohexyl.

"Cyanoalkyl" denotes an alkyl group substituted with one cyano group. Examples of "cyanoalkyl" include $NCCH_2$, $NCCH_2CH_2$ and $CH_3CH(CN)CH_2$. "Hydroxyalkyl" denotes an alkyl group substituted with one hydroxy group. Examples of "hydroxyalkyl" include $HOCH_2CH_2$, $CH_3CH_2(OH)CH$ and $HOCH_2CH_2CH_2CH_2$.

The total number of carbon atoms in a substituent group is indicated by the "$C_i$-$C_j$" prefix where i and j are numbers from 1 to 14. For example, $C_1$-$C_4$ alkylsulfonyl designates methylsulfonyl through butylsulfonyl; $C_2$ alkoxyalkyl designates $CH_3OCH_2$; $C_3$ alkoxyalkyl designates, for example, $CH_3CH(OCH_3)$, $CH_3OCH_2CH_2$ or $CH_3CH_2OCH_2$; and $C_4$ alkoxyalkyl designates the various isomers of an alkyl group substituted with an alkoxy group containing a total of four carbon atoms, examples including $CH_3CH_2CH_2OCH_2$ and $CH_3CH_2OCH_2CH_2$.

Generally when a molecular fragment (i.e. radical) is denoted by a series of atom symbols (e.g., C, H, N, O and S) the implicit point or points of attachment will be easily recognized by those skilled in the art. In some instances herein, particularly when alternative points of attachment are possible, the point or points of attachment may be explicitly indicated by a hyphen ("-"). For example, "-SCN" indicates that the point of attachment is the sulfur atom (i.e. thiocyanato, not isothiocyanato).

As used herein, the term "alkylating agent" refers to a chemical compound in which a carbon-containing radical is bound through a carbon atom to a leaving group such as halide or sulfonate, which is displaceable by bonding of a

nucleophile to said carbon atom. Unless otherwise indicated, the term "alkylating" does not limit the carbon-containing radical to alkyl; the carbon-containing radicals in alkylating agents include the variety of carbon-bound substituent radicals specified, for example, for $R^2$.

The term "unsubstituted" in connection with a group such as a ring or ring system means the group does not have any substituents other than its one or more attachments to the remainder of Formula 1. The term "optionally substituted" means that the number of substituents can be zero. Unless otherwise indicated, optionally substituted groups may be substituted with as many optional substituents as can be accommodated by replacing a hydrogen atom with a non-hydrogen substituent on any available carbon or nitrogen atom. Commonly, the number of optional substituents (when present) ranges from 1 to 3. As used herein, the term "optionally substituted" is used interchangeably with the phrase "substituted or unsubstituted" or with the term "(un)substituted."

The number of optional substituents may be restricted by an expressed limitation. For example, the phrase "optionally substituted with up to 3 substituents independently selected from $R^2$" means that 0, 1, 2 or 3 substituents can be present (if the number of potential connection points allows). When a range specified for the number of substituents (e.g., x being an integer from 0 to 2 in Exhibit A) exceeds the number of positions available for substituents on a ring (e.g., 1 position available for $(R^2)_x$ on U-7 in Exhibit A), the actual higher end of the range is recognized to be the number of available positions.

When a compound is substituted with a substituent bearing a subscript that indicates the number of said substituents can vary (e.g., $(R^2)_x$ in Exhibit A wherein x is 1 to 2), then said substituents are independently selected from the group of defined substituents, unless otherwise indicated. When a variable group is shown to be optionally attached to a position, for example $(R^2)_x$ in Exhibit A wherein x may be 0, then hydrogen may be at the position even if not recited in the definition of the variable group.

Naming of substituents in the present disclosure uses recognized terminology providing conciseness in precisely conveying to those skilled in the art the chemical structure. For the sake of conciseness, locant descriptors may be omitted.

Unless otherwise indicated, a "ring" or "ring system" as a component of Formula 1 (e.g., $R^1$ and J) is carbocyclic or heterocyclic. The term "ring system" denotes two or more connected rings. The term "spirocyclic ring system" denotes a ring system consisting of two rings connected at a single atom (so the rings have a single atom in common). The term "bicyclic ring system" denotes a ring system consisting of two rings sharing two or more common atoms. In a "fused bicyclic ring system" the common atoms are adjacent, and therefore the rings share two adjacent atoms and a bond connecting them.

The term "ring member" refers to an atom (e.g., C, O, N or S) or other moiety (e.g., C(=O), C(=S), S(=O) and $S(=O)_2$) forming the backbone of a ring or ring system. The term "aromatic" indicates that each of the ring atoms is essentially in the same plane and has a p-orbital perpendicular to the ring plane, and that $(4n + 2)$ $\pi$ electrons, where n is a positive integer, are associated with the ring to comply with Hückel's rule

The term "carbocyclic ring" denotes a ring wherein the atoms forming the ring backbone are selected only from carbon. Unless otherwise indicated, a carbocyclic ring can be a saturated, partially unsaturated, or fully unsaturated ring. When a fully unsaturated carbocyclic ring satisfies Hückel's rule, then said ring is also called an "aromatic ring". "Saturated carbocyclic" refers to a ring having a backbone consisting of carbon atoms linked to one another by single bonds; unless otherwise specified, the remaining carbon valences are occupied by hydrogen atoms.

As used herein, the term "partially unsaturated ring" or "partially unsaturated heterocycle" refers to a ring which contains unsaturated ring atoms and one or more double bonds but is not aromatic.

The terms "heterocyclic ring" or "heterocycle" denotes a ring wherein at least one of the atoms forming the ring backbone is other than carbon. Unless otherwise indicated, a heterocyclic ring can be a saturated, partially unsaturated, or fully unsaturated ring. When a fully unsaturated heterocyclic ring satisfies Hückel's rule, then said ring is also called a "heteroaromatic ring" or aromatic heterocyclic ring. "Saturated heterocyclic ring" refers to a heterocyclic ring containing only single bonds between ring members.

Unless otherwise indicated, heterocyclic rings and ring systems are attached to the remainder of Formula **1** through any available carbon or nitrogen atom by replacement of a hydrogen on said carbon or nitrogen atom.

Compounds of this invention can exist as one or more stereoisomers. Stereoisomers are isomers of identical constitution but differing in the arrangement of their atoms in space and include enantiomers, diastereomers, *cis*- and *trans*-isomers (also known as geometric isomers) and atropisomers. Atropisomers result from restricted rotation about single bonds where the rotational barrier is high enough to permit isolation of the isomeric species. One skilled in the art will appreciate that one stereoisomer may be more active and/or may exhibit beneficial effects when enriched relative to the other stereoisomer(s) or when separated from the other stereoisomer(s). Additionally, the skilled artisan knows how to separate, enrich, and/or to selectively prepare said stereoisomers. For a comprehensive discussion of all aspects of stereoisomerism, see Ernest L. Eliel and Samuel H. Wilen, Stereochemistry of Organic Compounds, John Wiley & Sons, 1994.

Compounds of this invention can exist as one or more conformational isomers due to restricted rotation about an amide bond (e.g., C(=O)-N) in Formula **1.** This invention comprises mixtures of conformational isomers. In addition,

this invention includes compounds that are enriched in one conformer relative to others.

This invention comprises all stereoisomers, conformational isomers and mixtures thereof in all proportions as well as isotopic forms such as deuterated compounds.

One skilled in the art will appreciate that not all nitrogen containing heterocycles can form *N*-oxides since the nitrogen requires an available lone pair for oxidation to the oxide; one skilled in the art will recognize those nitrogen-containing heterocycles which can form *N*-oxides. One skilled in the art will also recognize that tertiary amines can form *N*-oxides. Synthetic methods for the preparation of *N*-oxides of heterocycles and tertiary amines are very well known by one skilled in the art including the oxidation of heterocycles and tertiary amines with peroxy acids such as peracetic and *m*-chloroperbenzoic acid (MCPBA), hydrogen peroxide, alkyl hydroperoxides such as *t*-butyl hydroperoxide, sodium perborate, and dioxiranes such as dimethyldioxirane. These methods for the preparation of *N*-oxides have been extensively described and reviewed in the literature, see for example: T. L. Gilchrist in Comprehensive Organic Synthesis, vol. 7, pp 748-750, S. V. Ley, Ed., Pergamon Press; M. Tisler and B. Stanovnik in Comprehensive Heterocyclic Chemistry, vol. 3, pp 18-20, A. J. Boulton and A. McKillop, Eds., Pergamon Press; M. R. Grimmett and B. R. T. Keene in Advances in Heterocyclic Chemistry, vol. 43, pp 149-161, A. R. Katritzky, Ed., Academic Press; M. Tisler and B. Stanovnik in Advances in Heterocyclic Chemistry, vol. 9, pp 285-291, A. R. Katritzky and A. J. Boulton, Eds., Academic Press; and G. W. H. Cheeseman and E. S. G. Werstiuk in Advances in Heterocyclic Chemistry, vol. 22, pp 390-392, A. R. Katritzky and A. J. Boulton, Eds., Academic Press.

One skilled in the art recognizes that because in the environment and under physiological conditions salts of chemical compounds are in equilibrium with their corresponding nonsalt forms, salts share the biological utility of the nonsalt forms. Thus a wide variety of salts of the compounds of Formula **1** are useful for control of plant diseases caused by fungal plant pathogens (i.e. are agriculturally suitable). The salts of the compounds of Formula **1** include acid-addition salts with inorganic or organic acids such as hydrobromic, hydrochloric, nitric, phosphoric, sulfuric, acetic, butyric, fumaric, lactic, maleic, malonic, oxalic, propionic, salicylic, tartaric, 4-toluenesulfonic or valeric acids. When a compound of Formula **1** contains an acidic moiety such as a carboxylic acid, salts also include those formed with organic or inorganic bases such as pyridine, triethylamine or ammonia, or amides, hydrides, hydroxides or carbonates of sodium, potassium, lithium, calcium, magnesium or barium. Accordingly, the present invention comprises compounds selected from Formula **1,** *N*-oxides and agriculturally suitable salts, solvates and hydrates thereof.

Compounds selected from Formula **1,** stereoisomers, tautomers, A-oxides, and salts thereof, typically exist in more than one form, and Formula **1** thus includes all crystalline and non-crystalline forms of the compounds that Formula **1** represents. Non-crystalline forms include embodiments which are solids such as waxes and gums as well as embodiments which are liquids such as solutions and melts. Crystalline forms include embodiments which represent essentially a single crystal type and embodiments which represent a mixture of polymorphs (i.e. different crystalline types). The term "polymorph" refers to a particular crystalline form of a chemical compound that can crystallize in different crystalline forms, these forms having different arrangements and/or conformations of the molecules in the crystal lattice. Although polymorphs can have the same chemical composition, they can also differ in composition due to the presence or absence of co-crystallized water or other molecules, which can be weakly or strongly bound in the lattice. Polymorphs can differ in such chemical, physical and biological properties as crystal shape, density, hardness, color, chemical stability, melting point, hygroscopicity, suspensibility, dissolution rate and biological availability. One skilled in the art will appreciate that a polymorph of a compound represented by Formula **1** can exhibit beneficial effects (e.g., suitability for preparation of useful formulations, improved biological performance) relative to another polymorph or a mixture of polymorphs of the same compound represented by Formula **1.** Preparation and isolation of a particular polymorph of a compound represented by Formula **1** can be achieved by methods known to those skilled in the art including, for example, crystallization using selected solvents and temperatures. For a comprehensive discussion of polymorphism see R. Hilfiker, Ed., Polymorphism in the Pharmaceutical Industry, Wiley-VCH, Weinheim, 2006.

The compounds herein, and the agriculturally acceptable salts thereof, may exist in a continuum of solid states ranging from fully amorphous to fully crystalline. They may also exist in unsolvated and solvated forms. The term "solvate" describes a molecular complex comprising the compound and one or more agriculturally acceptable solvent molecules (e.g., EtOH). The term "hydrate" is a solvate in which the solvent is water. Agriculturally acceptable solvates include those in which the solvent may be isotopically substituted (e.g., $D_2O$, $d_6$-acetone, $d_6$-DMSO).

A currently accepted classification system for solvates and hydrates of organic compounds is one that distinguishes between isolated site, channel, and metal-ion coordinated solvates and hydrates. See, e.g., K. R. Morris (H. G. Brittain ed.) Polymorphism in Pharmaceutical Solids (1995). Isolated site solvates and hydrates are ones in which the solvent (e.g., water) molecules are isolated from direct contact with each other by intervening molecules of the organic compound. In channel solvates, the solvent molecules lie in lattice channels where they are next to other solvent molecules. In metal-ion coordinated solvates, the solvent molecules are bonded to the metal ion.

As described in the Summary of the Invention, an aspect of the present invention is directed at a composition comprising (a) at least one compound selected from Formula **1,** *N*-oxides, and salts thereof, with (b) at least one additional fungicidal compound. More particularly, Component (b) is selected from the group consisting of

(b1) methyl benzimidazole carbamate (MBC) fungicides;
(b2) dicarboximide fungicides;
(b3) demethylation inhibitor (DMI) fungicides;
(b4) phenylamide (PA) fungicides;
(b5) amine/morpholine fungicides;
(b6) phospholipid biosynthesis inhibitor fungicides;
(b7) succinate dehydrogenase inhibitor (SDHI) fungicides;
(b8) hydroxy(2-amino-)pyrimidine fungicides;
(b9) anilinopyrimidine (AP) fungicides;
(b10) *N*-phenyl carbamate fungicides;
(b11) quinone outside inhibitor (QoI) fungicides;
(b12) phenylpyrrole (PP) fungicides;
(b13) azanaphthalene fungicides;
(b14) cell peroxidation inhibitor fungicides;
(b15) melanin biosynthesis inhibitor-reductase (MBI-R) fungicides;
(b16a) melanin biosynthesis inhibitor-dehydratase (MBI-D) fungicides;
(b16b) melanin biosynthesis inhibitor-polyketide synthase (MBI-P) fungicides;
(b17) keto reductase inhibitor (KRI) fungicides;
(b18) squalene-epoxidase inhibitor fungicides;
(b19) polyoxin fungicides;
(b20) phenylurea fungicides;
(b21) quinone inside inhibitor (QiI) fungicides;
(b22) benzamide and thiazole carboxamide fungicides;
(b23) enopyranuronic acid antibiotic fungicides;
(b24) hexopyranosyl antibiotic fungicides;
(b25) glucopyranosyl antibiotic: protein synthesis fungicides;
(b26) glucopyranosyl antibiotic fungicides;
(b27) cyanoacetamideoxime fungicides;
(b28) carbamate fungicides;
(b29) oxidative phosphorylation uncoupling fungicides;
(b30) organo tin fungicides;
(b31) carboxylic acid fungicides;
(b32) heteroaromatic fungicides;
(b33) phosphonate fungicides;
(b34) phthalamic acid fungicides;
(b35) benzotriazine fungicides;
(b36) benzene-sulfonamide fungicides;
(b37) pyridazinone fungicides;
(b38) thiophene-carboxamide fungicides;
(b39) complex I NADH oxido-reductase inhibitor fungicides;
(b40) carboxylic acid amide (CAA) fungicides;
(b41) tetracycline antibiotic fungicides;
(b42) thiocarbamate fungicides;
(b43) benzamide fungicides;
(b44) microbial fungicides;
(b45) quinone outside inhibitor, stigmatellin binding (QoSI) fungicides;
(b46) plant extract fungicides;
(b47) cyanoacrylate fungicides;
(b48) polyene fungicides;
(b49) oxysterol binding protein inhibitor (OSBPI) fungicides;
(b50) aryl-phenyl-ketone fungicides;
(b51) host plant defense induction fungicides;
(b52) multi-site activity fungicides;
(b53) biologicals with multiple modes of action;
(b54) fungicides other than fungicides of component (a) and components (b1) through (b53); and
salts of compounds of (b1) through (b54).

Of note are embodiments wherein component (b) comprises at least one fungicidal compound from each of

two different groups selected from (b1) through (b54).

"Methyl benzimidazole carbamate (MBC) fungicides (b1)" (FRAC code 1) inhibit mitosis by binding to β-tubulin during microtubule assembly. Inhibition of microtubule assembly can disrupt cell division, transport within the cell and cell structure. Methyl benzimidazole carbamate fungicides include benzimidazole and thiophanate fungicides. The benzimidazoles include benomyl, carbendazim, fuberidazole and thiabendazole. The thiophanates include thiophanate and thiophanate-methyl.

"Dicarboximide fungicides (b2)" (FRAC code 2) inhibit a mitogen-activated protein (MAP)/histidine kinase in osmotic signal transduction. Examples include chlozolinate, dimethachlone, iprodione, procymidone and vinclozolin.

"Demethylation inhibitor (DMI) fungicides (b3)" (FRAC code 3) (Sterol Biosynthesis Inhibitors (SBI): Class I) inhibit C14-demethylase, which plays a role in sterol production. Sterols, such as ergosterol, are needed for membrane structure and function, making them essential for the development of functional cell walls. Therefore, exposure to these fungicides results in abnormal growth and eventually death of sensitive fungi. DMI fungicides are divided between several chemical classes: piperazines, pyridines, pyrimidines, imidazoles, triazoles and triazolinthiones. The piperazines include triforine. The pyridines include buthiobate, pyrifenox, pyrisoxazole and (α*S*)-[3-(4-chloro-2-fluorophenyl)-5-(2,4-difluorophenyl)-4-isoxazolyl]-3-pyridinemethanol. The pyrimidines include fenarimol, nuarimol and triarimol. The imidazoles include econazole, imazalil, oxpoconazole, pefurazoate, prochloraz and triflumizole. The triazoles include azaconazole, bitertanol, bromuconazole, cyproconazole, difenoconazole, diniconazole (including diniconazole-M), epoxiconazole, etaconazole, fenbuconazole, fluquinconazole, flusilazole, flutriafol, hexaconazole, imibenconazole, ipconazole, ipfentrifluconazole, mefentrifluconazole, metconazole, myclobutanil, penconazole, propiconazole, quinconazole, simeconazole, tebuconazole, tetraconazole, triadimefon, triadimenol, triticonazole, uniconazole, uniconazole-P, α-(1-chlorocyclopropyl)-α-[2-(2,2-dichlorocyclopropyl)ethyl]-1*H*-1,2,4-triazole-1-ethanol, *rel*-1-[[(2*R*,3*S*)-3-(2-chlorophenyl)-2-(2,4-difluorophenyl)-2-oxiranyl]methyl]-1*H*-1,2,4-triazole, *rel*-2-[[(2*R*,3*S*)-3-(2-chlorophenyl)-2-(2,4-difluorophenyl)-2-oxiranyl]methyl]-1,2-dihydro-3*H* 1,2,4-triazole-3-thione and *rel-1*-[[(2*R*,3*S*)-3-(2-chlorophenyl)-2-(2,4-difluorophenyl)-2-oxlranyl]methyl]-5-(2-propen-1-ylthio)-1*H*-1,2,4-triazole. The triazolinthiones include prothioconazole. Biochemical investigations have shown that all of the above mentioned fungicides are DMI fungicides as described by K. H. Kuck et al. in Modern Selective Fungicides - Properties, Applications and Mechanisms of Action, H. Lyr (Ed.), Gustav Fischer Verlag: New York, 1995, 205-258.

"Phenylamide (PA) fungicides (b4)" (FRAC code 4) are specific inhibitors of RNA polymerase in Oomycete fungi. Sensitive fungi exposed to these fungicides show a reduced capacity to incorporate uridine into rRNA. Growth and development in sensitive fungi is prevented by exposure to this class of fungicide. Phenylamide fungicides include acylalanine, oxazolidinone and butyrolactone fungicides. The acylalanines include benalaxyl, benalaxyl-M (also known as kiralaxyl), furalaxyl, metalaxyl and metalaxyl-M (also known as mefenoxam). The oxazolidinones include oxadixyl. The butyrolactones include ofurace.

"Amine/morpholine fungicides (b5)" (FRAC code 5) (SBI: Class II) inhibit two target sites within the sterol biosynthetic pathway, $\Delta^8 \rightarrow \Delta^7$ isomerase and $\Delta^{14}$ reductase. Sterols, such as ergosterol, are needed for membrane structure and function, making them essential for the development of functional cell walls. Therefore, exposure to these fungicides results in abnormal growth and eventually death of sensitive fungi. Amine/morpholine fungicides (also known as non-DMI sterol biosynthesis inhibitors) include morpholine, piperidine and spiroketal-amine fungicides. The morpholines include aldimorph, dodemorph, fenpropimorph, tridemorph and trimorphamide. The piperidines include fenpropidin and piperalin. The spiroketal-amines include spiroxamine.

"Phospholipid biosynthesis inhibitor fungicides (b6)" (FRAC code 6) inhibit growth of fungi by affecting phospholipid biosynthesis. Phospholipid biosynthesis fungicides include phophorothiolate and dithiolane fungicides. The phosphorothiolates include edifenphos, iprobenfos and pyrazophos. The dithiolanes include isoprothiolane.

"Succinate dehydrogenase inhibitor (SDHI) fungicides (b7)" (FRAC code 7) inhibit complex II fungal respiration by disrupting a key enzyme in the Krebs Cycle (TCA cycle) named succinate dehydrogenase. Inhibiting respiration prevents the fungus from making ATP, and thus inhibits growth and reproduction. SDHI fungicides include phenylbenzamide, phenyloxoethylthiophene amide, pyridinylethylbenzamide, furan carboxamide, oxathiin carboxamide, thiazole carboxamide, pyrazole-4-carboxamide, *N*-cyclopropyl-*N*-benzyl-pyrazole carboxamide, *N*-methoxy-(phenyl-ethyl)-pyrazole carboxamide, pyridine carboxamide and pyrazine carboxamide fungicides. The phenylbenzamides include benodanil, flutolanil and mepronil. The phenyloxoethylthiophene amides include isofetamid. The pyridinylethylbenzamides include fluopyram. The furan carboxamides include fenfuram. The oxathiin carboxamides include carboxin and oxycarboxin. The thiazole carboxamides include thifluzamide. The pyrazole-4-carboxamides include benzovindiflupyr, bixafen, flubeneteram (provisional common name, Registry Number 1676101-39-5), fluindapyr, fluxapyroxad, furametpyr, inpyrfluxam, isopyrazam, penflufen, penthiopyrad, pyrapropoyne (provisional common name, Registry Number 1803108-03-3), sedaxane and *N*-[2-(2,4-dichlorophenyl)-2-methoxy-1-methylethyl]-3-(difluoromethyl)-1-methyl-1*H*-pyrazole-4-carboxamide. The *N*-cyclopropyl-N-benzyl-pyrazole carboxamides include isoflucypram. The *N*-methoxy-(phenylethyl)-pyrazole carboxamides include pydiflumetofen. The pyridine carboxamides include boscalid. The pyrazine carboxamides include pyraziflumid.

"Hydroxy-(2-amino-)pyrimidine fungicides (b8)" (FRAC code 8) inhibit nucleic acid synthesis by interfering with adenosine deaminase. Examples include bupirimate, dimethirimol and ethirimol.

"Anilinopyrimidine (AP) fungicides (b9)" (FRAC code 9) are proposed to inhibit biosynthesis of the amino acid methionine and to disrupt the secretion of hydrolytic enzymes that lyse plant cells during infection. Examples include cyprodinil, mepanipyrim and pyrimethanil.

"*N*-Phenyl carbamate fungicides (b10)" (FRAC code 10) inhibit mitosis by binding to β-tubulin and disrupting microtubule assembly. Inhibition of microtubule assembly can disrupt cell division, transport within the cell and cell structure. Examples include diethofencarb.

"Quinone outside inhibitor (QoI) fungicides (b11)" (FRAC code 11) inhibit complex III mitochondrial respiration in fungi by affecting ubiquinol oxidase. Oxidation of ubiquinol is blocked at the "quinone outside" (Qo) site of the cytochrome $bc_1$ complex, which is located in the inner mitochondrial membrane of fungi. Inhibiting mitochondrial respiration prevents normal fungal growth and development. Quinone outside inhibitor fungicides include methoxyacrylate, methoxyacetamide, methoxycarbamate, oximinoacetate, oximinoacetamide and dihydrodioxazine fungicides (collectively also known as strobilurin fungicides), and oxazolidinedione, imidazolinone and benzylcarbamate fungicides. The methoxyacrylates include azoxystrobin, coumoxystrobin, enoxastrobin (also known as enestroburin), flufenoxystrobin, picoxystrobin and pyraoxystrobin. The methoxyacetamides include mandestrobin. The methoxy-carbamates include pyraclostrobin, pyrametostrobin and triclopyricarb. The oximinoacetates include kresoxim-methyl and trifloxystrobin. The oximinoacetamides include dimoxystrobin, fenaminstrobin, metominostrobin and orysastrobin. The dihydrodioxazines include fluoxastrobin. The oxazolidinediones include famoxadone. The imidazolinones include fenamidone. The benzylcarbamates include pyribencarb.

"Phenylpyrrole (PP) fungicides (b12)" (FRAC code 12) inhibit a MAP/histidine kinase associated with osmotic signal transduction in fungi. Fenpiclonil and fludioxonil are examples of this fungicide class.

"Azanaphthalene fungicides (b13)" (FRAC code 13) are proposed to inhibit signal transduction by a mechanism which is as yet unknown. They have been shown to interfere with germination and/or appressorium formation in fungi that cause powdery mildew diseases. Azanaphthalene fungicides include aryloxyquinolines and quinazolinones. The aryloxyquinolines include quinoxyfen. The quinazolinones include proquinazid.

"Cell peroxidation inhibitor fungicides (b14)" (FRAC code 14) are proposed to inhibit lipid peroxidation which affects membrane synthesis in fungi. Members of this class, such as etridiazole, may also affect other biological processes such as respiration and melanin biosynthesis. Cell peroxidation fungicides include aromatic hydrocarbon and 1,2,4-thiadiazole fungicides. The aromatic hydrocarboncarbon fungicides include biphenyl, chloroneb, dicloran, quintozene, tecnazene and tolclofos-methyl. The 1,2,4-thiadiazoles include etridiazole.

"Melanin biosynthesis inhibitor-reductase (MBI-R) fungicides (b15)" (FRAC code 16.1) inhibit the naphthal reduction step in melanin biosynthesis. Melanin is required for host plant infection by some fungi. Melanin biosynthesis inhibitor-reductase fungicides include isobenzofuranone, pyrroloquinolinone and triazolobenzothiazole fungicides. The isobenzofuranones include fthalide. The pyrroloquinolinones include pyroquilon. The triazolobenzothiazoles include tricyclazole.

"Melanin biosynthesis inhibitor-dehydratase (MBI-D) fungicides (b16a)" (FRAC code 16.2) inhibit scytalone dehydratase in melanin biosynthesis. Melanin is required for host plant infection by some fungi. Melanin biosynthesis inhibitor-dehydratase fungicides include cyclopropanecarboxamide, carboxamide and propionamide fungicides. The cyclopropanecarboxamides include carpropamid. The carboxamides include diclocymet. The propionamides include fenoxanil.

"Melanin biosynthesis inhibitor-polyketide synthase (MBI-P) fungicides (b16b)" (FRAC code 16.3) inhibit polyketide synthase in melanin biosynthesis. Melanin is required for host plant infection by some fungi. Melanin biosynthesis inhibitor-polyketide synthase fungicides include trifluoroethylcarbamate fungicides. The trifluoroethylcarbamates include tolprocarb.

"Keto reductase inhibitor (KRI) fungicides (b17)" (FRAC code 17) inhibit 3-keto reductase during C4-demethylation in sterol production. Keto reductase inhibitor fungicides (also known as Sterol Biosynthesis Inhibitors (SBI): Class III) include hydroxyanilides and amino-pyrazolinones. Hydroxyanilides include fenhexamid. Amino-pyrazolinones include fenpyrazamine. Quinofumelin (provisional common name, Registry Number 861647-84-9) and ipflufenoquin (provisional common name, Registry Number 1314008-27-9) are also believed to be keto reductase inhibitor fungicides.

"Squalene-epoxidase inhibitor fungicides (b18)" (FRAC code 18) (SBI: Class IV) inhibit squalene-epoxidase in the sterol biosynthesis pathway. Sterols such as ergosterol are needed for membrane structure and function, making them essential for the development of functional cell walls. Therefore exposure to these fungicides results in abnormal growth and eventually death of sensitive fungi. Squalene-epoxidase inhibitor fungicides include thiocarbamate and allylamine fungicides. The thiocarbamates include pyributicarb. The allylamines include naftifine and terbinafine.

"Polyoxin fungicides (b19)" (FRAC code 19) inhibit chitin synthase. Examples include polyoxin.

"Phenylurea fungicides (b20)" (FRAC code 20) are proposed to affect cell division. Examples include pencycuron.

"Quinone inside inhibitor (QiI) fungicides (b21)" (FRAC code 21) inhibit complex III mitochondrial respiration in fungi by affecting ubiquinone reductase. Reduction of ubiquinone is blocked at the "quinone inside" (Qi) site of the cytochrome *bc*$_1$ complex, which is located in the inner mitochondrial membrane of fungi. Inhibiting mitochondrial respiration prevents normal fungal growth and development. Quinone inside inhibitor fungicides include cyanoimidazole, sulfamoyltriazole and picolinamide fungicides. The cyanoimidazoles include cyazofamid. The sulfamoyltriazoles include amisulbrom. The picolinamides include fenpicoxamid (Registry Number 517875-34-2).

"Benzamide and thiazole carboxamide fungicides (b22)" (FRAC code 22) inhibit mitosis by binding to β-tubulin and disrupting microtubule assembly. Inhibition of microtubule assembly can disrupt cell division, transport within the cell and cell structure. The benzamides include toluamides such as zoxamide. The thiazole carboxamides include ethylaminothiazole carboxamides such as ethaboxam.

"Enopyranuronic acid antibiotic fungicides (b23)" (FRAC code 23) inhibit growth of fungi by affecting protein biosynthesis. Examples include blasticidin-S.

"Hexopyranosyl antibiotic fungicides (b24)" (FRAC code 24) inhibit growth of fungi by affecting protein biosynthesis. Examples include kasugamycin.

"Glucopyranosyl antibiotic: protein synthesis fungicides (b25)" (FRAC code 25) inhibit growth of fungi by affecting protein biosynthesis. Examples include streptomycin.

"Glucopyranosyl antibiotic fungicides (b26)" (FRAC code U18, previously FRAC code 26 reclassified to U18) are proposed to inhibit trehalase and inositol biosynthesis. Examples include validamycin.

"Cyanoacetamideoxime fungicides (b27)" (FRAC code 27) include cymoxanil.

"Carbamate fungicides (b28)" (FRAC code 28) are considered multi-site inhibitors of fungal growth. They are proposed to interfere with the synthesis of fatty acids in cell membranes, which then disrupts cell membrane permeability. Iodocarb, propamacarb and prothiocarb are examples of this fungicide class.

"Oxidative phosphorylation uncoupling fungicides (b29)" (FRAC code 29) inhibit fungal respiration by uncoupling oxidative phosphorylation. Inhibiting respiration prevents normal fungal growth and development. This class includes dinitrophenyl crotonates such as binapacryl, meptyldinocap and dinocap, and 2,6-dinitroanilines such as fluazinam.

"Organo tin fungicides (b30)" (FRAC code 30) inhibit adenosine triphosphate (ATP) synthase in oxidative phosphorylation pathway. Examples include fentin acetate, fentin chloride and fentin hydroxide.

"Carboxylic acid fungicides (b31)" (FRAC code 31) inhibit growth of fungi by affecting deoxyribonucleic acid (DNA) topoisomerase type II (gyrase). Examples include oxolinic acid.

"Heteroaromatic fungicides (b32)" (FRAC code 32) are proposed to affect DNA/ribonucleic acid (RNA) synthesis. Heteroaromatic fungicides include isoxazoles and isothiazolones. The isoxazoles include hymexazole and the isothiazolones include octhilinone.

"Phosphonate fungicides (b33)" (FRAC code P07, previously FRAC code 33 reclassified to P07) include phosphorous acid and its various salts, including fosetyl-aluminum.

"Phthalamic acid fungicides (b34)" (FRAC code 34) include teclofthalam.

"Benzotriazine fungicides (b35)" (FRAC code 35) include triazoxide.

"Benzene-sulfonamide fungicides (b36)" (FRAC code 36) include flusulfamide.

"Pyridazinone fungicides (b37)" (FRAC code 37) include diclomezine.

"Thiophene-carboxamide fungicides (b38)" (FRAC code 38) are proposed to affect ATP production. Examples include silthiofam.

"Complex I NADH oxidoreductase inhibitor fungicides (b39)" (FRAC code 39) inhibit electron transport in mitochondria and include pyrimidinamines such as diflumetorim, pyrazole-5-carboxamides such as tolfenpyrad, and quinazoline such as fenazaquin.

"Carboxylic acid amide (CAA) fungicides (b40)" (FRAC code 40) inhibit cellulose synthase which prevents growth and leads to death of the target fungus. Carboxylic acid amide fungicides include cinnamic acid amide, valinamide carbamate and mandelic acid amide fungicides. The cinnamic acid amides include dimethomorph, flumorph and pyrimorph. The valinamide carbamates include benthiavalicarb, benthiavalicarb-isopropyl, iprovalicarb, tolprocarb and valifenalate (also known as valiphenal). The mandelic acid amides include mandipropamid, N-[2-[4-[[3-(4-chlorophenyl)-2-propyn-1-yl]oxy]-3-methoxyphenyl]ethyl]-3-methyl-2-[(methylsulfonyl)amino]butanamide and *N*-[2-[4-[[3-(4-chlorophenyl)-2-propyn-1-yl]oxy]-3-methoxyphenyl]ethyl]-3-methyl-2-[(ethylsulfonyl)amino]butanamide.

"Tetracycline antibiotic fungicides (b41)" (FRAC code 41) inhibit growth of fungi by affecting protein synthesis. Examples include oxytetracycline.

"Thiocarbamate fungicides (b42)" (FRAC code M12, previously FRAC code 42 reclassified to M12) include methasulfocarb.

"Benzamide fungicides (b43)" (FRAC code 43) inhibit growth of fungi by delocalization of spectrin-like proteins. Examples include pyridinylmethyl benzamides such as fluopicolide and fluopimomide.

"Microbial fungicides (b44)" (FRAC code BM02, previously FRAC code 44 reclassified to BM02) disrupt fungal pathogen cell membranes. Microbial fungicides include *Bacillus* species such as *Bacillus amyloliquefaciens* strains AP-136, AP-188, AP-218, AP-219, AP-295, QST713, FZB24, F727, MB1600, D747, TJ100 (also called strain 1 BE; known from EP2962568), and the fungicidal lipopeptides which they produce.

"Quinone outside inhibitor, stigmatellin binding (QoSI) fungicides (b45)" (FRAC code 45) inhibit complex III mitochondrial respiration in fungi by affecting ubiquinone reductase at the "quinone outside" (Qo) site, stigmatellin binding sub-site, of the cytochrome $bc_1$ complex. Inhibiting mitochondrial respiration prevents normal fungal growth and development. QoSI fungicides include triazolopyrimidylamines such as ametoctradin.

"Plant extract fungicides (b46)" (FRAC code 46) cause cell membrane disruption. Plant extract fungicides include terpene hydrocarbons, terpene alcohols and terpen phenols such as the extract from *Melaleuca alternifolia* (tea tree) and plant oils (mixtures) such as eugenol, geraniol and thymol.

"Cyanoacrylate fungicides (b47)" (FRAC code 47) bind to the myosin motor domain and effect motor activity and actin assembly. Cyanoacrylates include fungicides such as phenamacril.

"Polyene fungicides (b48)" (FRAC code 48) cause disruption of the fungal cell membrane by binding to ergosterol, the main sterol in the membrane. Examples include natamycin (pimaricin).

"Oxysterol binding protein inhibitor (OSBPI) Fungicides (b49)" (FRAC code 49) bind to the oxysterol-binding protein in oomycetes causing inhibition of zoospore release, zoospore motility and sporangia germination. Oxysterol binding fungicides include piperdinylthiazoleisoxazolines such as oxathiapiprolin and fluoxapiprolin.

"Aryl-phenyl-ketone fungicides (b50)" (FRAC code 50, previously FRAC code U8 reclassified to 50) inhibit the growth of mycelium in fungi. Aryl-phenyl ketone fungicides include benzophenones such as metrafenone, and benzoylpyridines such as pyriofenone.

"Host plant defense induction fungicides (b51)" induce host plant defense mechanisms. Host plant defense induction fungicides include benzothiadiazole (FRAC code P01), benzisothiazole (FRAC code P02), thiadiazole carboxamide (FRAC code P03), polysaccharide (FRAC code P04), plant extract (FRAC code P05), microbial (FRAC code P06) and phosphonate fungicides (FRAC code P07, see (b33) above). The benzothiadiazoles include acibenzolar-S-methyl. The benzisothiazoles include probenazole. The thiadiazole carboxamides include tiadinil and isotianil. The polysaccharides include laminarin. The plant extracts include extract from *Reynoutria sachalinensis* (giant knotweed). The microbials include *Bacillus mycoides* isolate J and cell walls of *Saccharomyces cerevisiae* strain LAS 117.

"Multi-site activity fungicides (b52)" inhibit fungal growth through multiple sites of action and have contact/preventive activity. Multi-site activity fungicides include copper fungicides (FRAC code M01), sulfur fungicides (FRAC code M02), dithiocarbamate fungicides (FRAC code M03), phthalimide fungicides (FRAC code M04), chloronitrile fungicides (FRAC code M05), sulfamide fungicides (FRAC code M06), multi-site contact guanidine fungicides (FRAC code M07), triazine fungicides (FRAC code M08), quinone fungicides (FRAC code M09), quinoxaline fungicides (FRAC code M10), maleimide fungicides (FRAC code M11) and thiocarbamate (FRAC code M12, see (b42) above) fungicides. Copper fungicides are inorganic compounds containing copper, typically in the copper(II) oxidation state; examples include copper oxychloride, copper sulfate and copper hydroxide, including compositions such as Bordeaux mixture (tribasic copper sulfate) and Kocide. Sulfur fungicides are inorganic chemicals containing rings or chains of sulfur atoms; examples include elemental sulfur. Dithiocarbamate fungicides contain a dithiocarbamate molecular moiety; examples include ferbam, mancozeb, maneb, metiram, propineb, thiram, zinc thiazole, zineb and ziram. Phthalimide fungicides contain a phthalimide molecular moiety; examples include folpet, captan and captafol. Chloronitrile fungicides contain an aromatic ring substituted with chloro and cyano; examples include chlorothalonil. Sulfamide fungicides include dichlofluanid and tolyfluanid. Multi-site contact guanidine fungicides include, guazatine, iminoctadine albesilate and iminoctadine triacetate. Triazine fungicides include anilazine. Quinone fungicides include dithianon. Quinoxaline fungicides include quinomethionate (also known as chinomethionate). Maleimide fungicides include fluoroimide.

"Biologicals with multiple modes of action (b53)" include agents from biological origins showing multiple mechanisms of action without evidence of a dominating mode of action. This class of fungicides includes polypeptide (lectin), phenol, sesquiterpene, tritepenoid and coumarin fungicides (FRAC code BM01) such as extract from the cotyledons of lupine plantlets. This class also includes microbial fungicides (FRAC code BM02, see (b44) above).

"Fungicides other than fungicides of component (a) and components (b1) through (b53); (b54)"; include certain fungicides whose mode of action may be unknown. These include: (b54.1) "phenyl-acetamide fungicides" (FRAC code U06), (b54.2) "guanidine fungicides" (FRAC code U12), (b54.3) "thiazolidine fungicides" (FRAC code U13), (b54.4) "pyrimidinone-hydrazone fungicides" (FRAC code U14), (b54.5) "4-quinolylacetate fungicides" (FRAC code U16), (54.6) "tetrazolyloxime fungicides" (FRAC code U17) and "glucopyranosyl antibiotic fungicides" (FRAC code U18, see (b26) above). The phenyl-acetamides include cyflufenamid. The guanidines include dodine. The thiazolidines include flutianil. The pyrimidinonehydrazones include ferimzone. The 4-quinolylacetates include tebufloquin. The tetrazolyloximes include picarbutrazox.

The (b54) class also includes bethoxazin, dichlobentiazox (provisional common name, Registry Number 957144-77-3), dipymetitrone (provisional common name, Registry Number 16114-35-5), flometoquin, neo-asozin (ferric methanearsonate), pyrrolnitrin, tolnifanide (Registry Number 304911-98-6), *N'*-[4-[4-chloro-3-(trifluoromethyl)phenoxy]-2,5-dimethylphenyl]-*N*-ethyl-*N*-methylmethanimidamide, 5-fluoro-2-[(4-fluorophenyl)methoxy]-4-pyrimidinamine and 4-fluorophenyl *N*-[1-[[[1-(4-cyanophenyl)ethyl]sulfonyl]methyl]propyl]carbamate.

The (b54) class additionally includes fungicides whose mode of action may be unknown, or may not yet be classified, such as a fungicidal compound selected from components (b54.7) through (b54.14), as described below.

Component (54.7) relates to (1*S*)-2,2-bis(4-fluorophenyl)-1-methylethyl *N*-[[3-(acetyloxy)-4-methoxy-2-pyridinyl]carbonyl]-L-alaninate (provisional common name florylpicoxamid, Registry Number 1961312-55-9) which is believed to be a Quinone inside inhibitor (QiI) fungicide (FRAC code 21) inhibiting the Complex III mitochondrial respiration in fungi.

Component (54.8) relates to 1-[2-[[[1-(4-chlorophenyl)-1*H*-pyrazol-3-yl]oxy]methyl]-3-methylphenyl]-1,4-dihydro-4-methyl-5*H*-tetrazol-5-one (provisional common name metyltetraprole, Registry Number 1472649-01-6), which is believed to be a quinone outside inhibitor (QoI) fungicide (FRAC code 45) inhibiting the Complex III mitochondrial respiration in fungi, and is effective against QoI resistant strains.

Component (54.9) relates to 3-chloro-4-(2,6-difluorophenyl)-6-methyl-5-phenylpyridazine (provisional common name pyridachlometyl, Registry Number 1358061-55-8), which is believed to be promoter tubulin polymerization, resulting antifungal activity against fungal species belonging to the phyla Ascomycota and Basidiomycota.

Component (54.10) relates to (4-phenoxyphenyl)methyl 2-amino-6-methyl-pyridine-3-carboxylate (provisional common name aminopyrifen, Registry Number 1531626-08-0) which is believed to inhibit GWT-1 protein in glycosylphosphatidylinositol-anchor biosynthesis in Neurospora crassa.

Component (b54.11) relates to a compound of Formula **b54.11**

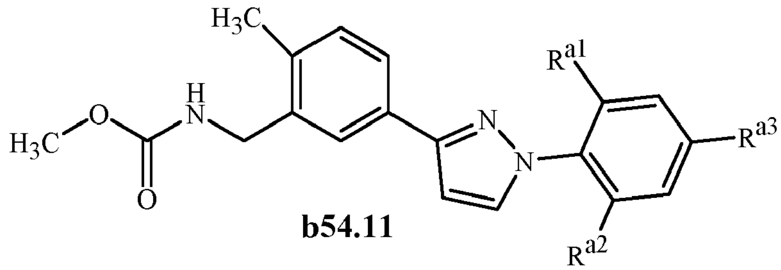

**b54.11**

wherein

$R^{a1}$ and $R^{a2}$ are each independently halogen; and
$R^{a3}$ is H, halogen, $C_1$-$C_3$ alkyl, $C_1$-$C_3$ haloalkyl or $C_3$-$C_6$ cycloalkyl.

Examples of compounds of Formula **b54.11** include (b54.11a) methyl *N*-[[5-[1-[2,6-difluoro-4-(1-methylethyl)phenyl]-1*H*-pyrazol-3-yl]-2-methylphenyl]methyl]carbamate, (b54.11b) methyl *N*-[[5-[1-(4-chloro-2,6-difluorophenyl)-1*H*-pyrazol-3-yl]-2-methylphenyl] methyl]carbamate, (b54.11c) methyl *N*-[[5-[1-[2,6-difluoro-4-(trifluoromethyl)phenyl]-1*H*-pyrazol-3-yl]-2-methylphenyl]methyl]carbamate, (b54.11d) methyl *N*-[[5-[1-(4-cyclopropyl-2,6-diffuorophenyl)-1*H*-pyrazol-3-yl]-2-methylphenyl]methyl]carbamate and (b54.11e) methyl *N*-[[5-[1-(2,6-dichloro-4-cyclopropylphenyl)-1*H*-pyrazol-3-yl]-2-methylphenyl]methyl]carbamate. Compounds of Formula **b54.11,** their use as fungicides and methods of preparation are generally known; see, for example, PCT Patent Publications WO 2008/124092 and WO 2020/097012.

Component (b54.12) relates to a compound of Formula **b54.12**

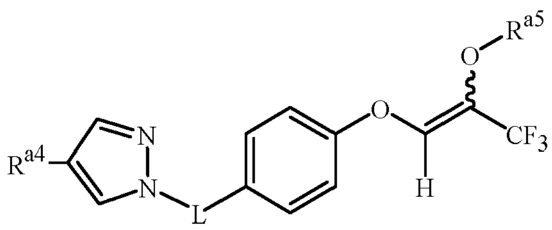

**b54.12**

wherein

$R^{a4}$ is $C_2$-$C_5$ alkoxycarbonyl or $C_3$-$C_5$ alkenyloxycarbonyl, each optionally substituted with up to 3 substituents independently selected from $R^{a6}$;
L is $CH_2$, $CH_2CH_2$ or $CH_2O$, wherein O is connected to the phenyl ring;

$R^{35}$ is $C_1$-$C_4$ alkyl, $C_1$-$C_4$ haloalkyl, $C_2$-$C_4$ alkenyl and $C_2$-$C_4$ haloalkenyl; and
$R^{a6}$ is cyano, halogen, cyclopropyl or methoxy;

In Formula **b54.12** the wavy bond indicates a single bond which is linked to an adjacent double bond wherein the geometry about the adjacent double bond is either (*Z*)-configuration (syn-isomer or *cis*-isomer) or (*E*)-configuration (anti-isomer or *trans*-isomer), or a mixture thereof.

Examples of compounds of Formula **b54.12** include (b54.12a) ethyl 1-[[4-[[(1*Z*)-2-ethoxy-3,3,3-trifluoro-1-propen-1-yl]oxy]phenyl]methyl]-1*H*-pyrazole-4-carboxylate and its (*E*)-isomer and (b54.12b) ethyl 1-[2-[4-[[(1*Z*)-2-ethoxy-3,3,3-trifluoro-1-propen-1-yl]oxy]phenyl]ethyl]-1*H* pyrazole-4-carboxylate and its (*E*)-isomer. Compounds of Formula **b54.12,** their use as fungicides and methods of preparation are generally known; see, for example, PCT Patent Publication WO2020/056090.

Component (b54.13) relates to a compound of Formula **b54.13**

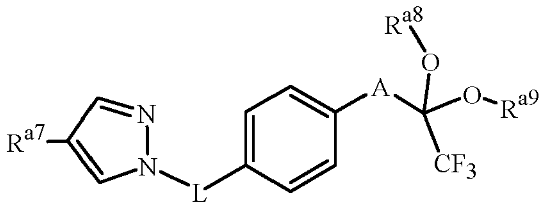

**b54.13**

wherein

$R^{a7}$ is $C_2$-$C_5$ alkoxycarbonyl or $C_3$-$C_5$ alkenyloxycarbonyl, each optionally substituted with up to 3 substituents independently selected from $R^{a10}$;
L is $CH_2$, $CH_2CH_2$ or $CH_2O$, wherein O is connected to the phenyl ring;
$R^{a8}$ and $R^{9b}$ are each independently H, $C_1$-$C_4$ alkyl; or
$R^{a8}$ and $R^{a9}$ are taken together with the oxygen atoms to which they are attached to form a 5-membered saturated ring containing ring members, in addition to the oxygen atoms, selected from carbon atoms, the ring optionally substituted with up to 2 substituents independently selected from halogen, cyano, $C_1$-$C_2$ alkyl, $C_1$-$C_2$ haloalkyl and $C_1$-$C_2$ alkoxy on carbon atom ring members; and
$R^{a10}$ is cyano, halogen, cyclopropyl or methoxy.

Examples of compounds of Formula **b54.13** include (b54.13a) ethyl 1-[[4-[[2-(trifluoromethyl)-1,3-dioxolan-2-yl]methoxy]phenyl]methyl]-1*H*-pyrazole-4-carboxylate, (b54.13b) 2-methylpropyl 1-[[4-[[2-(trifluoromethyl)-1,3-dioxolan-2-yl]methoxy]-phenyl]methyl]-1*H*-pyrazole-4-carboxylate, (b54.13c) 2-butyn-1-yl 1-[[4-[[2-(trifluoromethyl)-1,3-dioxolan-2-yl]methoxy]phenyl]methyl]-1*H*-pyrazole-4-carboxylate and (b54.13d) ethyl 1-[[3-fluoro-4-[[2-(trifluoromethyl)-1,3-dioxolan-2-yl]methoxy]phenyl]methyl]-1*H*-pyrazole-4-carboxylate. Compounds of Formula **b54.13,** their use as fungicides and methods of preparation are generally known; see, for example, PCT Patent Publication WO2020/056090.

Component (b54.14) relates a compound of Formula **b54.14**

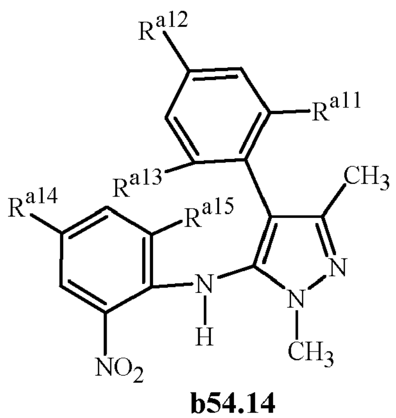

**b54.14**

wherein

$R^{b11}$, $R^{b12}$ and $R^{b13}$ are each independently H, halogen or cyano; and
$R^{b14}$ and $R^{b15}$ are each independently H, halogen, $C_1$-$C_3$ alkyl or $C_1$-$C_3$ methoxy.

Examples of compounds of Formula **b54.14** include (b54.14a) 4-(2-chloro-4-fluorophenyl)-*N*-(2-fluoro-4-methyl-6-nitrophenyl)-1,3-dimethyl-1*H*-pyrazol-5-amine, (b54.14b) 4-(2-chloro-4-fluorophenyl)-*N*-(2-fluoro-6-nitrophenyl)-1,3-dimethyl-1-*H*-pyrazol-5-amine, (b54.14c) 3,5-difluoro-4-[5-[(4-methoxy-2-nitrophenyl)amino]-1,3-dimethyl-1*H*-pyrazol-4-yl]-benzonitrile, (b54.14d) *N*-(2-chloro-4-fluoro-6-nitrophenyl)-4-(2-chloro-4-fluorophenyl)-1,3-dimethyl-1*H*-pyrazol-5-amine, (b54.14e) 4-(2-chloro-4,6-difluorophenyl)-l,3-dimethyl-*N*-(2-nitrophenyl)-1*H*-pyrazol-5-amine and (b54.14f) 4-(2-chloro-4,6-difluorophenyl)-1,3-dimethyl-*N*-(4-methyl-2-nitrophenyl)-1*H* pyrazol-5-amine. Compounds of Formula **b54.14,** their use as fungicides and methods of preparation are generally known; see, for example, PCT Patent Publication WO 2020/051402.

Embodiments of the present invention as described in the Summary of the Invention include those described below. In the following Embodiments, Formula **1** includes stereoisomers, *N*-oxides, and salts thereof, and reference to "a compound of Formula **1"** includes the definitions of substituents specified in the Summary of the Invention unless further defined in the Embodiments.

Embodiment 1. The composition comprising components (a) and (b) described in the Summary of the Invention wherein in Formula **1,** $R^1$ is selected from U-1 through U-118 as depicted in Exhibit A

Exhibit A

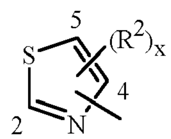
U-1
,
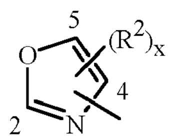
U-2
,
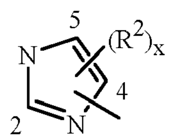
U-3
,
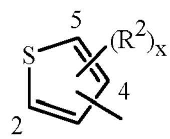
U-4
,
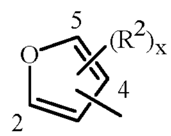
U-5
,
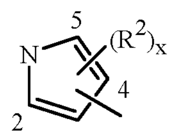
U-6
,
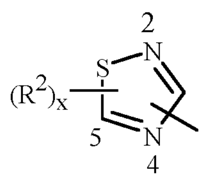
U-7
,
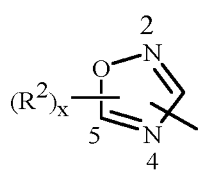
U-8
,
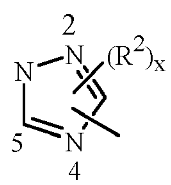
U-9
,
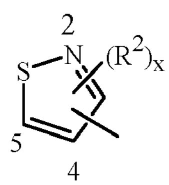
U-10
,
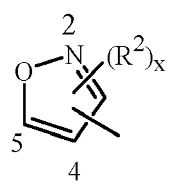
U-11
,
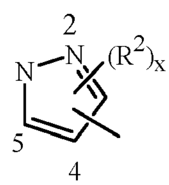
U-12
,

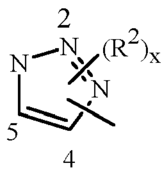 , 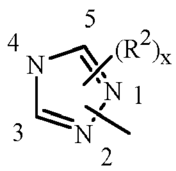 , 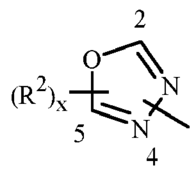 , 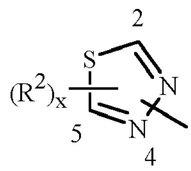 ,

U-13 U-14 U-15 U-16

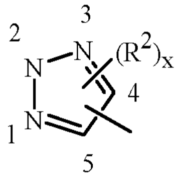 , 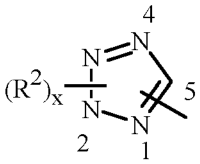 , 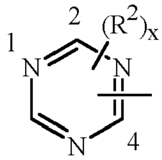 , 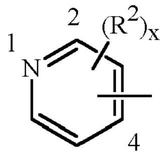 ,

U-17 U-18 U-19 U-20

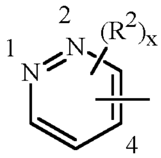 , 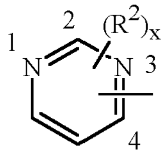 , 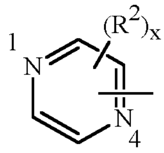 , 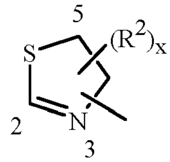 ,

U-21 U-22 U-23 U-24

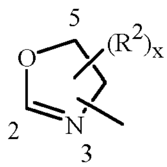 , 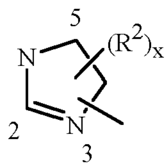 , 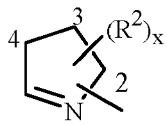 , 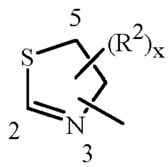 ,

U-25 U-26 U-27 U-28

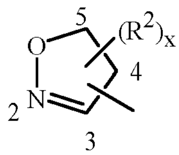 , 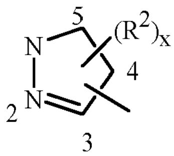 , 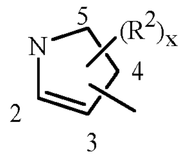 , 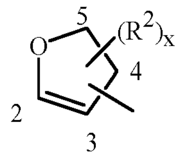 ,

U-29 U-30 U-31 U-32

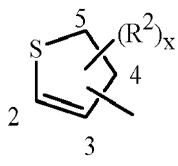 , 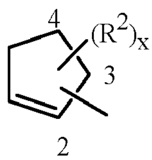 , 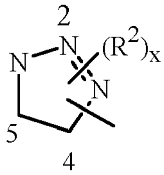 , 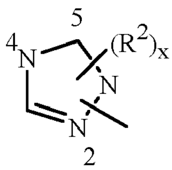 ,

U-33 U-34 U-35 U-36

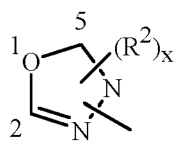 , 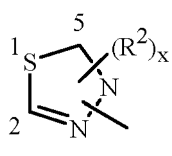 , 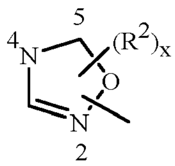 , 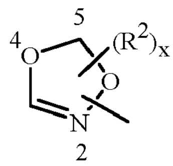 ,

U-37 U-38 U-39 U-40

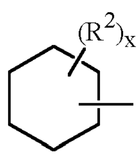 , 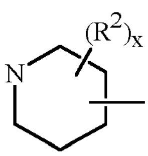 , 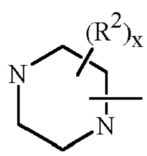 , 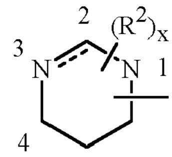 ,

U-41 U-42 U-43 U-44

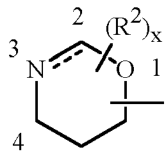 , 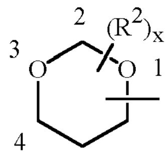 , 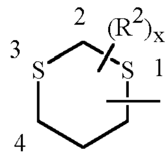 , 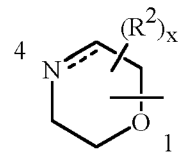 ,

U-45 U-46 U-47 U-48

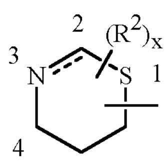 , 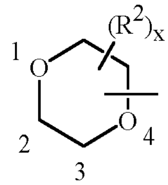 , 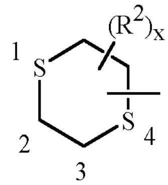 , 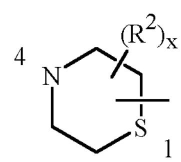 ,

U-49 U-50 U-51 U-52

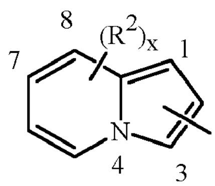 , 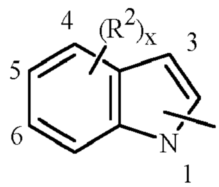 , 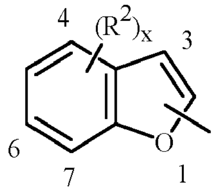 , 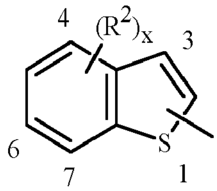 ,

U-53 U-54 U-55 U-56

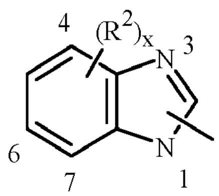 , 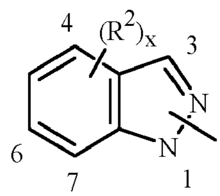 , 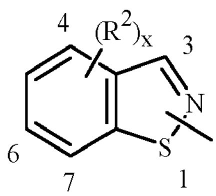 , 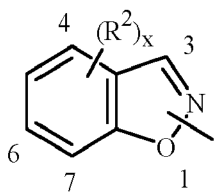 ,

U-57 U-58 U-59 U-60

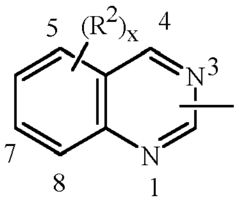 , 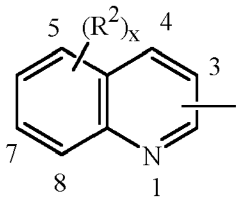 , 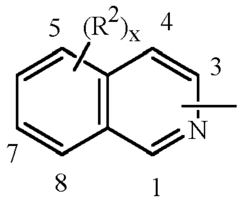 , 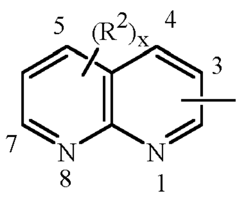 ,

U-61 U-62 U-63 U-64

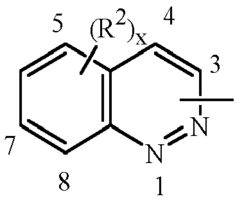 , 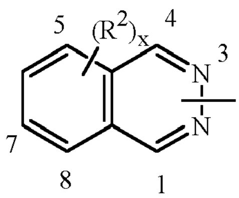 , 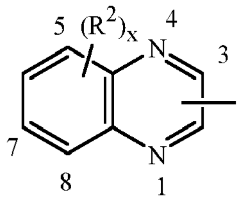 , 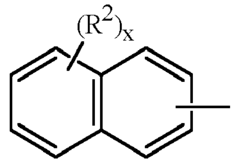 ,

U-65 U-66 U-67 U-68

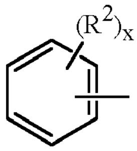 , 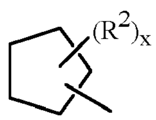 , 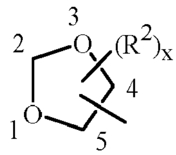 , 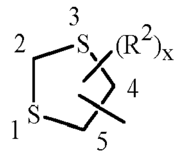 ,

U-69 U-70 U-71 U-72

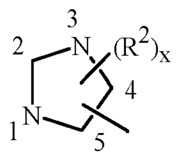 , 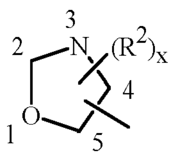 , 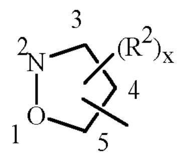 , 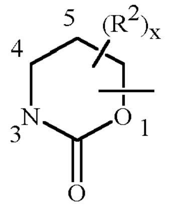 ,

U-73 U-74 U-75 U-76

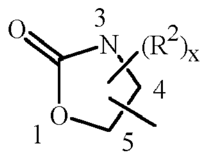 , 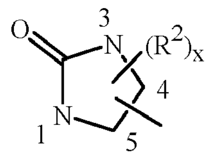 , 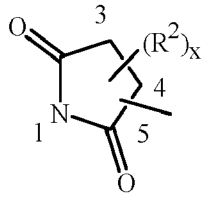 , 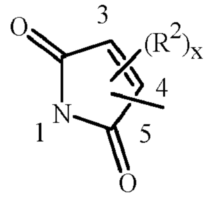 ,

U-77 U-78 U-79 U-80

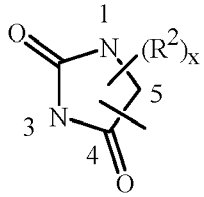 , 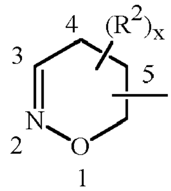 , 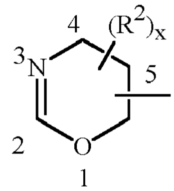 , 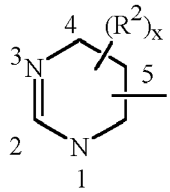 ,

U-81 U-82 U-83 U-84

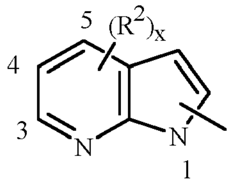 , 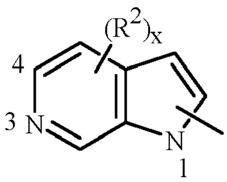 , 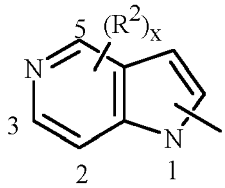 , 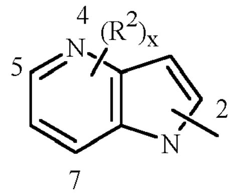 ,

U-85 U-86 U-87 U-88

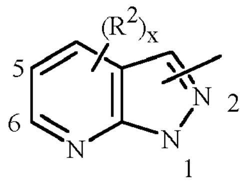 , 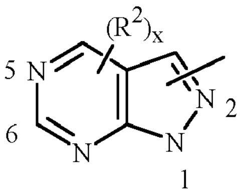 , 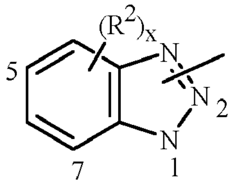 , 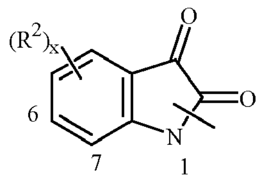 ,

U-89 U-90 U-91 U-92

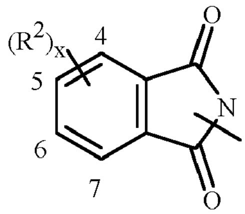 , 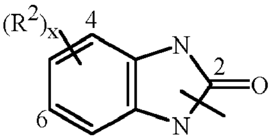 , 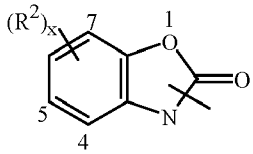 , 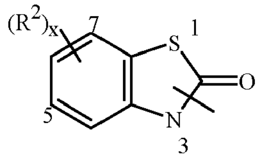 ,

U-93 U-94 U-95 U-96

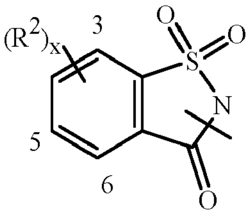 , 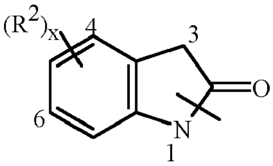 , 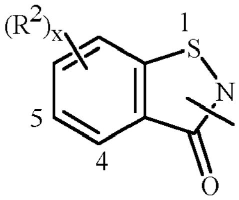 , 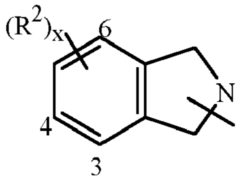 ,

U-97 U-98 U-99 U-100

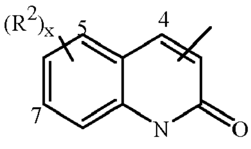 , 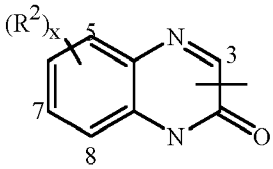 , 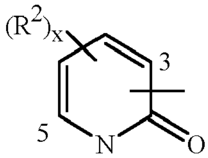 , 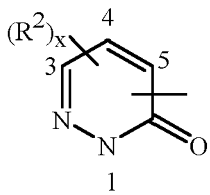 ,

U-101 U-102 U-103 U-104

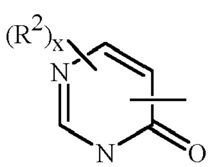 , 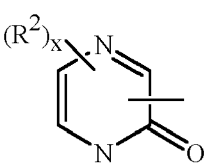 , 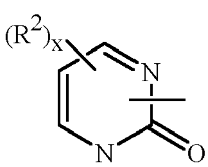 , 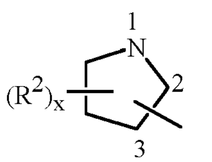 ,

U-105 U-106 U-107 U-108

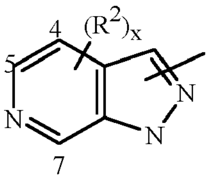

U-109

,

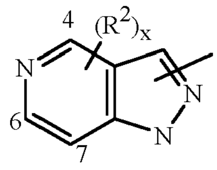

U-110

,

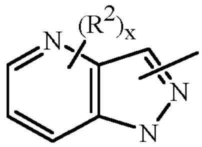

U-111

,

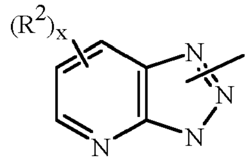

U-112

,

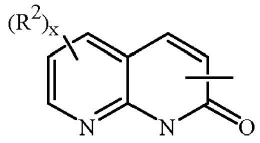

U-113

,

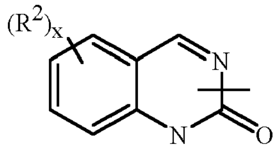

U-114

,

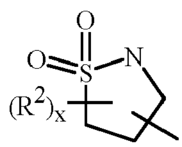

U-115

,

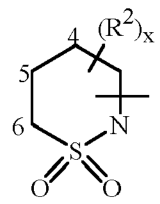

U-116

,

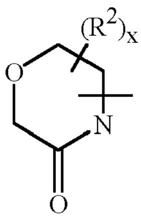

U-117

and

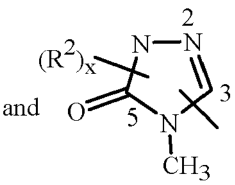

U-118

;

wherein the floating bond is connected to L in Formula **1** through any available carbon or nitrogen atom of the depicted ring or ring system; and x is 0, 1 or 2.

Embodiment 2. The composition comprising components (a) and (b) described in the Summary of the Invention wherein in Formula **1,** $R^1$ is U-1 through U-16, U-20, U-22, U-24, U-25, U-26, U-28, U-29, U-30, U-37, U-38, U-42 through U-47 or U-71 through U-114.

Embodiment 3. The composition comprising components (a) and (b) described in the Summary of the Invention wherein in Formula **1,** is U-1, U-2, U-3, U-4, U-5, U-7, U-8, U-10, U-11, U-12, U-24, U-26, U-28, U-29, U-30, U-37, U-38, U-42 through U-46, U-71, U-74, U-76, U-77, U-78, U-82, U-83, U-84 through U-91, U-93 through U-96, U-99 or U-101 through U-114.

Embodiment 4. The composition comprising components (a) and (b) described in the Summary of the Invention wherein in Formula **1,** $R^1$ is U-1, U-2, U-3, U-4, U-5, U-7, U-8, U-10, U-11, U-12, U-24, U-26, U-28, U-29, U-30, U-42 through U-46, U-71, U-76, U-77, U-78, U-82, U-83, U-84, U-89, U-90, U-91, U-93, U-103, U-104 or U-109 through U-112.

Embodiment 5. The composition comprising components (a) and (b) described in the Summary of the Invention wherein in Formula **1,** $R^1$ is U-1, U-2, U-3, U-4, U-5, U-7, U-8, U-10, U-11, U-12, U-26, U-29, U-30, U-42 through U-46, U-71, U-76, U-77, U-78, U-82, U-83, U-89, U-90, U-103 or U-104.

Embodiment 6. The composition comprising components (a) and (b) described in the Summary of the Invention wherein in Formula **1,** $R^1$ is U-1, U-2, U-3, U-4, U-5, U-7, U-8, U-10, U-11, U-12 or U-29.

Embodiment 7. The composition comprising components (a) and (b) described in the Summary of the Invention wherein in Formula **1,** $R^1$ is U-1, U-2, U-3, U-4, U-8, U-10, U-11, U-12.

Embodiment 8. The composition comprising components (a) and (b) described in the Summary of the Invention wherein in Formula **1,** $R^1$ is U-1, U-2, U-8, U-10, U-11 or U-12.

Embodiment 8a. The composition comprising components (a) and (b) described in the Summary of the Invention wherein in Formula **1,** $R^1$ is U-1, U-2, U-8, U-11 or U-12.

Embodiment 9. The composition comprising components (a) and (b) described in the Summary of the Invention wherein in Formula **1,** $R^1$ is U-1, U-2, U-8 or U-12.

Embodiment 10. The composition comprising components (a) and (b) described in the Summary of the Invention wherein in Formula **1,** $R^1$ is U-1, U-2 or U-12.

Embodiment 11. The composition comprising components (a) and (b) described in the Summary of the Invention wherein in Formula **1,** $R^1$ is U-2 or U-12.

Embodiment 12. The composition comprising components (a) and (b) described in the Summary of the Invention

wherein in Formula **1,** $R^1$ is U-1.

Embodiment 13. The composition comprising components (a) and (b) described in the Summary of the Invention wherein in Formula **1,** $R^1$ is U-2.

Embodiment 14. The composition comprising components (a) and (b) described in the Summary of the Invention wherein in Formula **1,** $R^1$ is U-8

Embodiment 15. The composition comprising components (a) and (b) described in the Summary of the Invention wherein in Formula **1,** $R^1$ is U-12.

Embodiment 16. The composition comprising components (a) and (b) described in the Summary of the Invention wherein in Formula **1,** $R^1$ is U-1 connected at its 2-position to L.

Embodiment 17. The composition comprising components (a) and (b) described in the Summary of the Invention wherein in Formula **1,** $R^1$ is U-2 connected at its 2-position to L.

Embodiment 18. The composition comprising components (a) and (b) described in the Summary of the Invention wherein in Formula **1,** $R^1$ is U-8 connected at its 5-position to L.

Embodiment 19. The composition comprising components (a) and (b) described in the Summary of the Invention wherein in Formula **1,** $R^1$ is U-12 connected at its 1-position to L.

Embodiment 20. The composition comprising components (a) and (b) described in the Summary of the Invention wherein in Formula **1,** $R^1$ is U-12 connected at its 3-position to L.

Embodiment 21. The composition comprising components (a) and (b) described in the Summary of the Invention wherein in Formula **1,** $R^1$ is U-12 connected at its 5-position to L.

Embodiment 22. The composition comprising components (a) and (b) described in the Summary of the Invention wherein in Formula **1,** $R^1$ is U-2 connected at its 2-position to L and its 4-position to $R^2$.

Embodiment 22a. The composition comprising components (a) and (b) described in the Summary of the Invention wherein in Formula **1,** $R^1$ is U-12 connected at its 1-position to L and its 4-position to $R^2$.

Embodiment 23. The composition comprising components (a) and (b) described in the Summary of the Invention wherein in Formula **1,** $R^1$ is U-11 connected at its 1-position to L and its 5-position to $R^2$.

Embodiment 24. The composition comprising components (a) and (b) described in the Summary of the Invention or any one of Embodiments 1 through 23 wherein in Formula **1,** x is 1 or 2.

Embodiment 25. The composition of Embodiment 24 wherein x is 1.

Embodiment 26. The composition of Embodiment 24 wherein x is 2.

Embodiment 27. The composition comprising components (a) and (b) described in the Summary of the Invention or any one of Embodiments 1 through 26 wherein in Formula **1,** L is O, $(CR^{4a}R^{4b})_n$, $OCH_2$, $CH_2O$, $OCH_2CH_2$, $CH_2CH_2O$ or $CH_2OCH_2$, wherein the atom to the left is connected to $R^1$, and the atom to the right is connected to J, each carbon atom is optionally substituted with up to 2 substituents independently selected from halogen, cyano, hydroxy, $C_1$-$C_3$ alkyl, $C_1$-$C_3$ haloalkyl, $C_1$-$C_2$ alkoxy and $C_1$-$C_2$ haloalkoxy.

Embodiment 28. The composition of Embodiment 27 wherein L is $(CR^{4a}R^{4b})_n$, $OCH_2$, $CH_2O$, $OCH_2CH_2$, $CH_2CH_2O$ or $CH_2OCH_2$, wherein the atom to the left is connected to $R^1$, and the atom to the right is connected to J, each carbon atom is optionally substituted with up to 1 substituent selected from halogen, cyano, hydroxy, methyl, halomethyl or methoxy.

Embodiment 29. The composition of Embodiment 28 wherein L is $(CR^{4a}R^{4b})_n$, $OCH_2$, $CH_2O$, $OCH_2CH_2$, $CH_2CH_2O$ or $CH_2OCH_2$, wherein the atom to the left is connected to $R^1$, and the atom to the right is connected to J.

Embodiment 30. The composition of Embodiment 28 wherein L is $(CR^{4a}R^{4b})_n$, $CH_2O$ or $CH_2OCH_2$, wherein the atom to the left is connected to $R^1$, and the atom to the right is connected to J, each carbon atom is optionally substituted with up to 1 substituent selected from halogen, cyano, hydroxy, methyl, halomethyl or methoxy.

Embodiment 31. The composition of Embodiment 30 wherein L is $(CR^{4a}R^{4b})_n$, $CH_2O$ or $CH_2OCH_2$, wherein the atom to the left is connected to $R^1$, and the atom to the right is connected to J.

Embodiment 32. The composition of Embodiment 31 wherein L is $(CR^{4a}R^{4b})_n$ or $CH_2O$, wherein the atom to the left is connected to $R^1$, and the atom to the right is connected to J.

Embodiment 33. The composition of Embodiment 31 wherein L is $(CR^{4a}R^{4b})_n$.

Embodiment 34. The composition of Embodiment 31 wherein L is $CH_2O$, wherein the atom to the left is connected to $R^1$, and the atom to the right is connected to J.

Embodiment 35. The composition comprising components (a) and (b) described in the Summary of the Invention or any one of Embodiments 1 through 34 wherein in Formula **1,** n is 1 or 2.

Embodiment 36. The composition of Embodiment 35 wherein n is 1.

Embodiment 37. The composition comprising components (a) and (b) described in the Summary of the Invention or any one of Embodiments 1 through 36 wherein in Formula **1,** J is a phenyl ring optionally substituted with up to 2 substituents independently selected from $R^5$; or a 3- to 7-membered carbocyclic ring, wherein up to 2 ring members are independently selected from C(=O) and C(=S), each ring optionally substituted with up to 2 substituents independently selected from $R^5$; or a 5- to 6-membered heterocyclic ring, each ring containing ring members selected from carbon

atoms and 1 to 4 heteroatoms independently selected from up to 2 O, up to 2 S and up to 4 N atoms, wherein up to 2 ring members are independently selected from C(=O) and C(=S), each ring optionally substituted with up to 2 substituents independently selected from $R^5$.

Embodiment 38. The composition comprising components (a) and (b) described in the Summary of the Invention or any one of Embodiments 1 through 37 wherein in Formula **1,** J is selected from J-1 through J-93 as depicted in Exhibit B

Exhibit B

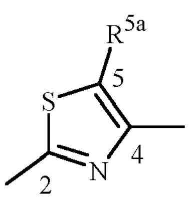
J-1
,
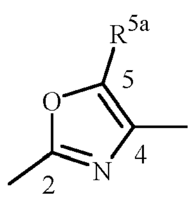
J-2
,
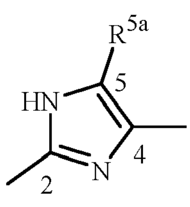
J-3
,
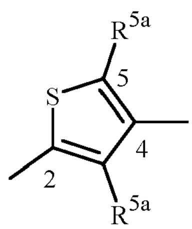
J-4
,
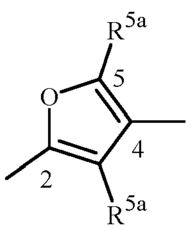
J-5
,
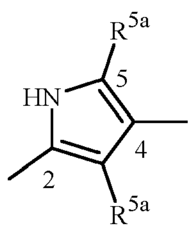
J-6
,
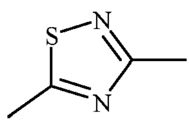
J-7
,
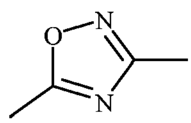
J-8
,
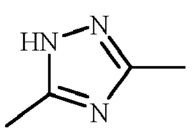
J-9
,
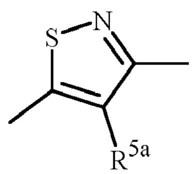
J-10
,
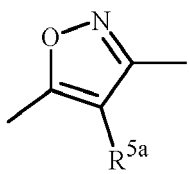
J-11
,
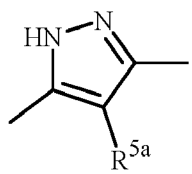
J-12
,
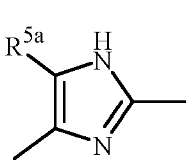
J-13
,
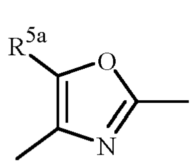
J-14
,
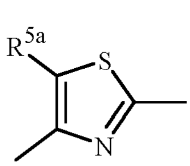
J-15
,
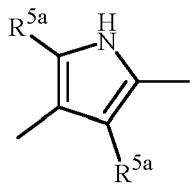
J-16
,
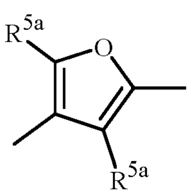
J-17
,
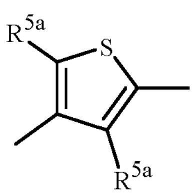
J-18
,
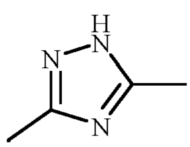
J-19
,
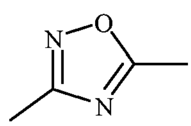
J-20
,

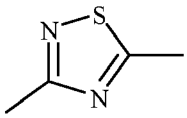 , 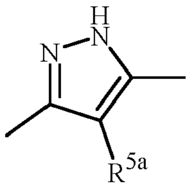 , 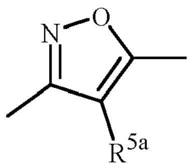 , 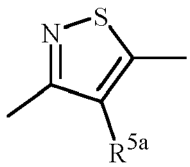 ,

J-21 J-22 J-23 J-24

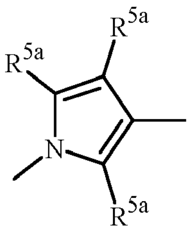 , 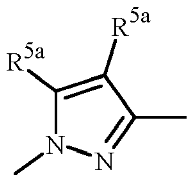 , 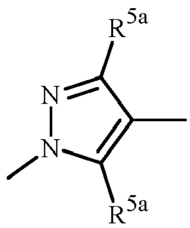 , 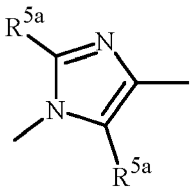 ,

J-25 J-26 J-27 J-28

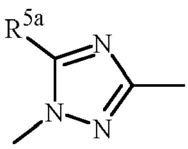 , 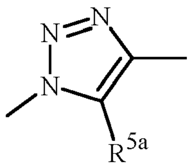 , 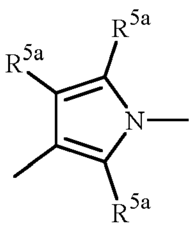 , 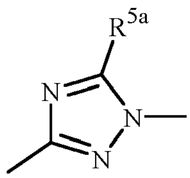 ,

J-29 J-30 J-31 J-32

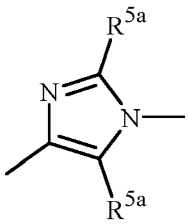 , 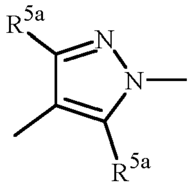 , 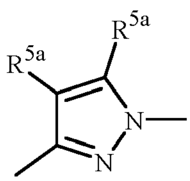 , 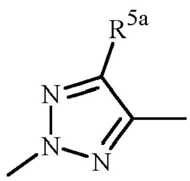 ,

J-33 J-34 J-35 J-36

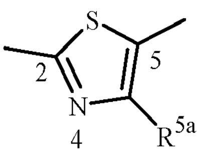 , 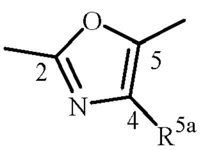 , 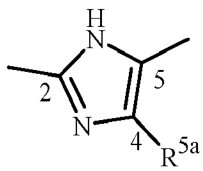 , 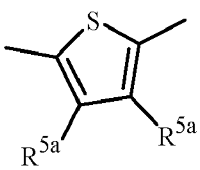 ,

J-37 J-38 J-39 J-40

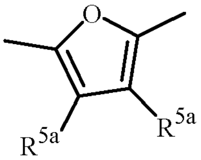 , 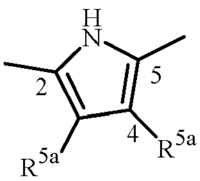 , 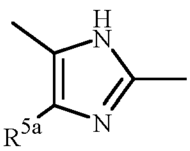 , 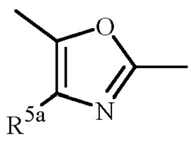 ,

J-41 J-42 J-43 J-44

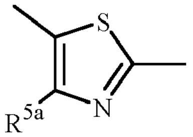 , 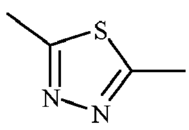 , 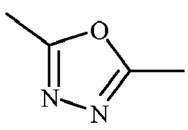 , 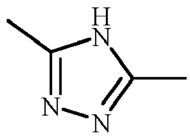 ,

J-45 J-46 J-47 J-48

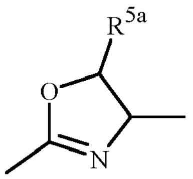 , 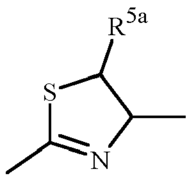 , 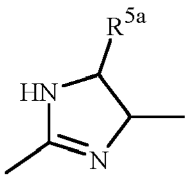 , 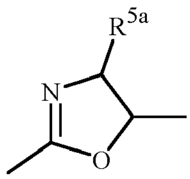 ,

J-49 J-50 J-51 J-52

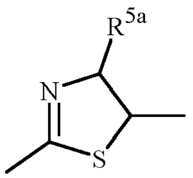 , 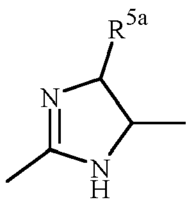 , 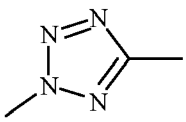 , 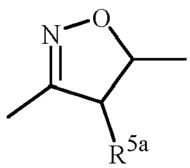 ,

J-53 J-54 J-55 J-56

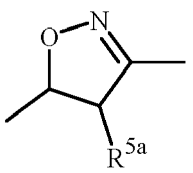 , 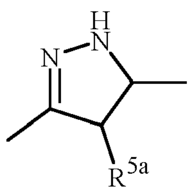 , 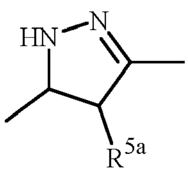 , 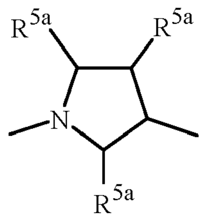 ,

J-57 J-58 J-59 J-60

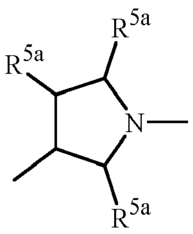 , 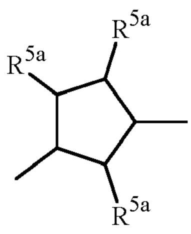 , 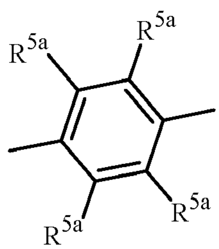 , 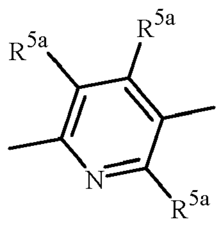 ,

J-61 J-62 J-63 J-64

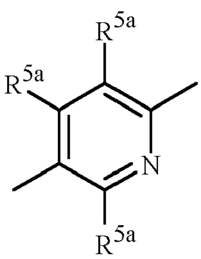 , 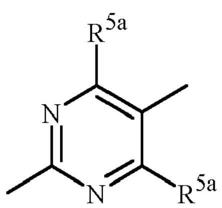 , 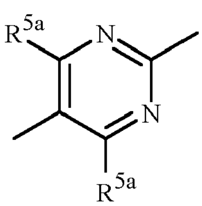 , 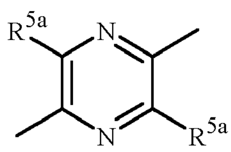 ,

J-65 J-66 J-67 J-68

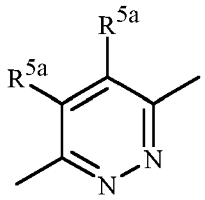
J-69
,
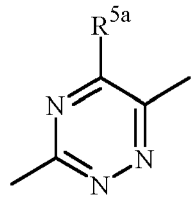
J-70
,
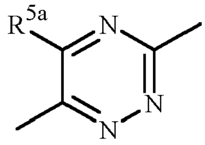
J-71
,
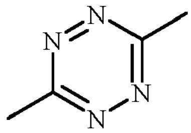
J-72
,
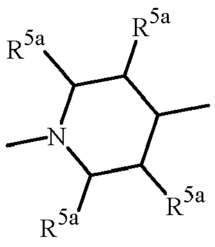
J-73
,
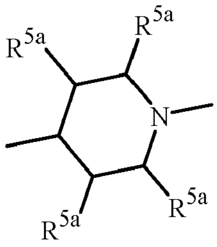
J-74
,
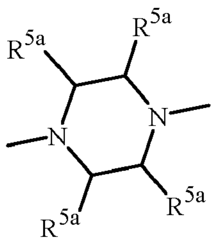
J-75
,
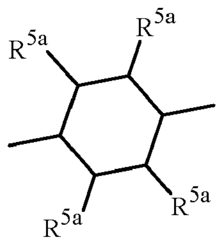
J-76
,
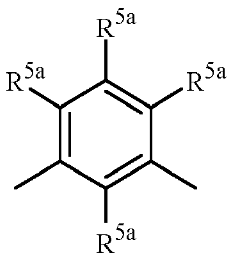
J-77
,
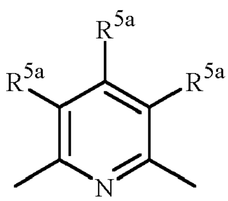
J-78
,
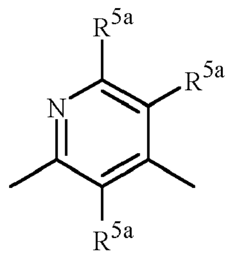
J-79
,
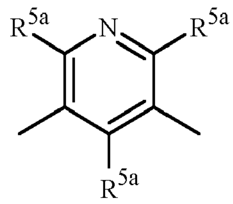
J-80
,
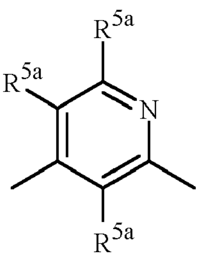
J-81
,
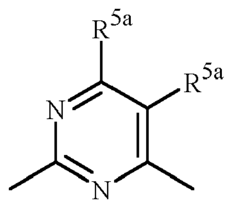
J-82
,
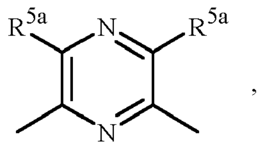
J-83
,
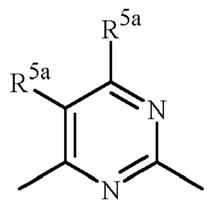
J-84
,
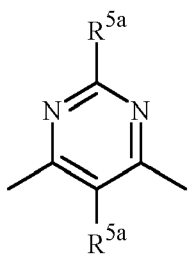
J-85
,
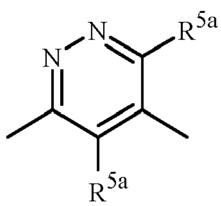
J-86
,
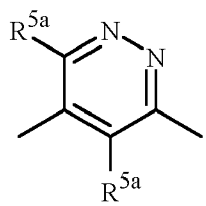
J-87
,
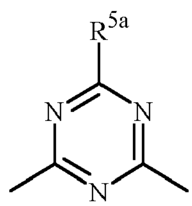
J-88
,

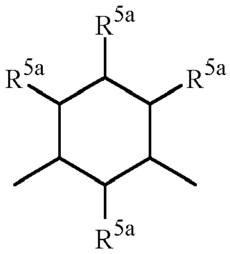, 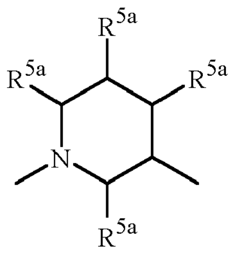, 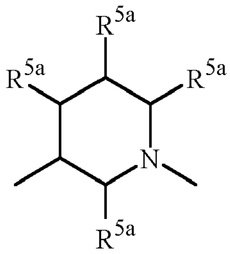, 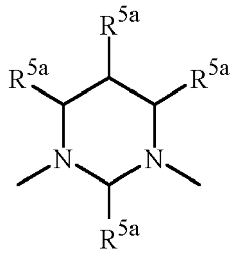

J-89 J-90 J-91 J-92

and

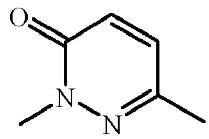;

J-93

wherein the bond projecting to the left is bonded to L, and the bond projecting to the right is bonded to the oxadiazole ring in Formula **1**; and each $R^{5a}$ is independently H or $R^5$; provided that at most only two $R^{5a}$ substituents are other than H.

Embodiment 39. The composition of Embodiment 38 wherein J is J-1 through J-5, J-17, J-18, J-27, J-37 through J-41, J-60, J-63 through J-71, J-73, J-74, J-75 or J-77 through J-85.

Embodiment 40. The composition of Embodiment 39 wherein J is J-4, J-5, J-18, J-27, J-37, J-40, J-41, J-63 through J-69, J-73 or J-77 through J-85.

Embodiment 41. The composition of Embodiment 40 wherein J is J-4, J-18, J-27, J-37, J-40, J-63 through J-69 or J-73.

Embodiment 42. The composition of Embodiment 41 wherein J is J-4, J-18, J-27, J-40 or J-63.

Embodiment 43. The composition of Embodiment 42 wherein J is J-40 or J-63.

Embodiment 44. The composition of Embodiment 43 wherein J is J-40.

Embodiment 45. The composition of Embodiment 43 wherein J is J-63.

Embodiment 46. The composition of any one of Embodiments 38 through 45 wherein each $R^{5a}$ is H.

Embodiment 47. The composition comprising components (a) and (b) described in the Summary of the Invention or any one of Embodiments 1 through 46 wherein in Formula **1,** each $R^2$ is independently halogen, cyano, CH(=O), C(=O)OH, C(=O)$NR^{3a}R^{3b}$, C($R^6$)=$NR^7$ or -U-V-Q; or $C_1$-$C_6$ alkyl, $C_2$-$C_6$ alkenyl, $C_2$-$C_6$ alkynyl, $C_3$-$C_7$ cycloalkyl, $C_1$-$C_6$ alkoxy, $C_2$-$C_6$ alkenyloxy, $C_2$-$C_6$ alkynyloxy, $C_3$-$C_7$ cycloalkoxy, $C_1$-$C_6$ alkylthio, $C_2$-$C_6$ alkoxycarbonyl, $C_3$-$C_6$ alkenyloxycarbonyl, $C_3$-$C_6$ alkynyloxycarbonyl, $C_4$-$C_7$ cycloalkoxycarbonyl, $C_2$-$C_6$ alkylcarbonyloxy or $C_2$-$C_6$ alkylcarbonylamino, each optionally substituted with up to 3 substituents independently selected from $R^{10}$.

Embodiment 48. The composition of Embodiment 48 wherein each $R^2$ is independently halogen, CH(=O), C(=O)OH, C(=O)$NR^{3a}R^{3b}$, C($R^6$)=$NR^7$ or -U-V-Q; or $C_1$-$C_6$ alkyl, $C_2$-$C_6$ alkenyl, $C_2$-$C_6$ alkynyl, $C_3$-$C_7$ cycloalkyl, $C_1$-$C_6$ alkoxy, $C_2$-$C_6$ alkenyloxy, $C_2$-$C_6$ alkynyloxy, $C_3$-$C_7$ cycloalkoxy, $C_1$-$C_6$ alkylthio, $C_2$-$C_6$ alkoxycarbonyl, $C_3$-$C_6$ alkenyloxycarbonyl, $C_3$-$C_6$ alkynyloxycarbonyl, $C_4$-$C_7$ cycloalkoxycarbonyl, $C_2$-$C_6$ alkylcarbonyloxy or $C_2$-$C_6$ alkylcarbonylamino, each optionally substituted with up to 2 substituents independently selected from $R^{10}$.

Embodiment 49. The composition of Embodiment 48 wherein each $R^2$ is independently halogen, C(=O)$NR^{3a}R^{3b}$, C($R^6$)=$NR^7$ or -U-V-Q; or $C_1$-$C_6$ alkyl, $C_2$-$C_6$ alkenyl, $C_2$-$C_6$ alkynyl, $C_3$-$C_7$ cycloalkyl, $C_1$-$C_6$ alkoxy, $C_2$-$C_6$ alkenyloxy, $C_2$-$C_6$ alkynyloxy, $C_3$-$C_7$ cycloalkoxy, $C_1$-$C_6$ alkylthio, $C_2$-$C_6$ alkoxycarbonyl, $C_3$-$C_6$ alkenyloxycarbonyl, $C_3$-$C_6$ alkynyloxycarbonyl, $C_4$-$C_7$ cycloalkoxycarbonyl, $C_2$-$C_6$ alkylcarbonyloxy or $C_2$-$C_6$ alkylcarbonylamino, each optionally substituted with up to 1 substituent selected from $R^{10}$.

Embodiment 50. The composition of Embodiment 49 wherein each $R^2$ is independently halogen, C(=O)$NR^{3a}R^{3b}$ or -U-V-Q; or $C_1$-$C_6$ alkyl, $C_2$-$C_6$ alkenyl, $C_2$-$C_6$ alkynyl, $C_1$-$C_6$ alkoxy, $C_2$-$C_6$ alkoxycarbonyl, $C_3$-$C_6$ alkenyloxycarbonyl, $C_3$-$C_6$ alkynyloxycarbonyl or $C_4$-$C_7$ cycloalkoxycarbonyl, each optionally substituted with up to 1 substituent selected from $R^{10}$.

Embodiment 51. The composition of Embodiment 50 wherein each $R^2$ is independently C(=O)$NR^{3a}R^{3b}$ or -U-V-Q; or $C_1$-$C_3$ alkyl, $C_2$-$C_6$ alkoxycarbonyl, $C_3$-$C_6$ alkenyloxycarbonyl or $C_3$-$C_6$ alkynyloxycarbonyl, each optionally substituted with up to 1 substituent selected from $R^{10}$.

Embodiment 52. The composition of Embodiment 51 wherein each $R^2$ is independently C(=O)$NR^{3a}R^{3b}$; or $C_1$-$C_2$ alkyl, $C_2$-$C_6$ alkoxycarbonyl, $C_3$-$C_6$ alkenyloxycarbonyl or $C_3$-$C_6$ alkynyloxycarbonyl, each optionally substituted

with up to 1 substituent selected from $R^{10}$.

Embodiment 53. The composition of Embodiment 52 wherein each $R^2$ is independently $C(=O)NR^{3a}R^{3b}$; or $C_1$-$C_2$ alkyl or $C_2$-$C_6$ alkoxycarbonyl, each optionally substituted with up to 1 substituent selected from $R^{10}$.

Embodiment 53a. The composition of Embodiment 51 wherein each $R^2$ is independently $C(=O)NR^{3a}R^{3b}$ or -U-V-Q; or $C_1$-$C_2$ alkyl or $C_2$-$C_6$ alkoxycarbonyl, each optionally substituted with up to 1 substituent selected from $R^{10}$.

Embodiment 54. The composition of Embodiments 53 or 53a wherein each $R^2$ is independently $C(=O)NR^{3a}R^{3b}$; or $C_2$-$C_6$ alkoxycarbonyl optionally substituted with up to 1 substituent selected from $R^{10}$.

Embodiment 55. The composition of Embodiment 54 wherein each $R^2$ is independently $C(=O)NR^{3a}R^{3b}$ or $C_2$-$C_6$ alkoxycarbonyl.

Embodiment 56. The composition of Embodiment 55 wherein each $R^2$ is independently $C(=O)NR^{3a}R^{3b}$ or $C_2$-$C_4$ alkoxycarbonyl.

Embodiment 57. The composition of Embodiment 56 wherein each $R^2$ is independently $C(=O)NR^{3a}R^{3b}$ or $C_2$-$C_3$ alkoxycarbonyl.

Embodiment 58. The composition of Embodiment 57 wherein each $R^2$ is independently $C(=O)NR^{3a}R^{3b}$.

Embodiment 59. The composition of Embodiment 57 wherein each $R^2$ is independently $C_2$-$C_3$ alkoxycarbonyl.

Embodiment 60. The composition comprising components (a) and (b) described in the Summary of the Invention or any one of Embodiments 1 through 59 wherein in Formula **1,** $R^3$ is H, cyano, hydroxy, $C_1$-$C_4$ alkyl, $C_1$-$C_4$ haloalkyl, $C_2$-$C_4$ alkenyl, $C_2$-$C_4$ alkynyl or $C_1$-$C_4$ alkoxy.

Embodiment 61. The composition of Embodiment 60 wherein $R^3$ is H, cyano, hydroxy, methyl or methoxy.

Embodiment 62. The composition of Embodiment 61 wherein $R^3$ is H.

Embodiment 63. The composition comprising components (a) and (b) described in the Summary of the Invention or any one of Embodiments 1 through 62 wherein in Formula **1,** when each $R^{3a}$ is separate (i.e. not taken together with $R^{3b}$), then each $R^{3a}$ is independently H, cyano, hydroxy, $C_1$-$C_4$ alkyl, $C_1$-$C_4$ haloalkyl, $C_2$-$C_4$ alkenyl, $C_2$-$C_4$ alkynyl, $C_3$-$C_5$ alkoxy, $C_2$-$C_4$ alkoxyalkyl, $C_2$-$C_4$ alkylthioalkyl, $C_2$-$C_4$ alkylcarbonyl, $C_2$-$C_4$ haloalkylcarbonyl, $C_4$-$C_6$ cycloalkylcarbonyl, $C_2$-$C_5$ alkoxycarbonyl or $C_3$-$C_5$ alkoxycarbonylalkyl.

Embodiment 64. The composition of Embodiment 63 wherein each $R^{3a}$ is independently H, cyano, hydroxy, $C_1$-$C_4$ alkyl, $C_1$-$C_4$ haloalkyl, $C_2$-$C_4$ alkenyl, $C_2$-$C_4$ alkynyl, $C_1$-$C_4$ alkoxy, $C_2$-$C_4$ alkoxyalkyl, $C_2$-$C_4$ alkylcarbonyl, $C_2$-$C_4$ haloalkylcarbonyl or $C_3$-$C_5$ alkoxycarbonylalkyl.

Embodiment 65. The composition of Embodiment 64 wherein each $R^{3a}$ is independently H, $C_1$-$C_4$ alkyl, $C_1$-$C_4$ haloalkyl, $C_2$-$C_4$ alkenyl, $C_2$-$C_4$ alkynyl, $C_1$-$C_4$ alkoxy, $C_2$-$C_4$ alkoxyalkyl, $C_2$-$C_4$ alkylcarbonyl, $C_2$-$C_4$ haloalkylcarbonyl or $C_3$-$C_5$ alkoxycarbonylalkyl.

Embodiment 66. The composition of Embodiment 65 wherein each $R^{3a}$ is independently H, $C_1$-$C_4$ alkyl, $C_1$-$C_4$ haloalkyl, $C_2$-$C_4$ alkenyl, $C_2$-$C_4$ alkynyl, $C_2$-$C_4$ alkoxyalkyl or $C_3$-$C_5$ alkoxycarbonylalkyl.

Embodiment 67. The composition of Embodiment 66 wherein each $R^{3a}$ is independently H, $C_1$-$C_3$ alkyl, $C_1$-$C_3$ haloalkyl or $C_2$-$C_3$ alkoxyalkyl.

Embodiment 68. The composition of Embodiment 67 wherein each $R^{3a}$ is H.

Embodiment 69. The composition comprising components (a) and (b) described in the Summary of the Invention or any one of Embodiments 1 through 68 wherein in Formula **1,** when each $R^{3b}$ is separate (i.e. not taken together with $R^{3a}$), then each $R^{3b}$ is independently H, $C_1$-$C_6$ alkyl, $C_1$-$C_6$ haloalkyl, $C_2$-$C_6$ alkenyl, $C_2$-$C_6$ haloalkenyl, $C_2$-$C_6$ alkynyl, $C_2$-$C_6$ haloalkynyl, $C_1$-$C_6$ hydroxyalkyl, $C_2$-$C_6$ cyanoalkyl, $C_3$-$C_8$ cycloalkyl, $C_3$-$C_8$ halocycloalkyl, $C_4$-$C_{10}$ alkylcycloalkyl, $C_4$-$C_{10}$ cycloalkylalkyl, $C_4$-$C_{10}$ halocycloalkylalkyl, $C_2$-$C_6$ alkoxyalkyl, $C_2$-$C_6$ haloalkoxyalkyl or $C_4$-$C_{10}$ cycloalkoxyalkyl, each optionally substituted with up to 1 substituent selected from cyano, hydroxy, $C_2$-$C_4$ alkylcarbonyl, $C_2$-$C_4$ alkoxycarbonyl, $C_3$-$C_{15}$ trialkylsilyl and $C_3$-$C_{15}$ halotrialkylsilyl.

Embodiment 70. A compound of Embodiment 69 wherein each $R^{3b}$ is independently H, $C_1$-$C_6$ alkyl, $C_1$-$C_6$ haloalkyl, $C_2$-$C_6$ alkenyl, $C_2$-$C_6$ haloalkenyl, $C_2$-$C_6$ alkynyl, $C_2$-$C_6$ haloalkynyl, $C_3$-$C_8$ halocycloalkyl, $C_4$-$C_{10}$ cycloalkylalkyl, $C_4$-$C_{10}$ halocycloalkylalkyl, $C_2$-$C_6$ alkoxyalkyl or $C_2$-$C_6$ haloalkoxyalkyl, each optionally substituted with up to 1 substituent selected from cyano, $C_2$-$C_4$ alkylcarbonyl, $C_2$-$C_4$ alkoxycarbonyl and $C_3$-$C_{15}$ trialkylsilyl.

Embodiment 71. The composition of Embodiment 70 wherein each $R^{3b}$ is independently H, $C_1$-$C_6$ alkyl, $C_1$-$C_6$ haloalkyl, $C_2$-$C_6$ alkenyl, $C_2$-$C_6$ haloalkenyl, $C_2$-$C_6$ alkynyl, $C_2$-$C_6$ haloalkynyl, $C_4$-$C_{10}$ cycloalkylalkyl, $C_4$-$C_{10}$ halocycloalkylalkyl, $C_2$-$C_6$ alkoxyalkyl or $C_2$-$C_6$ haloalkoxyalkyl, each optionally substituted with up to 1 substituent selected from cyano, $C_2$-$C_4$ alkylcarbonyl and $C_2$-$C_4$ alkoxycarbonyl.

Embodiment 72. The composition of Embodiment 71 wherein each $R^{3b}$ is independently H, $C_1$-$C_6$ alkyl, $C_1$-$C_6$ haloalkyl, $C_2$-$C_6$ alkoxyalkyl or $C_2$-$C_6$ haloalkoxyalkyl, each optionally substituted with up to 1 substituent selected from cyano, $C_2$-$C_4$ alkylcarbonyl and $C_2$-$C_4$ alkoxycarbonyl.

Embodiment 73. The composition of Embodiment 72 wherein each $R^{3b}$ is independently H, $C_1$-$C_3$ alkyl, $C_1$-$C_3$ haloalkyl, $C_2$-$C_3$ alkoxyalkyl or $C_2$-$C_3$ haloalkoxyalkyl, each optionally substituted with up to 1 substituent selected from cyano, $C_2$-$C_3$ alkylcarbonyl and $C_2$-$C_3$ alkoxycarbonyl.

Embodiment 74. The composition of Embodiment 73 wherein each $R^{3b}$ is independently H, $C_1$-$C_3$ alkyl, $C_1$-$C_3$

haloalkyl, $C_2$-$C_3$ alkoxyalkyl or $C_2$-$C_3$ haloalkoxyalkyl, each optionally substituted with up to 1 substituent selected from cyano.

Embodiment 75. The composition of Embodiment 74 wherein each $R^{3b}$ is independently H, $C_1$-$C_3$ alkyl, $C_1$-$C_3$ cyanoalkyl, $C_1$-$C_3$ haloalkyl, $C_2$-$C_3$ alkoxyalkyl or $C_2$-$C_3$ haloalkoxyalkyl.

Embodiment 76. The composition of Embodiment 75 wherein each $R^{3b}$ is independently $C_1$-$C_2$ haloalkyl.

Embodiment 77. The composition of Embodiment 76 wherein each $R^{3b}$ is $CF_3CH_2$-.

Embodiment 78. The composition comprising components (a) and (b) described in the Summary of the Invention or any one of Embodiments 1 through 77 wherein in Formula **1,** when a pair of $R^{3a}$ and $R^{3b}$ substituents attached to the same nitrogen atom are taken together to form a 5- to 6-membered fully saturated heterocyclic ring, then said ring contains ring members, in addition to the connecting nitrogen atom, selected from carbon atoms and up to 1 heteroatom selected from up to 1 O, up to 1 S and up to 1 N atom, each ring optionally substituted with up to 2 methyl.

Embodiment 79. The composition of Embodiment 78 wherein a pair of $R^{3a}$ and $R^{3b}$ substituents attached to the same nitrogen atom are taken together to form an azetidinyl, morpholinyl, pyrrolidinyl, piperidinyl, piperazinyl or thiomorpholinyl ring, each ring optionally substituted with up to 3 substituents independently selected from halogen.

Embodiment 80. The composition of Embodiment 79 wherein a pair of $R^{3a}$ and $R^{3b}$ substituents attached to the same nitrogen atom are taken together to form an azetidinyl, pyrrolidinyl or piperidinyl ring, each ring optionally substituted with up to 3 substituents independently selected from halogen.

Embodiment 81. The composition of Embodiment 80 wherein a pair of $R^{3a}$ and $R^{3b}$ substituents attached to the same nitrogen atom are taken together to form a pyrrolidinyl ring optionally substituted with up to 2 substituents independently selected from halogen.

Embodiment 82. The composition of Embodiment 81 wherein a pair of $R^{3a}$ and $R^{3b}$ substituents attached to the same nitrogen atom are taken together to form a pyrrolidinyl ring optionally substituted with up to 2 fluorine atoms.

Embodiment 83. The composition comprising components (a) and (b) described in the Summary of the Invention or any one of Embodiments 1 through 82 wherein in Formula **1,** when each $R^{4a}$ and $R^{4b}$ is separate (i.e. not taken together), then each $R^{4a}$ and $R^{4b}$ is independently H, halogen, cyano, hydroxy, methyl or methoxy.

Embodiment 84. The composition of Embodiment 83 wherein each $R^{4a}$ and $R^{4b}$ is independently H, halogen, hydroxy, methyl or methoxy.

Embodiment 85. The composition of Embodiment 84 wherein each $R^{4a}$ and $R^{4b}$ is independently H or methyl.

Embodiment 86. The composition of Embodiment 85 wherein each $R^{4a}$ and $R^{4b}$ is H.

Embodiment 87. The composition comprising components (a) and (b) described in the Summary of the Invention or any one of Embodiments 1 through 86 wherein in Formula **1,** when a pair of $R^{4a}$ and $R^{4b}$ substituents attached to the same carbon atom are taken together to form a ring, said ring is a cyclopropyl ring optionally substituted with up to 2 substituents independently selected from halogen, methyl, methoxy or methylthio.

Embodiment 88. The composition of Embodiment 87 wherein a pair of $R^{4a}$ and $R^{4b}$ substituents attached to the same carbon atom are taken together to form a cyclopropyl ring.

Embodiment 89. The composition comprising components (a) and (b) described in the Summary of the Invention or any one of Embodiments 1 through 88 wherein in Formula **1,** each $R^5$ is independently cyano, halogen, $C_1$-$C_4$ alkyl, $C_1$-$C_4$ haloalkyl or $C_1$-$C_4$ alkoxy.

Embodiment 90. The composition of Embodiment 89 wherein each $R^5$ is independently cyano, halogen, $C_1$-$C_3$ alkyl, $C_1$-$C_3$ haloalkyl or $C_1$-$C_3$ alkoxy.

Embodiment 91. The composition of Embodiment 90 wherein each $R^5$ is independently cyano, halogen, methyl or methoxy.

Embodiment 92. The composition of Embodiment 91 wherein each $R^5$ is independently methyl or methoxy.

Embodiment 93. The composition comprising components (a) and (b) described in the Summary of the Invention or any one of Embodiments 1 through 92 wherein in Formula **1,** each $R^6$ is independently H, cyano, halogen methyl or methoxy. Embodiment 94. The composition of Embodiment 93 wherein each $R^6$ is independently H, Cl or methyl.

Embodiment 95. The composition of Embodiment 94 wherein each $R^6$ is H.

Embodiment 96. The composition comprising components (a) and (b) described in the Summary of the Invention or any one of Embodiments 1 through 95 wherein in Formula **1,** each $R^7$ is independently hydroxy or $NR^{11a}R^{11b}$; or $C_1$-$C_4$ alkoxy, $C_2$-$C_4$ alkenyloxy, $C_2$-$C_4$ alkynyloxy or $C_2$-$C_4$ alkylcarbonyloxy, each optionally substituted with up to 1 substituent selected cyano, hydroxy and -C(=O)OH. Embodiment 97. The composition of Embodiment 96 wherein each $R^7$ is independently $C_1$-$C_4$ alkoxy, $C_2$-$C_4$ alkenyloxy or $C_2$-$C_4$ alkynyloxy, each optionally substituted with up to 1 substituent selected cyano, hydroxy and -C(=O)OH.

Embodiment 98. The composition of Embodiment 97 wherein each $R^7$ is independently $C_1$-$C_4$ alkoxy, $C_2$-$C_4$ alkenyloxy or $C_2$-$C_4$ alkynyloxy.

Embodiment 99. The composition comprising components (a) and (b) described in the Summary of the Invention or any one of Embodiments 1 through 98 wherein in Formula **1,** each $R^8$ is independently H, methyl or methoxy.

Embodiment 100. The composition of Embodiment 99 wherein each $R^8$ is independently H or methyl.

Embodiment 101. The composition of Embodiment 100 wherein each $R^8$ is H.

Embodiment 102. The composition comprising components (a) and (b) described in the Summary of the Invention or any one of Embodiments 1 through 101 wherein in Formula **1,** when each $R^{9a}$ and $R^{9b}$ is separate (i.e. not taken together), then each $R^{9a}$ and $R^{9b}$ is independently H, methyl or ethyl.

Embodiment 103. The composition of Embodiment 102 wherein each $R^{9a}$ and $R^{9b}$ is independently H or methyl.

Embodiment 104. The composition comprising components (a) and (b) described in the Summary of the Invention or any one of Embodiments 1 through 103 wherein in Formula **1,** each $R^{10}$ is independently halogen, cyano, $C_1$-$C_4$ alkyl, $C_1$-$C_4$ haloalkyl, $C_3$-$C_6$ cycloalkyl, $C_3$-$C_6$ halocycloalkyl, $C_1$-$C_4$ alkoxy, $C_1$-$C_4$ haloalkoxy, $C_2$-$C_4$ alkoxyalkoxy, $C_1$-$C_4$ alkylthio, $C_1$-$C_4$ alkylsulfinyl, $C_1$-$C_4$ alkylsulfonyl, $C_1$-$C_4$ haloalkylsulfonyl, $C_2$-$C_4$ alkylcarbonyl, $C_2$-$C_4$ haloalkylcarbonyl, $C_2$-$C_5$ alkoxycarbonyl, $C_3$-$C_5$ alkylthioalkylcarbonyl, $C_3$-$C_{15}$ trialkylsily or $C(R^{13})=NOR^{14}$.

Embodiment 105. The composition of Embodiment 104 wherein each $R^{10}$ is independently halogen, cyano, $C_1$-$C_4$ alkyl, $C_1$-$C_4$ haloalkyl, $C_1$-$C_4$ alkoxy, $C_1$-$C_4$ haloalkoxy, $C_2$-$C_4$ alkoxyalkoxy, $C_1$-$C_4$ alkylsulfonyl, $C_1$-$C_4$ haloalkylsulfonyl, $C_2$-$C_4$ alkylcarbonyl, $C_2$-$C_4$ haloalkylcarbonyl, $C_2$-$C_5$ alkoxycarbonyl or $C(R^{13})=NOR^{14}$.

Embodiment 105a. The composition of Embodiment 104 wherein each $R^{10}$ is cyano.

Embodiment 106. The composition of Embodiment 105 wherein each $R^{10}$ is independently halogen, $C_1$-$C_4$ alkyl, $C_1$-$C_4$ haloalkyl, $C_1$-$C_4$ alkoxy, $C_1$-$C_4$ haloalkoxy, $C_2$-$C_4$ alkylcarbonyl, $C_2$-$C_4$ haloalkylcarbonyl, $C_2$-$C_5$ alkoxycarbonyl or $C(R^{13})=NOR^{14}$.

Embodiment 107. The composition of Embodiment 106 wherein each $R^{10}$ is independently halogen, $C_1$-$C_4$ alkyl, $C_1$-$C_4$ alkoxy, $C_2$-$C_4$ alkylcarbonyl, $C_2$-$C_4$ haloalkylcarbonyl, $C_2$-$C_5$ alkoxycarbonyl or $C(R^{13})=NOR^{14}$.

Embodiment 108. The composition of Embodiment 107 wherein each $R^{10}$ is independently halogen, $C_1$-$C_4$ alkyl, $C_1$-$C_4$ alkoxy, $C_2$-$C_4$ alkylcarbonyl, $C_2$-$C_4$ haloalkylcarbonyl or $C_2$-$C_5$ alkoxycarbonyl.

Embodiment 108a. The composition of Embodiment 108 wherein each $R^{10}$ is independently halogen, $C_1$-$C_2$ alkoxy, $C_2$-$C_3$ alkylcarbonyl or $C_2$-$C_3$ haloalkylcarbonyl.

Embodiment 108b. The composition of Embodiment 105 wherein each $R^{10}$ is independently cyano, halogen, $C_1$-$C_2$ alkoxy, $C_2$-$C_3$ alkylcarbonyl or $C_2$-$C_3$ haloalkylcarbonyl.

Embodiment 109. The composition comprising components (a) and (b) described in the Summary of the Invention or any one of Embodiments 1 through 108 wherein in Formula **1**, each U is independently a direct bond, C(=O)O or $C(=O)NR^{17}$. Embodiment 110. The composition of Embodiment 109 wherein each U is independently C(=O)O or $C(=O)NR^{17}$.

Embodiment 111. The composition of Embodiment 110 wherein each U is independently $C(=O)NR^{17}$.

Embodiment 112. The composition comprising components (a) and (b) described in the Summary of the Invention or any one of Embodiments 1 through 111 wherein in Formula **1,** each V is independently a direct bond; or $C_1$-$C_3$ alkylene or $C_2$-$C_3$ alkenylene, each optionally substituted with up to 2 substituents independently selected from halogen, cyano, nitro, hydroxy, $C_1$-$C_2$ alkyl, $C_1$-$C_2$ haloalkyl, $C_1$-$C_2$ alkoxy and $C_1$-$C_2$ haloalkoxy. Embodiment 113. The composition of Embodiment 112 wherein each V is independently a direct bond; or $C_1$-$C_2$ alkylene optionally substituted with up to 1 substituent $C_1$-$C_2$ alkyl, $C_1$-$C_2$ alkoxy and $C_1$-$C_2$ haloalkoxy.

Embodiment 114. The composition of Embodiment 113 wherein each V is independently $C_1$-$C_2$ alkylene.

Embodiment 115. The composition comprising components (a) and (b) described in the Summary of the Invention or any one of Embodiments 1 through 114 wherein in Formula **1,** each Q is independently phenyl, each optionally substituted with up to 2 substituents independently selected from $R^{12}$; or pyridinyl, pyrazolyl, imidazolyl, triazolyl, thiazolyl or oxazolyl, each ring optionally substituted with up to 2 substituents independently selected from $R^{12}$.

Embodiment 116. The composition of Embodiment 115 wherein each Q is independently phenyl, each optionally substituted with up to 2 substituents independently selected from $R^{12}$; or pyridinyl or pyrazolyl, each ring optionally substituted with up to 2 substituents independently selected from $R^{12}$.

Embodiment 117. The composition of Embodiment 116 wherein each Q is pyrazolyl.

Embodiment 118. The composition comprising components (a) and (b) described in the Summary of the Invention or any one of Embodiments 1 through 117 wherein in Formula **1**, each $R^{12}$ is independently halogen, cyano, $C_1$-$C_4$ alkyl, $C_1$-$C_4$ haloalkyl or $C_1$-$C_4$ alkoxy.

Embodiment 119. The composition of Embodiment 118 wherein each $R^{12}$ is independently halogen, cyano, $C_4$-$C_3$ alkyl, $C_1$-$C_3$ haloalkyl or $C_1$-$C_3$ alkoxy.

Embodiment 120. The composition of Embodiment 119 wherein each $R^{12}$ is independently halogen, cyano, $C_1$-$C_2$ alkyl, $C_1$-$C_2$ haloalkyl or $C_1$-$C_2$ alkoxy.

Embodiment 121. The composition of Embodiment 120 wherein each $R^{12}$ is independently halogen, methyl or methoxy.

Embodiment 122. The composition comprising components (a) and (b) described in the Summary of the Invention or any one of Embodiments 1 through 121 wherein in Formula **1,** each $R^{13}$ and $R^{15}$ is independently H, cyano, halogen, methyl, halomethyl or methoxy.

Embodiment 123. The composition of Embodiment 122 wherein each $R^{13}$ and $R^{15}$ is independently H, halogen,

methyl or methoxy.

Embodiment 124. The composition of Embodiment 123 wherein each $R^{13}$ and $R^{15}$ is H.

Embodiment 125. The composition comprising components (a) and (b) described in the Summary of the Invention or any one of Embodiments 1 through 124 wherein in Formula **1**, 151 wherein each $R^{14}$ is independently H, $C_1$-$C_2$ alkyl, $C_1$-$C_2$ haloalkyl, $C_2$-$C_4$ alkenyl, $C_2$-$C_4$ alkylcarbonyl or $C_2$-$C_4$ alkoxycarbonyl.

Embodiment 126. The composition of Embodiment 125 wherein each $R^{14}$ is independently H, methyl, halomethyl, $C_2$-$C_4$ alkylcarbonyl or $C_2$-$C_4$ alkoxycarbonyl.

Embodiment 127. The composition comprising components (a) and (b) described in the Summary of the Invention or any one of Embodiments 1 through 126 wherein in Formula **1**, each $R^{16}$ is independently H, cyano, methyl, halomethyl, methoxy, $C_2$-$C_4$ alkylcarbonyl or $C_2$-$C_4$ alkoxycarbonyl.

Embodiment 128. The composition of Embodiment 127 wherein each $R^{16}$ is independently H, cyano, methyl, halomethyl or methoxy.

Embodiment 129. The composition of Embodiment 128 wherein each $R^{16}$ is independently H or methyl.

Embodiment 130. The composition of Embodiment 129 wherein each $R^{16}$ is H.

Embodiment 131. The composition comprising components (a) and (b) described in the Summary of the Invention or any one of Embodiments 1 through 130 wherein in Formula **1**, each $R^{17}$ and $R^{18}$ is independently H, cyano, methyl or halomethyl.

Embodiment 132. The composition of Embodiment 131 wherein in Formula **1**, each $R^{17}$ and $R^{18}$ is H.

Embodiment 133. The composition comprising components (a) and (b) described in the Summary of the Invention or any one of Embodiments 1 through 132 wherein component (a) does not comprise an *N*-oxide of a compound of Formula **1**.

Embodiment 133a. The composition comprising components (a) and (b) described in the Summary of the Invention or any one of Embodiments 1 through 132 wherein component (a) does not comprise a salt of a compound of Formula **1**.

Embodiment 134. The composition comprising components (a) and (b) described in the Summary of the Invention or any one of Embodiments 1 through 133a wherein component (a) comprises a compound selected from the group consisting of

methyl 1-[[4-[5-(trifluoromethyl)-1,2,4-oxadiazol-3-yl]phenyl]methyl]-1*H*-pyrazole-4-carboxylate (Compound 21);
cyanomethyl 1-[[4-[5-(trifluoromethyl)-1,2,4-oxadiazol-3-yl]phenyl]methyl]-1*H*-pyrazole-4-carboxylate (Compound 57);
ethyl 5-methyl-1-[[4-[5-(trifluoromethyl)-1,2,4-oxadiazol-3-yl]phenyl]methyl]-1*H*-pyrazole-3-carboxylate (Compound 110);
*N*-(2-methoxyethyl)-5-methyl-1-[[4-[5-(triffuoromethyl)-1,2,4-oxadiazol-3-yl]phenyl]methyl]-1*H*-pyrazole-3-carboxamide (Compound 183);
propyl 1-[[5-[5-(trifluoromethyl)-1,2,4-oxadiazol-3-yl]-2-thienyl]methyl]-1*H*-pyrazole-4-carboxylate (Compound 198);
*N*-(2-chloroethyl)-2-[[4-[5-(trifluoromethyl)-1,2,4-oxadiazol-3-yl]phenyl]methyl]-4-oxazolecarboxamide (Compound 204);
*N*-(2,2-difluoroethyl)-2-[[4-[5-(trifluoromethyl)-1,2,4-oxadiazol-3-yl]phenyl]methyl]-4-oxazolecarboxamide (Compound 225);
*N*-(2-cyanoethyl)-2-[[4-[5-(trifluoromethyl)-1,2,4-oxadiazol-3-yl]phenyl]methyl]-4-oxazolecarboxamide (Compound 229);
*N*-(2-methoxyethyl)-2-[[4-[5-(trifluoromethyl)-1,2,4-oxadiazol-3 -yl]phenyl]methyl]-4-oxazolecarboxamide (Compound 257);
*N*-[2-(1*H*-pyrazol-1-yl)ethyl]-2-[[4-[5-(trifluoromethyl)-1,2,4-oxadiazol-3-yl]phenyl]methyl]-4-oxazolecarboxamide (Compound 264);
*N*-(2,2,2-trifluoroethyl)-2-[[4-[5-(trifluoromethyl)-1,2,4-oxadiazol-3-yl]phenyl]methyl]-4-oxazolecarboxamide (Compound 316);
2-[[4-[5-(trifluoromethyl)-1,2,4-oxadiazol-3-yl]phenyl]methyl]-*N*-(3,3,3-trifluoropropyl)-4-oxazolecarboxamide (Compound 330);
ethyl 1-[[4-[5-(trifluoromethyl)-1,2,4-oxadiazol-3-yl]phenoxy]methyl]-1H pyrazole-4-carboxylate (Compound 376);
*N*-(2-methoxyethyl)-2-[[4-[5-(trifluoromethyl)-1,2,4-oxadiazol-3-yl]phenyl]methyl]-4-thiazolecarboxamide (Compound 394);
*N*-(2-fluoroethyl)-2-[[4-[5-(trifluoromethyl)-1,2,4-oxadiazol-3-yl]phenyl]methyl]-4-oxazolecarboxamide (Compound 400);
*N*-[2-(trifluoromethoxy)ethyl]-2-[[4-[5-(trifluoromethyl)-1,2,4-oxadiazol-3-yl]phenyl]methyl]-4-oxazolecarboxamide (Compound 401);
(3,3-diffuoro-1-pyrrolidinyl)[2-[[4-[5-(trifluoromethyl)-1,2,4-oxadiazol-3-yl]phenyl]methyl]-4-oxazolyl]methanone

(Compound 402);
*N*-(2,2,2-trifluoroethyl)-2-[[4-[5-(trifluoromethyl)-1,2,4-oxadiazol-3-yl]phenyl]methyl]-4-thiazolecarboxamide (Compound 463);
*N*-(2,2-difluoroethyl)-2-[[4-[5-(trifluoromethyl)-1,2,4-oxadiazol-3-yl]phenyl]methyl]-4-thiazolecarboxamide (Compound 505);
*N*-(2,2,2-trifluoroethyl)-5-[[4-[5-(trifluoromethyl)-1,2,4-oxadiazol-3-yl]phenyl]methyl]-1,2,4-oxadiazole-3-carboxamide (Compound 506);
3-[4-[(1-cyanomethyl-1*H*-pyrazol-3-yl)methyl]phenyl]-5-(trifluoromethyl)-1,2,4-oxadiazole (Compound 507);
*N*-ethyl-1-[[[4-[5-(trifluoromethyl)-1,2,4-oxadiazol-3-yl]phenyl]methoxy]methyl]-1*H*-pyrazole-4-carboxamide (Compound 508);
1-methyl-*N*-(2,2,2-trifluoroethyl)-5-[[4-[5-(trifluoromethyl)-1,2,4-oxadiazol-3-yl]phenyl]methyl]-1*H*-pyrazole-3-carboxamide (Compound 509); 5-[[4-[5-(trifluoromethyl)-1,2,4-oxadiazol-3-yl]phenyl]methyl]-3-isoxazoleacetonitrile (Compound 510);
*N*-(2-methoxyethyl)-5-[[4-[5-(trifluoromethyl)-1,2,4-oxadiazol-3-yl]phenyl]methyl]-1,2,4-oxadiazole-3-carboxamide (Compound 511); and
1-methyl-*N*-(2,2,2-trifluoroethyl)-3-[[4-[5-(trifluoromethyl)-1,2,4-oxadiazol-3-yl]phenyl]methyl]-1*H*-pyrazole-5-carboxamide (Compound 512).

Embodiment 135. The composition of Embodiment 134 wherein component (a) comprises a compound selected from the group consisting of Compounds 204, 225, 229, 257, 264, 316, 330, 376, 394, 400, 401, 402, 463, 505, 507, 508 and 509.

Embodiment 135a. The composition of Embodiment 134 wherein component (a) comprises a compound selected from the group consisting of Compounds 225, 229, 257, 316, 376, 394, 506, 507, 508, 509, 510, 511 and 512.

Embodiment 135b. The composition of Embodiment 134a wherein component (a) comprises a compound selected from the group consisting of Compounds 507, 508, 509 and 510.

Embodiment 136. The composition of Embodiment 135 wherein component (a) comprises a compound selected from the group consisting of Compounds 204, 225, 264, 316, 330, 376, 394, 463, 505, 508 and 509.

Embodiment 137. The composition of Embodiment 136 wherein component (a) comprises a compound selected from the group consisting of Compounds 225, 316, 330, 376, and 505.

Embodiment 138. The composition of Embodiment 137 wherein component (a) comprises a compound selected from the group consisting of Compounds 316 and 376.

Embodiment 139. The composition of Embodiment 138 wherein component (a) is Compound 316.

Embodiment 140. The composition of Embodiment 138 wherein component (a) is Compound 376.

Embodiment 141. The composition comprising components (a) and (b) described in the Summary of the Invention or any one of Embodiments 1 through 140 wherein component (a) is *N*-(2-chloroethyl)-2-[[4-[5-(trifluoromethyl)-1,2,4-oxadiazol-3-yl]phenyl]methyl]-4-oxazolecarboxamide.

Embodiment 142. The composition comprising components (a) and (b) described in the Summary of the Invention or any one of Embodiments 1 through 140 wherein component (a) is *N*-(2,2-difluoroethyl)-2-[[4-[5-(trifluoromethyl)-1,2,4-oxadiazol-3-yl]phenyl]methyl]-4-oxazolecarboxamide.

Embodiment 143. The composition comprising components (a) and (b) described in the Summary of the Invention or any one of Embodiments 1 through 140 wherein component (a) is *N*-(2-cyanoethyl)-2-[[4-[5-(trifluoromethyl)-1,2,4-oxadiazol-3-yl]phenyl]methyl]-4-oxazolecarboxamide.

Embodiment 144. The composition comprising components (a) and (b) described in the Summary of the Invention or any one of Embodiments 1 through 140 wherein component (a) is *N*-(2-methoxyethyl)-2-[[4-[5-(trifluoromethyl)-1,2,4-oxadiazol-3-yl]phenyl]methyl]-4-oxazolecarboxamide.

Embodiment 145. The composition comprising components (a) and (b) described in the Summary of the Invention or any one of Embodiments 1 through 140 wherein component (a) is *N*-[2-(1*H*-pyrazol-1-yl)ethyl]-2-[[4-[5-(trifluoromethyl)-1,2,4-oxadiazol-3-yl]phenyl]methyl]-4-oxazolecarboxamide.

Embodiment 146. The composition comprising components (a) and (b) described in the Summary of the Invention or any one of Embodiments 1 through 140 wherein component (a) is *N*-(2,2,2-trifluoroethyl)-2-[[4-[5-(trifluoromethyl)-1,2,4-oxadiazol-3-yl]phenyl]methyl]-4-oxazolecarboxamide.

Embodiment 147. The composition comprising components (a) and (b) described in the Summary of the Invention or any one of Embodiments 1 through 140 wherein component (a) is 2-[[4-[5-(trifluoromethyl)-1,2,4-oxadiazol-3-yl]phenyl]methyl]-*N*-(3,3,3-trifluoropropyl)-4-oxazolecarboxamide.

Embodiment 148. The composition comprising components (a) and (b) described in the Summary of the Invention or any one of Embodiments 1 through 140 wherein component (a) is ethyl 1-[[4-[5-(trifluoromethyl)-1,2,4-oxadiazol-3-yl]phenoxy]methyl]-1*H*-pyrazole-4-carboxylate.

Embodiment 149. The composition comprising components (a) and (b) described in the Summary of the Invention or any one of Embodiments 1 through 140 wherein component (a) is *N*-(2-methoxyethyl)-2-[[4-[5-(trifluoromethyl)-1,2,4-oxadiazol-3-yl]phenyl]methyl]-4-thiazolecarboxamide.

Embodiment 150. The composition comprising components (a) and (b) described in the Summary of the Invention or any one of Embodiments 1 through 140 wherein component (a) is *N*-(2-fluoroethyl)-2-[[4-[5-(trifluoromethyl)-1,2,4-oxadiazol-3-yl]phenyl]methyl]-4-oxazolecarboxamide.

Embodiment 151. The composition comprising components (a) and (b) described in the Summary of the Invention or any one of Embodiments 1 through 140 wherein component (a) is *N*-[2-(trifluoromethoxy)ethyl]-2-[[4-[5-(trifluoromethyl)-1,2,4-oxadiazol-3-yl]phenyl]methyl]-4-oxazolecarboxamide.

Embodiment 152. The composition comprising components (a) and (b) described in the Summary of the Invention or any one of Embodiments 1 through 140 wherein component (a) is (3,3-difluoro-1-pyrrolidinyl)[2-[[4-[5-(trifluoromethyl)-1,2,4-oxadiazol-3-yl]phenyl]methyl]-4-oxazolyl]methanone.

Embodiment 153. The composition comprising components (a) and (b) described in the Summary of the Invention or any one of Embodiments 1 through 140 wherein component (a) is *N*-(2,2,2-trifluoroethyl)-2-[[4-[5-(trifluoromethyl)-1,2,4-oxadiazol-3-yl]phenyl]methyl]-4-thiazolecarboxamide.

Embodiment 154. The composition comprising components (a) and (b) described in the Summary of the Invention or any one of Embodiments 1 through 140 wherein component (a) is *N*-(2,2-difluoroethyl)-2-[[4-[5-(trifluoromethyl)-1,2,4-oxadiazol-3-yl]phenyl]methyl]-4-thiazolecarboxamide.

Embodiment 155. The composition comprising components (a) and (b) described in the Summary of the Invention or any one of Embodiments 1 through 140 wherein component (a) is *N*-(2,2,2-trifluoroethyl)-5-[[4-[5-(trifluoromethyl)-1,2,4-oxadiazol-3-yl]phenyl]methyl]-1,2,4-oxadiazole-3-carboxamide.

Embodiment 156. The composition comprising components (a) and (b) described in the Summary of the Invention or any one of Embodiments 1 through 140 wherein component (a) is 3-[4-[(1-cyanomethyl-1*H*-pyrazol-3-yl)methyl]phenyl]-5-(trifluoromethyl)-1,2,4-oxadiazole.

Embodiment 157. The composition comprising components (a) and (b) described in the Summary of the Invention or any one of Embodiments 1 through 140 wherein component (a) is *N*-ethyl-1-[[[4-[5-(trifluoromethyl)-1,2,4-oxadiazol-3-yl]phenyl]methoxy]methyl]-1*H*-pyrazole-4-carboxamide.

Embodiment 158. The composition comprising components (a) and (b) described in the Summary of the Invention or any one of Embodiments 1 through 140 wherein component (a) is 1-methyl-*N*-(2,2,2-trifluoroethyl)-5-[[4-[5-(trifluoromethyl)-1,2,4-oxadiazol-3-yl]phenyl]methyl]-1*H*-pyrazole-3-carboxamide.

Embodiment 159. The composition comprising components (a) and (b) described in the Summary of the Invention or any one of Embodiments 1 through 140 wherein component (a) is 5-[[4-[5-(trifluoromethyl)-1,2,4-oxadiazol-3-yl]phenyl]methyl]-3-isoxazoleacetonitrile.

Embodiment 160. The composition comprising components (a) and (b) described in the Summary of the Invention or any one of Embodiments 1 through 140 wherein component (a) is *N*-(2-methoxyethyl)-5-[[4-[5-(trifluoromethyl)-1,2,4-oxadiazol-3-yl]phenyl]methyl]-1,2,4-oxadiazole-3-carboxamide.

Embodiment 161. The composition comprising components (a) and (b) described in the Summary of the Invention or any one of Embodiments 1 through 140 wherein component (a) is 1-methyl-*N*-(2,2,2-trifluoroethyl)-3-[[4-[5-(trifluoromethyl)-1,2,4-oxadiazol-3-yl]phenyl]methyl]-1*H*-pyrazole-5-carboxamide.

Embodiment 162. The composition of Embodiments 141 through 158 wherein component (b) comprises at least two fungicidal compounds, and when component (b) consists of a binary combination of two fungicidal compounds, wherein one of the fungicidal compounds is cyproconazole, difenoconazole, epoxiconazole, flutriafol, metconazole, prothioconazole or tebuconazole then the other fungicidal compound is other than azoxystrobin, benzovindiflupyr, bixafen, boscalid, fluopyram, fluindapyr, fluxapyroxad, isopyrazam, kresoxim-methyl, penthiopyrad, picoxystrobin, proquinazid, pyraclostrobin, quinoxyfen, sedaxane or trifloxystrobin.

Embodiment 163. The composition of Embodiments 141 through 158 wherein component (b) comprises at least two fungicidal compounds, and when component (b) consists of a binary combination of two fungicidal compounds, wherein one of the fungicidal compounds is cyproconazole, difenoconazole, epoxiconazole, flutriafol, prothioconazole or tebuconazole then the other fungicidal compound is other than azoxystrobin, benzovindiflupyr, bixafen, fluindapyr, fluxapyroxad, isopyrazam, picoxystrobin, pyraclostrobin, or trifloxystrobin.

Embodiment 164. The composition of Embodiment 160 wherein (b) comprises at least two fungicidal compounds, and when component (b) consists of a binary combination of two fungicidal compounds, wherein one of the fungicidal compounds is cyproconazole, difenoconazole, epoxiconazole, flutriafol, prothioconazole or tebuconazole then the other fungicidal compound is other than azoxystrobin, benzovindiflupyr, bixafen, fluindapyr, fluxapyroxad, isopyrazam, picoxystrobin, pyraclostrobin or trifloxystrobin.

Embodiments of this invention, including Embodiments 1-164 above as well as any other embodiments described herein, can be combined in any manner, and the descriptions of variables in the embodiments pertain not only to the compositions comprising compounds of Formula 1 with at least one other fungicidal compound, but also to compositions comprising compounds of Formula 1 with at least one invertebrate pest control compound or agent, and

also to the compounds of Formula 1 and their compositions, and also to the starting compounds and intermediate compounds useful for preparing the compounds of Formula 1. In addition, embodiments of this invention, including Embodiments 1-164 above as well as any other embodiments described herein, and any combination thereof, pertain to the methods of the present invention. Therefore of note as a further embodiment is the composition disclosed above comprising (a) at least one compound selected from the compounds of Formula 1 described above, *N*-oxides, and salts thereof; and at least one invertebrate pest control compound or agent.

Combinations of Embodiments 1-164 are illustrated by:

Embodiment A. The composition comprising components (a) and (b) described in the Summary of the Invention wherein component (a) comprises a compound of Formula 1 or salt thereof, wherein in Formula **1**,

$R^1$ is selected from U-1 through U -118

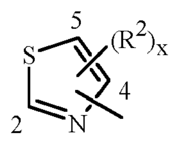
U-1
,
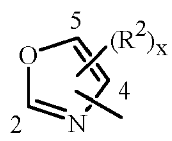
U-2
,
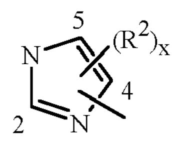
U-3
,
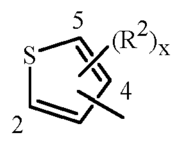
U-4
,

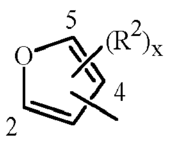
U-5
,
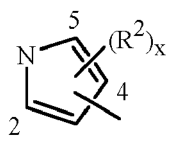
U-6
,
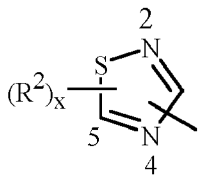
U-7
,
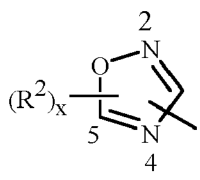
U-8
,

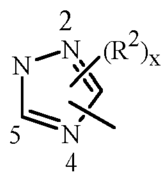
U-9
,
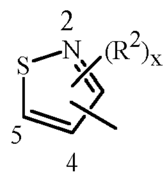
U-10
,
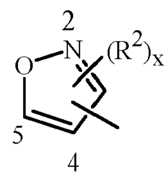
U-11
,
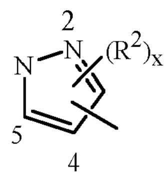
U-12
,

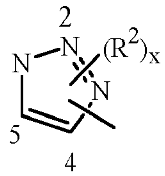
U-13
,
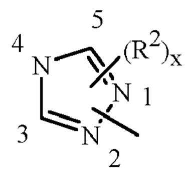
U-14
,
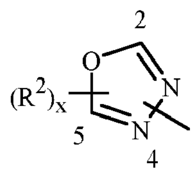
U-15
,
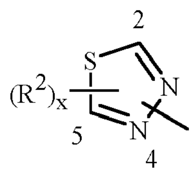
U-16
,

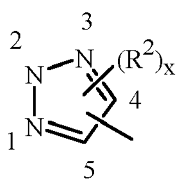
U-17
,
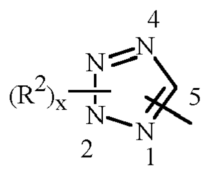
U-18
,
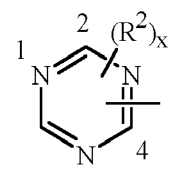
U-19
,
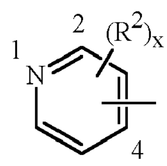
U-20
,

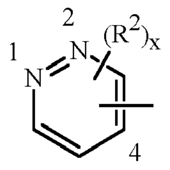 , 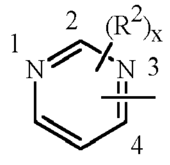 , 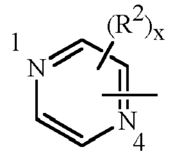 , 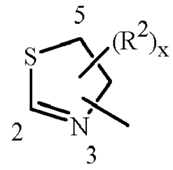 ,

U-21 U-22 U-23 U-24

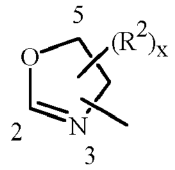 , 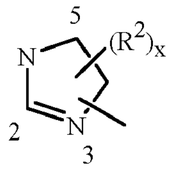 , 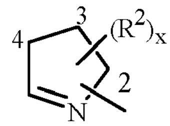 , 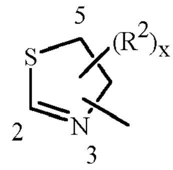 ,

U-25 U-26 U-27 U-28

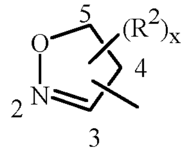 , 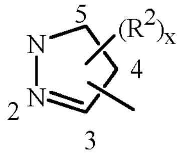 , 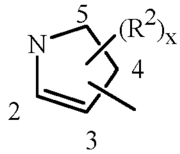 , 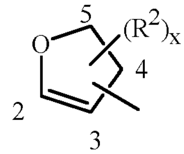 ,

U-29 U-30 U-31 U-32

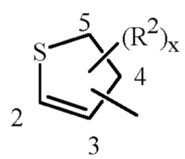 , 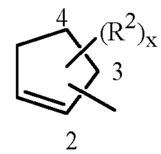 , 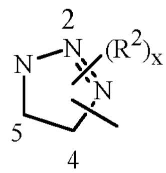 , 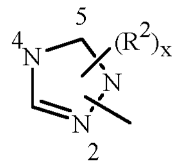 ,

U-33 U-34 U-35 U-36

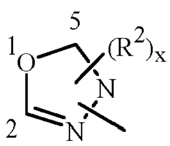 , 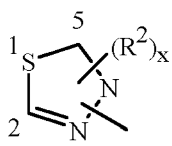 , 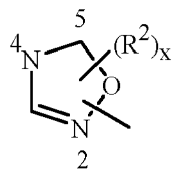 , 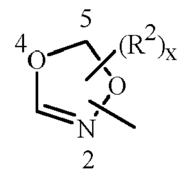 ,

U-37 U-38 U-39 U-40

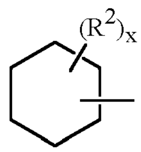 , 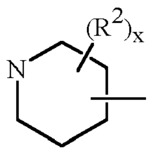 , 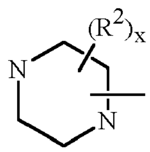 , 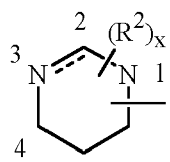 ,

U-41 U-42 U-43 U-44

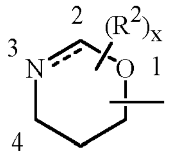 , 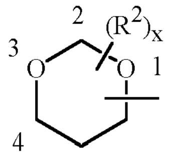 , 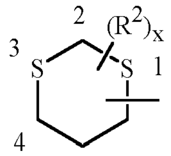 , 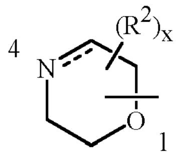 ,

U-45 U-46 U-47 U-48

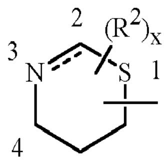 , 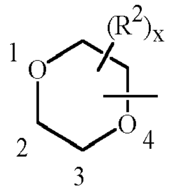 , 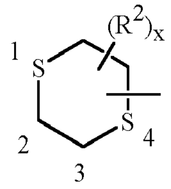 , 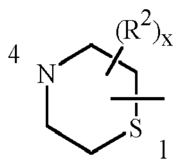 ,

U-49 U-50 U-51 U-52

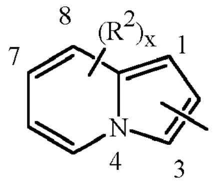 , 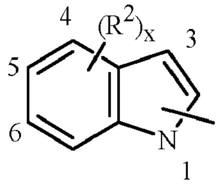 , 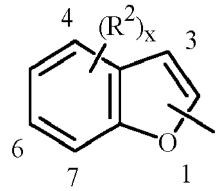 , 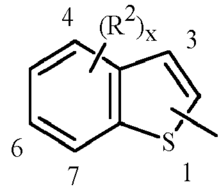 ,

U-53 U-54 U-55 U-56

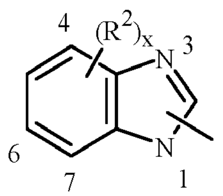 , 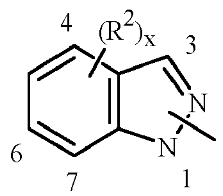 , 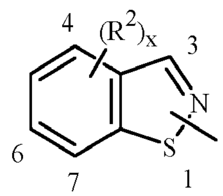 , 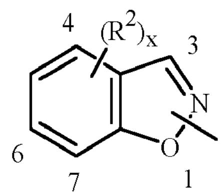 ,

U-57 U-58 U-59 U-60

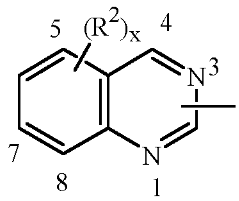 , 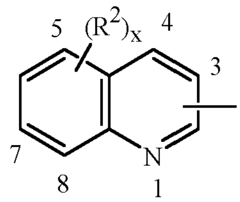 , 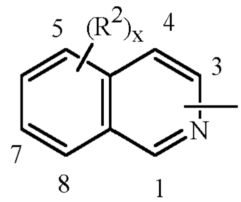 , 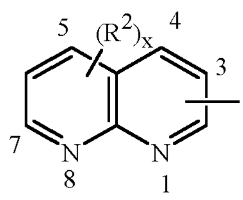 ,

U-61 U-62 U-63 U-64

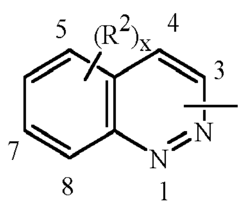 , 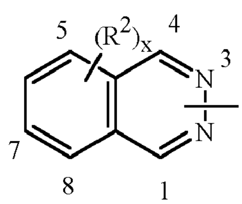 , 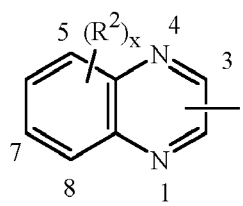 , 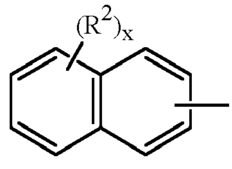 ,

U-65 U-66 U-67 U-68

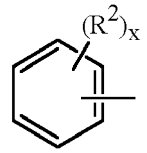
U-69 ,

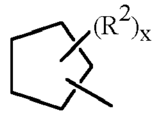
U-70 ,

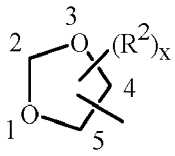
U-71 ,

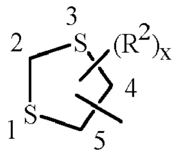
U-72 ,

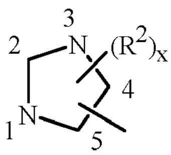
U-73 ,

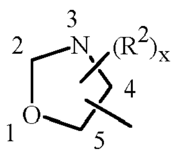
U-74 ,

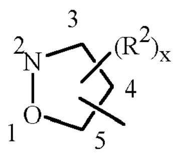
U-75 ,

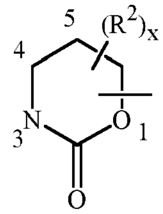
U-76 ,

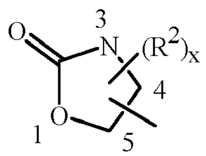
U-77 ,

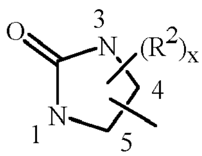
U-78 ,

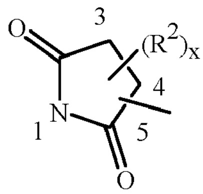
U-79 ,

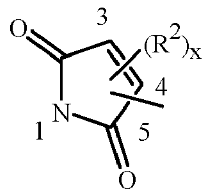
U-80 ,

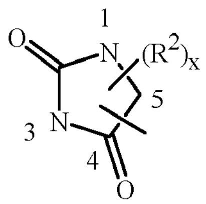
U-81 ,

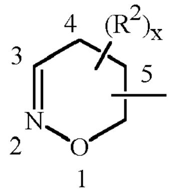
U-82 ,

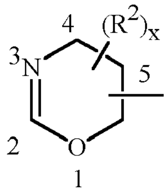
U-83 ,

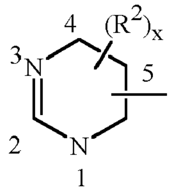
U-84 ,

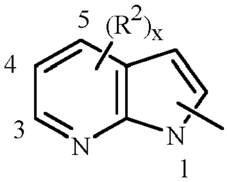
U-85 ,

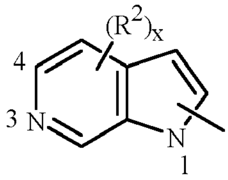
U-86 ,

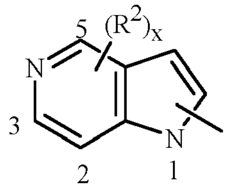
U-87 ,

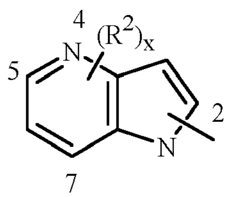
U-88 ,

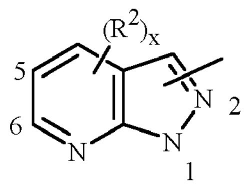
U-89 ,

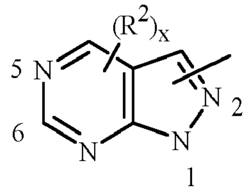
U-90 ,

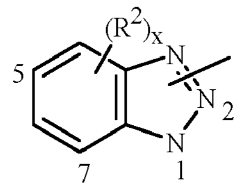
U-91 ,

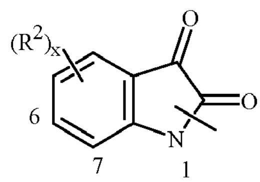
U-92 ,

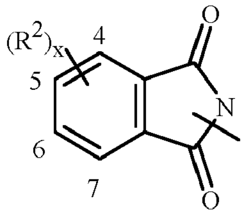 ,
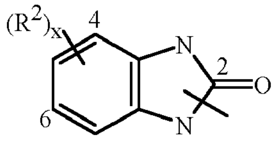 ,
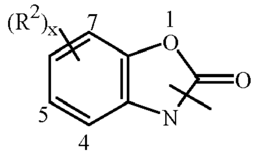 ,
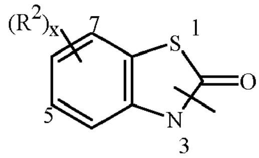 ,

U-93 U-94 U-95 U-96

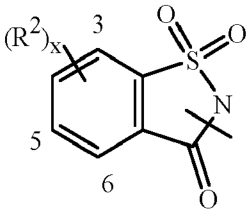 ,
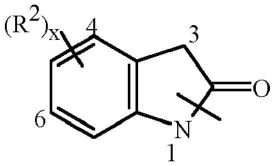 ,
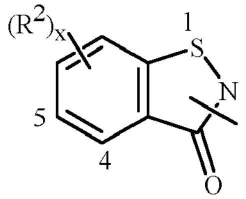 ,
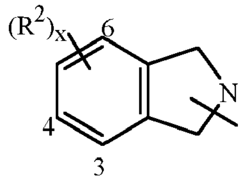 ,

U-97 U-98 U-99 U-100

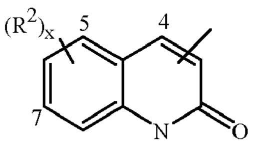 ,
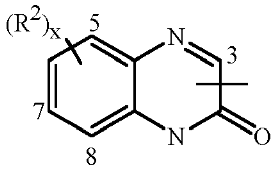 ,
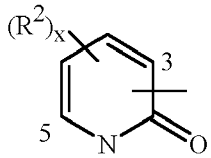 ,
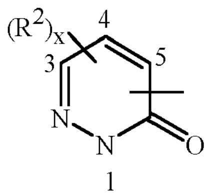 ,

U-101 U-102 U-103 U-104

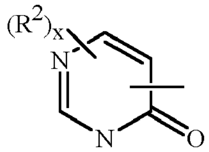 ,
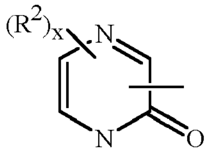 ,
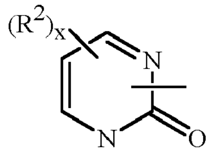 ,
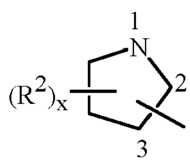 ,

U-105 U-106 U-107 U-108

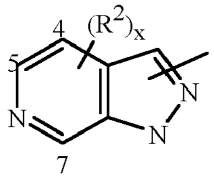 ,
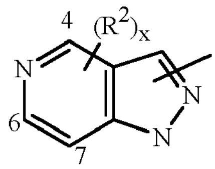 ,
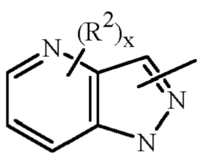 ,
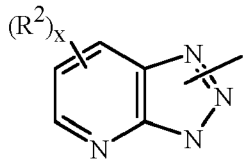 ,

U-109 U-110 U-111 U-112

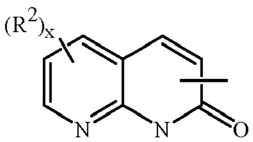 ,
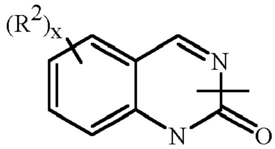 ,
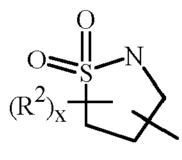 ,
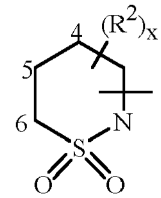 ,

U-113 U-114 U-115 U-116

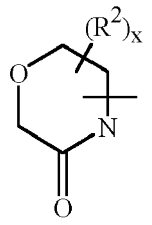 and 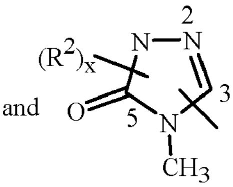 ;

U-117 U-118

wherein the floating bond is connected to L in Formula **1** through any available carbon or nitrogen atom of the depicted ring or ring system;

x is 0, 1 or 2;

L is $(CR^{4a}R^{4b})_n$, $OCH_2$, $CH_2O$, $OCH_2CH_2$, $CH_2CH_2O$ or $CH_2OCH_2$, wherein the atom to the left is connected to $R^1$, and the atom to the right is connected to J, each carbon atom is optionally substituted with up to 1 substituent selected from halogen, cyano, hydroxy, methyl, halomethyl or methoxy;

n is 1 or 2;

J is selected from J-1 through J-93

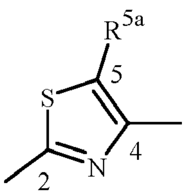 , 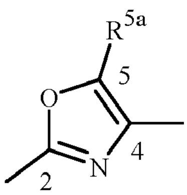 , 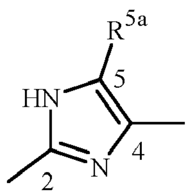 , 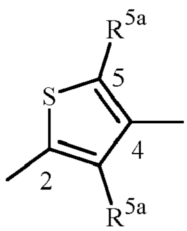 ,

J-1 J-2 J-3 J-4

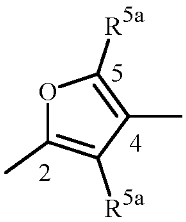 , 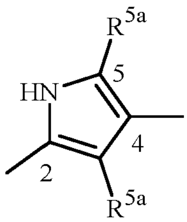 , 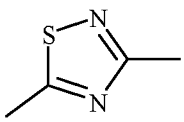 , 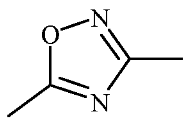 ,

J-5 J-6 J-7 J-8

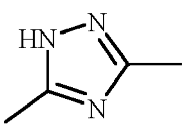 , 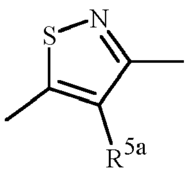 , 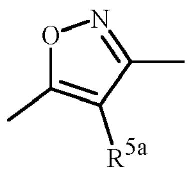 , 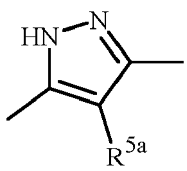 ,

J-9 J-10 J-11 J-12

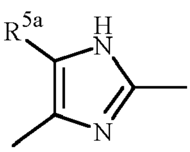 , 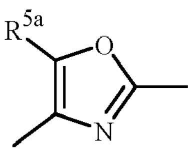 , 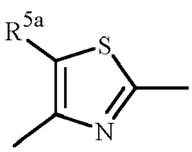 , 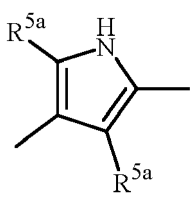 ,

J-13 J-14 J-15 J-16

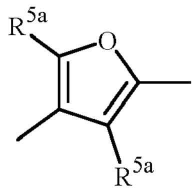 , 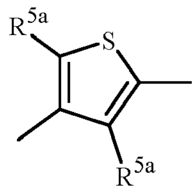 , 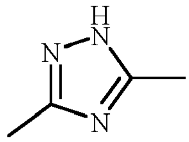 , 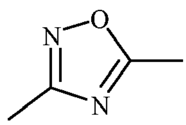 ,

J-17 J-18 J-19 J-20

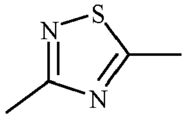 , 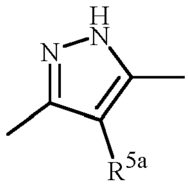 , 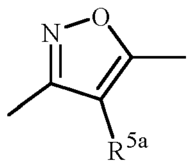 , 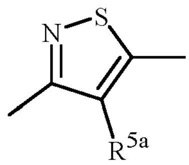 ,

J-21 J-22 J-23 J-24

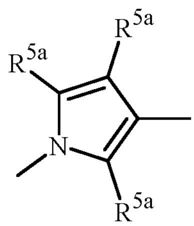 , 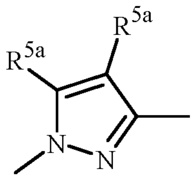 , 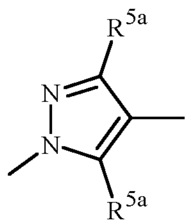 , 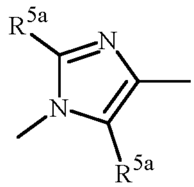 ,

J-25 J-26 J-27 J-28

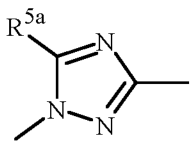 , 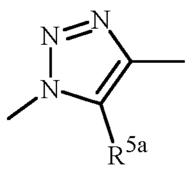 , 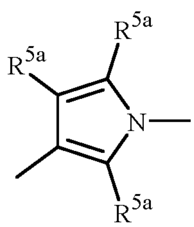 , 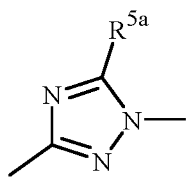 ,

J-29 J-30 J-31 J-32

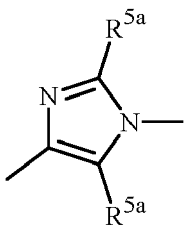 , 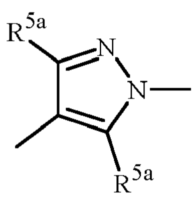 , 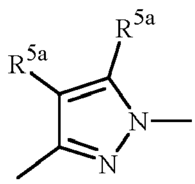 , 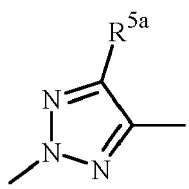 ,

J-33 J-34 J-35 J-36

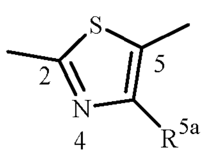 , 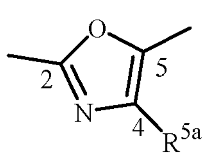 , 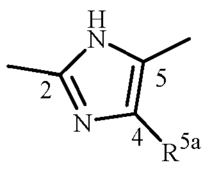 , 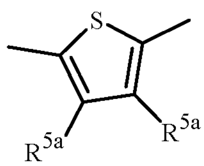 ,

J-37 J-38 J-39 J-40

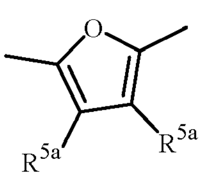 , 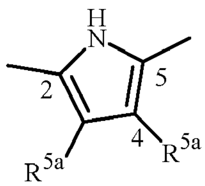 , 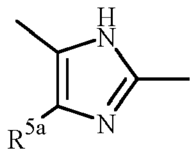 , 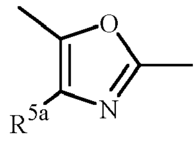 ,

J-41 J-42 J-43 J-44

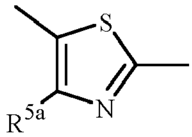 , 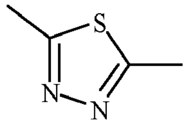 , 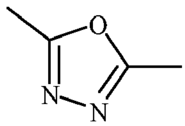 , 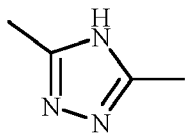 ,

J-45 J-46 J-47 J-48

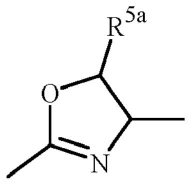 , 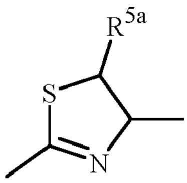 , 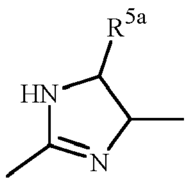 , 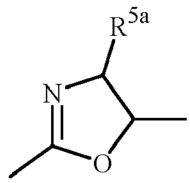 ,

J-49 J-50 J-51 J-52

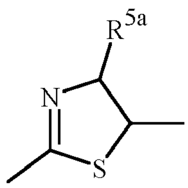 , 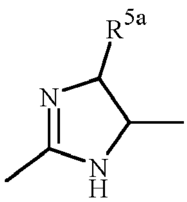 , 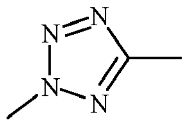 , 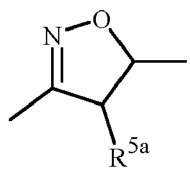 ,

J-53 J-54 J-55 J-56

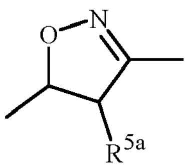 , 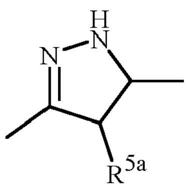 , 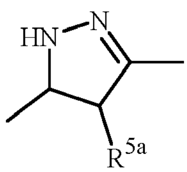 , 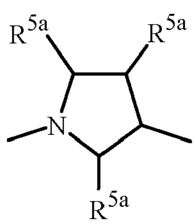 ,

J-57 J-58 J-59 J-60

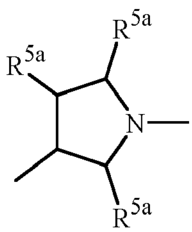 , 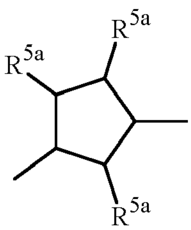 , 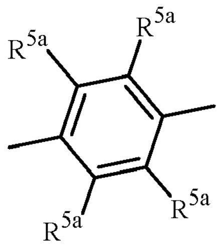 , 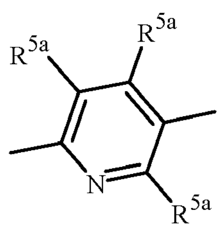 ,

J-61 J-62 J-63 J-64

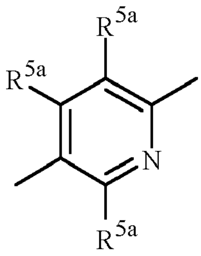
J-65
,

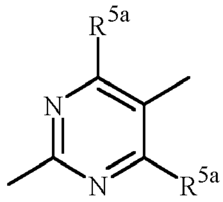
J-66
,

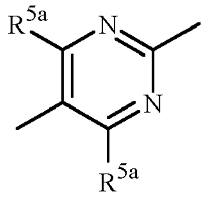
J-67
,

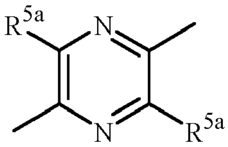
J-68
,

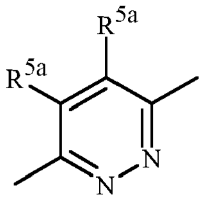
J-69
,

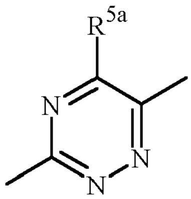
J-70
,

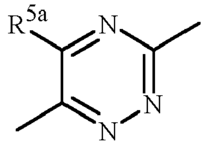
J-71
,

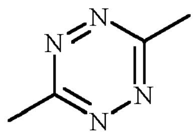
J-72
,

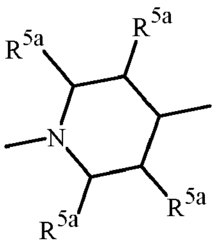
J-73
,

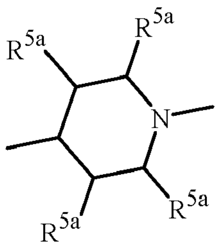
J-74
,

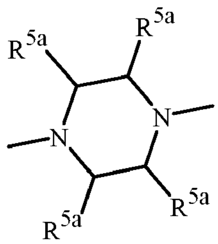
J-75
,

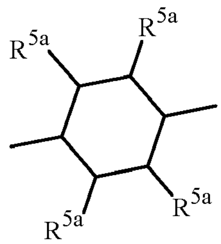
J-76
,

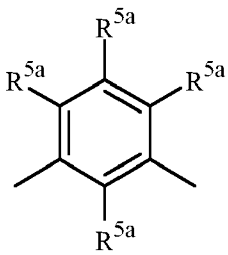
J-77
,

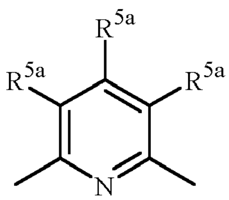
J-78
,

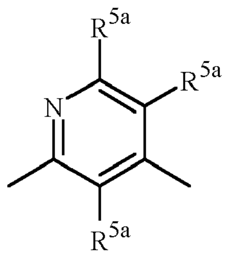
J-79
,

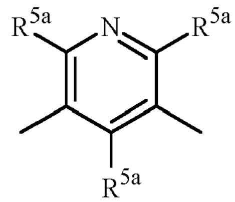
J-80
,

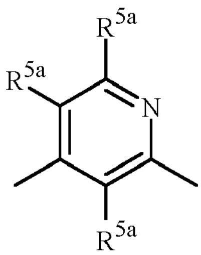
J-81
,

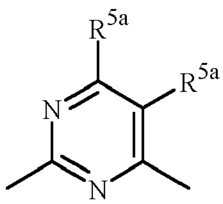
J-82
,

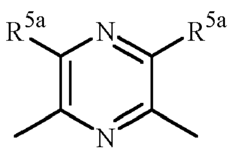
J-83
,

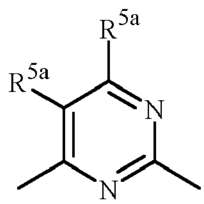
J-84
,

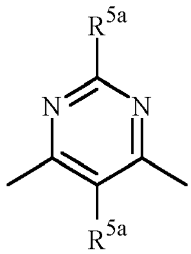
,
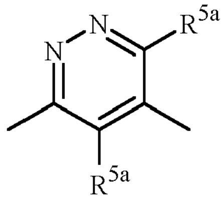
,
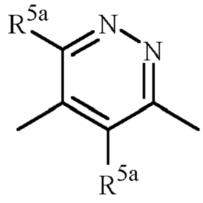
,
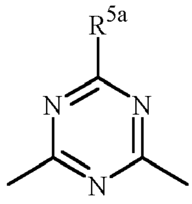
,

J-85 J-86 J-87 J-88

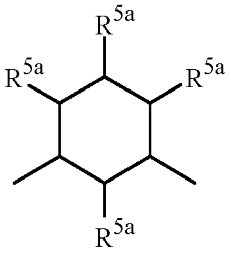
,
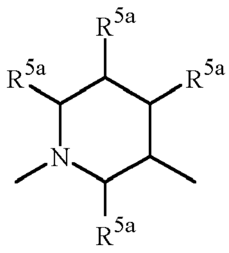
,
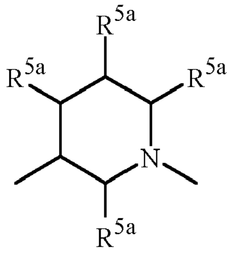
,
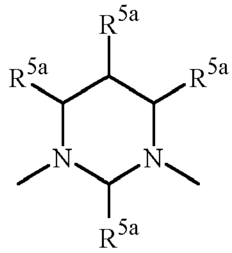

J-89 J-90 J-91 J-92

and

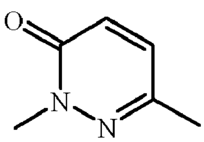
;

J-93

wherein the bond projecting to the left is bonded to L, and the bond projecting to the right is bonded to the oxadiazole ring in Formula **1**;

each $R^{5a}$ is independently H or $R^5$; provided that at most only two $R^{5a}$ substituents are other than H;

each $R^2$ is independently halogen, C(=O)$NR^{3a}R^{3b}$, C($R^6$)=$NR^7$ or -U-V-Q; or $C_1$-$C_6$ alkyl, $C_2$-$C_6$ alkenyl, $C_2$-$C_6$ alkynyl, $C_3$-$C_7$ cycloalkyl, $C_1$-$C_6$ alkoxy, $C_2$-$C_6$ alkenyloxy, $C_2$-$C_6$ alkynyloxy, $C_3$-$C_7$ cycloalkoxy, $C_1$-$C_6$ alkylthio, $C_2$-$C_6$ alkoxycarbonyl, $C_3$-$C_6$ alkenyloxycarbonyl, $C_3$-$C_6$ alkynyloxycarbonyl, $C_4$-$C_7$ cycloalkoxycarbonyl, $C_2$-$C_6$ alkylcarbonyloxy or $C_2$-$C_6$ alkylcarbonylamino, each optionally substituted with up to 1 substituent selected from $R^{10}$;

each $R^{3a}$ is independently H, cyano, hydroxy, $C_1$-$C_4$ alkyl, $C_1$-$C_4$ haloalkyl, $C_2$-$C_4$ alkenyl, $C_2$-$C_4$ alkynyl, $C_1$-$C_4$ alkoxy, $C_2$-$C_4$ alkoxyalkyl, $C_2$-$C_4$ alkylcarbonyl, $C_2$-$C_4$ haloalkylcarbonyl or $C_3$-$C_5$ alkoxycarbonylalkyl;

each $R^{3b}$ is independently H, $C_1$-$C_6$ alkyl, $C_1$-$C_6$ haloalkyl, $C_2$-$C_6$ alkenyl, $C_2$-$C_6$ haloalkenyl, $C_2$-$C_6$ alkynyl, $C_2$-$C_6$ haloalkynyl, $C_4$-$C_{10}$ cycloalkylalkyl, $C_4$-$C_{10}$ halocycloalkylalkyl, $C_2$-$C_6$ alkoxyalkyl or $C_2$-$C_6$ haloalkoxy-alkyl, each optionally substituted with up to 1 substituent selected from cyano, $C_2$-$C_4$ alkylcarbonyl and $C_2$-$C_4$ alkoxycarbonyl; or

a pair of $R^{3a}$ and $R^{3b}$ substituents attached to the same nitrogen atom are taken together to form a pyrrolidinyl ring optionally substituted with up to 2 substituents independently selected from halogen;

each $R^{4a}$ and $R^{4b}$ is independently H, halogen, hydroxy, methyl or methoxy;

each $R^5$ is independently cyano, halogen, methyl or methoxy;

each $R^6$ is independently H, cyano, halogen methyl or methoxy;

each $R^7$ is independently $C_1$-$C_4$ alkoxy, $C_2$-$C_4$ alkenyloxy or $C_2$-$C_4$ alkynyloxy;

each $R^{10}$ is independently halogen, $C_1$-$C_4$ alkyl, $C_1$-$C_4$ haloalkyl, $C_1$-$C_4$ alkoxy, $C_1$-$C_4$ haloalkoxy, $C_2$-$C_4$ alkyl-carbonyl, $C_2$-$C_4$ haloalkylcarbonyl, $C_2$-$C_5$ alkoxycarbonyl or C($R^{13}$)=$NOR^{14}$;

each U is independently a direct bond, C(=O)O or C(=O)$NR^{17}$;

each V is independently a direct bond; or $C_1$-$C_2$ alkylene optionally substituted with up to 1 substituent selected from $C_1$-$C_2$ alkyl, $C_1$-$C_2$ alkoxy and $C_1$-$C_2$ haloalkoxy;

each Q is independently phenyl optionally substituted with up to 2 substituents independently selected from $R^{12}$; or pyridinyl or pyrazolyl, each ring optionally substituted with up to 2 substituents independently selected

from $R^{12}$;
each $R^{12}$ is independently halogen, cyano, $C_1$-$C_3$ alkyl, $C_1$-$C_3$ haloalkyl or $C_1$-$C_3$ alkoxy;
each $R^{13}$ is independently H, halogen, methyl or methoxy;
each $R^{14}$ is independently H, methyl, halomethyl, $C_2$-$C_4$ alkylcarbonyl or $C_2$-$C_4$ alkoxycarbonyl; and
each $R^{17}$ is independently H, cyano, methyl or halomethyl.

Embodiment B. The composition of Embodiment A wherein in Formula **1**,

$R^1$ is U-1, U-2, U-3, U-4, U-5, U-7, U-8, U-10, U-11, U-12 or U-29;
x is 1 or 2;
L is $(CR^{4a}R^{4b})_n$, $CH_2O$ or $CH_2OCH_2$, wherein the atom to the left is connected to $R^1$, and the atom to the right is connected to J, each carbon atom is optionally substituted with up to 1 substituent selected from halogen, cyano, hydroxy, methyl, halomethyl or methoxy;
J is J-4, J-18, J-27, J-40 or J-63;
each $R^2$ is independently $C(=O)NR^{3a}R^{3b}$ or -U-V-Q; or $C_1$-$C_3$ alkyl, $C_2$-$C_6$ alkoxycarbonyl, $C_3$-$C_6$ alkenyloxycarbonyl or $C_3$-$C_6$ alkynyloxycarbonyl, each optionally substituted with up to 1 substituent selected from $R^{10}$;
each $R^{3a}$ is independently H, $C_1$-$C_4$ alkyl, $C_1$-$C_4$ haloalkyl, $C_2$-$C_4$ alkenyl, $C_2$-$C_4$ alkynyl, $C_2$-$C_4$ alkoxyalkyl or $C_3$-$C_5$ alkoxycarbonylalkyl;
each $R^{3b}$ is independently H, $C_1$-$C_6$ alkyl, $C_1$-$C_6$ haloalkyl, $C_2$-$C_6$ alkoxyalkyl or $C_2$-$C_6$ haloalkoxyalkyl, each optionally substituted with up to 1 substituent selected from cyano, $C_2$-$C_4$ alkylcarbonyl and $C_2$-$C_4$ alkoxycarbonyl; or
a pair of $R^{3a}$ and $R^{3b}$ substituents attached to the same nitrogen atom are taken together to form a pyrrolidinyl ring optionally substituted with up to 2 fluorine atoms;
each $R^{4a}$ and $R^{4b}$ is independently H or methyl;
each $R^5$ is independently methyl or methoxy;
each $R^{10}$ is independently halogen, $C_1$-$C_4$ alkyl, $C_1$-$C_4$ alkoxy, $C_2$-$C_4$ alkylcarbonyl, $C_2$-$C_4$ haloalkylcarbonyl or $C_2$-$C_5$ alkoxycarbonyl;
each U is independently C(=O)O or $C(=O)NR^{17}$;
each V is independently $C_1$-$C_2$ alkylene; and
each $R^{12}$ is independently halogen, methyl or methoxy.

Embodiment C. The composition of Embodiment B wherein in Formula **1,**

$R^1$ is U-1, U-2, U-8, U-11 or U-12;
L is $(CR^{4a}R^{4b})_n$, $CH_2O$ or $CH_2OCH_2$,
n is 1;
J is J-63;
each $R^2$ is independently $C(=O)NR^{3a}R^{3b}$; or $C_1$-$C_2$ alkyl, $C_2$-$C_6$ alkoxycarbonyl, $C_3$-$C_6$ alkenyloxycarbonyl or $C_3$-$C_6$ alkynyloxycarbonyl, each optionally substituted with up to 1 substituent selected from $R^{10}$;
each $R^{3a}$ is independently H, $C_1$-$C_3$ alkyl, $C_1$-$C_3$ haloalkyl or $C_2$-$C_3$ alkoxyalkyl;
each $R^{3b}$ is independently H, $C_1$-$C_3$ alkyl, $C_1$-$C_3$ haloalkyl, $C_2$-$C_3$ alkoxyalkyl or $C_2$-$C_3$ haloalkoxyalkyl, each optionally substituted with up to 1 substituent selected from cyano;
each $R^{4a}$ and $R^{4b}$ is H;
each $R^{5a}$ is H; and
each $R^{10}$ is independently cyano, halogen, $C_1$-$C_2$ alkoxy, $C_2$-$C_3$ alkylcarbonyl or $C_2$-$C_3$ haloalkylcarbonyl.

Embodiment CC. The composition of Embodiment C wherein in Formula **1**,

$R^1$ is U-1 connected at its 2-position to L; or
$R^1$ is U-2 connected at its 2-position to L; or
$R^1$ is U-8 connected at its 5-position to L; or
$R^1$ is U-11 connected at its 5-position to L; or
$R^1$ is U-12 connected at its 1-position, 3-position or 5-position to L;
each $R^{3a}$ is independently H;
each $R^{3b}$ is independently H, $C_1$-$C_3$ alkyl, $C_1$-$C_3$ haloalkyl or $C_2$-$C_3$ alkoxyalkyl, each optionally substituted with up to 1 substituent selected from cyano;
each $R^{10}$ is independently cyano.

Embodiment CCC. The composition of Embodiment B wherein in Formula **1**,

$R^1$ is U-1, U-2, U-8 or U-12;
L is $(CR^{4a}R^{4b})_n$ or $CH_2O$;
n is 1;
J is J-63;
each $R^2$ is independently $C(=O)NR^{3a}R^{3b}$; or $C_1$-$C_2$ alkyl, $C_2$-$C_6$ alkoxycarbonyl, $C_3$-$C_6$ alkenyloxycarbonyl or $C_3$-$C_6$ alkynyloxycarbonyl, each optionally substituted with up to 1 substituent selected from $R^{10}$;
each $R^{3a}$ is independently H, $C_1$-$C_3$ alkyl, $C_1$-$C_3$ haloalkyl or $C_2$-$C_3$ alkoxyalkyl;
each $R^{3b}$ is independently H, $C_1$-$C_3$ alkyl, $C_1$-$C_3$ haloalkyl, $C_2$-$C_3$ alkoxyalkyl or $C_2$-$C_3$ haloalkoxyalkyl, each optionally substituted with up to 1 substituent selected from cyano;
each $R^{4a}$ and $R^{4b}$ is H;
each $R^{5a}$ is H; and
each $R^{10}$ is independently halogen, $C_1$-$C_2$ alkoxy, $C_2$-$C_3$ alkylcarbonyl or $C_2$-$C_3$ haloalkylcarbonyl.

Embodiment D. The composition of Embodiment CCC wherein in Formula 1,

$R^1$ is U-2 or U-12;
x is 1;
$R^2$ is $C(=O)NR^{3a}R^{3b}$ or $C_2$-$C_6$ alkoxycarbonyl;
$R^{3a}$ is H; and
$R^{3b}$ is H, $C_1$-$C_3$ alkyl, $C_1$-$C_3$ cyanoalkyl, $C_1$-$C_3$ haloalkyl, $C_2$-$C_3$ alkoxyalkyl or $C_2$-$C_3$ haloalkoxyalkyl.

Embodiment E. The composition of Embodiment D wherein in Formula **1**,

$R^1$ is U-2 connected at its 2-position to L and L is $CH_2$; or
$R^1$ is U-12 connected at its 1-position to L and L is $CH_2O$; and
$R^2$ is $C(=O)NR^{3a}R^{3b}$ or $C_2$-$C_3$ alkoxycarbonyl.

Embodiment F. The composition comprising components (a) and (b) described in the Summary of the Invention wherein component (a) comprises a compound of Formula 1 or salt thereof, wherein in Formula **1**,

$R^1$ is

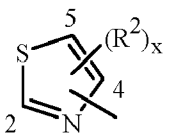
U-1
,
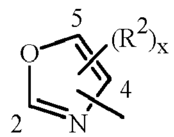
U-2
,
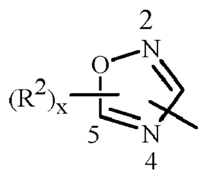
U-8
or
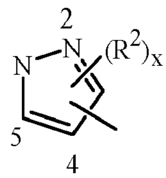
U-12
;

wherein the floating bond is connected to L in Formula **1** through any available carbon or nitrogen atom of the depicted ring;
x is 1 or 2;
L is $CH_2$, $CH_2O$ or $CH_2OCH_2$, wherein the atom to the left is connected to $R^1$, and the atom to the right is connected to J;
J is

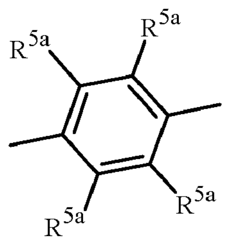
;

J-63

wherein the bond projecting to the left is bonded to L, and the bond projecting to the right is bonded to the oxadiazole ring in Formula **1**;
each $R^{5a}$ is H;
each $R^2$ is independently halogen, C(=O)$NR^{3a}R^{3b}$ or -U-V-Q; or $C_1$-$C_6$ alkyl, $C_2$-$C_6$ alkenyl, $C_2$-$C_6$ alkynyl, $C_1$-$C_6$ alkoxy, $C_2$-$C_6$ alkoxycarbonyl, $C_3$-$C_6$ alkenyloxycarbonyl, $C_3$-$C_6$ alkynyloxycarbonyl or $C_4$-$C_7$ cycloalkoxycarbonyl, each optionally substituted with up to 1 substituent selected from $R^{10}$;
each $R^{3a}$ is independently H, $C_1$-$C_3$ alkyl, $C_1$-$C_3$ haloalkyl or $C_2$-$C_3$ alkoxyalkyl; and
each $R^{3b}$ is independently H, $C_1$-$C_3$ alkyl, $C_1$-$C_3$ haloalkyl, $C_2$-$C_3$ alkoxyalkyl or $C_2$-$C_3$ haloalkoxyalkyl, each optionally substituted with up to 1 substituent selected from cyano;
each $R^{10}$ is cyano;
each U is independently C(=O)$NR^{17}$;
each $R^{17}$ is H;
each V is independently $C_1$-$C_2$ alkylene; and
each Q is pyrazolyl.

Embodiment G. The composition of Embodiment F wherein in Formula **1,**

$R^1$ is U-2 or U-12;
L is $CH_2$ or $CH_2O$;
each $R^2$ is independently C(=O)$NR^{3a}R^{3b}$, $C_1$-$C_6$ alkyl or $C_2$-$C_6$ alkoxycarbonyl;
each $R^{3a}$ is H; and
each $R^{3b}$ is independently $C_1$-$C_3$ alkyl, $C_1$-$C_3$ haloalkyl, $C_2$-$C_3$ alkoxyalkyl or $C_2$-$C_3$ haloalkoxyalkyl.

Embodiment H. The composition of Embodiment G wherein in Formula **1**,

x is 1;
$R^2$ is C(=O)$NR^{3a}R^{3b}$; and
$R^{3b}$ is $C_1$-$C_3$ haloalkyl, $C_2$-$C_3$ alkoxyalkyl or $C_2$-$C_3$ haloalkoxyalkyl.

Embodiment I. The composition of Embodiment H wherein in Formula **1**,

$R^1$ is U-2 connected at its 2-position to L, L is $CH_2$ and $R^{3b}$ is $C_1$-$C_3$ haloalkyl; or
$R^1$ is U-12 connected at its 1-position to L, L is $CH_2O$ and $R^{3b}$ is $C_2$-$C_3$ alkoxyalkyl.

Embodiment J. The composition any one of Embodiments A through I wherein component (a) comprises a compound selected from the group consisting of: Compound 204, Compound 225, Compound 229, Compound 257, Compound 264, Compound 316, Compound 330, Compound 376, Compound 394, Compound 400, Compound 401, Compound 402, Compound 463, Compound 505, Compound 506, Compound 507, Compound 508 and Compound 509.
Embodiment K. The composition of Embodiment J wherein component (a) comprises a compound selected from the group consisting of: Compound 204, Compound 225, Compound 264, Compound 316, Compound 330, Compound 376, Compound 394, Compound 463, Compound 505, Compound 508 and Compound 509.
Embodiment L. The composition of Embodiment K wherein component (a) comprises a Compound 225, Compound 316, Compound 330 and Compound 505.
Embodiment M. The composition of Embodiment L wherein component (a) comprises Compound 316 and Compound 376.
Embodiment N. The composition of Embodiment M wherein component (a) comprises Compound 316.
Embodiment O. The composition of Embodiment M wherein component (a) comprises Compound 376.

Embodiment P. The composition any one of Embodiments A through I wherein component (a) comprises a compound selected from the group consisting of: 225, 229, 257, 316, 376, 394, 506, 507, 508, 509, 510, 511 and 512.

Embodiment Q. The composition of Embodiment P wherein component (a) comprises a compound selected from the group consisting of: 507, 508, 509 and 510.

Embodiment B1. The composition described in the Summary of the Invention (including but not limited to the composition of any one of Embodiments 1 through 164 and A through Q) wherein component (b) includes at least one compound selected from (b1) methyl benzimidazole carbamate fungicides such as benomyl, carbendazim, fuberidazole thiabendazole, thiophanate and thiophanate-methyl.

Embodiment B2. The composition described in the Summary of the Invention (including but not limited to the composition of any one of Embodiments 1 through 164 and A through Q) wherein component (b) includes at least one compound selected from (b2) dicarboximide fungicides such as chlozolinate, dimethachlone, iprodione, procymidone and vinclozolin.

Embodiment B3. The composition described in the Summary of the Invention (including but not limited to the composition of any one of Embodiments 1 through 164 and A through Q) wherein component (b) includes at least one compound selected from (b3) demethylation inhibitor fungicides such as triforine, buthiobate, pyrifenox, pyrisoxazole, fenarimol, nuarimol, triarimol econazole, imazalil, oxpoconazole, pefurazoate, prochloraz, triflumizole, azaconazole, bitertanol, bromuconazole, cyproconazole, difenoconazole, diniconazole (including diniconazole-M), epoxiconazole, etaconazole, fenbuconazole, fluquinconazole, flusilazole, flutriafol, hexaconazole, imibenconazole, ipconazole, mefentrifluconazole, metconazole, myclobutanil, penconazole, propiconazole, prothioconazole, ipfentrifluconazole, quinconazole, simeconazole, tebuconazole, tetraconazole triadimefon, triadimenol, triticonazole, uniconazole and uniconazole-P.

Embodiment B4. The composition described in the Summary of the Invention (including but not limited to the composition of any one of Embodiments 1 through 164 and A through Q) wherein component (b) includes at least one compound selected from (b4) phenylamide fungicides such as metalaxyl, metalaxyl-M, benalaxyl, benalaxyl-M, furalaxyl, ofurace and oxadixyl.

Embodiment B5. The composition described in the Summary of the Invention (including but not limited to the composition of any one of Embodiments 1 through 164 and A through Q) wherein component (b) includes at least one compound selected from (b5) amine/morpholine fungicides such as aldimorph, dodemorph, fenpropimorph, tridemorph, trimorphamide, fenpropidin, piperalin and spiroxamine.

Embodiment B6. The composition described in the Summary of the Invention (including but not limited to the composition of any one of Embodiments 1 through 164 and A through Q) wherein component (b) includes at least one compound selected from (b6) phospholipid biosynthesis inhibitor fungicides such as edifenphos, iprobenfos, pyrazophos and isoprothiolane.

Embodiment B7. The composition described in the Summary of the Invention (including but not limited to the composition of any one of Embodiments 1 through 164 and A through Q) wherein component (b) includes at least one compound selected from (b7) succinate dehydrogenase inhibitor fungicides such as benodanil, flutolanil, mepronil, isofetamid, fluopyram, fenfuram, carboxin, oxycarboxin thifluzamide, benzovindiflupyr, bixafen, fluindapyr, fluxapyroxad, furametpyr, inpyrfluxam, isopyrazam, penflufen, penthiopyrad, pyrapropoyne, sedaxane, flubeneteram, isoflucypram, pydiflumetofen, boscalid and pyraziflumid.

Embodiment B8. The composition described in the Summary of the Invention (including but not limited to the composition of any one of Embodiments 1 through 164 and A through Q) wherein component (b) includes at least one compound selected from (b8) hydroxy(2-amino-)pyrimidine fungicides such as bupirimate, dimethirimol and ethirimol.

Embodiment B9. The composition described in the Summary of the Invention (including but not limited to the composition of any one of Embodiments1 through 164 and A through Q) wherein component (b) includes at least one compound selected from (b9) anilinopyrimidine fungicides such as cyprodinil, mepanipyrim and pyrimethanil.

Embodiment B10. The composition described in the Summary of the Invention (including but not limited to the composition of any one of Embodiments 1 through 164 and A through Q) wherein component (b) includes at least one compound selected from (b10) *N*-phenyl carbamate fungicides such as diethofencarb.

Embodiment B11. The composition described in the Summary of the Invention (including but not limited to the composition of any one of Embodiments 1 through 164 and A through Q) wherein component (b) includes at least one compound selected from (b11) fungicides quinone outside inhibitor fungicides such as azoxystrobin, coumoxystrobin, enoxastrobin, flufenoxystrobin, picoxystrobin, pyraoxystrobin, mandestrobin, pyraclostrobin, pyrametostrobin, triclopyricarb, kresoxim-methyl, trifloxystrobin, dimoxystrobin, fenaminstrobin, metominostrobin, orysastrobin, fluoxastrobin, famoxadone, fenamidone and pyribencarb.

Embodiment B12. The composition described in the Summary of the Invention (including but not limited to the composition of any one of Embodiments 1 through 164 and A through Q) wherein component (b) includes at least one compound selected from (b12) phenylpyrrole fungicides compound such as fenpiclonil and fludioxonil.

Embodiment B13. The composition described in the Summary of the Invention (including but not limited to the

composition of any one of Embodiments 1 through 164 and A through Q) wherein component (b) includes at least one compound selected from (b13) azanaphthalene fungicides such as quinoxyfen and proquinazid.

Embodiment B14. The composition described in the Summary of the Invention (including but not limited to the composition of any one of Embodiments 1 through 164 and A through Q) wherein component (b) includes at least one compound selected from (b14) cell peroxidation inhibitor fungicides such as biphenyl, chloroneb, dicloran, quintozene, tecnazene, tolclofos-methyl and etridiazole.

Embodiment B15. The composition described in the Summary of the Invention (including but not limited to the composition of any one of Embodiments 1 through 164 and A through Q) wherein component (b) includes at least one compound selected from (b15) melanin biosynthesis inhibitors-reductase fungicides such as fthalide, pyroquilon and tricyclazole.

Embodiment B16a. The composition described in the Summary of the Invention (including but not limited to the composition of any one of Embodiments 1 through 164 and A through Q) wherein component (b) includes at least one compound selected from (b16a) melanin biosynthesis inhibitors-dehydratase fungicides such as carpropamid, diclocymet, and fenoxanil.

Embodiment B16b. The composition described in the Summary of the Invention (including but not limited to the composition of any one of Embodiments 1 through 164 and A through Q) wherein component (b) includes at least one compound selected from (b16b) melanin biosynthesis inhibitor-polyketide synthase fungicides such as tolprocarb.

Embodiment B17. The composition described in the Summary of the Invention (including but not limited to the composition of any one of Embodiments 1 through 164 and A through Q) wherein component (b) includes at least one compound selected from (b17) keto reductase inhibitor fungicides such as fenhexamid, fenpyrazamine, quinofumelin and ipflufenoquin.

Embodiment B18. The composition described in the Summary of the Invention (including but not limited to the composition of any one of Embodiments 1 through 164 and A through Q) wherein component (b) includes at least one compound selected from (b18) squalene-epoxidase inhibitor fungicides such as pyributicarb, naftifine and terbinafine.

Embodiment B19. The composition described in the Summary of the Invention (including but not limited to the composition of any one of Embodiments 1 through 164 and A through Q) wherein component (b) includes at least one compound selected from (b19) polyoxin fungicides such as polyoxin.

Embodiment B20. The composition described in the Summary of the Invention (including but not limited to the composition of any one of Embodiments 1 through 164 and A through Q) wherein component (b) includes at least one compound selected from (b20) phenylurea fungicides such as pencycuron.

Embodiment B21. The composition described in the Summary of the Invention (including but not limited to the composition of any one of Embodiments 1 through 164 and A through Q) wherein component (b) includes at least one compound selected from (b21) quinone inside inhibitor fungicides such as cyazofamid, amisulbrom and fenpicoxamid (Registry Number 517875-34-2).

Embodiment B22. The composition described in the Summary of the Invention (including but not limited to the composition of any one of Embodiments 1 through 164 and A through Q) wherein component (b) includes at least one compound selected from (b22) benzamide and thiazole carboxamide fungicides such as zoxamide and ethaboxam.

Embodiment B23. The composition described in the Summary of the Invention (including but not limited to the composition of any one of Embodiments 1 through 164 and A through Q) wherein component (b) includes at least one compound selected from (b23) enopyranuronic acid antibiotic fungicides such as blasticidin-S.

Embodiment B24. The composition described in the Summary of the Invention (including but not limited to the composition of any one of Embodiments 1 through 164 and A through Q) wherein component (b) includes at least one compound selected from (b24) hexopyranosyl antibiotic fungicides such as kasugamycin.

Embodiment B25. The composition described in the Summary of the Invention (including but not limited to the composition of any one of Embodiments 1 through 164 and A through Q) wherein component (b) includes at least one compound selected from (b25) glucopyranosyl antibiotic: protein synthesis fungicides such as streptomycin.

Embodiment B26. The composition described in the Summary of the Invention (including but not limited to the composition of any one of Embodiments 1 through 164 and A through Q) wherein component (b) includes at least one compound selected from (b26) glucopyranosyl antibiotic: trehalase and inositol biosynthesis fungicides such as validamycin.

Embodiment B27. The composition described in the Summary of the Invention (including but not limited to the composition of any one of Embodiments 1 through 164 and A through Q) wherein component (b) includes at least one compound selected from (b27) cyanoacetamideoxime fungicides such as cymoxanil.

Embodiment B28. The composition described in the Summary of the Invention (including but not limited to the composition of any one of Embodiments 1 through 164 and A through Q) wherein component (b) includes at least

one compound selected from (b28) carbamate fungicides such as propamacarb, prothiocarb and iodocarb.

Embodiment B29. The composition described in the Summary of the Invention (including but not limited to the composition of any one of Embodiments 1 through 164 and A through Q) wherein component (b) includes at least one compound selected from (b29) oxidative phosphorylation uncoupling fungicides such as fluazinam, binapacryl, meptyldinocap and dinocap.

Embodiment B30. The composition described in the Summary of the Invention (including but not limited to the composition of any one of Embodiments 1 through 164 and A through Q) wherein component (b) includes at least one compound selected from (b30) organo tin fungicides such as fentin acetate, fentin chloride and fentin hydroxide.

Embodiment B31. The composition described in the Summary of the Invention (including but not limited to the composition of any one of Embodiments 1 through 164 and A through Q) wherein component (b) includes at least one compound selected from (b31) carboxylic acid fungicides such as oxolinic acid.

Embodiment B32. The composition described in the Summary of the Invention (including but not limited to the composition of any one of Embodiments 1 through 164 and A through Q) wherein component (b) includes at least one compound selected from (b32) heteroaromatic fungicides such as hymexazole and octhilinone.

Embodiment B33. The composition described in the Summary of the Invention (including but not limited to the composition of any one of Embodiments 1 through 164 and A through Q) wherein component (b) includes at least one compound selected from (b33) phosphonate fungicides such as phosphorous acid and its various salts, including fosetyl-aluminum.

Embodiment B34. The composition described in the Summary of the Invention (including but not limited to the composition of any one of Embodiments 1 through 164 and A through Q) wherein component (b) includes at least one compound selected from (b34) phthalamic acid fungicides such as teclofthalam.

Embodiment B35. The composition described in the Summary of the Invention (including but not limited to the composition of any one of Embodiments 1 through 164 and A through Q) wherein component (b) includes at least one compound selected from (b35) benzotriazine fungicides such as triazoxide.

Embodiment B36. The composition described in the Summary of the Invention (including but not limited to the composition of any one of Embodiments 1 through 164 and A through Q) wherein component (b) includes at least one compound selected from (b36) benzenesulfonamide fungicides such as flusulfamide.

Embodiment B37. The composition described in the Summary of the Invention (including but not limited to the composition of any one of Embodiments 1 through 164 and A through Q) wherein component (b) includes at least one compound selected from (b37) pyridazinone fungicides such as diclomezine.

Embodiment B38. The composition described in the Summary of the Invention (including but not limited to the composition of any one of Embodiments 1 through 164 and A through Q) wherein component (b) includes at least one compound selected from (b38) thiophenecarboxamide fungicides such as silthiofam.

Embodiment B39. The composition described in the Summary of the Invention (including but not limited to the composition of any one of Embodiments 1 through 164 and A through Q) wherein component (b) includes at least one compound selected from (b39) complex I NADH oxidoreductase inhibitor fungicides such as diflumetorim, tolfenpyrad and fenazaquin.

Embodiment B40. The composition described in the Summary of the Invention (including but not limited to the composition of any one of Embodiments 1 through 164 and A through Q) wherein component (b) includes at least one compound selected from (b40) carboxylic acid amide fungicides such as dimethomorph, benthiavalicarb, benthiavalicarb-isopropyl, iprovalicarb, valifenalate, mandipropamid, flumorph, dimethomorph, flumorph, pyrimorph, benthiavalicarb, benthiavalicarb-isopropyl, iprovalicarb, tolprocarb, valifenalate and mandipropamid.

Embodiment B41. The composition described in the Summary of the Invention (including but not limited to the composition of any one of Embodiments 1 through 164 and A through Q) wherein component (b) includes at least one compound selected from (b41) tetracycline antibiotic fungicides such as oxytetracycline.

Embodiment B42. The composition described in the Summary of the Invention (including but not limited to the composition of any one of Embodiments 1 through 164 and A through Q) wherein component (b) includes at least one compound selected from (b42) thiocarbamate fungicides such as methasulfocarb.

Embodiment B43. The composition described in the Summary of the Invention (including but not limited to the composition of any one of Embodiments 1 through 164 and A through Q) wherein component (b) includes at least one compound selected from (b43) benzamide fungicides such as fluopicolide and fluopimomide.

Embodiment B44. The composition described in the Summary of the Invention (including but not limited to the composition of any one of Embodiments 1 through 164 and A through Q) wherein component (b) includes at least one compound selected from (b44) microbial fungicides such as *Bacillus amyloliquefaciens* strains QST713, FZB24, MB1600, D747, F727, TJ100 (also called strain 1 BE; known from EP2962568) and the fungicidal lipopeptides which they produce.

Embodiment B45. The composition described in the Summary of the Invention (including but not limited to the composition of any one of Embodiments 1 through 164 and A through Q) wherein component (b) includes at least

one compound selected from (b45) quinone outside inhibitor, stigmatellin binding fungicides such as ametoctradin.

Embodiment B46. The composition described in the Summary of the Invention (including but not limited to the composition of any one of Embodiments 1 through 164 and A through Q) wherein component (b) includes at least one compound selected from (b46) plant extract fungicides such as *Melaleuca alternifolia,* eugenol, geraniol and thymol.

Embodiment B47. The composition described in the Summary of the Invention (including but not limited to the composition of any one of Embodiments 1 through 164 and A through Q) wherein component (b) includes at least one compound selected from (b47) cyanoacrylate fungicides such as phenamacril.

Embodiment B48. The composition described in the Summary of the Invention (including but not limited to the composition of any one of Embodiments 1 through 164 and A through Q) wherein component (b) includes at least one compound selected from (b48) polyene fungicides such as natamycin.

Embodiment B49. The composition described in the Summary of the Invention (including but not limited to the composition of any one of Embodiments 1 through 164 and A through Q) wherein component (b) includes at least one compound selected from (b49) oxysterol binding protein inhibitor fungicides such as oxathiapiprolin and fluoxapiprolin.

Embodiment B50. The composition described in the Summary of the Invention (including but not limited to the composition of any one of Embodiments 1 through 164 and A through Q) wherein component (b) includes at least one compound selected from (b50) aryl-phenyl-ketone fungicides such as metrafenone and pyriofenone.

Embodiment B51. The composition described in the Summary of the Invention (including but not limited to the composition of any one of Embodiments 1 through 164 and A through Q) wherein component (b) includes at least one compound selected from (b51) host plant defense induction fungicides such as acibenzolar-S-methyl, probenazole, tiadinil, isotianil, laminarin, extract from *Reynoutria sachalinensis* and *Bacillus mycoides* isolate J and cell walls of *Saccharomyces cerevisiae* strain LAS117.

Embodiment B52. The composition described in the Summary of the Invention (including but not limited to the composition of any one of Embodiments 1 through 164 and A through Q) wherein component (b) includes at least one compound selected from (b52) multi-site activity fungicides such as copper oxychloride, copper sulfate, copper hydroxide (e.g., Kocide), Bordeaux composition (tribasic copper sulfide), elemental sulfur, ferbam, mancozeb, maneb, metiram, propineb, thiram, zinc thiazole, zineb, ziram, folpet, captan, captafol, chlorothalonil, dichlofluanid, tolyfluanid, guazatine, iminoctadine albesilate, iminoctadine triacetate, anilazine, dithianon, quinomethionate and fluoroimide.

Embodiment B53. The composition described in the Summary of the Invention (including but not limited to the composition of any one of Embodiments 1 through 164 and A through Q) wherein component (b) includes at least one compound selected from (b53) biological fungicides with multiple modes of action such as extract from the cotyledons of lupine plantlets.

Embodiment B54. The composition described in the Summary of the Invention (including but not limited to the composition of any one of Embodiments 1 through 164 and A through Q) wherein component (b) includes at least one compound selected from (b54) fungicides other than fungicides of component (a) and components (b1) through (b53), such as cyflufenamid, bethoxazin, neo-asozin, pyrrolnitrin, tebuffoquin, dodine, flutianil, ferimzone, picarbutrazox, dichlobentiazox (Registry Number 957144-77-3), dipymetitrone (Registry Number 16114-35-5), flometoquin, tolnifanide (Registry Number 304911-98-6), N-[4-[4-chloro-3-(trifluoromethyl)phenoxy]-2,5-dimethylphenyl]-*N*-ethyl-*N*-methylmethanimid-amide, 5-fluoro-2-[(4-fluorophenyl)methoxy]-4-pyrimidinamine and 4-fluorophenyl N-[1-[[[1-(4-cyanophenyl)ethyl]sulfonyl]methyl]propyl]carbamate (XR-539).

Embodiment B55. The composition described in the Summary of the Invention (including but not limited to the composition of any one of Embodiments 1 through 164 and A through Q) wherein component (b) includes (1*S*)-2,2-bis(4-fluorophenyl)-1-methylethyl *N*-[[3-(acetyloxy)-4-methoxy-2-pyridinyl]carbonyl]-L-alaninate (provisional common name florylpicoxamid).

Embodiment B56. The composition described in the Summary of the Invention (including but not limited to the composition of any one of Embodiments 1 through 164 and A through Q) wherein component (b) includes 1-[2-[[[1-(4-chlorophenyl)-1*H*-pyrazol-3-yl]oxy]methyl]-3-methylphenyl]-1,4-dihydro-4-methyl-5*H*-tetrazol-5-one (provisional common name metyltetraprole).

Embodiment B57. The composition described in the Summary of the Invention (including but not limited to the composition of any one of Embodiments 1 through 164 and A through Q) wherein component (b) includes 3-chloro-4-(2,6-difluorophenyl)-6-methyl-5-phenylpyridazine (provisional common name pyridachlometyl).

Embodiment B58. The composition described in the Summary of the Invention (including but not limited to the composition of any one of Embodiments 1 through 164 and A through Q) wherein component (b) includes (4-phenoxyphenyl)methyl 2-amino-6-methyl-pyridine-3-carboxylate (provisional common name aminopyrifen).

Embodiment B59. The composition described in the Summary of the Invention (including but not limited to the composition of any one of Embodiments 1 through 164 and A through Q) wherein component (b) includes at least

one compound selected from (b54.11) (i.e. Formula **b54.11**)

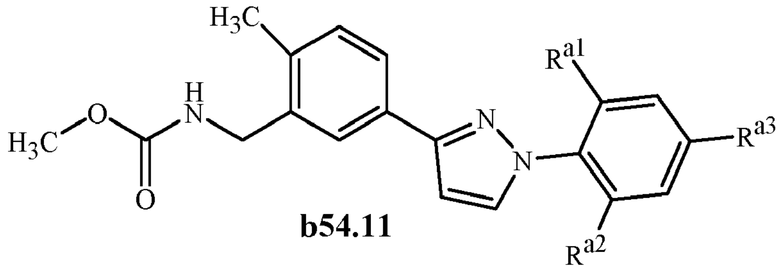

**b54.11**

wherein

$R^{a1}$ and $R^{a2}$ are each independently halogen; and
$R^{a3}$ is H, halogen, $C_1$-$C_3$ alkyl, $C_1$-$C_3$ haloalkyl or $C_3$-$C_6$ cycloalkyl.

Embodiment B60. The composition of Embodiment B59 wherein component (b) includes at least one fungicidal compound selected from the group consisting of methyl *N*-[[5-[1-[2,6-difluoro-4-(1-methylethyl)phenyl]-1*H*-pyrazol-3-yl]-2-methylphenyl]methyl]carbamate, methyl *N*-[[5-[1-(4-chloro-2,6-difluorophenyl)-1*H*-pyrazol-3-yl]-2-methylphenyl]methyl] carbamate, methyl *N*-[[5-[1-[2,6-difluoro-4-(trifluoromethyl)phenyl]-1*H*-pyrazol-3-yl]-2-methylphenyl]methyl]carbamate, methyl *N*-[[5-[1-(4-cyclopropyl-2,6-difluorophenyl)-1*H*-pyrazol-3-yl]-2-methylphenyl]methyl]carbamate and methyl *N*-[[5-[1-(2,6-dichloro-4-cyclopropylphenyl)-1*H*-pyrazol-3-yl]-2-methylphenyl]methyl]carbamate.

Embodiment B60a. The composition of Embodiment B60 wherein component (b) includes at least one fungicidal compound selected from the group consisting of methyl *N*-[[5-[1-[2,6-difluoro-4-(1-methylethyl)phenyl]-1H pyrazol-3-yl]-2-methylphenyl]methyl]carbamate, methyl *N*-[[5-[1-(4-chloro-2,6-difluorophenyl)-1*H*-pyrazol-3-yl]-2-methylphenyl]methyl] carbamate, methyl *N*-[[5-[1-[2,6-difluoro-4-(trifluoromethyl)phenyl]-1*H*-pyrazol-3-yl]-2-methylphenyl]methyl]carbamate and methyl *N*-[[5-[1-(4-cyclopropyl-2,6-difluorophenyl)-1*H*-pyrazol-3-yl]-2-methylphenyl]methyl]carbamate.

Embodiment B60b. The composition of Embodiment B60a wherein component (b) includes methyl *N*-[[5-[1-[2,6-difluoro-4-(1-methylethyl)phenyl]-1*H*-pyrazol-3-yl]-2-methylphenyl] methyl]carbamate.

Embodiment B61. The composition described in the Summary of the Invention (including but not limited to the composition of any one of Embodiments 1 through 164 and A through Q) wherein component (b) includes at least one compound selected from (b54.12) (i.e. Formula **b54.12**)

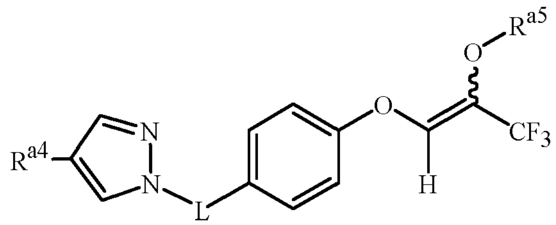

**b54.12**

wherein

$R^{a1}$and $R^{a2}$ are each independently halogen; and
$R^{a3}$ is H, halogen, $C_1$-$C_3$ alkyl, $C_1$-$C_3$ haloalkyl or $C_3$-$C_6$ cycloalkyl.

Embodiment B62. The composition of Embodiment B61 wherein component (b) includes at least one fungicidal compound selected from the group consisting of ethyl 1-[[4-[[(1*Z*)-2-ethoxy-3,3,3-trifluoro-1-propen-1-yl]oxy]phenyl]methyl]-1*H*-pyrazole-4-carboxylate and its (E)-isomer and (b54.12b) ethyl 1-[2-[4-[[(1*Z*)-2-ethoxy-3,3,3-trifluoro-1-propen-1-yl]oxy]phenyl]ethyl]-1*H*-pyrazole-4-carboxylate and its (*E)*-isomer.

Embodiment B62a. The composition of Embodiment B62 wherein component (b) includes ethyl 1-[[4-[[(1*Z*)-2-ethoxy-3,3,3-trifluoro-1-propen-1-yl]oxy]phenyl]methyl]-1*H*-pyrazole-4-carboxylate.

Embodiment B63. The composition described in the Summary of the Invention (including but not limited to the composition of any one of Embodiments 1 through 164 and A through Q) wherein component (b) includes at least one compound selected from (b54.13) (i.e. Formula **b54.13**)

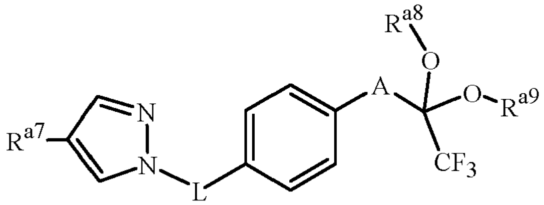

**b54.13**

wherein

$R^{a7}$ is $C_2$-$C_5$ alkoxycarbonyl or $C_3$-$C_5$ alkenyloxycarbonyl, each optionally substituted with up to 3 substituents independently selected from $R^{a10}$;
L is $CH_2$, $CH_2CH_2$ or $CH_2O$, wherein O is connected to the phenyl ring;
$R^{a8}$ and $R^{9b}$ are each independently H, $C_1$-$C_4$ alkyl; or
$R^{a8}$ and $R^{a9}$ are taken together with the oxygen atoms to which they are attached to form a 5-membered saturated ring containing ring members, in addition to the oxygen atoms, selected from carbon atoms, the ring optionally substituted with up to 2 substituents independently selected from halogen, cyano, $C_1$-$C_2$ alkyl, $C_1$-$C_2$ haloalkyl and $C_1$-$C_2$ alkoxy on carbon atom ring members; and
$R^{a10}$ is cyano, halogen, cyclopropyl or methoxy.

Embodiment B64. The composition of Embodiment B63 wherein component (b) includes at least one fungicidal compound selected from the group consisting of ethyl 1-[[4-[[2-(trifluoromethyl)-1,3-dioxolan-2-yl]methoxy]phenyl]methyl]-1*H*-pyrazole-4-carboxylate, 2-methylpropyl 1-[[4-[[2-(trifluoromethyl)-1,3-dioxolan-2-yl]methoxy]phenyl]methyl]-1*H*-pyrazole-4-carboxylate, 2-butyn-1-yl 1-[[4-[[2-(trifluoromethyl)-1,3-dioxolan-2-yl]methoxy]phenyl]methyl]-1*H*-pyrazole-4-carboxylate and ethyl 1-[[3-fluoro-4-[[2-(trifluoromethyl)-1,3-dioxolan-2-yl]methoxy]phenyl]methyl]-1H pyrazole-4-carboxylate.
Embodiment B64a. The composition of Embodiment B64 wherein component (b) includes ethyl 1-[[4-[[2-(trifluoromethyl)-1,3-dioxolan-2-yl]methoxy]phenyl]methyl]-1*H*-pyrazole-4-carboxylate.
Embodiment B65. The composition described in the Summary of the Invention (including but not limited to the composition of any one of Embodiments 1 through 164 and A through Q) wherein component (b) includes at least one compound selected from (b54.14) (i.e. Formula **b54.14**)

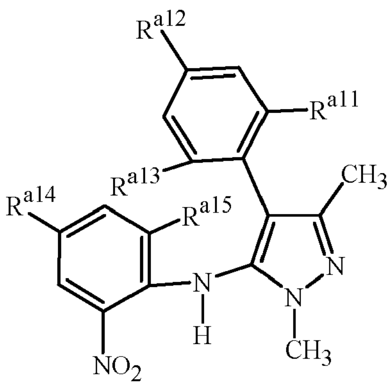

**b54.14**

wherein

$R^{b11}$, $R^{b12}$ and $R^{b13}$ are each independently H, halogen or cyano; and
$R^{b14}$ and $R^{b15}$ are each independently H, halogen, $C_1$-$C_3$ alkyl or $C_1$-$C_3$ methoxy. Embodiment B66. The composition of Embodiment B65 wherein component (b) includes at least one fungicidal compound selected

from the group consisting of 4-(2-chloro-4-fluorophenyl)-*N*-(2-fluoro-4-methyl-6-nitrophenyl)-1,3-dimethyl-1*H*-pyrazol-5-amine, 4-(2-chloro-4-fluorophenyl)-N-(2-fluoro-6-nitrophenyl)-1,3-dimethyl-1*H*-pyrazol-5-amine, 3,5-difluoro-4-[5-[(4-methoxy-2-nitrophenyl)amino]-1,3-dimethyl-1*H*-pyrazol-4-yl]-benzonitrile, N-(2-chloro-4-fluoro-6-nitrophenyl)-4-(2-chloro-4-fluorophenyl)-1,3-dimethyl-1H-pyrazol-5-amine, 4-(2-chloro-4,6-difluorophenyl)-1,3-dimethyl-N-(2-nitrophenyl)-1*H*-pyrazol-5-amine and 4-(2-chloro-4,6-difluorophenyl)-1,3-dimethyl-N-(4-methyl-2-nitrophenyl)-1*H*-pyrazol-5-amine.

Embodiment B66a. The composition of Embodiment B66 wherein component (b) includes at least one fungicidal compound selected from the group consisting of 4-(2-chloro-4-fluorophenyl)-*N*-(2-fluoro-4-methyl-6-nitrophenyl)-1,3-dimethyl-1*H*-pyrazol-5-amine, 4-(2-chloro-4-fluorophenyl)-N-(2-fluoro-6-nitrophenyl)-1,3-dimethyl-IH-pyrazol-5-amine, 4-(2-chloro-4,6-difluorophenyl)-1,3-dimethyl-*N*-(2-nitrophenyl)-1*H*-pyrazol-5-amine and 4-(2-chloro-4,6-difluorophenyl)-1,3-dimethyl-*N*-(4-methyl-2-nitrophenyl)-1*H*-pyrazol-5-amine.

Embodiment B67. The composition described in the Summary of the Invention (including but not limited to the composition of any one of Embodiments 1 through 164 and A through Q) wherein component (b) includes at least one fungicidal compound (fungicide) selected from the group consisting of azoxystrobin, benzovindiflupyr, boscalid (nicobifen), bixafen, bromuconazole, carbendazim, chlorothalonil, cyflufenamid, cyproconazole, difenoconazole, dimoxystrobin, epoxiconazole, famoxadone, fenbuconazole, fenpropidin, fenpropimorph, fluindapyr, flusilazole, flutriafol, fluxapyroxad, hexaconazole, ipconazole kresoxim-methyl, manzate, metconazole, metominostrobin, metrafenone, myclobutanil, penconazole, penthiopyrad, picoxystrobin, prochloraz, propiconazole, proquinazid, prothioconazole, pydiflumetofen, pyraclostrobin, pyrametostrobin, pyraoxystrobin, pyriofenone quinoxyfen, tebuconazole, trifloxystrobin, triticonazole, methyl *N*-[[5-[1-(2,6-dichloro-4-cyclopropylphenyl)-1*H*-pyrazol-3-yl]-2-methylphenyl]methyl]carbamate, methyl N-[[5-[1-(4-chloro-2,6-difluorophenyl)-1H-pyrazol-3-yl]-2-methylphenyl]methyl]carbamate and methyl *N*-[[5-[1-(4-cyclopropyl-2,6-difluorophenyl)-1*H*-pyrazol-3-yl]-2-methylphenyl]methyl]carbamate.

Embodiment B68. The composition of Embodiment B67 wherein component (b) includes at least one compound selected from the group consisting of azoxystrobin, benzovindiflupyr, bixafen, chlorothalonil cyflufenamid, cyproconazole, difenoconazole, dimoxystrobin, epoxiconazole, famoxadone, fenpropidin, fenpropimorph, fluindapyr, flusilazole, flutriafol, fluxapyroxad, kresoxim-methyl, manzate, metconazole, metominostrobin, metrafenone, myclobutanil, penthiopyrad, picoxystrobin, propiconazole, proquinazid, prothioconazole, pydiflumetofen, pyraclostrobin, pyrametostrobin, pyraoxystrobin, pyriofenone, quinoxyfen, tebuconazole, trifloxystrobin, triticonazole, methyl *N*-[[5-[1-(2,6-dichloro-4-cyclopropylphenyl)-1*H*-pyrazol-3-yl]-2-methylphenyl]methyl]carbamate, methyl N-[[5-[1-(4-chloro-2,6-difluorophenyl)-1H-pyrazol-3-yl]-2-methylphenyl]methyl]carbamate and methyl *N*-[[5-[1-(4-cyclopropyl-2,6-difluorophenyl)-1*H*-pyrazol-3-yl]-2-methylphenyl]methyl]carbamate.

Embodiment B69. The composition of Embodiment B68 wherein component (b) includes at least one compound selected from the group consisting of azoxystrobin, benzovindiflupyr, bixafen, chlorothalonil, cyproconazole, difenoconazole, epoxiconazole, fenpropimorph, fluindapyr, flutriafol, fluxapyroxad, mancozeb, picoxystrobin, prothioconazole, pydiflumetofen, pyraclostrobin, tebuconazole, trifloxystrobin, methyl *N*-[[5-[1-(2,6-dichloro-4-cyclopropylphenyl)-1*H*-pyrazol-3-yl]-2-methylphenyl]methyl]carbamate, methyl *N*-[[5-[1-(4-chloro-2,6-difluorophenyl)-1H-pyrazol-3-yl]-2-methyl-phenyl]methyl]carbamate and methyl *N*-[[5-[1-(4-cyclopropyl-2,6-difluorophenyl)-1*H*-pyrazol-3-yl]-2-methylphenyl]methyl]carbamate.

Embodiment B70. The composition of Embodiment B69 wherein component (b) includes at least one compound selected from the group consisting of azoxystrobin, benzovindiflupyr, bixafen, chlorothalonil, cyproconazole, difenoconazole, epoxiconazole, fenpropimorph, fluindapyr, flutriafol, fluxapyroxad, manzate, picoxystrobin, prothioconazole, pydiflumetofen, pyraclostrobin, tebuconazole and trifloxystrobin.

Embodiment B71. The composition of Embodiment B70 wherein component (b) includes at least one compound selected from the group consisting of azoxystrobin, benzovindiflupyr, bixafen, chlorothalonil, cyproconazole, epoxiconazole, fenpropimorph, fluindapyr, fluxapyroxad, manzate, picoxystrobin, prothioconazole, pydiflumetofen, tebuconazole and trifloxystrobin.

Embodiment B72. The composition described in the Summary of the Invention (including but not limited to the composition of any one of Embodiments 1 through 164 and A through Q) wherein component (b) includes at least one fungicidal compound (fungicide) selected from the group consisting of azoxystrobin, benzovindiflupyr, boscalid (nicobifen), bixafen, bromuconazole, carbendazim, chlorothalonil, cyflufenamid, cyproconazole, difenoconazole, dimoxystrobin, epoxiconazole, famoxadone, fenbuconazole, fenpropidin, fenpropimorph, fluindapyr, flusilazole, flutriafol, fluxapyroxad, hexaconazole, ipconazole kresoxim-methyl, manzate, metconazole, metominostrobin, metrafenone, myclobutanil, penconazole, penthiopyrad, picoxystrobin, prochloraz, propiconazole, proquinazid, prothioconazole, pydiflumetofen, pyraclostrobin, pyrametostrobin, pyraoxystrobin, pyriofenone quinoxyfen, tebuconazole, trifloxystrobin, triticonazole, methyl N-[[S-[1-[2,6-difluoro-4-(1-methylethyl) phenyl]-1*H*-pyrazol-3-yl]-2-methylphenyl]methyl]carbamate, ethyl 1-[[4-[[(1*Z*)-2-ethoxy-3,3,3-trifluoro-1-propen-1-yl]oxy]phenyl]methyl]-1*H*-pyrazole-4-carboxylate, ethyl 1-[[4-[[2-(trifluoromethyl)-1,3-dioxolan-2-yl]methoxy]phenyl]methyl]-1H pyrazole-4-carboxylate,

4-(2-chloro-4-fluorophenyl)-N-(2-fluoro-4-methyl-6-nitrophenyl)-1,3-dimethyl-1*H*-pyrazol-5-amine, 4-(2-chloro-4-fluorophenyl)-N-(2-fluoro-6-nitrophenyl)-1,3-dimethyl-1*H*-pyrazol-5-amine, 4-(2-chloro-4,6-difluorophenyl)-1,3-dimethyl-N-(2-nitrophenyl)-1*H*-pyrazol-5-amine and 4-(2-chloro-4,6-difluorophenyl)-1,3-dimethyl-N-(4-methyl-2-nitrophenyl)-1H pyrazol-5-amine.

Embodiment B73. The composition of Embodiment B72 wherein component (b) includes at least one compound selected from the group consisting of azoxystrobin, benzovindiflupyr, bixafen, chlorothalonil cyflufenamid, cyproconazole, difenoconazole, dimoxystrobin, epoxiconazole, famoxadone, fenpropidin, fenpropimorph, fluindapyr, flusilazole, flutriafol, fluxapyroxad, kresoxim-methyl, manzate, metconazole, metominostrobin, metrafenone, myclobutanil, penthiopyrad, picoxystrobin, propiconazole, proquinazid, prothioconazole, pydiflumetofen, pyraclostrobin, pyrametostrobin, pyraoxystrobin, pyriofenone, quinoxyfen, tebuconazole, trifloxystrobin, triticonazole, methyl N-[[S-[1-[2,6-difluoro-4-(1-methylethyl) phenyl]-1*H*-pyrazol-3-yl]-2-methylphenyl]methyl]carbamate, ethyl 1-[[4-[[(1*Z*)-2-ethoxy-3,3,3-trifluoro-1-propen-1-yl]oxy]phenyl]methyl]-1*H*-pyrazole-4-carboxylate, ethyl 1-[[4-[[2-(trifluoromethyl)-1,3-dioxolan-2-yl]methoxy]phenyl]methyl]-1H pyrazole-4-carboxylate, 4-(2-chloro-4-fluorophenyl)-N-(2-fluoro-4-methyl-6-nitrophenyl)-1,3-dimethyl-1*H*-pyrazol-5-amine, 4-(2-chloro-4-fluorophenyl)-*N*-(2-fluoro-6-nitrophenyl)-1,3-dimethyl-1*H*-pyrazol-5-amine, 4-(2-chloro-4,6-difluorophenyl)-1,3-dimethyl-*N*-(2-nitrophenyl)-IH-pyrazol-5-amine and 4-(2-chloro-4,6-difluorophenyl)-1,3-dimethyl-N (4-methyl-2-nitrophenyl)-1*H*-pyrazol-5-amine.

Embodiment B74. The composition of Embodiment B73 wherein component (b) includes at least one compound selected from the group consisting of azoxystrobin, benzovindiflupyr, bixafen, chlorothalonil, cyproconazole, difenoconazole, epoxiconazole, fenpropimorph, fluindapyr, flutriafol, fluxapyroxad, mancozeb, picoxystrobin, prothioconazole, pydiflumetofen, pyraclostrobin, tebuconazole, trifloxystrobin, methyl *N*-[[5-[1-[2,6-difluoro-4-(1-methylethyl)phenyl]-1H pyrazol-3-yl]-2-methylphenyl]methyl]carbamate, ethyl 1-[[4-[[(1*Z*)-2-ethoxy-3,3,3-trifluoro-1-propen-1-yl]oxy]phenyl]methyl]-1*H*-pyrazole-4-carboxylate, ethyl 1-[[4-[[2-(trifluoromethyl)-1,3-dioxolan-2-yl]methoxy]phenyl]methyl]-1*H*-pyrazole-4-carboxylate, 4-(2-chloro-4-fluorophenyl)-*N*-(2-fluoro-4-methyl-6-nitrophenyl)-1,3-dimethyl-1*H*-pyrazol-5-amine, 4-(2-chloro-4-fluorophenyl)-N (2-fluoro-6-nitrophenyl)-1,3-dimethyl-1*H*-pyrazol-5-amine, 4-(2-chloro-4,6-difluorophenyl)-1,3-dimethyl-*N*-(2-nitrophenyl)-1*H*-pyrazol-5-amine and 4-(2-chloro-4,6-difluorophenyl)-1,3-dimethyl-*N*-(4-methyl-2-nitrophenyl)-1*H*-pyrazol-5-amine.

Of note is the composition of any one of the embodiments described herein, including any Embodiments 1 through 164, A through Q, and B1 through B74, wherein reference to Formula 1 includes salts thereof but not A-oxides thereof; therefore the phrase "a compound of Formula 1" can be replaced by the phrase "a compound of Formula 1 or a salt thereof. In this composition of note, component (a) comprises a compound of Formula 1 or a salt thereof.

Also noteworthy as embodiments are fungicidal compositions of the present invention comprising a fungicidally effective amount of a composition of Embodiments 1 through 164, A through Q, and B1 through B74, and at least one additional component selected from the group consisting of surfactants, solid diluents and liquid diluents.

Embodiments of the invention further include methods for controlling plant diseases caused by fungal plant pathogens comprising applying to the plant or portion thereof, or to the plant seed or seedling, a fungicidally effective amount of a composition of Embodiments 1 through 164, A through Q, and B1 through B74 (e.g., as a composition including formulation ingredients as described herein). Embodiments of the invention also include methods for protecting a plant or plant seed from diseases caused by fungal pathogens comprising applying a fungicidally effective amount of a composition of any one of Embodiments 1 through 164, A through Q, and B1 through B65 to the plant or plant seed.

Some embodiments of the invention involve control of a plant disease or protection from a plant disease that primarily afflicts plant foliage and/or applying the composition of the invention to plant foliage (i.e. plants instead of seeds). The preferred methods of use include those involving the above preferred compositions; and the diseases controlled with particular effectiveness include plant diseases caused by fungal plant pathogens. Combinations of fungicides used in accordance with this invention can facilitate disease control and retard resistance development.

Method embodiments further include:

Embodiment C1. A method for protecting a plant from a disease selected from rust, powdery mildew and *Septoria* diseases comprising applying to the plant a fungicidally effective amount of the composition comprising components (a) and (b) described in the Summary of the Invention or any one of Embodiments 1 through 164.

Embodiment C2. The method of Embodiment C1 wherein the disease is a rust disease and component (b) of the composition includes at least one fungicidal compound selected from (b3) demethylation inhibitor (DMI) fungicides, (b5) amine/morpholine fungicides, (b7) succinate dehydrogenase inhibitor fungicides, (b11) quinone outside inhibitor (QoI) fungicides, (b13) methyl benzimidazole carbamate fungicides and (b52) multi-site activity fungicides.

Embodiment C3. The method of Embodiment C2 wherein component (b) of the composition includes at least one fungicidal compound selected from (b3) demethylation inhibitor (DMI) fungicides, (b5) amine/morpholine fungicides, (b7) succinate dehydrogenase inhibitor fungicides, (b11) quinone outside inhibitor (QoI) fungicides and (b52) multi-site activity fungicides.

Embodiment C4. The method of Embodiment C3 wherein component (b) of the composition includes at least one fungicidal compound selected from the group consisting of azoxystrobin, benzovindiflupyr, bixafen, chlorothalonil, cyproconazole, epoxiconazole, fenpropimorph, fluindapyr, fluxapyroxad, mancozeb, picoxystrobin, prothioconazole, pydiflumetofen, tebuconazole and trifloxystrobin.

Embodiment C5. The method of Embodiment C4 wherein component (b) of the composition includes at least one fungicidal compound selected from the group consisting of azoxystrobin, cyproconazole, epoxiconazole, fluindapyr, fluxapyroxad, picoxystrobin, prothioconazole, pydiflumetofen, tebuconazole and trifloxystrobin.

Embodiment C6. The method of any one of Embodiments C2 through C5 wherein the disease is Asian soybean rust caused by *Puccinia recondite.*

Embodiment C7. The method of any one of Embodiments C2 through C5 wherein the disease is wheat leaf rust caused by *Phakopsora pachyrhizi.*

Embodiment C8. The method of Embodiment C1 wherein the disease is a powdery mildew disease and component (b) of the composition includes at least one fungicidal compound selected from (b3) demethylation inhibitor (DMI) fungicides, (b11) quinine outside inhibitor (QoI) fungicides and (b13) azanaphthalene fungicides.

Embodiment C9. The method of Embodiment C8 wherein the disease is wheat powdery mildew.

Embodiment C10. The method of Embodiment C8 wherein the disease is grape downy mildew.

Embodiment C11. The method of any one of Embodiments C8 through C10 wherein component (b) includes at least one fungicidal compound selected from (b3) DMI fungicides.

Embodiment C12. The method of Embodiment C11 wherein component (b) includes at least one fungicidal compound selected from the group consisting of cyproconazole, difenoconazole, epoxiconazole, prothioconazole and tebuconazole.

Embodiment C13. The method of Embodiment C12 wherein component (b) includes at least one fungicidal compound selected from the group consisting of cyproconazole, difenoconazole and prothioconazole.

Embodiment C14. The method of any one of Embodiments C8 through C10 wherein component (b) includes at least one fungicidal compound selected from (b11) QoI fungicides.

Embodiment C15. The method of Embodiment C14 wherein component (b) includes at least one fungicidal compound selected from the group consisting of azoxystrobin, picoxystrobin and pyraclostrobin.

Embodiment C16. The method of Embodiment C1 wherein the disease is a *Septoria* disease and component (b) of the composition includes at least one fungicidal compound selected from the group consisting of epoxiconazole, metalaxyl (including metalaxyl-M), iprovalicarb and fenpropimorph.

Embodiment C17. The method of Embodiment C16 wherein the disease is wheat leaf blotch.

Embodiment C18. The method of any one of Embodiments C1 through C17 wherein components (a) and (b) are applied in synergistically effective amounts (and in a synergistic ratio relative to each other).

Of note are embodiments that are counterparts of Embodiments C1 through C18 relating to a method for controlling plant diseases caused by fungal plant pathogens comprising applying to the plant or portion thereof, a fungicidally effective amount of a fungicidal composition of the invention.

As noted in the Summary of the Invention, this invention also relates to a compound of Formula 1, or an A-oxide or salt thereof. Also already noted is that the embodiments of this invention, including Embodiments 1-164, relate also to compounds of Formula 1. Of note is a compound selected from the group consisting of:

3-[4-[(1-cyanomethyl-1*H*-pyrazol-3-yl)methyl]phenyl]-5-(trifluoromethyl)-1,2,4-oxadiazole (Compound 507);

*N*-ethyl-1-[[[4-[5-(trifluoromethyl)-1,2,4-oxadiazol-3-yl]phenyl]methoxy]methyl]-1*H*-pyrazole-4-carboxamide (Compound 508);

1-methyl-*N*-(2,2,2-trifluoroethyl)-5-[[4-[5-(trifluoromethyl)-1,2,4-oxadiazol-3-yl]phenyl]methyl]-1*H*-pyrazole-3-carboxamide (Compound 509); and

5-[[4-[5-(trifluoromethyl)-1,2,4-oxadiazol-3-yl]phenyl]methyl]-3-isoxazoleacetonitrile (Compound 510).

One or more of the following methods and variations as described in Schemes 1-13 can be used to prepare the compounds of Formula **1**. The definitions of $R^1$, $R^2$, L and J in the compounds of Formulae **1-21** below are as defined above in the Summary of the Invention unless otherwise noted. Compounds of Formulae **1a**, **1b**, **1c**, **3a**, **6a** and **6b** are various subsets of Formula **1**, and all substituents for Formulae **1a**, **1b**, **1c**, **3a**, **6a** and **6b** are as defined above for Formula **1** unless otherwise noted.

As shown in Scheme 1, compounds of Formula **1** can be prepared by reaction of an amide oxime of Formula **2** with trifluoroacetic anhydride (TFAA) or an equivalent. The reaction can be carried out without solvent other than the

compounds of Formula **2** and TFAA. Typically the reaction is conducted in a liquid phase with a solvent such as tetrahydrofuran, acetonitrile *N*,*N*-dimethylformamide or toluene at a temperature between about 0 to 100 °C, optionally in the presence of a base such as pyridine, A,A-diisopropylethylamine or trimethylamine. Preparation of oxadiazole rings by this method and others are known in the art; see, for example, Comprehensive Heterocyclic Chemistry, Vol. 6, Part 4B, pages 365-391, Kevin T. Potts editor, Pergamon Press, New York, 1984. The method of Scheme 1 is also illustrated in present Example 10, Step C and Example 11, Step A (second paragraph).

Scheme 1

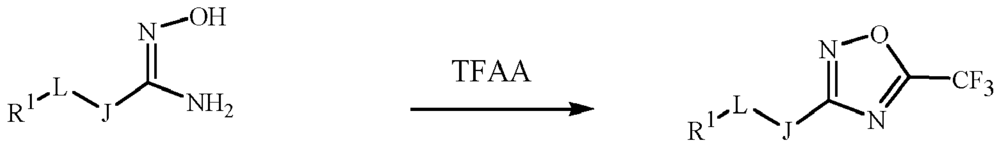


2 1

As shown in Scheme 2, oximes of Formula **2** can be prepared from corresponding nitriles of Formula **3** and hydroxylamine or a hydroxylamine salt (e.g., hydroxylamine hydrochloride) in a solvent such as ethanol, methanol or *N*,*N*-dimethylformamide at temperatures generally ranging from about 0 to 80 °C. The hydroxylamine may be used in the form of a solution in water; alternatively, the hydroxylamine can be generated *in situ* by treating an acid salt of hydroxylamine with a base such as an alkali metal hydroxide or carbonate, preferably sodium hydroxide or sodium carbonate. Hydroxylamine salts include salts which hydroxylamine forms with inorganic acids such as sulfuric acid, hydrochloric acid, nitric acid and phosphoric acid or with organic acids such as formic acid, acetic acid, propionic acid and sulfonic acids. For reaction conditions see present Example 10, Step B and Example 11, Step A (first paragraph).

Scheme 2

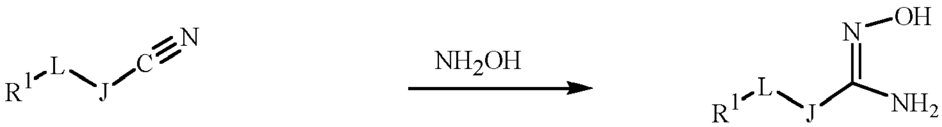


3 2

Compounds of Formula **1** wherein L is $(CR^{4a}R^{4b})_n$, and the like, and $R^1$ is a heterocyclic ring or ring system, such as pyrazole, indazole, imidazole, pyrrole and triazole, linked to L via a nitrogen atom, can be prepared by displacement of an appropriate leaving group $X^1$ of compounds of Formula **4** with nitrogen-containing heterocycles of Formula **5** in the presence of a base as depicted in Scheme 3. Suitable bases include inorganic bases such as alkali or alkaline earth metal (e.g., lithium, sodium, potassium and cesium) hydrides, alkoxides, carbonates, phosphates and hydroxides. A variety of solvents are suitable for the reaction including, for example, N,N-dimethylformamide, *N*,*N*-dimethylacetamide, *N*-methylpyrrolidinone, acetonitrile and acetone. Particularly useful reaction conditions include using cesium carbonate or potassium carbonate and *N*,*N*-dimethylformamide or acetonitrile as the solvent at temperatures ranging between about 0 to 80 °C. Suitable leaving groups in the compounds of Formula 4 include bromide, chlorine, iodide, mesylate ($OS(O)_2CH_3$), triflate ($OS(O)_2CF_3$), and the like. The method of Scheme 3 is illustrated in present Example 1, Step C; Examples 2-9; and Example 11, Step C.

Scheme 3

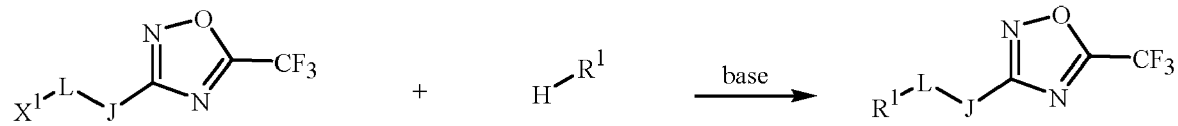


**4**
$X^1$ is a leaving group such as Br, Cl, I, $OS(O)_2Me$ or $OS(O)_2CF_3$ and L is $(CR^{4a}R^{4b})_n$, and the like

**5**
$R^1$ is a nitrogen containing heterocycle unsubstituted on N, e.g., 1*H*-pyrazole

**1**
$R^1$ is a heterocycle linked to L via a nitrogen atom and L is $(CR^{4a}R^{4b})_n$, and the like

Compounds of Formula **4** can be prepared by conversion of the corresponding alcohols of Formula **6** to an appropriate leaving group (i.e. $X^1$) as shown in Scheme 4. For example, alcohols of Formula **6** can be converted to alkyl chlorides of Formula **4** by treatment with thionyl chloride, oxalyl chloride or phosphorus trichloride (for conditions, see Example 1, Step B). Alkyl bromides can be prepared in a similar reaction using phosphorus tribromide or phosphorus oxybromide. Sulfonates can be prepared by reaction of Formula **6** with a sulfonating agent such as methanesulfonyl chloride, typically in the presence of a base, under conditions well known to one skilled in the art of organic synthesis.

Scheme 4

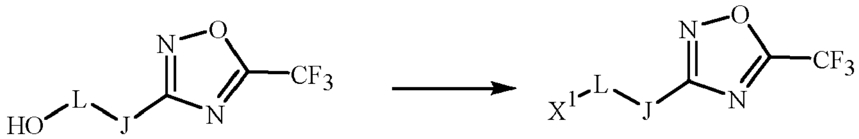

**6**
L is $(CR^{4a}R^{4b})_n$, and the like

**4**
$X^1$ is a leaving group such as Br, Cl, I, $OS(O)_2Me$ or $OS(O)_2CF_3$ and L is $(CR^{4a}R^{4b})_n$, and the like

Alternatively, compounds of Formula 1 wherein $R^1$ is a nitrogen-linked heterocycle can be prepared by reaction of primary or secondary alcohols of Formula **6** with nitrogen-containing heterocycles of Formula **5** using Mitsunobu coupling reaction conditions as shown in Scheme 5. Mitsunobu reactions are typically run in tetrahydrofuran with triphenylphosphine and diisopropyl azodicarboxylate (DIAD) or diethyl azodicarboxylate (DEAD) at room temperature. Polymer supported triphenylphosphine can be used to ease purification. For a review of the Mitsunobu reaction, see Mitsunobu, O. Comprehensive Organic Synthesis; Trost, B. M., Fleming, I., Eds.; Pergamon: Oxford, 1991; Vol. 6, pages 65-101. Also, Step C of Example 12 illustrates the preparation of a compound of Formula 1 using Mitsunobu conditions.

Scheme 5

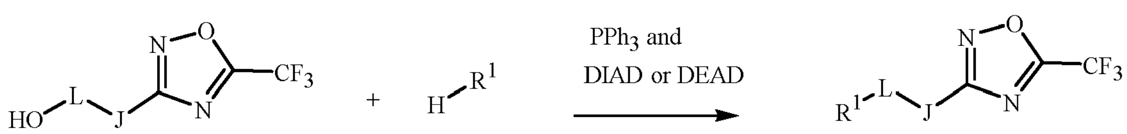


**6**
L is $(CR^{4a}R^{4b})_n$, and the like

**5**
$R^1$ is a nitrogen containing heterocycle unsubstituted on N, e.g., 1*H*-pyrazole

**1**
$R^1$ is a heterocycle linked to L via nitrogen atom

Compounds of Formula **6** can be prepared by a number of known methods including, for example, treating corresponding nitriles with hydroxylamine or a hydroxylamine salt followed by TFAA, analogous to the methods described in Schemes 1 and 2, and as illustrated in present Example 1, Step A. Additionally, secondary alcohols of Formula **6** can be prepared by oxidation of the corresponding alcohol to the aldehyde, and then reaction of the aldehyde with a Grignard reagent. The oxidation reaction can be performed by a variety of means, such as by treatment of the alcohol of Formula

**6** with manganese dioxide, Dess-Martin periodinane, pyridinium chlorochromate or pyridinium dichromate. For example, as shown in Scheme 6, compounds of Formula **6b** (i.e. Formula **6** wherein L in CHMe) can be synthesized by conversion of the alcohol of Formula **6a** (i.e. Formula **6** wherein L is $CH_2$) to the aldehyde of Formula **7**, and then treatment with methylmagnesium bromide. The method of Scheme 6 is illustrated in Example 12, Steps A-B.

Scheme 6

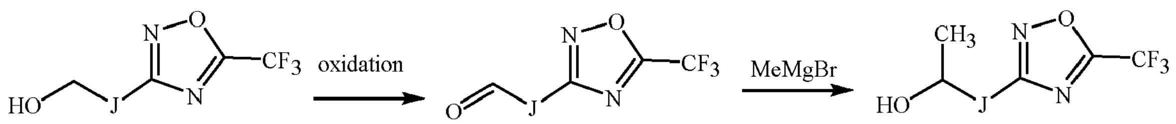


**6a** **7** **6b**

Compounds of Formula **1** can also be prepared by reaction of suitably functionalized compounds of Formula **8** with suitably functionalized compounds of Formula **9** as shown in Scheme 7. The functional groups $Y^1$ and $Y^2$ are selected from, but not limited to, moieties such as aldehydes, ketones, esters, acids, amides, thioamides, nitriles, amines, alcohols, thiols, hydrazines, oximes, amidines, amide oximes, olefins, acetylenes, halides, alkyl halides, methanesulfonates, trifluoromethanesulfonates (triflate), boronic acids, boronates, and the like, which under the appropriate reaction conditions, will allow for the construction of the various $R^1$ rings. As an example, reaction of a compound of Formula **8** where $Y^1$ is a chlorooxime moiety with a compound of Formula **9** where $Y^2$ is a vinyl or acetylene group in the presence of a base will give a compound of Formula **1** where $R^1$ is an isoxazoline or isoxazole, respectively. Present Example 13, Step C illustrates the preparation of a compound of Formula **1** wherein $R^1$ is isoxazoline. The synthetic literature describes many general methods for forming heterocyclic rings and ring systems (e.g., such as those shown in U-1 through U-118); see, for example, Comprehensive Heterocyclic Chemistry, Volumes 4-6, A. R. Katritzky and C. W. Rees editors-in-chief, Pergamon Press, Oxford, 1984; Comprehensive Heterocyclic Chemistry II, Volumes 2-4, A. R. Katritzky, C. W. Rees and E. F. V. Scriven editors-in-chief, Pergamon Press, Oxford, 1996; and the series, The Chemistry of Heterocyclic Compounds, E. C. Taylor, editor, Wiley, New York. One skilled in the art knows how to select the appropriate functional groups $Y^1$ and $Y^2$ to construct the desired $R^1$ ring. Compounds of Formula **8** are known or can be prepared by general methods known in the art. Compounds of Formula **9** can be prepared by treating corresponding nitriles with hydroxylamine or a hydroxylamine salt followed by TFAA analogous to the reactions described in Schemes 1 and 2, and as illustrated in present Example 13, Steps A-B.

Scheme 7

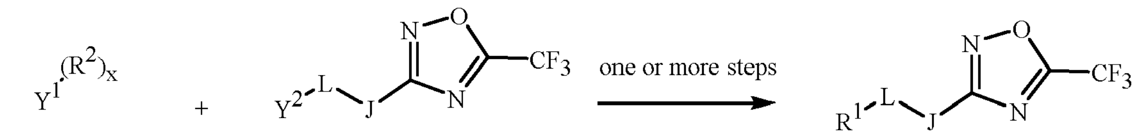


**8** **9** **1**

wherein x is 0-3

wherein $Y^1$ and $Y^2$ are suitable functional groups capable of undergoing transformation to form the desired $R^1$ ring

Compounds of Formula **1a** (i.e. Formula **1** wherein $R^1$ is oxazoline) can also be prepared as outlined in Scheme 8. In this method a compound of Formula **10** is contacted with an amine of Formula **11** in the presence of *O*-(7-azabenzotriazol-1-yl)-*N*,*N*,*N'*,*N'*-tetramethyluronium hexafluorophosphate (HATU) or 2-chloro-1-methylpyridinium iodide and a base such as triethylamine, *N*,*N*-diisopropylethylamine or 4-methylmorpholine at a temperature ranging from 0 to 100 °C to provide an amide of Formula **12**. In a subsequent step, the amide of Formula **12** is dehydrated using a dehydrating agent such as diethylaminosulfur trifluoride (DAST) in a suitable solvent. The reaction is typically carried out by adding 0.9 to 2 equivalents, preferably 1.5 equivalents, of diethylaminosulfur trifluoride to a mixture of a compound of Formula **12** in a solvent such as dichloromethane, at a temperature ranging from - 78 to 0 °C. The method of Scheme 8 is illustrated by Steps B-C of Example 15. Compounds of Formula **11** are commercially available or their preparation is known in the art. Compounds of Formula **10** can be prepared by treating corresponding nitriles with hydroxylamine or a hydroxylamine salt followed by TFAA, analogous to the reactions described in Schemes 1 and 2 and illustrated in Example 15, Step A.

Scheme 8

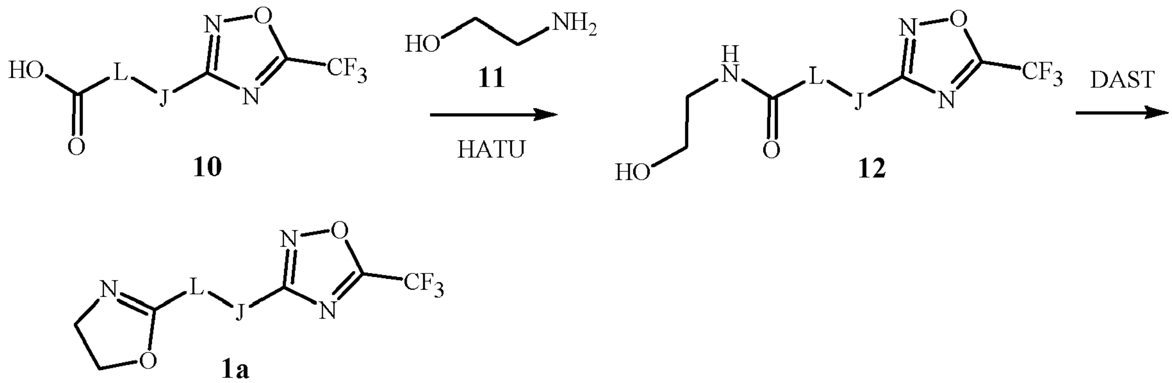

10 11 12 1a

As shown in Scheme 9, compounds of Formula **1b** (i.e. Formula **1** wherein L is NHCH(CN)) can be prepared from amines of Formula **14** and aldehydes of Formula **13** in the presence of a cyanide source under Strecker reaction conditions. A variety of solvents and cyanide sources can be employed in the method of Scheme 9. The presence of a Lewis acid such as titanium(IV) isopropoxide can be advantageous. For conditions and variations of this reaction see the following references and references cited therein: D. T. Mowry, Chemical Reviews 1948, 42, 236, H. Groeger, Chemical Reviews 2003, 103, 2795-2827, and M. North in Comprehensive Organic Functional Group Transformations A. R. Katritsky, O. Meth-Cohn and C. W. Rees Editors., Volume 3, 615-617; Pergamon, Oxford, 1995. The method of Scheme 9 is also illustrated in Step E of Example 14. For less reactive amines of Formula **14**, such as aryl amines containing ortho electron withdrawing groups, the use of trimethylsilyl cyanide in combination with a catalyst such as guanidine hydrochloride can be advantageous. For a reference see, for example, Heydari et al., Journal of Molecular Catalysis A: Chemical 2007, 277(1-2), 142-144.

Scheme 9

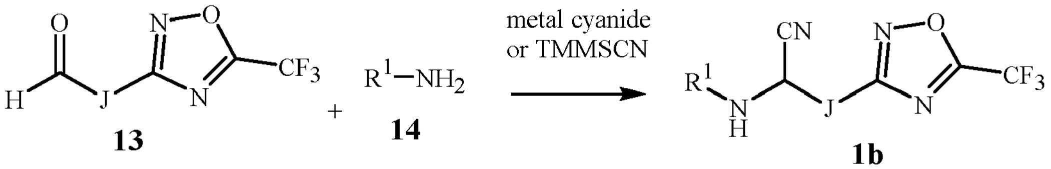

13 + 14 1b

As shown in Scheme 10, Compounds of Formula 3 can be prepared from compounds of Formulae 15 and 16 wherein $Y^3$ and $Y^4$ are suitable functional groups which under the appropriate reaction conditions will allow for the construction of the various L groups. Suitable functional groups include, but are not limited to, ionizable hydrogen (e.g., a hydrogen attached to a nitrogen atom of a heterocyclic ring or a hydrogen attached to a carbon atom adjacent to a C(=O) moiety), carbonyl, aldehyde, ketone, ester, acid, acid chloride, amine, alcohol, thiol, hydrazine, oxime, olefin, acetylene, halide, alkyl halide, boronic acid, boronate, and the like. For example, compounds of Formula **3** wherein L is $CH_2$ can be prepared by reacting a compound of Formula **15** wherein $Y^3$ is hydrogen (i.e. an ionizable hydrogen attached to a nitrogen atom ring member of an $R^1$ ring) with a base such as potassium carbonate or sodium hydride, followed by treatment with a compound of Formula **16** wherein $Y^4$ is a methyl halide (e.g., $BrCH_2$-); while treatment with a compound of Formula **16** wherein $Y^4$ is CH(=O)- will give a compound of Formula **3** wherein L is CH(OH). Compounds of Formula **3** wherein L is O can be prepared by reacting a compound of Formula **15** wherein $Y^3$ is Br with a compound of Formula **16** wherein $Y^4$ is OH in the presence of a base such as sodium hydride. Compounds of Formula **3** wherein L is $CH_2O$ can be prepared by reacting a compound of Formula **15** wherein $Y^3$ is $BrCH_2$- with a compound of Formula **16** wherein $Y^4$ is OH in the presence of a base. Compounds of Formula 3 wherein L is $OCH_2CH_2$ can be prepared by reacting a compound of Formula **15** wherein $Y^3$ is OH with a compound of Formula **16** wherein $Y^4$ is ethyl halide (e.g., $ICH_2CH_2$-) in the presence of a base. The synthetic literature describes many general methods for forming a saturated chain containing 1- to 3-atoms consisting of carbon and heteroatoms such as the L groups of the present invention; see, for example, Comprehensive Organic Functional Group Transformations, Vol. 1, 2, 3 and 5, A. R. Katritzky editor, Pergamon Press, New York, 1995; Vogel's Textbook of Practical Organic Chemistry, 5th Ed., pp 470-823, Longman Group, London, 1989; and Advanced Organic Chemistry, 4th Ed. Jerry March, Wiley, New York 1992. One skilled in the art can easily determine how to select appropriate compounds of Formula **15** and Formula **16** to construct the desired L group.

Scheme 10

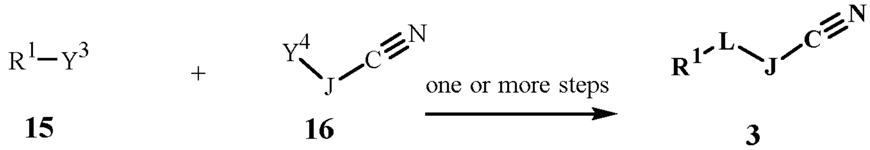


15 16 3

wherein $Y^3$ and $Y^4$ are a suitable functional groups for the construction of the desired L group

Scheme 11 illustrates a specific example of the general method of Scheme 10 for the preparation of a compound of Formula **3a** (i.e. Formula **3** wherein $R^1$ is pyrazole and L is $CH_2$). In this method a pyrazole of Formula **17** is reacted with a methyl bromide of Formula **18** in the presence of a base such as sodium or potassium hydroxide, sodium hydride or potassium carbonate in a solvent such as tetrahydrofuran, *N*,*N*-dimethylformamide, ethanol or acetonitrile typically at a temperature between about 0 to 80 °C. Present Example 10, Step A illustrates the method of Scheme 11.

Scheme 11

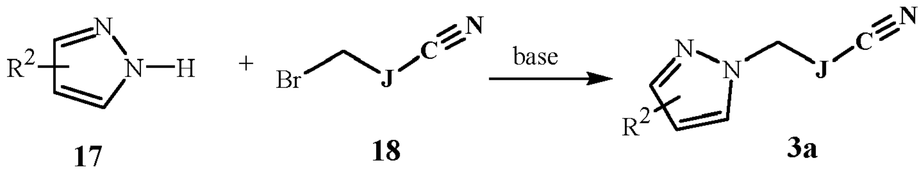


17 18 3a

One skilled in the art will recognize that the method of Scheme 10 can also be performed when the substituent-C=N in Formula **16** is replaced with 5-(trifluoromethyl)-1,2,4-oxadiazol-3-yl (i.e. Formula **19**) thus providing a compound of Formula **1** as shown below in Scheme 12.

Scheme 12

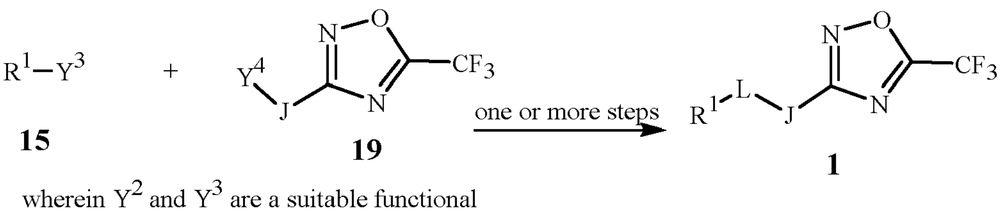


15 19 1

wherein $Y^2$ and $Y^3$ are a suitable functional groups for construction of the desired L group

Scheme 13 illustrates a specific example of the general method of Scheme 12 for the preparation of a compound of Formula **1c** (i.e. Formula **1** wherein $R^1$ is oxazole and L is $CH_2O$). In this method a methyl chloride of Formula **20** is reacted with an alcohol of Formula **21** in the presence of a base such as sodium or potassium hydroxide, sodium hydride or potassium carbonate in a solvent such as tetrahydrofuran, *N*,*N*-dimethylformamide, ethanol or acetonitrile typically at a temperature between about 0 to 80 °C. Present Examples 17 and 18 illustrate the method of Scheme 19.

Scheme 13

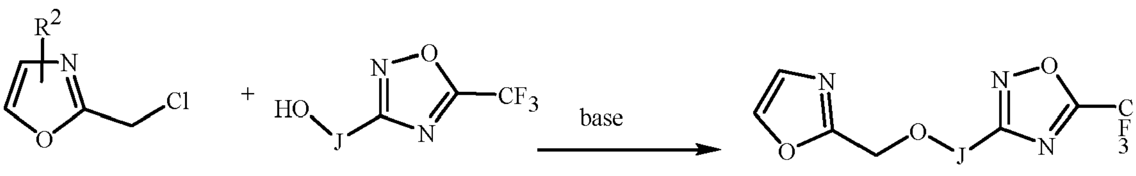


20 21 1c

It is recognized that some reagents and reaction conditions described above for preparing compounds of Formula **1** may not be compatible with certain functionalities present in the intermediates. In these instances, the incorporation of protection/deprotection sequences or functional group interconversions into the synthesis will aid in obtaining the desired products. The use and choice of the protecting groups will be apparent to one skilled in chemical synthesis (see, for example, T. W. Greene and P. G. M. Wuts, Protective Groups in Organic Synthesis, 2nd ed.; Wiley: New York, 1991). One skilled in the art will recognize that, in some cases, after the introduction of a given reagent as it is depicted in any individual scheme, it may be necessary to perform additional routine synthetic steps not described in detail to complete the synthesis of compounds of Formula **1**. One skilled in the art will also recognize that it may be necessary to perform a combination of the steps illustrated in the above schemes in an order other than that implied by the particular sequence presented to prepare the compounds of Formula **1**.

One skilled in the art will also recognize that compounds of Formula **1** and the intermediates described herein can be subjected to various electrophilic, nucleophilic, radical, organometallic, oxidation, and reduction reactions to add substituents or modify existing substituents.

Without further elaboration, it is believed that one skilled in the art using the preceding description can utilize the present invention to its fullest extent. The following Examples are, therefore, to be construed as merely illustrative, and not limiting of the disclosure in any way whatsoever. Steps in the following Examples illustrate a procedure for each step in an overall synthetic transformation, and the starting material for each step may not have necessarily been prepared by a particular preparative run whose procedure is described in other Examples or Steps. Percentages are by weight except for chromatographic solvent mixtures or where otherwise indicated. Parts and percentages for chromatographic solvent mixtures are by volume unless otherwise indicated. $^1$H NMR spectra are reported in ppm downfield from tetramethylsilane; "s" means singlet, "d" means doublet, "t" means triplet, "q" means quartet, "m" means multiplet, "dd" means doublet of doublets, "dt" means doublet of triplets, "br s" means broad singlet and "br d" means broad doublet. $^{19}$F NMR spectra are reported in ppm using trichlorofluoromethane as the reference.

EXAMPLE 1

Preparation 1-[[4-[5-(trifluoromethyl)-1,2,4-oxadiazol-3-yl]phenyl]methyl]-1*H*-pyrazolo[3,4-*b*]pyridine (Compound 147)

Step A: Preparation of 4-[5-(trifluoromethyl)-1,2,4-oxadiazol-3-yl]benzenemethanol

A mixture of 4-(hydroxymethyl)benzonitrile (52.8 g, 397 mmol), hydroxylamine hydrochloride (33.1 g, 476 mmol), *N*,*N*-diisopropylethylamine (107 mL, 595 mmol) and 8-quinolinol (0.3 g) in ethanol (400 mL) was heated at reflux for 5 h. The reaction mixture was concentrated under reduced pressure to provide the intermediate compound *N*-hydroxy-4-(hydroxymethyl)benzenecarboximidamide.

To a mixture of *N*-hydroxy-4-(hydroxymethyl)benzenecarboximidamide in acetonitrile and tetrahydrofuran (1:1, 400 mL) was added pyridine (70 mL, 873 mmol) and trifluoroacetic anhydride (121 mL, 873 mmol) dropwise at room temperature. The reaction mixture was heated at reflux for 15 h, allowed to cool to room temperature, and then saturated aqueous sodium bicarbonate solution (300 mL) was slowly added, followed by ethyl acetate (400 mL) and water. The resulting mixture was separated and the organic layer was washed with saturated aqueous sodium chloride solution (100 mL), dried over sodium sulfate, filtered and concentrated under reduce pressure to provide the title compound.

$^1$H NMR ($CDCl_3$): δ 4.77 (s, 2H), 7.50 (d, 2H), 8.08 (d, 2H).
$^{19}$F NMR ($CDCl_3$): δ -65.47.

Step B: Preparation of 3-[4-(chloromethyl)phenyl]-5-(trifluoromethyl)-1,2,4-oxadiazole

To a mixture of 4-[5-(trifluoromethyl)-1,2,4-oxadiazol-3-yl]benzenemethanol (i.e. the product of Step A) (98.0 g, 397 mmol) and N,N dimethylformamide (4 drops) in dichloromethane (500 mL) at 5 °C was added thionyl chloride (57 mL). The reaction mixture was heated at approximately 42 °C for 30 minutes, and then concentrated under reduced pressure to remove the dichloromethane. The resulting mixture was diluted with acetonitrile (80 mL) and poured into ice water (700 mL). The resulting solid precipitate was collected by filtration, rinsed with water, and dried in a vacuum oven under nitrogen to provide the title compound as a solid (55 g).

$^1$H NMR ($CDCl_3$): δ 4.64 (s, 2H), 7.54-7.55 (d, 2H), 8.10-8.12 (d, 2H).
$^{19}$F NMR ($CDCl_3$): δ -65.45.

Step C: Preparation of 1-[[4-[5-(trifluoromethyl)-1,2,4-oxadiazol-3-yl]phenyl]methyl]-1*H*-pyrazolo[3,4-*b*]pyridine

A mixture of 3-[4-(chloromethyl)phenyl]-5-(trifluoromethy)-1,2,4-oxadiazole (i.e. the product of Step B) (0.3 g, 1.1 mmol), 1*H*-pyrazolo[3,4-*b*]pyridine (0.136 g, 1.1 mmol) and cesium carbonate (0.38 g, 1.1 mmol) in *N*,*N*-dimethylformamide (2.5 ml) was stirred at room temperature for 12 h. The reaction mixture was partitioned between ethyl acetate (25 ml) and water (5 ml). The organic layer was separated and washed with saturated aqueous sodium chloride solution, dried over sodium sulfate, filtered and concentrated under reduced pressure. The resulting material was purified by silica gel chromatography (eluting with a gradient of 5 to 50% ethyl acetate in hexanes) to provide the title compound, a compound of the present invention, as a solid (0.090 g).

$^1$H NMR ($CDCl_3$): δ 5.80 (s, 2H), 7.13-7.21 (m, 1H), 7.42-7.51 (m, 2H), 8.01-8.13 (m, 4H), 8.58 (dd, 1H).
$^{19}$F NMR ($CDCl_3$): δ -65.43.

EXAMPLE 2

Preparation of 1-[[4-[5-(trifluoromethyl)-1,2,4-oxadiazol-3-yl]phenyl]methyl]-1*H*-pyrazole-4-carbonitrile (Compound 95)

A mixture of 3-[4-(chloromethyl)phenyl]-5-(trifluoromethyl)-1,2,4-oxadiazole (i.e. product of Example 1, Step B) (3.60 g, 13.7 mmol), 1*H*-pyrazole-4-carbonitrile (1.91 g, 20.6 mmol), potassium carbonate (3.41 g, 24.7 mmol) and sodium bromide (1.62 g, 15.8 mmol) in acetonitrile (100 mL) was heated at reflux for 18 h. The reaction mixture was allowed to cool to room temperature, filtered, and the filtrate was concentrated under reduced pressure. The resulting material was purified by silica gel chromatography (eluting with a gradient of 0 to 100% ethyl acetate in hexanes) to provide a white solid (4.38 g). The solid was crystalized from ethanol to provide the title compound, a compound of the present invention, as a white solid (3.29 g) melting at 106-108 °C.

$^1$H NMR ($CDCl_3$): δ 5.42 (s, 2H), 7.38-7.39 (d, 2H), 7.86-7.87 (m, 2H), 8.13-8.15 (d, 2H).
$^{19}$F NMR ($CDCl_3$): δ -65.33.

EXAMPLE 3

Preparation of 6-chloro-2-[[4-[5-(trifluoromethyl)-1,2,4-oxadiazol-3-yl]phenyl]methyl]-3(2*H*)-pyridazinone (Compound 4)

A mixture of 3-[4-(chloromethyl)phenyl]-5-(trifluoromethyl)-1,2,4-oxadiazole (i.e. product of Example 1, Step B) (0.2 g, 0.76 mmol), 6-chloro-3(2*H*)-pyridazinone (0.099 g, 0.76 mmol) and potassium carbonate (0.21 g, 1.52 mmol) in *N*,*N*-dimethylformamide (5 mL) was stirred at room temperature overnight. The reaction mixture was diluted with water and extracted with diethyl ether (2x). The combined extracts were washed with saturated aqueous sodium chloride solution, dried over sodium sulfate, filtered and concentrated under reduced pressure. The resulting material was purified by silica gel chromatography (eluting with 1:1 ethyl acetate in hexanes) to provide the title compound, a compound of the present invention, as a white solid (0.18 g).
$^1$H NMR ($CDCl_3$): δ 8.10 (d, 2H), 7.58 (d, 2H), 7.19 (d, 1H), 6.95 (d, 1H), 5.32 (s, 2H).

EXAMPLE 4

Preparation of methyl 2-[[4-[5-(trifluoromethyl)-1,2,4-oxadiazol-3-yl]phenyl]methyl]-2*H*-indazole-4-carboxylate (Compound 36) and methyl 1-[[4-[5-(trifluoromethyl)-1,2,4-oxadiazol-3-yl]phenyl]methyl]-1*H*-indazole-4-carboxylate (Compound 49)

A mixture of 3-[4-(chloromethyl)phenyl]-5-(trifluoromethyl)-1,2,4-oxadiazole (i.e. the product of Example 1, Step B) (0.3 g, 1.1 mmol), methyl 1*H*-indazole-4-carboxylate (0.2 g, 1.1 mmol) and cesium carbonate (0.56 g, 1.7 mmol) in *N*,*N*-dimethylformamide (2.5 ml) was stirred at room temperature for 12 h. The reaction mixture was partitioned between ethyl acetate (25 ml) and water (5 ml). The organic layer was separated and washed with saturated aqueous sodium chloride solution, dried over sodium sulfate, filtered and concentrated under reduced pressure. The resulting material was purified by silica gel chromatography (eluting with a gradient of 5 to 50% ethyl acetate in hexanes) to provide the title Compound 49 (faster eluting product) as a solid (0.11 g). Also obtained was the title Compound 36, (slower eluting product) as a solid (0.04 g).

$^1$H NMR ($CDCl_3$): δ 3.97 (s, 3H), 5.73 (s, 2H), 7.33-7.42 (m, 1H), 7.42-7.49 (m, 2H), 7.90-8.02 (m, 2H), 8.07-8.16 (m, 2H), 8.53 (s, 1H) (Compound 36).

$^{19}F$ NMR ($CDCl_3$): δ -65.39 (Compound 36).

$^{1}H$ NMR ($CDCl_3$): δ 3.98-4.08 (m, 3H), 5.67-5.76 (m, 2H), 7.28-7.35 (m, 2H), 7.37-7.48 (m, 1H), 7.50-7.59 (m, 1H), 7.89-7.98 (m, 1H), 8.01-8.10 (m, 2H), 8.59 (s, 1H) (Compound 49).

$^{19}F$ NMR ($CDCl_3$): δ -65.42 (Compound 49).

EXAMPLE 5

Preparation of 1-[[4-[5-(trifluoromethyl)-1,2,4-oxadiazol-3-yl]phenyl]methyl]-1*H*-pyrrolo[3,2-*b*]pyridine (Compound 136)

A mixture of 3-[4-(chloromethyl)phenyl]-5-(trifluoromethyl)-1,2,4-oxadiazole (i.e. the product of Example 1, Step B) (0.3 g, 1.1 mmol), 1*H*-pyrrolo[3,2-*b*]pyridine (0.14 g, 1.1 mmol) and cesium carbonate (0.56 g, 1.7 mmol) in *N*,*N*-dimethylformamide (2.5 ml) was stirred at room temperature for 12 h. The reaction mixture was partitioned between ethyl acetate (25 ml) and water (5 ml). The organic layer was separated and washed with saturated aqueous sodium chloride solution, dried over sodium sulfate, filtered and concentrated under reduced pressure. The resulting material was purified by silica gel chromatography (eluting with a gradient of 5 to 50% ethyl acetate in hexanes) to provide the title compound, a compound of the present invention, as a solid (0.045 g).

$^{1}H$ NMR ($CDCl_3$): δ 5.42 (m, 2H), 6.79-6.88 (m, 1H), 7.06-7.15 (m, 1H), 7.23 (m, 2H), 7.41 (d, 1H), 7.54 (d, 1H), 8.07 (d, 2H), 8.49 (br d, 1H).

$^{19}F$ NMR ($CDCl_3$): δ -65.40.

EXAMPLE 6

Preparation of 1-[[4-[5-(trifluoromethyl)-1,2,4-oxadiazol-3-yl]phenyl]methyl]-2(1*H*)-quinoxalinone (Compound 158)

A mixture of 3-[4-(chloromethyl)phenyl]-5-(trifluoromethyl)-1,2,4-oxadiazole (i.e. the product of Example 1, Step B) (0.3 g, 1.1 mmol), 2(1*H*)-quinoxalinone (0.17 g, 1.1 mmol) and cesium carbonate (0.56 g, 1.7 mmol) in *N*,*N*-dimethylformamide (2.5 ml) was stirred at room temperature for 12 h. The reaction mixture was partitioned between ethyl acetate (25 ml) and water (5 ml). The organic layer was separated and washed with saturated aqueous sodium chloride solution, dried over sodium sulfate, filtered and concentrated under reduced pressure. The resulting material was purified by silica gel chromatography (eluting with a gradient of 5 to 50% ethyl acetate in hexanes) to provide the title compound, a compound of the present invention, as a solid (0.08 g).

$^{1}H$ NMR ($CDCl_3$): δ 5.57 (s, 2H), 7.19-7.24 (m, 1H), 7.32-7.38 (m, 1H), 7.40 (d, 2H), 7.45-7.53 (m, 1H), 7.90-7.97 (m, 1H), 8.08 (d, 2H), 8.44 (s, 1H).

$^{19}F$ NMR ($CDCl_3$): δ -65.40.

EXAMPLE 7

Preparation of 1,3-dihydro-1-methyl-3-[[4-[5-(trifluoromethyl)-1,2,4-oxadiazol-3-yl]phenyl]methyl]-2*H*-benzimidazol-2-one (Compound 159)

A mixture of 3-[4-(chloromethyl)phenyl]-5-(trifluoromethyl)-1,2,4-oxadiazole (i.e. the product of Example 1, Step B) (0.3 g, 1.1 mmol), 1,3-dihydro-1-methyl-2H benzimidazol-2-one (0.17 g, 1.1 mmol) and cesium carbonate (0.56 g, 1.7 mmol) in *N*,*N*-dimethylformamide (2.5 ml) was stirred at room temperature for 12 h. The reaction mixture was partitioned between ethyl acetate (25 ml) and water (5 ml). The organic layer was separated and washed with saturated aqueous sodium chloride solution, dried over sodium sulfate, filtered and concentrated under reduced pressure. The resulting material was purified by silica gel chromatography (eluting with a gradient of 0 to 50% ethyl acetate in hexanes) to provide the title compound, a compound of the present invention, as a solid (0.095 g).
$^{1}H$ NMR ($CDCl_3$): δ 3.49 (s, 3H), 5.16 (s, 2H), 6.81-6.90 (m, 1H), 6.97-7.06 (m, 2H), 7.06-7.18 (m, 1H), 7.47 (d, 2H), 8.07 (d, 2H).

EXAMPLE 8

Preparation of methyl 1,2-dihydro-2-oxo-1-[[4-[5-(trifluoromethyl)-1,2,4-oxadiazol-3-yl]phenyl]methyl]-4-pyridinecarboxylate (Compound 24)

A mixture of 3-[4-(chloromethyl)phenyl]-5-(trifluoromethyl)-1,2,4-oxadiazole (i.e. the product of Example 1, Step

B) (1.0 g, 3.8 mmol), methyl 1,2-dihydro-2-oxo-4-pyridinecarboxylate (0.59 g, 3.81 mmol) and cesium carbonate (1.90 g, 5.72 mmol) in *N,N*-dimethylformamide (5 ml) was stirred at room temperature for 12 h. The reaction mixture was partitioned between ethyl acetate (25 ml) and water (5 ml). The organic layer was separated and washed with saturated aqueous sodium chloride solution, dried over sodium sulfate, filtered and concentrated under reduced pressure. The resulting material was purified by silica gel chromatography (eluting with a gradient of 5 to 50% ethyl acetate in hexanes) to provide the title compound, a compound of the present invention, as a solid (0.35 g).

$^1$H NMR ($CDCl_3$): δ 3.92 (s, 3H), 5.23 (s, 2H), 6.64-6.73 (m, 1H), 7.27 (d, 1H), 7.33-7.40 (m, 1H), 7.40-7.49 (m, 2H), 8.04-8.17 (m, 2H).
$^{19}$F NMR ($CDCl_3$): δ -65.39.

EXAMPLE 9

Preparation of 2-[[4-[5-(trifluoromethyl)-1,2,4-oxadiazol-3-yl]phenyl]methyl]-1*H*-isoindole-1,3(2*H*)-dione (Compound 90)

A mixture of 3-[4-(chloromethyl)phenyl]-5-(trifluoromethyl)-1,2,4-oxadiazole (i.e. the product of Example 1, Step B) (0.25 g, 0.95 mmol) and potassium phthalimide (0.17 g, 0.95 mmol) in *N,N*-dimethylformamide (2.5 ml) was stirred at room temperature for 12 h. The reaction mixture was partitioned between ethyl acetate (25 ml) and water (5 ml). The organic layer was separated and washed with saturated aqueous sodium chloride solution, dried over sodium sulfate, filtered and concentrated under reduced pressure. The resulting material was purified by silica gel chromatography (eluting with a gradient of 5 to 50% ethyl acetate in hexanes) to provide the title compound, a compound of the present invention, as a solid (0.21 g).

$^1$H NMR ($CDCl_3$): δ 4.92 (s, 2H), 7.50-7.63 (m, 2H), 7.68-7.79 (m, 2H), 7.82-7.93 (m, 2H), 8.01-8.13 (m, 2H).
$^{19}$F NMR ($CDCl_3$): δ -65.39.

EXAMPLE 10

Preparation of ethyl 1-[[4-[5-(trifluoromethyl)-1,2,4-oxadiazol-3-yl]phenyl]methyl]-1*H*-pyrazole-4-carboxylate (Compound 83)

Step A: Preparation of ethyl 1-[(4-cyanophenyl)methyl]-1*H*-pyrazole-4-carboxylate

A mixture of ethyl 1*H*-pyrazole-4-carboxylate (4.96 g, 35.4 mmol), 4-(bromomethyl)benzonitrile (6.92 g, 35.3 mmol) and potassium carbonate (6.0 g, 43.5 mmol) in acetonitrile (100 mL) was heated at 60 °C for 6 h, and then stirred at room temperature overnight. The reaction mixture was diluted with water and the resulting solid precipitate was filtered, washed with water and air dried to provide the title compound as a white solid (8.65 g).
$^1$H NMR ($CDCl_3$): δ 1.34 (t, 3H), 4.30 (q, 2H), 5.37 (s, 2H), 7.29-7.31 (m, 2H), 7.65-7.66 (m, 2H), 7.93 (s, 1H), 7.96 (s, 1H).

Step B: Preparation of ethyl 1-[[4-[(hydroxyamino)iminomethyl]phenyl]methyl]-1*H*-pyrazole-4-carboxylate

A mixture ethyl 1-[(4-cyanophenyl)methyl]-1*H*-pyrazole-4-carboxylate (i.e. the product of Step A) (29.1 g, 114 mmol) and hydroxylamine (50% aqueous solution, 12 mL, 194 mmol) in *N,N*-dimethylformamide (200 mL) was stirred at room temperature for 3 days. The reaction mixture was poured into ice water and the resulting solid precipitate was filtered and washed with water. The wet solid was mixed with acetonitrile (500 mL) and concentrated under reduced pressure to provide the title compound as a white solid (31.32 g)
$^1$H NMR (DMSO-$d_6$): δ 1.26 (t, 3H), 4.21 (q, 2H), 5.38 (s, 2H), 5.79 (s, 2H), 7.25-7.27 (m, 2H), 7.63-7.65 (m, 2H), 7.87 (s, 1H), 8.47 (s, 1H), 9.63 (s, 1H).

Step C: Preparation of ethyl 1-[[4-[5-(trifluoromethyl)-1,2,4-oxadiazol-3-yl]phenyl]methyl]-1*H*-pyrazole-4-carboxylate

To a mixture of ethyl 1-[[4-[(hydroxyamino)iminomethyl]phenyl]methyl]-1*H*-pyrazole-4-carboxylate (i.e. the product of Step B) (33.65 g, 117 mmol) and pyridine (13 mL, 160 mmol) in *N,N*-dimethylformamide (100 mL) at 0 °C was added trifluoroacetic anhydride (19 mL, 140 mmol) dropwise over 20 minutes. The reaction mixture was heated at 70 °C for 2 h, cooled and allowed to stir at room temperature overnight. The reaction mixture was poured into ice water and the resulting solid precipitate was filtered and washed with water. The solid was crystallized from ethanol (250 mL) to provide the title compound, a compound of the present invention, as solid needles (35.1 g) melting at 127-129 °C.

$^1H$ NMR ($CDCl_3$): δ 1.34 (t, 3H), 4.30 (q, 2H), 5.39 (s, 2H), 7.37-7.39 (m, 2H), 7.93 (s, 1H), 7.97 (s, 1H), 8.11-8.13 (m, 2H).
$^{19}F$ NMR ($CDCl_3$): δ -65.34.

EXAMPLE 11

Preparation of 3-[5-[(4-bromo-1*H*-pyrazol-1-yl)methyl]-2-thienyl]-5-(trifluoromethyl)-1,2,4-oxadiazole (Compound 71)

Step A: Preparation of 3-(5-methyl-2-thienyl)-5-(trifluoromethyl)-1,2,4-oxadiazole

A mixture of 5-methyl-2-thiophenecarbonitrile (4.0 g, 32 mmol), hydroxylamine hydrochloride (3.3 g, 48 mmol), diisopropylethylamine (10 ml, 56 mmol) and 8-quinolinol (0.074 g) in ethanol (65 ml) was refluxed for 5 h. The reaction mixture was concentrated under reduced pressure to provide the intermediate compound N-hydroxy-5-methyl-2-thiophenecarboximidamide.

*N*-hydroxy-5-methyl-2-thiophenecarboximidamide in tetrahydrofuran (65 ml) was added dropwise to a mixture of trifluoroacetic anhydride (13 mL, 96 mmol), diisopropylethylamine (20 mL, 112 mmol) and 4-dimethylaminopyridine (1.0 g, 8 mmol). The reaction mixture was stirred at room temperature 15 h, and then diluted with saturated aqueous sodium bicarbonate solution (30 mL). The aqueous mixture was extracted with ethyl acetate (80 ml) and the organic layer was washed with saturated aqueous sodium chloride solution, dried over sodium sulfate, filtered and concentrated under reduced pressure. The resulting material was purified by silica gel chromatography (eluting with 1:10 ethyl acetate in hexanes) to provide the title compound as a solid (3.2 g).

$^1H$ NMR ($CDCl_3$): δ 2.57 (s, 3H), 6.85 (d, 1H), 7.68 (d, 1H).
$^{19}F$ NMR ($CDCl_3$): δ -65.46.

Step B: Preparation of 3-[5-(bromomethyl)-2-thienyl]-5-(trifluoromethyl)-1,2,4-oxadiazole

To a mixture of 3-(5-methyl-2-thienyl)-5-(trifluoromethyl)-1,2,4-oxadiazole (i.e. the product of Step A) (3.1 g, 13.2 mmol) in dichloromethane (30 mL) was added *N*-bromosuccinimide (2.6 g, 14.8 mmol) and 2,2'-azodiisobutyronitrile (0.2 g, 1.3 mmol). The reaction mixture was heated at reflux for 6 h, cool to room temperature, and concentrated under reduced pressure. The resulting material was purified by silica gel chromatography (eluting with 1:10 ethyl acetate in hexanes) to provide the title compound as a white solid (2.8 g).

$^1H$ NMR ($CDCl_3$): δ 4.73 (s, 2H), 7.18 (m, 1H), 7.73 (m, 1H).
$^{19}F$ NMR ($CDCl_3$): δ -65.40.

Step C: Preparation of 3-[5-[(4-bromo-1*H*-pyrazol-1-yl)methyl]-2-thienyl]-5-(trifluoromethyl)-1,2,4-oxadiazole

A mixture of 3-[5-(bromomethyl)-2-thienyl]-5-(trifluoromethyl)-1,2,4-oxadiazole (i.e. the product of Step B) (0.16 g, 0.5 mmol), 4-bromo-1*H*-pyrazole (0.080 g, 0.55 mmol) and potassium carbonate (0.152 g, 1.1 mmol) in acetonitrile (4 mL) was heated at refluxed for 2 h. After cooling to room temperature, the reaction mixture was concentrated under reduced pressure. The resulting material was purified by silica gel chromatography (eluting with 3:1 hexanes in ethyl acetate) to provide the title compound, a compound of the present invention, as an oil (0.088 g).

$^1H$ NMR ($CDCl_3$): δ 5.47 (s, 2H), 7.09 (m, 1H), 7.48 (s, 1H), 7.50 (s, 1H), 7.74 (m, 1H).
$^{19}F$ NMR ($CDCl_3$): δ -65.41.

EXAMPLE 12

Preparation of ethyl 1-[1-[4-[5-(trifluoromethyl)-1,2,4-oxadiazol-3-yl]phenyl]ethyl]-1*H*-pyrazole-4-carboxylate (Compound 2)

Step A: Preparation of 4-[5-(trifluoromethyl)-1,2,4-oxadiazol-3-yl]benzaldehyde

To a mixture of 4-[5-(trifluoromethyl)-1,2,4-oxadiazol-3-yl]benzenemethanol (2.00 g, 8.19 mmol) in dichloromethane (20 ml) was added 1,1,1-tris(acetoxy)-1,1-dihydro-1,2-benziodoxol-3(1*H*)-one (Dess-Martin periodinane) (3.47 g, 8.19 mmol) portionwise at room temperature. After stirring for 3 h, the reaction mixture was diluted with dichloromethane and saturated aqueous sodium bicarbonate solution. The organic layer was separated and washed with

saturated aqueous sodium chloride solution, dried over sodium sulfate, filtered and concentrated under reduced pressure. The resulting material was purified by silica gel chromatography (eluting with a gradient of 5 to 50% ethyl acetate in hexanes) to provide the title compound as an oil (1.53 g).

$^1$H NMR ($CDCl_3$): $\delta$ 8.05 (d, 2H), 8.32 (d, 2H), 10.12 (s, 1H).
$^{19}$F NMR ($CDCl_3$): $\delta$ -65.32.

Step B: Preparation of $\alpha$-methyl-4-[5-(trifluoromethyl)-1,2,4-oxadiazol-3-yl]benzenemethanol

To a mixture of 4-[5-(trifluoromethyl)-1,2,4-oxadiazol-3-yl]benzaldehyde (i.e. the product of Step A) (1.53 g, 6.3 mmol) in tetrahydrofuran (20 ml) at -12 °C was added a solution of methylmagnesium bromide (3M in diethyl ether; 2.1 mL, 6.3 mmol) dropwise. The reaction mixture was stirred for 2 h at -12 °C, and then quenched with saturated aqueous ammonium chloride solution. The resulting mixture was poured into water and extracted with ethyl acetate. The combined organic extracts were washed with saturated aqueous sodium chloride solution, dried over sodium sulfate, filtered and concentrated under reduced pressure. The resulting material was purified by silica gel chromatography (eluting with a gradient of 5 to 50% ethyl acetate in hexanes) to provide the title compound as a solid (1.63 g).

$^1$H NMR ($CDCl_3$): $\delta$ 1.40-1.61 (m, 3H), 4.84-5.06 (m, 1 H), 7.50 (d, 2H), 8.06 (d, 2H).
$^{19}$F NMR ($CDCl_3$): $\delta$ -65.49.

Step C: Preparation of ethyl 1-[1-[4-[5-(trifluoromethyl)-1,2,4-oxadiazol-3-yl]phenyl]ethyl]-1*H*-pyrazole-4-carboxylate

A mixture of $\alpha$-methyl-4-[5-(trifluoromethyl)-1,2,4-oxadiazol-3-yl]benzenemethanol (i.e. the product of Step B) (0.372 g, 0.95 mmol), ethyl 1*H*-pyrazole-4-carboxylate (0.202 g, 0.95 mmol) and triphenylphosphine (0.377 g, 0.95 mmol) in tetrahydrofuran (10 mL) was stirred at room temperature for 10 minutes, and then 1,2-bis(1-methylethyl) 1,2-diazenedicarboxylate (DIAD) (0.285 mL, 0.95 mmol) was added. After 12 h, the reaction mixture was concentrated under reduced pressure. The resulting material was purified by silica gel flash chromatography (eluting with a gradient of 5 to 50% ethyl acetate in hexanes) to provide the title compound, a compound of the present invention, as a solid (0.11 g).

$^1$H NMR ($CDCl_3$): $\delta$ 1.25-1.38 (m, 3H), 1.88-2.00 (m, 3H), 4.28 (q, 2H), 5.49-5.67 (m, 1H), 7.29-7.41 (m, 2H), 7.89-8.02 (m, 2H), 8.03-8.16 (m, 2H).
$^{19}$F NMR ($CDCl_3$): $\delta$ -65.44.

EXAMPLE 13

Preparation of 4,5-dihydro-*N*,*N*-dimethyl-5-[[4-[5-(trifluoromethyl)-1,2,4-oxadiazol-3-yl]phenyl]methyl]-3-isoxazolecarboxamide (Compound 12)

Step A: Preparation of *N*-hydroxy-4-(2-propen-1-yl)benzenecarboximidamide

A mixture of 4-(2-propen-1-yl)benzonitrile (5.0 g, 35 mmol) and hydroxylamine (50% aqueous solution, 4.5 mL, 73 mmol) in absolute ethanol (50 mL) was stirred at room temperature for 16 h. The reaction mixture was concentrated under reduced pressure, diluted with acetonitrile (50 mL), concentrated under reduced pressure, and again diluted with acetonitrile (50 mL) and concentrated under reduced pressure to provide the title compound as a colorless oil which crystalized on standing (6.1 g).
$^1$H NMR ($CDCl_3$): $\delta$ 3.39-3.41 (m, 2H), 4.90 (br s, 2H), 5.07-5.10 (m, 2H), 5.90-6.00 (m, 1H), 7.20-7.22 (m, 2H), 7.54-7.56 (m, 2H), 8.5-9.5 (br s, 1H).

Step B: Preparation of 3-[4-(2-propen-1-yl)phenyl]-5-(trifluoromethyl)-1,2,4-oxadiazole

To a mixture of *N*-hydroxy-4-(2-propen-1-yl)benzenecarboximidamide (i.e. the product of Step A) (6.0 g, 34 mmol) and pyridine (3.5 mL, 43 mmol) in acetonitrile (25 mL) at 0 °C was added trifluoroacetic anhydride (5.5 mL, 40 mmol) dropwise over 20 minutes. The reaction mixture was heated at 60 °C for 4 h, cooled, and then poured into ice water and extracted with diethyl ether (3 $\times$ 100 mL). The combined organic extracts were washed with aqueous hydrochloric acid solution (1N), saturated aqueous sodium bicarbonate solution, saturated aqueous sodium chloride solution, dried over magnesium sulfate, filtered and concentrated under reduced pressure to provide the title compound as a light-yellow oil (8.0 g).

$^1H$ NMR ($CDCl_3$): δ 3.46-3.48 (m, 2H), 5.10-5.15 (m, 2H), 5.90-6.05 (m, 1H), 7.34-7.36 (m, 2H), 8.03-8.05 (m, 2H).
$^{19}F$ NMR ($CDCl_3$): δ -65.40.

Step C: Preparation of 4,5-dihydro-*N*,*N*-dimethyl-5-[[4-[5-(trifluoromethyl)-1,2,4-oxadiazol-3-yl]phenyl]methyl]-3-isoxazolecarboxamide

A mixture of 3-[4-(2-propen-1-yl)phenyl]-5-(trifluoromethyl)-1,2,4-oxadiazole (i.e. the product of Step B) (0.254 g, 1.0 mmol), 2-(dimethylamino)-N-hydroxy-2-oxo-acetimidoyl chloride (0.152 g, 1.0 mmol) and sodium bicarbonate (0.3 g, 3.5 mmol) in ethyl acetate (20 mL) was stirred at room temperature for 24 h. The reaction mixture was filtered washing with a small amount of ethyl acetate and the filtrate was concentrated under reduced pressure. The resulting material was purified by silica gel flash chromatography (eluting with a gradient of 0-100% ethyl acetate in hexanes) to provide the title compound, a compound of the present invention, as a white solid (0.27 g).

$^1H$ NMR ($CDCl_3$): δ 3.00-3.15 (m, 2H), 3.03 (s, 3H), 3.17 (s, 3H), 3.33-3.40 (m, 1H), 4.92-5.00 (m, 1H), 7.40-7.44 (m, 2H), 8.06-8.10 (m, 2H).
$^{19}F$ NMR ($CDCl_3$): δ -65.36.

EXAMPLE 14

Preparation of α-(phenylamino)-4-[5-(trifluoromethyl)-1,2,4-oxadiazol-3 -yl]benzeneacetonitrile (Compound 13)

Step A: Preparation of 4-(1,3-dioxolan-2-yl)benzonitrile

To a mixture of 4-formylbenzonitrile (25.16 g, 191.9 mmol) in toluene (250 mL) was added ethylene glycol (35.73 g, 576 mmol) and p-toluenesulfonic acid monohydrate (2.92 g, 15.3 mmol). The reaction mixture was heated at reflux for 18 h with use of a Dean-Stark trap for the azeotropic removal of water. After cooling to room temperature, the reaction mixture was washed with saturated aqueous sodium bicarbonate solution, saturated aqueous sodium chloride solution, dried over magnesium sulfate, filtered and concentrated under reduced pressure to provide the title compound as a white solid (33.6 g).
$^1H$ NMR ($CDCl_3$): δ 4.04-4.13 (m, 4H), 5.85 (s, 1H), 7.57-7.62 (m, 2H), 7.66-7.70 (m, 2H).

Step B: Preparation of 4-(1,3-dioxolan-2-yl)-N-hydroxybenzenecarboximidamide

A mixture of 4-(1,3-dioxolan-2-yl)benzonitrile (i.e. the product of Step A) (33.6 g, 192 mmol) and hydroxylamine (50% aqueous solution, 14 mL, 228 mmol) in ethanol (200 mL) was heated at 70 °C for 1 h. After cooling to room temperature, the reaction mixture was concentrated under reduced pressure. The resulting material was diluted with acetonitrile and concentrated under reduced pressure to provide the title compound as a white solid (40.2 g).
$^1H$ NMR (DMSO-$d_6$): δ 3.91-4.09 (m, 4H), 5.74 (s, 1H), 5.77-5.88 (m, 2H), 7.42-7.44 (m, 2H), 7.68-7.71 (m, 2H), 9.67 (s, 1H).

Step C: Preparation of 3-[4-(1,3-dioxolan-2-yl)phenyl]-5-(trifluoromethyl)-1,2,4-oxadiazole

To a mixture of 4-(1,3-dioxolan-2-yl)-*N*-hydroxybenzenecarboximidamide (i.e. the product of Step B) (40.2 g, 192 mmol) and pyridine (18.3 mL, 226 mmol) in acetonitrile (350 mL) at 0 °C was added trifluoroacetic anhydride (28.8 mL, 207 mmol) dropwise over 10 minutes. The reaction mixture was allowed to warm to room temperature and stirred overnight. The reaction mixture was concentrated under reduced pressure and the resulting material was partitioned between dichloromethane and water. The organic layer was separated and washed with saturated aqueous sodium chloride solution, dried over magnesium sulfate, filtered and concentrated under reduced pressure. The resulting material was purified by silica gel chromatography (eluting with a gradient of 0 to 100% ethyl acetate in hexanes) to provide the title compound as a white solid melting at 53-55 °C.

$^1H$ NMR ($CDCl_3$): δ 4.05-4.23 (m, 4H), 5.88 (s, 1H), 7.65 (d, 2H), 8.14 (d, 2H).
$^{19}F$ NMR ($CDCl_3$): δ -65.36.

Step D: Preparation of 4-[5-(trifluoromethyl)-1,2,4-oxadiazol-3-yl)]benzaldehyde

A mixture of 3-[4-(1,3-dioxolan-2-yl)phenyl]-5-(trifluoromethyl)-1,2,4-oxadiazole (i.e. the product of Step C) (2.48 g 8.67 mmol), tetrahydrofuran (25 mL), water (25 mL) and concentrated hydrochloric acid (25 mL) was stirred for

30 minutes at room temperature. The reaction mixture was diluted with ethyl acetate (100 mL) and the layers were separated. The organic layer was washed with water and saturated aqueous sodium chloride solution, dried over magnesium sulfate, filtered and concentrated under reduced pressure to provide the title compound as a white solid (2.09 g) melting at 50-52 °C.
$^1$H NMR ($CDCl_3$): δ 8.04-8.06 (m, 2H) 8.31-8.33 (m, 2H) 10.12 (s, 1H).

Step E: Preparation of α-(phenylamino)-4-[5-(trifluoromethyl)-1,2,4-oxadiazol-3-yl]benzeneacetonitrile

A mixture of 4-[5-(trifluoromethyl)-1,2,4-oxadiazol-3-yl)benzaldehyde (i.e. the product of Step D) (2.59 g, 10.7 mmol), benzenamine (95 uL, 1.0 mmol) and titanium(IV) isopropoxide (500 uL, 170 mmol) in tetrahydrofuran (5 mL) was stirred at room temperature for 2 h, and then trimethylsilyl cyanide (500 uL, 3.9 mol) was added. The reaction mixture was stirred at room temperature overnight, and then added to a vigorously stirred mixture of ice and ethyl acetate. After 1 h, the mixture was filtered and the filtrate was washed with water and saturated aqueous sodium chloride solution, dried over magnesium sulfate, filtered and concentrated under reduced pressure to provide a yellow solid. The solid was crystalized from diethyl ether (4 mL) and hexanes (10 mL) to provide the title compound, a compound of the present invention, as a colorless solid (163 mg).

$^1$H NMR ($CDCl_3$): δ 4.12 (d, 1H), 5.55 (d, 1H), 6.80 (m, 2H), 6.95 (m, 1H), 7.27-7.32 (m, 2H), 7.79-7.81 (m, 2H), 8.20-8.24 (m, 2H).
$^{19}$F NMR ($CDCl_3$): δ -65.31.

EXAMPLE 15

Preparation of methyl 4,5-dihydro-2-[[4-[5-(trifluoromethyl)-1,2,4-oxadiazol-3-yl]phenyl]methyl]-4-oxazolecarboxylate (Compound 74)

Step A: Preparation of 4-[5-(trifluoromethyl)-1,2,4-oxadiazol-3-yl]benzeneacetic acid

A mixture of 4-cyanobenzeneacetic acid (25.0 g, 155 mmol) and hydroxylamine (50% aqueous solution, 23.8 mL, 780 mmol) in ethanol (500 mL) was heated at reflux overnight. The reaction mixture was concentrated under reduced pressure and the resulting solid was dried in a vacuum oven overnight. The solid (i.e. the intermediate compound 4-[(hydroxyamino)-iminomethyl]benzeneacetic acid) was suspended in tetrahydrofuran (500 mL) and cooled to 0 °C, and then trifluoroacetic anhydride (48 mL, 340 mmol) and triethylamine (47 mL, 340 mmol) were added. The reaction mixture was stirred at room temperature overnight, and then concentrated under reduced. The resulting material was partitioned between water and dichloromethane. The organic layer was separated, dried over sodium sulfate, filtered, and concentrated onto Celite® (diatomaceous filter aid). The Celite® mixture was purified by medium pressure silica gel chromatography (eluting with a gradient of 0 to 100% of ethyl acetate in hexanes) to provide the title compound as a white solid (17.8 g).

$^1$H NMR ($CDCl_3$): δ 8.10 (d, 2H), 7.46 (d, 2H), 3.76 (s, 2H).
$^{19}$F NMR ($CDCl_3$): δ -65.35.

Step B: Preparation of *N*-[2-[4-[5-(trifluoromethyl)-1,2,4-oxadiazol-3-yl]phenyl]acetyl]serine methyl ester

To 4-[5-(trifluoromethyl)-1,2,4-oxadiazol-3-yl]benzeneacetic acid (i.e. the product of Step A) (17.8 g, 65.3 mmol) in *N*,*N*-dimethylformamide (220 mL) was added *DL*-serine methyl ester hydrochloride (1:1) (12.2 g, 78.4 mmol), 1-[bis(dimethylamino)methylene]-1*H*-1,2,3-triazolo[4,5-*b*]pyridinium 3-oxide hexafluorophosphate (HATU) (29.8 g, 78.4 mmol) and 4-methylmorpholine (14.4 mL, 131 mmol). The reaction was stirred at room temperature overnight, and then diluted with water and extracted with ethyl acetate (3x). The combined organic layers were washed with water and saturated aqueous sodium chloride solution, dried over magnesium sulfate, filtered, and concentrated onto Celite® (diatomaceous filter aid). The Celite® mixture was purified by medium pressure silica gel chromatography (eluting with a gradient of 0% to 100% ethyl acetate in hexanes) to provide the title compound.

$^1$H NMR ($CDCl_3$): δ 8.12 (d, 2H), 7.48 (d, 2H), 6.47, (br s, 1H), 4.68 (dt, 1H), 4.00 (dd, 1H), 3.92 (m, 1H), 3.78 (s, 3H), 3.72 (s, 2H).
$^{19}$F NMR ($CDCl_3$): δ -65.34.

Step C: Preparation of methyl 4,5-dihydro-2-[[4-[5-(trifluoromethyl)-1,2,4-oxadiazol-3-yl]phenyl]methyl]-4-oxazolecarboxylate

To *N*-[2-[4-[5-(trifluoromethyl)-1,2,4-oxadiazol-3-yl]phenyl]acetyl]serine methyl ester (i.e. the product of Step B) (65.3 mmol) in dichloromethane (650 mL) at -78 °C was added diethylaminosulfur trifluoride (DAST) (13 mL, 98 mmol). The reaction was stirred for 1.5 h at -78 °C, and then quenched with saturated aqueous sodium bicarbonate solution and the layers were separated. The aqueous layer was further extracted with dichloromethane and the combined organic layers were washed with saturated aqueous sodium bicarbonate solution, dried over magnesium sulfate, filtered, concentrated onto Celite® (diatomaceous filter aid). The Celite® mixture was purified by medium pressure silica gel chromatography (eluting with a gradient of 0% to 100% ethyl acetate in hexanes) to provide the title compound, a compound of the present invention, as a solid (8.17 g).

$^1$H NMR ($CDCl_3$): δ 8.08 (d, 2H), 7.48 (d, 2H), 4.78 (m, 1H), 4.53 (m, 1H), 4.43 (m, 1H), 3.80 (s, 3H), 3.76 (m, 2H).
$^{19}$F NMR ($CDCl_3$): δ -65.33.

EXAMPLE 16

Preparation of 4,5-dihydro-*N,N*-dimethyl-2-[[4-[5-(trifluoromethyl)-1 ,2,4-oxadiazol-3-yl]phenyl]methyl]-4-oxazolecarboxamide (Compound 178)

To a mixture of methyl 4,5-dihydro-2-[[4-[5-(trifluoromethyl)-1,2,4-oxadiazol-3-yl]phenyl]methyl]-4-oxazolecarboxylate (i.e. the product of Example 15, Step C) (0.25 g, 0.7 mmol) in methanol (7 mL) was added *N,N*-dimethylamine (5.6 N in ethanol, 0.63 mL) The reaction mixture was stirred overnight at 65 °C, and then cooled to room temperature and concentrated onto Celite® (diatomaceous filter aid). The Celite® mixture was purified by medium pressure silica gel chromatography (eluting with a gradient of 0% to 100% ethyl acetate in hexanes) to provide the title compound, a compound of the present invention, as a solid (0.009 g).

$^1$H NMR ($CDCl_3$): δ 8.06 (d, 2H), 7.46 (d, 2H), 4.95 (m, 2H), 4.29 (dd, 1H), 3.71 (m, 2H), 3.26 (s, 3H), 3.00 (s, 3H).
$^{19}$F NMR ($CDCl_3$): δ -65.36.

EXAMPLE 17

Preparation of 4,5-dihydro-2-[[4-[5-(trifluoromethyl)-1,2,4-oxadiazol-3-yl]phenyl]methyl]-4-oxazolecarboxamide (Compound 75)

A mixture of methyl 4,5-dihydro-2-[[4-[5-(trifluoromethyl)-1,2,4-oxadiazol-3-yl]phenyl]methyl]-4-oxazolecarboxylate (i.e. the product of Example 15, Step C) (0.5 g, 1.4 mmol) and ammonia (7 N in methanol, 14 mL) was stirred at room temperature overnight. The reaction mixture was concentrated under reduced pressure to provide the title compound, a compound of the present invention, as a solid (0.134 g).

$^1$H NMR ($CDCl_3$): δ 8.10 (d, 2H), 7.46 (d, 2H), 4.68 (m, 2H), 4.52, m, 2H), 3.73 (m, 2H).
$^{19}$F NMR ($CDCl_3$): δ -65.32.

EXAMPLE 18

Preparation of methyl 2-[[4-[5-(trifluoromethyl)-1,2,4-oxadiazol-3-yl]¬phenoxy]¬methyl]oxazole-4-carboxylate (Compound 403)

Step A: Preparation of 4-[5-(trifluoromethyl)-1,2,4-oxadiazol-3-yl]phenol

To a mixture of 4-hydroxybenzonitrile (20 g, 168 mmol) in ethanol (177 mL) was added hydroxylamine (50% aqueous solution,13.4 mL, 440 mmol). The reaction mixture was stirred for 18 h, and then concentrated under reduced pressure. The solid (i.e. the intermediate compound N,4-dihydroxybenzenecarboximidamide) was dissolved in dichloromethane (336 mL) and trifluoroacetic anhydride (47 mL, 336 mmol) was added. The reaction mixture was heated to reflux for 18 h, and then cooled to room temperature and quenched with water. The layers were separated, and the aqueous layer further extracted with dichloromethane two times. The combined organics were washed with saturated aqueous sodium bicarbonate solution and aqueous sodium chloride solution, dried over magnesium sulfate and filtered, and concentrated onto Celite® (diatomaceous filter aid). The Celite® mixture was purified by medium pressure liquid

chromatography (0% to 100% ethyl acetate in hexanes as eluent) to provide the title compound (16.3 g).

$^1$H NMR ($CDCl_3$): δ 8.10 (d, 2H), 6.96 (d, 2H).
$^{19}$F NMR ($CDCl_3$): δ -65.45.

Step B: Preparation of methyl 2-[[4-[5-(trifluoromethyl)-1,2,4-oxadiazol-3-yl]¬phenoxy]¬methyl]oxazole-4-carboxylate

To 4-[5-(trifluoromethyl)-1,2,4-oxadiazol-3-yl]phenol (i.e. the product of Step A) (1.13 g, 5 mmol) in acetonitrile (50 mL) was added methyl 2-(chloromethyl)oxazole-4-carboxylate (0.95 g, 5.4 mmol), potassium carbonate (1.52 g, 11 mmol) and tetrabutylammonium iodide (0.18 g, 0.5 mmol). The reaction mixture was heated to 80 °C for 18 h, and then cooled to room temperature and concentrated onto Celite® (diatomaceous filter aid). The Celite® mixture was purified by medium pressure liquid chromatography (0% to 100% ethyl acetate in hexanes as eluent) to provide the title compound, a compound of the present invention, as a solid (1.14 g).
$^1$H NMR ($CDCl_3$): δ 8.29 (s, 1H), 8.07 (d, 2H), 7.13 (d, 2H), 5.27 (s, 2H), 3.94 (s, 3H). $^{19}$F NMR ($CDCl_3$): δ -65.38.

By the procedures described herein, together with methods known in the art, the following compounds of Tables 1, 1A-92A, 2 and 1B-92B can be prepared. The following abbreviations are used in the Tables: *t* means tertiary, *s* means secondary, *n* means normal, *i* means iso, *c* means cyclo, Me means methyl, Et means ethyl, Pr means propyl, *i*-Pr means isopropyl, *c*-Pr means cyclopropyl, Bu means butyl, *i*-Bu means isobutyl, *t*-Bu means *tert*-butyl, and Ph means phenyl.

Table 1

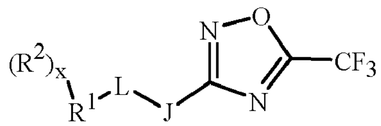

The definitions of $R^1$ and J in Table 1 are as defined Exhibits A and B in the above Embodiments. In the column $R^1$, the number in parentheses following the U-ring number refers to the attachment point of the ring to L. The $(R^2)_x$ column refers to the substituent(s) attached to the U-ring as shown in Exhibit A above. A dash "-" in the $(R^2)_x$ column means that no $R^2$ substituent is present and the remaining valences are occupied by hydrogen atoms.

| L is $CH_2$ and J is J-40. | | L is $CH_2$ and J is J-40. | |
|---|---|---|---|
| $R^1$ | $(R^2)_x$ | $R^1$ | $R^1$ |
| U-1 (4) | - | U-8 (5) | - |
| U-1 (4) | 2-Me | U-8 (5) | 3-Me |
| U-1 (4) | 2-Et | U-8 (5) | 3-Et |
| U-1 (4) | 2-*n*-Pr | U-8 (5) | 3-*n*-Pr |
| U-1 (4) | 2-*i*-Pr | U-8 (5) | 3-*i*-Pr |
| U-1 (4) | 2-*c*-Pr | U-8 (5) | 3-*c*-Pr |
| U-1 (4) | 2-*n*-Bu | U-8 (5) | 3-*n*-Bu |
| U-1 (4) | 2-*i*-Bu | U-8 (5) | 3-*i*-Bu |
| U-1 (4) | 2-*t*-Bu | U-8 (5) | 3-*t*-Bu |
| U-1 (4) | 2-F | U-8 (5) | 3-F |
| U-1 (4) | 2-Cl | U-8 (5) | 3-Cl |
| U-1 (4) | 2-Br | U-8 (5) | 3-Br |
| U-1 (4) | 2-$CF_3$ | U-8 (5) | 3-$CF_3$ |
| L is $CH_2$ and J is J-40. | | L is $CH_2$ and J is J-40. | |
| $R^1$ | $(R^2)_x$ | $R^1$ | $R^1$ |
| U-1 (4) | 2-HO | U-8 (5) | 3-HO |
| U-1 (4) | 2-N≡C | U-8 (5) | 3-N≡C |
| U-1 (4) | 2-N≡$CCH_2$ | U-8 (5) | 3-N≡$CCH_2$ |
| U-1 (4) | 2-(MeO) | U-8 (5) | 3-(MeO) |
| U-1 (4) | 2-($MeOCH_2$) | U-8 (5) | 3-($MeOCH_2$) |
| U-1 (4) | 2-($EtOCH_2$) | U-8 (5) | 3-($EtOCH_2$) |

(continued)

| L is $CH_2$ and J is J-40. | | L is $CH_2$ and J is J-40. | |
|---|---|---|---|
| $R^1$ | $(R^2)_x$ | $R^1$ | $R^1$ |
| U-1 (4) | 2-(CH(=O)) | U-8 (5) | 3-(CH(=O)) |
| U-1 (4) | 2-(HOC(=O)) | U-8 (5) | 3-(HOC(=O)) |
| U-1 (4) | 2-(MeOC(=O)) | U-8 (5) | 3-(MeOC(=O)) |
| U-1 (4) | 2-(EtOC(=O)) | U-8 (5) | 3-(EtOC(=O)) |
| U-1 (4) | 2-(*i*-PrOC(=O)) | U-8 (5) | 3-(*i*-PtOC(=O)) |
| U-1 (4) | 2-(*n*-PrOC(=0)) | U-8 (5) | 3-(*n*-PrOC(=O)) |
| U-1 (4) | 2-(BuOC(=O)) | U-8 (5) | 4-(BuOC(=O)) |
| U-1 (4) | 2-(*i*-BuOC(=O)) | U-8 (5) | 3-(*i*-BuOC(=O)) |
| U-1 (4) | 2-(*t*-BuOC(=O)) | U-8 (5) | 3-(*t*-BuOC(=O)) |
| U-1 (4) | 2-($CF_3CH_2OC$(=O) | U-8 (5) | 3-($CF_3CH_2OC$(=O) |
| U-1 (4) | 2-($CH_2$=CHOC(=O)) | U-8 (5) | 3-($CH_2$=CHOC(=O)) |
| U-1 (4) | 2-($CH_2$=$CHCH_2OC$(=O)) | U-8 (5) | 3-($CH_2$=$CHCH_2OC$(=O)) |
| U-1 (4) | 2-($CH_2$=$CBrCH_2OC$(=O)) | U-8 (5) | 3-($CH_2$=$CBrCH_2OC$(=O)) |
| U-1 (4) | 2-($CH_2$=$CHCF_2OC$(=O)) | U-8 (5) | 3-($CH_2$=$CHCF_2OC$(=O)) |
| U-1 (4) | 2-($Me_2C$=$CHCH_2OC$(=O)) | U-8 (5) | 3-($Me_2C$=$CHCH_2OC$(=O)) |
| U-1 (4) | 2-($CH_2$=C(Me)$CH_2OC$(=O)) | U-8 (5) | 3-($CH_2$=C(Me)$CH_2OC$(=O)) |
| U-1 (4) | 2-(CH≡$CCH_2OC$(=O)) | U-8 (5) | 3-(CH≡$CCH_2OC$(=O)) |
| U-1 (4) | 2-(N≡$CCH_2OC$(=O)) | U-8 (5) | 3-(N≡$CCH_2OC$(=O)) |
| U-1 (4) | 2-(MeNHC(=O)) | U-8 (5) | 3-(MeNHC(=O)) |
| U-1 (4) | 2-($Me_2NC$(=O)) | U-8 (5) | 3-($Me_2NC$(=O)) |
| U-1 (4) | 2-(MeNHC(=O)) | U-8 (5) | 3-(MeNHC(=O)) |
| U-1 (4) | 2-(EtNHC(=O)) | U-8 (5) | 3-(EtNHC(=O)) |
| U-1 (4) | 2-(PrNHC(=O)) | U-8 (5) | 3-(PrNHC(=O)) |
| U-1 (4) | 2-(*i*-PrNHC(=O)) | U-8 (5) | 3-(*i*-PrNHC(=O)) |
| U-1 (4) | 2-(BuNHC(=O)) | U-8 (5) | 3-(BuNHC(=O)) |
| U-1 (4) | 2-(*t*-PuNHC(=O)) | U-8 (5) | 3-(*t*-BuNHC(=O)) |
| U-1 (4) | 2-(*i*-BuNHC(=O)) | U-8 (5) | 3-(*i*-BuNHC(=O)) |
| U-1 (4) | 2-($CF_3CH_2NHC$(=O)) | U-8 (5) | 3-($CF_3CH_2NHC$(=O)) |
| U-1 (4) | 2-(*c*-$PrCH_2NHC$(=O)) | U-8 (5) | 3-(*c*-$PrCH_2NHC$(=O)) |
| U-1 (4) | 2-($MeOCH_2NHC$(=O)) | U-8 (5) | 3-($MeOCH_2NHC$(=O)) |
| U-1 (4) | 2-($MeOCH_2CH_2NHC$(=O)) | U-8 (5) | 3-($MeOCH_2CH_2NHC$(=O)) |
| U-1 (4) | 2-($CH_2$=$CHCH_2NHC$(=O)) | U-8 (5) | 3-($CH_2$=$CHCH_2NHC$(=O)) |
| U-1 (4) | 2-(N≡$CCH_2NHC$(=O)) | U-8 (5) | 3-(N≡$CCH_2NHC$(=O)) |
| U-1 (4) | 2-(OH-N=CH) | U-8 (5) | 3-(OH-N=CH) |
| U-1 (4) | 2-($Me_2NN$=CH) | U-8 (5) | 3-($Me_2NN$=CH) |
| U-1 (4) | 2-(MeOC(=O)NHN=CH) | U-8 (5) | 3-(MeOC(=O)NHN=CH) |
| U-1 (4) | 2-(OHC(=O)$CH_2ON$=CH) | U-8 (5) | 3-(OHC(=O)$CH_2ON$=CH) |
| U-1 (2) | - | U-12 (3) | 1-Me |
| U-1 (2) | 4-Me | U-12 (3) | 1,5-di-Me |
| U-1 (2) | 4-Et | U-12 (3) | 1-Me, 5-Et |
| U-1 (2) | 4-*n*-Pr | U-12 (3) | 1-Me, 5-*n*-Pr |
| U-1 (2) | 4-*i*-Pr | U-12 (3) | 1-Me, 5-*i*-Pr |
| U-1 (2) | 4-*c*-Pr | U-12 (3) | 1-Me, 5-*c*-Pr |
| U-1 (2) | 4-*n*-Bu | U-12 (3) | 1-Me, 5-*n*-Bu |
| U-1 (2) | 4-*i*-Bu | U-12 (3) | 1-Me, 5-*i*-Bu |
| U-1 (2) | 4-*t*-Bu | U-12 (3) | 1-Me, 5-*t*-Bu |

(continued)

| L is $CH_2$ and J is J-40. | | L is $CH_2$ and J is J-40. | |
|---|---|---|---|
| $R^1$ | $(R^2)_x$ | $R^1$ | $R^1$ |
| U-1 (2) | 4-F | U-12 (3) | 1-Me, 5-F |
| U-1 (2) | 4-Cl | U-12 (3) | 1-Me, 5-Cl |
| U-1 (2) | 4-Br | U-12 (3) | 1-Me, 5-Br |
| U-1 (2) | $4-CF_3$ | U-12 (3) | 1-Me, $5-CF_3$ |
| U-1 (2) | 4-HO | U-12 (3) | 1-Me, 5-HO |
| U-1 (2) | 4-N≡C | U-12 (3) | 1-Me, 5-N≡C |
| U-1 (2) | $4-N{\equiv}CCH_2$ | U-12 (3) | 1-Me, $5-N{\equiv}CCH_2$ |
| U-1 (2) | 4-(MeO) | U-12 (3) | 1-Me, 5-(MeO) |
| U-1 (2) | $4-(MeOCH_2)$ | U-12 (3) | 1-Me, $5-(MeOCH_2)$ |
| U-1 (2) | $4-(EtOCH_2)$ | U-12 (3) | 1-Me, $5-(EtOCH_2)$ |
| U-1 (2) | 4-(CH(=O)) | U-12 (3) | 1-Me, 5-(CH(=O)) |
| U-1 (2) | 4-(HOC(=O)) | U-12 (3) | 1-Me, 5-(HOC(=O)) |
| U-1 (2) | 4-(MeOC(=O)) | U-12 (3) | 1-Me, 5-(MeOC(=O)) |
| U-1 (2) | 4-(EtOC(=O)) | U-12 (3) | 1-Me, 5-(EtOC(=O)) |
| U-1 (2) | 4-(*i*-PtOC(=O)) | U-12 (3) | 1-Me, 5-(*i*-PrOC(=O)) |
| U-1 (2) | 4-(*n*-PtOC(=O)) | U-12 (3) | 1-Me, 5-(*n*-PrOC(=O)) |
| U-1 (2) | 4-(BuOC(=O)) | U-12 (3) | 1-Me, 5-(BuOC(=O)) |
| U-1 (2) | 4-(*i*-BuOC(=O)) | U-12 (3) | 1-Me, 5-(*i*-BuOC(=O)) |
| U-1 (2) | 4-(*t*-BuOC(=O)) | U-12 (3) | 1-Me, 5-(*t*-BuOC(=O)) |
| U-1 (2) | $4-(CF_3CH_2OC(=O)$ | U-12 (3) | 1-Me, $5-(CF_3CH_2OC(=O)$ |
| U-1 (2) | $4-(CH_2=CHOC(=O))$ | U-12 (3) | 1-Me, $5-(CH_2=CHOC(=O))$ |
| U-1 (2) | $4-(CH_2=CHCH_2OC(=O))$ | U-12 (3) | 1-Me, $5-(CH_2=CHCH_2OC(=O))$ |
| U-1 (2) | $4-(CH_2=CBrCH_2OC(=O))$ | U-12 (3) | 1-Me, $5-(CH_2=CBrCH_2OC(=O))$ |
| U-1 (2) | $4-(CH_2=CHCF_2OC(=O))$ | U-12 (3) | 1-Me, $5-(CH_2=CHCF_2OC(=O))$ |
| U-1 (2) | $4-(Me_2C=CHCH_2OC(=O))$ | U-12 (3) | 1-Me, $5-(Me_2C=CHCH_2OC(=O))$ |
| U-1 (2) | $4-(CH_2=C(Me)CH_2OC(=O))$ | U-12 (3) | 1-Me, $5-(CH_2=C(Me)CH_2OC(=O))$ |
| U-1 (2) | $4-(CH{\equiv}CCH_2OC(=O))$ | U-12 (3) | 1-Me, $5-(CH{\equiv}CCH_2OC(=O))$ |
| U-1 (2) | $4-(N{\equiv}CCH_2OC(=O))$ | U-12 (3) | 1-Me, $5-(N{\equiv}CCH_2OC(=O))$ |
| U-1 (2) | 4-(MeNHC(=O)) | U-12 (3) | 1-Me, 5-(MeNHC(=O)) |
| U-1 (2) | $4-(Me_2NC(=O))$ | U-12 (3) | 1-Me, $5-(Me_2NC(=O))$ |
| U-1 (2) | 4-(MeNHC(=O)) | U-12 (3) | 1-Me, 5-(MeNHC(=O)) |
| U-1 (2) | 4-(EtNHC(=O)) | U-12 (3) | 1-Me, 5-(EtNHC(=O)) |
| U-1 (2) | 4-(PrNHC(=O)) | U-12 (3) | 1-Me, 5-(PrNHC(=O)) |
| U-1 (2) | 4-(*i*-PrNHC(=O)) | U-12 (3) | 1-Me, 5-(*i*-PrNHC(=O)) |
| U-1 (2) | 4-(BuNHC(=O)) | U-12 (3) | 1-Me, 5-(BuNHC(=O)) |
| U-1 (2) | 4-(*t*-BuNHC(=O)) | U-12 (3) | 1-Me, 5-(*t*-BuNHC(=O)) |
| U-1 (2) | 4-(*i*-BuNHC(=O)) | U-12 (3) | 1-Me, 5-(*i*-BuNHC(=O)) |
| U-1 (2) | $4-(CF_3CH_2NHC(=O))$ | U-12 (3) | 1-Me, $5-(CF_3CH_2NHC(=O))$ |
| U-1 (2) | 4-(*c*-$PrCH_2NHC(=O))$ | U-12 (3) | 1-Me, 5-(*c*-$PrCH_2NHC(=O))$ |
| U-1 (2) | $4-(MeOCH_2NHC(=O))$ | U-12 (3) | 1-Me, $5-(MeOCH_2NHC(=O))$ |
| U-1 (2) | $4-(MeOCH_2CH_2NHC(=O))$ | U-12 (3) | 1-Me, $5-(MeOCH_2CH_2NHC(=O))$ |
| U-1 (2) | $4-(CH_2=CHCH_2NHC(=O))$ | U-12 (3) | 1-Me, $5-(CH_2=CHCH_2NHC(=O))$ |
| U-1 (2) | $4-(N{\equiv}CCH_2NHC(=O))$ | U-12 (3) | 1-Me, $5-(N{\equiv}CCH_2NHC(=O))$ |
| U-1 (2) | 4-(OH-N=CH) | U-12 (3) | 1-Me, 5-(OH-N=CH) |

(continued)

| L is $CH_2$ and J is J-40. | | L is $CH_2$ and J is J-40. | |
|---|---|---|---|
| $R^1$ | $(R^2)_x$ | $R^1$ | $R^1$ |
| U-1 (2) | 4-($Me_2NN=CH$) | U-12 (3) | 1-Me, 5-($Me_2NN=CH$) |
| U-1 (2) | 4-(MeOC(=O)NHN=CH) | U-12 (3) | 1-Me, 5-(MeOC(=O)NHN=CH) |
| U-1 (2) | 4-(OHC(=O)$CH_2$ON=CH) | U-12 (3) | 1-Me, 5-(OHC(=O)$CH_2$ON=CH) |
| U-2 (2) | - | U-12 (1) | - |
| U-2 (2) | 4-Me | U-12 (1) | 4-Me |
| U-2 (2) | 4-Et | U-12 (1) | 4-Et |
| U-2 (2) | 4-*n*-Pr | U-12 (1) | 4-*n*-Pr |
| U-2 (2) | 4-*i*-Pr | U-12 (1) | 4-*i*-Pr |
| U-2 (2) | 4-*c*-Pr | U-12 (1) | 4-*c*-Pr |
| U-2 (2) | 4-*n*-Bu | U-12 (1) | 4-*n*-Bu |
| U-2 (2) | 4-*i*-Bu | U-12 (1) | 4-*i*-Bu |
| U-2 (2) | 4-*t*-Bu | U-12 (1) | 4-*t*-Bu |
| U-2 (2) | 4-F | U-12 (1) | 4-F |
| U-2 (2) | 4-Cl | U-12 (1) | 4-Cl |
| U-2 (2) | 4-Br | U-12 (1) | 4-Br |
| U-2 (2) | 4-$CF_3$ | U-12 (1) | 4-$CF_3$ |
| U-2 (2) | 4-HO | U-12 (1) | 4-HO |
| U-2 (2) | 4-N≡C | U-12 (1) | 4-N≡C |
| U-2 (2) | 4-N≡$CCH_2$ | U-12 (1) | 4-N≡$CCH_2$ |
| U-2 (2) | 4-(MeO) | U-12 (1) | 4-(MeO) |
| U-2 (2) | 4-($MeOCH_2$) | U-12 (1) | 4-($MeOCH_2$) |
| U-2 (2) | 4-($EtOCH_2$) | U-12 (1) | 4-($EtOCH_2$) |
| U-2 (2) | 4-(CH(=O)) | U-12 (1) | 4-(CH(=O)) |
| U-2 (2) | 4-(HOC(=O)) | U-12 (1) | 4-(HOC(=O)) |
| U-2 (2) | 4-(MeOC(=O)) | U-12 (1) | 4-(MeOC(=O)) |
| U-2 (2) | 4-(EtOC(=O)) | U-12 (1) | 4-(EtOC(=O)) |
| U-2 (2) | 4-(*i*-PtOC(=O)) | U-12 (1) | 4-(*i*-PtOC(=O)) |
| U-2 (2) | 4-(*n*-PtOC(=O)) | U-12 (1) | 4-(*n*-PtOC(=O)) |
| U-2 (2) | 4-(BuOC(=O)) | U-12 (1) | 4-(BuOC(=O)) |
| U-2 (2) | 4-(*i*-BuOC(=O)) | U-12 (1) | 4-(*i*-BuOC(=O)) |
| U-2 (2) | 4-(*t*-BuOC(=O)) | U-12 (1) | 4-(*t*-BuOC(=O)) |
| U-2 (2) | 4-($CF_3CH_2OC$(=O) | U-12 (1) | 4-($CF_3CH_2OC$(=O) |
| U-2 (2) | 4-($CH_2$=CHOC(=O)) | U-12 (1) | 4-($CH_2$=CHOC(=O)) |
| U-2 (2) | 4-($CH_2=CHCH_2OC$(=O)) | U-12 (1) | 4-($CH_2=CHCH_2OC$(=O)) |
| U-2 (2) | 4-($CH_2=CBrCH_2OC$(=O)) | U-12 (1) | 4-($CH_2=CBrCH_2OC$(=O)) |
| U-2 (2) | 4-($CH_2=CHCF_2OC$(=O)) | U-12 (1) | 4-($CH_2=CHCF_2OC$(=O)) |
| U-2 (2) | 4-($Me_2C=CHCH_2OC$(=O)) | U-12 (1) | 4-($Me_2C=CHCH_2OC$(=O)) |
| U-2 (2) | 4-($CH_2$=C(Me)$CH_2$OC(=O)) | U-12 (1) | 4-($CH_2$=C(Me)$CH_2$OC(=O)) |
| U-2 (2) | 4-(CH≡$CCH_2OC$(=O)) | U-12 (1) | 4-(CH≡$CCH_2OC$(=O)) |
| U-2 (2) | 4-(N≡$CCH_2OC$(=O)) | U-12 (1) | 4-(N≡$CCH_2OC$(=O)) |
| U-2 (2) | 4-(MeNHC(=O)) | U-12 (1) | 4-(MeNHC(=O)) |
| U-2 (2) | 4-($Me_2NC$(=O)) | U-12 (1) | 4-($Me_2NC$(=O)) |
| U-2 (2) | 4-(MeNHC(=O)) | U-12 (1) | 4-(MeNHC(=O)) |
| U-2 (2) | 4-(EtNHC(=O)) | U-12 (1) | 4-(EtNHC(=O)) |
| U-2 (2) | 4-(PrNHC(=O)) | U-12 (1) | 4-(PrNHC(=O)) |
| U-2 (2) | 4-(*i*-PrNHC(=O)) | U-12 (1) | 4-(*i*-PrNHC(=O)) |

(continued)

| L is $CH_2$ and J is J-40. | | L is $CH_2$ and J is J-40. | |
|---|---|---|---|
| $R^1$ | $(R^2)_x$ | $R^1$ | $R^1$ |
| U-2 (2) | 4-(BuNHC(=O)) | U-12 (1) | 4-(BuNHC(=O)) |
| U-2 (2) | 4-(*t*-BuNHC(=O)) | U-12 (1) | 4-(*t*-BuNHC(=O)) |
| U-2 (2) | 4-(*i*-BuNHC(=O)) | U-12 (1) | 4-(*i*-BuNHC(=O)) |
| U-2 (2) | 4-($CF_3CH_2$NHC(=O)) | U-12 (1) | 4-($CF_3CH_2$NHC(=O)) |
| U-2 (2) | 4-(*c*-Pr$CH_2$NHC(=O)) | U-12 (1) | 4-(*c*-Pr$CH_2$NHC(=O)) |
| U-2 (2) | 4-(MeO$CH_2$NHC(=O)) | U-12 (1) | 4-(MeO$CH_2$NHC(=O)) |
| U-2 (2) | 4-(MeO$CH_2CH_2$NHC(=O)) | U-12 (1) | 4-(MeO$CH_2CH_2$NHC(=O)) |
| U-2 (2) | 4-($CH_2$=CH$CH_2$NHC(=O)) | U-12 (1) | 4-($CH_2$=CH$CH_2$NHC(=O)) |
| U-2 (2) | 4-(N≡C$CH_2$NHC(=O)) | U-12 (1) | 4-(N≡C$CH_2$NHC(=O)) |
| U-2 (2) | 4-(OH-N=CH) | U-12 (1) | 4-(OH-N=CH) |
| U-2 (2) | 4-($Me_2$NN=CH) | U-12 (1) | 4-($Me_2$NN=CH) |
| U-2 (2) | 4-(MeOC(=O)NHN=CH) | U-12 (1) | 4-(MeOC(=O)NHN=CH) |
| U-2 (2) | 4-(OHC(=O)$CH_2$ON=CH) | U-12 (1) | 4-(OHC(=O)$CH_2$ON=CH) |
| U-2 (4) | - | U-69 (1) | - |
| U-2 (4) | 2-Me | U-69 (1) | 4-Me |
| U-2 (4) | 2-Et | U-69 (1) | 4-Et |
| U-2 (4) | 2-*n*-Pr | U-69 (1) | 4-*n*-Pr |
| U-2 (4) | 2-*i*-Pr | U-69 (1) | 4-*i*-Pr |
| U-2 (4) | 2-*c*-Pr | U-69 (1) | 4-*c*-Pr |
| U-2 (4) | 2-*n*-Bu | U-69 (1) | 4-*n*-Bu |
| U-2 (4) | 2-*i*-Bu | U-69 (1) | 4-*i*-Bu |
| U-2 (4) | 2-*t*-Bu | U-69 (1) | 4-*t*-Bu |
| U-2 (4) | 2-F | U-69 (1) | 4-F |
| U-2 (4) | 2-Cl | U-69 (1) | 4-Cl |
| U-2 (4) | 2-Br | U-69 (1) | 4-Br |
| U-2 (4) | 2-$CF_3$ | U-69 (1) | 4-$CF_3$ |
| U-2 (4) | 2-HO | U-69 (1) | 4-HO |
| U-2 (4) | 2-N≡C | U-69 (1) | 4-N≡C |
| U-2 (4) | 2-N≡C$CH_2$ | U-69 (1) | 4-N≡C$CH_2$ |
| U-2 (4) | 2-(MeO) | U-69 (1) | 4-(MeO) |
| U-2 (4) | 2-(MeO$CH_2$) | U-69 (1) | 4-(MeO$CH_2$) |
| U-2 (4) | 2-(EtO$CH_2$) | U-69 (1) | 4-(EtO$CH_2$) |
| U-2 (4) | 2-(CH(=O)) | U-69 (1) | 4-(CH(=O)) |
| U-2 (4) | 2-(HOC(=O)) | U-69 (1) | 4-(HOC(=O)) |
| U-2 (4) | 2-(MeOC(=O)) | U-69 (1) | 4-(MeOC(=O)) |
| U-2 (4) | 2-(EtOC(=O)) | U-69 (1) | 4-(EtOC(=O)) |
| U-2 (4) | 2-(*i*-PrOC(=O)) | U-69 (1) | 4-(*i*-PtOC(=O)) |
| U-2 (4) | 2-(*n*-PrOC(=0)) | U-69 (1) | 4-(*n*-PtOC(=O)) |
| U-2 (4) | 2-(BuOC(=O)) | U-69 (1) | 4-(BuOC(=O)) |
| U-2 (4) | 2-(*i*-BuOC(=O)) | U-69 (1) | 4-(*i*-BuOC(=O)) |
| U-2 (4) | 2-(*t*-BuOC(=O)) | U-69 (1) | 4-(*t*-BuOC(=O)) |
| U-2 (4) | 2-($CF_3CH_2$OC(=O) | U-69 (1) | 4-($CF_3CH_2$OC(=O) |
| U-2 (4) | 2-($CH_2$=CHOC(=O)) | U-69 (1) | 4-($CH_2$=CHOC(=O)) |
| U-2 (4) | 2-($CH_2$=CH$CH_2$OC(=O)) | U-69 (1) | 4-($CH_2$=CH$CH_2$OC(=O)) |
| U-2 (4) | 2-($CH_2$=CBr$CH_2$OC(=O)) | U-69 (1) | 4-($CH_2$=CBr$CH_2$OC(=O)) |
| U-2 (4) | 2-($CH_2$=CH$CF_2$OC(=O)) | U-69 (1) | 4-($CH_2$=CH$CF_2$OC(=O)) |

(continued)

| L is $CH_2$ and J is J-40. | | L is $CH_2$ and J is J-40. | |
|---|---|---|---|
| $R^1$ | $(R^2)_x$ | $R^1$ | $R^1$ |
| U-2 (4) | 2-($Me_2C$=$CHCH_2OC$(=O)) | U-69 (1) | 4-($Me_2C$=$CHCH_2OC$(=O)) |
| U-2 (4) | 2-($CH_2$=C(Me)$CH_2OC$(=O)) | U-69 (1) | 4-($CH_2$=C(Me)$CH_2OC$(=O)) |
| U-2 (4) | 2-(CH≡$CCH_2OC$(=O)) | U-69 (1) | 4-(CH≡$CCH_2OC$(=O)) |
| U-2 (4) | 2-(N≡$CCH_2OC$(=O)) | U-69 (1) | 4-(N≡$CCH_2OC$(=O)) |
| U-2 (4) | 2-(MeNHC(=O)) | U-69 (1) | 4-(MeNHC(=O)) |
| U-2 (4) | 2-($Me_2NC$(=O)) | U-69 (1) | 4-($Me_2NC$(=O)) |
| U-2 (4) | 2-(MeNHC(=O)) | U-69 (1) | 4-(MeNHC(=O)) |
| U-2 (4) | 2-(EtNHC(=O)) | U-69 (1) | 4-(EtNHC(=O)) |
| U-2 (4) | 2-(PrNHC(=O)) | U-69 (1) | 4-(PrNHC(=O)) |
| U-2 (4) | 2-(*i*-PrNHC(=O)) | U-69 (1) | 4-(*i*-PrNHC(=O)) |
| U-2 (4) | 2-(BuNHC(=O)) | U-69 (1) | 4-(BuNHC(=O)) |
| U-2 (4) | 2-(*t*-BuNHC(=O)) | U-69 (1) | 4-(*t*-BuNHC(=O)) |
| U-2 (4) | 2-(*i*-BuNHC(=O)) | U-69 (1) | 4-(*i*-BuNHC(=O)) |
| U-2 (4) | 2-($CF_3CH_2NHC$(=O)) | U-69 (1) | 4-($CF_3CH_2NHC$(=O)) |
| U-2 (4) | 2-(*c*-$PrCH_2NHC$(=O)) | U-69 (1) | 4-(*c*-$PrCH_2NHC$(=O)) |
| U-2 (4) | 2-($MeOCH_2NHC$(=O)) | U-69 (1) | 4-($MeOCH_2NHC$(=O)) |
| U-2 (4) | 2-($MeOCH_2CH_2NHC$(=O)) | U-69 (1) | 4-($MeOCH_2CH_2NHC$(=O)) |
| U-2 (4) | 2-($CH_2$=$CHCH_2NHC$(=O)) | U-69 (1) | 4-($CH_2$=$CHCH_2NHC$(=O)) |
| U-2 (4) | 2-(N≡$CCH_2NHC$(=O)) | U-69 (1) | 4-(N≡$CCH_2NHC$(=O)) |
| U-2 (4) | 2-(OH-N=CH) | U-69 (1) | 4-(OH-N=CH) |
| U-2 (4) | 2-($Me_2NN$=CH) | U-69 (1) | 4-($Me_2NN$=CH) |
| U-2 (4) | 2-(MeOC(=O)NHN=CH) | U-69 (1) | 4-(MeOC(=O)NHN=CH) |
| U-2 (4) | 2-(OHC(=O)$CH_2ON$=CH) | U-69 (1) | 4-(OHC(=O)$CH_2ON$=CH) |

The present disclosure also includes Tables 1A through 47A, each of which is constructed the same as Table 1 above, except that the row heading in Table 1 (i.e. "L is $CH_2$ and J is J-40") is replaced with the respective row heading shown below.

| Table | Row Heading | Table | Row Heading |
|---|---|---|---|
| 1A | L is $CH_2CH_2$ and J is J-40. | 25A | L is $OCH_2$ and J is J-27. |
| 2A | L is $CH_2$(Me) and J is J-40. | 26A | L is $CH_2O$ and J is J-27. |
| 3A | L is $CH_2CH_2CH_2$ and J is J-40. | 27A | L is $CH_2OCH_2$ and J is J-27. |
| 4A | L is $OCH_2$ and J is J-40. | 28A | L is $CH_2$ and J is J-63. |
| 5A | L is $CH_2O$ and J is J-40. | 29A | L is $CH_2CH_2$ and J is J-63. |
| 6A | L is $CH_2OCH_2$ and J is J-40. | 30A | L is $CH_2$(Me) and J is J-63. |
| 7A | L is $CH_2$ and J is J-4. | 31A | L is $CH_2CH_2CH_2$ and J is J-63. |
| 8A | L is $CH_2CH_2$ and J is J-4. | 32A | L is $OCH_2$ and J is J-63. |
| 9A | L is $CH_2$(Me) and J is J-4. | 33A | L is $CH_2O$ and J is J-63. |
| 10A | L is $CH_2CH_2CH_2$ and J is J-4. | 34A | L is $CH_2OCH_2$ and J is J-63. |
| 11A | L is $OCH_2$ and J is J-4. | 35A | L is $CH_2$ and J is J-73. |
| 12A | L is $CH_2O$ and J is J-4. | 36A | L is $CH_2CH_2$ and J is J-73. |
| 13A | L is $CH_2OCH_2$ and J is J-4. | 37A | L is $CH_2$(Me) and J is J-73. |
| 14A | L is $CH_2$ and J is J-18. | 38A | L is $CH_2CH_2CH_2$ and J is J-73. |
| 15A | L is $CH_2CH_2$ and J is J-18. | 39A | L is $OCH_2$ and J is J-73. |

(continued)

| Table | Row Heading | Table | Row Heading |
|---|---|---|---|
| 16A | L is $CH_2(Me)$ and J is J-18. | 40A | L is $CH_2O$ and J is J-73. |
| 17A | L is $CH_2CH_2CH_2$ and J is J-18. | 41A | L is $CH_2OCH_2$ and J is J-73. |
| 18A | L is $OCH_2$ and J is J-18. | 42A | L is $CH_2$ and J is J-93. |
| 19A | L is $CH_2O$ and J is J-18. | 43A | L is $CH_2CH_2$ and J is J-93. |
| 20A | L is $CH_2OCH_2$ and J is J-18. | 44A | L is $CH_2(Me)$ and J is J-93. |
| 21A | L is $CH_2$ and J is J-27. | 45A | L is $CH_2CH_2CH_2$ and J is J-93. |
| 22A | L is $CH_2CH_2$ and J is J-27. | 46A | L is $OCH_2$ and J is J-93. |
| 23A | L is $CH_2(Me)$ and J is J-27. | 47A | L is $CH_2O$ and J is J-93. |
| 24A | L is $CH_2CH_2CH_2$ and J is J-27. | 48A | L is $CH_2OCH_2$ and J is J-93. |

Table 2 discloses specific compounds Formula **3** which are useful as process intermediates for preparing compounds of Formula **1,** as described in Schemes 2 and 10 above.

TABLE 2

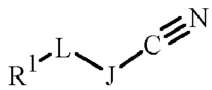

**3**

The definitions of $R^1$ and J in Table 2 are as defined Exhibits A and B in the above Embodiments. In the column $R^1$, the number in parentheses following the U-ring refers to the attachment point of the ring to L. The $(R^2)_x$ column refers to the substituent(s) attached to the U-ring as shown in Exhibit A above. A dash "-" in the $(R^2)_x$ column means that no $R^2$ substituent is present and the remaining valences are occupied by hydrogen atoms.

| L is $CH_2$ and J is J-40. | | L is $CH_2$ and J is J-40. | |
|---|---|---|---|
| $R^1$ | $(R^2)_x$ | $R^1$ | $R^1$ |
| U-1 (4) | - | U-8 (5) | - |
| U-1 (4) | 2-Me | U-8 (5) | 3-Me |
| U-1 (4) | 2-Et | U-8 (5) | 3-Et |
| U-1 (4) | 2-*n*-Pr | U-8 (5) | 3-*n*-Pr |
| U-1 (4) | 2-*i*-Pr | U-8 (5) | 3-*i*-Pr |
| U-1 (4) | 2-*c*-Pr | U-8 (5) | 3-*c*-Pr |
| U-1 (4) | 2-*n*-Bu | U-8 (5) | 3-*n*-Bu |
| U-1 (4) | 2-*i*-Bu | U-8 (5) | 3-*i*-Bu |
| U-1 (4) | 2-*t*-Bu | U-8 (5) | 3-*t*-Bu |
| U-1 (4) | 2-F | U-8 (5) | 3-F |
| U-1 (4) | 2-Cl | U-8 (5) | 3-Cl |
| L is $CH_2$ and J is J-40. | | L is $CH_2$ and J is J-40. | |
| $R^1$ | $(R^2)_x$ | $R^1$ | $R^1$ |
| U-1 (4) | 2-Br | U-8 (5) | 3-Br |
| U-1 (4) | 2-$CF_3$ | U-8 (5) | 3-$CF_3$ |
| U-1 (4) | 2-HO | U-8 (5) | 3-HO |
| U-1 (4) | 2-N≡C | U-8 (5) | 3-N≡C |
| U-1 (4) | 2-N≡$CCH_2$ | U-8 (5) | 3-N≡$CCH_2$ |
| U-1 (4) | 2-(MeO) | U-8 (5) | 3-(MeO) |
| U-1 (4) | 2-($MeOCH_2$) | U-8 (5) | 3-($MeOCH_2$) |
| U-1 (4) | 2-($EtOCH_2$) | U-8 (5) | 3-($EtOCH_2$) |

(continued)

| L is CH$_2$ and J is J-40. | | L is CH$_2$ and J is J-40. | |
|---|---|---|---|
| $R^1$ | $(R^2)_x$ | $R^1$ | $R^1$ |
| U-1 (4) | 2-(CH(=O)) | U-8 (5) | 3-(CH(=O)) |
| U-1 (4) | 2-(HOC(=O)) | U-8 (5) | 3-(HOC(=O)) |
| U-1 (4) | 2-(MeOC(=O)) | U-8 (5) | 3-(MeOC(=O)) |
| U-1 (4) | 2-(EtOC(=O)) | U-8 (5) | 3-(EtOC(=O)) |
| U-1 (4) | 2-(*i*-PrOC(=O)) | U-8 (5) | 3-(*i*-PrOC(=O)) |
| U-1 (4) | 2-(*n*-PrOC(=O)) | U-8 (5) | 3-(*n*-PrOC(=O)) |
| U-1 (4) | 2-(BuOC(=O)) | U-8 (5) | 4-(BuOC(=O)) |
| U-1 (4) | 2-(*i*-BuOC(=O)) | U-8 (5) | 3-(*i*-BuOC(=O)) |
| U-1 (4) | 2-(*t*-BuOC(=O)) | U-8 (5) | 3-(*t*-BuOC(=O)) |
| U-1 (4) | 2-(CF$_3$CH$_2$OC(=O) | U-8 (5) | 3-(CF$_3$CH$_2$OC(=O) |
| U-1 (4) | 2-(CH$_2$=CHOC(=O)) | U-8 (5) | 3-(CH$_2$=CHOC(=O)) |
| U-1 (4) | 2-(CH$_2$=CHCH$_2$OC(=O)) | U-8 (5) | 3-(CH$_2$=CHCH$_2$OC(=O)) |
| U-1 (4) | 2-(CH$_2$=CBrCH$_2$OC(=O)) | U-8 (5) | 3-(CH$_2$=CBrCH$_2$OC(=O)) |
| U-1 (4) | 2-(CH$_2$=CHCF$_2$OC(=O)) | U-8 (5) | 3-(CH$_2$=CHCF$_2$OC(=O)) |
| U-1 (4) | 2-(Me$_2$C=CHCH$_2$OC(=O)) | U-8 (5) | 3-(Me$_2$C=CHCH$_2$OC(=O)) |
| U-1 (4) | 2-(CH$_2$=C(Me)CH$_2$OC(=O)) | U-8 (5) | 3-(CH$_2$=C(Me)CH$_2$OC(=O)) |
| U-1 (4) | 2-(CH≡CCH$_2$OC(=O)) | U-8 (5) | 3-(CH≡CCH$_2$OC(=O)) |
| U-1 (4) | 2-(N≡CCH$_2$OC(=O)) | U-8 (5) | 3-(N≡CCH$_2$OC(=O)) |
| U-1 (4) | 2-(MeNHC(=O)) | U-8 (5) | 3-(MeNHC(=O)) |
| U-1 (4) | 2-(Me$_2$NC(=O)) | U-8 (5) | 3-(Me$_2$NC(=O)) |
| U-1 (4) | 2-(MeNHC(=O)) | U-8 (5) | 3-(MeNHC(=O)) |
| U-1 (4) | 2-(EtNHC(=O)) | U-8 (5) | 3-(EtNHC(=O)) |
| U-1 (4) | 2-(PrNHC(=O)) | U-8 (5) | 3-(PrNHC(=O)) |
| U-1 (4) | 2-(*i*-PrNHC(=O)) | U-8 (5) | 3-(*i*-PrNHC(=O)) |
| U-1 (4) | 2-(BuNHC(=O)) | U-8 (5) | 3-(BuNHC(=O)) |
| U-1 (4) | 2-(*t*-BuNHC(=O)) | U-8 (5) | 3-(*t*-BuNHC(=O)) |
| U-1 (4) | 2-(*i*-BuNHC(=O)) | U-8 (5) | 3-(*i*-BuNHC(=O)) |
| U-1 (4) | 2-(CF$_3$CH$_2$NHC(=O)) | U-8 (5) | 3-(CF$_3$CH$_2$NHC(=O)) |
| U-1 (4) | 2-(*c*-PrCH$_2$NHC(=O)) | U-8 (5) | 3-(*c*-PrCH$_2$NHC(=O)) |
| U-1 (4) | 2-(MeOCH$_2$NHC(=O)) | U-8 (5) | 3-(MeOCH$_2$NHC(=O)) |
| U-1 (4) | 2-(MeOCH$_2$CH$_2$NHC(=O)) | U-8 (5) | 3-(MeOCH$_2$CH$_2$NHC(=O)) |
| U-1 (4) | 2-(CH$_2$=CHCH$_2$NHC(=O)) | U-8 (5) | 3-(CH$_2$=CHCH$_2$NHC(=O)) |
| U-1 (4) | 2-(N≡CCH$_2$NHC(=O)) | U-8 (5) | 3-(N≡CCH$_2$NHC(=O)) |
| U-1 (4) | 2-(OH-N=CH) | U-8 (5) | 3-(OH-N=CH) |
| U-1 (4) | 2-(Me$_2$NN=CH) | U-8 (5) | 3-(Me$_2$NN=CH) |
| U-1 (4) | 2-(MeOC(=O)NHN=CH) | U-8 (5) | 3-(MeOC(=O)NHN=CH) |
| U-1 (4) | 2-(OHC(=O)CH$_2$ON=CH) | U-8 (5) | 3-(OHC(=O)CH$_2$ON=CH) |
| U-1 (2) | - | U-12 (3) | 1-Me |
| U-1 (2) | 4-Me | U-12 (3) | 1,5-di-Me |
| U-1 (2) | 4-Et | U-12 (3) | 1-Me, 5-Et |
| U-1 (2) | 4-*n*-Pr | U-12 (3) | 1-Me, 5-*n*-Pr |
| U-1 (2) | 4-*i*-Pr | U-12 (3) | 1-Me, 5-*i*-Pr |
| U-1 (2) | 4-*c*-Pr | U-12 (3) | 1-Me, 5-*c*-Pr |
| U-1 (2) | 4-*n*-Bu | U-12 (3) | 1-Me, 5-*n*-Bu |
| U-1 (2) | 4-*i*-Bu | U-12 (3) | 1-Me, 5-*i*-Bu |
| U-1 (2) | 4-*t*-Bu | U-12 (3) | 1-Me, 5-*f*-Bu |

(continued)

| L is $CH_2$ and J is J-40. | | L is $CH_2$ and J is J-40. | |
|---|---|---|---|
| $R^1$ | $(R^2)_x$ | $R^1$ | $R^1$ |
| U-1 (2) | 4-F | U-12 (3) | 1-Me, 5-F |
| U-1 (2) | 4-Cl | U-12 (3) | 1-Me, 5-Cl |
| U-1 (2) | 4-Br | U-12 (3) | 1-Me, 5-Br |
| U-1 (2) | 4-$CF_3$ | U-12 (3) | 1-Me, 5-$CF_3$ |
| U-1 (2) | 4-HO | U-12 (3) | 1-Me, 5-HO |
| U-1 (2) | 4-N≡C | U-12 (3) | 1-Me, 5-N≡C |
| U-1 (2) | 4-N≡$CCH_2$ | U-12 (3) | 1-Me, 5-N≡$CCH_2$ |
| U-1 (2) | 4-(MeO) | U-12 (3) | 1-Me, 5-(MeO) |
| U-1 (2) | 4-($MeOCH_2$) | U-12 (3) | 1-Me, 5-($MeOCH_2$) |
| U-1 (2) | 4-($EtOCH_2$) | U-12 (3) | 1-Me, 5-($EtOCH_2$) |
| U-1 (2) | 4-(CH(=O)) | U-12 (3) | 1-Me, 5-(CH(=O)) |
| U-1 (2) | 4-(HOC(=O)) | U-12 (3) | 1-Me, 5-(HOC(=O)) |
| U-1 (2) | 4-(MeOC(=O)) | U-12 (3) | 1-Me, 5-(MeOC(=O)) |
| U-1 (2) | 4-(EtOC(=O)) | U-12 (3) | 1-Me, 5-(EtOC(=O)) |
| U-1 (2) | 4-(*i*-PtOC(=O)) | U-12 (3) | 1-Me, 5-(*i*-PrOC(=O)) |
| U-1 (2) | 4-(*n*-PtOC(=O)) | U-12 (3) | 1-Me, 5-(*n*-PrOC(=O)) |
| U-1 (2) | 4-(BuOC(=O)) | U-12 (3) | 1-Me, 5-(BuOC(=O)) |
| U-1 (2) | 4-(*i*-BuOC(=O)) | U-12 (3) | 1-Me, 5-(*i*-BuOC(=O)) |
| U-1 (2) | 4-(*t*-BuOC(=O)) | U-12 (3) | 1-Me, 5-(*t*-BuOC(=O)) |
| U-1 (2) | 4-($CF_3CH_2OC$(=O) | U-12 (3) | 1-Me, 5-($CF_3CH_2OC$(=O) |
| U-1 (2) | 4-($CH_2$=CHOC(=O)) | U-12 (3) | 1-Me, 5-($CH_2$=CHOC(=O)) |
| U-1 (2) | 4-($CH_2$=$CHCH_2OC$(=O)) | U-12 (3) | 1-Me, 5-($CH_2$=$CHCH_2OC$(=O)) |
| U-1 (2) | 4-($CH_2$=$CBrCH_2OC$(=O)) | U-12 (3) | 1-Me, 5-($CH_2$=$CBrCH_2OC$(=O)) |
| U-1 (2) | 4-($CH_2$=$CHCF_2OC$(=O)) | U-12 (3) | 1-Me, 5-($CH_2$=$CHCF_2OC$(=O)) |
| U-1 (2) | 4-($Me_2C$=$CHCH_2OC$(=O)) | U-12 (3) | 1-Me, 5-($Me_2C$=$CHCH_2OC$(=O)) |
| U-1 (2) | 4-($CH_2$=C(Me)$CH_2OC$(=O)) | U-12 (3) | 1-Me, 5-($CH_2$=C(Me)$CH_2OC$(=O)) |
| U-1 (2) | 4-(CH≡$CCH_2OC$(=O)) | U-12 (3) | 1-Me, 5-(CH≡$CCH_2OC$(=O)) |
| U-1 (2) | 4-(N≡$CCH_2OC$(=O)) | U-12 (3) | 1-Me, 5-(N≡$CCH_2OC$(=O)) |
| U-1 (2) | 4-(MeNHC(=O)) | U-12 (3) | 1-Me, 5-(MeNHC(=O)) |
| U-1 (2) | 4-($Me_2NC$(=O)) | U-12 (3) | 1-Me, 5-($Me_2NC$(=O)) |
| U-1 (2) | 4-(MeNHC(=O)) | U-12 (3) | 1-Me, 5-(MeNHC(=O)) |
| U-1 (2) | 4-(EtNHC(=O)) | U-12 (3) | 1-Me, 5-(EtNHC(=O)) |
| U-1 (2) | 4-(PrNHC(=O)) | U-12 (3) | 1-Me, 5-(PrNHC(=O)) |
| U-1 (2) | 4-(*i*-PrNHC(=O)) | U-12 (3) | 1-Me, 5-(*i*-PrNHC(=O)) |
| U-1 (2) | 4-(BuNHC(=O)) | U-12 (3) | 1-Me, 5-(BuNHC(=O)) |
| U-1 (2) | 4-(*t*-BuNHC(=O)) | U-12 (3) | 1-Me, 5-(*t*-BuNHC(=O)) |
| U-1 (2) | 4-(*i*-BuNHC(=O)) | U-12 (3) | 1-Me, 5-(*i*-BuNHC(=O)) |
| U-1 (2) | 4-($CF_3CH_2NHC$(=O)) | U-12 (3) | 1-Me, 5-($CF_3CH_2NHC$(=O)) |
| U-1 (2) | 4-(*c*-$PrCH_2NHC$(=O)) | U-12 (3) | 1-Me, 5-(*c*-$PrCH_2NHC$(=O)) |
| U-1 (2) | 4-($MeOCH_2NHC$(=O)) | U-12 (3) | 1-Me, 5-($MeOCH_2NHC$(=O)) |
| U-1 (2) | 4-($MeOCH_2CH_2NHC$(=O)) | U-12 (3) | 1-Me, 5-($MeOCH_2CH_2NHC$(=O)) |
| U-1 (2) | 4-($CH_2$=$CHCH_2NHC$(=O)) | U-12 (3) | 1-Me, 5-($CH_2$=$CHCH_2NHC$(=O)) |
| U-1 (2) | 4-(N≡$CCH_2NHC$(=O)) | U-12 (3) | 1-Me, 5-(N≡$CCH_2NHC$(=O)) |
| U-1 (2) | 4-(OH-N=CH) | U-12 (3) | 1-Me, 5-(OH-N=CH) |
| U-1 (2) | 4-($Me_2NN$=CH) | U-12 (3) | 1-Me, 5-($Me_2NN$=CH) |

(continued)

| L is $CH_2$ and J is J-40. | | L is $CH_2$ and J is J-40. | |
|---|---|---|---|
| $R^1$ | $(R^2)_x$ | $R^1$ | $R^1$ |
| U-1 (2) | 4-(MeOC(=O)NHN=CH) | U-12 (3) | 1-Me, 5-(MeOC(=O)NHN=CH) |
| U-1 (2) | 4-(OHC(=O)$CH_2$ON=CH) | U-12 (3) | 1-Me, 5-(OHC(=O)$CH_2$ON=CH) |
| U-2 (2) | - | U-12 (1) | - |
| U-2 (2) | 4-Me | U-12 (1) | 4-Me |
| U-2 (2) | 4-Et | U-12 (1) | 4-Et |
| U-2 (2) | 4-*n*-Pr | U-12 (1) | 4-*n*-Pr |
| U-2 (2) | 4-*i*-Pr | U-12 (1) | 4-*i*-Pr |
| U-2 (2) | 4-*c*-Pr | U-12 (1) | 4-*c*-Pr |
| U-2 (2) | 4-*n*-Bu | U-12 (1) | 4-*n*-Bu |
| U-2 (2) | 4-*i*-Bu | U-12 (1) | 4-*i*-Bu |
| U-2 (2) | 4-*t*-Bu | U-12 (1) | 4-*t*-Bu |
| U-2 (2) | 4-F | U-12 (1) | 4-F |
| U-2 (2) | 4-Cl | U-12 (1) | 4-Cl |
| U-2 (2) | 4-Br | U-12 (1) | 4-Br |
| U-2 (2) | 4-$CF_3$ | U-12 (1) | 4-$CF_3$ |
| U-2 (2) | 4-HO | U-12 (1) | 4-HO |
| U-2 (2) | 4-N≡C | U-12 (1) | 4-N≡C |
| U-2 (2) | 4-N≡$CCH_2$ | U-12 (1) | 4-N≡$CCH_2$ |
| U-2 (2) | 4-(MeO) | U-12 (1) | 4-(MeO) |
| U-2 (2) | 4-($MeOCH_2$) | U-12 (1) | 4-($MeOCH_2$) |
| U-2 (2) | 4-($EtOCH_2$) | U-12 (1) | 4-($EtOCH_2$) |
| U-2 (2) | 4-(CH(=O)) | U-12 (1) | 4-(CH(=O)) |
| U-2 (2) | 4-(HOC(=O)) | U-12 (1) | 4-(HOC(=O)) |
| U-2 (2) | 4-(MeOC(=O)) | U-12 (1) | 4-(MeOC(=O)) |
| U-2 (2) | 4-(EtOC(=O)) | U-12 (1) | 4-(EtOC(=O)) |
| U-2 (2) | 4-(*i*-PtOC(=O)) | U-12 (1) | 4-(*i*-PtOC(=O)) |
| U-2 (2) | 4-(*n*-PtOC(=O)) | U-12 (1) | 4-(*n*-PtOC(=O)) |
| U-2 (2) | 4-(BuOC(=O)) | U-12 (1) | 4-(BuOC(=O)) |
| U-2 (2) | 4-(*i*-BuOC(=O)) | U-12 (1) | 4-(*i*-BuOC(=O)) |
| U-2 (2) | 4-(*t*-BuOC(=O)) | U-12 (1) | 4-(*t*-BuOC(=O)) |
| U-2 (2) | 4-($CF_3CH_2$OC(=O) | U-12 (1) | 4-($CF_3CH_2$OC(=O) |
| U-2 (2) | 4-($CH_2$=CHOC(=O)) | U-12 (1) | 4-($CH_2$=CHOC(=O)) |
| U-2 (2) | 4-($CH_2$=$CHCH_2$OC(=O)) | U-12 (1) | 4-($CH_2$=$CHCH_2$OC(=O)) |
| U-2 (2) | 4-($CH_2$=$CBrCH_2$OC(=O)) | U-12 (1) | 4-($CH_2$=$CBrCH_2$OC(=O)) |
| U-2 (2) | 4-($CH_2$=$CHCF_2$OC(=O)) | U-12 (1) | 4-($CH_2$=$CHCF_2$OC(=O)) |
| U-2 (2) | 4-($Me_2$C=$CHCH_2$OC(=O)) | U-12 (1) | 4-($Me_2$C=$CHCH_2$OC(=O)) |
| U-2 (2) | 4-($CH_2$=C(Me)$CH_2$OC(=O)) | U-12 (1) | 4-($CH_2$=C(Me)$CH_2$OC(=O)) |
| U-2 (2) | 4-(CH≡$CCH_2$OC(=O)) | U-12 (1) | 4-(CH≡$CCH_2$OC(=O)) |
| U-2 (2) | 4-(N≡$CCH_2$OC(=O)) | U-12 (1) | 4-(N≡$CCH_2$OC(=O)) |
| U-2 (2) | 4-(MeNHC(=O)) | U-12 (1) | 4-(MeNHC(=O)) |
| U-2 (2) | 4-($Me_2$NC(=O)) | U-12 (1) | 4-($Me_2$NC(=O)) |
| U-2 (2) | 4-(MeNHC(=O)) | U-12 (1) | 4-(MeNHC(=O)) |
| U-2 (2) | 4-(EtNHC(=O)) | U-12 (1) | 4-(EtNHC(=O)) |
| U-2 (2) | 4-(PrNHC(=O)) | U-12 (1) | 4-(PrNHC(=O)) |
| U-2 (2) | 4-(*i*-PrNHC(=O)) | U-12 (1) | 4-(*i*-PrNHC(=O)) |
| U-2 (2) | 4-(BuNHC(=O)) | U-12 (1) | 4-(BuNHC(=O)) |

(continued)

| L is $CH_2$ and J is J-40. | | L is $CH_2$ and J is J-40. | |
|---|---|---|---|
| $R^1$ | $(R^2)_x$ | $R^1$ | $R^1$ |
| U-2 (2) | 4-(*t*-BuNHC(=O)) | U-12 (1) | 4-(*t*-BuNHC(=O)) |
| U-2 (2) | 4-(*i*-BuNHC(=O)) | U-12 (1) | 4-(*i*-BuNHC(=O)) |
| U-2 (2) | 4-($CF_3CH_2NHC(=O)$) | U-12 (1) | 4-($CF_3CH_2NHC(=O)$) |
| U-2 (2) | 4-(*c*-$PrCH_2NHC(=O)$) | U-12 (1) | 4-(*c*-$PrCH_2NHC(=O)$) |
| U-2 (2) | 4-($MeOCH_2NHC(=O)$) | U-12 (1) | 4-($MeOCH_2NHC(=O)$) |
| U-2 (2) | 4-($MeOCH_2CH_2NHC(=O)$) | U-12 (1) | 4-($MeOCH_2CH_2NHC(=O)$) |
| U-2 (2) | 4-($CH_2=CHCH_2NHC(=O)$) | U-12 (1) | 4-($CH_2=CHCH_2NHC(=O)$) |
| U-2 (2) | 4-($N\equiv CCH_2NHC(=O)$) | U-12 (1) | 4-($N\equiv CCH_2NHC(=O)$) |
| U-2 (2) | 4-(OH-N=CH) | U-12 (1) | 4-(OH-N=CH) |
| U-2 (2) | 4-($Me_2NN=CH$) | U-12 (1) | 4-($Me_2NN=CH$) |
| U-2 (2) | 4-(MeOC(=O)NHN=CH) | U-12 (1) | 4-(MeOC(=O)NHN=CH) |
| U-2 (2) | 4-($OHC(=O)CH_2ON=CH$) | U-12 (1) | 4-($OHC(=O)CH_2ON=CH$) |
| U-2 (4) | - | U-69 (1) | - |
| U-2 (4) | 2-Me | U-69 (1) | 4-Me |
| U-2 (4) | 2-Et | U-69 (1) | 4-Et |
| U-2 (4) | 2-*n*-Pr | U-69 (1) | 4-*n*-Pr |
| U-2 (4) | 2-*i*-Pr | U-69 (1) | 4-*i*-Pr |
| U-2 (4) | 2-*c*-Pr | U-69 (1) | 4-*c*-Pr |
| U-2 (4) | 2-*n*-Bu | U-69 (1) | 4-*n*-Bu |
| U-2 (4) | 2-*i*-Bu | U-69 (1) | 4-*i*-Bu |
| U-2 (4) | 2-*t*-Bu | U-69 (1) | 4-*t*-Bu |
| U-2 (4) | 2-F | U-69 (1) | 4-F |
| U-2 (4) | 2-Cl | U-69 (1) | 4-Cl |
| U-2 (4) | 2-Br | U-69 (1) | 4-Br |
| U-2 (4) | 2-$CF_3$ | U-69 (1) | 4-$CF_3$ |
| U-2 (4) | 2-HO | U-69 (1) | 4-HO |
| U-2 (4) | 2-$N\equiv C$ | U-69 (1) | 4-$N\equiv C$ |
| U-2 (4) | 2-$N\equiv CCH_2$ | U-69 (1) | 4-$N\equiv CCH_2$ |
| U-2 (4) | 2-(MeO) | U-69 (1) | 4-(MeO) |
| U-2 (4) | 2-($MeOCH_2$) | U-69 (1) | 4-($MeOCH_2$) |
| U-2 (4) | 2-($EtOCH_2$) | U-69 (1) | 4-($EtOCH_2$) |
| U-2 (4) | 2-(CH(=O)) | U-69 (1) | 4-(CH(=O)) |
| U-2 (4) | 2-(HOC(=O)) | U-69 (1) | 4-(HOC(=O)) |
| U-2 (4) | 2-(MeOC(=O)) | U-69 (1) | 4-(MeOC(=O)) |
| U-2 (4) | 2-(EtOC(=O)) | U-69 (1) | 4-(EtOC(=O)) |
| U-2 (4) | 2-(*i*-PrOC(=O)) | U-69 (1) | 4-(*i*-PtOC(=O)) |
| U-2 (4) | 2-(*n*-PiOC(=O)) | U-69 (1) | 4-(*n*-PtOC(=O)) |
| U-2 (4) | 2-(BuOC(=O)) | U-69 (1) | 4-(BuOC(=O)) |
| U-2 (4) | 2-(*i*-BuOC(=O)) | U-69 (1) | 4-(*i*-BuOC(=O)) |
| U-2 (4) | 2-(*t*-BuOC(=O)) | U-69 (1) | 4-(*t*-BuOC(=O)) |
| U-2 (4) | 2-($CF_3CH_2OC(=O)$ | U-69 (1) | 4-($CF_3CH_2OC(=O)$ |
| U-2 (4) | 2-($CH_2=CHOC(=O)$) | U-69 (1) | 4-($CH_2=CHOC(=O)$) |
| U-2 (4) | 2-($CH_2=CHCH_2OC(=O)$) | U-69 (1) | 4-($CH_2=CHCH_2OC(=O)$) |
| U-2 (4) | 2-($CH_2=CBrCH_2OC(=O)$) | U-69 (1) | 4-($CH_2=CBrCH_2OC(=O)$) |
| U-2 (4) | 2-($CH_2=CHCF_2OC(=O)$) | U-69 (1) | 4-($CH_2=CHCF_2OC(=O)$) |
| U-2 (4) | 2-($Me_2C=CHCH_2OC(=O)$) | U-69 (1) | 4-($Me_2C=CHCH_2OC(=O)$) |

(continued)

| L is $CH_2$ and J is J-40. | | L is $CH_2$ and J is J-40. | |
|---|---|---|---|
| $R^1$ | $(R^2)_x$ | $R^1$ | $R^1$ |
| U-2 (4) | 2-($CH_2$=C(Me)$CH_2$OC(=O)) | U-69 (1) | 4-($CH_2$=C(Me)$CH_2$OC(=O)) |
| U-2 (4) | 2-(CH≡$CCH_2$OC(=O)) | U-69 (1) | 4-(CH≡$CCH_2$OC(=O)) |
| U-2 (4) | 2-(N≡$CCH_2$OC(=O)) | U-69 (1) | 4-(N≡$CCH_2$OC(=O)) |
| U-2 (4) | 2-(MeNHC(=O)) | U-69 (1) | 4-(MeNHC(=O)) |
| U-2 (4) | 2-($Me_2$NC(=O)) | U-69 (1) | 4-($Me_2$NC(=O)) |
| U-2 (4) | 2-(MeNHC(=O)) | U-69 (1) | 4-(MeNHC(=O)) |
| U-2 (4) | 2-(EtNHC(=O)) | U-69 (1) | 4-(EtNHC(=O)) |
| U-2 (4) | 2-(PrNHC(=O)) | U-69 (1) | 4-(PrNHC(=O)) |
| U-2 (4) | 2-(*i*-PrNHC(=O)) | U-69 (1) | 4-(*i*-PrNHC(=O)) |
| U-2 (4) | 2-(BuNHC(=O)) | U-69 (1) | 4-(BuNHC(=O)) |
| U-2 (4) | 2-(*t*-BuNHC(=O)) | U-69 (1) | 4-(*t*-BuNHC(=O)) |
| U-2 (4) | 2-(*i*-BuNHC(=O)) | U-69 (1) | 4-(*i*-BuNHC(=O)) |
| U-2 (4) | 2-($CF_3CH_2$NHC(=O)) | U-69 (1) | 4-($CF_3CH_2$NHC(=O)) |
| U-2 (4) | 2-(*c*-$PrCH_2$NHC(=O)) | U-69 (1) | 4-(*c*-$PrCH_2$NHC(=O)) |
| U-2 (4) | 2-($MeOCH_2$NHC(=O)) | U-69 (1) | 4-($MeOCH_2$NHC(=O)) |
| U-2 (4) | 2-($MeOCH_2CH_2$NHC(=O)) | U-69 (1) | 4-($MeOCH_2CH_2$NHC(=O)) |
| U-2 (4) | 2-($CH_2$=$CHCH_2$NHC(=O)) | U-69 (1) | 4-($CH_2$=$CHCH_2$NHC(=O)) |
| U-2 (4) | 2-(N≡$CCH_2$NHC(=O)) | U-69 (1) | 4-(N≡$CCH_2$NHC(=O)) |
| U-2 (4) | 2-(OH-N=CH) | U-69 (1) | 4-(OH-N=CH) |
| U-2 (4) | 2-($Me_2$NN=CH) | U-69 (1) | 4-($Me_2$NN=CH) |
| U-2 (4) | 2-(MeOC(=O)NHN=CH) | U-69 (1) | 4-(MeOC(=O)NHN=CH) |
| U-2 (4) | 2-(OHC(=O)$CH_2$ON=CH) | U-69 (1) | 4-(OHC(=O)$CH_2$ON=CH) |

The present disclosure also includes Tables 1B through 47B, each of which is constructed the same as Table 2 above, except that the row heading in Table 2 (i.e. "L is $CH_2$ and J is J-40") is replaced with the respective row heading shown below.

| Table | Row Heading | Table | Row Heading |
|---|---|---|---|
| 1B | L is $CH_2CH_2$ and J is J-40. | 25B | L is $OCH_2$ and J is J-27. |
| 2B | L is $CH_2$(Me) and J is J-40. | 26B | L is $CH_2O$ and J is J-27. |
| 3B | L is $CH_2CH_2CH_2$ and J is J-40. | 27B | L is $CH_2OCH_2$ and J is J-27. |
| 4B | L is $OCH_2$ and J is J-40. | 28B | L is $CH_2$ and J is J-63. |
| 5B | L is $CH_2O$ and J is J-40. | 29B | L is $CH_2CH_2$ and J is J-63. |
| 6B | L is $CH_2OCH_2$ and J is J-40. | 30B | L is $CH_2$(Me) and J is J-63. |
| 7B | L is $CH_2$ and J is J-4. | 31B | L is $CH_2CH_2CH_2$ and J is J-63. |
| 8B | L is $CH_2CH_2$ and J is J-4. | 32B | L is $OCH_2$ and J is J-63. |
| 9B | L is $CH_2$(Me) and J is J-4. | 33B | L is $CH_2O$ and J is J-63. |
| 10B | L is $CH_2CH_2CH_2$ and J is J-4. | 34B | L is $CH_2OCH_2$ and J is J-63. |
| 11B | L is $OCH_2$ and J is J-4. | 35B | L is $CH_2$ and J is J-73. |
| 12B | L is $CH_2O$ and J is J-4. | 36B | L is $CH_2CH_2$ and J is J-73. |
| 13B | L is $CH_2OCH_2$ and J is J-4. | 37B | L is $CH_2$(Me) and J is J-73. |
| 14B | L is $CH_2$ and J is J-18. | 38B | L is $CH_2CH_2CH_2$ and J is J-73. |
| 15B | L is $CH_2CH_2$ and J is J-18. | 39B | L is $OCH_2$ and J is J-73. |
| 16B | L is $CH_2$(Me) and J is J-18. | 40B | L is $CH_2O$ and J is J-73. |

(continued)

| Table | Row Heading | Table | Row Heading |
|---|---|---|---|
| 17B | L is $CH_2CH_2CH_2$ and J is J-18. | 41B | L is $CH_2OCH_2$ and J is J-73. |
| 18B | L is $OCH_2$ and J is J-18. | 42B | L is $CH_2$ and J is J-93. |
| 19B | L is $CH_2O$ and J is J-18. | 43B | L is $CH_2CH_2$ and J is J-93. |
| 20B | L is $CH_2OCH_2$ and J is J-18. | 44B | L is $CH_2$(Me) and J is J-93. |
| 21B | L is $CH_2$ and J is J-27. | 45B | L is $CH_2CH_2CH_2$ and J is J-93. |
| 22B | L is $CH_2CH_2$ and J is J-27. | 46B | L is $OCH_2$ and J is J-93. |
| 23B | L is $CH_2$(Me) and J is J-27. | 47B | L is $CH_2O$ and J is J-93. |
| 24B | L is $CH_2CH_2CH_2$ and J is J-27. | 48B | L is $CH_2OCH_2$ and J is J-93. |

Formulation/Utility

A compound of Formula **1** of this invention (including *N*-oxides and salts thereof), or a mixture (i.e. composition) comprising the compound with at least one additional fungicidal compound as described in the Summary of the Invention, will generally be used as a fungicidal active ingredient in a composition, i.e. formulation, with at least one additional component selected from the group consisting of surfactants, solid diluents and liquid diluents, which serve as a carrier. The formulation or composition ingredients are selected to be consistent with the physical properties of the active ingredient, mode of application and environmental factors such as soil type, moisture and temperature.

The mixtures of component (a) (i.e. at least one compound of Formula **1,** *N*-oxides, or salts thereof) with component (b) (e.g., selected from (b1) to (b54) and salts thereof as described above) and/or one or more other biologically active compound or agent (i.e. insecticides, other fungicides, nematocides, acaricides, herbicides and other biological agents) can be formulated in a number of ways, including:

(i) component (a), component (b) and/or one or more other biologically active compounds or agents can be formulated separately and applied separately or applied simultaneously in an appropriate weight ratio, e.g., as a tank mix; or
(ii) component (a), component (b) and/or one or more other biologically active compounds or agents can be formulated together in the proper weight ratio.

Useful formulations include both liquid and solid compositions. Liquid compositions include solutions (including emulsifiable concentrates), suspensions, emulsions (including microemulsions, oil-in-water emulsions, flowable concentrates and/or suspoemulsions) and the like, which optionally can be thickened into gels. The general types of aqueous liquid compositions are soluble concentrate, suspension concentrate, capsule suspension, concentrated emulsion, microemulsion, oil-in-water emulsion, flowable concentrate and suspoemulsion. The general types of nonaqueous liquid compositions are emulsifiable concentrate, microemulsifiable concentrate, dispersible concentrate and oil dispersion.

The general types of solid compositions are dusts, powders, granules, pellets, prills, pastilles, tablets, filled films (including seed coatings) and the like, which can be water-dispersible ("wettable") or water-soluble. Films and coatings formed from film-forming solutions or flowable suspensions are particularly useful for seed treatment. Active ingredient can be (micro)encapsulated and further formed into a suspension or solid formulation; alternatively the entire formulation of active ingredient can be encapsulated (or "overcoated"). Encapsulation can control or delay release of the active ingredient. An emulsifiable granule combines the advantages of both an emulsifiable concentrate formulation and a dry granular formulation. High-strength compositions are primarily used as intermediates for further formulation.

Of note is a composition embodiment wherein granules of a solid composition comprising a compound of Formula **1** (or an *N*-oxide or salt thereof) is mixed with granules of a solid composition comprising component (b). These mixtures can be further mixed with granules comprising additional agricultural protectants. Alternatively, two or more agricultural protectants (e.g., a component (a) (Formula **1)** compound, a component (b) compound, an agricultural protectant other than component (a) or (b)) can be combined in the solid composition of one set of granules, which is then mixed with one or more sets of granules of solid compositions comprising one or more additional agricultural protectants. These granule mixtures can be in accordance with the general granule mixture disclosure of PCT Patent Publication WO 94/24861 or more preferably the homogeneous granule mixture teaching of U.S. Patent 6,022,552.

Sprayable formulations are typically extended in a suitable medium before spraying. Such liquid and solid formulations are formulated to be readily diluted in the spray medium, usually water, but occasionally another suitable medium like an aromatic or paraffinic hydrocarbon or vegetable oil. Spray volumes can range from about one to several thousand liters per hectare, but more typically are in the range from about ten to several hundred liters per hectare.

Sprayable formulations can be tank mixed with water or another suitable medium for foliar treatment by aerial or ground application, or for application to the growing medium of the plant. Liquid and dry formulations can be metered directly into drip irrigation systems or metered into the furrow during planting. Liquid and solid formulations can be applied onto seeds of crops and other desirable vegetation as seed treatments before planting to protect developing roots and other subterranean plant parts and/or foliage through systemic uptake.

The formulations will typically contain effective amounts of active ingredient, diluent and surfactant within the following approximate ranges which add up to 100 percent by weight.

| | Weight Percent | | |
|---|---|---|---|
| | Active Ingredient | Diluent | Surfactant |
| Water-Dispersible and Water-soluble Granules, Tablets and Powders | 0.001-90 | 0-99.999 | 0-15 |
| Oil Dispersions, Suspensions, Emulsions, Solutions (including Emulsifiable Concentrates) | 1-50 | 40-99 | 0-50 |
| Dusts | 1-25 | 70-99 | 0-5 |
| Granules and Pellets | 0.001-95 | 5-99.999 | 0-15 |
| High Strength Compositions | 90-99 | 0-10 | 0-2 |

Solid diluents include, for example, clays such as bentonite, montmorillonite, attapulgite and kaolin, gypsum, cellulose, titanium dioxide, zinc oxide, starch, dextrin, sugars (e.g., lactose, sucrose), silica, talc, mica, diatomaceous earth, urea, calcium carbonate, sodium carbonate and bicarbonate, and sodium sulfate. Typical solid diluents are described in Watkins et al., *Handbook of Insecticide Dust Diluents and Carriers,* 2nd Ed., Dorland Books, Caldwell, New Jersey.

Liquid diluents include, for example, water, *N,N*-dimethylalkanamides (e.g., N,N-dimethylformamide), limonene, dimethyl sulfoxide, *N*-alkylpyrrolidones (e.g., *N*-methylpyrrolidinone), alkyl phosphates (e.g., triethyl phosphate), ethylene glycol, triethylene glycol, propylene glycol, dipropylene glycol, polypropylene glycol, propylene carbonate, butylene carbonate, paraffins (e.g., white mineral oils, normal paraffins, isoparaffins), alkylbenzenes, alkylnaphthalenes, glycerine, glycerol triacetate, sorbitol, aromatic hydrocarbons, dearomatized aliphatics, alkylbenzenes, alkylnaphthalenes, ketones such as cyclohexanone, 2-heptanone, isophorone and 4-hydroxy-4-methyl-2-pentanone, acetates such as isoamyl acetate, hexyl acetate, heptyl acetate, octyl acetate, nonyl acetate, tridecyl acetate and isobornyl acetate, other esters such as alkylated lactate esters, dibasic esters, alkyl and aryl benzoates and $\gamma$-butyrolactone, and alcohols, which can be linear, branched, saturated or unsaturated, such as methanol, ethanol, *n*-propanol, isopropyl alcohol, *n*-butanol, isobutyl alcohol, *n*-hexanol, 2-ethylhexanol, *n*-octanol, decanol, isodecyl alcohol, isooctadecanol, cetyl alcohol, lauryl alcohol, tridecyl alcohol, oleyl alcohol, cyclohexanol, tetrahydrofurfuryl alcohol, diacetone alcohol, cresol and benzyl alcohol. Liquid diluents also include glycerol esters of saturated and unsaturated fatty acids (typically $C_6$-$C_{22}$), such as plant seed and fruit oils (e.g., oils of olive, castor, linseed, sesame, corn (maize), peanut, sunflower, grapeseed, safflower, cottonseed, soybean, rapeseed, coconut and palm kernel), animal-sourced fats (e.g., beef tallow, pork tallow, lard, cod liver oil, fish oil), and mixtures thereof. Liquid diluents also include alkylated fatty acids (e.g., methylated, ethylated, butylated) wherein the fatty acids may be obtained by hydrolysis of glycerol esters from plant and animal sources, and can be purified by distillation. Typical liquid diluents are described in Marsden, Solvents Guide, 2nd Ed., Interscience, New York, 1950.

The solid and liquid compositions of the present invention often include one or more surfactants. When added to a liquid, surfactants (also known as "surface-active agents") generally modify, most often reduce, the surface tension of the liquid. Depending on the nature of the hydrophilic and lipophilic groups in a surfactant molecule, surfactants can be useful as wetting agents, dispersants, emulsifiers or defoaming agents.

Surfactants can be classified as nonionic, anionic or cationic. Nonionic surfactants useful for the present compositions include, but are not limited to: alcohol alkoxylates such as alcohol alkoxylates based on natural and synthetic alcohols (which may be branched or linear) and prepared from the alcohols and ethylene oxide, propylene oxide, butylene oxide or mixtures thereof; amine ethoxylates, alkanolamides and ethoxylated alkanolamides; alkoxylated triglycerides such as ethoxylated soybean, castor and rapeseed oils; alkylphenol alkoxylates such as octylphenol ethoxylates, nonylphenol ethoxylates, dinonyl phenol ethoxylates and dodecyl phenol ethoxylates (prepared from the phenols and ethylene oxide, propylene oxide, butylene oxide or mixtures thereof); block polymers prepared from ethylene oxide or propylene oxide and reverse block polymers where the terminal blocks are prepared from propylene oxide; ethoxylated fatty acids; ethoxylated fatty esters and oils; ethoxylated methyl esters; ethoxylated tristyrylphenol (including those prepared from ethylene oxide, propylene oxide, butylene oxide or mixtures thereof); fatty acid esters, glycerol esters, lanolin-based derivatives, polyethoxylate esters such as polyethoxylated sorbitan fatty acid esters, polyethoxylated

sorbitol fatty acid esters and polyethoxylated glycerol fatty acid esters; other sorbitan derivatives such as sorbitan esters; polymeric surfactants such as random copolymers, block copolymers, alkyd peg (polyethylene glycol) resins, graft or comb polymers and star polymers; polyethylene glycols (pegs); polyethylene glycol fatty acid esters; silicone-based surfactants; and sugar-derivatives such as sucrose esters, alkyl polyglycosides; alkyl polysaccharides; and glucamides such as mixtures of octyl-*N*-methylglucamide and decyl-*N*-methylglucamide (e.g., products is obtainable under the Synergen® GA name from Clariant).

Useful anionic surfactants include, but are not limited to: alkylaryl sulfonic acids and their salts; carboxylated alcohol or alkylphenol ethoxylates; diphenyl sulfonate derivatives; lignin and lignin derivatives such as lignosulfonates; maleic or succinic acids or their anhydrides; olefin sulfonates; phosphate esters such as phosphate esters of alcohol alkoxylates, phosphate esters of alkylphenol alkoxylates and phosphate esters of styryl phenol ethoxylates; protein-based surfactants; sarcosine derivatives; styryl phenol ether sulfate; sulfates and sulfonates of oils and fatty acids; sulfates and sulfonates of ethoxylated alkylphenols; sulfates of alcohols; sulfates of ethoxylated alcohols; sulfonates of amines and amides such as *N*,*N*-alkyltaurates; sulfonates of benzene, cumene, toluene, xylene, and dodecyl and tridecylbenzenes; sulfonates of condensed naphthalenes; sulfonates of naphthalene and alkyl naphthalene; sulfonates of fractionated petroleum; sulfosuccinamates; and sulfosuccinates and their derivatives such as dialkyl sulfosuccinate salts.

Useful cationic surfactants include, but are not limited to: amides and ethoxylated amides; amines such as *N*-alkyl propanediamines, tripropylenetriamines and dipropylenetetramines, and ethoxylated amines, ethoxylated diamines and propoxylated amines (prepared from the amines and ethylene oxide, propylene oxide, butylene oxide or mixtures thereof); amine salts such as amine acetates and diamine salts; quaternary ammonium salts such as quaternary salts, ethoxylated quaternary salts and diquaternary salts; and amine oxides such as alkyldimethylamine oxides and bis-(2-hydroxyethyl)-alkylamine oxides.

Also useful for the present compositions are mixtures of nonionic and anionic surfactants or mixtures of nonionic and cationic surfactants. Nonionic, anionic and cationic surfactants and their recommended uses are disclosed in a variety of published references including McCutcheon's Emulsifiers and Detergents, annual American and International Editions published by McCutcheon's Division, The Manufacturing Confectioner Publishing Co.; Sisely and Wood, Encyclopedia of Surface Active Agents, Chemical Publ. Co., Inc., New York, 1964; and A. S. Davidson and B. Milwidsky, Synthetic Detergents, Seventh Edition, John Wiley and Sons, New York, 1987.

Compositions of this invention may also contain formulation auxiliaries and additives, known to those skilled in the art as formulation aids (some of which may be considered to also function as solid diluents, liquid diluents or surfactants). Such formulation auxiliaries and additives may control: pH (buffers), foaming during processing (antifoams such polyorganosiloxanes), sedimentation of active ingredients (suspending agents), viscosity (thixotropic thickeners), in-container microbial growth (antimicrobials), product freezing (antifreezes), color (dyes/pigment dispersions), wash-off (film formers or stickers), evaporation (evaporation retardants), and other formulation attributes. Film formers include, for example, polyvinyl acetates, polyvinyl acetate copolymers, polyvinylpyrrolidone-vinyl acetate copolymer, polyvinyl alcohols, polyvinyl alcohol copolymers and waxes. Examples of formulation auxiliaries and additives include those listed *in* McCutcheon's Volume 2: Functional Materials, annual International and North American editions published by McCutcheon's Division, The Manufacturing Confectioner Publishing Co.; and PCT Publication WO 03/024222.

The compound of Formula **1** and any other active ingredients are typically incorporated into the present compositions by dissolving the active ingredient in a solvent or by grinding in a liquid or dry diluent. Solutions, including emulsifiable concentrates, can be prepared by simply mixing the ingredients. If the solvent of a liquid composition intended for use as an emulsifiable concentrate is water-immiscible, an emulsifier is typically added to emulsify the active-containing solvent upon dilution with water. Active ingredient slurries, with particle diameters of up to 2,000 $\mu$m can be wet milled using media mills to obtain particles with average diameters below 3 $\mu$m. Aqueous slurries can be made into finished suspension concentrates (see, for example, U.S. 3,060,084) or further processed by spray drying to form water-dispersible granules. Dry formulations usually require dry milling processes, which produce average particle diameters in the 2 to 10 $\mu$m range. Dusts and powders can be prepared by blending and usually grinding (such as with a hammer mill or fluid-energy mill). Granules and pellets can be prepared by spraying the active material upon preformed granular carriers or by agglomeration techniques. See Browning, "Agglomeration", Chemical Engineering, December 4, 1967, pp 147-48, Perry's Chemical Engineer's Handbook, 4th Ed., McGraw-Hill, New York, 1963, pp 8-57 and following, and WO 91/13546. Pellets can be prepared as described in U.S. 4,172,714. Water-dispersible and water-soluble granules can be prepared as taught in U.S. 4,144,050, U.S. 3,920,442 and DE 3,246,493. Tablets can be prepared as taught in U.S. 5,180,587, U.S. 5,232,701 and U.S. 5,208,030. Films can be prepared as taught in GB 2,095,558 and U.S. 3,299,566.

One embodiment of the present invention relates to a method for controlling fungal pathogens, comprising diluting the fungicidal composition of the present invention (a compound of Formula **1** formulated with surfactants, solid diluents and liquid diluents or a formulated mixture of a compound of Formula **1** and at least one other fungicide) with water, and optionally adding an adjuvant to form a diluted composition, and contacting the fungal pathogen or its environment with an effective amount of said diluted composition.

Although a spray composition formed by diluting with water a sufficient concentration of the present fungicidal

composition can provide sufficient efficacy for controlling fungal pathogens, separately formulated adjuvant products can also be added to spray tank mixtures. These additional adjuvants are commonly known as "spray adjuvants" or "tank-mix adjuvants", and include any substance mixed in a spray tank to improve the performance of a pesticide or alter the physical properties of the spray mixture. Adjuvants can be anionic or nonionic surfactants, emulsifying agents, petroleum-based crop oils, crop-derived seed oils, acidifiers, buffers, thickeners or defoaming agents. Adjuvants are used to enhancing efficacy (e.g., biological availability, adhesion, penetration, uniformity of coverage and durability of protection), or minimizing or eliminating spray application problems associated with incompatibility, foaming, drift, evaporation, volatilization and degradation. To obtain optimal performance, adjuvants are selected with regard to the properties of the active ingredient, formulation and target (e.g., crops, insect pests).

The amount of adjuvants added to spray mixtures is generally in the range of about 0.1 % to 2.5% by volume. The application rates of adjuvants added to spray mixtures are typically between about 1 to 5 L per hectare. Representative examples of spray adjuvants include: Adigor® (Syngenta) 47% methylated rapeseed oil in liquid hydrocarbons, Silwet® (Helena Chemical Company) polyalkyleneoxide modified heptamethyltrisiloxane and Assist® (BASF) 17% surfactant blend in 83% paraffin based mineral oil.

One method of seed treatment is by spraying or dusting the seed with a compound of the invention (i.e. as a formulated composition) before sowing the seeds. Compositions formulated for seed treatment generally comprise a film former or adhesive agent. Therefore typically a seed coating composition of the present invention comprises a biologically effective amount of a compound of Formula **1** and a film former or adhesive agent. Seeds can be coated by spraying a flowable suspension concentrate directly into a tumbling bed of seeds and then drying the seeds. Alternatively, other formulation types such as wetted powders, solutions, suspoemulsions, emulsifiable concentrates and emulsions in water can be sprayed on the seed. This process is particularly useful for applying film coatings on seeds. Various coating machines and processes are available to one skilled in the art. Suitable processes include those listed in P. Kosters et al., Seed Treatment: Progress and Prospects, 1994 BCPC Mongraph No. 57, and references listed therein.

For further information regarding the art of formulation, see T. S. Woods, "The Formulator's Toolbox - Product Forms for Modern Agriculture" in Pesticide Chemistry and Bioscience, The Food-Environment Challenge, T. Brooks and T. R. Roberts, Eds., Proceedings of the 9th International Congress on Pesticide Chemistry, The Royal Society of Chemistry, Cambridge, 1999, pp. 120-133. Also see U.S. 3,235,361, Col. 6, line 16 through Col. 7, line 19 and Examples 10-41; U.S. 3,309,192, Col. 5, line 43 through Col. 7, line 62 and Examples 8, 12, 15, 39, 41, 52, 53, 58, 132, 138-140, 162-164, 166, 167 and 169-182; U.S. 2,891,855, Col. 3, line 66 through Col. 5, line 17 and Examples 1-4; Klingman, Weed Control as a Science, John Wiley and Sons, Inc., New York, 1961, pp 81-96; Hance et al., Weed Control Handbook, 8th Ed., Blackwell Scientific Publications, Oxford, 1989; and Developments in formulation technology, PJB Publications, Richmond, UK, 2000.

In the following Examples, all percentages are by weight and all formulations are prepared in conventional ways. Active ingredient refers to the compounds in Index Tables A-T disclosed herein. Without further elaboration, it is believed that one skilled in the art using the preceding description can utilize the present invention to its fullest extent. The following Examples are, therefore, to be constructed as merely illustrative, and not limiting of the disclosure in any way whatsoever.

Example A

High Strength Concentrate

| | |
|---|---|
| Compound 204 | 98.5% |
| silica aerogel | 0.5% |
| synthetic amorphous fine silica | 1.0% |

Example B

Wettable Powder

| | |
|---|---|
| Compound 225 | 65.0% |
| dodecylphenol polyethylene glycol ether | 2.0% |
| sodium ligninsulfonate | 4.0% |

(continued)

| | |
|---|---|
| sodium silicoaluminate | 6.0% |
| montmorillonite (calcined) | 23.0% |

Example C

Granule

| | |
|---|---|
| Compound 264 | 10.0% |
| attapulgite granules (low volatile matter, 0.71/0.30 mm; U.S.S. No. 25-50 sieves) | 90.0% |

Example D

Extruded Pellet

| | |
|---|---|
| Compound 316 | 25.0% |
| anhydrous sodium sulfate | 10.0% |
| crude calcium ligninsulfonate | 5.0% |
| sodium alkylnaphthalenesulfonate | 1.0% |
| calcium/magnesium bentonite | 59.0% |

Example E

Emulsifiable Concentrate

| | |
|---|---|
| Compound 330 | 10.0% |
| polyoxyethylene sorbitol hexoleate | 20.0% |
| $C_6$-$C_{10}$ fatty acid methyl ester | 70.0% |

Example F

Microemulsion

| | |
|---|---|
| Compound 376 | 5.0% |
| polyvinylpyrrolidone-vinyl acetate copolymer | 30.0% |
| alkylpolyglycoside | 30.0% |
| glyceryl monooleate | 15.0% |
| water | 20.0% |

Example G

Seed Treatment

| | |
|---|---|
| Compound 394 | 20.00% |
| polyvinylpyrrolidone-vinyl acetate copolymer | 5.00% |
| montan acid wax | 5.00% |
| calcium ligninsulfonate | 1.00% |
| polyoxyethylene/polyoxypropylene block copolymers | 1.00% |
| stearyl alcohol (POE 20) | 2.00% |
| polyorganosilane | 0.20% |
| colorant red dye | 0.05% |
| water | 65.75% |

Example H

Fertilizer Stick

| | |
|---|---|
| Compound 463 | 2.50% |
| pyrrolidone-styrene copolymer | 4.80% |
| tristyrylphenyl 16-ethoxylate | 2.30% |
| talc | 0.80% |
| corn starch | 5.00% |
| slow-release fertilizer | 36.00% |
| kaolin | 38.00% |
| water | 10.60% |

Example I

Suspension Concentrate

| | |
|---|---|
| Compound 505 | 35% |
| butyl polyoxyethylene/polypropylene block copolymer | 4.0% |
| stearic acid/polyethylene glycol copolymer | 1.0% |
| styrene acrylic polymer | 1.0% |
| xanthan gum | 0.1% |
| propylene glycol | 5.0% |
| silicone based defoamer | 0.1% |
| 1,2-benzisothiazolin-3 -one | 0.1% |
| water | 53.7% |

Example J

Emulsion in Water

| | |
|---|---|
| Compound 508 | 10.0% |
| butyl polyoxyethylene/polypropylene block copolymer | 4.0% |
| stearic acid/polyethylene glycol copolymer | 1.0% |
| styrene acrylic polymer | 1.0% |
| xanthan gum | 0.1% |

(continued)

| | |
|---|---|
| propylene glycol | 5.0% |
| silicone based defoamer | 0.1% |
| 1,2-benzisothiazolin-3 -one | 0.1% |
| aromatic petroleum based hydrocarbon | 20.0 |
| water | 58.7% |

Example K

Oil Dispersion

| | |
|---|---|
| Compound 509 | 25% |
| polyoxyethylene sorbitol hexaoleate | 15% |
| organically modified bentonite clay | 2.5% |
| fatty acid methyl ester | 57.5% |

Example L

Suspoemulsion

| | |
|---|---|
| Compound 316 | 10.0% |
| imidacloprid | 5.0% |
| butyl polyoxyethylene/polypropylene block copolymer | 4.0% |
| stearic acid/polyethylene glycol copolymer | 1.0% |
| styrene acrylic polymer | 1.0% |
| xanthan gum | 0.1% |
| propylene glycol | 5.0% |
| silicone based defoamer | 0.1% |
| 1,2-benzisothiazolin-3 -one | 0.1% |
| aromatic petroleum based hydrocarbon | 20.0% |
| water | 53.7% |

Water-soluble and water-dispersible formulations are typically diluted with water to form aqueous compositions before application. Aqueous compositions for direct applications to the plant or portion thereof (e.g., spray tank compositions) typically contain at least about 1 ppm or more (e.g., from 1 ppm to 100 ppm) of the compound(s) of this invention.

Seed is normally treated at a rate of from about 0.001 g (more typically about 0.1 g) to about 10 g per kilogram of seed (i.e. from about 0.0001 to 1% by weight of the seed before treatment). A flowable suspension formulated for seed treatment typically comprises from about 0.5 to about 70% of the active ingredient, from about 0.5 to about 30% of a film-forming adhesive, from about 0.5 to about 20% of a dispersing agent, from 0 to about 5% of a thickener, from 0 to about 5% of a pigment and/or dye, from 0 to about 2% of an antifoaming agent, from 0 to about 1% of a preservative, and from 0 to about 75% of a volatile liquid diluent.

The compositions of this invention are useful as plant disease control agents. The present invention therefore further comprises a method for controlling plant diseases caused by fungal plant pathogens comprising applying to the plant or portion thereof to be protected, or to the plant seed to be protected, an effective amount of a compound of the invention or a fungicidal composition containing said compound. The compounds and/or compositions of this invention provide control of diseases caused by a broad spectrum of fungal plant pathogens in the Ascomycota, Basidiomycota, Zygomycota phyla, and the fungal-like Oomycota class. They are effective in controlling a broad spectrum of plant diseases, particularly foliar pathogens of ornamental, turf, vegetable, field, cereal, and fruit crops. These pathogens include but are not limited to those listed in Table 1-1. For Ascomycetes and Basidiomycetes, names for both the sexual/teleomorph/perfect stage as well as names for the asexual/anamorph/imperfect stage (in parentheses) are listed

where known. Synonymous names for pathogens are indicated by an equal sign. For example, the sexual/teleomorph/perfect stage name Phaeosphaeria nodorum is followed by the corresponding asexual/anamorph/imperfect stage name Stagnospora nodorum and the synonymous older name Septoria nodorum.

Table 1-1

| |
|---|
| Ascomycetes in the order Pleosporales *including Alternaria solani, A. alternata* and *A. brassicae, Guignardia bidwellii, Venturia inaequalis, Pyrenophora tritici-repentis (Dreschlera tritici-repentis = Helminthosporium tritici-repentis)* and *Pyrenophora teres* (*Dreschlera teres = Helminthosporium teres*), *Corynespora cassiicola, Phaeosphaeria nodorum (Stagonospora nodorum = Septoria nodorum), Cochliobolus carbonum* and *C. heterostrophus, Leptosphaeria biglobosa and L. maculans*; |
| Ascomycetes in the order Mycosphaerellales including *Mycosphaerella graminicola (Zymoseptoria tritici = Septoria tritici),* M. *berkeleyi (Cercosporidium personatum), M. arachidis (Cercospora arachidicola*), *Passalora sojina* (*Cercospora sojina*), *Cercospora zeae-maydis* and *C. beticola;* |
| Ascomycetes in the order Erysiphales (the powdery mildews) such as *Blumeria graminis* f.sp. *tritici* and *Blumeria graminis* f.sp. *hordei, Erysiphe polygoni, E. necator (= Uncinula necator), Podosphaera fuliginea (= Sphaerotheca fuliginea),* and *Podosphaera leucotricha* (= *Sphaerotheca fuliginea*); |
| Ascomycetes in the order Helotiales such as *Botryotinia fuckeliana (Botrytis cinerea), Oculimacula yallundae (= Tapesia yallundae;* anamorph *Helgardia herpotrichoides = Pseudocercosporella herpetrichoides), Monilinia fructicola, Sclerotinia sclerotiorum, Sclerotinia minor,* and *Sclerotinia homoeocarpa;* |
| Ascomycetes in the order Hypocreales such as *Giberella zeae (Fusarium graminearum), G. monoliformis (Fusarium moniliforme), Fusarium solani* and *Verticillium dahliae;* |
| Ascomycetes in the order Eurotiales such as *Aspergillus flavus* and *A. parasiticus;* |
| Ascomycetes in the order Diaporthales such as *Cryptosphorella viticola (= Phomopsis viticola), Phomopsis longicolla,* and *Diaporthe phaseolorum;* |
| Other Ascomycete pathogens including *Magnaporthe grisea, Gaeumannomyces graminis, Rhynchosporium secalis,* and anthracnose pathogens such as *Glomerella acutata (Colletotrichum acutatum), G. graminicola (C. graminicola)* and *G. lagenaria (C. orbiculare);* |
| Basidiomycetes in the order Urediniales (the rusts) including *Puccinia recondita, P. striiformis, Puccinia hordei, P. graminis* and *P. arachidis), Hemileia vastatrix* and *Phakopsora pachyrhizi;* |
| Basidiomycetes in the order Ceratobasidiales such as *Thanatophorum cucumeris* (*Rhizoctonia solani)* and *Ceratobasidium oryzae-sativae* (*Rhizoctonia oryzae);* |
| Basidiomycetes in the order Polyporales such as *Athelia rolfsii* (*Sclerotium rolfsii)*; |
| Basidiomycetes in the order Ustilaginales such as *Ustilago maydis;* |
| Zygomycetes in the order Mucorales such as *Rhizopus stolonifer*; |
| Oomycetes in the order Pythiales, including *Phytophthora infestans, P. megasperma, P. parasitica, P. sojae, P. cinnamomi* and *P. capsici,* and *Pythium* pathogens such as *Pythium aphanidermatum, P. graminicola, P. irregulare, P. ultimum* and *P. dissoticum;* |
| Oomycetes in the order *Peronosporales* such as *Plasmopara viticola, P. halstedii, Peronospora hyoscyami (=Peronospora tabacina), P. manshurica, Hyaloperonospora parasitica (=Peronospora parasitica), Pseudoperonospora cubensis* and *Bremia lactucae*; |
| and other genera and species closely related to all of the above pathogens. |

In addition to their fungicidal activity, the compositions or combinations also have activity against bacteria such as *Erwinia amylovora, Xanthomonas campestris, Pseudomonas syringae,* and other related species. By controlling harmful microorganisms, the compositions of this invention are useful for improving (i.e. increasing) the ratio of beneficial to harmful microorganisms in contact with crop plants or their propagules (e.g., seeds, corms, bulbs, tubers, cuttings) or in the agronomic environment of the crop plants or their propagules.

Compositions of this invention are useful in treating all plants, plant parts and seeds. Plant and seed varieties and cultivars can be obtained by conventional propagation and breeding methods or by genetic engineering methods. Genetically modified plants or seeds (transgenic plants or seeds) are those in which a heterologous gene (transgene)

has been stably integrated into the plant's or seed's genome. A transgene that is defined by its particular location in the plant genome is called a transformation or transgenic event.

Genetically modified plant cultivars which can be treated according to the invention include those that are resistant against one or more biotic stresses (pests such as nematodes, insects, mites, fungi, etc.) or abiotic stresses (drought, cold temperature, soil salinity, etc.), or that contain other desirable characteristics. Plants can be genetically modified to exhibit traits of, for example, herbicide tolerance, insect-resistance, modified oil profiles or drought tolerance.

Treatment of genetically modified plants and seeds with compounds of the invention may result in super-additive or enhanced effects. For example, reduction in application rates, broadening of the activity spectrum, increased tolerance to biotic/abiotic stresses or enhanced storage stability may be greater than expected from just simple additive effects of the application of compounds of the invention on genetically modified plants and seeds.

Compounds and compositions of this invention are useful in seed treatments for protecting seeds from plant diseases. In the context of the present disclosure and claims, treating a seed means contacting the seed with a biologically effective amount of a compound of this invention, which is typically formulated as a composition of the invention. This seed treatment protects the seed from soil-borne disease pathogens and generally can also protect roots and other plant parts in contact with the soil of the seedling developing from the germinating seed. The seed treatment may also provide protection of foliage by translocation of the compound of this invention or a second active ingredient within the developing plant. Seed treatments can be applied to all types of seeds, including those from which plants genetically transformed to express specialized traits will germinate. Representative examples include those expressing proteins toxic to invertebrate pests, such as *Bacillus thuringiensis* toxin or those expressing herbicide resistance such as glyphosate acetyltransferase, which provides resistance to glyphosate. Seed treatments with compounds and compositions of this invention can also increase vigor of plants growing from the seed.

Compounds and compositions of this invention are particularly useful in seed treatment for crops including, but not limited to, maize or corn, soybeans, cotton, cereal (e.g., wheat, oats, barley, rye and rice), potatoes, vegetables and oilseed rape.

Furthermore, the compounds and compositions of this invention are useful in treating postharvest diseases of fruits and vegetables caused by fungi, oomycetes and bacteria. These infections can occur before, during and after harvest. For example, infections can occur before harvest and then remain dormant until some point during ripening (e.g., host begins tissue changes in such a way that infection can progress or conditions become conducive for disease development); also infections can arise from surface wounds created by mechanical or insect injury. In this respect, the compositions of this invention can reduce losses (i.e. losses resulting from quantity and quality) due to postharvest diseases which may occur at any time from harvest to consumption. Treatment of postharvest diseases with compounds of the invention can increase the period of time during which perishable edible plant parts (e.g., fruits, seeds, foliage, stems, bulbs, tubers) can be stored refrigerated or un-refrigerated after harvest, and remain edible and free from noticeable or harmful degradation or contamination by fungi or other microorganisms. Treatment of edible plant parts before or after harvest with compounds of the invention can also decrease the formation of toxic metabolites of fungi or other microorganisms, for example, mycotoxins such as aflatoxins.

Plant disease control is ordinarily accomplished by applying an effective amount of a compound of this invention either pre- or post-infection, to the portion of the plant to be protected such as the roots, stems, foliage, fruits, seeds, tubers or bulbs, or to the media (soil or sand) in which the plants to be protected are growing. The compounds can also be applied to seeds to protect the seeds and seedlings developing from the seeds. The compounds can also be applied through irrigation water to treat plants. Control of postharvest pathogens which infect the produce before harvest is typically accomplished by field application of a compound of this invention, and in cases where infection occurs after harvest the compounds can be applied to the harvested crop as dips, sprays, fumigants, treated wraps and box liners.

The compounds and compositions of this invention can also be applied using an unmanned aerial vehicle (UAV) for the dispension of the compositions disclosed herein over a planted area. In some embodiments the planted area is a crop-containing area. In some embodiments, the crop is selected from a monocot or dicot. In some embodiments, the crop is selected form rice, corn, barley, sobean, wheat, vegetable, tobacco, tea tree, fruit tree and sugar cane. In some embodiments, the compositions disclosed herein are formulated for spraying at an ultra-low volume. Products applied by drones may use water or oil as the spray carrier. Typical spray volume (including product) used for drone applications globally. 5.0 liters/ha - 100 liters/ha (approximately 0.5-10 gpa). This includes the range of ultra low spray volume (ULV) to low spray volume (LV). Although not common there may be situations where even lower spray volumes could be used as low as 1.0 liter/ha (0.1 gpa).

Suitable rates of application (e.g., fungicidally effective amounts) of component (a) (i.e. at least one compound selected from compounds of Formula **1**, *N*-oxides and salts thereof) as well as suitable rates of applicaton (e.g., biologically effective amounts, fungicidally effective amounts or insecticidally effective amounts) for the mixtures and compositions comprising component (a) according to this invention can be influenced by factors such as the plant diseases to be controlled, the plant species to be protected, the population structure of the pathogen to be controlled, ambient moisture and temperature and should be determined under actual use conditions. One skilled in the art can easily determine

through simple experimentation the fungicidally effective amount necessary for the desired level of plant disease control. Foliage can normally be protected when treated at a rate of from less than about 1 g/ha to about 5,000 g/ha of active ingredient. Seed and seedlings can normally be protected when seed is treated at a rate of from about 0.001 g (more typically about 0.1 g) to about 10 g per kilogram of seed. One skilled in the art can easily determine through simple experimentation the application rates of component (a), and mixtures and compositions thereof, containing particular combinations of active ingredients according to this invention needed to provide the desired spectrum of plant protection and control of plant diseases and optionally other plant pests.

Compounds and compositions of the present invention may also be useful for increasing vigor of a crop plant. This method comprises contacting the crop plant (e.g., foliage, flowers, fruit or roots) or the seed from which the crop plant is grown with a composition comprising a compound of Formula **1** in amount sufficient to achieve the desired plant vigor effect (i.e. biologically effective amount). Typically the compound of Formula **1** is applied in a formulated composition. Although the compound of Formula **1** is often applied directly to the crop plant or its seed, it can also be applied to the locus of the crop plant, i.e. the environment of the crop plant, particularly the portion of the environment in close enough proximity to allow the compound of Formula **1** to migrate to the crop plant. The locus relevant to this method most commonly comprises the growth medium (i.e. medium providing nutrients to the plant), typically soil in which the plant is grown. Treatment of a crop plant to increase vigor of the crop plant thus comprises contacting the crop plant, the seed from which the crop plant is grown or the locus of the crop plant with a biologically effective amount of a compound of Formula **1.**

Increased crop vigor can result in one or more of the following observed effects: (a) optimal crop establishment as demonstrated by excellent seed germination, crop emergence and crop stand; (b) enhanced crop growth as demonstrated by rapid and robust leaf growth (e.g., measured by leaf area index), plant height, number of tillers (e.g., for rice), root mass and overall dry weight of vegetative mass of the crop; (c) improved crop yields, as demonstrated by time to flowering, duration of flowering, number of flowers, total biomass accumulation (i.e. yield quantity) and/or fruit or grain grade marketability of produce (i.e. yield quality); (d) enhanced ability of the crop to withstand or prevent plant disease infections and arthropod, nematode or mollusk pest infestations; and (e) increased ability of the crop to withstand environmental stresses such as exposure to thermal extremes, suboptimal moisture or phytotoxic chemicals.

The compounds and compositions of the present invention may increase the vigor of treated plants compared to untreated plants by preventing and/or curing plant diseases caused by fungal plant pathogens in the environment of the plants. In the absence of such control of plant diseases, the diseases reduce plant vigor by consuming plant tissues or sap, or transmiting plant pathogens such as viruses. Even in the absence of fungal plant pathogens, the compounds of the invention may increase plant vigor by modifying metabolism of plants. Generally, the vigor of a crop plant will be most significantly increased by treating the plant with a compound of the invention if the plant is grown in a nonideal environment, i.e. an environment comprising one or more aspects adverse to the plant achieving the full genetic potential it would exhibit in an ideal environment.

Of note is a method for increasing vigor of a crop plant wherein the crop plant is grown in an environment comprising plant diseases caused by fungal plant pathogens. Also of note is a method for increasing vigor of a crop plant wherein the crop plant is grown in an environment not comprising plant diseases caused by fungal plant pathogens. Also of note is a method for increasing vigor of a crop plant wherein the crop plant is grown in an environment comprising an amount of moisture less than ideal for supporting growth of the crop plant.

Compounds and compositions of this invention can also be mixed with one or more other biologically active compounds or agents including fungicides, insecticides, nematicides, bactericides, acaricides, herbicides, herbicide safeners, growth regulators such as insect molting inhibitors and rooting stimulants, chemosterilants, semiochemicals, repellents, attractants, pheromones, feeding stimulants, plant nutrients, other biologically active compounds or entomopathogenic bacteria, virus or fungi to form a multi-component pesticide giving an even broader spectrum of agricultural protection. Thus the present invention also pertains to a composition comprising a compound of Formula **1** (in a fungicidally effective amount) and at least one additional biologically active compound or agent (in a biologically effective amount) and can further comprise at least one of a surfactant, a solid diluent or a liquid diluent. The other biologically active compounds or agents can be formulated in compositions comprising at least one of a surfactant, solid or liquid diluent. For mixtures of the present invention, one or more other biologically active compounds or agents can be formulated together with a compound of Formula **1,** to form a premix, or one or more other biologically active compounds or agents can be formulated separately from the compound of Formula **1,** and the formulations combined together before application (e.g., in a spray tank) or, alternatively, applied in succession.

As mentioned in the Summary of the Invention, one aspect of the present invention is a fungicidal composition comprising (i.e. a mixture or combination of) a compound of Formula **1,** an *N*-oxide, or a salt thereof (i.e. component (a)), and at least one other fungicide (i.e. component (b)). Of note is such a combination where the other fungicidal active ingredient has different site of action from the compound of Formula **1.** In certain instances, a combination with at least one other fungicidal active ingredient having a similar spectrum of control but a different site of action will be particularly advantageous for resistance management. Thus, a composition of the present invention can further comprise a fungicidally effective amount of at least one additional fungicidal active ingredient having a similar spectrum of control but a different site of action.

Examples of component (b) fungicides include acibenzolar-S-methyl, aldimorph, ametoctradin, amisulbrom, anilazine, azaconazole, azoxystrobin, benalaxyl (including benalaxyl-M), benodanil, benomyl, benthiavalicarb (including benthiavalicarb-isopropyl), benzovindiflupyr, bethoxazin, binapacryl, biphenyl, bitertanol, bixafen, blasticidin-S, boscalid, bromuconazole, bupirimate, buthiobate, captafol, captan, carbendazim, carboxin, carpropamid, chloroneb, chlorothalonil, chlozolinate, clotrimazole, copper hydroxide (e.g., Kocide), copper oxychloride, copper sulfate, coumoxystrobin, cyazofamid, cyflufenamid, cymoxanil, cyproconazole, cyprodinil, dichlofluanid, diclocymet, diclomezine, dicloran, diethofencarb, difenoconazole, diflumetorim, dimethirimol, dimethomorph, dimoxystrobin, diniconazole (including diniconazole-M), dinocap, dithianon, dithiolanes, dodemorph, dodine, dipymetitrone, econazole, edifenphos, enoxastrobin (also known as enestroburin), epoxiconazole, etaconazole, ethaboxam, ethirimol, etridiazole, famoxadone, fenamidone, fenarimol, fenaminstrobin, fenbuconazole, fenfuram, fenhexamid, fenoxanil, fenpiclonil, fenpropidin, fenpropimorph, fenpyrazamine, fentin acetate, fentin chloride, fentin hydroxide, ferbam, ferimzone, flometoquin, florylpicoxamid, fluazinam, fludioxonil, flufenoxystrobin, fluindapyr, flumorph, fluopicolide, fluopimomide, fluopyram, flouroimide, fluoxastrobin, fluquinconazole, flusilazole, flusulfamide, flutianil, flutolanil, flutriafol, fluxapyroxad, folpet, fthalide, fuberidazole, furalaxyl, furametpyr, guazatine, hexaconazole, hymexazole, imazalil, imibenconazole, iminoctadine albesilate, iminoctadine triacetate, iodocarb, ipconazole, ipfentrifluconazole, iprobenfos, iprodione, iprovalicarb, isoconazole, isofetamid, isoprothiolane, isoflucypram, isopyrazam, isotianil, kasugamycin, kresoxim-methyl, mancozeb, mandepropamid, mandestrobin, maneb, mepanipyrim, mepronil, meptyldinocap, metalaxyl (including metalaxyl-M/mefenoxam), mefentrifluconazole, metconazole, methasulfocarb, metiram, metominostrobin, metrafenone, miconazole, myclobutanil, naftifine, neo-asozin, nuarimol, octhilinone, ofurace, orysastrobin, oxadixyl, oxathiapiprolin, oxolinic acid, oxpoconazole, oxycarboxin, oxytetracycline, pefurazoate, penconazole, pencycuron, penflufen, penthiopyrad, phosphorous acid (including salts thereof, e.g., fosetyl-aluminum), picarbutrazox, picoxystrobin, piperalin, polyoxin, probenazole, prochloraz, procymidone, propamacarb, propiconazole, propineb, proquinazid, prothiocarb, prothioconazole, pydiflumetofen, pyraclostrobin, pyrametostrobin, pyraoxystrobin, pyrazophos, pyribencarb, pyributicarb, pyrifenox, pyrimethanil, pyriofenone, pyrisoxazole, pyroquilon, pyrrolnitrin, quinconazole, quinofumelin (Registry Number 861647-84-9) quinomethionate, quinoxyfen, quintozene, sedaxane, silthiofam, simeconazole, spiroxamine, streptomycin, sulfur, tebuconazole, tebufloquin, teclofthalam, tecnazene, terbinafine, tetraconazole, thiabendazole, thifluzamide, thiophanate, thiophanate-methyl, thiram, tiadinil, tolclofos-methyl, tolnifanide, tolprocarb, tolyfluanid, triadimefon, triadimenol, triarimol, triticonazole, triazoxide, tribasic copper sulfate, tricyclazole, triclopyricarb, tridemorph, trifloxystrobin, triflumizole, triforine, trimorphamide, uniconazole, uniconazole-P, validamycin, valifenalate (also known as valiphenal), vinclozolin, zineb, ziram, zoxamide, *N*-[2-(1*S*,2*R*)-[1,1'-bicyclopropyl]-2-ylphenyl]-3-(difluoromethyl)-1-methyl-1*H*pyrazole-4-carboxamide, α-(1-chlorocyclopropyl)-α-[2-(2,2-dichlorocyclopropyl)ethyl]-1*H*-1,2,4-triazole-1-ethanol, (α*S*)-[3-(4-chloro-2-fluorophenyl)-5-(2,4-difluorophenyl)-4-isoxazolyl]-3-pyridinemethanol, *rel*-1-[[(2*R*,3*S*)-3-(2-chlorophenyl)-2-(2,4-difluorophenyl)-2-oxiranyl]methyl]-1*H*-1,2,4-triazole, *rel*-2-[[(2*R*,3*S*)-3-(2-chlorophenyl)-2-(2,4-difluorophenyl)-2-oxiranyl]methyl]-1,2-dihydro-3*H*-1,2,4-triazole-3-thione, *rel*-1-[[(2*R*,3*S*)-3-(2-chlorophenyl)-2-(2,4-difluorophenyl)-2-oxiranyl]-methyl]-5-(2-propen-1-ylthio)-1*H*-1,2,4-triazole, *N*-[2-[4-[[3-(4-chlorophenyl)-2-propyn-1-yl]-oxy]-3-methoxyphenyl]ethyl]-3-methyl-2-[(methylsulfonyl)amino]butanamide, *N*-[2-[4-[[3-(4-chlorophenyl)-2-propyn-1-yl]oxy]-3-methoxyphenyl]ethyl]-3-methyl-2-[(ethylsulfonyl)amino]-butanamide, *N'*-[4-[4-chloro-3-(trifluoromethyl)phenoxy]-2,5-dimethylphenyl]-*N*-ethyl-*N*-methylmethanimidamide, *N*-[[(cyclopropylmethoxy)amino][6-(difluoromethoxy)-2,3-difluorophenyl]methylene]benzeneacetamide, *N*-[2-(2,4-dichlorophenyl)-2-methoxy-1-methylethyl]-3-(difluoromethyl)-1-methyl-1*H*-pyrazole-4-carboxamide, *N*-(3',4'-difluoro[1,1'-biphenyl]-2-yl)-3-(trifluoromethyl)-2-pyrazinecarboxamide, 3-(difluoromethyl)-*N*-(2,3-dihydro-1,1,3 -trimethyl-1*H*-inden-4-yl)-1-methyl-1*H*-pyrazole-4-carboxamide, 5,8-difluoro-*N*-[2-[3-methoxy-4-[[4-(trifluoromethyl)-2-pyridinyl]oxy]phenyl]ethyl]-4-quinazolinamine, 1-[4-[4-[5*R*-[(2,6-difluoro-phenoxy)methyl]-4,5-dihydro-3-isoxazolyl]-2-thiazolyl]-1-piperdinyl]-2-[5-methyl-3-(trifluoromethyl)-1*H*-pyrazol-1-yl]ethanone, 4-fluorophenyl *N*-[1-[[[1-(4-cyanophenyl)ethyl]-sulfonyl]methyl]propyl]carbamate, 5-fluoro-2-[(4-fluorophenyl)methoxy]-4-pyrimidinamine, α-(methoxyimino)-*N*-methyl-2-[[[1-[3-(trifluoromethyl)phenyl]ethoxy]imino]methyl]benzeneacetamide, [[4-methoxy-2-[[[(3*S*,7*R*,8*R*,9*S*)-9-methyl-8-(2-methyl-1-oxopropoxy)-2,6-dioxo-7-(phenylmethyl)-1,5-dioxonan-3-yl]amino]carbonyl]-3-pyridinyl]oxy]methyl 2-methylpropanoate, methyl *N*-[[5-[1-[2,6-difluoro-4-(1-methylethyl)phenyl]-1*H*-pyrazol-3-yl]-2-methylpheny1] methyl]carbamate (b54.11a), methyl *N*-[[5-[1-(4-cyclopropyl-2,6-difluorophenyl)-1*H*-pyrazol-3-yl]-2-methylphenyl]methyl]carbamate (b54.11d), ethyl 1-[[4-[[(1*Z*)-2-ethoxy-3,3,3-trifluoro-1-propen-1-yl]oxy]phenyl]methyl]-1*H*-pyrazole-4-carboxylate and its (*E*)-isomer (b54.12a) and ethyl 1-[[4-[[2-(trifluoromethyl)-1,3-dioxolan-2-yl]methoxy]phenyl]methyl]-1*H*-pyrazole-4-carboxylate (b54.13a).

Therefore of note is a fungicidal composition comprising as component (a) a compound of Formula **1** (or an *N*-oxide or salt thereof) and as component (b) at least one fungicide selected from the preceding list.

Of particular note are combinations of compounds of Formula **1** (or an *N*-oxide or salt thereof) (i.e. Component (a) in compositions) with component (b) compounds selected from aminopyrifen (Registry Number 1531626-08-0), azoxystrobin, benzovindiflupyr, bixafen, captan, carpropamid, chlorothalonil, copper hydroxide (e.g., Kocide), copper

oxychloride, copper sulfate, cymoxanil, cyproconazole, cyprodinil, dichlobentiazox (Registry Number 957144-77-3), diethofencarb, difenoconazole, dimethomorph, dipymetitrone, epoxiconazole, ethaboxam, fenarimol, fenhexamid, fenpropimorph, fluazinam, fludioxonil, fluindapyr, fluopyram, flusilazole, flutianil, flutriafol, fluxapyroxad, folpet, ipflufenoquin (Registry Number 1314008-27-9), iprodione, isofetamid, isoflucypram, isopyrazam, kresoxim-methyl, mancozeb, mandestrobin, meptyldinocap, metalaxyl (including metalaxyl-M/mefenoxam), mefentrifluconazole, metconazole, metrafenone, metyltetraprole (Registry Number 1472649-01-6), myclobutanil, oxathiapiprolin, penflufen, penthiopyrad, phosphorous acid (including salts thereof, e.g., fosetyl-aluminum), picoxystrobin, propiconazole, proquinazid, prothioconazole, pydiflumetofen, pyridachlometyl (Registry Number 1358061-55-8), pyraclostrobin, pyrapropoyne (Registry Number 1803108-03-3), pyrimethanil, sedaxane spiroxamine, sulfur, tebuconazole, thiophanate-methyl, trifloxystrobin, zoxamide, α-(1-chlorocyclopropyl)-α-[2-(2,2-dichlorocyclopropyl)ethyl]-1*H*-1,2,4-triazole-1-ethanol, *N*-[2-(2,4-dichlorophenyl)-2-methoxy-1-methylethyl]-3-(difluoromethyl)-1-methyl-1*H*-pyrazole-4-carboxamide, 3-(difluoromethyl)-*N*-(2,3-dihydro-1,1,3-trimethyl-1*H*-inden-4-yl)-1-methyl-1*H*-pyrazole-4-carboxamide, 1-[4-[4-[5*R*-(2,6-difluorophenyl)-4,5-dihydro-3-isoxazolyl]-2-thiazolyl]-1-piperidinyl]-2-[5-methyl-3-(trifluoromethyl)-1*H*-pyrazol-1-yl]ethanone, 1,1-dimethylethyl *N*-[6-[[[[(1-methyl-1*H*-tetrazol-5-yl)phenylmethylene]amino]oxy]methyl]-2-pyridinyl]carbamate, 5-fluoro-2-[(4-fluorophenyl)-methoxy]-4-pyrimidinamine, (α*S*)-[3-(4-chloro-2-fluorophenyl)-5-(2,4-difluorophenyl)-4-isoxazolyl]-3-pyridinemethanol, *rel*-1-[[(2*R*,3*S*)-3-(2-chlorophenyl)-2-(2,4-difluorophenyl)-2-ox-iranyl]methyl]-1*H*-1,2,4-triazole, *rel*-2-[[(2*R*,3*S*)-3-(2-chlorophenyl)-2-(2,4-difluorophenyl)-2-oxiranyl]methyl]-1,2-dihydro-3*H*-1,2,4-triazole-3-thione, *rel*-1-[[(2*R*,3*S*)-3-(2-chlorophenyl)-2-(2,4-difluorophenyl)-2-oxiranyl]methyl]-5-(2-propen-1-ylthio)-1*H*-1,2,4-triazole, methyl *N*-[[5-[1-[2,6-difluoro-4-(1-methylethyl)phenyl]-1*H*-pyrazol-3-yl]-2-methylphenyl]methyl]carbamate (b54.11a), methyl *N*-[[5-[1-(4-cyclopropyl-2,6-diffuorophenyl)-1*H*-pyrazol-3-yl]-2-methylphenyl]methyl]carbamate (b54.11d), ethyl 1-[[4-[[(1*Z*)-2-ethoxy-3,3,3-trifluoro-1-propen-1-yl]oxy]phenyl]methyl]-1*H*-pyrazole-4-carboxylate and its (*E*)-isomer (b54.12a) and ethyl 1-[[4-[[2-(trifluoromethyl)-1,3-dioxolan-2-yl]methoxy]phenyl]methyl]-1*H*-pyrazole-4-carboxylate (b54.13a) (i.e. as Component (b) in compositons).

Generally preferred for better control of plant diseases caused by fungal plant pathogens (e.g., lower use rate or broader spectrum of plant pathogens controlled) or resistance management are mixtures of a compound of Formula **1,** an *N*-oxide, or salt thereof, with a fungicidal compound selected from the group: amisulbrom, azoxystrobin, benzovindiflupyr, bixafen, boscalid, carbendazim, carboxin, chlorothalonil, copper hydroxide (e.g., Kocide), cymoxanil, cyproconazole, difenoconazole, dimethomorph, dimoxystrobin, epoxiconazole, fenpropimorph, florylpicoxamid, fluazinam, fludioxonil, flufenoxystrobin, fluindapyr, fluquinconazole, fluopicolide, fluoxastrobin, flutriafol, fluxapyroxad, ipconazole, ipfentrifluconazole, iprodione, kresoxim-methyl, mancozeb, metalaxyl, mefenoxam, mefentrifluconazole, metconazole, metominostrobin, myclobutanil, paclobutrazole, penflufen, picoxystrobin, prothioconazole, pydiflumetofen, pyraclostrobin, pyrametostrobin, pyraoxystrobin, pyriofenone, sedaxane, silthiofam, tebuconazole, thiabendazole, thiophanate-methyl, thiram, trifloxystrobin, triticonazole, methyl *N*-[[5-[1-[2,6-diffuoro-4-(1-methylethyl)phenyl]-1*H*-pyrazol-3-yl]-2-methylphenyl]methyl]carbamate (b54.11a), methyl *N*-[[5-[1-(4-cyclopropyl-2,6-difluorophenyl)-1*H*-pyrazol-3-yl]-2-methylphenyl]methyl]carbamate (b54.11d), ethyl 1-[[4-[[(1*Z*)-2-ethoxy-3,3,3-triffuoro-1-propen-1-yl]oxy]phenyl]methyl]-1*H*-pyrazole-4-carboxylate and its (*E*)-isomer (b54.12a) and ethyl 1-[[4-[[2-(trifluoromethyl)-1,3-dioxolan-2-yl]methoxy]phenyl]methyl]-1*H*-pyrazole-4-carboxylate (b54.13a).

In the fungicidal compositions of the present invention, component (a) (i.e. at least one compound selected from compounds of Formula **1,** *N*-oxides, and salts thereof) and component (b) are present in fungicidally effective amounts. The weight ratio of component (a) component to component (b) (i.e. one or more additional fungicidal compounds) to is generally between about 1:3000 to about 3000: 1, and more typically between about 1:500 and about 500:1. Of note are compositions where in the weight ratio of component (a) to component (b) is from about 125:1 to about 1:125. With many fungicidal compounds of component (b), these compositions are particularly effective for controlling plant diseases caused by fungal plant pathogens. Of particular note are compositions wherein the weight ratio of component (a) to component (b) is from about 25:1 to about 1:25, or from about 5:1 to about 1:5. One skilled in the art can easily determine through simple experimentation the weight ratios and application rates of fungicidal compounds necessary for the desired spectrum of fungicidal protection and control. It will be evident that including additional fungicidal compounds in component (b) may expand the spectrum of plant diseases controlled beyond the spectrum controlled by component (a) alone. Furthermore, Tables A1 through A26 and C1 through C26 exemplify weight ratios for combinations of fungicidal compounds of the present invention. Table B1 lists typical, more typical and most typical ranges of ratios involving particular fungicidal compounds of component (b).

Specific mixtures (compound numbers refer to compounds in Index Tables A through T) are listed in Tables A1 through A26. In Table A1, each line below the column headings "Component (a)" and "Component (b)" specifically discloses a mixture of Component (a), (i.e. Compound 21), with a Component (b) fungicidal compound. The entries under the heading "Illustrative Ratios" disclose three specific weight ratios of Component (a) to Component (b) for the disclosed mixture. For example, the first line of Table A1 discloses a mixture of Compound 21 with acibenzolar-S-methyl and lists weight ratios of Compound 21 relative to acibenzolar-S-methyl of 1:1, 1:4 or 1:18.

Table A1

| Component (a) | Component (b) | Illustrative Ratios(*) | | |
|---|---|---|---|---|
| Compound 21 | acibenzolar-S-methyl | 1:1 | 1:4 | 1:18 |
| Compound 21 | aldimorph | 7:1 | 3:1 | 1:1 |
| Compound 21 | ametoctradin | 3:1 | 1:1 | 1:3 |
| Compound 21 | amisulbrom | 1:1 | 1:2 | 1:6 |
| Compound 21 | anilazine | 22:1 | 8:1 | 4:1 |
| Compound 21 | azaconazole | 2:1 | 1:2 | 1:4 |
| Compound 21 | azoxystrobin | 3:1 | 1:1 | 1:3 |
| Compound 21 | benalaxyl | 1:1 | 1:2 | 1:6 |
| Compound 21 | benalaxyl-M | 1:1 | 1:3 | 1:8 |
| Compound 21 | benodanil | 4:1 | 2:1 | 1:2 |
| Compound 21 | benomyl | 11:1 | 4:1 | 1:1 |
| Compound 21 | benthiavalicarb | 1:1 | 1:4 | 1:12 |
| Compound 3 | benthiavalicarb-isopropyl | 1:1 | 1:4 | 1:12 |
| Compound 21 | bethoxazin | 15:1 | 5:1 | 2:1 |
| Compound 21 | binapacryl | 15:1 | 5:1 | 2:1 |
| Compound 21 | biphenyl | 15:1 | 5:1 | 2:1 |
| Compound 21 | bitertanol | 3:1 | 1:1 | 1:2 |
| Compound 21 | bixafen | 2:1 | 1:1 | 1:3 |
| Compound 21 | blasticidin-S | 1:4 | 1:12 | 1:30 |
| Compound 21 | Bordeaux mixture (tribasic copper sulfate) | 45:1 | 15:1 | 5:1 |
| Compound 21 | boscalid | 4:1 | 2:1 | 1:2 |
| Compound 21 | bromuconazole | 3:1 | 1:1 | 1:3 |
| Compound 21 | bupirimate | 1:3 | 1:10 | 1:30 |
| Compound 21 | captafol | 15:1 | 5:1 | 2:1 |
| Compound 21 | captan | 15:1 | 5:1 | 2:1 |
| Compound 21 | carbendazim | 11:1 | 4:1 | 2:1 |
| Compound 21 | carboxin | 4:1 | 2:1 | 1:2 |
| Compound 21 | carpropamid | 3:1 | 1:1 | 1:3 |
| Compound 21 | chloroneb | 100:1 | 35:1 | 14:1 |
| Compound 21 | chlorothalonil | 15:1 | 5:1 | 2:1 |
| Compound 21 | chlozolinate | 11:1 | 4:1 | 2:1 |
| Compound 21 | clotrimazole | 3:1 | 1:1 | 1:3 |
| Compound 21 | copper hydroxide | 45:1 | 15:1 | 5:1 |
| Compound 21 | copper oxychloride | 45:1 | 15:1 | 5:1 |
| Compound 21 | cyazofamid | 1:1 | 1:2 | 1:6 |
| Compound 21 | cyflufenamid | 1:2 | 1:6 | 1:24 |
| Compound 21 | cymoxanil | 1:1 | 1:2 | 1:5 |
| Compound 21 | cyproconazole | 1:1 | 1:2 | 1:6 |
| Compound 21 | cyprodinil | 4:1 | 2:1 | 1:2 |
| Compound 21 | dichlofluanid | 15:1 | 5:1 | 2:1 |
| Compound 21 | diclocymet | 15:1 | 5:1 | 2:1 |
| Compound 21 | diclomezine | 3:1 | 1:1 | 1:3 |
| Compound 21 | dicloran | 15:1 | 5:1 | 2:1 |
| Compound 21 | diethofencarb | 7:1 | 2:1 | 1:2 |
| Compound 21 | difenoconazole | 1:1 | 1:3 | 1:12 |
| Compound 21 | diflumetorim | 15:1 | 5:1 | 2:1 |
| Compound 21 | dimethirimol | 1:3 | 1:8 | 1:30 |
| Compound 21 | dimethomorph | 3:1 | 1:1 | 1:2 |
| Compound 21 | dimoxystrobin | 2:1 | 1:1 | 1:4 |

(continued)

| Component (a) | Component (b) | Illustrative Ratios(*) | | |
|---|---|---|---|---|
| Compound 21 | diniconazole | 1:1 | 1:3 | 1:8 |
| Compound 21 | diniconazole-M | 1:1 | 1:3 | 1:12 |
| Compound 21 | dinocap | 2:1 | 1:1 | 1:3 |
| Compound 21 | dithianon | 5:1 | 2:1 | 1:2 |
| Compound 21 | dodemorph | 7:1 | 3:1 | 1:1 |
| Compound 21 | dodine | 10:1 | 4:1 | 2:1 |
| Compound 21 | edifenphos | 3:1 | 1:1 | 1:3 |
| Compound 21 | enestroburin | 2:1 | 1:1 | 1:4 |
| Compound 21 | epoxiconazole | 1:1 | 1:3 | 1:7 |
| Compound 21 | etaconazole | 1:1 | 1:3 | 1:7 |
| Compound 21 | ethaboxam | 2:1 | 1:1 | 1:3 |
| Compound 21 | ethirimol | 7:1 | 3:1 | 1:1 |
| Compound 21 | etridiazole | 7:1 | 2:1 | 1:2 |
| Compound 21 | famoxadone | 2:1 | 1:1 | 1:4 |
| Compound 21 | fenamidone | 2:1 | 1:1 | 1:4 |
| Compound 21 | fenaminstrobin | 3:1 | 1:1 | 1:3 |
| Compound 21 | fenarimol | 1:2 | 1:7 | 1:24 |
| Compound 21 | fenbuconazole | 1:1 | 1:3 | 1:10 |
| Compound 21 | fenfuram | 4:1 | 1:1 | 1:2 |
| Compound 21 | fenhexamid | 10:1 | 4:1 | 2:1 |
| Compound 21 | fenoxanil | 15:1 | 4:1 | 1:1 |
| Compound 21 | fenpiclonil | 15:1 | 5:1 | 2:1 |
| Compound 21 | fenpropidin | 7:1 | 2:1 | 1:1 |
| Compound 21 | fenpropimorph | 7:1 | 2:1 | 1:1 |
| Compound 21 | fenpyrazamine | 3:1 | 1:1 | 1:3 |
| Compound 21 | fentin salt such as fentin acetate, fentin chloride or fentin hydroxide | 3:1 | 1:1 | 1:3 |
| Compound 21 | ferbam | 30:1 | 10:1 | 4:1 |
| Compound 21 | ferimzone | 7:1 | 2:1 | 1:2 |
| Compound 21 | fluazinam | 3:1 | 1:1 | 1:2 |
| Compound 21 | fludioxonil | 2:1 | 1:1 | 1:4 |
| Compound 21 | flumetover | 3:1 | 1:1 | 1:2 |
| Compound 21 | flumorph | 3:1 | 1:1 | 1:3 |
| Compound 21 | fluopicolide | 1:1 | 1:2 | 1:6 |
| Compound 21 | fluopyram | 3:1 | 1:1 | 1:3 |
| Compound 21 | fluoroimide | 37:1 | 14:1 | 5:1 |
| Compound 21 | fluoxastrobin | 1:1 | 1:2 | 1:6 |
| Compound 21 | fluquinconazole | 1:1 | 1:2 | 1:4 |
| Compound 21 | flusilazole | 3:1 | 1:1 | 1:3 |
| Compound 21 | flusulfamide | 15:1 | 5:1 | 2:1 |
| Compound 21 | flutianil | 1:1 | 1:2 | 1:6 |
| Compound 21 | flutolanil | 4:1 | 1:1 | 1:2 |
| Compound 21 | flutriafol | 1:1 | 1:2 | 1:4 |
| Compound 21 | fluxapyroxad | 2:1 | 1:1 | 1:3 |
| Compound 21 | folpet | 15:1 | 5:1 | 2:1 |
| Compound 21 | fosetyl-aluminum | 30:1 | 12:1 | 5:1 |
| Compound 21 | fuberidazole | 11:1 | 4:1 | 2:1 |
| Compound 21 | furalaxyl | 1:1 | 1:2 | 1:6 |
| Compound 21 | furametpyr | 15:1 | 5:1 | 2:1 |
| Compound 21 | guazatine | 15:1 | 5:1 | 2:1 |
| Compound 21 | hexaconazole | 1:1 | 1:2 | 1:5 |

(continued)

| Component (a) | Component (b) | Illustrative Ratios(*) | | |
|---|---|---|---|---|
| Compound 21 | hymexazol | 75:1 | 25:1 | 9:1 |
| Compound 21 | imazalil | 1:1 | 1:2 | 1:5 |
| Compound 21 | imibenconazole | 1:1 | 1:2 | 1:5 |
| Compound 21 | iminoctadine | 15:1 | 4:1 | 1:1 |
| Compound 21 | iodocarb | 15:1 | 5:1 | 2:1 |
| Compound 21 | ipconazole | 1:1 | 1:2 | 1:5 |
| Compound 21 | iprobenfos | 15:1 | 5:1 | 2:1 |
| Compound 21 | iprodione | 15:1 | 5:1 | 2:1 |
| Compound 21 | iprovalicarb | 2:1 | 1:1 | 1:3 |
| Compound 21 | isoprothiolane | 45:1 | 15:1 | 5:1 |
| Compound 21 | isopyrazam | 2:1 | 1:1 | 1:3 |
| Compound 21 | isotianil | 2:1 | 1:1 | 1:3 |
| Compound 21 | kasugamycin | 1:2 | 1:7 | 1:24 |
| Compound 21 | kresoxim-methyl | 2:1 | 1:1 | 1:4 |
| Compound 21 | mancozeb | 22:1 | 7:1 | 3:1 |
| Compound 21 | mandipropamid | 2:1 | 1:1 | 1:4 |
| Compound 21 | maneb | 22:1 | 7:1 | 3:1 |
| Compound 21 | mepanipyrim | 6:1 | 2:1 | 1:1 |
| Compound 21 | mepronil | 1:1 | 1:2 | 1:6 |
| Compound 21 | meptyldinocap | 2:1 | 1:1 | 1:3 |
| Compound 21 | metalaxyl | 1:1 | 1:2 | 1:6 |
| Compound 21 | metalaxyl-M | 1:1 | 1:4 | 1:12 |
| Compound 21 | metconazole | 1:1 | 1:2 | 1:6 |
| Compound 21 | methasulfocarb | 15:1 | 5:1 | 2:1 |
| Compound 21 | metiram | 15:1 | 5:1 | 2:1 |
| Compound 21 | metominostrobin | 3:1 | 1:1 | 1:3 |
| Compound 21 | metrafenone | 2:1 | 1:1 | 1:4 |
| Compound 21 | myclobutanil | 1:1 | 1:3 | 1:8 |
| Compound 21 | naftifine | 15:1 | 5:1 | 2:1 |
| Compound 21 | neo-asozin (ferric methanearsonate) | 15:1 | 5:1 | 2:1 |
| Compound 21 | nuarimol | 3:1 | 1:1 | 1:3 |
| Compound 21 | octhilinone | 15:1 | 4:1 | 1:1 |
| Compound 21 | ofurace | 1:1 | 1:2 | 1:6 |
| Compound 21 | orysastrobin | 3:1 | 1:1 | 1:3 |
| Compound 21 | oxadixyl | 1:1 | 1:2 | 1:6 |
| Compound 21 | oxolinic acid | 7:1 | 2:1 | 1:2 |
| Compound 21 | oxpoconazole | 1:1 | 1:2 | 1:5 |
| Compound 21 | oxycarboxin | 4:1 | 1:1 | 1:2 |
| Compound 21 | oxytetracycline | 3:1 | 1:1 | 1:3 |
| Compound 21 | pefurazoate | 15:1 | 5:1 | 2:1 |
| Compound 21 | penconazole | 1:2 | 1:6 | 1:15 |
| Compound 21 | pencycuron | 11:1 | 4:1 | 2:1 |
| Compound 21 | penflufen | 2:1 | 1:1 | 1:3 |
| Compound 21 | penthiopyrad | 2:1 | 1:1 | 1:3 |
| Compound 21 | phosphorous acid or a salt thereof | 15:1 | 6:1 | 2:1 |
| Compound 21 | phthalide | 15:1 | 6:1 | 2:1 |
| Compound 21 | picoxystrobin | 1:1 | 1:2 | 1:5 |
| Compound 21 | piperalin | 3:1 | 1:1 | 1:3 |
| Compound 21 | polyoxin | 3:1 | 1:1 | 1:3 |
| Compound 21 | probenazole | 3:1 | 1:1 | 1:3 |

(continued)

| Component (a) | Component (b) | Illustrative Ratios(*) | | |
|---|---|---|---|---|
| Compound 21 | prochloraz | 7:1 | 2:1 | 1:2 |
| Compound 21 | procymidone | 11:1 | 4:1 | 2:1 |
| Compound 21 | propamocarb or propamocarb-hydrochloride | 10:1 | 4:1 | 2:1 |
| Compound 21 | propiconazole | 1:1 | 1:2 | 1:5 |
| Compound 21 | propineb | 11:1 | 4:1 | 2:1 |
| Compound 21 | proquinazid | 1:1 | 1:3 | 1:12 |
| Compound 21 | prothiocarb | 3:1 | 1:1 | 1:3 |
| Compound 21 | prothioconazole | 1:1 | 1:2 | 1:5 |
| Compound 21 | pyraclostrobin | 2:1 | 1:1 | 1:4 |
| Compound 21 | pyrametostrobin | 2:1 | 1:1 | 1:4 |
| Compound 21 | pyraoxystrobin | 2:1 | 1:1 | 1:4 |
| Compound 21 | pyrazophos | 15:1 | 4:1 | 1:1 |
| Compound 21 | pyribencarb | 4:1 | 1:1 | 1:2 |
| Compound 21 | pyributicarb | 15:1 | 4:1 | 1:1 |
| Compound 21 | pyrifenox | 3:1 | 1:1 | 1:3 |
| Compound 21 | pyrimethanil | 3:1 | 1:1 | 1:2 |
| Compound 21 | pyriofenone | 2:1 | 1:1 | 1:4 |
| Compound 21 | pyrisoxazole | 3:1 | 1:1 | 1:3 |
| Compound 21 | pyroquilon | 3:1 | 1:1 | 1:3 |
| Compound 21 | pyrrolnitrin | 15:1 | 5:1 | 2:1 |
| Compound 21 | quinconazole | 1:1 | 1:2 | 1:4 |
| Compound 21 | quinomethionate | 15:1 | 5:1 | 2:1 |
| Compound 21 | quinoxyfen | 1:1 | 1:2 | 1:6 |
| Compound 21 | quintozene | 15:1 | 5:1 | 2:1 |
| Compound 21 | silthiofam | 2:1 | 1:1 | 1:4 |
| Compound 21 | simeconazole | 1:1 | 1:2 | 1:5 |
| Compound 21 | spiroxamine | 5:1 | 2:1 | 1:2 |
| Compound 21 | streptomycin | 3:1 | 1:1 | 1:3 |
| Compound 21 | sulfur | 75:1 | 25:1 | 9:1 |
| Compound 21 | tebuconazole | 1:1 | 1:2 | 1:5 |
| Compound 21 | tebufloquin | 3:1 | 1:1 | 1:3 |
| Compound 21 | tecloftalam | 15:1 | 5:1 | 2:1 |
| Compound 21 | tecnazene | 15:1 | 5:1 | 2:1 |
| Compound 21 | terbinafine | 15:1 | 5:1 | 2:1 |
| Compound 21 | tetraconazole | 1:1 | 1:2 | 1:5 |
| Compound 21 | thiabendazole | 11:1 | 4:1 | 2:1 |
| Compound 21 | thifluzamide | 3:1 | 1:1 | 1:3 |
| Compound 21 | thiophanate | 11:1 | 4:1 | 2:1 |
| Compound 21 | thiophanate-methyl | 11:1 | 4:1 | 2:1 |
| Compound 21 | thiram | 37:1 | 14:1 | 5:1 |
| Compound 21 | tiadinil | 2:1 | 1:1 | 1:3 |
| Compound 21 | tolclofos-methyl | 37:1 | 14:1 | 5:1 |
| Compound 21 | tolnifanide | 3:1 | 1:1 | 1:3 |
| Compound 21 | tolylfluanid | 15:1 | 5:1 | 2:1 |
| Compound 21 | triadimefon | 1:1 | 1:2 | 1:5 |
| Compound 21 | triadimenol | 1:1 | 1:2 | 1:5 |
| Compound 21 | triarimol | 1:2 | 1:7 | 1:24 |
| Compound 21 | triazoxide | 15:1 | 5:1 | 2:1 |
| Compound 21 | tricyclazole | 3:1 | 1:1 | 1:3 |
| Compound 21 | tridemorph | 7:1 | 2:1 | 1:1 |

(continued)

| Component (a) | Component (b) | Illustrative Ratios(*) | | |
|---|---|---|---|---|
| Compound 21 | trifloxystrobin | 2:1 | 1:1 | 1:4 |
| Compound 21 | triflumizole | 3:1 | 1:1 | 1:3 |
| Compound 21 | triforine | 3:1 | 1:1 | 1:3 |
| Compound 21 | trimorphamide | 7:1 | 2:1 | 1:2 |
| Compound 21 | triticonazole | 1:1 | 1:2 | 1:5 |
| Compound 21 | uniconazole | 1:1 | 1:2 | 1:5 |
| Compound 21 | validamycin | 3:1 | 1:1 | 1:3 |
| Compound 21 | valifenalate | 2:1 | 1:1 | 1:4 |
| Compound 21 | vinclozolin | 15:1 | 6:1 | 2:1 |
| Compound 21 | zineb | 37:1 | 14:1 | 5:1 |
| Compound 21 | ziram | 37:1 | 14:1 | 5:1 |
| Compound 21 | zoxamide | 2:1 | 1:1 | 1:4 |
| Compound 21 | 5-chloro-6-(2,4,6-trifluorophenyl)-7-(4-methylpiperidin-1-yl)[1,2,4] triazolo[1,5-a]pyrimidine (DPX-BAS600F) | 1:1 | 1:2 | 1:6 |
| Compound 21 | *N*-[2-[4-[[3-(4-chlorophenyl)-2-propyn-1-yl]oxy]-3-methoxyphenyl] ethyl]-3-methyl-2-[(methylsulfonyl)amino]- butanamide | 2:1 | 1:1 | 1:4 |
| Compound 21 | *N*-[2-[4-[[3-(4-chlorophenyl)-2-propyn-1-yl]oxy]-3-methoxyphenyl] ethyl]-3-methyl-2-[(ethylsulfonyl)amino]butanamide | 2:1 | 1:1 | 1:4 |
| Compound 21 | 4-fluorophenyl *N*-[1-[[[1-(4-cyanophenyl)ethyl]sulfonyl]-methyl] propyl]carbamate | 2:1 | 1:1 | 1:4 |
| Compound 21 | *N*-[[(cyclopropylmethoxy)amino][6-(difluoromethoxy)-2,3-difluorophenyl]methylene]benzeneacetamide | 1:2 | 1:7 | 1:24 |
| Compound 21 | $\alpha$-[methoayimino]-*N*-methyl-2-[[[1-[3-(trifluoromethyl)-phenyl] ethoxy]imino]methyl]benzeneacetamide | 3:1 | 1:1 | 1:3 |
| Compound 21 | *N'*-[4-[4-chloro-3-(trifluoromethyl)phenoxy]-2,5-dimethylphenyl]-*N*-ethyl-*N*-methylmethanimidamide | 3:1 | 1:1 | 1:3 |

(*) Ratios of Component (a) relative to Component (b) by weight.

Tables A2 through A26 are each constructed the same as Table A1 above except that entries below the "Component (a)" column heading are replaced with the respective Component (a) Column Entry shown below. Thus, for example, in Table A2 the entries below the "Component (a)" column heading all recite "Compound 57", and the first line below the column headings in Table A2 specifically discloses a mixture of Compound 57 with acibenzolar-5-methyl. Tables A3 through A26 are constructed similarly.

| Table Number | Component (a) Column Entry | Table Number | Component (a) Column Entry |
|---|---|---|---|
| A2 | Compound 57 | A15 | Compound 400 |
| A3 | Compound 110 | A16 | Compound 401 |
| A4 | Compound 183 | A17 | Compound 402 |
| A5 | Compound 198 | A18 | Compound 463 |
| A6 | Compound 204 | A19 | Compound 505 |
| A7 | Compound 225 | A20 | Compound 506 |
| A8 | Compound 229 | A21 | Compound 507 |
| A9 | Compound 257 | A22 | Compound 508 |
| A10 | Compound 264 | A23 | Compound 509 |
| A11 | Compound 316 | A24 | Compound 510 |
| A12 | Compound 330 | A25 | Compound 511 |
| A13 | Compound 376 | A26 | Compound 512 |
| A14 | Compound 394 | | |

Table B1 lists specific combinations of a Component (b) compound with Component (a) illustrative of the

mixtures, compositions and methods of the present invention. The first column of Table B1 lists the specific Component (b) compound (e.g., "acibenzolar-5-methyl" in the first line). The second, third and fourth columns of Table B1 lists ranges of weight ratios for rates at which the Component (a) compound is typically applied to a field-grown crop relative to Component (b). Thus, for example, the first line of Table B1 discloses the combination of a compound of Component (a) with acibenzolar-S-methyl is typically applied in a weight ratio of the Component (a) to Component (b) of between 2:1 to 1 :180. The remaining lines of Table B1 are to be construed similarly. Of particular note is a composition comprising a mixture of any one of the compounds listed in Embodiment 134 as Component (a) with a compound listed in the Component (b) column of Table B1 according to the weight ratios disclosed in Table B1. Table B1 thus supplements the specific ratios disclosed in Tables A1 through A23 with ranges of ratios for these combinations.

Table B1

| Component (b) | Typical Weight Ratio | Typical Weight Ratio | Most Typical Weight Ratio |
|---|---|---|---|
| acibenzolar-*S*-methyl | 2:1 to 1:180 | 1:1 to 1:60 | 1:1 to 1:18 |
| aldimorph | 30:1 to 1:3 | 10:1 to 1:1 | 7:1 to 1:1 |
| ametoctradin | 9:1 to 1:18 | 3:1 to 1:6 | 3:1 to 1:3 |
| amisulbrom | 6:1 to 1:18 | 2:1 to 1:6 | 1:1 to 1:6 |
| anilazine | 90:1 to 2:1 | 30:1 to 4:1 | 22:1 to 4:1 |
| azaconazole | 7:1 to 1:18 | 2:1 to 1:6 | 2:1 to 1:4 |
| azoxystrobin | 9:1 to 1:12 | 3:1 to 1:4 | 3:1 to 1:3 |
| benalaxyl | 4:1 to 1:18 | 1:1 to 1:6 | 1:1 to 1:6 |
| benalaxyl-M | 4:1 to 1:36 | 1:1 to 1:12 | 1:1 to 1:8 |
| benodanil | 18:1 to 1:6 | 6:1 to 1:2 | 4:1 to 1:2 |
| benomyl | 45:1 to 1:4 | 15:1 to 1:1 | 11:1 to 1:1 |
| benthiavalicarb or benthiavalicarb-isopropyl | 2:1 to 1:36 | 1:1 to 1:12 | 1:1 to 1:12 |
| bethoxazin | 150:1 to 1:36 | 50:1 to 1:12 | 15:1 to 2:1 |
| binapacryl | 150:1 to 1:36 | 50:1 to 1:12 | 15:1 to 2:1 |
| biphenyl | 150:1 to 1:36 | 50:1 to 1:12 | 15:1 to 2:1 |
| bitertanol | 15:1 to 1:5 | 5:1 to 1:2 | 3:1 to 1:2 |
| bixafen | 12:1 to 1:9 | 4:1 to 1:3 | 2:1 to 1:3 |
| blasticidin-S | 3:1 to 1:90 | 1:1 to 1:30 | 1:4 to 1:30 |
| boscalid | 18:1 to 1:6 | 6:1 to 1:2 | 4:1 to 1:2 |
| bromuconazole | 15:1 to 1:9 | 5:1 to 1:3 | 3:1 to 1:3 |
| bupirimate | 3:1 to 1:90 | 1:1 to 1:30 | 1:3 to 1:30 |
| captafol | 90:1 to 1:4 | 30:1 to 1:2 | 15:1 to 2:1 |
| captan | 90:1 to 1:4 | 30:1 to 1:2 | 15:1 to 2:1 |
| carbendazim | 45:1 to 1:4 | 15:1 to 1:2 | 11:1 to 2:1 |
| carboxin | 18:1 to 1:6 | 6:1 to 1:2 | 4:1 to 1:2 |
| carpropamid | 15:1 to 1:9 | 5:1 to 1:3 | 3:1 to 1:3 |
| chloroneb | 300:1 to 2:1 | 100:1 to 4:1 | 100:1 to 14:1 |
| chlorothalonil | 90:1 to 1:4 | 30:1 to 1:2 | 15:1 to 2:1 |
| chlozolinate | 45:1 to 1:2 | 15:1 to 2:1 | 11:1 to 2:1 |
| clotrimazole | 15:1 to 1:9 | 5:1 to 1:3 | 3:1 to 1:3 |
| copper salts such as Bordeaux mixture (tribasic copper sulfate), copper oxychloride, copper sulfate and copper hydroxide such as Kocide | 450:1 to 1:1 | 150:1 to 4:1 | 45:1 to 5:1 |
| cyazofamid | 4:1 to 1:18 | 1:1 to 1:6 | 1:1 to 1:6 |
| cyflufenamid | 1:1 to 1:90 | 1:2 to 1:30 | 1:2 to 1:24 |
| cymoxanil | 6:1 to 1:18 | 2:1 to 1:6 | 1:1 to 1:5 |
| cyproconazole | 4:1 to 1:18 | 1:1 to 1:6 | 1:1 to 1:6 |
| cyprodinil | 22:1 to 1:9 | 7:1 to 1:3 | 4:1 to 1:2 |
| dichlofluanid | 150:1 to 1:36 | 50:1 to 1:12 | 15:1 to 2:1 |
| diclocymet | 150:1 to 1:36 | 50:1 to 1:12 | 15:1 to 2:1 |

(continued)

| Component (b) | Typical Weight Ratio | Typical Weight Ratio | Most Typical Weight Ratio |
|---|---|---|---|
| diclomezine | 15:1 to 1:9 | 5:1 to 1:3 | 3:1 to 1:3 |
| dicloran | 150:1 to 1:36 | 50:1 to 1:12 | 15:1 to 2:1 |
| diethofencarb | 22:1 to 1:9 | 7:1 to 1:3 | 7:1 to 1:2 |
| difenoconazole | 4:1 to 1:36 | 1:1 to 1:12 | 1:1 to 1:12 |
| diflumetorim | 150:1 to 1:36 | 50:1 to 1:12 | 15:1 to 2:1 |
| dimethirimol | 3:1 to 1:90 | 1:1 to 1:30 | 1:3 to 1:30 |
| dimethomorph | 9:1 to 1:6 | 3:1 to 1:2 | 3:1 to 1:2 |
| dimoxystrobin | 9:1 to 1:18 | 3:1 to 1:6 | 2:1 to 1:4 |
| diniconazole | 3:1 to 1:36 | 1:1 to 1:12 | 1:1 to 1:8 |
| diniconazole M | 3:1 to 1:90 | 1:1 to 1:30 | 1:1 to 1:12 |
| dinocap | 7:1 to 1:9 | 2:1 to 1:3 | 2:1 to 1:3 |
| dithianon | 15:1 to 1:4 | 5:1 to 1:2 | 5:1 to 1:2 |
| dodemorph | 30:1 to 1:3 | 10:1 to 1:1 | 7:1 to 1:1 |
| dodine | 30:1 to 1:2 | 10:1 to 2:1 | 10:1 to 2:1 |
| edifenphos | 30:1 to 1:9 | 10:1 to 1:3 | 3:1 to 1:3 |
| enestroburin | 9:1 to 1:18 | 3:1 to 1:6 | 2:1 to 1:4 |
| epoxiconazole | 3:1 to 1:36 | 1:1 to 1:12 | 1:1 to 1:7 |
| etaconazole | 3:1 to 1:36 | 1:1 to 1:12 | 1:1 to 1:7 |
| ethaboxam | 7:1 to 1:9 | 2:1 to 1:3 | 2:1 to 1:3 |
| ethirimol | 30:1 to 1:3 | 10:1 to 1:1 | 7:1 to 1:1 |
| etridiazole | 30:1 to 1:9 | 10:1 to 1:3 | 7:1 to 1:2 |
| famoxadone | 9:1 to 1:18 | 3:1 to 1:6 | 2:1 to 1:4 |
| fenamidone | 6:1 to 1:18 | 2:1 to 1:6 | 2:1 to 1:4 |
| fenaminstrobin | 9:1 to 1:18 | 3:1 to 1:6 | 3:1 to 1:3 |
| fenarimol | 3:1 to 1:90 | 1:1 to 1:30 | 1:2 to 1:24 |
| fenbuconazole | 3:1 to 1:30 | 1:1 to 1:10 | 1:1 to 1:10 |
| fenfuram | 18:1 to 1:6 | 6:1 to 1:2 | 4:1 to 1:2 |
| fenhexamid | 30:1 to 1:2 | 10:1 to 2:1 | 10:1 to 2:1 |
| fenoxanil | 150:1 to 1:36 | 50:1 to 1:12 | 15:1 to 1:1 |
| fenpiclonil | 75:1 to 1:9 | 25:1 to 1:3 | 15:1 to 2:1 |
| fenpropidin | 30:1 to 1:3 | 10:1 to 1:1 | 7:1 to 1:1 |
| fenpropimorph | 30:1 to 1:3 | 10:1 to 1:1 | 7:1 to 1:1 |
| fenpyrazamine | 100:1 to 1: 100 | 10:1 to 1:10 | 3:1 to 1:3 |
| fentin salt such as the acetate, chloride or hydroxide | 15:1 to 1:9 | 5:1 to 1:3 | 3:1 to 1:3 |
| ferbam | 300:1 to 1:2 | 100:1 to 2:1 | 30:1 to 4:1 |
| ferimzone | 30:1 to 1:5 | 10:1 to 1:2 | 7:1 to 1:2 |
| fluazinam | 22:1 to 1:5 | 7:1 to 1:2 | 3:1 to 1:2 |
| fludioxonil | 7:1 to 1:12 | 2:1 to 1:4 | 2:1 to 1:4 |
| flumetover | 9:1 to 1:6 | 3:1 to 1:2 | 3:1 to 1:2 |
| flumorph | 9:1 to 1:18 | 3:1 to 1:6 | 3:1 to 1:3 |
| fluopicolide | 3:1 to 1:18 | 1:1 to 1:6 | 1:1 to 1:6 |
| fluopyram | 15:1 to 1:90 | 5:1 to 1:30 | 3:1 to 1:3 |
| fluoromide | 150:1 to 2:1 | 50:1 to 4:1 | 37:1 to 5:1 |
| fluoxastrobin | 4:1 to 1:18 | 1:1 to 1:6 | 1:1 to 1:6 |
| fluquinconazole | 4:1 to 1:12 | 1:1 to 1:4 | 1:1 to 1:4 |
| flusilazole | 15:1 to 1:9 | 5:1 to 1:3 | 3:1 to 1:3 |
| flusulfamide | 90:1 to 1:2 | 30:1 to 2:1 | 15:1 to 2:1 |
| flutianil | 7:1 to 1:36 | 2:1 to 1:12 | 1:1 to 1:6 |

(continued)

| Component (b) | Typical Weight Ratio | Typical Weight Ratio | Most Typical Weight Ratio |
|---|---|---|---|
| flutolanil | 18:1 to 1:6 | 6:1 to 1:2 | 4:1 to 1:2 |
| flutriafol | 4:1 to 1:12 | 1:1 to 1:4 | 1:1 to 1:4 |
| fluxapyroxad | 12:1 to 1:9 | 4:1 to 1:3 | 2:1 to 1:3 |
| folpet | 90:1 to 1:4 | 30:1 to 1:2 | 15:1 to 2:1 |
| fosetyl-aluminum | 225:1 to 2:1 | 75:1 to 5:1 | 30:1 to 5:1 |
| fuberidazole | 45:1 to 1:4 | 15:1 to 1:2 | 11:1 to 2:1 |
| furalaxyl | 15:1 to 1:45 | 5:1 to 1:15 | 1:1 to 1:6 |
| furametpyr | 150:1 to 1:36 | 50:1 to 1:12 | 15:1 to 2:1 |
| guazatine or iminoctadine | 150:1 to 1:36 | 50:1 to 1:12 | 15:1 to 2:1 |
| hexaconazole | 15:1 to 1:36 | 5:1 to 1:12 | 1:1 to 1:5 |
| hymexazol | 225:1 to 2:1 | 75:1 to 4:1 | 75:1 to 9:1 |
| imazalil | 7:1 to 1:18 | 2:1 to 1:6 | 1:1 to 1:5 |
| imibenconazole | 15:1 to 1:36 | 5:1 to 1:12 | 1:1 to 1:5 |
| iodocarb | 150:1 to 1:36 | 50:1 to 1:12 | 15:1 to 2:1 |
| ipconazole | 15:1 to 1:36 | 5:1 to 1:12 | 1:1 to 1:5 |
| iprobenfos | 150:1 to 1:36 | 50:1 to 1:12 | 15:1 to 2:1 |
| iprodione | 120:1 to 1:2 | 40:1 to 2:1 | 15:1 to 2:1 |
| iprovalicarb | 9:1 to 1:9 | 3:1 to 1:3 | 2:1 to 1:3 |
| isoprothiolane | 150:1 to 2:1 | 50:1 to 4:1 | 45:1 to 5:1 |
| isopyrazam | 12:1 to 1:9 | 4:1 to 1:3 | 2:1 to 1:3 |
| isotianil | 12:1 to 1:9 | 4:1 to 1:3 | 2:1 to 1:3 |
| kasugamycin | 7:1 to 1:90 | 2:1 to 1:30 | 1:2 to 1:24 |
| kresoxim-methyl | 7:1 to 1:18 | 2:1 to 1:6 | 2:1 to 1:4 |
| mancozeb | 180:1 to 1:3 | 60:1 to 2:1 | 22:1 to 3:1 |
| mandipropamid | 6:1 to 1:18 | 2:1 to 1:6 | 2:1 to 1:4 |
| maneb | 180:1 to 1:3 | 60:1 to 2:1 | 22:1 to 3:1 |
| mepanipyrim | 18:1 to 1:3 | 6:1 to 1:1 | 6:1 to 1:1 |
| mepronil | 7:1 to 1:36 | 2:1 to 1:12 | 1:1 to 1:6 |
| meptyldinocap | 7:1 to 1:9 | 2:1 to 1:3 | 2:1 to 1:3 |
| metalaxyl | 15:1 to 1:45 | 5:1 to 1:15 | 1:1 to 1:6 |
| metalaxyl-M | 7:1 to 1:90 | 2:1 to 1:30 | 1:1 to 1:12 |
| metconazole | 3:1 to 1:18 | 1:1 to 1:6 | 1:1 to 1:6 |
| methasulfocarb | 150:1 to 1:36 | 50:1 to 1:12 | 15:1 to 1:1 |
| metiram | 150:1 to 1:36 | 50:1 to 1:12 | 15:1 to 1:1 |
| metominostrobin | 9:1 to 1:12 | 3:1 to 1:4 | 3:1 to 1:3 |
| metrafenone | 6:1 to 1:12 | 2:1 to 1:4 | 2:1 to 1:4 |
| myclobutanil | 5:1 to 1:26 | 1:1 to 1:9 | 1:1 to 1:8 |
| naftifine | 150:1 to 1:36 | 50:1 to 1:12 | 15:1 to 2:1 |
| neo-asozin (ferric methanearsonate) | 150:1 to 1:36 | 50:1 to 1:12 | 15:1 to 2:1 |
| nuarimol | 15:1 to 1:9 | 5:1 to 1:3 | 3:1 to 1:3 |
| octhilinone | 150:1 to 1:36 | 50:1 to 1:12 | 15:1 to 1:1 |
| ofurace | 15:1 to 1:45 | 5:1 to 1:15 | 1:1 to 1:6 |
| orysastrobin | 9:1 to 1:12 | 3:1 to 1:4 | 3:1 to 1:3 |
| oxadixyl | 15:1 to 1:45 | 5:1 to 1:15 | 1:1 to 1:6 |
| oxolinic acid | 30:1 to 1:9 | 10:1 to 1:3 | 7:1 to 1:2 |
| oxpoconazole | 15:1 to 1:36 | 5:1 to 1:12 | 1:1 to 1:5 |
| oxycarboxin | 18:1 to 1:6 | 6:1 to 1:2 | 4:1 to 1:2 |
| oxytetracycline | 15:1 to 1:9 | 5:1 to 1:3 | 3:1 to 1:3 |
| pefurazoate | 150:1 to 1:36 | 50:1 to 1:12 | 15:1 to 2:1 |

(continued)

| Component (b) | Typical Weight Ratio | Typical Weight Ratio | Most Typical Weight Ratio |
|---|---|---|---|
| penconazole | 1:1 to 1:45 | 1:2 to 1:15 | 1:2 to 1:15 |
| pencycuron | 150:1 to 1:2 | 50:1 to 2:1 | 11:1 to 2:1 |
| penflufen | 12:1 to 1:9 | 4:1 to 1:3 | 2:1 to 1:3 |
| penthiopyrad | 12:1 to 1:9 | 4:1 to 1:3 | 2:1 to 1:3 |
| phosphorous acid and salts thereof | 150:1 to 1:36 | 50:1 to 1:12 | 15:1 to 2:1 |
| phthalide | 150:1 to 1:36 | 50:1 to 1:12 | 15:1 to 2:1 |
| picoxystrobin | 7:1 to 1:18 | 2:1 to 1:6 | 1:1 to 1:5 |
| piperalin | 15:1 to 1:9 | 5:1 to 1:3 | 3:1 to 1:3 |
| polyoxin | 15:1 to 1:9 | 5:1 to 1:3 | 3:1 to 1:3 |
| probenazole | 15:1 to 1:9 | 5:1 to 1:3 | 3:1 to 1:3 |
| prochloraz | 22:1 to 1:4 | 7:1 to 1:1 | 7:1 to 1:2 |
| procymidone | 45:1 to 1:3 | 15:1 to 1:1 | 11:1 to 2:1 |
| propamocarb or propamocarb-hydrochloride | 30:1 to 1:2 | 10:1 to 2:1 | 10:1 to 2:1 |
| propiconazole | 4:1 to 1:18 | 1:1 to 1:6 | 1:1 to 1:5 |
| propineb | 45:1 to 1:2 | 15:1 to 2:1 | 11:1 to 2:1 |
| proquinazid | 3:1 to 1:36 | 1:1 to 1:12 | 1:1 to 1:12 |
| prothiocarb | 9:1 to 1:18 | 3:1 to 1:6 | 3:1 to 1:3 |
| prothioconazole | 6:1 to 1:18 | 2:1 to 1:6 | 1:1 to 1:5 |
| pyraclostrobin | 9:1 to 1:18 | 3:1 to 1:6 | 2:1 to 1:4 |
| pyrametostrobin | 9:1 to 1:18 | 3:1 to 1:6 | 2:1 to 1:4 |
| pyraoxystrobin | 9:1 to 1:18 | 3:1 to 1:6 | 2:1 to 1:4 |
| pyrazophos | 150:1 to 1:36 | 50:1 to 1:12 | 15:1 to 1:1 |
| pyribencarb | 15:1 to 1:6 | 5:1 to 1:2 | 4:1 to 1:2 |
| pyrifenox | 15:1 to 1:9 | 5:1 to 1:3 | 3:1 to 1:3 |
| pyrimethanil | 30:1 to 1:6 | 10:1 to 1:2 | 3:1 to 1:2 |
| pyriofenone | 6:1 to 1:12 | 2:1 to 1:4 | 2:1 to 1:4 |
| pyrisoxazole | 15:1 to 1:9 | 5:1 to 1:3 | 3:1 to 1:3 |
| pyroquilon | 15:1 to 1:9 | 5:1 to 1:3 | 3:1 to 1:3 |
| pyrrolnitrin | 150:1 to 1:36 | 50:1 to 1:12 | 15:1 to 2:1 |
| quinconazole | 4:1 to 1:12 | 1:1 to 1:4 | 1:1 to 1:4 |
| quinmethionate | 150:1 to 1:36 | 50:1 to 1:12 | 15:1 to 2:1 |
| quinoxyfen | 4:1 to 1:18 | 1:1 to 1:6 | 1:1 to 1:6 |
| quintozene | 150:1 to 1:36 | 50:1 to 1:12 | 15:1 to 2:1 |
| silthiofam | 7:1 to 1:18 | 2:1 to 1:6 | 2:1 to 1:4 |
| simeconazole | 15:1 to 1:36 | 5:1 to 1:12 | 1:1 to 1:5 |
| spiroxamine | 22:1 to 1:4 | 7:1 to 1:2 | 5:1 to 1:2 |
| streptomycin | 15:1 to 1:9 | 5:1 to 1:3 | 3:1 to 1:3 |
| sulfur | 300:1 to 3:1 | 100:1 to 9:1 | 75:1 to 9:1 |
| tebuconazole | 7:1 to 1:18 | 2:1 to 1:6 | 1:1 to 1:5 |
| tebufloquin | 100:1 to 1: 100 | 10:1 to 1:10 | 3:1 to 1:3 |
| tecloftalam | 150:1 to 1:36 | 50:1 to 1:12 | 15:1 to 2:1 |
| tecnazene | 150:1 to 1:36 | 50:1 to 1:12 | 15:1 to 2:1 |
| terbinafine | 150:1 to 1:36 | 50:1 to 1:12 | 15:1 to 2:1 |
| tetraconazole | 15:1 to 1:36 | 5:1 to 1:12 | 1:1 to 1:5 |
| thiabendazole | 45:1 to 1:4 | 15:1 to 1:2 | 11:1 to 2:1 |
| thifluzamide | 15:1 to 1:9 | 5:1 to 1:3 | 3:1 to 1:3 |
| thiophanate | 45:1 to 1:3 | 15:1 to 2:1 | 11:1 to 2:1 |
| thiophanate-methyl | 45:1 to 1:3 | 15:1 to 2:1 | 11:1 to 2:1 |

(continued)

| Component (b) | Typical Weight Ratio | Typical Weight Ratio | Most Typical Weight Ratio |
|---|---|---|---|
| thiram | 150:1 to 1:2 | 50:1 to 2:1 | 37:1 to 5:1 |
| tiadinil | 12:1 to 1:9 | 4:1 to 1:3 | 2:1 to 1:3 |
| tolclofos-methyl | 150:1 to 1:2 | 50:1 to 2:1 | 37:1 to 5:1 |
| tolnifanide | 15:1 to 1:18 | 5:1 to 1:6 | 3:1 to 1:3 |
| tolylfluanid | 150:1 to 1:36 | 50:1 to 1:12 | 15:1 to 2:1 |
| triadimefon | 15:1 to 1:36 | 5:1 to 1:12 | 1:1 to 1:5 |
| triadimenol | 15:1 to 1:36 | 5:1 to 1:12 | 1:1 to 1:5 |
| triarimol | 3:1 to 1:90 | 1:1 to 1:30 | 1:2 to 1:24 |
| triazoxide | 150:1 to 1:36 | 50:1 to 1:12 | 15:1 to 2:1 |
| tricyclazole | 15:1 to 1:9 | 5:1 to 1:3 | 3:1 to 1:3 |
| tridemorph | 30:1 to 1:3 | 10:1 to 1:1 | 7:1 to 1:1 |
| trifloxystrobin | 6:1 to 1:18 | 2:1 to 1:6 | 2:1 to 1:4 |
| triflumizole | 15:1 to 1:9 | 5:1 to 1:3 | 3:1 to 1:3 |
| triforine | 15:1 to 1:9 | 5:1 to 1:3 | 3:1 to 1:3 |
| trimorphamide | 45:1 to 1:9 | 15:1 to 1:3 | 7:1 to 1:2 |
| triticonazole | 15:1 to 1:36 | 5:1 to 1:12 | 1:1 to 1:5 |
| uniconazole | 15:1 to 1:36 | 5:1 to 1:12 | 1:1 to 1:5 |
| validamycin | 150:1 to 1:36 | 50:1 to 1:12 | 3:1 to 1:3 |
| valifenalate | 6:1 to 1:18 | 2:1 to 1:6 | 2:1 to 1:4 |
| vinclozolin | 120:1 to 1:2 | 40:1 to 2:1 | 15:1 to 2:1 |
| zineb | 150:1 to 1:2 | 50:1 to 2:1 | 37:1 to 5:1 |
| ziram | 150:1 to 1:2 | 50:1 to 2:1 | 37:1 to 5:1 |
| zoxamide | 6:1 to 1:18 | 2:1 to 1:6 | 2:1 to 1:4 |
| 5 -chloro-6-(2,4,6-trifluorophenyl)-7-(4-methylpiperidin-1-yl)[1,2,4] triazolo-[1,5-a]pyrimidine (DPX-BAS600F) | 15:1 to 1:36 | 5:1 to 1:12 | 1:1 to 1:6 |
| *N*-[2-[4-[[3-(4-chlorophenyl)-2-propyn-1-yl]oxy]-3-methoxyphenyl] ethyl]- 3-methyl-2-[(methylsulfonyl)amino]-butanamide | 6:1 to 1:18 | 2:1 to 1:6 | 2:1 to 1:4 |
| *N*-[2-[4-[[3-(4-chlorophenyl)-2-propyn-1-yl]oxy]-3-methoxyphenyl] ethyl]-3-methyl-2-[(ethylsulfonyl)amino]butanamide | 6:1 to 1:18 | 2:1 to 1:6 | 2:1 to 1:4 |
| 4-fluorophenyl *N*-[1-[[[1-(4-cyanophenyl)-ethyl]sulfonyl]methyl] propyl]carbamate | 6:1 to 1:18 | 2:1 to 1:6 | 2:1 to 1:4 |
| *N*-[[(cyclopropylmethoxy)amino] [6-(difluoromethoxy)-2,3-difluorophenyl]-methylene]benzeneacetamide | 1:1 to 1:90 | 1:2 to 1:30 | 1:2 to 1:24 |
| α-[methoxyimino]-*N*-methyl-2-[[[1-[3-(trifluoromethyl)phenyl] ethoxy]imino]-methyl]benzeneacetamide | 9:1 to 1:18 | 3:1 to 1:6 | 3:1 to 1:3 |
| *N'*-[4-[4-chloro-3-(trifluoromethyl)-phenoxy]-2,5-dimethylphenyl]-*N*-ethyl-*N*-methylmethanimidamide | 15:1 to 1:18 | 5:1 to 1:6 | 3:1 to 1:3 |

As already noted, the present invention includes embodiments wherein in the composition comprising components (a) and (b), component (b) comprises at least one fungicidal compound from each of two groups selected from (b1) through (b54). Tables C1 through C23 list specific mixtures (compound numbers refer to compounds in Index Tables A through T) to illustrate embodiments wherein component (b) includes at least one fungicidal compound from each of two groups selected from (b1) through (b54). In Table C1, each line below the column headings "Component (a)" and "Component (b)" specifically discloses a mixture of Component (a), which is Compound 21, with at least two Component (b) fungicidal compounds. The entries under the heading "Illustrative Ratios" disclose three specific weight ratios of Component (a) to each Component (b) fungicidal compound in sequence for the disclosed mixture. For example, the first line discloses a mixture of Compound 21 with cyproconazole and azoxystrobin and lists weight ratios of Compound 21 to cyproconazole to azoxystrobin of 1:1:1 , 2:1:1 or 3:1:1.

Table C1

| Component (a) | Component (b) | Component (b) | Component (b) | Illustrative Ratios(*) | | |
|---|---|---|---|---|---|---|
| Compound 21 | cyproconazole | azoxystrobin | | 1:1:1 | 2:1:1 | 3:1:1 |
| Compound 21 | cyproconazole | kresoxim-methyl | | 1:1:1 | 2:1:1 | 3:1:1 |
| Compound 21 | cyproconazole | picoxystrobin | | 1:1:1 | 2:1:1 | 3:1:1 |
| Compound 21 | cyproconazole | pyraclostrobin | | 1:1:1 | 2:1:1 | 3:1:1 |
| Compound 21 | cyproconazole | pyrametrostrobin | | 1:1:1 | 2:1:1 | 3:1:1 |
| Compound 21 | cyproconazole | pyraoxystrobin | | 1:1:1 | 2:1:1 | 3:1:1 |
| Compound 21 | cyproconazole | trifloxystrobin | | 1:1:1 | 2:1:1 | 3:1:1 |
| Compound 21 | cyproconazole | bixafen | | 1:1:2 | 2:1:2 | 3:1:2 |
| Compound 21 | cyproconazole | boscalid | | 1:1:2 | 2:1:2 | 3:1:2 |
| Compound 21 | cyproconazole | cyflufenamid | | 1:2:1 | 2:2:1 | 3:2:1 |
| Compound 21 | cyproconazole | fluopyram | | 1:1:2 | 2:1:2 | 3:1:2 |
| Compound 21 | cyproconazole | isopyrazam | | 1:1:2 | 2:1:2 | 3:1:2 |
| Compound 21 | cyproconazole | metrafenone | | 1:1:2 | 2:1:2 | 3:1:2 |
| Compound 21 | cyproconazole | penthiopyrad | | 1:1:2 | 2:1:2 | 3:1:2 |
| Compound 21 | cyproconazole | proquinazid | | 1:1:1 | 2:1:1 | 3:1:1 |
| Compound 21 | cyproconazole | pyriofenone | | 1:1:2 | 2:1:2 | 3:1:2 |
| Compound 21 | cyproconazole | quinoxyfen | | 1:1:1 | 2:1:1 | 3:1:1 |
| Compound 21 | cyproconazole | sedaxane | | 1:1:2 | 2:1:2 | 3:1:2 |
| Compound 21 | cyproconazole | picoxystrobin | proquinazid | 1:1:1:1 | 2:1:1:1 | 3:1:1:1 |
| Compound 21 | cyproconazole | trifloxystrobin | proquinazid | 1:1:1:1 | 2:1:1:1 | 3:1:1:1 |
| Compound 21 | difenconazole | azoxystrobin | | 1:1:1 | 2:1:1 | 3:1:1 |
| Compound 21 | difenconazole | kresoxim-methyl | | 1:1:1 | 2:1:1 | 3:1:1 |
| Compound 21 | difenconazole | picoxystrobin | | 1:1:1 | 2:1:1 | 3:1:1 |
| Compound 21 | difenconazole | pyraclostrobin | | 1:1:1 | 2:1:1 | 3:1:1 |
| Compound 21 | difenconazole | pyrametostrobin | | 1:1:1 | 2:1:1 | 3:1:1 |
| Compound 21 | difenoconazole | pyraoxystrobin | | 1:1:1 | 2:1:1 | 3:1:1 |
| Compound 21 | difenconazole | trifloxystrobin | | 1:1:1 | 2:1:1 | 3:1:1 |
| Compound 21 | difenconazole | bixafen | | 1:1:2 | 2:1:2 | 3:1:2 |
| Compound 21 | difenconazole | boscalid | | 1:1:2 | 2:1:2 | 3:1:2 |
| Compound 21 | difenconazole | cyflufenamid | | 1:2:1 | 2:2:1 | 3:2:1 |
| Compound 21 | difenconazole | fluopyram | | 1:1:2 | 2:1:2 | 3:1:2 |
| Compound 21 | difenconazole | isopyrazam | | 1:1:2 | 2:1:2 | 3:1:2 |
| Compound 21 | difenconazole | metrafenone | | 1:1:2 | 2:1:2 | 3:1:2 |
| Compound 21 | difenconazole | penthiopyrad | | 1:1:2 | 2:1:2 | 3:1:2 |
| Compound 21 | difenconazole | proquinazid | | 1:1:1 | 2:1:1 | 3:1:1 |
| Compound 21 | difenconazole | pyriofenone | | 1:1:2 | 2:1:2 | 3:1:2 |
| Compound 21 | difenconazole | quinoxyfen | | 1:1:1 | 2:1:1 | 3:1:1 |
| Compound 21 | difenconazole | sedaxane | | 1:1:2 | 2:1:2 | 3:1:2 |
| Compound 21 | difenconazole | picoxystrobin | proquinazid | 1:1:1:1 | 2:1:1:1 | 3:1:1:1 |
| Compound 21 | difenconazole | trifloxystrobin | proquinazid | 1:1:1:1 | 2:1:1:1 | 3:1:1:1 |
| Compound 21 | epoxiconazole | azoxystrobin | | 1:1:1 | 2:1:1 | 3:1:1 |
| Compound 21 | epoxiconazole | kresoxim-methyl | | 1:1:1 | 2:1:1 | 3:1:1 |
| Compound 21 | epoxiconazole | picoxystrobin | | 1:1:1 | 2:1:1 | 3:1:1 |
| Compound 21 | epoxiconazole | pyraclostrobin | | 1:1:1 | 2:1:1 | 3:1:1 |
| Compound 21 | epoxiconazole | pyrametostrobin | | 1:1:1 | 2:1:1 | 3:1:1 |
| Compound 21 | epoxiconazole | pyraoxy strobin | | 1:1:1 | 2:1:1 | 3:1:1 |
| Compound 21 | epoxiconazole | trifloxystrobin | | 1:1:1 | 2:1:1 | 3:1:1 |
| Compound 21 | epoxiconazole | bixafen | | 1:1:2 | 2:1:2 | 3:1:2 |
| Compound 21 | epoxiconazole | boscalid | | 1:1:2 | 2:1:2 | 3:1:2 |

(continued)

| Component (a) | Component (b) | Component (b) | Component (b) | Illustrative Ratios(*) | | |
|---|---|---|---|---|---|---|
| Compound 21 | epoxiconazole | cyflufenamid | | 1:2:1 | 2:2:1 | 3:2:1 |
| Compound 21 | epoxiconazole | fluopyram | | 1:1:2 | 2:1:2 | 3:1:2 |
| Compound 21 | epoxiconazole | isopyrazam | | 1:1:2 | 2:1:2 | 3:1:2 |
| Compound 21 | epoxiconazole | metrafenone | | 1:1:2 | 2:1:2 | 3:1:2 |
| Compound 21 | epoxiconazole | penthiopyrad | | 1:1:2 | 2:1:2 | 3:1:2 |
| Compound 21 | epoxiconazole | proquinazid | | 1:1:1 | 2:1:1 | 3:1:1 |
| Compound 21 | epoxiconazole | pyriofenone | | 1:1:2 | 2:1:2 | 3:1:2 |
| Compound 21 | epoxiconazole | quinoxyfen | | 1:1:1 | 2:1:1 | 3:1:1 |
| Compound 21 | epoxiconazole | sedaxane | | 1:1:2 | 2:1:2 | 3:1:2 |
| Compound 21 | epoxiconazole | picoxystrobin | proquinazid | 1:1:1:1 | 2:1:1:1 | 3:1:1:1 |
| Compound 21 | epoxiconazole | trifloxystrobin | proquinazid | 1:1:1:1 | 2:1:1:1 | 3:1:1:1 |
| Compound 21 | metconazole | azoxystrobin | | 1:1:1 | 2:1:1 | 3:1:1 |
| Compound 21 | metconazole | kresoxim-methyl | | 1:1:1 | 2:1:1 | 3:1:1 |
| Compound 21 | metconazole | picoxystrobin | | 1:1:1 | 2:1:1 | 3:1:1 |
| Compound 21 | metconazole | pyraclostrobin | | 1:1:1 | 2:1:1 | 3:1:1 |
| Compound 21 | metconazole | pyrametostrobin | | 1:1:1 | 2:1:1 | 3:1:1 |
| Compound 21 | metconazole | pyraoxy strobin | | 1:1:1 | 2:1:1 | 3:1:1 |
| Compound 21 | metconazole | trifloxystrobin | | 1:1:1 | 2:1:1 | 3:1:1 |
| Compound 21 | metconazole | bixafen | | 1:1:2 | 2:1:2 | 3:1:2 |
| Compound 21 | metconazole | boscalid | | 1:1:2 | 2:1:2 | 3:1:2 |
| Compound 21 | metconazole | cyflufenamid | | 1:2:1 | 2:2:1 | 3:2:1 |
| Compound 21 | metconazole | fluopyram | | 1:1:2 | 2:1:2 | 3:1:2 |
| Compound 21 | metconazole | isopyrazam | | 1:1:2 | 2:1:2 | 3:1:2 |
| Compound 21 | metconazole | metrafenone | | 1:1:2 | 2:1:2 | 3:1:2 |
| Compound 21 | metconazole | penthiopyrad | | 1:1:2 | 2:1:2 | 3:1:2 |
| Compound 21 | metconazole | proquinazid | | 1:1:1 | 2:1:1 | 3:1:1 |
| Compound 21 | metconazole | pyriofenone | | 1:1:2 | 2:1:2 | 3:1:2 |
| Compound 21 | metconazole | quinoxyfen | | 1:1:1 | 2:1:1 | 3:1:1 |
| Compound 21 | metconazole | sedaxane | | 1:1:2 | 2:1:2 | 3:1:2 |
| Compound 21 | metconazole | picoxystrobin | proquinazid | 1:1:1:1 | 2:1:1:1 | 3:1:1:1 |
| Compound 21 | metconazole | trifloxystrobin | proquinazid | 1:1:1:1 | 2:1:1:1 | 3:1:1:1 |
| Compound 21 | myclobutanil | azoxystrobin | | 1:1:1 | 2:1:1 | 3:1:1 |
| Compound 21 | myclobutanil | kresoxim-methyl | | 1:1:1 | 2:1:1 | 3:1:1 |
| Compound 21 | myclobutanil | picoxystrobin | | 1:1:1 | 2:1:1 | 3:1:1 |
| Compound 21 | myclobutanil | pyraclostrobin | | 1:1:1 | 2:1:1 | 3:1:1 |
| Compound 21 | myclobutanil | pyrametostrobin | | 1:1:1 | 2:1:1 | 3:1:1 |
| Compound 21 | myclobutanil | pyraoxy strobin | | 1:1:1 | 2:1:1 | 3:1:1 |
| Compound 21 | myclobutanil | trifloxystrobin | | 1:1:1 | 2:1:1 | 3:1:1 |
| Compound 21 | myclobutanil | bixafen | | 1:1:2 | 2:1:2 | 3:1:2 |
| Compound 21 | myclobutanil | boscalid | | 1:1:2 | 2:1:2 | 3:1:2 |
| Compound 21 | myclobutanil | cyflufenamid | | 1:2:1 | 2:2:1 | 3:2:1 |
| Compound 21 | myclobutanil | fluopyram | | 1:1:2 | 2:1:2 | 3:1:2 |
| Compound 21 | myclobutanil | isopyrazam | | 1:1:2 | 2:1:2 | 3:1:2 |
| Compound 21 | myclobutanil | metrafenone | | 1:1:2 | 2:1:2 | 3:1:2 |
| Compound 21 | myclobutanil | penthiopyrad | | 1:1:2 | 2:1:2 | 3:1:2 |
| Compound 21 | myclobutanil | proquinazid | | 1:1:1 | 2:1:1 | 3:1:1 |
| Compound 21 | myclobutanil | pyriofenone | | 1:1:2 | 2:1:2 | 3:1:2 |
| Compound 21 | myclobutanil | quinoxyfen | | 1:1:1 | 2:1:1 | 3:1:1 |
| Compound 21 | myclobutanil | sedaxane | | 1:1:2 | 2:1:2 | 3:1:2 |
| Compound 21 | myclobutanil | picoxystrobin | proquinazid | 1:1:1:1 | 2:1:1:1 | 3:1:1:1 |

(continued)

| Component (a) | Component (b) | Component (b) | Component (b) | Illustrative Ratios(*) | | |
|---|---|---|---|---|---|---|
| Compound 21 | myclobutanil | trifloxystrobin | proquinazid | 1:1:1:1 | 2:1:1:1 | 3:1:1:1 |
| Compound 21 | prothioconazole | azoxystrobin | | 1:1:1 | 2:1:1 | 3:1:1 |
| Compound 21 | prothioconazole | kresoxim-methyl | | 1:1:1 | 2:1:1 | 3:1:1 |
| Compound 21 | prothioconazole | picoxystrobin | | 1:1:1 | 2:1:1 | 3:1:1 |
| Compound 21 | prothioconazole | pyraclostrobin | | 1:1:1 | 2:1:1 | 3:1:1 |
| Compound 21 | prothioconazole | pyrametostrobin | | 1:1:1 | 2:1:1 | 3:1:1 |
| Compound 21 | prothioconazole | pyraoxystrobin | | 1:1:1 | 2:1:1 | 3:1:1 |
| Compound 21 | prothioconazole | trifloxystrobin | | 1:1:1 | 2:1:1 | 3:1:1 |
| Compound 21 | prothioconazole | bixafen | | 1:1:2 | 2:1:2 | 3:1:2 |
| Compound 21 | prothioconazole | boscalid | | 1:1:2 | 2:1:2 | 3:1:2 |
| Compound 21 | prothioconazole | cyflufenamid | | 1:2:1 | 2:2:1 | 3:2:1 |
| Compound 21 | prothioconazole | fluopyram | | 1:1:2 | 2:1:2 | 3:1:2 |
| Compound 21 | prothioconazole | isopyrazam | | 1:1:2 | 2:1:2 | 3:1:2 |
| Compound 21 | prothioconazole | metrafenone | | 1:1:2 | 2:1:2 | 3:1:2 |
| Compound 21 | prothioconazole | penthiopyrad | | 1:1:2 | 2:1:2 | 3:1:2 |
| Compound 21 | prothioconazole | proquinazid | | 1:1:1 | 2:1:1 | 3:1:1 |
| Compound 21 | prothioconazole | pyriofenone | | 1:1:2 | 2:1:2 | 3:1:2 |
| Compound 21 | prothioconazole | quinoxyfen | | 1:1:1 | 2:1:1 | 3:1:1 |
| Compound 21 | prothioconazole | sedaxane | | 1:1:2 | 2:1:2 | 3:1:2 |
| Compound 21 | prothioconazole | picoxystrobin | proquinazid | 1:1:1:1 | 2:1:1:1 | 3:1:1:1 |
| Compound 21 | prothioconazole | trifloxystrobin | proquinazid | 1:1:1:1 | 2:1:1:1 | 3:1:1:1 |
| Compound 21 | tebuconazole | azoxystrobin | | 1:1:1 | 2:1:1 | 3:1:1 |
| Compound 21 | tebuconazole | kresoxim-methyl | | 1:1:1 | 2:1:1 | 3:1:1 |
| Compound 21 | tebuconazole | picoxystrobin | | 1:1:1 | 2:1:1 | 3:1:1 |
| Compound 21 | tebuconazole | pyraclostrobin | | 1:1:1 | 2:1:1 | 3:1:1 |
| Compound 21 | tebuconazole | pyrametostrobin | | 1:1:1 | 2:1:1 | 3:1:1 |
| Compound 21 | tebuconazole | pyraoxystrobin | | 1:1:1 | 2:1:1 | 3:1:1 |
| Compound 21 | tebuconazole | trifloxystrobin | | 1:1:1 | 2:1:1 | 3:1:1 |
| Compound 21 | tebuconazole | bixafen | | 1:1:2 | 2:1:2 | 3:1:2 |
| Compound 21 | tebuconazole | boscalid | | 1:1:2 | 2:1:2 | 3:1:2 |
| Compound 21 | tebuconazole | cyflufenamid | | 1:2:1 | 2:2:1 | 3:2:1 |
| Compound 21 | tebuconazole | fluopyram | | 1:1:2 | 2:1:2 | 3:1:2 |
| Compound 21 | tebuconazole | isopyrazam | | 1:1:2 | 2:1:2 | 3:1:2 |
| Compound 21 | tebuconazole | metrafenone | | 1:1:2 | 2:1:2 | 3:1:2 |
| Compound 21 | tebuconazole | penthiopyrad | | 1:1:2 | 2:1:2 | 3:1:2 |
| Compound 21 | tebuconazole | proquinazid | | 1:1:1 | 2:1:1 | 3:1:1 |
| Compound 21 | tebuconazole | pyriofenone | | 1:1:2 | 2:1:2 | 3:1:2 |
| Compound 21 | tebuconazole | quinoxyfen | | 1:1:1 | 2:1:1 | 3:1:1 |
| Compound 21 | tebuconazole | sedaxane | | 1:1:2 | 2:1:2 | 3:1:2 |
| Compound 21 | tebuconazole | picoxystrobin | proquinazid | 1:1:1:1 | 2:1:1:1 | 3:1:1:1 |
| Compound 21 | tebuconazole | trifloxystrobin | proquinazid | 1:1:1:1 | 2:1:1:1 | 3:1:1:1 |

(*) Ratios of Component (a) relative to Component (b) in sequence, by weight.

Tables C2 through C26 are each constructed the same as Table C1 above except that entries below the "Component (a)" column heading are replaced with the respective Component (a) Column Entry shown below. Thus, for example, in Table C2 the entries below the "Component (a)" column heading all recite "Compound 57", and the first line in below the column headings in Table C2 specifically discloses a mixture of Compound 57 with cyproconazole and azoxystrobin, and the illustrative weight ratios of 1:1:1, 2:1:1 and 3:1:1 of Compound 57:cyproconazole:azoxystrobin. Tables C3 through C26 are constructed similarly.

| Table Number | Component (a) Column Entry | Table Number | Component (a) Column Entry |
|---|---|---|---|
| C2 | Compound 57 | C15 | Compound 400 |
| C3 | Compound 110 | C16 | Compound 401 |
| C4 | Compound 183 | C17 | Compound 402 |
| C5 | Compound 198 | C18 | Compound 463 |
| C6 | Compound 204 | C19 | Compound 505 |
| C7 | Compound 225 | C20 | Compound 506 |
| C8 | Compound 229 | C21 | Compound 507 |
| C9 | Compound 257 | C22 | Compound 508 |
| C10 | Compound 264 | C23 | Compound 509 |
| C11 | Compound 316 | C24 | Compound 510 |
| C12 | Compound 330 | C25 | Compound 511 |
| C13 | Compound 376 | C26 | Compound 512 |
| C14 | Compound 394 | | |

Of note is a composition of the present invention comprising a compound of Formula **1** (or an *N*-oxide or salt thereof) with at least one other fungicidal compound that has a different site of action from the compound of Formula **1.** In certain instances, a combination with at least one other fungicidal compound having a similar spectrum of control but a different site of action will be particularly advantageous for resistance management. Thus, a composition of the present invention can advantageously comprise at least one fungicidal active compound selected from the group consisting of (b1) through (b54) as described above, having a similar spectrum of control but a different site of action.

Compositions of component (a), or component (a) with component (b), can be further mixed with one or more other biologically active compounds or agents including insecticides, nematocides, bactericides, acaricides, herbicides, herbicide safeners, growth regulators such as insect molting inhibitors and rooting stimulants, chemosterilants, semiochemicals, repellents, attractants, pheromones, feeding stimulants, plant nutrients, other biologically active compounds or entomopathogenic bacteria, virus or fungi to form a multi-component pesticide giving an even broader spectrum of agricultural protection. Thus the present invention also pertains to a composition comprising a fungicidally effective amount of component (a), or a mixture of component (a) with component (b), and a biologically effective amount of at least one additional biologically active compound or agent and can further comprise at least one of a surfactant, a solid diluent or a liquid diluent. The other biologically active compounds or agents can also be separately formulated in compositions comprising at least one of a surfactant, solid or liquid diluent. For compositions of the present invention, one or more other biologically active compounds or agents can be formulated together with one or both of components (a) and (b) to form a premix, or one or more other biologically active compounds or agents can be formulated separately from components (a) and (b) and the formulations combined together before application (e.g., in a spray tank) or, alternatively, applied in succession.

Examples of such biologically active compounds or agents with which compositions of component (a), or component (a) with component (b), can be formulated are: insecticides such as abamectin, acephate, acequinocyl, acetamiprid, acrinathrin, acynonapyr, afidopyropen ([(3*S*,4*R*,4a*R*,6*S*,6a*S*,12*R*,12a*S*,12b*S*)-3-[(cyclopropylcarbonyl)oxy]-1,3,4,4a,5,6,6a,12,12a,12b-decahydro-6,12-dihydroxy-4,6a,12b-trimethyl-11-oxo-9-(3-pyridinyl)-2*H*,11*H*-naphtho[2,1-*b*]pyrano[3,4-*e*]pyran-4-yl]methyl cyclopropanecarboxylate), amidoflumet, amitraz, avermectin, azadirachtin, azinphos-methyl, benfuracarb, bensultap, benzpyrimoxan, bifenthrin, kappa-bifenthrin, bifenazate, bistrifluron, borate, broflanilide, buprofezin, cadusafos, carbaryl, carbofuran, cartap, carzol, chlorantraniliprole, chlorfenapyr, chlorfluazuron, chloroprallethrin, chlorpyrifos, chlorpyrifos-e, chlorpyrifos-methyl, chromafenozide, clofentezin, chloroprallethrin, clothianidin, cyantraniliprole (3-bromo-1-(3-chloro-2-pyridinyl)-*N*-[4-cyano-2-methyl-6-[(methylamino)carbonyl]phenyl]-1*N*-pyrazole-5-carboxamide), cyclaniliprole (3-bromo-*N*-[2-bromo-4-chloro-6-[[(1-cyclopropylethyl)amino]carbonyl]phenyl]-1-(3-chloro-2-pyridinyl)-1*H*-pyrazole-5-carboxamide), cycloprothrin, cycloxaprid ((5*S*,8*R*)-1-[(6-chloro-3-pyridinyl)methyl]-2,3,5,6,7,8-hexahydro-9-nitro-5,8-Epoxy-1*H*-imidazo[1,2-*a*]azepine), cyenopyrafen, cyflumetofen, cyfluthrin, beta-cyfluthrin, cyhalodiamide, cyhalothrin, gamma-cyhalothrin, lambda-cyhalothrin, cypermethrin, alpha-cypermethrin, zeta-cypermethrin, cyromazine, deltamethrin, diafenthiuron, diazinon, dicloromesotiaz, dieldrin, diffubenzuron, dimefluthrin, dimehypo, dimethoate, dimpropyridaz, dinotefuran, diofenolan, emamectin, emamectin benzoate, endosulfan, esfenvalerate, ethiprole, etofenprox, epsilon-metofluthrin, etoxazole, fenbutatin oxide, fenitrothion, fenothiocarb, fenoxycarb, fenpropathrin, fenvalerate, fipronil, flometoquin (2-ethyl-3,7-dimethyl-6-[4-(trifluoromethoxy)phenoxy]-4-quinolinyl methyl carbonate), flonicamid, fluazaindolizine, flubendiamide, flucythrinate, flufenerim, flufenoxuron, flufenoxystrobin (methyl ($\alpha E$)-2-[[2-chloro-4-(trifluoromethyl)phenoxy]methyl]-$\alpha$-(methoxymethylene)ben-

zene-acetate), fluensulfone (5-chloro-2-[(3,4,4-trifluoro-3-buten-1-yl)sulfonyl]thiazole), fluhexafon, fluopyram, flupiprole (1-[2,6-dichloro-4-(trifluoromethyl)phenyl]-5-[(2-methyl-2-propen-1-yl)-amino]-4-[(trifluoromethyl)sulfinyl]-1*H*-pyrazole-3-carbonitrile), flupyradifurone (4-[[(6-chloro-3-pyridinyl)methyl](2,2-difluoroethyl)amino]-2(5*H*)-furanone), flupyrimin, fluvalinate, tau-fluvalinate, fluxametamide, fonophos, formetanate, fosthiazate, gamma-cyhalothrin, halofenozide, heptafluthrin ([2,3,5,6-tetrafluoro-4-(methoxymethyl)phenyl]methyl 2,2-dimethyl-3-[(1*Z*)-3,3,3-trifluoro-1-propen-1-yl]cyclopropanecarboxylate), hexaflumuron, hexythiazox, hydramethylnon, imidacloprid, indoxacarb, insecticidal soaps, isofenphos, isocycloseram, kappa-tefluthrin, lambda-cyhalothrin, lufenuron, malathion, meperfluthrin ([2,3,5,6-tetrafluoro-4-(methoxymethyl)phenyl]methyl (1*R*,3*S*)-3-(2,2-dichloroethenyl)-2,2-dimethylcyclopropanecarboxylate), metaflumizone, metaldehyde, methamidophos, methidathion, methiocarb, methomyl, methoprene, methoxychlor, metofluthrin, methoxyfenozide, epsilon-metofluthrin, epsilon-momfluorothrin, monocrotophos, monofluorothrin ([2,3,5,6-tetrafluoro-4-(methoxymethyl)phenyl]methyl 3-(2-cyano-1-propen-1-yl)-2,2-dimethylcyclopropanecarboxylate), nicotine, nitenpyram, nithiazine, novaluron, noviflumuron, oxamyl, oxazosulfyl, parathion, parathion-methyl, permethrin, phorate, phosalone, phosmet, phosphamidon, pirimicarb, profenofos, profluthrin, propargite, protrifenbute, pyflubumide (1,3,5-trimethyl-*N*-(2-methyl-1-oxopropyl)-*N*-[3-(2-methylpropyl)-4-[2,2,2-trifluoro-1-methoxy-1-(trifluoromethyl)ethyl]-phenyl]-1*H*-pyrazole-4-carboxamide), pymetrozine, pyrafluprole, pyrethrin, pyridaben, pyridalyl, pyrifluquinazon, pyriminostrobin (methyl ($\alpha E$)-2-[[[2-[(2,4-dichlorophenyl)amino]-6-(trifluoromethyl)-4-pyrimidinyl]oxy]methyl]-$\alpha$-(methoxymethylene)benzeneacetate), pyriprole, pyriproxyfen, rotenone, ryanodine, silafluofen, spinetoram, spinosad, spirodiclofen, spiromesifen, spiropidion, spirotetramat, sulprofos, sulfoxaflor (*N*-[methyloxido[1-[6-(trifluoromethyl)-3-pyridinyl]ethyl]-$\lambda^4$-sulfanylidene]cyanamide), tebufenozide, tebufenpyrad, teflubenzuron, tefluthrin, kappa-tefluthrin, terbufos, tetrachlorantraniliprole, tetrachlorvinphos, tetramethrin, tetramethylfluthrin ([2,3,5,6-tetrafluoro-4-(methoxymethyl)phenyl]methyl 2,2,3,3-tetramethylcyclopropanecarboxylate), tetraniliprole, thiacloprid, thiamethoxam, thiodicarb, thiosultap-sodium, tioxazafen (3-phenyl-5-(2-thienyl)-1,2,4-oxadiazole), tolfenpyrad, tralomethrin, triazamate, trichlorfon, triflumezopyrim (2,4-dioxo-1-(5-pyrimidinylmethyl)-3-[3-(trifluoromethyl)phenyl]-2*H*-pyrido[1,2-*a*]pyrimidinium inner salt), triflumuron, tyclopyrazoflor, zeta-cypermethrin, *Bacillus thuringiensis* delta-endotoxins, entomopathogenic bacteria, entomopathogenic viruses or entomopathogenic fungi.

One embodiment of biological agents for mixing with compounds of this disclosure include entomopathogenic bacteria such as *Bacillus thuringiensis,* and the encapsulated delta-endotoxins of *Bacillus thuringiensis* such as MVP® and MVPII® bioinsecticides prepared by the CellCap® process (CellCap®, MVP® and MVPII® are trademarks of Mycogen Corporation, Indianapolis, Indiana, USA); entomopathogenic fungi such as green muscardine fungus; and entomopathogenic (both naturally occurring and genetically modified) viruses including baculovirus, nucleopolyhedro virus (NPV) such as *Helicoverpa zea* nucleopolyhedrovirus (HzNPV), *Anagrapha falcifera* nucleopolyhedrovirus (AfNPV); and granulosis virus (GV) such as *Cydia pomonella* granulosis virus (CpGV).

General references for these agricultural protectants (i.e. insecticides, fungicides, nematocides, acaricides, herbicides and biological agents) include The Pesticide Manual, 13th Edition, C. D. S. Tomlin, Ed., British Crop Protection Council, Farnham, Surrey, U.K., 2003 and The BioPesticide Manual, 2nd Edition, L. G. Copping, Ed., British Crop Protection Council, Farnham, Surrey, U.K., 2001.

For embodiments where one or more of these various mixing partners are used, the weight ratio of these various mixing partners (in total) to component (a), or a mixture of component (a) with component (b), is generally between about 1:3000 and about 3000:1. Of note are weight ratios between about 1:100 and about 3000:1, or between about 1:30 and about 300:1 (for example ratios between about 1:1 and about 30:1). It will be evident that including these additional components may expand the spectrum of diseases controlled beyond the spectrum controlled by component (a), or a mixture of component (a) with component (b).

Component (a) compounds and/or combinations thereof with component (b) compounds and/or one or more other biologically active compounds or agents can be applied to plants genetically transformed to express proteins toxic to invertebrate pests (such as *Bacillus thuringiensis* delta-endotoxins). The effect of the exogenously applied present component (a) alone or in combination with component (b) may be synergistic with the expressed toxin proteins.

Of note is the combination or the composition comprising component (a), or components (a) and (b), as described in the Summary of the Invention further comprising at least one invertebrate pest control compound or agent (e.g., insecticide, acaricide). Of particular note is a composition comprising component (a) and at least one (i.e. one or more) invertebrate pest control compound or agent, which then can be subsequently combined with component (b) to provide a composition comprising components (a) and (b) and the one or more invertebrate pest control compounds or agents. Alternatively without first mixing with component (b), a biologically effective amount of the composition comprising component (a) with at least one invertebrate pest control agent can be applied to a plant or plant seed (directly or through the environment of the plant or plant seed) to protect the plant or plant seed from diseases caused by fungal pathogens and injury caused by invertebrate pests.

For embodiments where one or more of invertebrate pest control compounds are used, the weight ratio of these compounds (in total) to the component (a) compounds is typically between about 1:3000 and about 3000:1. Of note are weight ratios between about 1:300 and about 300:1 (for example ratios between about 1:30 and about 30:1). One skilled

in the art can easily determine through simple experimentation the biologically effective amounts of active ingredients necessary for the desired spectrum of biological activity.

Of note is a composition of the present invention which comprises in addition to a component (a) compound, alone or in combination with component (b), at least one invertebrate pest control compound or agent selected from the group consisting abamectin, acetamiprid, acrinathrin, acynonapyr, afidopyropen, amitraz, avermectin, azadirachtin, benfuracarb, bensultap, bifenthrin, buprofezin, broflanilide, cadusafos, carbaryl, cartap, chlorantraniliprole, chloroprallethrin, chlorfenapyr, chlorpyrifos, clothianidin, cyantraniliprole, cyclaniliprole, cycloprothrin, cyfluthrin, beta-cyfluthrin, cyhalothrin, gamma-cyhalothrin, lambda-cyhalothrin, cypermethrin, alpha-cypermethrin, zeta-cypermethrin, cyromazine, deltamethrin, dieldrin, dinotefuran, diofenolan, emamectin, endosulfan, epsilon-metofluthrin, esfenvalerate, ethiprole, etofenprox, etoxazole, fenitrothion, fenothiocarb, fenoxycarb, fenvalerate, fipronil, flometoquin, fluxametamide, flonicamid, flubendiamide, fluensulfone, flufenoxuron, flufenoxystrobin, flufensulfone, flupiprole, flupyrimin, flupyradifurone, fluvalinate, formetanate, fosthiazate, gamma-cyhalothrin, heptafluthrin, hexaflumuron, hydramethylnon, imidacloprid, indoxacarb, isocycloseram, kappa-tefluthrin, lambda-cyhalothrin, lufenuron, meperfluthrin, metaflumizone, methiodicarb, methomyl, methoprene, methoxyfenozide, metofluthrin, monofluorothrin, nitenpyram, nithiazine, novaluron, oxamyl, pyflubumide, pymetrozine, pyrethrin, pyridaben, pyridalyl, pyriminostrobin, pyriproxyfen, ryanodine, spinetoram, spinosad, spirodiclofen, spiromesifen, spirotetramat, sulfoxaflor, tebufenozide, tetramethrin, tetramethylfluthrin, thiacloprid, thiamethoxam, thiodicarb, thiosultap-sodium, tralomethrin, triazamate, triflumezopyrim, triflumuron, tyclopyrazoflor, zeta-cypermethrin, *Bacillus thuringiensis* delta-endotoxins, all strains of *Bacillus thuringiensis* and all strains of nucleo polyhedrosis viruses.

In certain instances, combinations of a a component (a) compound of this invention, alone or in mixture with component (b), with other biologically active (particularly fungicidal) compounds or agents (i.e. active ingredients) can result in a greater-than-additive (i.e. synergistic) effect. Reducing the quantity of active ingredients released in the environment while ensuring effective pest control is always desirable. When an enhanced effect of fungicidal active ingredients occurs at application rates giving agronomically satisfactory levels of fungal control, such combinations can be advantageous for reducing crop production cost and decreasing environmental load.

Table D1 lists specific combinations of invertebrate pest control agents with Compound 21 (compound numbers refer to compounds in Index Tables A through T) as a component (a) compound illustrative of mixtures and compositions comprising these active ingredients and methods using them according to the present invention. The second column of Table D1 lists the specific invertebrate pest control agents (e.g., "Abamectin" in the first line). The third column of Table D1 lists the mode of action (if known) or chemical class of the invertebrate pest control agents. The fourth column of Table D1 lists embodiment(s) of ranges of weight ratios for rates at which the invertebrate pest control agent is typically applied relative to Compound 21 alone or in combination with component (b) (e.g., "50:1 to 1:50" of abamectin relative to a Compound 21 by weight). Thus, for example, the first line of Table D1 specifically discloses the combination of Compound 21 with abamectin is typically applied in a weight ratio between 50:1 to 1:50. The remaining lines of Table D1 are to be construed similarly. Thus, for example, the first line of Table D1 specifically discloses the combination of Compound 21 with abamectin is typically applied in a weight ratio between 50:1 to 1:50. The remaining lines of Table D1 are to be construed similarly.

Table D1

| Table Number | Invertebrate Pest Control Agent | Mode of Action or Chemical Class | Typical Weight Ratio |
|---|---|---|---|
| Compound 21 | Abamectin | macrocyclic lactones | 50:1 to 1:50 |
| Compound 21 | Acetamiprid | neonicotinoids | 150:1 to 1:200 |
| Compound 21 | Amitraz | octopamine receptor ligands | 200:1 to 1:100 |
| Compound 21 | Avermectin | macrocyclic lactones | 50:1 to 1:50 |
| Compound 21 | Azadirachtin | ecdysone agonists | 100:1 to 1:120 |
| Compound 21 | Beta-cyfluthrin | sodium channel modulators | 150:1 to 1:200 |
| Compound 21 | Bifenthrin | sodium channel modulators | 100:1 to 1:10 |
| Compound 21 | Buprofezin | chitin synthesis inhibitors | 500:1 to 1:50 |
| Compound 21 | Cartap | nereistoxin analogs | 100:1 to 1:200 |
| Compound 21 | Chlorantraniliprole | ryanodine receptor ligands | 100:1 to 1:120 |
| Compound 21 | Chlorfenapyr | mitochondrial electron transport inhibitors | 300:1 to 1:200 |
| Compound 21 | Chlorpyrifos | cholinesterase inhibitors | 500:1 to 1:200 |
| Compound 21 | Clothianidin | neonicotinoids | 100:1 to 1:400 |
| Compound 21 | Cyantraniliprole | ryanodine receptor ligands | 100:1 to 1:120 |

(continued)

| Table Number | Invertebrate Pest Control Agent | Mode of Action or Chemical Class | Typical Weight Ratio |
|---|---|---|---|
| Compound 21 | Cyfluthrin | sodium channel modulators | 150:1 to 1:200 |
| Compound 21 | Cyhalothrin | sodium channel modulators | 150:1 to 1:200 |
| Compound 21 | Cypermethrin | sodium channel modulators | 150:1 to 1:200 |
| Compound 21 | Cyromazine | chitin synthesis inhibitors | 400:1 to 1:50 |
| Compound 21 | Deltamethrin | sodium channel modulators | 50:1 to 1:400 |
| Compound 21 | Dieldrin | cyclodiene insecticides | 200:1 to 1:100 |
| Compound 21 | Dinotefuran | neonicotinoids | 150:1 to 1:200 |
| Compound 21 | Diofenolan | molting inhibitor | 150:1 to 1:200 |
| Compound 21 | Emamectin | macrocyclic lactones | 50:1 to 1:10 |
| Compound 21 | Endosulfan | cyclodiene insecticides | 200:1 to 1:100 |
| Compound 21 | Esfenvalerate | sodium channel modulators | 100:1 to 1:400 |
| Compound 21 | Ethiprole | GABA-regulated chloride channel blockers | 200:1 to 1:100 |
| Compound 21 | Fenothiocarb | | 150:1 to 1:200 |
| Compound 21 | Fenoxycarb | juvenile hormone mimics | 500:1 to 1:100 |
| Compound 21 | Fenvalerate | sodium channel modulators | 150:1 to 1:200 |
| Compound 21 | Fipronil | GABA-regulated chloride channel blockers | 150:1 to 1:100 |
| Compound 21 | Flonicamid | | 200:1 to 1:100 |
| Compound 21 | Flubendiamide | ryanodine receptor ligands | 100:1 to 1:120 |
| Compound 21 | Flufenoxuron | chitin synthesis inhibitors | 200:1 to 1:100 |
| Compound 21 | Hexaflumuron | chitin synthesis inhibitors | 300:1 to 1:50 |
| Compound 21 | Hydramethylnon | mitochondrial electron transport inhibitors | 150:1 to 1:250 |
| Compound 21 | Imidacloprid | neonicotinoids | 1000:1 to 1:1000 |
| Compound 21 | Indoxacarb | sodium channel modulators | 200:1 to 1:50 |
| Compound 21 | Lambda-cyhalothrin | sodium channel modulators | 50:1 to 1:250 |
| Compound 21 | Lufenuron | chitin synthesis inhibitors | 500:1 to 1:250 |
| Compound 21 | Meperfluthrin | sodium channel modulators | 100:1 to 1:400 |
| Compound 21 | Metaflumizone | | 200:1 to 1:200 |
| Compound 21 | Methomyl | cholinesterase inhibitors | 500:1 to 1:100 |
| Compound 21 | Methoprene | juvenile hormone mimics | 500:1 to 1:100 |
| Compound 21 | Methoxyfenozide | ecdysone agonists | 50:1 to 1:50 |
| Compound 21 | Nitenpyram | neonicotinoids | 150:1 to 1:200 |
| Compound 21 | Nithiazine | neonicotinoids | 150:1 to 1:200 |
| Compound 21 | Novaluron | chitin synthesis inhibitors | 500:1 to 1:150 |
| Compound 21 | Oxamyl | cholinesterase inhibitors | 200:1 to 1:200 |
| Compound 21 | Pymetrozine | | 200:1 to 1:100 |
| Compound 21 | Pyrethrin | sodium channel modulators | 100:1 to 1:10 |
| Compound 21 | Pyridaben | mitochondrial electron transport inhibitors | 200:1 to 1:100 |
| Compound 21 | Pyridalyl | | 200:1 to 1:100 |
| Compound 21 | Pyriproxyfen | juvenile hormone mimics | 500:1 to 1:100 |
| Compound 21 | Ryanodine | ryanodine receptor ligands | 100:1 to 1:120 |
| Compound 21 | Spinetoram | macrocyclic lactones | 150:1 to 1:100 |
| Compound 21 | Spinosad | macrocyclic lactones | 500:1 to 1:10 |
| Compound 21 | Spirodiclofen | lipid biosynthesis inhibitors | 200:1 to 1:200 |
| Compound 21 | Spiromesifen | lipid biosynthesis inhibitors | 200:1 to 1:200 |
| Compound 21 | Sulfoxaflor | | 200:1 to 1:200 |

(continued)

| Table Number | Invertebrate Pest Control Agent | Mode of Action or Chemical Class | Typical Weight Ratio |
|---|---|---|---|
| Compound 21 | Tebufenozide | ecdysone agonists | 500:1 to 1:250 |
| Compound 21 | Tetramethylfluthrin | sodium channel modulators | 100:1 to 1:40 |
| Compound 21 | Thiacloprid | neonicotinoids | 100:1 to 1:200 |
| Compound 21 | Thiamethoxam | neonicotinoids | 1250:1 to 1:1000 |
| Compound 21 | Thiodicarb | cholinesterase inhibitors | 500:1 to 1:400 |
| Compound 21 | Thiosultap-sodium | | 150:1 to 1:100 |
| Compound 21 | Tralomethrin | sodium channel modulators | 150:1 to 1:200 |
| Compound 21 | Triazamate | cholinesterase inhibitors | 250:1 to 1:100 |
| Compound 21 | Triflumuron | chitin synthesis inhibitors | 200:1 to 1:100 |
| Compound 21 | *Bacillus thuringiensis* | biological agents | 50:1 to 1:10 |
| Compound 21 | *Bacillus thuringiensis* delta-endotoxin | biological agents | 50:1 to 1:10 |
| Compound 21 | NPV (e.g., Gemstar) | biological agents | 50:1 to 1:10 |

Tables D2 through D26 are each constructed the same as Table D 1 above except that entries below the "Component (a)" column heading are replaced with the respective Component (a) Column Entry shown below. Thus, for example, in Table D2 the entries below the "Component (a)" column heading all recite "Compound 57", and the first line in below the column headings in Table D2 specifically discloses a mixture of Compound 57 with abamectin. Tables D3 through D26 are constructed similarly.

| Table Number | Component (a) Column Entry | Table Number | Component (a) Column Entry |
|---|---|---|---|
| D2 | Compound 57 | D15 | Compound 400 |
| D3 | Compound 110 | D16 | Compound 401 |
| D4 | Compound 183 | D17 | Compound 402 |
| D5 | Compound 198 | D18 | Compound 463 |
| D6 | Compound 204 | D19 | Compound 505 |
| D7 | Compound 225 | D20 | Compound 506 |
| D8 | Compound 229 | D21 | Compound 507 |
| D9 | Compound 257 | D22 | Compound 508 |
| D10 | Compound 264 | D23 | Compound 509 |
| D11 | Compound 316 | D24 | Compound 510 |
| D12 | Compound 330 | D25 | Compound 511 |
| D13 | Compound 376 | D26 | Compound 512 |
| D14 | Compound 394 | | |

Compositions comprising compounds of Formula **1** useful for seed treatment can further comprise bacteria and fungi that have the ability to provide protection from the harmful effects of plant pathogenic fungi or bacteria and/or soil born animals such as nematodes. Bacteria exhibiting nematicidal properties may include but are not limited to *Bacillus firmus, Bacillus cereus, Bacillius subtiliis* and *Pasteuria penetrans.* A suitable *Bacillus firmus* strain is strain CNCM I-1582 (GB-126) which is commercially available as BioNem™. A suitable *Bacillus cereus* strain is strain NCMM I-1592. Both *Bacillus* strains are disclosed in US 6,406,690. Other suitable bacteria exhibiting nematicidal activity are *B. amyloliquefaciens* IN937a and *B. subtilis* strain GB03. Bacteria exhibiting fungicidal properties may include but are not limited to *B. pumilus* strain GB34. Fungal species exhibiting nematicidal properties may include but are not limited to *Myrothecium verrucaria, Paecilomyces lilacinus* and *Purpureocillium lilacinum.*

Seed treatments can also include one or more nematicidal agents of natural origin such as the elicitor protein called harpin which is isolated from certain bacterial plant pathogens such as *Erwinia amylovora.* An example is the Harpin-N-Tek seed treatment technology available as N-Hibit™ Gold CST.

Seed treatments can also include one or more species of legume-root nodulating bacteria such as the microsymbiotic nitrogen-fixing bacteria *Bradyrhizobium japonicum.* These inocculants can optionally include one or more lipo-chitooligosaccharides (LCOs), which are nodulation (Nod) factors produced by rhizobia bacteria during the initiation

of nodule formation on the roots of legumes. For example, the Optimize® brand seed treatment technology incorporates LCO Promoter Technology™ in combination with an inocculant.

Seed treatments can also include one or more isoflavones which can increase the level of root colonization by mycorrhizal fungi. Mycorrhizal fungi improve plant growth by enhancing the root uptake of nutrients such as water, sulfates, nitrates, phosphates and metals. Examples of isoflavones include, but are not limited to, genistein, biochanin A, formononetin, daidzein, glycitein, hesperetin, naringenin and pratensein. Formononetin is available as an active ingredient in mycorrhizal inocculant products such as PHC Colonize® AG.

Seed treatments can also include one or more plant activators that induce systemic acquired resistance in plants following contact by a pathogen. An example of a plant activator which induces such protective mechanisms is acibenzolar-5-methyl.

In the present fungicidal compositions, the Formula **1** compounds of component (a) can work synergically with the additional fungicidal compounds of component (b) to provide such beneficial results as broadening the spectrum of plant diseases controlled, extending duration of preventative and curative protection, and suppressing proliferation of resistant fungal pathogens. In particular embodiments, compositions are provided in accordance with this invention that comprise proportions of component (a) and component (b) that are especially useful for controlling particular fungal diseases (such as *Alternaria solani, Blumeria graminis* f. sp. *tritici, Botrytis cinerea, Puccinia recondita* f. sp. *tritici, Rhizoctonia solani, Septoria nodorum, Septoria tritici*).

Mixtures of fungicides may also provide significantly better disease control than could be predicted based on the activity of the individual components. This synergism has been described as "the cooperative action of two components of a mixture, such that the total effect is greater or more prolonged than the sum of the effects of the two (or more) taken independently" (see P. M. L. Tames, *Neth. J. Plant Pathology* **1964,** *70*, 73-80). In methods providing plant disease control in which synergy is exhibited from a combination of active ingredients (e.g., fungicidal compounds) applied to the plant or seed, the active ingredients are applied in a synergistic weight ratio and synergistic (i.e. synergistically effective) amounts. Measures of disease control, inhibition and prevention cannot exceed 100%. Therefore expression of substantial synergism typically requires use of application rates of active ingredients wherein the active ingredients separately provide much less than 100% effect, so that their additive effect is substantially less than 100% to allow the possibility of increase in effect as result of synergism. On the other hand, application rates of active ingredients that are too low may show not show much activity in mixtures even with the benefit of synergism. One skilled in the art can easily identify and optimize through simple experimentation the weight ratios and application rates (i.e. amounts) of fungicidal compounds providing synergy.

The presence of a synergistic effect between two active ingredients was established with the aid of the Colby equation (see Colby, S. R. "Calculating Synergistic and Antagonistic Responses of Herbicide Combinations", Weeds, (1967), 15, 20-22):

$$p = A + B - \left[\frac{A \times B}{100}\right].$$

Using the method of Colby, the presence of a synergistic interaction between two active ingredients is established by first calculating the predicted activity, p, of the mixture based on activities of the two components applied alone. If p is lower than the experimentally established effect, synergism has occurred. In the equation above, A is the fungicidal activity in percentage control of one component applied alone at rate x. The B term is the fungicidal activity in percentage control of the second component applied at rate y. The equation estimates p, the expected fungicidal activity of the mixture of A at rate x with B at rate y if their effects are strictly additive and no interaction has occurred.

The following TESTS demonstrate the control efficacy of compounds of this invention on specific pathogens. The pathogen control protection afforded by the compounds is not limited, however, to these species. See Index Tables A through T below for compound descriptions. The following abbreviations are used in the Index Tables: Me means methyl, CN means cyano, $NO_2$ means nitro, Et means ethyl, *n*-Pr means *n*-propyl, *i*-Pr means *iso*-propyl, *c*-Pr means cyclopropyl, *i*-Bu means *iso*-butyl, *t*-Bu means *tert-butyl,* Ph means phenyl, MeO means methoxy, EtO means ethoxy and Ac means acetyl. The abbreviation "Cmpd. No." stands for "Compound Number", and the abbreviation "Ex." stands for "Example" and is followed by a number indicating in which example the compound is prepared. $^{19}F$ NMR spectra are reported in ppm relative to trichlorofluoromethane in $CDCl_3$ solution unless indicated otherwise. The numerical value reported in the column "MS" is the molecular weight of the highest isotopic abundance positively charged parent ion (M+1) formed by addition of $H^+$ (molecular weight of 1) to the molecule having the highest isotopic abundance, or the highest isotopic abundance negatively charged ion (M-1) formed by loss of $H^+$ (molecular weight of 1). The presence of molecular ions containing one or more higher atomic weight isotopes of lower abundance (e.g., $^{37}Cl$, $^{81}Br$) is not reported. The reported MS peaks were observed by mass spectrometry using electrospray ionization (ESI) or atmospheric pressure

chemical ionization (APCI).

INDEX TABLE A

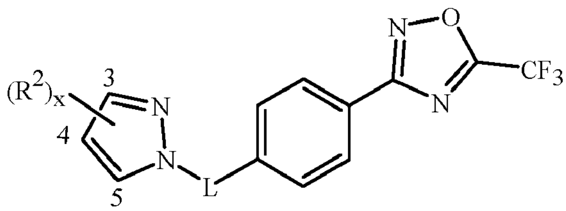

A dash "-" in the $(R^2)_X$ column means that no $R^2$ substituent is present and the remaining carbon valences are occupied by hydrogen atoms. In the L column, the atom to the right is connected to the phenyl ring and the atom to the left is connected to the pyrazolyl ring.

| Cmpd. No. | $(R^2)_X$ | L | $^{19}F$ NMR | MS |
|---|---|---|---|---|
| 1 | 3-(EtOC(=O)), 4-Br, 5-Me | $CH_2$ | -65.40 | |
| 2 (Ex. 12) | 4-(EtOC(=O)) | CH(Me) | -65.44 | |
| 6 | 3-N≡C | $CH_2$ | -65.39 | |
| 7 | 5-N≡C | $CH_2$ | -65.39 | |
| 17 | 4-(CH(=O)) | $CH_2$ | -65.34 | |
| 18 | 4-(MeOC(=O)NHN=CH) | $CH_2$ | -65.34 | |
| 19 | 4-($Me_2$NN=CH) | $CH_2$ | -65.35 | |
| 20 | 4-(OH-N=CH) | $CH_2$ | -65.34, -66.15 | |
| 21 | 4-(MeOC(=O)) | $CH_2$ | -65.41 | |
| 25 | 4-(MeON=CH) | $CH_2$ | -65.34 | |
| 26 | 4-(OHC(=O)$CH_2$ON=CH) | $CH_2$ | -65.34 | |
| 55 | 4-(OHC(=O)) | $CH_2$ | -65.39 | |
| 56 | 4-(HC≡C$CH_2$OC(=O)) | $CH_2$ | -65.39 | |
| 57 | 4-(N≡C$CH_2$OC(=O)) | $CH_2$ | -65.38 | |
| 58 | 4-(*i*-PrOC(=O)) | $CH_2$ | -65.39 | |
| 59 | 4-(MeO$CH_2CH_2$OC(=O)) | $CH_2$ | -65.39 | |
| 60 | 4-(*n*-PrOC(=O)) | $CH_2$ | -65.39 | |
| 61 | 4-(MeNHC(=O)) | $CH_2$ | -64.74[a] | |
| 62 | 4-($Me_2$NC(=O)) | $CH_2$ | -65.41 | |
| 63 | 4-(*i*-PrNHC(=O)) | $CH_2$ | -64.74[a] | |
| 64 | 4-($CH_2$=CH$CH_2$NHC(=O)) | $CH_2$ | -64.74[a] | |
| 65 | 4-(N≡C$CH_2$NHC(=O)) | $CH_2$ | -64.74[a] | |
| 66 | 3,5-di-Me, 4-(EtOC(=O)) | $CH_2$ | -65.35 | |
| 67 | 4-(EtNHC(=O)) | $CH_2$ | -64.73[a] | |
| 68 | 4-(*n*-PrNHC(=O)) | $CH_2$ | -64.74[a] | |
| 69 | 4-(*c*-PrNHC(=O)) | $CH_2$ | -64.75[a] | |
| 78 | 4-(*t*-BuNHC(=O) | $CH_2$ | -65.38 | |
| 79 | 3-(EtOC(=O)), 5-Et | $CH_2$ | -65.42 | |
| 80 | 3-(EtOC(=O)), 5-*i*-Pr | $CH_2$ | -65.39 | |
| 81 | 3-Et, 5-(EtOC(=O)) | $CH_2$ | -65.43 | |
| 82 | 3-*i*-Pr, 5-(EtOC(=O)) | $CH_2$ | -65.41 | |
| 83 (Ex. 10) | 4-(EtOC(=O)) | $CH_2$ | -65.38 | |

(continued)

| Cmpd. No. | $(R^2)_X$ | L | $^{19}F$ NMR | MS |
|---|---|---|---|---|
| 85 | 3-Ph | $CH_2$ | -65.35 | |
| 95 (Ex. 2) | 4-N≡C | $CH_2$ | -65.33 | |
| 97 | 3-Br | $CH_2$ | | 375 (M+1) |
| 98 | 3-*t*-Bu | $CH_2$ | | 351 (M+1) |
| 109 | 3-Me, 5-(EtOC(=O)) | $CH_2$ | -65.47 | |
| 110 | 3-(EtOC(=O)), 5-Me | $CH_2$ | -65.53 | |
| 111 | 3-$CF_3$, 4-(EtOC(=O)) | $CH_2$ | -65.38 | |
| 114 | 3-(EtOC(=O)) | $CH_2$ | | 367 (M+1) |
| 122 | 3-(4-Cl-Ph) | $CH_2$ | | 405 (M+1) |
| 127 | 3-Me, 5-$CF_3$ | $CH_2$ | -65.42 | |
| 132 | 3-$CF_3$, 5-Me | $CH_2$ | -65.43 | |
| 140 | 3,5-di-(OHC(=O)) | $CH_2$ | | 381 (M+1) |
| 142 | 3-(2-Cl-Ph) | $CH_2$ | | 405 (M+1) |
| 144 | 3,5-di-$CF_3$ | $CH_2$ | | 431 (M+1) |
| 145 | 3,5-di-Me | $CH_2$ | | 323 (M+1) |
| 146 | 3-(2-Cl-Ph), 4-Br | $CH_2$ | | 485 (M+1) |
| 153 | 3-$CF_3$ | $CH_2$ | -65.35, -61.93 | |
| 154 | 3,5-di-(EtOC(=O)) | $CH_2$ | -65.42 | |
| 162 | - | $CH_2$ | -65.36 | |
| 163 | 4-Br | $CH_2$ | -65.34 | |
| 166 | 4-($CH_2$=$CHCH_2OC$(=O)) | $CH_2$ | -65.39 | |
| 167 | 4-($CH_2$=$CBrCH_2OC$(=O)) | $CH_2$ | -65.39 | |
| 168 | 4-($CH_2$=$CHCF_2OC$(=O)) | $CH_2$ | -65.39, -83.18 | |
| 169 | 4-($Me_2C$=$CHCH_2OC$(=O)) | $CH_2$ | -65.39 | |
| 170 | 4-($CH_2$=C(Me)$CH_2OC$(=O)) | $CH_2$ | -65.39 | |
| 171 | 4-(*i*-BuOC(=O)) | $CH_2$ | -65.39 | |
| 172 | 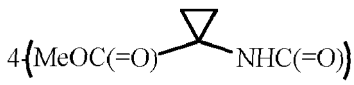 | $CH_2$ | -65.39 | |
| 173 | 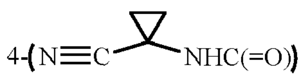 | $CH_2$ | -65.39 | |
| 174 | 3,4,5-tri-(EtOC(=O)) | $CH_2$ | -65.48 | |
| 179 | 3-(OHC(=O)), 5-Me | $CH_2$ | -65.40 | |
| 180 | 3-($CF_3C$(=O)OC(=O)), 5-Me | $CH_2$ | -65.40, -72.88 | |
| 181 | 3-(N≡$CCH_2NHC$(=O)), 5-Me | $CH_2$ | -65.39 | |
| 182 | 3-($Me_2NC$(=O)), 5-Me | $CH_2$ | -65.40 | |
| 183 | 3-($MeOCH_2CH_2NHC$(=O)), 5-Me | $CH_2$ | -65.40 | |
| 184 | 3-(N≡$CCH_2OC$(=O)), 5-Me | $CH_2$ | -65.40 | |
| 185 | 3-(N≡$CCH_2OC$(=O)), 5-Me | $CH_2$ | -65.40 | |
| 186 | 3-($CH_2$=$CHCH_2OC$(=O)), 5-Me | $CH_2$ | -65.41 | |
| 187 | 3-(EtOC(=O)), 5-t-Bu | $CH_2$ | -65.44 | |
| 212 | 4-($CF_3CH_2NHC$(=O)) | $CH_2O$ | | 436 (M+1) |
| 213 | 4-($MeOCH_2CH_2NHC$(=O)) | $CH_2O$ | | 412 (M+1) |
| 214 | 4-(N≡$CCH_2NHC$(=O)) | $CH_2O$ | | 393 (M+1) |

(continued)

| Cmpd. No. | $(R^2)_X$ | L | $^{19}F$ NMR | MS |
|---|---|---|---|---|
| 215 | 4-(1*H*-pyrazol-1-yl-$CH_2CH_2NHC(=O)$) | $CH_2O$ | | 448 (M+1) |
| 216 | 4-(*c*-$PrCH_2OC(=O)$) | $CH_2O$ | | 409 (M+1) |
| 217 | 4-(*n*-PiOC(=O)) | $CH_2O$ | | 397 (M+1) |
| 218 | 4-[(tetrahydro-2*H*-pyran-2-yl)ON=C (Me)$CH_2OC(=O)$] | $CH_2$ | | 492 (M-1) |
| 219 | 4-(*n*-BuON=C(Me)$CH_2OC(=O)$) | $CH_2$ | | 466 (M+1) |
| 220 | 4-(*t*-BuON=C(Me)$CH_2OC(=O)$) | $CH_2$ | | 466 (M+1) |
| 221 | 4-(EtON=C(Me)$CH_2OC(=O)$) | $CH_2$ | | 438 (M+1) |
| 222 | 4-(*i*-PrON=C(Me)$CH_2OC(=O)$) | $CH_2$ | | 452 (M+1) |
| 223 | 4-(HO-N=C(Me)$CH_2OC(=O)$) | $CH_2$ | | 410 (M+1) |
| 241 | 4-(EtOC(=O)) | $CH_2SCH_2$ | | 411 (M-1) |
| 242 | 4-(EtOC(=O)) | $CH_2S(O)CH_2$ | | 429 (M+1) |
| 243 | 4-(EtOC(=O)) | $CH_2S(O)_2CH_2$ | | 445 (M+1) |
| 244 | 4-(PhC(=O)$CH_2OC(=O)$) | $CH_2$ | | 457 (M+1) |
| 245 | 4-(MeON=C(Ph)$CH_2OC(=O)$) | $CH_2$ | | 486 (M+1) |
| 268 | 4-(2-EtO-Ph-$OCH_2CH_2OC(=O)$) | $CH_2$ | -65.30 | |
| 269 | 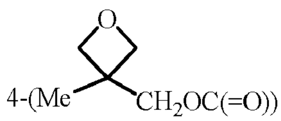 | $CH_2$ | -65.30 | |
| 270 | 4-(*i*-PtONHC(=O)) | $CH_2$ | -64.70[a] | |
| 272 | 4-(MeONHC(=O)) | $CH_2$ | -64.50[a] | |
| 273 | 4-(t-BuONHC(=O)) | $CH_2$ | | 408 (M+1) |
| 274 | 4-(N≡$CCH_2CH_2CH_2OC(=O)$) | $CH_2$ | -65.30 | |
| 275 | 4-(MeOC(=O)CH=$CHCH_2OC(=O)$) | $CH_2$ | -65.30 | |
| 299 | 4-(Ph-C≡$CCH_2OC(=O)$) | $CH_2$ | -65.30 | |
| 300 | 4-(N≡CCH(Me)OC(=O)) | $CH_2$ | | 392 (M+1) |
| 301 | 4-(4-CN-Ph-$CH_2OC(=O)$) | $CH_2$ | -65.30 | |
| 308 | 4-(EtOC(=O)) | $CH_2CH_2O$ | | 395 (M-1) |
| 339 | 4-(EtC≡$CCH_2OC(=O)$) | $CH_2$ | -65.30 | |
| 340 | 4-((1-Me-2-pyrrolidinyl)$CH_2OC(=O)$) | $CH_2$ | -65.30 | |
| 348 | 4-($(Me)_3$SiC≡$CCH_2OC(=O)$) | $CH_2$ | | 449 (M+1) |
| 349 | 4-(MeC(=O)$CH_2OC(=O)$) | $CH_2$ | | 395 (M+1) |
| 350 | 4-(MeON=C(Me)$CH_2OC(=O)$) | $CH_2$ | | 424 (M+1) |
| 359 | 4-N≡C | $CH_2CH_2CH_2$ | | 348 (M+1) |
| 360 | 3-Ph | $CH_2CH_2CH_2$ | | 399 (M+1) |
| 361 | 3-(4-Cl-Ph) | $CH_2CH_2CH_2$ | | 433 (M+1) |
| 362 | 4-(EtOC(=O)) | $CH_2OCH_2$ | | 397 (M+1) |
| 363 | 4-(HOC(=O)) | $CH_2CH_2CH_2$ | | 367 (M+1) |
| 364 | 4-($CH_2$=C(Me)$CH_2OC(=O)$) | $CH_2CH_2CH_2$ | | 422 (M+1) |
| 365 | 4-(*n*-PiOC(=O)) | $CH_2CH_2CH_2$ | | 409 (M+1) |
| 373 | 4-($CH_2$=$CHCH_2OC(=O)$) | $CH_2CH_2CH_2$ | | 407 (M+1) |
| 374 | 4-(CH≡$CCH_2OC(=O)$) | $CH_2CH_2CH_2$ | | 405 (M+1) |
| 376 | 4-(EtOC(=O)) | $CH_2O$ | | 383 (M+1) |

(continued)

| Cmpd. No. | $(R^2)_X$ | L | $^{19}F$ NMR | MS |
|---|---|---|---|---|
| 378 | 4-(EtOC(=O)$CH_2$NHC(=O)) | $CH_2$ | -65.30 | |
| 379 | 4-(N≡$CCH_2$N(Me)C(=O)) | $CH_2$ | | 391 (M+1) |
| 380 | 4-(EtONHC(=O)) | $CH_2$ | -64.70[a] | |
| 381 | 4-$NH_2$ | $CH_2$ | | 310 (M+1) |
| 382 | 4-$NO_2$ | $CH_2$ | -65.40 | |
| 383 | 4-1 | $CH_2$ | -65.40 | |
| 384 | 4-(EtO-N=CH) | $CH_2$ | -65.35 | |
| 385 | 4-(*n*-PrO-N=CH) | $CH_2$ | -65.33 | |
| 386 | 4-($CH_2$=$CHCH_2$O-N=CH) | $CH_2$ | -65.35 | |
| 387 | 4-(CH≡$CCH_2$O-N=CH) | $CH_2$ | -65.34 | |
| 388 | 4-(*i*-PrO-N=CH) | $CH_2$ | -65.36 | |
| 389 | 4-($CH_3$C(=O)NHN=CH) | $CH_2$ | -65.35 | |
| 390 | 4-(MeS$(=O)_2$$CH_2CH_2$OC(=O)) | $CH_2$ | -65.30 | |
| 391 | 4-($(EtO)_2$$CHCH_2$C(=O)) | $CH_2$ | -65.30 | |
| 392 | 4-($F_2$$CHCH_2$O(=O)) | $CH_2$ | | 403 (M+1) |
| 406 | 4-($(Me)_2$NC(=O) | $CH_2CH_2CH_2$ | -65.37 | 394 (M+1) |
| 410 | 4-(n-BuOC(=O)) | $CH_2$ | -65.30 | |
| 411 | 4-(*c*-$PrCH_2$OC(=O)) | $CH_2$ | -65.30 | |
| 412 | 4-($PhCH_2$OC(=O)) | $CH_2$ | -65.30 | |
| 415 | 4-(MeNHC(=O)) | $CH_2CH_2CH_2$ | -65.37 | |
| 427 | 4-(MeOC(=O)) | $CH_2CH_2CH_2$ | | 381 (M+1) |
| 428 | 4-(EtOC(=O)) | $CH_2CH_2CH_2$ | | 395 (M+1) |
| 434 | 4-($MeSCH_2$C(=O)$CH_2$OC(=O)) | $CH_2$ | | 441 (M+1) |
| 435 | 4-CN | $CH_2CH_2$ | -65.35 | |
| 436 | 4-(MeOC(=O)) | $CH_2CH_2$ | -65.36 | |
| 437 | 4-(EtOC(=O)) | $CH_2CH_2$ | -65.38 | |
| 441 | 4-(MeC(=O)NH) | $CH_2$ | -65.40 | |
| 442 | 4-(MeS$(=O)_2$NH) | $CH_2$ | -65.40 | |
| 443 | 4-(EtOC(=O)NH) | $CH_2$ | -65.40 | |
| 447 | 4-($CH_3(CH_2)_4$OC(=O)) | $CH_2$ | -65.30 | |
| 448 | 4-($ClCH_2CH_2CH_2$O(=O)) | $CH_2$ | -65.30 | |
| 449 | 4-($CF_3CH_2$NHC(=O)) | $CH_2$ | | 420 (M+1) |
| 474 | 4-(MeO$(CH_2)_3$OC(=O)) | $CH_2$ | -65.30 | |
| 475 | 4-((4-morpliolinyl)$CH_2CH_2$OC(=O)) | $CH_2$ | -65.30 | |
| 476 | 4-(EtOC(=O)$CH_2$OC(=O)) | $CH_2$ | -65.30 | |
| 477 | 4-($ClCH_2CH_2$OC(=O)) | $CH_2$ | -65.30 | |
| 478 | 4-($BrCH_2CH_2$OC(=O)) | $CH_2$ | -85.30 | |
| 479 | 4-((2-pyridinyl)$CH_2$OC(=O)) | $CH_2$ | -65.30 | |
| 480 | 4-($(Me)_2$$CHCH_2CH_2$OC(=O)) | $CH_2$ | -65.30 | |
| 481 | 4-(*sec*-BuOC(=O)) | $CH_2$ | -65.40 | |
| 482 | 4-($CH_2$=C(Cl)$CH_2$OC(=O)) | $CH_2$ | -65.30 | |
| 483 | 4-((3-pyridinyl)$CH_2$NHC(=O)) | $CH_2$ | -65.30 | |
| 484 | 4-($PhCH_2$NH(=O)) | $CH_2$ | -65.30 | |

(continued)

| Cmpd. No. | $(R^2)_X$ | L | $^{19}$F NMR | MS |
|---|---|---|---|---|
| 485 | 4-(PhNH(=O)) | $CH_2$ | -65.30 | |
| 486 | 4-($CH_2$=C(CN)$CH_2$OC(=O)) | $CH_2$ | | 404 (M+1) |
| 494 | 4-(MeON=C($CH_2$Cl)$CH_2$OC(=O)) | $CH_2$ | | 458 (M+1) |
| 495 | 4-(Cl$CH_2$C(=O)$CH_2$OC(=O)) | $CH_2$ | | 429 (M+1) |
| 496 | 4-[(2,2-dimetliyl-1,3-dioxolan-4-yl) $CH_2$OC(=O)] | $CH_2$ | | 453 (M+1) |
| 503 | 3-($F_5$S) | $CH_2$ | -65.31, -72.69 (d), -84.24 (quint) | |
| 504 | 5-($F_5$S) | $CH_2$ | -65.33, -63.66 (d), -80.27 (quint) | |
| 508 | 4-(EtNHC(=O)) | $CH_2OCH_2$ | | 396 (M+1) |

a. $^{19}$F NMR in DMSO-$d_6$ solution.

INDEX TABLE B

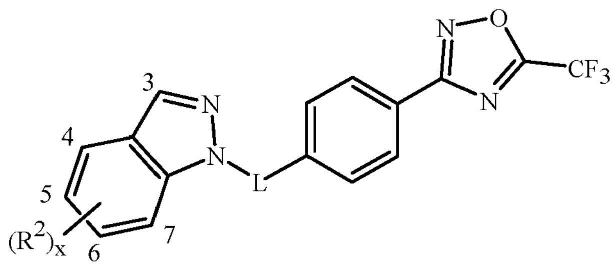

A dash "-" in the $(R^2)_X$ column means that no $R^2$ substituent is present and the remaining carbon valences are occupied by hydrogen atoms.

| Cmpd. No. | $(R^2)_X$ | L | $^{19}$F NMR | MS |
|---|---|---|---|---|
| 30 | 3-Br, 5-$NO_2$ | $CH_2$ | -65.40 | |
| 32 | 5-Br, 6-Me | $CH_2$ | -65.42 | |
| 34 | 3-Me, 4-Br | $CH_2$ | -65.41 | |
| 39 | 4-I, 6-Cl | $CH_2$ | -65.41 | |
| 41 | 6-(MeOC(=O)) | $CH_2$ | -65.42 | |
| 43 | 3-(MeON(Me)C(=O)) | $CH_2$ | -65.41 | |
| 44 | 3-(N≡C$CH_2$NHC(=O)) | $CH_2$ | -65.40 | |
| 45 | 3-(MeNHC(=O)) | $CH_2$ | -65.41 | |
| 46 | 3-(MeO$CH_2CH_2$NHC (=O)) | $CH_2$ | -65.41 | |
| 47 | 7-(MeOC(=O)) | $CH_2$ | -65.45 | |
| 48 | 5-(MeOC(=O)) | $CH_2$ | | 403 (M+1) |
| 49 (Ex. 4) | 4-(MeOC(=O)) | $CH_2$ | -65.42 | |
| 51 | 3-Me, 5-Br | $CH_2$ | -65.41 | |
| 53 | 4-Cl, 6-Br | $CH_2$ | -65.40 | |
| 94 | 5-$NO_2$ | $CH_2$ | -65.39 | |
| 99 | 3-Me | $CH_2$ | -65.43 | |

(continued)

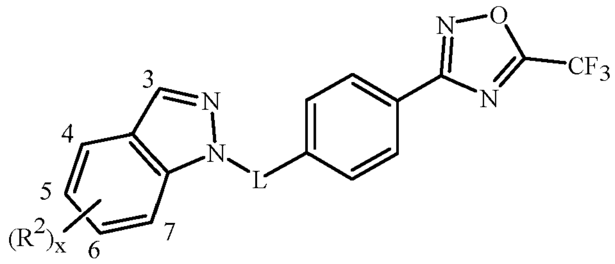

| Cmpd. No. | $(R^2)_x$ | L | $^{19}$F NMR | MS |
|---|---|---|---|---|
| A dash "-" in the $(R^2)_X$ column means that no $R^2$ substituent is present and the remaining carbon valences are occupied by hydrogen atoms. | | | | |
| 100 | 4-Me | $CH_2$ | -65.42 | |
| 101 | 7-Me | $CH_2$ | -65.43 | |
| 104 | 3-N≡C | $CH_2$ | -65.40 | |
| 105 | 4-N≡C | $CH_2$ | -65.41 | |
| 106 | 5-N≡C | $CH_2$ | -65.41 | |
| 112 | 3-Br | $CH_2$ | -65.41 | |
| 115 | 5-$NH_2$ | $CH_2$ | | 360 (M+1) |
| 116 | 3-Cl | $CH_2$ | -65.41 | |
| 117 | 3-(EtOC(=O)) | $CH_2$ | -65.42 | |
| 119 | 5-Cl | $CH_2$ | -65.42 | |
| 120 | 3-CN, 6-MeO | $CH_2$ | -65.40 | |
| 125 | 4-F | $CH_2$ | -65.42 | |
| 126 | 5-MeO | $CH_2$ | -65.42 | |
| 128 | 5-Br | $CH_2$ | -65.41 | |
| 134 | 3-(MeOC(=O)) | $CH_2$ | -65.41 | |
| 143 | - | $CH_2$ | | 345 (M+1) |
| 148 | 3-($Me_2$NC(=O)) | $CH_2$ | -65.41 | |
| 341 | 4-(MeOC(=O)) | $CH_2$ | -65.40 | |

INDEX TABLE C

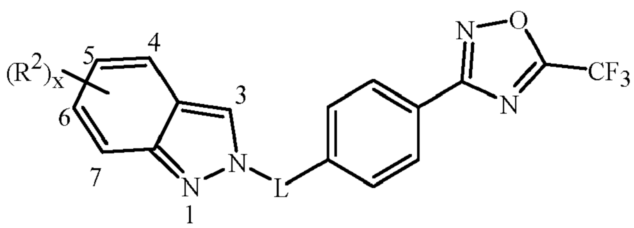

| Cmpd. No. | $(R^2)_x$ | L | $^{19}$F NMR |
|---|---|---|---|
| 33 | 5-Br, 6-Me | $CH_2$ | -65.39 |
| 35 | 5-(MeOC(=O)) | $CH_2$ | -65.39 |
| 36 (Ex. 4) | 4-(MeOC(=O)) | $CH_2$ | -65.39 |
| 37 | 3-Me, 4-Br | $CH_2$ | -65.39 |
| 38 | 4-I, 6-Cl | $CH_2$ | -65.38 |
| 42 | 6-(MeOC(=O)) | $CH_2$ | -65.39 |

(continued)

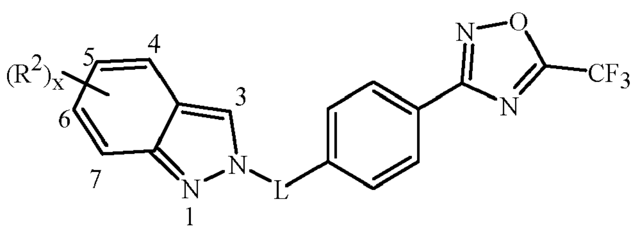

| Cmpd. No. | $(R^2)_x$ | L | $^{19}$F NMR |
|---|---|---|---|
| 50 | 7-(MeOC(=O)) | $CH_2$ | -65.42 |
| 52 | 3-Me, 5-Br | $CH_2$ | -65.40 |
| 54 | 4-Cl, 6-Br | $CH_2$ | -65.38 |
| 102 | 4-Me | $CH_2$ | -65.39 |
| 103 | 7-Me | $CH_2$ | -65.40 |
| 107 | 4-N≡C | $CH_2$ | -65.39 |
| 108 | 5-N≡C | $CH_2$ | -65.38 |
| 113 | 3-Br | $CH_2$ | -65.40 |
| 121 | 3-N≡C, 6-MeO | $CH_2$ | -65.39 |
| 123 | 3-(EtOC(=O)) | $CH_2$ | -65.44 |
| 130 | 4-F | $CH_2$ | -65.39 |
| 131 | 5-MeO | $CH_2$ | -65.39 |
| 133 | 5-Br | $CH_2$ | -65.39 |
| 141 | 3-(MeOC(=O)) | $CH_2$ | -65.41 |

INDEX TABLE D

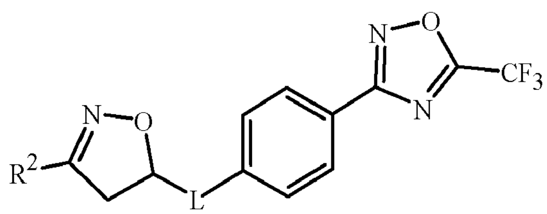

| Cmpd. No. | $R^2$ | L | $^{19}$F NMR |
|---|---|---|---|
| 12 (Ex. 13) | $Me_2NC(=O)$ | $CH_2$ | -65.36 |
| 29 | EtOC(=O) | $CH_2$ | -65.36 |
| 164 | $NH_2C(=O)$ | $CH_2$ | -65.37 |
| 165 | MeNHC(=O) | $CH_2$ | -65.36 |
| 175 | EtNHC(=O) | $CH_2$ | -65.36 |
| 176 | $CH{\equiv}CCH_2NHC(=O)$ | $CH_2$ | -65.34 |
| 177 | 1-azetidinyl-C(=O) | $CH_2$ | -65.36 |

INDEX TABLE E

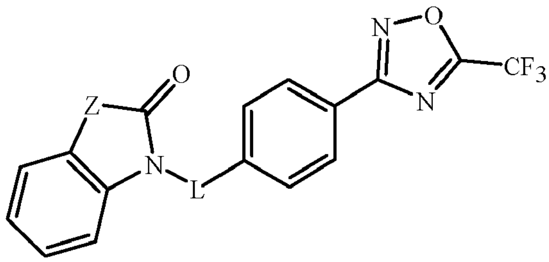

| Cmpd. No. | Z | L | $^{19}F$ NMR | MS |
|---|---|---|---|---|
| 87 | C=O | $CH_2$ | -65.38 | |
| 118 | O | $CH_2$ | -65.41 | |
| 156 | S | $CH_2$ | | 378 (M+1) |
| 159 (Ex. 7) | N-Me | $CH_2$ | | 375 (M+1) |
| 161 | $CF_2$ | $CH_2$ | -65.41 | |

INDEX TABLE F

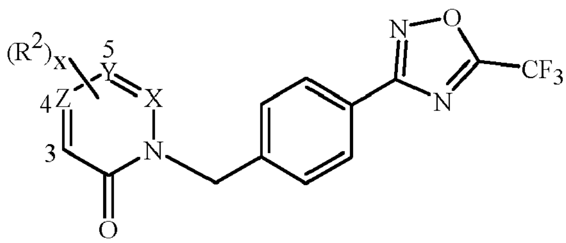

| Cmpd. No. | X | Y | Z | $(R^2)_x$ | $^{19}F$ NMR | MS |
|---|---|---|---|---|---|---|
| 3 | N | CH | C | 4-Cl | | 357 (M+1) |
| 4 (Ex. 3) | N | C | CH | 5-Cl | | 357 (M+1) |
| 5 | N | C | C | 4,5-di-Cl | | 391 (M+1) |
| 27 | N | C | C | 4-MeO, 5-Cl | | 387 (M+1) |
| 28 | N | CH | C | 4-MeO | | 353 (M+1) |
| Cmpd. No. | X | Y | Z | $(R^2)_x$ | $^{19}F$ NMR | MS |
| 89 | CH | CH | C | 4-CN | -65.35 | |
| 417 | CH | CH | CH | 3-CN | -65.30 | |
| 418 | CH | N | CH | 3-(EtOC(=O)) | -65.30 | |
| 419 | CH | C | N | 5-(EtOC(=O)) | -65.30 | |
| 420 | CH | C | CH | 5-(MeOC(=O)) | -65.30 | |
| 421 | CH | CH | C | 4-MeO | -65.30 | |
| 422 | CH | CH | CH | 3-(MeOC(=O)) | -65.30 | |
| 423 | CH | CH | N | 3-(MeOC(=O)) | | 381 (M+1) |

INDEX TABLE G

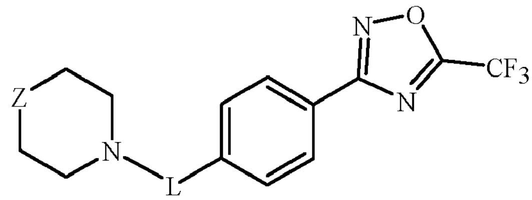

| Cmpd. No. | Z | L | $^{19}F$ NMR |
|---|---|---|---|
| 8 | (N≡C)CH | $CH_2$ | -65.44 |
| 14 | O | CH(C≡N) | -65.32 |
| 96 | O | $CH_2$ | -65.36 |
| 150 | C=O | $CH_2$ | -65.36 |

INDEX TABLE H

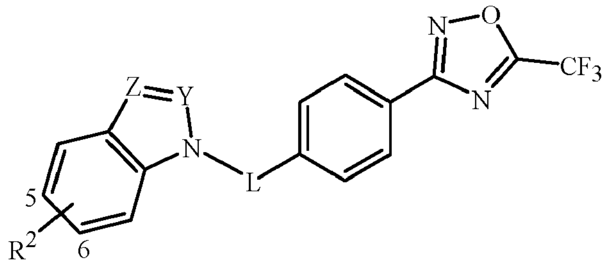

A dash "-" in the $R^2$ column means that no $R^2$ substituent is present and the remaining carbon valences are occupied by hydrogen atoms.

| Cmpd. No. | Z | Y | $R^2$ | L | $^{19}F$ NMR | MS |
|---|---|---|---|---|---|---|
| 22 | N | N | 5-(MeOC (=O)) | $CH_2$ | | 404 (M+1) |
| 88 | CH | CH | 6-CN | $CH_2$ | -65.38 | |
| 91 | N | CH | - | $CH_2$ | -65.38 | |
| Cmpd. No. | Z | Y | $R^2$ | L | $^{19}F$ NMR | MS |
| 92 | N | N | - | $CH_2$ | -65.39 | |

INDEX TABLE I

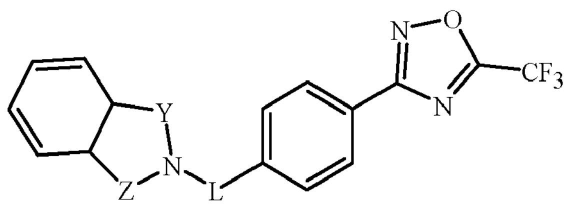

| Cmpd. No. | Z | Y | L | $^{19}F$ NMR |
|---|---|---|---|---|
| 11 | C(=O) | $S(=O)_2$ | $CH_2$ | -65.41 |
| 90 (Ex. 9) | C(=O) | C(=O) | $CH_2$ | -65.39 |
| 155 | S | C(=O) | $CH_2$ | -65.40 |
| 160 | $CH_2$ | $CH_2$ | $CH_2$ | -65.41 |

INDEX TABLE J

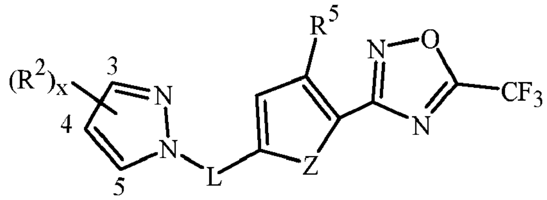

| Cmpd. No. | $R^2$ | L | Z | $R^5$ | $^{19}F$ NMR | MS | m.p. (°C) |
|---|---|---|---|---|---|---|---|
| 70 | 4-(EtOC(=O)) | $CH_2$ | S | H | -65.39 | | |
| 71 (Ex. 11) | 4-Br | $CH_2$ | S | H | -65.41 | | |
| 72 | 3-Br | $CH_2$ | S | H | -65.39 | | |
| 73 | 3-(EtOC(=O)), 5-Me | $CH_2$ | S | H | -65.44 | | |
| 76 | 3-CN, 4-Br | $CH_2$ | S | H | -65.36 | | |
| 77 | 4-Br, 5-CN | $CH_2$ | S | H | -65.38 | | |
| 188 | 4-(MeOC(=O)) | $CH_2$ | S | H | -65.40 | 372(M+1) | |
| 189 | 4-CN | $CH_2$ | S | H | -65.41 | | |
| 190 | 4-($CH\equiv CCH_2OC(=O)$) | $CH_2$ | S | H | -65.39 | | |
| 192 | 4-(EtOC(=O)) | CH(Me) | S | H | -65.47 | | |
| 197 | 4-CH(=O) | $CH_2$ | S | H | -65.38 | 329(M+1) | |
| 198 | 4-(*n*-PiOC(=O)) | $CH_2$ | S | H | -65.39 | 387(M+1) | |
| 199 | 4-($CH_2=CHCH_2OC(=O)$) | $CH_2$ | S | H | -65.44 | 385 (M+1) | |
| Cmpd. No. | $R^2$ | L | Z | $R^5$ | $^{19}F$ NMR | MS | m.p. (°C) |
| 200 | 4-(*i*-PtOC(=O)) | $CH_2$ | S | H | -65.39 | 387 (M+1) | |
| 209 | 4-(HOC(=O)) | $CH_2$ | S | MeO | | | 141-152.3 |
| 210 | 4-(EtOC(=O)) | $CH_2$ | O | H | | | 65-80.9 |
| 211 | 4-(*n*-PiOC(=O)) | $CH_2$ | O | H | | | 53.5-64.1 |
| 246 | 4-(EtOC(=O)) | *CH(Me) | S | H | | 387 (M+1) | |
| 247 | 4-(EtOC(=O)) | **CH(Me) | S | H | | 387 (M+1) | |
| 248 | 4-(*n*-PiOC(=O)) | *CH(Me) | S | H | | 401 (M+1) | |
| 249 | 4-(*n*-PiOC(=O)) | **CH(Me) | S | H | | 401 (M+1) | |
| 491 | 4-($CH\equiv CCH_2NHC(=O)$) | $CH_2$ | S | H | | 371 (M+1) | |
| 493 | 4-(*n*-PiOC(=O)) | CH(Me) | S | H | | | |
| 336 | 4-(EtOC(=O)) | $CH_2$ | S | MeO | | | 126.2-128.3 |
| 439 | 4-(*n*-PiOC(=O)) | $CH_2$ | S | MeO | | 417 (M+H) | |
| 440 | 4-($CH_2=CHCH_2OC(=O)$) | $CH_2$ | S | MeO | | 415 (M+H) | |

*S*-isomer at the carbon atom denoted by the asterisk "*" in the table above.
**R*-isomer at the carbon atom denoted by the double asterisk "**" in the table above.

INDEX TABLE K

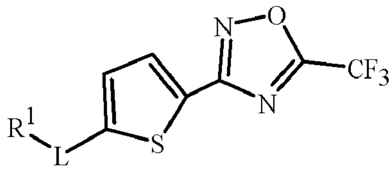

In the L column, the atom to the right is connected to the thienyl ring and the atom to the left is connected to R[1].

| Cmpd. No. | $R^1$ | L | $^{19}F$ NMR | MS | m.p. (°C) |
|---|---|---|---|---|---|
| 191 | *c*-Pr | $NHCH_2$ | -65.46 | | |
| 194 | 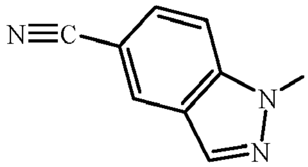 | $CH_2$ | -65.41 | | |
| 195 | 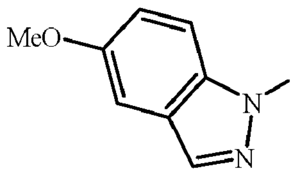 | $CH_2$ | -65.41 | | |
| 196 | 1H-indol-1-yl | $CH_2$ | -65.46 | 350 (M+1) | |
| 201 | 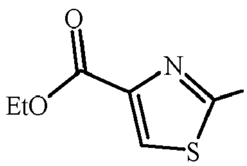 | $CH_2$ | | | 80-84 |
| 202 | 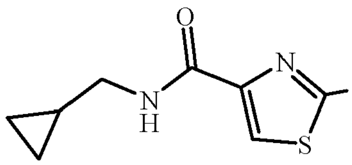 | $CH_2$ | | 415 (M+1) | |
| 323 | tetrahydro-1,1-dioxido-2H-1,2-thiazin-2-yl | $CH_2$ | | 368 (M+1) | |
| 324 | 1,1-dioxido-2-isothiazolidinyl | $CH_2$ | | 376 (M+23) | |
| 325 | 3-oxo-4-morpholinyl | $CH_2$ | | 334 (M+1) | |
| 375 | 2-Me-5-F-Ph | $OCH_2$ | -65.46 | | |
| 438 | 2-Me-Ph | $OCH_2$ | -66.04 | | |

INDEX TABLE L

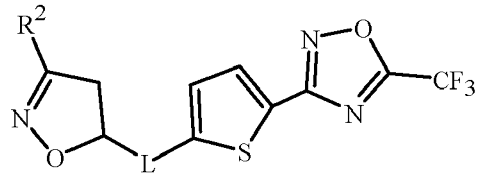

| Cmpd. No. | $R^1$ | L | MS | m.p. (°C) |
|---|---|---|---|---|
| 366 | EtOC(=O) | $CH_2$ | | 88.7-93.2 |
| 367 | HOC(=O) | $CH_2$ | | 111.5-125.6 |

(continued)

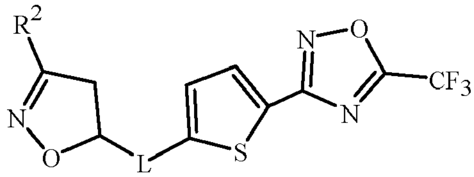

| Cmpd. No. | $R^1$ | L | MS | m.p. (°C) |
|---|---|---|---|---|
| 368 | MeNHC(=O) | $CH_2$ | | 95.3-120.3 |
| 369 | EtNHC(=O) | $CH_2$ | | 145-147 |
| 429 | $CH{\equiv}CCH_2NHC(=O)$ | $CH_2$ | | 113.5-119 |
| 430 | $(Et)_2NC(=O)$ | $CH_2$ | 403 (M+1) | |
| 460 | $(Me)_2NC(=O)$ | $CH_2$ | 375 (M+1) | |
| 461 | $MeOCH_2CH_2NHC(=O)$ | $CH_2$ | | 55.2-78.1 |

INDEX TABLE M

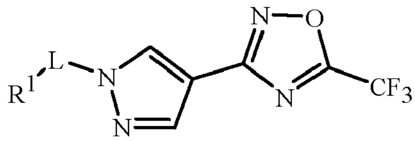

| Cmpd. No. | $R^1$ | L | $^{19}F$ NMR |
|---|---|---|---|
| 305 | 4-(EtOC(=O))-1*H*-pyrazol-1-yl | $CH_2$ | -65.39 |
| 306 | 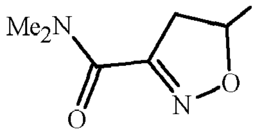 | $CH_2$ | -65.38 |
| 307 | 4-(EtOC(=O))-1*H*-pyrazol-1-yl | $CH_2CH_2$ | -65.44 |
| 310 | 4-(MeOC(=O))-Ph | $CH_2CH_2$ | -65.43 |
| 311 | 4-(MeOC(=O))-Ph | $CH_2$ | -65.43 |
| 326 | 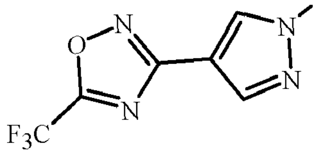 | $CH_2CH_2CH_2$ | -65.43 |

INDEX TABLE N

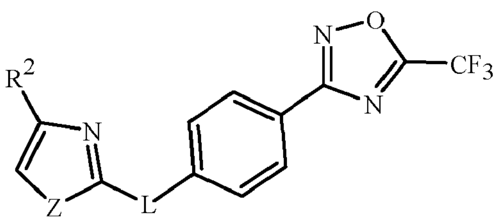

In the L column, the atom to the right is connected to the phenyl ring and the atom to the left is connected to the $R^1$ ring.

| Cmpd No. | $R^2$ | Z | L | m.p. (°C) | MS |
|---|---|---|---|---|---|
| 203 | $(N{\equiv}C)_2CHNHC(=O)$ | O | $CH_2$ | | 403 (M+1) |
| 204 | $ClCH_2CH_2NHC(=O)$ | O | $CH_2$ | | 401 (M+1) |

(continued)

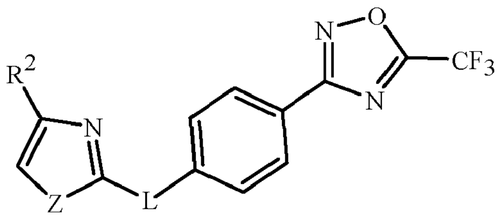

In the L column, the atom to the right is connected to the phenyl ring and the atom to the left is connected to the $R^1$ ring.

| Cmpd No. | $R^2$ | Z | L | m.p. (°C) | MS |
|---|---|---|---|---|---|
| 205 | 3,3-difluoro-1-piperidinyl-C(=O) | O | $CH_2$ | | 444 (M+1) |
| 206 | $(Me)_3SiCH_2CH_2NHC(=O)$ | O | $CH_2$ | | 439 (M+1) |
| 207 | 3,3-difluoro-1-pyrrolidinyl-C(=O) | S | $CH_2$ | | 445 (M+1) |
| 208 | $Cl_2C=CHCH_2NHC(=O)$ | S | $CH_2$ | | 463 (M+1) |
| 225 | $F_2CHCH_2NHC(=O)$ | O | $CH_2$ | | 403 (M+1) |
| 226 | 2,2-difluorocyclopropyl-$CH_2NHC(=O)$ | O | $CH_2$ | | 429 (M+1) |
| 227 | 1-pyrrolidinyl-C(=O) | O | $CH_2$ | | 393 (M+1) |
| 228 | 4,4-difluoro-1-piperidinyl-C(=O) | O | $CH_2$ | | 443 (M+1) |
| 229 | $N{\equiv}CCH_2CH_2NHC(=O)$ | O | $CH_2$ | | 392 (M+1) |
| 230 | $N{\equiv}CC(Me)_2NHC(=O)$ | O | $CH_2$ | | 406 (M+1) |
| 231 | 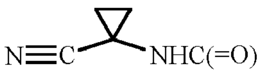 | O | $CH_2$ | | 404 (M+1) |

| Cmpd No. | $R^2$ | Z | L | m.p. (°C) | MS |
|---|---|---|---|---|---|
| 252 | EtOC(=O) | O | $CH_2$ | | 368 (M+1) |
| 253 | HOC(=O) | O | $CH_2$ | 199-203 | 340 (M+1) |
| 254 | $NH_2C(=O)$ | O | $CH_2$ | | 339 (M+1) |
| 256 | $N{\equiv}C$ | O | $CH_2$ | | 319 (M+1) |
| 257 | $MeOCH_2CH_2NHC(=O)$ | O | $CH_2$ | | 397 (M+1) |
| 258 | $N{\equiv}CCH_2NHC(=O)$ | O | $CH_2$ | | 378 (M+1) |
| 259 | *i*-PrNHC(=O)) | O | $CH_2$ | | 381 (M+1) |
| 260 | *c*-PrNHC(=O) | O | $CH_2$ | | 379 (M+1) |
| 261 | 1-azetidinyl-C(=O) | O | $CH_2$ | | 379 (M+1) |
| 262 | $Et_2NC(=O)$ | O | $CH_2$ | | 395 (M+1) |
| 263 | 4-morpholinyl-C(=O) | O | $CH_2$ | | 409 (M+1) |
| 264 | 1*H*-pyrazol-1-yl-$CH_2CH_2NHC(=O)$ | O | $CH_2$ | 125-126 | 433 (M+1) |
| 265 | 1-methyl-1*H*-pyrazol-3-yl-$CH_2NHC(=O)$ | O | $CH_2$ | | 433 (M+1) |
| 277 | 2-thiazolyl-$CH_2NHC(=O)$ | O | $CH_2$ | | 436 (M+1) |
| 278 | $(MeO)_2CHCH_2NHC(=O)$ | O | $CH_2$ | | 427 (M+1) |
| 279 | $(MeOCH_2)_2CHNHC(=O)$ | O | $CH_2$ | | 441 (M+1) |
| 280 | EtNHC(=O) | O | $CH_2$ | | 367 (M+1) |
| 315 | $(Me)_2NC(=O)$ | O | $CH_2$ | | 367 (M+1) |
| 316 | $F_3CCH_2NHC(=O)$ | O | $CH_2$ | 164-168 | 421 (M+1) |
| 327 | *c*-$PrCH_2NHC(=O)$ | O | $CH_2$ | | 393 (M+1) |
| 328 | $CF_3CF_2CH_2NHC(=O)$ | O | $CH_2$ | | 471 (M+1) |
| 329 | 3-thietanyl-NHC(=O) | O | $CH_2$ | | 411 (M+1) |
| 330 | $CF_3CH_2CH_2NHC(=O)$ | O | $CH_2$ | 104-105 | 435 (M+1) |

(continued)

| Cmpd No. | $R^2$ | Z | L | m.p. (°C) | MS |
|---|---|---|---|---|---|
| 331 | $CF_3C(Me)_2NHC(=O)$ | O | $CH_2$ | | 449 (M+1) |
| 332 | tetrahydro-2-oxo-3-furanyl-NHC(=O)) | O | $CH_2$ | | 423 (M+1) |
| 334 | $CH_2=CHCH_2OC(=O)$ | O | $CH_2$ | | 380 (M+1) |
| 335 | *i*-BuOC(=O) | O | $CH_2$ | | 396 (M+1) |
| 342 | c-PrNHC(=O) | S | $CH_2$ | 112-116 | |
| 343 | $H_2NC(=O)$ | S | $CH_2$ | 195-199 | |
| 344 | EtOC(=O) | S | $CH_2$ | 109-113 | |
| 345 | HOC(=O) | S | $CH_2$ | 200-204 | |
| 370 | N≡C | S | $CH_2$ | 97-101 | |
| 393 | $N{\equiv}CCH_2NHC(=O)$ | S | $CH_2$ | 143-147 | |
| 394 | $MeOCH_2CH_2NHC(=O)$ | S | $CH_2$ | 83-87 | |
| 395 | 4,4-difluorocycloliexyl-NHC(=O) | O | $CH_2$ | | 457 (M+1) |
| 396 | 1*H*-pyrazol-1-yl-$CH_2CH_2NHC(=O)$ | S | $CH_2$ | | 449 (M+1) |
| 397 | $CF_3CH_2CH_2NHC(=O)$ | S | $CH_2$ | | 451 (M+1) |
| 398 | $CF_3(CH_2)_3NHC(=O)$ | O | $CH_2$ | | 449 (M+1) |
| 399 | $Cl_2C=CHCH_2NHC(=O)$ | O | $CH_2$ | | 447 (M+1) |
| 400 | $FCH_2CH_2NHC(=O)$ | O | $CH_2$ | | 385 (M+1) |
| 401 | $CF_3OCH_2CH_2NHC(=O)$ | O | $CH_2$ | | 451 (M+1) |
| 402 | 3,3-di-F-pyrolidin-1-yl-C(=O) | O | $CH_2$ | | 429 (M+1) |
| 403 (Ex. 18) | MeOC(=O) | O | $CH_2O$ | | 370 (M+1) |
| 404 | EtOC(=O) | S | $CH_2O$ | | 400 (M+1) |
| 463 | $CF_3CH_2NHC(=O)$ | S | $CH_2$ | | 437 (M+1) |
| 464 | $MeOCH_2CH_2NHC(=O)$ | O | $CH_2O$ | | 413 (M+1) |
| 465 | $CF_3CH_2NHC(=O)$ | O | $CH_2O$ | | 437 (M+1) |
| 466 | 1*H*-pyrazol-1-yl-$CH_2CH_2NHC(=O)$ | O | $CH_2O$ | | 449 (M+1) |
| 467 | $CF_3CH_2CH_2NHC(=O)$ | O | $CH_2O$ | | 451 (M+1) |
| 468 | $N{\equiv}CCH_2NHC(=O)$ | O | $CH_2O$ | | 394 (M+1) |
| 469 | $MeOCH_2CH_2NHC(=O)$ | S | $CH_2O$ | | 429 (M+1) |
| 470 | $CF_3CH_2NHC(=O)$ | S | $CH_2O$ | | 453 (M+1) |
| 471 | 1*H*-pyrazol-1-yl-$CH_2CH_2NHC(=O)$ | S | $CH_2O$ | | 465 (M+1) |
| 472 | $CF_3CH_2CH_2NHC(=O)$ | S | $CH_2O$ | | 467 (M+1) |
| 473 | $N{\equiv}CCH_2NHC(=O)$ | S | $CH_2O$ | | 410 (M+1) |
| 487 | 4-morpholinyl-C(=O) | S | $CH_2$ | 86-90 | |
| 488 | EtNHC(=O) | S | $CH_2$ | 71-75 | |
| 489 | $(MeOCH_2)_2CHNHC(=O)$ | S | $CH_2$ | | 457 (M+1) |
| 490 | MeOC(=O) | O | $CH_2$ | | 354 (M+1) |
| 505 | $F_2CHCH_2NHC(=O)$ | S | $CH_2$ | 83-92 | 419 (M+1) |

INDEX TABLE O

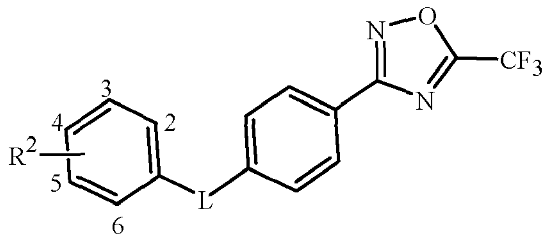

A dash "-" in the $R^2$ column means that no $R^2$ substituent is present and the remaining carbon valences are occupied by hydrogen atoms. In the L column, the atom to the right is connected to J (phenyl ring) and the atom to the left is connected to the $R^1$ phenyl ring bearing $R^2$.

| Cmpd No. | $R^2$ | L | $^{19}F$ NMR | MS |
|---|---|---|---|---|
| 13 (Ex. 14) | - | NHCH (C≡N) | -65.31 | |
| 15 | - | N(Ac) CH (C≡N) | -65.34 | |
| 281 | 4-($CF_3CH_2NHC(=O)$) | $CH_2$ | | 430 (M+1) |
| 282 | 4-($MeOCH_2CH_2NHC(=O)$) | $CH_2$ | | 406 (M+1) |
| 283 | 4-($N{\equiv}CCH_2NHC(=O)$) | $CH_2$ | | 387 (M+1) |
| 284 | 3-($MeOCH_2CH_2NHC(=O)$) | $CH_2$ | | 406 (M+1) |
| 285 | 3-($N{\equiv}CCH_2NHC(=O)$) | $CH_2$ | | 387 (M+1) |
| 309 | 4-MeO | CH (OH) | -65.37 | |
| 354 | 4-(MeOC(=O)) | $CH_2O$ | -65.41 | |
| 451 | - | $CH_2$ | -65.37 | |
| 453 | 4-(EtOC(=O)) | $CH_2$ | | 376 (M-1) |
| 454 | 3-(EtOC(=O)) | $CH_2$ | | 376 (M-1) |
| 497 | 2-Me | CH (OH) | | 333 (M-1) |
| 498 | 3-F | CH (OH) | | 337 (M-1) |
| 499 | 3-Cl | CH (OH) | | 353 (M-1) |
| 500 | 4-F | CH (OH) | | 337 (M-1) |
| 501 | 4-Me | CH (OH) | | 333 (M-1) |

INDEX TABLE P

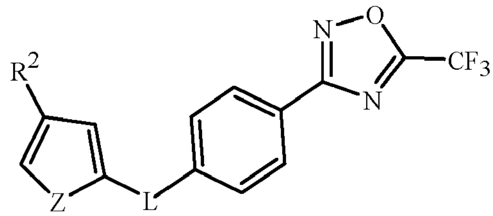

| Cmpd No. | Z | $R^2$ | L | MS |
|---|---|---|---|---|
| 286 | S | $CF_3CH_2NHC(=O)$ | $CH_2$ | 436 (M+1) |
| 287 | S | $MeOCH_2CH_2NHC(=O)$ | $CH_2$ | 412 (M+1) |
| 288 | S | $N{\equiv}CCH_2NHC(=O)$ | $CH_2$ | 393 (M+1) |
| 289 | S | 1*H*-pyrazol-1-yl-$CH_2CH_2NHC(=O))$ | $CH_2$ | 448 (M+1) |
| 290 | S | *c*-$PrCH_2NHC(=O)$ | $CH_2$ | 408 (M+1) |
| 292 | O | $MeOCH_2CH_2NHC(=O)$ | $CH_2$ | 396 (M+1) |
| Cmpd No. | Z | $R^2$ | L | MS |
| 293 | O | 1*H*-pyrazol-1-yl-$CH_2CH_2NHC(=O))$ | $CH_2$ | 432 (M+1) |
| 294 | O | $N{\equiv}CCH_2NHC(=O)$ | $CH_2$ | 377 (M+1) |
| 291 | O | $CF_3CH_2NHC(=O)$ | $CH_2$ | 420 (M-1) |
| 455 | S | MeOC(=O) | $CH_2$ | 368 (M-1) |
| 456 | O | MeOC(=O) | $CH_2$ | 352 (M-1) |

INDEX TABLE Q

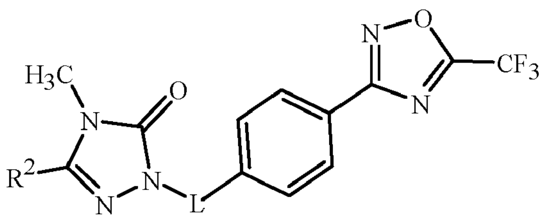

| Cmpd No. | $R^2$ | L | $^{19}F$ NMR | MS |
|---|---|---|---|---|
| 235 | Et | $CH_2$ | -65.36 | 354 (M+1) |
| 236 | *c*-Pr | $CH_2$ | -65.36 | 366 (M+1) |
| 237 | *i*-Pr | $CH_2$ | -65.36 | 368 (M+1) |
| 238 | *n*-Pr | $CH_2$ | -65.36 | 368 (M+1) |
| 239 | $CF_3$ | $CH_2CH_2$ | -65.37, -66.17 | 408 (M+1) |
| 240 | $CF_3$ | $CH_2CH_2CH_2$ | -65.37, -66.17 | 422 (M+1) |
| 267 | $CF_3$ | $CH_2$ | -65.37, -66.17 | 394 (M+1) |

INDEX TABLE R

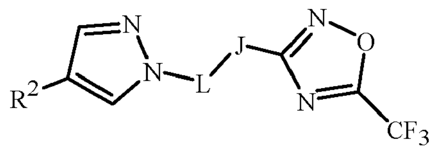

In the J column the bond to the left is connected to L and the bond to the right is connected to the oxadiazolyl ring.

| Cmpd No. | $R^2$ | L | J | $^{19}F$ NMR | MS | m.p. (°C) |
|---|---|---|---|---|---|---|
| 193 | EtOC(=O) | $CH_2$ | | -65.38 | | |
| 234 | EtOC(=O) | $CH_2$ | | -65.19 | | |
| 250 | *n*-PrOC(=O) | $CH_2$ | | -65.41 | | |
| 251 | *i*-PtOC(=O) | $CH_2$ | | -65.41 | | |
| 271 | EtOC(=O) | $CH_2$ | | | 371 (M+1) | |
| 304 | EtOC(=O) | $CH_2$ | | | 385 (M+1) | |
| 346 | EtOC(=O) | $CH_2$ | | | 385 (M+1) | |
| 347 | EtOC(=O) | $CH_2CH_2$ | | | 399 (M+1) | |
| 358 | EtOC(=O) | $CH_2CH_2CH_2$ | | | 413 (M+1) | |
| 377 | HOC(=O) | $CH_2$ | | -65.38 | | |
| 416 | *n*-PrOC(=O) | $CH_2$ | | | | 100.7-130 |
| 424 | EtOC(=O) | $CH_2$ | | -65.30 | | |
| 457 | EtOC(=O) | $CH_2$ | | | 417 (M+1) | |
| 458 | EtOC(=O) | $CH_2$ | | | 374 (M+1) | |
| Cmpd No. | $R^2$ | L | J | $^{19}F$ NMR | MS | m.p. (°C) |
| 459 | EtOC(=O) | $CH_2CH_2$ | | | 388 (M+1) | |

INDEX TABLE S

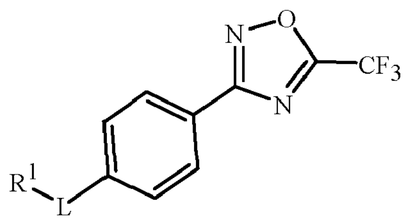

In the L column, the atom to the right is connected to the phenyl ring and the atom to the left is connected to $R^1$.

| Cmpd No. | $R^1$ | L | $^{19}F$ NMR | MS | m.p. (°C) |
|---|---|---|---|---|---|
| 9 | 3-cyano-1*H*-1,2,4-triazol-1-yl | $CH_2$ | -65.38 | | |
| 10 | 3-cyano-4*H*-1,2,4-triazol-4-yl | $CH_2$ | -65.39 | | |
| 16 | 4-(MeOC(=O))-1*H*-1,2,3-triazol-1-yl | $CH_2$ | -65.35 | | |
| 23 | 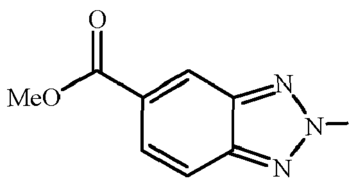 | $CH_2$ | | 404 (M+1) | |

(continued)

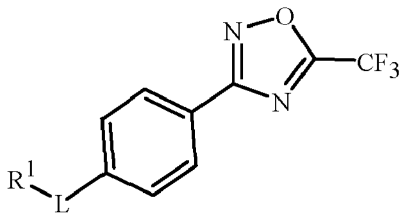

In the L column, the atom to the right is connected to the phenyl ring and the atom to the left is connected to $R^1$.

| Cmpd No. | $R^1$ | L | $^{19}F$ NMR | MS | m.p. (°C) |
|---|---|---|---|---|---|
| 24 (Ex. 8) | 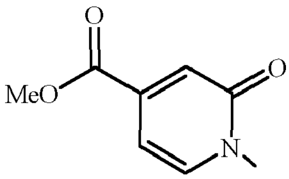 | $CH_2$ | -65.39 | | |
| 31 | 4-Me-5-MeS-2*H*-1,2,3-triazol-2-yl | $CH_2$ | -65.40 | | |
| 40 | 4-MeS-5-Me-1*H*-1,2,3-triazol-1-yl | $CH_2$ | -65.39 | | |
| 74 (Ex. 15) | 4-(MeOC(=O))-4,5-dihydro-2-oxazolyl | $CH_2$ | -65.33 | 356 (M+1) | 79-81 |
| 75 (Ex. 17) | 4-($NH_2$C(=O))-4,5-dihydro-2-oxazolyl | $CH_2$ | -65.32 | 341 (M+1) | |
| 84 | 5-Br-4-(EtOC(=O))-2*H*-1,2,3-triazol-2-yl | $CH_2$ | -70.14 | | |
| 86 | 1*H*-pyrrolo[2,3-b]pyridin-1-yl | $CH_2$ | -65.40 | | |
| 93 | 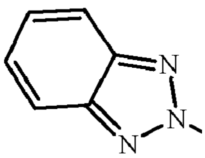 | $CH_2$ | -65.38 | | |
| 124 | 3-(EtOC(=O))-1-pyrrolidinyl | $CH_2$ | -65.46 | | |
| Cmpd No. | $R^1$ | L | $^{19}F$ NMR | MS | m.p. (°C) |
| 129 | 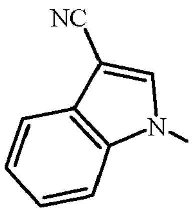 | $CH_2$ | -65.39 | | |
| 135 | 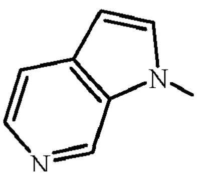 | $CH_2$ | -65.40 | | |
| 136 (Ex. 5) | 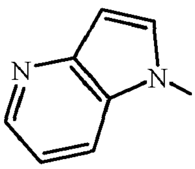 | $CH_2$ | -65.40 | | |

(continued)

| Cmpd No. | $R^1$ | L | $^{19}$F NMR | MS | m.p. (°C) |
|---|---|---|---|---|---|
| 137 | 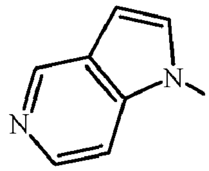 | $CH_2$ | -65.40 | | |
| 138 | 3-(MeOC(=O))-4*H*-1,2,4-triazol-4-yl | $CH_2$ | -65.41 | | |
| 139 | 3-(MeOC(=O))-1*H*-1,2,4,triazol-1-yl | $CH_2$ | -65.41 | | |
| 147 (Ex. 1) | 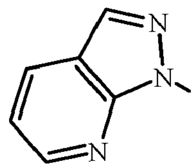 | $CH_2$ | -65.43 | | |
| 149 | 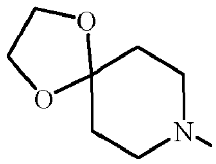 | $CH_2$ | -65.38 | | |
| 151 | 3-cyano-1-pyrrolidinyl | $CH_2$ | -65.41 | 323 (M+1) | |
| 152 | 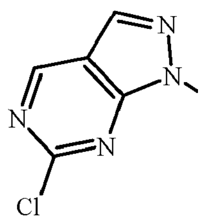 | $CH_2$ | -65.37 | 381 (M+1) | |
| 157 | 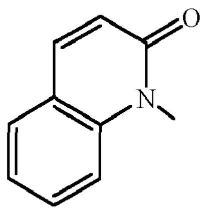 | $CH_2$ | -65.42 | | |
| 158 (Ex. 6) | 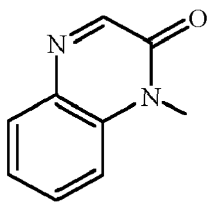 | $CH_2$ | -65.40 | | |
| 178 (Ex. 16) | 4-($Me_2NC(=O)$)-4,5-dihydro-2-oxazolyl | $CH_2$ | -65.36 | 370 (M+1) | |
| 255 | 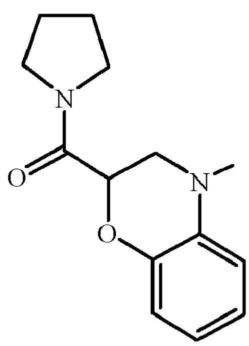 | $CH_2$ | | 473 (M+1) | |

(continued)

| Cmpd No. | $R^1$ | L | $^{19}F$ NMR | MS | m.p. (°C) |
|---|---|---|---|---|---|
| 297 | 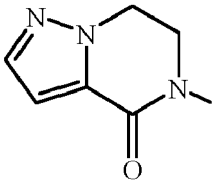 | $CH_2$ | | 364 (M+1) | |
| 298 | 2-Me-4-thiazolyl | $CH_2O$ | | 342 (M+1) | |
| 303 | 4-(EtOC(=O))-1*H*-1,2,3-triazol-1-yl | $CH_2$ | -65.30 | | |
| 312 | 3-(MeOC(=O))-5-isoxazolyl | $CH_2O$ | | 369 (M-1) | |
| 313 | 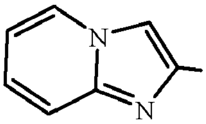 | $CH_2O$ | | 361 (M+1) | |
| 314 | 3-($Me_2$NC(=O))-5-isoxazolyl | $CH_2O$ | | 383 (M+1) | |
| 317 | 5-(MeOC(=O))-2-thienyl | $CH_2O$ | | 383 (M-1) | |
| 318 | 3-isoxazolyl | $CH_2O$ | | 312 (M+1) | |
| 319 | 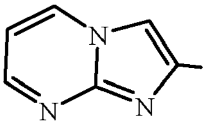 | $CH_2O$ | | 362 (M+1) | |
| 320 | 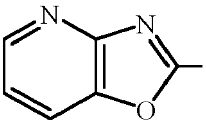 | $CH_2O$ | | 363 (M+1) | |
| 321 | tetrahydro-1,1-dioxido-2H-1,2-thiazin-2-yl | $CH_2$ | | 362 (M+1) | |
| 322 | 1,1-dioxido-2-isothiazolidinyl | $CH_2$ | | 370 (M+23) | |
| 337 | 1H-1,2,4-triazol-1-yl | $CH_2O$ | | 312 (M+1) | |
| 338 | 3,5-dimethyl-4-isoxazolyl | $CH_2O$ | | 340 (M+1) | |
| 351 | 3,4-dihydro-2(1*H*)-isoquinolinyl | $CH_2$ | -65.40 | | |
| 352 | 5-(EtNHC(=O))-2-thienyl | $CH_2O$ | | 398 (M+1) | |
| 353 | 3-pyridinyl | $CH_2O$ | | 322 (M+1) | |
| 355 | 2-benzoxazolyl | $CH_2O$ | | 362 (M+1) | |
| 356 | 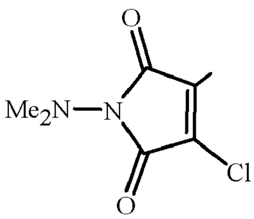 | O | -65.32 | | |
| 357 | 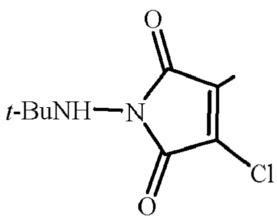 | O | -65.35 | | |
| 371 | 2-oxo-1-pyrrolidinyl | $CH_2$ | | 312 (M+1) | |
| 372 | 3-oxo-4-morpholinyl | $CH_2$ | | 327 (M+1) | |

(continued)

| Cmpd No. | $R^1$ | L | $^{19}F$ NMR | MS | m.p. (°C) |
|---|---|---|---|---|---|
| 407 | 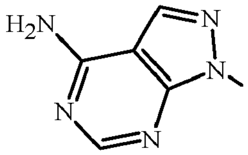 | $CH_2$ | -65.30 | | |
| 408 | 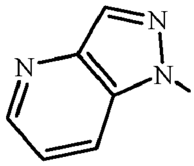 | $CH_2$ | -65.40 | | |
| 409 | 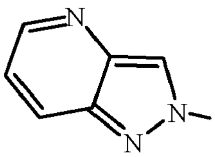 | $CH_2$ | | 346 (M+1) | |
| 414 | 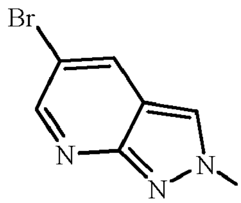 | $CH_2$ | -65.30 | | |
| 413 | 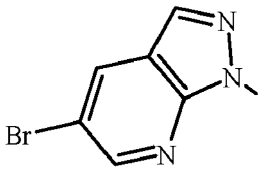 | $CH_2$ | -65.30 | | |
| 431 | 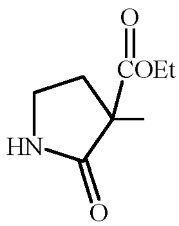 | $CH_2$ | | 384 (M+1) | |
| 432 | 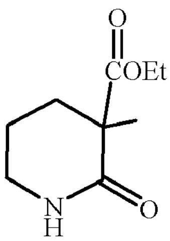 | $CH_2$ | | 398 (M+1) | |
| 433 | 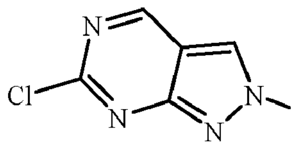 | $CH_2$ | -65.30 | | |

(continued)

| Cmpd No. | $R^1$ | L | $^{19}F$ NMR | MS | m.p. (°C) |
|---|---|---|---|---|---|
| 444 | 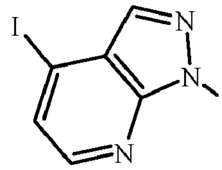 | $CH_2$ | -65.40 | | |
| 445 | 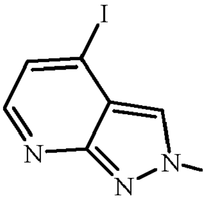 | $CH_2$ | | 472 (M+1) | |
| 446 | 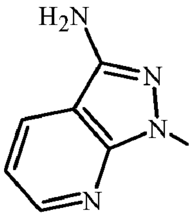 | $CH_2$ | -65.40 | | |
| 450 | 5-($CF_3C(=O)$)-2-furanyl | $CH_2$ | -65.35, -73.25 | | |
| 452 | | O | | 372 (M+1) | |
| 462 | 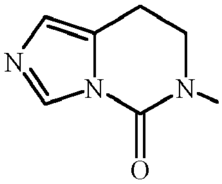 | $CH_2$ | | 364 (M+1) | |
| 492 | 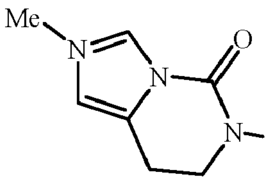 | $CH_2$ | | 379 (M+1) | |
| 506 | 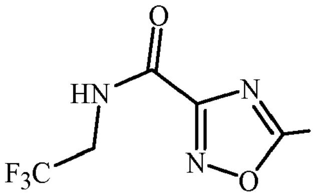 | $CH_2$ | | | 116-120 |
| 507 | 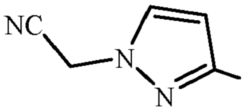 | $CH_2$ | | | 111-114 |
| 509 | 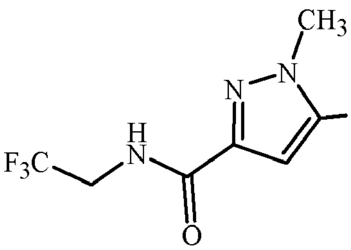 | $CH_2$ | | | 142-142.9 |

(continued)

| Cmpd No. | $R^1$ | L | $^{19}$F NMR | MS | m.p. (°C) |
|---|---|---|---|---|---|
| 510 | 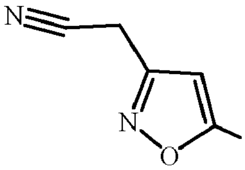 | $CH_2$ | | | 101-104 |
| 511 | 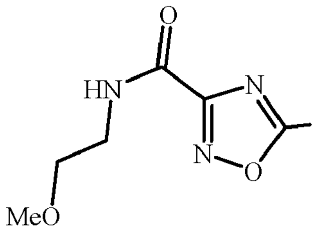 | $CH_2$ | | | 80-85 |
| 512 | 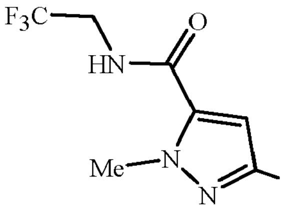 | $CH_2$ | | | 156-160 |

INDEX TABLE T

| Cmpd No. | Structure | $^{19}$F NMR | MS |
|---|---|---|---|
| 224 | 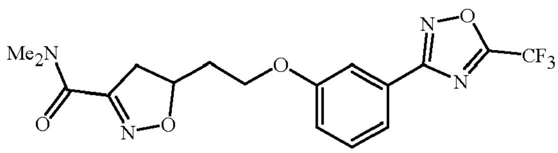 | -65.37 | 399 (M+1) |
| 232 | 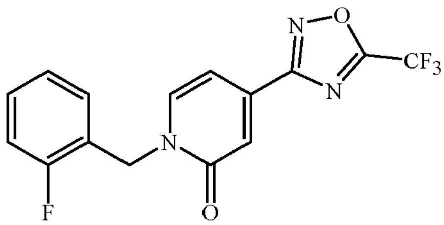 | -65.23 | |
| 233 | 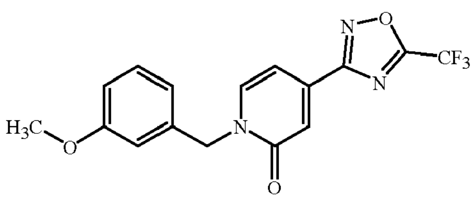 | -65.24 | |
| 276 | 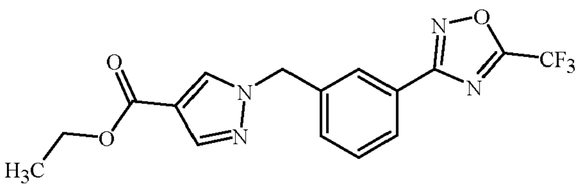 | | |

(continued)

| Cmpd No. | Structure | $^{19}F$ NMR | MS |
|---|---|---|---|
| 426 | 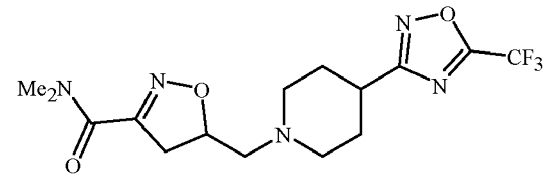 | -65.43 | 376 (M+1) |
| 502 | 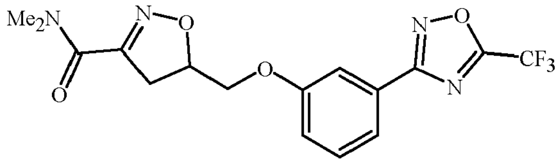 | -65.34 | |

BIOLOGICAL EXAMPLES OF THE INVENTION

General protocol for preparing test suspensions for Tests A-D: the test compounds were first dissolved in acetone in an amount equal to 3% of the final volume and then suspended at the desired concentration (in ppm) in acetone and purified water (50/50 mix by volume) containing 250 ppm of the surfactant PEG400 (polyhydric alcohol esters). The resulting test suspensions were then used in Tests A-D.

TEST A

The test suspension was sprayed to the point of run-off on soybean seedlings. The following day the seedlings were inoculated with a spore suspension of *Phakopsora pachyrhizi* (the causal agent of Asian soybean rust) and incubated in a saturated atmosphere at 22 °C for 24 h and then moved to a growth chamber at 22 °C for 8 days, after which time visual disease ratings were made.

TEST B

The test suspension was sprayed to the point of run-off on wheat seedlings. The following day the seedlings were inoculated with a spore suspension of *Zymoseptoria tritici* (the causal agent of wheat leaf blotch) and incubated in a saturated atmosphere at 24 °C for 48 h, and then moved to a growth chamber at 20 °C for 17 days, after which time visual disease ratings were made.

TEST C

The test suspension was sprayed to the point of run-off on wheat seedlings. The following day the seedlings were inoculated with a spore suspension of *Puccinia recondita* f. sp. *tritici;* (the causal agent of wheat leaf rust) and incubated in a saturated atmosphere at 20 °C for 24 h, and then moved to a growth chamber at 20 °C for 6 days, after which time visual disease ratings were made.

TEST D

The test suspension was sprayed to the point of run-off on wheat seedlings. The following day the seedlings were inoculated with a spore suspension of *Erysiphe graminis* f. sp. *tritici,* (the causal agent of wheat powdery mildew) and incubated in a saturated atmosphere at 20 °C for 8 days, after which time visual disease ratings were made. Of the compounds tested the following provided very good to excellent disease control (80% or greater): 85, 192, 263, 282, 337, 353, 455, 454, 490 and 497.

The test results for Tests A-C presented in Table A below for compounds of Formula 1 illustrate the fungicidal activity of component (a) contributing to the plant disease control utility of compositions comprising component (a) in combination with component (b) according to the present invention. In the Table, a rating of 100 indicates 100% disease control and a rating of 0 indicates no disease control (relative to the controls). A dash (-) indicates the compound was not tested. The test suspensions were sprayed at the concentration listed in the column "Rate in ppm", unless otherwise indicated. An asterisk "*" next to the rating indicates a 250 ppm test suspension was used, a double asterisk "**" next to the rating indicates a 100 ppm test suspension was used, and a triple asterisk "***" next to the rating indicates a 50

ppm test suspension was used.

TABLE A

| Cmpd No. | Rate in ppm | Test A | Test B | Test C |
|---|---|---|---|---|
| 1 | 10 | 100 | - | 68 |
| 2 | 10 | 100 | 98** | 99 |
| 3 | 50 | 100 | - | 100 |
| 4 | 50 | 100 | - | 100 |
| 5 | 50 | 100 | - | 94 |
| 6 | 250 | 100 | 0 | 100 |
| 7 | 250 | 100 | 0 | 100 |
| 8 | 270 | 100 | 18 | 100 |
| 9 | 260 | 100 | 100 | 100 |
| 10 | 270 | 100 | 5 | 100 |
| 11 | 10 | 100 | - | 74 |
| 12 | 255 | 100 | 98 | 100 |
| 13 | 250 | 100 | 85 | 100 |
| 14 | 250 | 100 | 86 | 100 |
| 15 | 250 | 100 | 0 | 96 |
| 16 | 260 | 100 | 93 | 100 |
| 17 | 250 | 100 | 98 | 100 |
| 18 | 250 | 100 | 93 | 100 |
| 19 | 250 | 100 | 100 | 100 |
| 20 | 250 | 100 | 99 | 100 |
| 21 | 10 | 100 | 97** | 100 |
| 22 | 10 | 100 | - | 96 |
| 23 | 10 | 100 | - | 100 |
| 24 | 10 | 100 | - | 95 |
| 25 | 250 | 100 | 86* | 100 |
| 26 | 250 | 100 | 46* | 100 |
| 27 | 50 | 100 | - | 100 |
| 28 | 50 | 100 | - | 100 |
| 29 | 10 | 73 | 94* | 19 |
| 30 | 10 | 25 | - | 0 |
| 31 | 10 | 100 | - | 100 |
| 32 | 10 | 96 | - | 68 |
| 33 | 10 | 100 | - | 68 |
| 34 | 10 | 71 | - | 55 |
| 35 | 10 | 100 | - | 98 |
| 36 | 10 | 100 | - | 100 |
| 37 | 10 | 99 | - | 86 |
| 38 | 10 | 78 | - | 68 |
| 39 | 10 | 44 | - | 28 |
| 40 | 10 | 100 | - | 92 |
| 41 | 10 | 100 | - | 98 |
| 42 | 10 | 100 | - | 92 |
| 43 | 10 | 100 | - | 96 |
| 44 | 10 | 100 | - | 95 |
| 45 | 10 | 100 | - | 99 |
| 46 | 10 | 100 | - | 100 |
| 47 | 10 | 100 | - | 74 |
| 48 | 10 | 100 | - | 89 |

(continued)

| Cmpd No. | Rate in ppm | Test A | Test B | Test C |
|---|---|---|---|---|
| 49 | 10 | 100 | - | 100 |
| 50 | 10 | 13 | - | 28 |
| 51 | 10 | 99 | - | 68 |
| 52 | 10 | 99 | - | 80 |
| 53 | 10 | 0 | - | 9 |
| 54 | 10 | 99 | - | 68 |
| 55 | 10 | 100 | - | 99 |
| 56 | 10 | 100 | 68** | 100 |
| 57 | 10 | 100 | 99** | 100 |
| 58 | 10 | 100 | 83** | 100 |
| 59 | 10 | 100 | 58** | 100 |
| 60 | 10 | 100 | 75** | 100 |
| 61 | 10 | 100 | - | 100 |
| 62 | 10 | 100 | - | 100 |
| 63 | 10 | 100 | - | 100 |
| 64 | 10 | 100 | - | 100 |
| 65 | 10 | 100 | - | 100 |
| 66 | 255 | 100 | - | 100 |
| 67 | 10 | 100 | - | 99 |
| 68 | 10 | 100 | - | 100 |
| 69 | 10 | 100 | - | 100 |
| 70 | 255 | 100 | 96 | 100 |
| 71 | 250 | 100 | 36 | 100 |
| 72 | 50 | 99 | - | 100 |
| 73 | 10 | 100 | - | 86 |
| 74 | 10 | 99 | - | 51 |
| 75 | 10 | 100 | - | 68 |
| 76 | 270 | 100 | 41 | 99 |
| 77 | 50 | 100 | - | 100 |
| 78 | 10 | 100 | - | 100 |
| 79 | 10 | 100 | - | 68 |
| 80 | 10 | 100 | - | 68 |
| 81 | 10 | 100 | - | 0 |
| 82 | 10 | 0 | - | 0 |
| 83 | 250 | 100 | 79 | 100 |
| 84 | 250 | 100 | 66 | 100 |
| 85 | 250 | 100*** | 9 | 100 |
| 86 | 250 | 100 | 89 | 100 |
| 87 | 250 | 100 | 82 | 100 |
| 88 | 250 | 100 | 93 | 100 |
| 89 | 250 | 100 | 83 | 100 |
| 90 | 250 | 100 | 33 | 99 |
| 91 | 250 | 100 | 96 | 100 |
| 92 | 250 | 0 | 96 | 100 |
| 93 | 250 | 100 | 39 | 99 |
| 94 | 250 | 100 | 35 | 78 |
| 95 | 250 | 100 | 87 | 100 |
| 96 | 250 | 100 | 0 | 99 |
| 97 | 50 | 99 | - | 100 |
| 98 | 50 | 99 | - | 68 |

(continued)

| Cmpd No. | Rate in ppm | Test A | Test B | Test C |
|---|---|---|---|---|
| 99 | 10 | 100 | - | 68 |
| 100 | 10 | 100 | - | 86 |
| 101 | 50 | 100 | - | 100 |
| 102 | 10 | 100 | - | 99 |
| 103 | 10 | 100 | - | 97 |
| 104 | 10 | 100 | - | 68 |
| 105 | 50 | 100 | - | 100 |
| 106 | 50 | 100 | - | 100 |
| 107 | 10 | 100 | - | 90 |
| 108 | 50 | 100 | - | 100 |
| 109 | 50 | 100 | - | 77 |
| 110 | 10 | 100 | - | 91 |
| 111 | 255 | 100 | 0 | 86 |
| 112 | 10 | 92 | - | 0 |
| 113 | 10 | 99 | - | 0 |
| 114 | 250 | 100 | 0 | 100 |
| 115 | 10 | 98 | - | 95 |
| 116 | 265 | 100 | 25 | 99 |
| 117 | 255 | 88 | 0 | 41 |
| 118 | 250 | 100 | 0 | 98 |
| 119 | 265 | 100 | 54 | 100 |
| 120 | 260 | 99 | 0 | 68 |
| 121 | 10 | 87 | - | 0 |
| 122 | 250 | 100 | 0 | 68 |
| 123 | 255 | 100 | 21 | 99 |
| 124 | 260 | 100 | 50 | 100 |
| 125 | 260 | 100 | 55 | 100 |
| 126 | 260 | 100 | - | 100 |
| 127 | 10 | 0 | - | 0 |
| 128 | 255 | 100 | 19 | 100 |
| 129 | 260 | 100 | 0 | 89 |
| 130 | 260 | 100 | 89 | 100 |
| 131 | 10 | 100 | - | 99 |
| 132 | 270 | 100 | 0 | 95 |
| 133 | 255 | 100 | 59 | 100 |
| 134 | 255 | 100 | 0 | 80 |
| 135 | 10 | 96 | - | 0 |
| 136 | 10 | 100 | - | 68 |
| 137 | 10 | 100 | - | 41 |
| 138 | 260 | 100 | 96 | 100 |
| 139 | 255 | 100 | 62 | 100 |
| 140 | 10 | 0 | - | 0 |
| 141 | 10 | 100 | - | 88 |
| 142 | 250 | 100 | 57 | 100 |
| 143 | 250 | 100 | 71 | 100 |
| 144 | 250 | 36 | 0 | 0 |
| 145 | 250 | 100 | 0 | 100 |
| 146 | 250 | 100 | 0 | 0 |
| 147 | 10 | 100 | - | 100 |
| 148 | 10 | 100 | - | 96 |

(continued)

| Cmpd No. | Rate in ppm | Test A | Test B | Test C |
|---|---|---|---|---|
| 149 | 260 | 100 | 72 | 100 |
| 150 | 255 | 100 | 19 | 100 |
| 151 | 50 | 100 | - | 98 |
| 152 | 10 | 100 | - | 100 |
| 153 | 250 | 100 | 0 | 89 |
| 154 | 50 | 100 | - | 100 |
| 155 | 10 | 60 | - | 0 |
| 156 | 255 | 100 | 86 | 100 |
| 157 | 10 | 100 | - | 100 |
| 158 | 270 | 100 | 90 | 100 |
| 159 | 260 | 100 | 87 | 100 |
| 160 | 10 | 99 | - | 98 |
| 161 | 265 | 100 | 33 | 99 |
| 162 | 250 | 100 | 0 | 100 |
| 163 | 250 | 100 | 0 | 100 |
| 164 | 250 | 100 | 88 | 100 |
| 165 | 260 | 100 | 95 | 100 |
| 166 | 10 | 100 | 90** | 100 |
| 167 | 10 | 100 | - | 100 |
| 168 | 10 | 100 | - | 100 |
| 169 | 10 | 100 | - | 99 |
| 170 | 10 | 100 | 97** | 100 |
| 171 | 10 | 100 | 37** | 100 |
| 172 | 10 | 100 | - | 99 |
| 173 | 10 | 100 | - | 96 |
| 174 | 10 | 85 | - | 91 |
| 175 | 255 | 100 | 95 | 100 |
| 176 | 250 | 100 | 92 | 100 |
| 177 | 250 | 100 | 97 | 100 |
| 178 | 10 | 96 | - | 68 |
| 179 | 10 | 98 | - | 9 |
| 180 | 10 | 97 | - | 0 |
| 181 | 10 | 100 | - | 100 |
| 182 | 10 | 100 | - | 100 |
| 183 | 10 | 100 | - | 100 |
| 184 | 10 | 100 | - | 55 |
| 185 | 10 | 100 | - | 98 |
| 186 | 10 | 100 | - | 74 |
| 187 | 10 | 0 | - | 0 |
| 188 | 10 | 100 | - | 100 |
| 189 | 50 | 100 | - | 100 |
| 190 | 10 | 100 | - | 100 |
| 191 | 50 | 100 | 96* | 100 |
| 192 | 10 | 100 | 99* | 100 |
| 193 | 50 | 100 | 28* | 100 |
| 194 | 10 | 100 | - | 100 |
| 195 | 10 | 100 | - | 98 |
| 196 | 10 | 100 | - | 93 |
| 197 | 10 | 99 | - | 98 |
| 198 | 10 | 100 | 94** | 99 |

(continued)

| Cmpd No. | Rate in ppm | Test A | Test B | Test C |
|---|---|---|---|---|
| 199 | 10 | 100 | - | 100 |
| 200 | 10 | 100 | - | 100 |
| 201 | 250 | 100 | 28 | 100 |
| 202 | 250 | 100 | 83 | 100 |
| 203 | 10 | 100 | - | 86 |
| 204 | 10 | 100 | - | 100 |
| 205 | 10 | 100 | - | 89 |
| 206 | 10 | 98 | - | 68 |
| 207 | 10 | 100 | - | 86 |
| 208 | 10 | 100 | - | 86 |
| 209 | - | - | - | - |
| 210 | 50 | 100 | 63* | 99 |
| 211 | 50 | 100 | 70* | 100 |
| 212 | 10 | 100 | 79* | 100 |
| 213 | 10 | 100 | 82* | 74 |
| 214 | 10 | 100 | 73* | 74 |
| 215 | 10 | 99 | 90* | 74 |
| 216 | 10 | 100 | 9* | 74 |
| 217 | 10 | 100 | 35* | 86 |
| 218 | 10 | 100 | - | 98 |
| 219 | 10 | 100 | - | 86 |
| 220 | 10 | 100 | - | 86 |
| 221 | 10 | 100 | - | 95 |
| 222 | 10 | 100 | - | 97 |
| 223 | 10 | 100 | - | 91 |
| 224 | 50 | 100 | 81* | 89 |
| 225 | 10 | 100 | 99* | 100 |
| 226 | 10 | 100 | 94* | 100 |
| 227 | 10 | 100 | 99* | 97 |
| 228 | 10 | 100 | 99* | 79 |
| 229 | 10 | 100 | 94* | 100 |
| 230 | 10 | 100 | 99* | 99 |
| 231 | 10 | 100 | 95* | 97 |
| 232 | 250 | 100 | 68 | 100 |
| 233 | 50 | 97 | 90* | 100 |
| 234 | 10 | 100 | 87* | 88 |
| 235 | 250 | 100 | 87 | 100 |
| 236 | 250 | 100 | 83 | 100 |
| 237 | 250 | 100 | 94 | 100 |
| 238 | 250 | 100 | 87 | 100 |
| 239 | 250 | 100 | 0 | 100 |
| 240 | 250 | 100 | 4 | 100 |
| 241 | 10 | 100 | - | 100 |
| 242 | 10 | 100 | - | 99 |
| 243 | 10 | 98 | - | 85 |
| 244 | 10 | 100 | - | 95 |
| 245 | 10 | 100 | - | 86 |
| 246 | 10 | 100 | - | 100 |
| 247 | 10 | 100 | - | 100 |
| 248 | 10 | 100 | - | 100 |

(continued)

| Cmpd No. | Rate in ppm | Test A | Test B | Test C |
|---|---|---|---|---|
| 249 | 10 | 100 | - | 100 |
| 250 | 10 | 100 | - | 74 |
| 251 | 10 | 100 | - | 74 |
| 252 | 10 | 100 | 92* | 84 |
| 253 | 10 | 98 | 46* | 74 |
| 254 | 10 | 100 | 70* | 100 |
| 255 | 50 | 100 | - | 100 |
| 256 | 10 | 100 | 98* | 99 |
| 257 | 10 | 100 | 99* | 100 |
| 258 | 10 | 100 | 99* | 99 |
| 259 | 10 | 100 | 99* | 99 |
| 260 | 10 | 100 | 47* | 100 |
| 261 | 10 | 100 | 88* | 99 |
| 262 | 10 | 100 | 96* | 98 |
| 263 | 10 | 100 | 95* | 94 |
| 264 | 10 | 100 | 99* | 99 |
| 265 | 10 | 100 | 93* | 100 |
| 267 | 50 | 99 | - | 74 |
| 268 | 10 | 100 | - | 86 |
| 269 | 10 | 100 | - | 100 |
| 270 | 10 | 100 | - | 100 |
| 271 | 10 | 99 | - | 68 |
| 272 | 10 | 100 | - | 100 |
| 273 | 10 | 100 | - | 100 |
| 274 | 10 | 100 | - | 100 |
| 275 | 10 | 100 | - | 100 |
| 276 | 50 | 100 | 35* | 80 |
| 277 | 10 | 100 | 99* | 100 |
| 278 | 10 | 100 | 98* | 100 |
| 279 | 10 | 100 | 94* | 100 |
| 280 | 10 | 100 | 99* | 99 |
| 281 | 10 | 100 | 34* | 95 |
| 282 | 10 | 100 | 94* | 97 |
| 283 | 10 | 100 | 0* | 98 |
| 284 | 10 | 100 | 91* | 100 |
| 285 | 10 | 100 | 94* | 97 |
| 286 | 10 | 100 | 3* | 100 |
| 287 | 10 | 100 | 98* | 100 |
| 288 | 10 | 100 | 91* | 100 |
| 289 | 10 | 100 | 80* | 100 |
| 290 | 10 | 100 | 53* | 100 |
| 291 | 10 | 100 | 13* | 97 |
| 292 | 10 | 100 | 96* | 85 |
| 293 | 10 | 100 | 91* | 79 |
| 294 | 10 | 100 | 50* | 79 |
| 297 | 10 | 100 | - | 93 |
| 298 | 10 | 50 | 73* | 0 |
| 299 | 10 | 100 | - | 86 |
| 300 | 10 | 94 | - | 9 |
| 301 | 10 | 100 | - | 100 |

(continued)

| Cmpd No. | Rate in ppm | Test A | Test B | Test C |
|---|---|---|---|---|
| 303 | 10 | 100 | - | 100 |
| 304 | 10 | 100 | - | 100 |
| 305 | 50 | 44 | 0* | 0 |
| 306 | 50 | 0 | 10* | 0 |
| 307 | 50 | 0 | 0* | 0 |
| 308 | 10 | 0 | - | 0 |
| 309 | 50 | 100 | - | 68 |
| 310 | 50 | 0 | 0* | 0 |
| 311 | 50 | 0 | 0* | 0 |
| 312 | 10 | 0 | 0* | 0 |
| 313 | 10 | 77 | 14* | 0 |
| 314 | 10 | 99 | 86* | 0 |
| 315 | 10 | 100 | 97* | 99 |
| 316 | 10 | 100 | 5* | 100 |
| 317 | 10 | 0 | 54* | 0 |
| 318 | 10 | 0 | 11* | 0 |
| 319 | 10 | 97 | 33* | 0 |
| 320 | 10 | 97 | 33* | 0 |
| 321 | 250 | 100 | 99* | 100 |
| 322 | 250 | 100 | 95* | 100 |
| 323 | 250 | 100 | 92* | 100 |
| 324 | 250 | 100 | 80* | 100 |
| 325 | 250 | 100 | 36* | 100 |
| 326 | 250 | 84 | 1* | 67 |
| 327 | 10 | 100 | 94* | 100 |
| 328 | 10 | 100 | 0* | 100 |
| 329 | 10 | 100 | 98* | 98 |
| 330 | 10 | 100 | 96* | 100 |
| 331 | 10 | 99 | 97* | 94 |
| 332 | 10 | 100 | 99* | 99 |
| 334 | 10 | 99 | 92* | 86 |
| 335 | 10 | 100 | 73* | 94 |
| 336 | 10 | 0 | - | 0 |
| 337 | 10 | 99 | 78* | 82 |
| 338 | 10 | 99 | 10* | 0 |
| 339 | 10 | 100 | - | 98 |
| 340 | 10 | 100 | - | 94 |
| 341 | 10 | 100 | - | 100 |
| 342 | 10 | 100 | 99* | 100 |
| 343 | 10 | 100 | 97* | 100 |
| 344 | 10 | 100 | 96* | 93 |
| 345 | 10 | 96 | 50* | 68 |
| 346 | 10 | 0 | 0* | 0 |
| 347 | 10 | 0 | 0* | 0 |
| 348 | 10 | 100 | - | 96 |
| 349 | 10 | 100 | - | 100 |
| 350 | 10 | 100 | - | 99 |
| 351 | 10 | 92 | - | 67 |
| 352 | 10 | 87 | 0* | 68 |
| 353 | 10 | 0 | 73* | 0 |

(continued)

| Cmpd No. | Rate in ppm | Test A | Test B | Test C |
|---|---|---|---|---|
| 354 | 10 | 0 | 20* | 0 |
| 355 | 10 | 0 | 4* | 0 |
| 356 | 250 | 99 | 96* | 94 |
| 357 | 250 | 98 | 99* | 91 |
| 358 | 10 | 0 | 0* | 0 |
| 359 | 10 | 100 | 99* | 95 |
| 360 | 10 | 100 | - | 68 |
| 361 | 10 | 100 | - | 0 |
| 362 | 50 | 100 | - | 100 |
| 363 | 50 | 100 | - | 100 |
| 364 | 50 | 100 | - | 100 |
| 365 | 50 | 100 | 80* | 99 |
| 366 | 50 | 100 | - | 100 |
| 367 | 50 | 96 | - | 67 |
| 368 | 50 | 100 | - | 100 |
| 369 | 50 | 100 | - | 100 |
| 370 | 250 | 100 | 98* | 100 |
| 371 | 250 | 100 | 95* | 100 |
| 372 | 250 | 100 | 99* | 100 |
| 373 | 250 | 100 | 99* | 100 |
| 374 | 10 | 100 | - | 96 |
| 375 | 10 | 100 | - | 98 |
| 376 | 10 | 100 | 49* | 68 |
| 377 | 10 | - | - | - |
| 378 | 10 | 100 | - | 98 |
| 379 | 10 | 100 | - | 97 |
| 380 | 10 | 100 | - | 100 |
| 381 | 10 | 0 | - | 0 |
| 382 | 10 | 100 | - | 96 |
| 383 | 10 | 71 | - | 68 |
| 384 | 10 | 100 | - | 100 |
| 385 | 10 | 100 | - | 100 |
| 386 | 10 | 100 | - | 100 |
| 387 | 10 | 100 | - | 99 |
| 388 | 10 | 100 | - | 100 |
| 389 | 10 | 100 | - | 100 |
| 390 | 10 | 100 | - | 100 |
| 391 | 10 | 100 | - | 100 |
| 392 | 10 | 100 | - | 100 |
| 393 | 10 | 100 | 94* | 100 |
| 394 | 10 | 100 | 99* | 100 |
| 395 | 10 | 95 | 0* | 97 |
| 396 | 10 | 100 | 28* | 100 |
| 397 | 10 | 100 | 82* | 98 |
| 398 | 10 | 100 | 80* | 97 |
| 399 | 10 | 100 | 10* | 99 |
| 400 | 10 | 100 | 99* | 100 |
| 401 | 10 | 100 | 77* | 99 |
| 402 | 10 | 100 | 96* | 90 |
| 403 | 10 | 60 | 47* | 0 |

(continued)

| Cmpd No. | Rate in ppm | Test A | Test B | Test C |
|---|---|---|---|---|
| 404 | 10 | 0 | 0* | 68 |
| 406 | 10 | 100 | - | 93 |
| 407 | 10 | 100 | - | 100 |
| 408 | 10 | 100 | - | 100 |
| 409 | 10 | 100 | - | 100 |
| 410 | 10 | 100 | - | 100 |
| 411 | 10 | 100 | - | 100 |
| 412 | 10 | 100 | - | 100 |
| 413 | 10 | 100 | - | 68 |
| 414 | 10 | 100 | - | 100 |
| 415 | 10 | 100 | - | 96 |
| 416 | 50 | 77 | - | 0 |
| 417 | 10 | 100 | - | 100 |
| 418 | 10 | 100 | - | 99 |
| 419 | 10 | 96 | - | 99 |
| 420 | 10 | 100 | - | 100 |
| 421 | 10 | 100 | - | 100 |
| 422 | 10 | 90 | - | 93 |
| 423 | 10 | 86 | - | 80 |
| 424 | 10 | 100 | - | 96 |
| 426 | 250 | 0 | 9* | 0 |
| 427 | 50 | 100 | - | 67 |
| 428 | 50 | 100 | - | 100 |
| 429 | 50 | 100 | - | 100 |
| 430 | 50 | 100 | - | 100 |
| 431 | 50 | 99 | - | 98 |
| 432 | 50 | 100 | - | 92 |
| 433 | 10 | 100 | - | 99 |
| 434 | 10 | 100 | - | 98 |
| 435 | 10 | 100 | - | 74 |
| 436 | 10 | 100 | - | 74 |
| 437 | 10 | 100 | - | 86 |
| 438 | 10 | - | - | - |
| 439 | 10 | 0 | - | 0 |
| 440 | 10 | 0 | - | 0 |
| 441 | 10 | 87 | - | 9 |
| 442 | 10 | 44 | - | 0 |
| 443 | 10 | 100 | - | 74 |
| 444 | 10 | 96 | - | 86 |
| 445 | 10 | 100 | - | 99 |
| 446 | 10 | 99 | - | 86 |
| 447 | 10 | 100 | - | 100 |
| 448 | 10 | 100 | - | 100 |
| 449 | 10 | 100 | - | 100 |
| 450 | 10 | 94 | 41* | 0 |
| 451 | 10 | 99 | 8* | 0 |
| 452 | 10 | 81 | 59* | 0 |
| 453 | 10 | 92 | 50* | 0 |
| 454 | 10 | 100 | 47* | 0 |
| 455 | 10 | 100 | 66* | 0 |

(continued)

| Cmpd No. | Rate in ppm | Test A | Test B | Test C |
|---|---|---|---|---|
| 456 | 10 | 100 | 65* | 68 |
| 457 | 50 | 77 | - | 0 |
| 458 | 50 | 0 | 0* | 0 |
| 459 | 50 | 0 | 0* | 0 |
| 460 | 50 | 100 | - | 99 |
| 461 | 50 | 100 | - | 100 |
| 462 | 10 | 100 | - | 89 |
| 463 | 10 | 100 | 65* | 100 |
| 464 | 10 | 79 | 94* | 23 |
| 465 | 10 | 100 | 0* | 80 |
| 466 | 10 | 100 | 3* | 55 |
| 467 | 10 | 97 | 6* | 68 |
| 468 | 10 | 84 | 22* | 0 |
| 469 | 10 | 96 | 65* | 86 |
| 470 | 10 | 100 | 0* | 94 |
| 471 | 10 | 77 | 15* | 80 |
| 472 | 10 | 77 | 22* | 0 |
| 473 | 10 | 94 | 28* | 89 |
| 474 | 10 | 100 | - | 100 |
| 475 | 10 | 100 | - | 91 |
| 476 | 10 | 100 | - | 86 |
| 477 | 10 | 100 | - | 100 |
| 478 | 50 | 81 | - | 19 |
| 479 | 10 | 100 | - | 100 |
| 480 | 10 | 100 | - | 98 |
| 481 | 10 | 100 | - | 100 |
| 482 | 10 | 100 | - | 100 |
| 483 | 10 | 100 | - | 54 |
| 484 | 10 | 100 | - | 100 |
| 485 | 10 | 100 | - | 99 |
| 486 | 10 | 100 | - | 89 |
| 487 | 10 | 100 | 98** | 100 |
| 488 | 10 | 100 | 99* | 100 |
| 489 | 10 | 100 | 92* | 99 |
| 490 | 10 | 100 | 94* | 74 |
| 491 | - | - | - | - |
| 492 | 10 | 100 | - | 85 |
| 493 | 10 | 100 | - | 100 |
| 494 | 10 | 100 | 47* | 0 |
| 495 | 10 | 100 | 43* | 0 |
| 496 | 10 | 100 | 48* | 0 |
| 497 | 10 | 100 | 5* | 0 |
| 498 | 10 | 100 | 43* | 0 |
| 499 | 250 | 100 | 48* | 100 |
| 500 | 250 | 100 | 5* | 100 |
| 501 | 10 | 100 | - | 0 |
| 502 | 250 | 100 | 62 | 100 |
| 503 | 10 | 43 | - | 0 |
| 504 | 10 | 74 | - | 0 |
| 505 | 10 | 100 | 100* | 100 |

(continued)

| Cmpd No. | Rate in ppm | Test A | Test B | Test C |
|---|---|---|---|---|
| 506 | 250 | 100 | 49 | 100 |
| 507 | 10 | 100 | - | 98 |
| 508 | 10 | 100 | - | 100 |
| 509 | 50 | 100 | - | 99 |
| 510 | 10 | 100 | - | 84 |
| 511 | 250 | 100 | 90 | 100 |
| 512 | 10 | 100 | 98* | 100 |

The test results for TEST A through D presented above illustrate the fungicidal activity of component (a) (i.e. compound of Formula 1) contributing to the plant disease control utility of compositions comprising component (a) in combination with component (b) and optionally at least one additional fungicidal compound according to the present invention.

The test results presented below for TEST E illustrate the fungicidal efficacy of mixtures of this invention (i.e. mixtures of component (a) and component (b)). The pathogen control protection afforded by the mixtures is not limited, however, to these test results.

The general protocol for preparing test compositions for TEST E was as follows: Compound 316, Compound 376, Compound 225, Compound 229, Compound 257, Compound 394, Compound 506, Compound 507, Compound 508, Compound 509, Compound 511, Compound 512, methyl *N*-[[5-[1-[2,6-difluoro-4-(1-methylethyl)phenyl]-1*H*-pyrazol-3-yl]-2-methylphenyl]methyl]carbamate (b54.1 1a), methyl *N*-[[5-[1-(4-cyclopropyl-2,6-difluorophenyl)-1*H*-pyrazol-3-yl]-2-methylphenyl]methyl]carbamate (b54.11d), ethyl 1-[[4-[[(1*Z*)-2-ethoxy-3,3,3-trifluoro-1-propen-1-yl]oxy]phenyl]methyl]-1*H*-pyrazole-4-carboxylate (b54.12a), ethyl 1-[[4-[[2-(trifluoromethyl)-1,3-dioxolan-2-yl]methoxy]phenyl]methyl]-1*H*-pyrazole-4-carboxylate (b54.13a), azoxystrobin, benzovindiflupyr, bixafen, chlorothalonil, cyproconazole, epoxiconazole, fenpropidin, fenpropimorph, fluindapyr, flutriafol, fluxapyroxad, inpyrfluxam, metominostrobin, picoxystrobin, prothioconazole, pydiflumetofen, pyraclostrobin, tebuconazole and trifloxystrobin were obtained as unformulated, technical-grade materials. Copper hydroxide and mancozeb was obtained as a formulated product marketed under the trademarks KOCIDE 3000 and MANZATE, respectively. Unformulated materials were first dissolved in acetone and then suspended at the desired concentration (in ppm) in acetone and purified water (50/50 mixture by volume) containing 250 ppm of the surfactant Trem® 014 (polyhydric alcohol esters). Formulated materials were dispersed in sufficient water to give the desired concentration, and neither organic solvent nor surfactant was added to the suspension. The resulting test mixtures were then used in TEST E. The tests were run on four individual plants and the results reported as the mean average of the four plants.

SYNERGY EVALUATION METHOD

The presence of a synergistic effect between two active ingredients can be established with the aid of the Colby equation (see Colby, S. R. "Calculating Synergistic and Antagonistic Responses of Herbicide Combinations", Weeds, (1967), 15, 20-22):

$$p = A + B - \left[\frac{A \times B}{100}\right].$$

Using the method of Colby, the presence of a synergistic interaction between two active ingredients is established by first calculating the predicted activity, p, of the mixture based on activities of the two components applied alone. If p is lower than the experimentally established effect, synergism has occurred. In the equation above, A is the fungicidal activity in percentage control of one component applied alone at rate x. The B term is the fungicidal activity in percentage control of the second component applied at rate y. The equation estimates p, the expected fungicidal activity of the mixture of A at rate x with B at rate y if their effects are strictly additive and no interaction has occurred.

TEST E

The test mixture was sprayed to the point of run-off on soybean seedlings. The following day the seedlings were inoculated with a spore suspension of *Phakopsora pachyrhizi* (the causal agent of Asian soybean rust) and incubated

in a saturated atmosphere at 22 °C for 24 h and then moved to a growth chamber at 22 °C for 8 days, after which time visual disease ratings were made.

Results for Test E are given below in Tables A-1 through K-2. Each table corresponds to a set of evaluations performed together at the same time. In each table, a rating of 100 indicates 100 % disease control and a rating of 0 indicates no disease control (relative to the controls). Columns labeled "Obsd" indicate the average of results observed from test run on four individual plants. Columns labeled "Exp" indicate the expected value for each treatment mixture using the Colby equation, as described above.

Table A-1

Observed and Expected Effects of Compound 316 Alone and Mixtures with **b54.13a** (ethyl 1-[[4-[[2-(trifluoromethyl)-1,3-dioxolan-2-yl]methoxy]phenyl]methyl]-1*H*-pyrazole-4-carboxylate), **b54.12a** (ethyl 1-[[4-[[(1*Z*)-2-ethoxy-3,3,3-trifluoro-1-propen-1-yl]oxy]phenyl]methyl]-1*H*-pyrazole-4-carboxylate), **b54.11a** (methyl *N*-[[5-[1-[2,6-difluoro-4-(1-methylethyl)phenyl]-1*H*-pyrazol-3-yl]-2-methylphenyl]methyl]carbamate) and Picoxystrobin in Controlling Asian Soybean Rust

| Application Rate (ppm) of Compound 316 (i.e. Component (a)) | Component (b) | Application Rate (ppm) of Component (b) | Test E | |
|---|---|---|---|---|
| | | | Obsd | Exp |
| 0 | None | 0 | 0 | - |
| 0.471 | None | 0 | 59 | - |
| 0.764 | None | 0 | 75 | - |
| 0 | b54.13a | 1.422 | 55 | - |
| 0 | b54.13a | 2.276 | 64 | - |
| 0.471 | b54.13a | 1.422 | 88 | 81 |
| 0.471 | b54.13a | 2.276 | 79 | 85 |
| 0.764 | b54.13a | 1.422 | 86 | 89 |
| 0.764 | b54.13a | 2.276 | 92 | 91 |
| 0 | b54.12a | 0.303 | 53 | - |
| 0 | b54.12a | 0.489 | 68 | - |
| 0.471 | b54.12a | 0.303 | 82 | 80 |
| 0.471 | b54.12a | 0.489 | 85 | 87 |
| 0.764 | b54.12a | 0.303 | 86 | 88 |
| 0.764 | b54.12a | 0.489 | 92 | 92 |
| 0 | b54.11a | 0.403 | 34 | - |
| 0 | b54.11a | 0.652 | 68 | - |
| 0.471 | b54.11a | 0.403 | 62 | 73 |
| 0.471 | b54.11a | 0.652 | 69 | 87 |
| 0.764 | b54.11a | 0.403 | 68 | 84 |
| 0.764 | b54.11a | 0.652 | 79 | 92 |
| 0 | picoxystrobin | 20.596 | 73 | - |
| 0 | picoxystrobin | 38.865 | 75 | - |
| 0.471 | picoxystrobin | 20.596 | 74 | 89 |
| 0.471 | picoxystrobin | 38.865 | 81 | 90 |
| 0.764 | picoxystrobin | 20.596 | 84 | 93 |
| 0.764 | picoxystrobin | 38.865 | 95 | 94 |

Table A-2

| Observed and Expected Effects of Compound 376 Alone and Mixtures with **b54.13a** (ethyl 1-[[4-[[2-(trifluoromethyl)-1,3-dioxolan-2-yl]methoxy]phenyl]methyl]-1*H*-pyrazole-4-carboxylate), **b54.12a** (ethyl 1-[[4-[[(1*Z*)-2-ethoxy-3,3,3-trifluoro-1-propen-1-yl]oxy]phenyl]methyl]-1*H*-pyrazole-4-carboxylate), **b54.11a** (methyl *N*-[[5-[1-[2,6-difluoro-4-(1-methylethyl)phenyl]-1*H*-pyrazol-3-yl]-2-methylphenyl]methyl]carbamate), **b54.11d** (methyl *N*-[[5-[1-(4-cyclopropyl-2,6-difluorophenyl)-1*H*-pyrazol-3-yl]-2-methylphenyl]methyl] carbamate) and Picoxystrobin in Controlling Asian Soybean Rust | | | | |
|---|---|---|---|---|
| Application Rate (ppm) of Compound 376 (i.e. Component (a)) | Component (b) | Application Rate (ppm) of Component (b) | Test E | |
| | | | Obsd | Exp |
| 0 | None | 0 | 0 | - |
| 0.753 | None | 0 | 70 | - |
| 1.228 | None | 0 | 66 | - |
| 0 | b54.13a | 1.422 | 55 | - |
| 0 | b54.13a | 2.276 | 64 | - |
| 0.753 | b54.13a | 1.422 | 68 | 87 |
| 0.753 | b54.13a | 2.276 | 87 | 89 |
| 1.228 | b54.13a | 1.422 | 75 | 85 |
| 1.228 | b54.13a | 2.276 | 75 | 88 |
| 0 | b54.12a | 0.303 | 53 | - |
| 0 | b54.12a | 0.489 | 68 | - |
| 0.753 | b54.12a | 0.303 | 81 | 86 |
| 0.753 | b54.12a | 0.489 | 86 | 90 |
| 1.228 | b54.12a | 0.303 | 83 | 84 |
| 1.228 | b54.12a | 0.489 | 89 | 89 |
| 0 | b54.11a | 0.403 | 34 | - |
| 0 | b54.11a | 0.652 | 68 | - |
| 0.753 | b54.11a | 0.403 | 61 | 80 |
| 0.753 | b54.11a | 0.652 | 77 | 90 |
| 1.228 | b54.11a | 0.403 | 76 | 77 |
| 1.228 | b54.11a | 0.652 | 93 | 89 |
| 0 | b54.11d | 0.346 | 29 | - |
| 0 | b54.11d | 0.764 | 62 | - |
| 0.753 | b54.11d | 0.346 | 70 | 79 |
| 0.753 | b54.11d | 0.764 | 86 | 89 |
| 1.228 | b54.11d | 0.346 | 89 | 76 |
| 1.228 | b54.11d | 0.764 | 88 | 87 |
| 0 | picoxystrobin | 20.596 | 73 | - |
| 0 | picoxystrobin | 33.865 | 75 | - |
| 0.753 | picoxystrobin | 20.596 | 66 | 92 |
| 0.753 | picoxystrobin | 33.865 | 80 | 93 |

(continued)

| Observed and Expected Effects of Compound 376 Alone and Mixtures with **b54.13a** (ethyl 1-[[4-[[2-(trifluoromethyl)-1,3-dioxolan-2-yl]methoxy]phenyl]methyl]-1*H*-pyrazole-4-carboxylate), **b54.12a** (ethyl 1-[[4-[[(1*Z*)-2-ethoxy-3,3,3-trifluoro-1-propen-1-yl]oxy]phenyl]methyl]-1*H*-pyrazole-4-carboxylate), **b54.11a** (methyl *N*-[[5-[1-[2,6-difluoro-4-(1-methylethyl)phenyl]-1*H*-pyrazol-3-yl]-2-methylphenyl]methyl]carbamate), **b54.11d** (methyl *N*-[[5-[1-(4-cyclopropyl-2,6-difluorophenyl)-1*H*-pyrazol-3-yl]-2-methylphenyl]methyl] carbamate) and Picoxystrobin in Controlling Asian Soybean Rust | | | | |
|---|---|---|---|---|
| Application Rate (ppm) of Compound 376 (i.e. Component (a)) | Component (b) | Application Rate (ppm) of Component (b) | Test E | |
| | | | Obsd | Exp |
| 1.228 | picoxystrobin | 20.596 | 80 | 91 |
| 1.228 | picoxystrobin | 33.865 | 89 | 91 |

Table B-1

| Observed and Expected Effects of Compound 316 Alone and Mixtures with Bixafen, Fluxapyroxad and Fluindapyr in Controlling Asian Soybean Rust | | | | |
|---|---|---|---|---|
| Application Rate (ppm) of Compound 316 (i.e. Component (a)) | Component (b) | Application Rate (ppm) of Component (b) | Test E | |
| | | | Obsd | Exp |
| 0 | None | 0 | 0 | - |
| 0.471 | None | 0 | 22 | - |
| 0.764 | None | 0 | 54 | - |
| 0 | bixafen | 11.327 | 54 | - |
| 0 | bixafen | 19.525 | 55 | - |
| 0.471 | bixafen | 11.327 | 60 | 65 |
| 0.471 | bixafen | 19.525 | 65 | 64 |
| 0.764 | bixafen | 11.327 | 60 | 79 |
| 0.764 | bixafen | 19.525 | 73 | 79 |
| 0 | fluxapyroxad | 2.757 | 19 | - |
| 0 | fluxapyroxad | 4.748 | 52 | - |
| 0.471 | fluxapyroxad | 2.757 | 32 | 36 |
| 0.471 | fluxapyroxad | 4.748 | 54 | 62 |
| 0.764 | fluxapyroxad | 2.757 | 59 | 62 |
| 0.764 | fluxapyroxad | 4.748 | 65 | 78 |
| 0 | fluindapyr | 8.820 | 61 | - |
| 0 | fluindapyr | 15.230 | 79 | - |
| 0.471 | fluindapyr | 8.820 | 53 | 70 |
| 0.471 | fluindapyr | 15.230 | 70 | 83 |
| 0.764 | fluindapyr | 8.820 | 74 | 82 |
| 0.764 | fluindapyr | 15.230 | 88 | 90 |

Table B-2

| Observed and Expected Effects of Compound 376 Alone and Mixtures with Bixafen, Fluxapyroxad and Fluindapyr in Controlling Asian Soybean Rust | | | | |
|---|---|---|---|---|
| Application Rate (ppm) of Compound 376 (i.e. Component (a)) | Component (b) | Application Rate (ppm) of Component (b) | Test E | |
| | | | Obsd | Exp |
| 0 | None | 0 | 0 | - |
| 0.753 | None | 0 | 15 | - |
| 1.228 | None | 0 | 21 | - |
| 0 | bixafen | 11.327 | 54 | - |
| 0 | bixafen | 19.525 | 55 | - |
| 0.753 | bixafen | 11.327 | 48 | 62 |
| 0.753 | bixafen | 19.525 | 40 | 61 |
| 1.228 | bixafen | 11.327 | 56 | 64 |
| 1.228 | bixafen | 19.525 | 78 | 63 |
| 0 | fluxapyroxad | 2.757 | 19 | - |
| 0 | fluxapyroxad | 4.748 | 52 | - |
| 0.753 | fluxapyroxad | 2.757 | 33 | 30 |
| 0.753 | fluxapyroxad | 4.748 | 75 | 59 |
| 1.228 | fluxapyroxad | 2.757 | 64 | 35 |
| 1.228 | fluxapyroxad | 4.748 | 72 | 62 |
| 0 | fluindapyr | 8.820 | 61 | - |
| 0 | fluindapyr | 15.230 | 79 | - |
| 0.753 | fluindapyr | 8.820 | 53 | 67 |
| 0.753 | fluindapyr | 15.230 | 51 | 82 |
| 1.228 | fluindapyr | 8.820 | 40 | 69 |
| 1.228 | fluindapyr | 15.230 | 61 | 83 |

Table C-1

| Observed and Expected Effects of Compound 316 Alone and Mixtures with Mancozeb, Fenpropimorph and Tebuconazole in Controlling Asian Soybean Rust | | | | |
|---|---|---|---|---|
| Application Rate (ppm) of Compound 316 (i.e. Component (a)) | Component (b) | Application Rate (ppm) of Component (b) | Test E | |
| | | | Obsd | Exp |
| 0 | None | 0 | 0 | - |
| 0.471 | None | 0 | 49 | - |
| 0.764 | None | 0 | 66 | - |
| 0 | mancozeb | 42.532 | 70 | - |
| 0 | mancozeb | 74.012 | 71 | - |
| 0.471 | mancozeb | 42.532 | 87 | 85 |
| 0.471 | mancozeb | 74.012 | 93 | 85 |

(continued)

| Observed and Expected Effects of Compound 316 Alone and Mixtures with Mancozeb, Fenpropimorph and Tebuconazole in Controlling Asian Soybean Rust | | | | |
|---|---|---|---|---|
| Application Rate (ppm) of Compound 316 (i.e. Component (a)) | Component (b) | Application Rate (ppm) of Component (b) | Test E | |
| | | | Obsd | Exp |
| 0.764 | mancozeb | 42.532 | 96 | 90 |
| 0.764 | mancozeb | 74.012 | 89 | 90 |
| 0 | fenpropimorph | 242.823 | 33 | - |
| 0 | fenpropimorph | 458.587 | 44 | - |
| 0.471 | fenpropimorph | 242.823 | 72 | 66 |
| 0.471 | fenpropimorph | 458.587 | 87 | 72 |
| 0.764 | fenpropimorph | 242.823 | 91 | 77 |
| 0.764 | fenpropimorph | 458.587 | 89 | 81 |
| 0 | tebuconazole | 486.921 | 30 | - |
| 0 | tebuconazole | 841.166 | 55 | - |
| 0.471 | tebuconazole | 486.921 | 62 | 65 |
| 0.471 | tebuconazole | 841.166 | 90 | 77 |
| 0.764 | tebuconazole | 486.921 | 91 | 76 |
| 0.764 | tebuconazole | 841.166 | 96 | 85 |

Table C-2

| Observed and Expected Effects of Compound 376 Alone and Mixtures with Mancozeb, Fenpropimorph and Tebuconazole in Controlling Asian Soybean Rust | | | | |
|---|---|---|---|---|
| Application Rate (ppm) of Compound 376 (i.e. Component (a)) | Component (b) | Application Rate (ppm) of Component (b) | Test E | |
| | | | Obsd | Exp |
| 0 | None | 0 | 0 | - |
| 0.753 | None | 0 | 54 | - |
| 1.228 | None | 0 | 71 | - |
| 0 | mancozeb | 42.532 | 70 | - |
| 0 | mancozeb | 74.012 | 71 | - |
| 0.753 | mancozeb | 42.532 | 86 | 86 |
| 0.753 | mancozeb | 74.012 | 94 | 87 |
| 1.228 | mancozeb | 42.532 | 87 | 91 |
| 1.228 | mancozeb | 74.012 | 96 | 92 |
| 0 | fenpropimorph | 242.823 | 33 | - |
| 0 | fenpropimorph | 458.587 | 44 | - |
| 0.753 | fenpropimorph | 242.823 | 72 | 69 |
| 0.753 | fenpropimorph | 458.587 | 92 | 74 |
| 1.228 | fenpropimorph | 242.823 | 78 | 81 |

(continued)

| Observed and Expected Effects of Compound 376 Alone and Mixtures with Mancozeb, Fenpropimorph and Tebuconazole in Controlling Asian Soybean Rust | | | | |
|---|---|---|---|---|
| Application Rate (ppm) of Compound 376 (i.e. Component (a)) | Component (b) | Application Rate (ppm) of Component (b) | Test E | |
| | | | Obsd | Exp |
| 1.228 | fenpropimorph | 458.587 | 95 | 84 |
| 0 | tebuconazole | 486.921 | 30 | - |
| 0 | tebuconazole | 841.166 | 55 | - |
| 0.753 | tebuconazole | 486.921 | 56 | 68 |
| 0.753 | tebuconazole | 841.166 | 67 | 79 |
| 1.228 | tebuconazole | 486.921 | 75 | 80 |
| 1.228 | tebuconazole | 841.166 | 70 | 87 |

Table D-1

| Observed and Expected Effects of Compound 316 Alone and Mixtures with Cyproconazole, Azoxystrobin and Trifloxystrobin in Controlling Asian Soybean Rust | | | | |
|---|---|---|---|---|
| Application Rate (ppm) of Compound 316 (i.e. Component (a)) | Component (b) | Application Rate (ppm) of Component (b) | Test E | |
| | | | Obsd | Exp |
| 0 | None | 0 | 0 | - |
| 0.471 | None | 0 | 20 | - |
| 0.764 | None | 0 | 70 | - |
| 0 | cyproconazole | 37.333 | 44 | - |
| 0 | cyproconazole | 64.883 | 68 | - |
| 0.471 | cyproconazole | 37.333 | 62 | 55 |
| 0.471 | cyproconazole | 64.883 | 76 | 74 |
| 0.764 | cyproconazole | 37.333 | 85 | 83 |
| 0.764 | cyproconazole | 64.883 | 85 | 90 |
| 0 | azoxystrobin | 95.873 | 70 | - |
| 0 | azoxystrobin | 169.226 | 88 | - |
| 0.471 | azoxystrobin | 95.873 | 86 | 76 |
| 0.471 | azoxystrobin | 169.226 | 88 | 91 |
| 0.764 | azoxystrobin | 95.873 | 84 | 91 |
| 0.764 | azoxystrobin | 169.226 | 86 | 97 |
| 0 | trifloxystrobin | 20.511 | 69 | - |
| 0 | trifloxystrobin | 35.310 | 86 | - |
| 0.471 | trifloxystrobin | 20.511 | 71 | 75 |
| 0.471 | trifloxystrobin | 35.310 | 93 | 89 |
| 0.764 | trifloxystrobin | 20.511 | 83 | 91 |
| 0.764 | trifloxystrobin | 35.310 | 79 | 96 |

Table D-2

| Observed and Expected Effects of Compound 376 Alone and Mixtures with Cyproconazole, Azoxystrobin and Trifloxystrobin in Controlling Asian Soybean Rust | | | | |
|---|---|---|---|---|
| Application Rate (ppm) of Compound 376 (i.e. Component (a)) | Component (b) | Application Rate (ppm) of Component (b) | Test E | |
| | | | Obsd | Exp |
| 0 | None | 0 | 0 | - |
| 0.753 | None | 0 | 67 | - |
| 1.228 | None | 0 | 68 | - |
| 0 | cyproconazole | 37.333 | 44 | - |
| 0 | cyproconazole | 64.883 | 68 | - |
| 0.753 | cyproconazole | 37.333 | 78 | 81 |
| 0.753 | cyproconazole | 64.883 | 62 | 89 |
| 1.228 | cyproconazole | 37.333 | 77 | 82 |
| 1.228 | cyproconazole | 64.883 | 77 | 90 |
| 0 | azoxystrobin | 95.873 | 70 | - |
| 0 | azoxystrobin | 169.226 | 88 | - |
| 0.753 | azoxystrobin | 95.873 | 75 | 90 |
| 0.753 | azoxystrobin | 169.226 | 85 | 96 |
| 1.228 | azoxystrobin | 95.873 | 84 | 90 |
| 1.228 | azoxystrobin | 169.226 | 92 | 96 |
| 0 | trifloxystrobin | 20.511 | 69 | - |
| 0 | trifloxystrobin | 35.310 | 86 | - |
| 0.753 | trifloxystrobin | 20.511 | 61 | 90 |
| 0.753 | trifloxystrobin | 35.310 | 79 | 95 |
| 1.228 | trifloxystrobin | 20.511 | 88 | 90 |
| 1.228 | trifloxystrobin | 35.310 | 88 | 96 |

Table E-1

| Observed and Expected Effects of Compound 316 Alone and Mixtures with Epoxiconazole and Pydiflumetofen in Controlling Asian Soybean Rust | | | | |
|---|---|---|---|---|
| Application Rate (ppm) of Compound 316 (i.e. Component (a)) | Component (b) | Application Rate (ppm) of Component (b) | Test E | |
| | | | Obsd | Exp |
| 0 | None | 0 | 0 | - |
| 0.471 | None | 0 | 61 | - |
| 0.764 | None | 0 | 58 | - |
| 0 | epoxiconazole | 46.795 | 23 | - |
| 0 | epoxiconazole | 89.631 | 70 | - |
| 0.471 | epoxiconazole | 46.795 | 43 | 70 |
| 0.471 | epoxiconazole | 89.631 | 64 | 88 |

(continued)

| Observed and Expected Effects of Compound 316 Alone and Mixtures with Epoxiconazole and Pydiflumetofen in Controlling Asian Soybean Rust | | | | |
|---|---|---|---|---|
| Application Rate (ppm) of Compound 316 (i.e. Component (a)) | Component (b) | Application Rate (ppm) of Component (b) | Test E | |
| | | | Obsd | Exp |
| 0.764 | epoxiconazole | 46.795 | 77 | 67 |
| 0.764 | epoxiconazole | 89.631 | 70 | 87 |
| 0 | pydiflumetofen | 70.950 | 0 | - |
| 0 | pydiflumetofen | 135.850 | 73 | - |
| 0.471 | pydiflumetofen | 70.950 | 52 | 61 |
| 0.471 | pydiflumetofen | 135.850 | 64 | 90 |
| 0.764 | pydiflumetofen | 70.950 | 60 | 58 |
| 0.764 | pydiflumetofen | 135.850 | 72 | 89 |

Table E-2

| Observed and Expected Effects of Compound 376 Alone and Mixtures with Epoxiconazole and Pydiflumetofen in Controlling Asian Soybean Rust | | | | |
|---|---|---|---|---|
| Application Rate (ppm) of Compound 376 (i.e. Component (a)) | Component (b) | Application Rate (ppm) of Component (b) | Test E | |
| | | | Obsd | Exp |
| 0 | None | 0 | 0 | - |
| 0.753 | None | 0 | 81 | - |
| 1.228 | None | 0 | 67 | - |
| 0 | epoxiconazole | 46.795 | 23 | - |
| 0 | epoxiconazole | 89.631 | 70 | - |
| 0.753 | epoxiconazole | 46.795 | 64 | 85 |
| 0.753 | epoxiconazole | 89.631 | 71 | 94 |
| 1.228 | epoxiconazole | 46.795 | 73 | 75 |
| 1.228 | epoxiconazole | 89.631 | 69 | 90 |
| 0 | pydiflumetofen | 70.950 | 0 | - |
| 0 | pydiflumetofen | 135.850 | 73 | - |
| 0.753 | pydiflumetofen | 70.950 | 59 | 81 |
| 0.753 | pydiflumetofen | 135.850 | 66 | 95 |
| 1.228 | pydiflumetofen | 70.950 | 76 | 67 |
| 1.228 | pydiflumetofen | 135.850 | 82 | 91 |

Table F-1

| Observed and Expected Effects of Compound 316 Alone and Mixtures with Benzovindiflupyr, Prothioconazole and Chlorothalonil in Controlling Asian Soybean Rust | | | | |
|---|---|---|---|---|
| Application Rate (ppm) of Compound 316 (i.e. Component (a)) | Component (b) | Application Rate (ppm) of Component (b) | Test E | |
| | | | Obsd | Exp |
| 0 | None | 0 | 0 | - |
| 0.471 | None | 0 | 61 | - |
| 0.764 | None | 0 | 68 | - |
| 0 | benzovindiflupyr | 0.606 | 67 | - |
| 0 | benzovindiflupyr | 0.980 | 67 | - |
| 0.471 | benzovindiflupyr | 0.606 | 80 | 87 |
| 0.471 | benzovindiflupyr | 0.980 | 85 | 87 |
| 0.764 | benzovindiflupyr | 0.606 | 84 | 89 |
| 0.764 | benzovindiflupyr | 0.980 | 91 | 89 |
| 0 | prothioconazole | 10.165 | 7 | - |
| 0 | prothioconazole | 16.339 | 65 | - |
| 0.471 | prothioconazole | 10.165 | 63 | 64 |
| 0.471 | prothioconazole | 16.339 | 88 | 86 |
| 0.764 | prothioconazole | 10.165 | 85 | 70 |
| 0.764 | prothioconazole | 16.339 | 82 | 89 |
| 0 | chlorothalonil | 138.129 | 59 | - |
| 0 | chlorothalonil | 222.081 | 85 | - |
| 0.471 | chlorothalonil | 138.129 | 74 | 84 |
| 0.471 | chlorothalonil | 222.081 | 94 | 94 |
| 0.764 | chlorothalonil | 138.129 | 78 | 87 |
| 0.764 | chlorothalonil | 222.081 | 99 | 95 |

Table F-2

| Observed and Expected Effects of Compound 376 Alone and Mixtures with Benzovindiflupyr, Prothioconazole and Chlorothalonil in Controlling Asian Soybean Rust | | | | |
|---|---|---|---|---|
| Application Rate (ppm) of Compound 376 (i.e. Component (a)) | Component (b) | Application Rate (ppm) of Component (b) | Test E | |
| | | | Obsd | Exp |
| 0 | None | 0 | 0 | - |
| 0.753 | None | 0 | 68 | - |
| 1.228 | None | 0 | 77 | - |
| 0 | benzovindiflupyr | 0.606 | 67 | - |
| 0 | benzovindiflupyr | 0.980 | 67 | - |
| 0.753 | benzovindiflupyr | 0.606 | 82 | 90 |
| 0.753 | benzovindiflupyr | 0.980 | 87 | 90 |
| 1.228 | benzovindiflupyr | 0.606 | 87 | 92 |

(continued)

| Observed and Expected Effects of Compound 376 Alone and Mixtures with Benzovindiflupyr, Prothioconazole and Chlorothalonil in Controlling Asian Soybean Rust | | | | |
|---|---|---|---|---|
| Application Rate (ppm) of Compound 376 (i.e. Component (a)) | Component (b) | Application Rate (ppm) of Component (b) | Test E | |
| | | | Obsd | Exp |
| 1.228 | benzovindiflupyr | 0.980 | 87 | 93 |
| 0 | prothioconazole | 10.165 | 7 | - |
| 0 | prothioconazole | 16.339 | 65 | - |
| 0.753 | prothioconazole | 10.165 | 60 | 70 |
| 0.753 | prothioconazole | 16.339 | 82 | 89 |
| 1.228 | prothioconazole | 10.165 | 72 | 79 |
| 1.228 | prothioconazole | 16.339 | 85 | 92 |
| 0 | chlorothalonil | 138.129 | 59 | - |
| 0 | chlorothalonil | 222.081 | 85 | - |
| 0.753 | chlorothalonil | 138.129 | 75 | 87 |
| 0.753 | chlorothalonil | 222.081 | 96 | 95 |
| 1.228 | chlorothalonil | 138.129 | 87 | 91 |
| 1.228 | chlorothalonil | 222.081 | 92 | 97 |

Table G-1

| Observed and Expected Effects of Compound 316 Alone and Mixtures with Pydiflumetofen, Pyraclostrobin and Metominostrobin in Controlling Asian Soybean Rust | | | | |
|---|---|---|---|---|
| Application Rate (ppm) of Compound 316 (i.e. Component (a)) | Component (b) | Application Rate (ppm) of Component (b) | Test E | |
| | | | Obsd | Exp |
| 0 | None | 0 | 0 | - |
| 0.471 | None | 0 | 31 | - |
| 0.764 | None | 0 | 64 | - |
| 0 | pydiflumetofen | 41.364 | 49 | - |
| 0 | pydiflumetofen | 128.793 | 36 | - |
| 0.471 | pydiflumetofen | 41.364 | 34 | 65 |
| 0.471 | pydiflumetofen | 128.793 | 70 | 56 |
| 0.764 | pydiflumetofen | 41.364 | 75 | 81 |
| 0.764 | pydiflumetofen | 128.793 | 77 | 77 |
| 0 | pyraclostrobin | 45.860 | 35 | - |
| 0 | pyraclostrobin | 137.503 | 45 | - |
| 0.471 | pyraclostrobin | 45.860 | 57 | 55 |
| 0.471 | pyraclostrobin | 137.503 | 68 | 62 |
| 0.764 | pyraclostrobin | 45.860 | 62 | 76 |
| 0.764 | pyraclostrobin | 137.503 | 75 | 80 |
| 0 | metominostrobin | 29.673 | 13 | - |

(continued)

| Observed and Expected Effects of Compound 316 Alone and Mixtures with Pydiflumetofen, Pyraclostrobin and Metominostrobin in Controlling Asian Soybean Rust | | | | |
|---|---|---|---|---|
| Application Rate (ppm) of Compound 316 (i.e. Component (a)) | Component (b) | Application Rate (ppm) of Component (b) | Test E | |
| | | | Obsd | Exp |
| 0 | metominostrobin | 86.003 | 37 | - |
| 0.471 | metominostrobin | 29.673 | 49 | 40 |
| 0.471 | metominostrobin | 86.003 | 75 | 56 |
| 0.764 | metominostrobin | 29.673 | 28 | 69 |
| 0.764 | metominostrobin | 86.003 | 61 | 77 |

Table G-2

| Observed and Expected Effects of Compound 376 Alone Alone and Mixtures with Pydiflumetofen, Pyraclostrobin and Metominostrobin in Controlling Asian Soybean Rust | | | | |
|---|---|---|---|---|
| Application Rate (ppm) of Compound 376 (i.e. Component (a)) | Component (b) | Application Rate (ppm) of Component (b) | Test E | |
| | | | Obsd | Exp |
| 0 | None | 0 | 0 | - |
| 0.753 | None | 0 | 52 | - |
| 1.228 | None | 0 | 37 | - |
| 0 | pydiflumetofen | 41.364 | 49 | - |
| 0 | pydiflumetofen | 128.793 | 36 | - |
| 0.753 | pydiflumetofen | 41.364 | 36 | 75 |
| 0.753 | pydiflumetofen | 128.793 | 58 | 69 |
| 1.228 | pydiflumetofen | 41.364 | 53 | 68 |
| 1.228 | pydiflumetofen | 128.793 | 49 | 60 |
| 0 | pyraclostrobin | 45.860 | 35 | - |
| 0 | pyraclostrobin | 137.503 | 45 | - |
| 0.753 | pyraclostrobin | 45.860 | 2 | 69 |
| 0.753 | pyraclostrobin | 137.503 | 34 | 73 |
| 1.228 | pyraclostrobin | 45.860 | 23 | 59 |
| 1.228 | pyraclostrobin | 137.503 | 46 | 65 |
| 0 | metominostrobin | 29.673 | 13 | - |
| 0 | metominostrobin | 86.003 | 37 | - |
| 0.753 | metominostrobin | 29.673 | 14 | 58 |
| 0.753 | metominostrobin | 86.003 | 57 | 70 |
| 1.228 | metominostrobin | 29.673 | 45 | 45 |
| 1.228 | metominostrobin | 86.003 | 73 | 60 |

Table H-1

| Observed and Expected Effects of Compound 316 Alone and Mixtures with Copper Hydroxide, Flutriafol and Fenpropidin in Controlling Asian Soybean Rust | | | | |
|---|---|---|---|---|
| Application Rate (ppm) of Compound 316 (i.e. Component (a)) | Component (b) | Application Rate (ppm) of Component (b) | Test E | |
| | | | Obsd | Exp |
| 0 | None | 0 | 0 | - |
| 0.471 | None | 0 | 44 | - |
| 0.764 | None | 0 | 80 | - |
| 0 | copper hydroxide | 3610.778 | 87 | - |
| 0 | copper hydroxide | 7371.507 | 65 | - |
| 0.471 | copper hydroxide | 3610.778 | 82 | 92 |
| 0.471 | copper hydroxide | 7371.507 | 82 | 80 |
| 0.764 | copper hydroxide | 3610.778 | 86 | 97 |
| 0.764 | copper hydroxide | 7371.507 | 81 | 93 |
| 0 | flutriafol | 544.265 | 13 | - |
| 0 | flutriafol | 1124.021 | 52 | - |
| 0.471 | flutriafol | 544.265 | 64 | 51 |
| 0.471 | flutriafol | 1124.021 | 66 | 73 |
| 0.764 | flutriafol | 544.265 | 67 | 83 |
| 0.764 | flutriafol | 1124.021 | 88 | 90 |
| 0 | fenpropidin | 78.393 | 45 | - |
| 0 | fenpropidin | 161.740 | 68 | - |
| 0.471 | fenpropidin | 78.393 | 66 | 69 |
| 0.471 | fenpropidin | 161.740 | 75 | 82 |
| 0.764 | fenpropidin | 78.393 | 63 | 89 |
| 0.764 | fenpropidin | 161.740 | 87 | 94 |

Table H-2

| Observed and Expected Effects of Compound 376 Alone Alone and Mixtures with Copper Hydroxide, Flutriafol and Fenpropidin in Controlling Asian Soybean Rust | | | | |
|---|---|---|---|---|
| Application Rate (ppm) of Compound 376 (i.e. Component (a)) | Component (b) | Application Rate (ppm) of Component (b) | Test E | |
| | | | Obsd | Exp |
| 0 | None | 0 | 0 | - |
| 0.753 | None | 0 | 62 | - |
| 1.228 | None | 0 | 81 | - |
| 0 | copper hydroxide | 3610.778 | 87 | - |
| 0 | copper hydroxide | 7371.507 | 65 | - |
| 0.753 | copper hydroxide | 3610.778 | 84 | 95 |
| 0.753 | copper hydroxide | 7371.507 | 87 | 87 |

(continued)

| Observed and Expected Effects of Compound 376 Alone Alone and Mixtures with Copper Hydroxide, Flutriafol and Fenpropidin in Controlling Asian Soybean Rust | | | | |
|---|---|---|---|---|
| Application Rate (ppm) of Compound 376 (i.e. Component (a)) | Component (b) | Application Rate (ppm) of Component (b) | Test E | |
| | | | Obsd | Exp |
| 1.228 | copper hydroxide | 3610.778 | 90 | 97 |
| 1.228 | copper hydroxide | 7371.507 | 87 | 93 |
| 0 | flutriafol | 544.265 | 13 | - |
| 0 | flutriafol | 1124.021 | 52 | - |
| 0.753 | flutriafol | 544.265 | 68 | 67 |
| 0.753 | flutriafol | 1124.021 | 76 | 82 |
| 1.228 | flutriafol | 544.265 | 75 | 83 |
| 1.228 | flutriafol | 1124.021 | 88 | 91 |
| 0 | fenpropidin | 78.393 | 45 | - |
| 0 | fenpropidin | 161.740 | 68 | - |
| 0.753 | fenpropidin | 78.393 | 78 | 79 |
| 0.753 | fenpropidin | 161.740 | 82 | 88 |
| 1.228 | fenpropidin | 78.393 | 85 | 89 |
| 1.228 | fenpropidin | 161.740 | 89 | 94 |

Table I-1

| Observed and Expected Effects of Compound 225 Alone and Mixtures with Benzovindiflupyr, Prothioconazole and Chlorothalonil in Controlling Asian Soybean Rust | | | | |
|---|---|---|---|---|
| Application Rate (ppm) of Compound 225 (i.e. Component (a)) | Component (b) | Application Rate (ppm) of Component (b) | Test E | |
| | | | Obsd | Exp |
| 0 | None | 0 | 0 | - |
| 0.292 | None | 0 | 19 | - |
| 0.589 | None | 0 | 68 | - |
| 0 | benzovindiflupyr | 0.606 | 51 | - |
| 0 | benzovindiflupyr | 0.980 | 68 | - |
| 0.292 | benzovindiflupyr | 0.606 | 77 | 74 |
| 0.292 | benzovindiflupyr | 0.980 | 87 | 60 |
| 0.589 | benzovindiflupyr | 0.606 | 75 | 90 |
| 0.589 | benzovindiflupyr | 0.980 | 84 | 84 |
| 0 | prothioconazole | 10.165 | 34 | - |
| 0 | prothioconazole | 16.339 | 56 | - |
| 0.292 | prothioconazole | 10.165 | 44 | 46 |
| 0.292 | prothioconazole | 16.339 | 65 | 64 |
| 0.589 | prothioconazole | 10.165 | 81 | 79 |
| 0.589 | prothioconazole | 16.339 | 79 | 86 |

(continued)

| Observed and Expected Effects of Compound 225 Alone and Mixtures with Benzovindiflupyr, Prothioconazole and Chlorothalonil in Controlling Asian Soybean Rust | | | | |
|---|---|---|---|---|
| Application Rate (ppm) of Compound 225 (i.e. Component (a)) | Component (b) | Application Rate (ppm) of Component (b) | Test E | |
| | | | Obsd | Exp |
| 0 | chlorothalonil | 138.129 | 76 | - |
| 0 | chlorothalonil | 222.081 | 88 | - |
| 0.292 | chlorothalonil | 138.129 | 87 | 81 |
| 0.292 | chlorothalonil | 222.081 | 87 | 90 |
| 0.589 | chlorothalonil | 138.129 | 80 | 92 |
| 0.589 | chlorothalonil | 222.081 | 85 | 96 |

Table 1-2

| Observed and Expected Effects of Compound 229 Alone and Mixtures with Benzovindiflupyr, Prothioconazole and Chlorothalonil in Controlling Asian Soybean Rust | | | | |
|---|---|---|---|---|
| Application Rate (ppm) of Compound 229 (i.e. Component (a)) | Component (b) | Application Rate (ppm) of Component (b) | Test E | |
| | | | Obsd | Exp |
| 0 | None | 0 | 0 | - |
| 0.964 | None | 0 | 59 | - |
| 1.915 | None | 0 | 77 | - |
| 0 | benzovindiflupyr | 0.606 | 51 | - |
| 0 | benzovindiflupyr | 0.980 | 68 | - |
| 0.964 | benzovindiflupyr | 0.606 | 81 | 92 |
| 0.964 | benzovindiflupyr | 0.980 | 83 | 89 |
| 1.915 | benzovindiflupyr | 0.606 | 87 | 87 |
| 1.915 | benzovindiflupyr | 0.980 | 89 | 80 |
| 0 | prothioconazole | 10.165 | 34 | - |
| 0 | prothioconazole | 16.339 | 56 | - |
| 0.964 | prothioconazole | 10.165 | 70 | 84 |
| 0.964 | prothioconazole | 16.339 | 68 | 90 |
| 1.915 | prothioconazole | 10.165 | 73 | 73 |
| 1.915 | prothioconazole | 16.339 | 74 | 82 |
| 0 | chlorothalonil | 138.129 | 76 | - |
| 0 | chlorothalonil | 222.081 | 88 | - |
| 0.964 | chlorothalonil | 138.129 | 76 | 94 |
| 0.964 | chlorothalonil | 222.081 | 89 | 97 |
| 1.915 | chlorothalonil | 138.129 | 66 | 90 |
| 1.915 | chlorothalonil | 222.081 | 93 | 95 |

Table I-3

| Observed and Expected Effects of Compound 257 Alone and Mixtures with Benzovindiflupyr, Prothioconazole and Chlorothalonil in Controlling Asian Soybean Rust | | | | |
|---|---|---|---|---|
| Application Rate (ppm) of Compound 257 (i.e. Component (a)) | Component (b) | Application Rate (ppm) of Component (b) | Test E | |
| | | | Obsd | Exp |
| 0 | None | 0 | 0 | - |
| 0.914 | None | 0 | 27 | - |
| 1.828 | None | 0 | 62 | - |
| 0 | benzovindiflupyr | 0.606 | 51 | - |
| 0 | benzovindiflupyr | 0.980 | 68 | - |
| 0.914 | benzovindiflupyr | 0.606 | 72 | 76 |
| 0.914 | benzovindiflupyr | 0.980 | 70 | 64 |
| 1.828 | benzovindiflupyr | 0.606 | 86 | 88 |
| 1.828 | benzovindiflupyr | 0.980 | 85 | 82 |
| 0 | prothioconazole | 10.165 | 34 | - |
| 0 | prothioconazole | 16.339 | 56 | - |
| 0.914 | prothioconazole | 10.165 | 50 | 52 |
| 0.914 | prothioconazole | 16.339 | 73 | 68 |
| 1.828 | prothioconazole | 10.165 | 70 | 75 |
| 1.828 | prothioconazole | 16.339 | 69 | 83 |
| 0 | chlorothalonil | 138.129 | 76 | - |
| 0 | chlorothalonil | 222.081 | 88 | - |
| 0.914 | chlorothalonil | 138.129 | 73 | 82 |
| 0.914 | chlorothalonil | 222.081 | 86 | 91 |
| 1.828 | chlorothalonil | 138.129 | 87 | 91 |
| 1.828 | chlorothalonil | 222.081 | 85 | 96 |

Table I-4

| Observed and Expected Effects of Compound 394 Alone and Mixtures with Benzovindiflupyr, Prothioconazole and Chlorothalonil in Controlling Asian Soybean Rust | | | | |
|---|---|---|---|---|
| Application Rate (ppm) of Compound 394 (i.e. Component (a)) | Component (b) | Application Rate (ppm) of Component (b) | Test E | |
| | | | Obsd | Exp |
| 0 | None | 0 | 0 | - |
| 0.490 | None | 0 | 73 | - |
| 0 | benzovindiflupyr | 0.606 | 51 | - |
| 0 | benzovindiflupyr | 0.980 | 68 | - |
| 0.490 | benzovindiflupyr | 0.606 | 76 | 68 |
| 0.490 | benzovindiflupyr | 0.980 | 91 | 51 |
| 0 | prothioconazole | 10.165 | 34 | - |
| 0 | prothioconazole | 16.339 | 56 | - |

(continued)

| Observed and Expected Effects of Compound 394 Alone and Mixtures with Benzovindiflupyr, Prothioconazole and Chlorothalonil in Controlling Asian Soybean Rust | | | | |
|---|---|---|---|---|
| Application Rate (ppm) of Compound 394 (i.e. Component (a)) | Component (b) | Application Rate (ppm) of Component (b) | Test E | |
| | | | Obsd | Exp |
| 0.490 | prothioconazole | 10.165 | 69 | 34 |
| 0.490 | prothioconazole | 16.339 | 81 | 56 |
| 0 | chlorothalonil | 138.129 | 76 | - |
| 0 | chlorothalonil | 222.081 | 88 | - |
| 0.490 | chlorothalonil | 138.129 | 55 | 76 |
| 0.490 | chlorothalonil | 222.081 | 81 | 88 |

Table I-5

| Observed and Expected Effects of Compound 506 Alone and Mixtures with Benzovindiflupyr, Prothioconazole and Chlorothalonil in Controlling Asian Soybean Rust | | | | |
|---|---|---|---|---|
| Application Rate (ppm) of Compound 506 (i.e. Component (a)) | Component (b) | Application Rate (ppm) of Component (b) | Test E | |
| | | | Obsd | Exp |
| 0 | None | 0 | 0 | - |
| 0.790 | None | 0 | 27 | - |
| 1.585 | None | 0 | 34 | - |
| 0 | benzovindiflupyr | 0.606 | 51 | - |
| 0 | benzovindiflupyr | 0.980 | 68 | - |
| 0.790 | benzovindiflupyr | 0.606 | 76 | 79 |
| 0.790 | benzovindiflupyr | 0.980 | 91 | 68 |
| 1.585 | benzovindiflupyr | 0.606 | 74 | 77 |
| 1.585 | benzovindiflupyr | 0.980 | 85 | 64 |
| 0 | prothioconazole | 10.165 | 34 | - |
| 0 | prothioconazole | 16.339 | 56 | - |
| 0.790 | prothioconazole | 10.165 | 74 | 56 |
| 0.790 | prothioconazole | 16.339 | 74 | 71 |
| 1.585 | prothioconazole | 10.165 | 58 | 52 |
| 1.585 | prothioconazole | 16.339 | 61 | 68 |
| 0 | chlorothalonil | 138.129 | 76 | - |
| 0 | chlorothalonil | 222.081 | 88 | - |
| 0.790 | chlorothalonil | 138.129 | 88 | 84 |
| 0.790 | chlorothalonil | 222.081 | 90 | 92 |
| 1.585 | chlorothalonil | 138.129 | 85 | 83 |
| 1.585 | chlorothalonil | 222.081 | 87 | 91 |

Table I-6

| Observed and Expected Effects of Compound 507 Alone and Mixtures with Benzovindiflupyr, Prothioconazole and Chlorothalonil in Controlling Asian Soybean Rust | | | | |
|---|---|---|---|---|
| Application Rate (ppm) of Compound 507 (i.e. Component (a)) | Component (b) | Application Rate (ppm) of Component (b) | Test E | |
| | | | Obsd | Exp |
| 0 | None | 0 | 0 | - |
| 0.896 | None | 0 | 57 | - |
| 1.823 | None | 0 | 74 | - |
| 0 | benzovindiflupyr | 0.606 | 51 | - |
| 0 | benzovindiflupyr | 0.980 | 68 | - |
| 0.896 | benzovindiflupyr | 0.606 | 76 | 86 |
| 0.896 | benzovindiflupyr | 0.980 | 80 | 79 |
| 1.823 | benzovindiflupyr | 0.606 | 80 | 92 |
| 1.823 | benzovindiflupyr | 0.980 | 89 | 87 |
| 0 | prothioconazole | 10.165 | 34 | - |
| 0 | prothioconazole | 16.339 | 56 | - |
| 0.896 | prothioconazole | 10.165 | 54 | 71 |
| 0.896 | prothioconazole | 16.339 | 82 | 81 |
| 1.823 | prothioconazole | 10.165 | 76 | 83 |
| 1.823 | prothioconazole | 16.339 | 81 | 88 |
| 0 | chlorothalonil | 138.129 | 76 | - |
| 0 | chlorothalonil | 222.081 | 88 | - |
| 0.896 | chlorothalonil | 138.129 | 77 | 90 |
| 0.896 | chlorothalonil | 222.081 | 89 | 95 |
| 1.823 | chlorothalonil | 138.129 | 89 | 94 |
| 1.823 | chlorothalonil | 222.081 | 87 | 97 |

Table I-7

| Observed and Expected Effects of Compound 508 Alone and Mixtures with Benzovindiflupyr, Prothioconazole and Chlorothalonil in Controlling Asian Soybean Rust | | | | |
|---|---|---|---|---|
| Application Rate (ppm) of Compound 508 (i.e. Component (a)) | Component (b) | Application Rate (ppm) of Component (b) | Test E | |
| | | | Obsd | Exp |
| 0 | None | 0 | 0 | - |
| 0.855 | None | 0 | 23 | - |
| 1.716 | None | 0 | 60 | - |
| 0 | benzovindiflupyr | 0.606 | 51 | - |
| 0 | benzovindiflupyr | 0.980 | 68 | - |
| 0.855 | benzovindiflupyr | 0.606 | 80 | 75 |
| 0.855 | benzovindiflupyr | 0.980 | 83 | 62 |
| 1.716 | benzovindiflupyr | 0.606 | 74 | 87 |

(continued)

| Observed and Expected Effects of Compound 508 Alone and Mixtures with Benzovindiflupyr, Prothioconazole and Chlorothalonil in Controlling Asian Soybean Rust | | | | |
|---|---|---|---|---|
| Application Rate (ppm) of Compound 508 (i.e. Component (a)) | Component (b) | Application Rate (ppm) of Component (b) | Test E | |
| | | | Obsd | Exp |
| 1.716 | benzovindiflupyr | 0.980 | 85 | 80 |
| 0 | prothioconazole | 10.165 | 34 | - |
| 0 | prothioconazole | 16.339 | 56 | - |
| 0.855 | prothioconazole | 10.165 | 60 | 49 |
| 0.855 | prothioconazole | 16.339 | 58 | 66 |
| 1.716 | prothioconazole | 10.165 | 67 | 73 |
| 1.716 | prothioconazole | 16.339 | 77 | 82 |
| 0 | chlorothalonil | 138.129 | 76 | - |
| 0 | chlorothalonil | 222.081 | 88 | - |
| 0.855 | chlorothalonil | 138.129 | 85 | 81 |
| 0.855 | chlorothalonil | 222.081 | 91 | 91 |
| 1.716 | chlorothalonil | 138.129 | 78 | 90 |
| 1.716 | chlorothalonil | 222.081 | 83 | 95 |

Table I-8

| Observed and Expected Effects of Compound 509 Alone and Mixtures with Benzovindiflupyr, Prothioconazole and Chlorothalonil in Controlling Asian Soybean Rust | | | | |
|---|---|---|---|---|
| Application Rate (ppm) of Compound 509 (i.e. Component (a)) | Component (b) | Application Rate (ppm) of Component (b) | Test E | |
| | | | Obsd | Exp |
| 0 | None | 0 | 0 | - |
| 2.796 | None | 0 | 54 | - |
| 5.625 | None | 0 | 35 | - |
| 0 | benzovindiflupyr | 0.606 | 51 | - |
| 0 | benzovindiflupyr | 0.980 | 68 | - |
| 2.796 | benzovindiflupyr | 0.606 | 80 | 79 |
| 2.796 | benzovindiflupyr | 0.980 | 62 | 68 |
| 5.625 | benzovindiflupyr | 0.606 | 68 | 85 |
| 5.625 | benzovindiflupyr | 0.980 | 79 | 77 |
| 0 | prothioconazole | 10.165 | 34 | - |
| 0 | prothioconazole | 16.339 | 56 | - |
| 2.796 | prothioconazole | 10.165 | 32 | 57 |
| 2.796 | prothioconazole | 16.339 | 45 | 71 |
| 5.625 | prothioconazole | 10.165 | 61 | 69 |
| 5.625 | prothioconazole | 16.339 | 74 | 80 |
| 0 | chlorothalonil | 138.129 | 76 | - |

(continued)

| Observed and Expected Effects of Compound 509 Alone and Mixtures with Benzovindiflupyr, Prothioconazole and Chlorothalonil in Controlling Asian Soybean Rust | | | | |
|---|---|---|---|---|
| Application Rate (ppm) of Compound 509 (i.e. Component (a)) | Component (b) | Application Rate (ppm) of Component (b) | Test E | |
| | | | Obsd | Exp |
| 0 | chlorothalonil | 222.081 | 88 | - |
| 2.796 | chlorothalonil | 138.129 | 74 | 84 |
| 2.796 | chlorothalonil | 222.081 | 87 | 92 |
| 5.625 | chlorothalonil | 138.129 | 76 | 89 |
| 5.625 | chlorothalonil | 222.081 | 77 | 95 |

Table I-9

| Observed and Expected Effects of Compound 511 Alone and Mixtures with Benzovindiflupyr, Prothioconazole and Chlorothalonil in Controlling Asian Soybean Rust | | | | |
|---|---|---|---|---|
| Application Rate (ppm) of Compound 511 (i.e. Component (a)) | Component (b) | Application Rate (ppm) of Component (b) | Test E | |
| | | | Obsd | Exp |
| 0 | None | 0 | 0 | - |
| 2.436 | None | 0 | 40 | - |
| 4.926 | None | 0 | 66 | - |
| 0 | benzovindiflupyr | 0.606 | 51 | - |
| 0 | benzovindiflupyr | 0.980 | 68 | - |
| 2.436 | benzovindiflupyr | 0.606 | 66 | 81 |
| 2.436 | benzovindiflupyr | 0.980 | 77 | 71 |
| 4.926 | benzovindiflupyr | 0.606 | 79 | 89 |
| 4.926 | benzovindiflupyr | 0.980 | 82 | 84 |
| 0 | prothioconazole | 10.165 | 34 | - |
| 0 | prothioconazole | 16.339 | 56 | - |
| 2.436 | prothioconazole | 10.165 | 60 | 60 |
| 2.436 | prothioconazole | 16.339 | 79 | 74 |
| 4.926 | prothioconazole | 10.165 | 77 | 78 |
| 4.926 | prothioconazole | 16.339 | 67 | 85 |
| 0 | chlorothalonil | 138.129 | 76 | - |
| 0 | chlorothalonil | 222.081 | 88 | - |
| 2.436 | chlorothalonil | 138.129 | 87 | 86 |
| 2.436 | chlorothalonil | 222.081 | 89 | 93 |
| 4.926 | chlorothalonil | 138.129 | 74 | 92 |
| 4.926 | chlorothalonil | 222.081 | 91 | 96 |

Table 1-10

| Observed and Expected Effects of Compound 512 Alone and Mixtures with Benzovindiflupyr, Prothioconazole and Chlorothalonil in Controlling Asian Soybean Rust | | | | |
|---|---|---|---|---|
| Application Rate (ppm) of Compound 512 (i.e. Component (a)) | Component (b) | Application Rate (ppm) of Component (b) | Test E | |
| | | | Obsd | Exp |
| 0 | None | 0 | 0 | - |
| 1.893 | None | 0 | 14 | - |
| 3.829 | None | 0 | 66 | - |
| 0 | benzovindiflupyr | 0.606 | 51 | - |
| 0 | benzovindiflupyr | 0.980 | 68 | - |
| 1.893 | benzovindiflupyr | 0.606 | 66 | 72 |
| 1.893 | benzovindiflupyr | 0.980 | 82 | 58 |
| 3.829 | benzovindiflupyr | 0.606 | 86 | 89 |
| 3.829 | benzovindiflupyr | 0.980 | 87 | 83 |
| 0 | prothioconazole | 10.165 | 34 | - |
| 0 | prothioconazole | 16.339 | 56 | - |
| 1.893 | prothioconazole | 10.165 | 64 | 43 |
| 1.893 | prothioconazole | 16.339 | 83 | 62 |
| 3.829 | prothioconazole | 10.165 | 65 | 78 |
| 3.829 | prothioconazole | 16.339 | 87 | 85 |
| 0 | chlorothalonil | 138.129 | 76 | - |
| 0 | chlorothalonil | 222.081 | 88 | - |
| 1.893 | chlorothalonil | 138.129 | 86 | 79 |
| 1.893 | chlorothalonil | 222.081 | 90 | 90 |
| 3.829 | chlorothalonil | 138.129 | 88 | 92 |
| 3.829 | chlorothalonil | 222.081 | 89 | 96 |

Table J-1

| Observed and Expected Effects of Compound 225 Alone and Mixtures with with Copper Hydroxide, Fenpropidin and Picoxystrobin in Controlling Asian Soybean Rust | | | | |
|---|---|---|---|---|
| Application Rate (ppm) of Compound 225 (i.e. Component (a)) | Component (b) | Application Rate (ppm) of Component (b) | Test E | |
| | | | Obsd | Exp |
| 0 | None | 0 | 0 | - |
| 0.292 | None | 0 | 0 | - |
| 0.589 | None | 0 | 55 | - |
| 0 | copper hydroxide | 3610.778 | 80 | - |
| 0 | copper hydroxide | 7371.507 | 73 | - |
| 0.292 | copper hydroxide | 3610.778 | 81 | 80 |

(continued)

| Observed and Expected Effects of Compound 225 Alone and Mixtures with with Copper Hydroxide, Fenpropidin and Picoxystrobin in Controlling Asian Soybean Rust | | | | |
|---|---|---|---|---|
| Application Rate (ppm) of Compound 225 (i.e. Component (a)) | Component (b) | Application Rate (ppm) of Component (b) | Test E | |
| | | | Obsd | Exp |
| 0.292 | copper hydroxide | 7371.507 | 71 | 73 |
| 0.589 | copper hydroxide | 3610.778 | 81 | 91 |
| 0.589 | copper hydroxide | 7371.507 | 72 | 88 |
| 0 | fenpropidin | 78.393 | 0 | - |
| 0 | fenpropidin | 161.740 | 52 | - |
| 0.292 | fenpropidin | 78.393 | 54 | 0 |
| 0.292 | fenpropidin | 161.740 | 70 | 52 |
| 0.589 | fenpropidin | 78.393 | 73 | 55 |
| 0.589 | fenpropidin | 161.740 | 76 | 79 |
| 0 | picoxystrobin | 20.596 | 28 | - |
| 0 | picoxystrobin | 33.865 | 43 | - |
| 0.292 | picoxystrobin | 20.596 | 39 | 28 |
| 0.292 | picoxystrobin | 33.865 | 76 | 43 |
| 0.589 | picoxystrobin | 20.596 | 59 | 68 |
| 0.589 | picoxystrobin | 33.865 | 77 | 74 |

Table J-2

| Observed and Expected Effects of Compound 229 Alone and Mixtures with Copper Hydroxide, Fenpropidin and Picoxystrobin in Controlling Asian Soybean Rust | | | | |
|---|---|---|---|---|
| Application Rate (ppm) of Compound 229 (i.e. Component (a)) | Component (b) | Application Rate (ppm) of Component (b) | Test E | |
| | | | Obsd | Exp |
| 0 | None | 0 | 0 | - |
| 0.964 | None | 0 | 63 | - |
| 1.915 | None | 0 | 73 | - |
| 0 | copper hydroxide | 3610.778 | 80 | - |
| 0 | copper hydroxide | 7371.507 | 73 | - |
| 0.964 | copper hydroxide | 3610.778 | 75 | 92 |
| 0.964 | copper hydroxide | 7371.507 | 76 | 90 |
| 1.915 | copper hydroxide | 3610.778 | 87 | 95 |
| 1.915 | copper hydroxide | 7371.507 | 78 | 93 |
| 0 | fenpropidin | 78.393 | 0 | - |
| 0 | fenpropidin | 161.740 | 52 | - |

(continued)

| Observed and Expected Effects of Compound 229 Alone and Mixtures with Copper Hydroxide, Fenpropidin and Picoxystrobin in Controlling Asian Soybean Rust | | | | |
|---|---|---|---|---|
| Application Rate (ppm) of Compound 229 (i.e. Component (a)) | Component (b) | Application Rate (ppm) of Component (b) | Test E | |
| | | | Obsd | Exp |
| 0.964 | fenpropidin | 78.393 | 67 | 63 |
| 0.964 | fenpropidin | 161.740 | 77 | 82 |
| 1.915 | fenpropidin | 78.393 | 78 | 73 |
| 1.915 | fenpropidin | 161.740 | 89 | 87 |
| 0 | picoxystrobin | 20.596 | 28 | - |
| 0 | picoxystrobin | 33.875 | 43 | - |
| 0.964 | picoxystrobin | 20.596 | 67 | 73 |
| 0.964 | picoxystrobin | 33.875 | 87 | 78 |
| 1.915 | picoxystrobin | 20.596 | 80 | 81 |
| 1.915 | picoxystrobin | 33.875 | 90 | 85 |

Table J-3

| Observed and Expected Effects of Compound 257 Alone and Mixtures with Copper Hydroxide, Fenpropidin and Picoxystrobin in Controlling Asian Soybean Rust | | | | |
|---|---|---|---|---|
| Application Rate (ppm) of Compound 257 (i.e. Component (a)) | Component (b) | Application Rate (ppm) of Component (b) | Test E | |
| | | | Obsd | Exp |
| 0 | None | 0 | 0 | - |
| 0.914 | None | 0 | 36 | - |
| 1.828 | None | 0 | 41 | - |
| 0 | copper hydroxide | 3610.778 | 80 | - |
| 0 | copper hydroxide | 7371.507 | 73 | - |
| 0.914 | copper hydroxide | 3610.778 | 79 | 87 |
| 0.914 | copper hydroxide | 7371.507 | 77 | 83 |
| 1.828 | copper hydroxide | 3610.778 | 85 | 88 |
| 1.828 | copper hydroxide | 7371.507 | 85 | 84 |
| 0 | fenpropidin | 78.393 | 0 | - |
| 0 | fenpropidin | 161.740 | 52 | - |
| 0.914 | fenpropidin | 78.393 | 55 | 36 |
| 0.914 | fenpropidin | 161.740 | 83 | 69 |
| 1.828 | fenpropidin | 78.393 | 0 | 41 |
| 1.828 | fenpropidin | 161.740 | 76 | 72 |
| 0 | picoxystrobin | 20.596 | 28 | - |
| 0 | picoxystrobin | 33.865 | 43 | - |
| 0.914 | picoxystrobin | 20.596 | 60 | 54 |

(continued)

| Observed and Expected Effects of Compound 257 Alone and Mixtures with Copper Hydroxide, Fenpropidin and Picoxystrobin in Controlling Asian Soybean Rust | | | | |
|---|---|---|---|---|
| Application Rate (ppm) of Compound 257 (i.e. Component (a)) | Component (b) | Application Rate (ppm) of Component (b) | Test E | |
| | | | Obsd | Exp |
| 0.914 | picoxystrobin | 33.865 | 62 | 63 |
| 1.828 | picoxystrobin | 20.596 | 66 | 58 |
| 1.828 | picoxystrobin | 33.865 | 70 | 66 |

Table J-4

| Observed and Expected Effects of Compound 394 Alone and Mixtures with Copper Hydroxide, Fenpropidin and Picoxystrobin in Controlling Asian Soybean Rust | | | | |
|---|---|---|---|---|
| Application Rate (ppm) of Compound 394 (i.e. Component (a)) | Component (b) | Application Rate (ppm) of Component (b) | Test E | |
| | | | Obsd | Exp |
| 0 | None | 0 | 0 | - |
| 0.241 | None | 0 | 4 | - |
| 0.490 | None | 0 | 0 | - |
| 0 | copper hydroxide | 3610.778 | 80 | - |
| 0 | copper hydroxide | 7371.507 | 73 | - |
| 0.241 | copper hydroxide | 3610.778 | 85 | 80 |
| 0.241 | copper hydroxide | 7371.507 | 71 | 74 |
| 0.490 | copper hydroxide | 3610.778 | 77 | 80 |
| 0.490 | copper hydroxide | 7371.507 | 72 | 73 |
| 0 | fenpropidin | 78.393 | 0 | - |
| 0 | fenpropidin | 161.740 | 52 | - |
| 0.241 | fenpropidin | 78.393 | 9 | 4 |
| 0.241 | fenpropidin | 161.740 | 34 | 54 |
| 0.490 | fenpropidin | 78.393 | 20 | 0 |
| 0.490 | fenpropidin | 161.740 | 71 | 52 |
| 0 | picoxystrobin | 20.596 | 28 | - |
| 0 | picoxystrobin | 33.865 | 43 | - |
| 0.241 | picoxystrobin | 20.596 | 22 | 31 |
| 0.241 | picoxystrobin | 33.865 | 85 | 45 |
| 0.490 | picoxystrobin | 20.596 | 14 | 28 |
| 0.490 | picoxystrobin | 33.865 | 66 | 43 |

Table J-5

| Observed and Expected Effects of Compound 506 Alone and Mixtures with Copper Hydroxide, Fenpropidin and Picoxystrobin in Controlling Asian Soybean Rust | | | | |
|---|---|---|---|---|
| Application Rate (ppm) of Compound 506 (i.e. Component (a)) | Component (b) | Application Rate (ppm) of Component (b) | Test E | |
| | | | Obsd | Exp |
| 0 | None | 0 | 0 | - |
| 0.790 | None | 0 | 9 | - |
| 1.585 | None | 0 | 13 | - |
| 0 | copper hydroxide | 3610.778 | 80 | - |
| 0 | copper hydroxide | 7371.507 | 73 | - |
| 0.790 | copper hydroxide | 3610.778 | 78 | 81 |
| 0.790 | copper hydroxide | 7371.507 | 75 | 75 |
| 1.585 | copper hydroxide | 3610.778 | 83 | 82 |
| 1.585 | copper hydroxide | 7371.507 | 84 | 76 |
| 0 | fenpropidin | 78.393 | 0 | - |
| 0 | fenpropidin | 161.740 | 52 | - |
| 0.790 | fenpropidin | 78.393 | 54 | 9 |
| 0.790 | fenpropidin | 161.740 | 79 | 56 |
| 1.585 | fenpropidin | 78.393 | 66 | 13 |
| 1.585 | fenpropidin | 161.740 | 79 | 59 |
| 0 | picoxystrobin | 20.596 | 28 | - |
| 0 | picoxystrobin | 33.865 | 43 | - |
| 0.790 | picoxystrobin | 20.596 | 65 | 34 |
| 0.790 | picoxystrobin | 33.865 | 77 | 48 |
| 1.585 | picoxystrobin | 20.596 | 77 | 38 |
| 1.585 | picoxystrobin | 33.865 | 72 | 50 |

Table J-6

| Observed and Expected Effects of Compound 507 Alone and Mixtures with Copper Hydroxide, Fenpropidin and Picoxystrobin in Controlling Asian Soybean Rust | | | | |
|---|---|---|---|---|
| Application Rate (ppm) of Compound 507 (i.e. Component (a)) | Component (b) | Application Rate (ppm) of Component (b) | Test E | |
| | | | Obsd | Exp |
| 0 | None | 0 | 0 | - |
| 0.896 | None | 0 | 31 | - |
| 1.823 | None | 0 | 56 | - |
| 0 | copper hydroxide | 3610.778 | 80 | - |
| 0 | copper hydroxide | 7371.507 | 73 | - |
| 0.896 | copper hydroxide | 3610.778 | 81 | 86 |
| 0.896 | copper hydroxide | 7371.507 | 76 | 81 |

(continued)

| Observed and Expected Effects of Compound 507 Alone and Mixtures with Copper Hydroxide, Fenpropidin and Picoxystrobin in Controlling Asian Soybean Rust | | | | |
|---|---|---|---|---|
| Application Rate (ppm) of Compound 507 (i.e. Component (a)) | Component (b) | Application Rate (ppm) of Component (b) | Test E | |
| | | | Obsd | Exp |
| 1.823 | copper hydroxide | 3610.778 | 87 | 91 |
| 1.823 | copper hydroxide | 7371.507 | 81 | 88 |
| 0 | fenpropidin | 78.393 | 0 | - |
| 0 | fenpropidin | 161.740 | 52 | - |
| 0.896 | fenpropidin | 78.393 | 38 | 31 |
| 0.896 | fenpropidin | 161.740 | 71 | 67 |
| 1.823 | fenpropidin | 78.393 | 56 | 56 |
| 1.823 | fenpropidin | 161.740 | 74 | 79 |
| 0 | picoxystrobin | 20.596 | 28 | - |
| 0 | picoxystrobin | 33.865 | 43 | - |
| 0.896 | picoxystrobin | 20.596 | 47 | 51 |
| 0.896 | picoxystrobin | 33.865 | 72 | 61 |
| 1.823 | picoxystrobin | 20.596 | 80 | 69 |
| 1.823 | picoxystrobin | 33.865 | 75 | 75 |

Table J-7

| Observed and Expected Effects of Compound 508 Alone and Mixtures with Copper Hydroxide, Fenpropidin and Picoxystrobin in Controlling Asian Soybean Rust | | | | |
|---|---|---|---|---|
| Application Rate (ppm) of Compound 508 (i.e. Component (a)) | Component (b) | Application Rate (ppm) of Component (b) | Test E | |
| | | | Obsd | Exp |
| 0 | None | 0 | 0 | - |
| 0.855 | None | 0 | 0 | - |
| 1.716 | None | 0 | 52 | - |
| 0 | copper hydroxide | 3610.778 | 80 | - |
| 0 | copper hydroxide | 7371.507 | 73 | - |
| 0.855 | copper hydroxide | 3610.778 | 76 | 80 |
| 0.855 | copper hydroxide | 7371.507 | 80 | 73 |
| 1.716 | copper hydroxide | 3610.778 | 69 | 90 |
| 1.716 | copper hydroxide | 7371.507 | 73 | 87 |
| 0 | fenpropidin | 78.393 | 0 | - |
| 0 | fenpropidin | 161.740 | 52 | - |
| 0.855 | fenpropidin | 78.393 | 25 | 0 |
| 0.855 | fenpropidin | 161.740 | 71 | 52 |
| 1.716 | fenpropidin | 78.393 | 69 | 52 |

(continued)

| Observed and Expected Effects of Compound 508 Alone and Mixtures with Copper Hydroxide, Fenpropidin and Picoxystrobin in Controlling Asian Soybean Rust | | | | |
|---|---|---|---|---|
| Application Rate (ppm) of Compound 508 (i.e. Component (a)) | Component (b) | Application Rate (ppm) of Component (b) | Test E | |
| | | | Obsd | Exp |
| 1.716 | fenpropidin | 161.740 | 84 | 77 |
| 0 | picoxystrobin | 20.596 | 28 | - |
| 0 | picoxystrobin | 33.865 | 43 | - |
| 0.855 | picoxystrobin | 20.596 | 75 | 28 |
| 0.855 | picoxystrobin | 33.865 | 88 | 43 |
| 1.716 | picoxystrobin | 20.596 | 70 | 66 |
| 1.716 | picoxystrobin | 33.865 | 71 | 72 |

Table J-8

| Observed and Expected Effects of Compound 509 Alone and Mixtures with Copper Hydroxide, Fenpropidin and Picoxystrobin in Controlling Asian Soybean Rust | | | | |
|---|---|---|---|---|
| Application Rate (ppm) of Compound 509 (i.e. Component (a)) | Component (b) | Application Rate (ppm) of Component (b) | Test E | |
| | | | Obsd | Exp |
| 0 | None | 0 | 0 | - |
| 2.796 | None | 0 | 36 | - |
| 5.625 | None | 0 | 47 | - |
| 0 | copper hydroxide | 3610.778 | 80 | - |
| 0 | copper hydroxide | 7371.507 | 73 | - |
| 2.796 | copper hydroxide | 3610.778 | 77 | 87 |
| 2.796 | copper hydroxide | 7371.507 | 71 | 83 |
| 5.625 | copper hydroxide | 3610.778 | 77 | 89 |
| 5.625 | copper hydroxide | 7371.507 | 79 | 86 |
| 0 | fenpropidin | 78.393 | 0 | - |
| 0 | fenpropidin | 161.740 | 52 | - |
| 2.796 | fenpropidin | 78.393 | 53 | 36 |
| 2.796 | fenpropidin | 161.740 | 72 | 69 |
| 5.625 | fenpropidin | 78.393 | 53 | 47 |
| 5.625 | fenpropidin | 161.740 | 78 | 75 |
| 0 | picoxystrobin | 20.596 | 28 | - |
| 0 | picoxystrobin | 33.865 | 43 | - |
| 2.796 | picoxystrobin | 20.596 | 73 | 54 |
| 2.796 | picoxystrobin | 33.865 | 79 | 63 |
| 5.625 | picoxystrobin | 20.596 | 68 | 62 |
| 5.625 | picoxystrobin | 33.865 | 85 | 70 |

Table J-9

| Observed and Expected Effects of Compound 511 Alone and Mixtures with Copper Hydroxide, Fenpropidin and Picoxystrobin in Controlling Asian Soybean Rust | | | | |
|---|---|---|---|---|
| Application Rate (ppm) of Compound 511 (i.e. Component (a)) | Component (b) | Application Rate (ppm) of Component (b) | Test E | |
| | | | Obsd | Exp |
| 0 | None | 0 | 0 | - |
| 2.436 | None | 0 | 21 | - |
| 4.926 | None | 0 | 68 | - |
| 0 | copper hydroxide | 3610.778 | 80 | - |
| 0 | copper hydroxide | 7371.507 | 73 | - |
| 2.436 | copper hydroxide | 3610.778 | 83 | 84 |
| 2.436 | copper hydroxide | 7371.507 | 77 | 78 |
| 4.926 | copper hydroxide | 3610.778 | 83 | 94 |
| 4.926 | copper hydroxide | 7371.507 | 76 | 91 |
| 0 | fenpropidin | 78.393 | 0 | - |
| 0 | fenpropidin | 161.740 | 52 | - |
| 2.436 | fenpropidin | 78.393 | 45 | 21 |
| 2.436 | fenpropidin | 161.740 | 68 | 62 |
| 4.926 | fenpropidin | 78.393 | 82 | 68 |
| 4.926 | fenpropidin | 161.740 | 85 | 85 |
| 0 | picoxystrobin | 20.596 | 28 | - |
| 0 | picoxystrobin | 33.865 | 43 | - |
| 2.436 | picoxystrobin | 20.596 | 40 | 43 |
| 2.436 | picoxystrobin | 33.865 | 79 | 54 |
| 4.926 | picoxystrobin | 20.596 | 82 | 77 |
| 4.926 | picoxystrobin | 33.865 | 87 | 82 |

Table J-10

| Observed and Expected Effects of Compound 512 Alone and Mixtures with Copper Hydroxide, Fenpropidin and Picoxystrobin in Controlling Asian Soybean Rust | | | | |
|---|---|---|---|---|
| Application Rate (ppm) of Compound 512 (i.e. Component (a)) | Component (b) | Application Rate (ppm) of Component (b) | Test E | |
| | | | Obsd | Exp |
| 0 | None | 0 | 0 | - |
| 1.893 | None | 0 | 9 | - |
| 3.829 | None | 0 | 58 | - |
| 0 | copper hydroxide | 3610.778 | 80 | - |
| 0 | copper hydroxide | 7371.507 | 73 | - |
| 1.893 | copper hydroxide | 3610.778 | 84 | 81 |
| 1.893 | copper hydroxide | 7371.507 | 80 | 75 |

(continued)

| Observed and Expected Effects of Compound 512 Alone and Mixtures with Copper Hydroxide, Fenpropidin and Picoxystrobin in Controlling Asian Soybean Rust | | | | |
|---|---|---|---|---|
| Application Rate (ppm) of Compound 512 (i.e. Component (a)) | Component (b) | Application Rate (ppm) of Component (b) | Test E | |
| | | | Obsd | Exp |
| 3.829 | copper hydroxide | 3610.778 | 81 | 91 |
| 3.829 | copper hydroxide | 7371.507 | 71 | 89 |
| 0 | fenpropidin | 78.393 | 0 | - |
| 0 | fenpropidin | 161.740 | 52 | - |
| 1.893 | fenpropidin | 78.393 | 47 | 9 |
| 1.893 | fenpropidin | 161.740 | 74 | 57 |
| 3.829 | fenpropidin | 78.393 | 74 | 58 |
| 3.829 | fenpropidin | 161.740 | 82 | 80 |
| 0 | picoxystrobin | 20.596 | 28 | - |
| 0 | picoxystrobin | 33.865 | 43 | - |
| 1.893 | picoxystrobin | 20.596 | 52 | 35 |
| 1.893 | picoxystrobin | 33.865 | 78 | 48 |
| 3.829 | picoxystrobin | 20.596 | 79 | 70 |
| 3.829 | picoxystrobin | 33.865 | 84 | 76 |

Table K-1

| Observed and Expected Effects of Compound 316 Alone and Mixtures with Inpyrfluxam in Controlling Asian Soybean Rust | | | | |
|---|---|---|---|---|
| Application Rate (ppm) of Compound 316 (i.e. Component (a)) | Component (b) | Application Rate (ppm) of Component (b) | Test E | |
| | | | Obsd | Exp |
| 0 | None | 0 | 0 | - |
| 0.471 | None | 0 | 24 | - |
| 0.764 | None | 0 | 82 | - |
| 0 | inpyrfluxam | 0.448 | 56 | - |
| 0 | inpyrfluxam | 1.059 | 79 | - |
| 0.471 | inpyrfluxam | 0.448 | 69 | 66 |
| 0.471 | inpyrfluxam | 1.059 | 77 | 84 |
| 0.764 | inpyrfluxam | 0.448 | 38 | 92 |
| 0.764 | inpyrfluxam | 1.059 | 79 | 96 |

Table K-2

| Observed and Expected Effects of Compound 376 Alone and Mixtures with Inpyrfluxam in Controlling Asian Soybean Rust | | | | |
|---|---|---|---|---|
| Application Rate (ppm) of Compound 376 (i.e. Component (a)) | Component (b) | Application Rate (ppm) of Component (b) | Test E | |
| | | | Obsd | Exp |
| 0 | None | 0 | 0 | - |
| 0.753 | None | 0 | 53 | - |
| 1.228 | None | 0 | 78 | - |
| 0 | inpyrfluxam | 0.448 | 56 | - |
| 0 | inpyrfluxam | 1.059 | 79 | - |
| 0.753 | inpyrfluxam | 0.448 | 55 | 79 |
| 0.753 | inpyrfluxam | 1.059 | 86 | 90 |
| 1.228 | inpyrfluxam | 0.448 | 81 | 90 |
| 1.228 | inpyrfluxam | 1.059 | 90 | 95 |

Embodiments of the invention incude the following:

Embodiment 1: A fungicidal composition comprising:

(a) at least one compound selected from the compounds of Formula 1, N-oxides, and salts thereof:

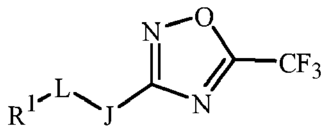

**1**

wherein

$R^1$ is a phenyl ring optionally substituted with up to 3 substituents independently selected from $R^2$; or
$R^1$ is a 5- to 6-membered heteroaromatic ring, each ring containing ring members selected from carbon atoms and 1 to 4 heteroatoms independently selected from up to 2 O, up to 2 S and up to 4 N atoms, wherein up to 2 ring members are independently selected from C(=O), C(=S), S(=O) and $S(=O)_2$, each ring optionally substituted with up to 3 substituents independently selected from $R^2$; or
$R^1$ is a 3- to 7-membered nonaromatic ring or an 8- to 11-membered bicyclic ring system, each ring or ring system containing ring members selected from carbon atoms and optionally up to 4 heteroatoms independently selected from up to 2 O, up to 2 S and up to 4 N atoms, wherein up to 2 ring members are independently selected from C(=O), C(=S), S(=O) and $S(=O)_2$, each ring or ring system optionally substituted with up to 3 substituents independently selected from $R^2$;
L is O, $NR^3$, $NR^3CH_2$, $CH_2NR^3$, $NR^3CH_2CH_2$, $CH_2CH_2NR^3$, $(CR^{4a}R^{4b})_n$, $OCH_2$, $CH_2O$, $OCH_2CH_2$, $CH_2CH_2O$ or $CH_2OCH_2$, wherein the atom to the left is connected to $R^1$, and the atom to the right is connected to J, each carbon atom is optionally substituted with up to 2 substituents independently selected from halogen, cyano, hydroxy, nitro, $C_1$-$C_3$ alkyl, $C_1$-$C_3$ haloalkyl, $C_1$-$C_2$ alkoxy and $C_1$-$C_2$ haloalkoxy;
J is a phenyl ring or a naphthalenyl ring system, each optionally substituted with up to 2 substituents independently selected from $R^5$; or a 3- to 7-membered carbocyclic ring, wherein up to 3 ring members are independently selected from C(=O) and C(=S), each ring optionally substituted with up to 2 substituents independently selected from $R^5$; or
J is a 5- to 6-membered heterocyclic ring, each ring containing ring members selected from carbon atoms and 1 to 4 heteroatoms independently selected from up to 2 O, up to 2 S and up to 4 N atoms, wherein up to 2 ring members are independently selected from C(=O), C(=S), S(=O) and $S(=O)_2$, each ring optionally substituted with up to 2 substituents independently selected from $R^5$;

each $R^2$ is independently halogen, cyano, hydroxy, nitro, thioyl, $SF_5$, CH(=O), C(=O)OH, $-NR^{3a}R^{3b}$, $C(=O)NR^{3a}R^{3b}$, $C(=O)C(=O)NR^{3a}R^{3b}$, $C(=S)NR^{3a}R^{3b}$, $C(R^6)=NR^7$, $N=CR^8NR^{9a}R^{9b}$ or -U-V-Q; or $C_1$-$C_6$ alkyl, $C_2$-$C_6$ alkenyl, $C_2$-$C_6$ alkynyl, $C_3$-$C_7$ cycloalkyl, $C_3$-$C_7$ cycloalkenyl, $C_1$-$C_6$ alkoxy, $C_2$-$C_6$ alkenyloxy, $C_2$-$C_6$ alkynyloxy, $C_3$-$C_7$ cycloalkoxy, $C_1$-$C_6$ alkylthio, $C_1$-$C_6$ alkylsulfinyl, $C_1$-$C_6$ alkylsulfonyl, $C_1$-$C_6$ alkylaminosulfinyl, $C_2$-$C_6$ dialkylaminosulfinyl, $C_1$-$C_6$ alkylsulfonyloxy, $C_1$-$C_6$ alkylsulfonylamino, $C_2$-$C_6$ alkylcarbonyl, $C_4$-$C_7$ cycloalkylcarbonyl, $C_2$-$C_6$ alkoxycarbonyl, $C_3$-$C_6$ alkenyloxycarbonyl, $C_3$-$C_6$ alkynyloxycarbonyl, $C_4$-$C_7$ cycloalkoxycarbonyl, $C_3$-$C_6$ alkyloxycarbonylcarbonyl, $C_2$-$C_6$ alkylcarbonyloxy, $C_4$-$C_7$ cycloalkylcarbonyloxy, $C_2$-$C_6$ alkoxycarbonyloxy, $C_4$-$C_7$ cycloalkoxycarbonyloxy, $C_2$-$C_6$ alkylaminocarbonyloxy, $C_4$-$C_7$ cycloalkylaminocarbonyloxy, $C_2$-$C_6$ alkylcarbonylamino, $C_4$-$C_7$ cycloalkylcarbonylamino, $C_2$-$C_6$ alkoxycarbonylamino, $C_4$-$C_7$ cycloalkoxycarbonylamino, $C_2$-$C_6$ alkylaminocarbonylamino, $C_4$-$C_7$ cycloalkylaminocarbonylamino or $C_2$-$C_6$ dialkoxyphosphinyl, each optionally substituted with up to 3 substituents independently selected from $R^{10}$;

each $R^3$ and $R^{3a}$ is independently H, cyano, hydroxy, $C_1$-$C_4$ alkyl, $C_1$-$C_4$ haloalkyl, $C_2$-$C_4$ alkenyl, $C_2$-$C_4$ haloalkenyl, $C_2$-$C_4$ alkynyl, $C_2$-$C_4$ haloalkynyl, $C_1$-$C_5$ alkoxy, $C_2$-$C_4$ alkoxyalkyl, $C_1$-$C_4$ alkylsulfonyl, $C_1$-$C_4$ haloalkylsulfonyl, $C_2$-$C_4$ alkylthioalkyl, $C_2$-$C_4$ alkylsulfinylalkyl, $C_2$-$C_4$ alkylsulfonylalkyl, $C_2$-$C_4$ alkylcarbonyl, $C_2$-$C_4$ haloalkylcarbonyl, $C_4$-$C_7$ cycloalkylcarbonyl, $C_2$-$C_5$ alkoxycarbonyl, $C_3$-$C_5$ alkoxycarbonylalkyl, $C_2$-$C_5$ alkylaminocarbonyl or $C_3$-$C_5$ dialkylaminocarbonyl;

each $R^{3b}$ is independently H, $C_1$-$C_6$ alkyl, $C_1$-$C_6$ haloalkyl, $C_2$-$C_6$ alkenyl, $C_2$-$C_6$ haloalkenyl, $C_2$-$C_6$ alkynyl, $C_2$-$C_6$ haloalkynyl, $C_1$-$C_6$ hydroxyalkyl, $C_2$-$C_6$ cyanoalkyl, $C_3$-$C_8$ cycloalkyl, $C_3$-$C_8$ halocycloalkyl, $C_3$-$C_8$ cycloalkenyl, $C_3$-$C_8$ halocycloalkenyl, $C_4$-$C_{10}$ alkylcycloalkyl, $C_4$-$C_{10}$ cycloalkylalkyl, $C_4$-$C_{10}$ halocycloalkylalkyl, $C_6$-$C_{14}$ cycloalkylcycloalkyl, $C_5$-$C_{10}$ alkylcycloalkylalkyl, $C_2$-$C_6$ alkoxyalkyl, $C_2$-$C_6$ haloalkoxyalkyl, $C_4$-$C_{10}$ cycloalkoxyalkyl, $C_3$-$C_8$ alkoxyalkoxyalkyl, $C_2$-$C_6$ alkylthioalkyl, $C_2$-$C_6$ alkylsulfinylalkyl, $C_2$-$C_6$ alkylsulfonylalkyl, $C_2$-$C_6$ alkylaminoalkyl, $C_2$-$C_6$ haloalkylaminoalkyl, $C_3$-$C_8$ dialkylaminoalkyl or $C_4$-$C_{10}$ cycloalkylaminoalkyl, each optionally substituted with up to 1 substituent selected from cyano, hydroxy, nitro, $C_2$-$C_4$ alkylcarbonyl, $C_2$-$C_4$ alkoxycarbonyl, $C_3$-$C_{15}$ trialkylsilyl and $C_3$-$C_{15}$ halotrialkylsilyl; or

a pair of $R^{3a}$ and $R^{3b}$ substituents are taken together with the nitrogen atom to which they are attached to form a 4- to 6-membered fully saturated heterocyclic ring, each ring containing ring members, in addition to the connecting nitrogen atom, selected from carbon atoms and up to 2 heteroatoms independently selected from up to 2 O, up to 2 S and up to 2 N atoms, each ring optionally substituted with up to 3 substituents independently selected from halogen and $C_1$-$C_3$ alkyl;

each $R^{4a}$ and $R^{4b}$ is independently H, halogen, cyano, hydroxy, nitro, $C_1$-$C_3$ alkyl, $C_1$-$C_3$ haloalkyl, $C_1$-$C_2$ alkoxy or $C_1$-$C_2$ haloalkoxy; or

a pair of $R^{4a}$ and $R^{4b}$ substituents attached to the same carbon atom are taken together to form a $C_3$-$C_5$ cycloalkyl ring optionally substituted with up to 2 substituents independently selected from halogen, methyl, methoxy and methylthio;

each $R^5$ is independently hydroxy, cyano, nitro, halogen, $C_1$-$C_4$ alkyl, $C_1$-$C_4$ haloalkyl, $C_2$-$C_4$ alkenyl or $C_1$-$C_4$ alkoxy;

each $R^6$ is independently H, cyano, halogen, methyl, methoxy, methylthio or methoxycarbonyl;

each $R^7$ is independently hydroxy or $NR^{11a}R^{11b}$; or $C_1$-$C_4$ alkoxy, $C_2$-$C_4$ alkenyloxy, $C_2$-$C_4$ alkynyloxy, $C_2$-$C_4$ alkylcarbonyloxy, $C_2$-$C_5$ alkoxycarbonyloxy, $C_2$-$C_5$ alkylaminocarbonyloxy or $C_3$-$C_5$ dialkylaminocarbonyloxy, each optionally substituted with up to 1 substituent selected from halogen, cyano, hydroxy and -C(=O)OH;

each $R^8$ is independently H, methyl, methoxy or methylthio;

each $R^{9a}$ and $R^{9b}$ is independently H or $C_1$-$C_4$ alkyl; or

a pair of $R^{9a}$ and $R^{9b}$ substituents are taken together with the nitrogen atom to which they are attached to form a 5- to 6-membered fully saturated heterocyclic ring, each ring containing ring members, in addition to the connecting nitrogen atom, selected from carbon atoms and up to 2 heteroatoms independently selected from up to 2 O, up to 2 S and up to 2 N atoms, each ring optionally substituted with up to 2 methyl;

each $R^{10}$ is independently halogen, amino, cyano, hydroxy, nitro, thioyl, $C_1$-$C_4$ alkyl, $C_1$-$C_4$ haloalkyl, $C_3$-$C_6$ cycloalkyl, $C_3$-$C_6$ halocycloalkyl, $C_1$-$C_4$ alkoxy, $C_1$-$C_4$ haloalkoxy, $C_2$-$C_4$ alkoxyalkoxy, $C_1$-$C_4$ alkylthio, $C_1$-$C_4$ alkylsulfinyl, $C_1$-$C_4$ alkylsulfonyl, $C_1$-$C_4$ haloalkylsulfonyl, $C_2$-$C_4$ alkylcarbonyl, $C_2$-$C_4$ haloalkylcarbonyl, $C_2$-$C_5$ alkoxycarbonyl, $C_1$-$C_6$ alkylamino, $C_2$-$C_6$ dialkylamino, $C_2$-$C_5$ alkylaminocarbonyl, $C_3$-$C_5$ dialkylaminocarbonyl, $C_3$-$C_5$ alkylthioalkylcarbonyl, $C_3$-$C_{15}$ trialkylsily, $C_3$-$C_{15}$ halotrialkylsilyl, $C(R^{13})=NOR^{14}$ or $C(R^{15})=NR^{16}$;

each U is independently a direct bond, C(=O)O, $C(=O)NR^{17}$ or $C(=S)NR^{18}$, wherein the atom to the left is connected to $R^1$, and the atom to the right is connected to V;

each V is independently a direct bond; or $C_1$-$C_6$ alkylene, $C_2$-$C_6$ alkenylene, $C_3$-$C_6$ alkynylene, $C_3$-$C_6$ cycloalkylene or $C_3$-$C_6$ cycloalkenylene, each optionally substituted with up to 3 substituents independently

selected from halogen, cyano, nitro, hydroxy, $C_1$-$C_2$ alkyl, $C_1$-$C_2$ haloalkyl, $C_1$-$C_2$ alkoxy and $C_1$-$C_2$ haloalkoxy;

each Q is independently phenyl or phenoxy, each optionally substituted with up to 2 substituents independently selected from $R^{12}$; or

each Q is independently a 5- to 6-membered heteroaromatic ring, each ring containing ring members selected from carbon atoms and 1 to 4 heteroatoms independently selected from up to 2 O, up to 2 S and up to 4 N atoms, wherein up to 2 ring members are independently selected from C(=O), C(=S), S(=O) and $S(=O)_2$, each ring optionally substituted with up to 2 substituents independently selected from $R^{12}$; or

each Q is independently a 3- to 7-membered nonaromatic heterocyclic ring, each ring containing ring members selected from carbon atoms and 1 to 4 heteroatoms independently selected from up to 2 O, up to 2 S and up to 4 N atoms, wherein up to 2 ring members are independently selected from C(=O), C(=S), S(=O) and $S(=O)_2$, each ring optionally substituted with up to 2 substituents independently selected from $R^{12}$;

each $R^{11a}$ is independently H, $C_1$-$C_4$ alkyl or $C_2$-$C_4$ alkylcarbonyl;

each $R^{11b}$ is independently H, cyano, $C_1$-$C_5$ alkyl, $C_2$-$C_5$ alkylcarbonyl, $C_2$-$C_5$ haloalkylcarbonyl, $C_4$-$C_7$ cycloalkylcarbonyl, $C_2$-$C_5$ alkoxycarbonyl, $C_3$-$C_5$ alkoxycarbonylalkyl, $C_2$-$C_5$ alkylaminocarbonyl or $C_3$-$C_5$ dialkylaminocarbonyl; or

a pair of $R^{11a}$ and $R^{11b}$ substituents are taken together with the nitrogen atom to which they are attached to form a 5- to 6-membered fully saturated heterocyclic ring, each ring containing ring members, in addition to the connecting nitrogen atom, selected from carbon atoms and up to 2 heteroatoms independently selected from up to 2 O, up to 2 S and up to 2 N atoms, each ring optionally substituted with up to 2 methyl;

each $R^{12}$ is independently halogen, cyano, hydroxy, nitro, $C_1$-$C_4$ alkyl, $C_1$-$C_4$ haloalkyl, $C_2$-$C_4$ alkenyl, $C_1$-$C_4$ alkoxy, $C_2$-$C_4$ alkylcarbonyl or $C_2$-$C_4$ alkoxycarbonyl;

each $R^{13}$ and $R^{15}$ is independently H, cyano, halogen, $C_1$-$C_3$ alkyl, $C_1$-$C_3$ haloalkyl, $C_3$-$C_6$ cycloalkyl or $C_1$-$C_3$ alkoxy; or a phenyl ring optionally substituted with up to 2 substituents independently selected from halogen and $C_1$-$C_3$ alkyl;

each $R^{14}$ is independently H, $C_1$-$C_5$ alkyl, $C_1$-$C_5$ haloalkyl, $C_2$-$C_5$ alkenyl, $C_2$-$C_5$ haloalkenyl, $C_2$-$C_5$ alkynyl, $C_3$-$C_6$ cycloalkyl, $C_3$-$C_6$ halocycloalkyl, $C_2$-$C_5$ alkylcarbonyl or $C_2$-$C_5$ alkoxycarbonyl; or

each $R^{14}$ is a phenyl ring optionally substituted with up to 2 substituents independently selected from halogen and $C_1$-$C_3$ alkyl; or a 5- to 6-membered fully saturated heterocyclic ring, each ring containing ring members selected from carbon atoms and up to 2 heteroatoms independently selected from up to 2 O, up to 2 S and up to 2 N atoms, each ring optionally substituted with up to 2 substituents independently selected from halogen and $C_1$-$C_3$ alkyl;

each $R^{16}$ is independently H, cyano, $C_1$-$C_3$ alkyl, $C_1$-$C_3$ haloalkyl, $C_1$-$C_4$ alkoxy, $C_2$-$C_4$ alkylcarbonyl or $C_2$-$C_4$ alkoxycarbonyl;

each $R^{17}$ and $R^{18}$ is independently H, cyano, hydroxy, $C_1$-$C_4$ alkyl, $C_1$-$C_4$ haloalkyl, $C_2$-$C_4$ alkylcarbonyl, $C_2$-$C_4$ haloalkylcarbonyl, $C_2$-$C_4$ alkoxycarbonyl or $C_2$-$C_4$ haloalkoxycarbonyl; and

n is 1, 2 or 3; and

(b) at least one additional fungicidal compound.

Embodiment 2. The composition of Embodiment 1 wherein component (a) comprises a compound of Formula 1 or salt thereof, wherein

$R^1$ is selected from U-1 through U-118

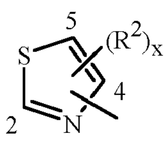
,
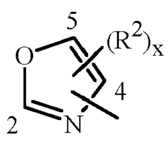
,
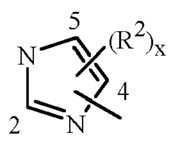
,
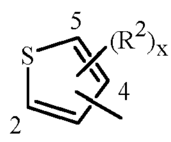
,

U-1 U-2 U-3 U-4

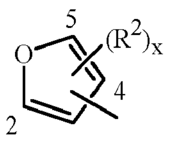 ,

U-5

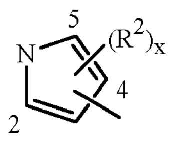 ,

U-6

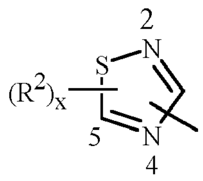 ,

U-7

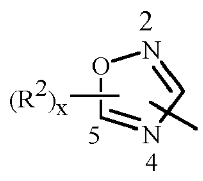 ,

U-8

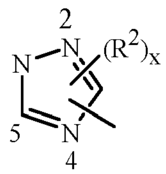 ,

U-9

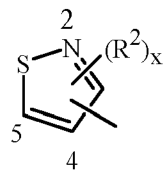 ,

U-10

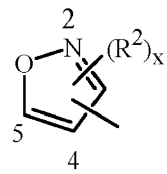 ,

U-11

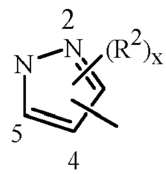 ,

U-12

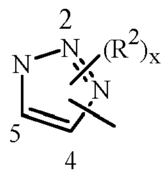 ,

U-13

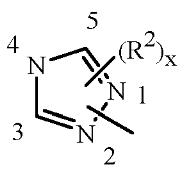 ,

U-14

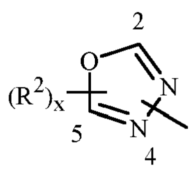 ,

U-15

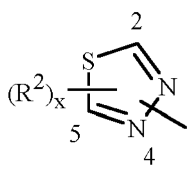 ,

U-16

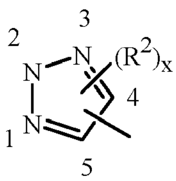 ,

U-17

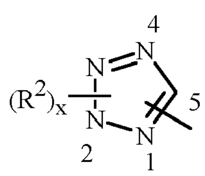 ,

U-18

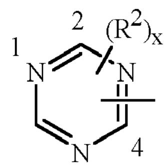 ,

U-19

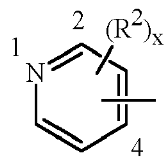 ,

U-20

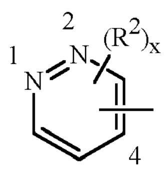 ,

U-21

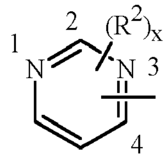 ,

U-22

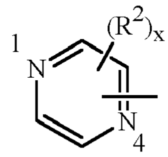 ,

U-23

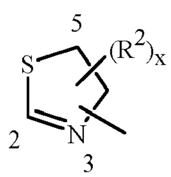 ,

U-24

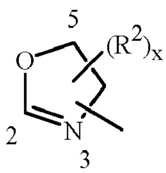 ,

U-25

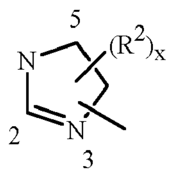 ,

U-26

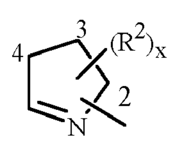 ,

U-27

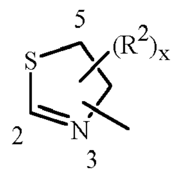 ,

U-28

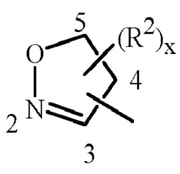 , 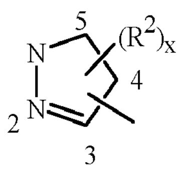 , 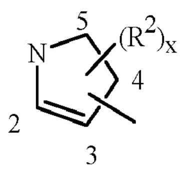 , 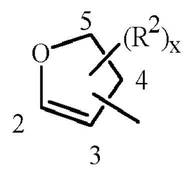 ,

U-29 U-30 U-31 U-32

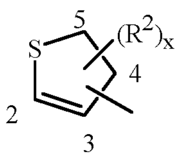 , 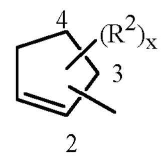 , 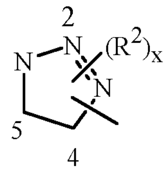 , 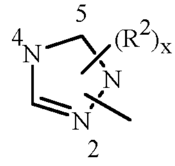 ,

U-33 U-34 U-35 U-36

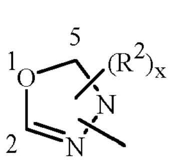 , 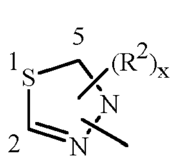 , 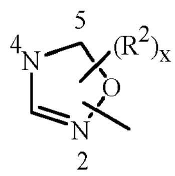 , 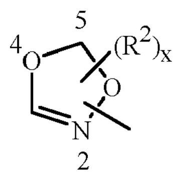 ,

U-37 U-38 U-39 U-40

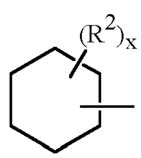 , 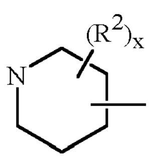 , 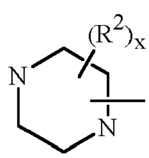 , 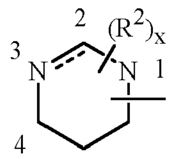 ,

U-41 U-42 U-43 U-44

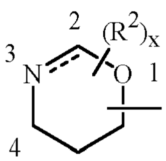 , 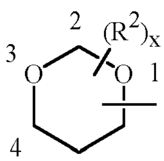 , 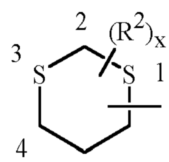 , 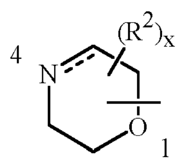 ,

U-45 U-46 U-47 U-48

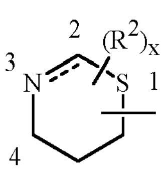 , 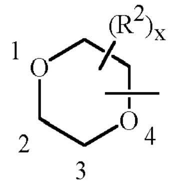 , 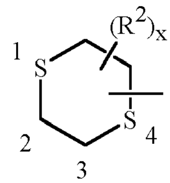 , 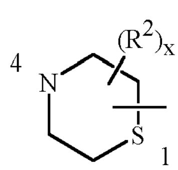 ,

U-49 U-50 U-51 U-52

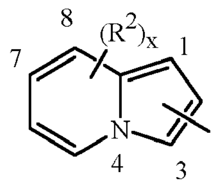 , 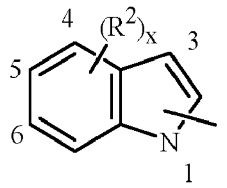 , 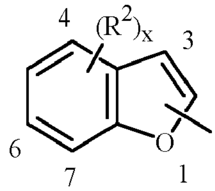 , 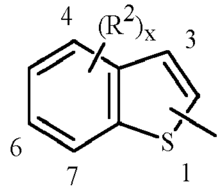 ,

U-53 U-54 U-55 U-56

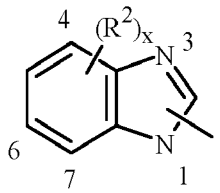 , 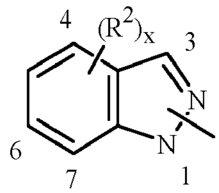 , 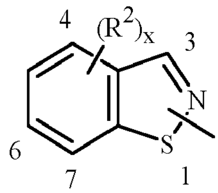 , 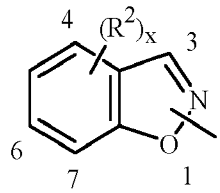 ,

U-57 U-58 U-59 U-60

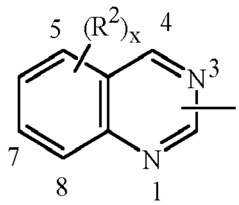 , 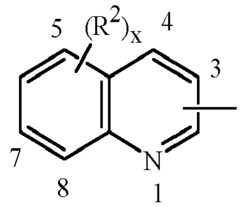 , 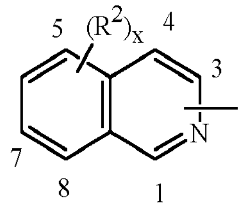 , 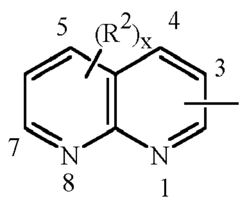 ,

U-61 U-62 U-63 U-64

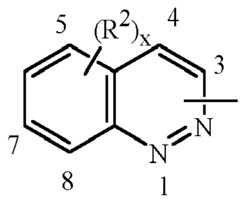 , 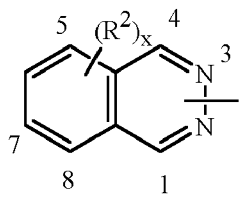 , 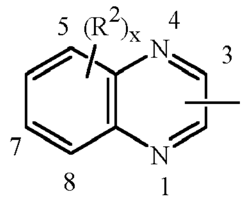 , 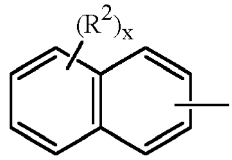 ,

U-65 U-66 U-67 U-68

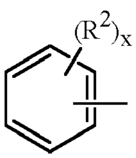 , 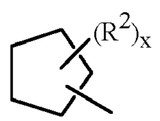 , 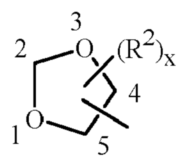 , 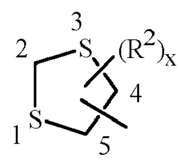 ,

U-69 U-70 U-71 U-72

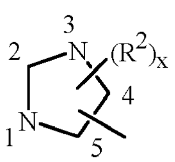 , 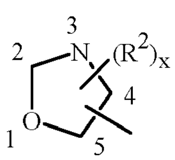 , 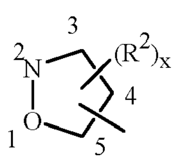 , 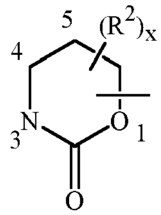 ,

U-73 U-74 U-75 U-76

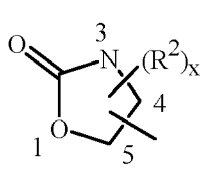 , 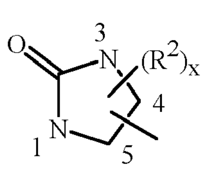 , 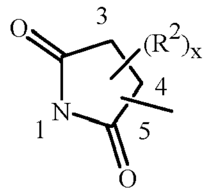 , 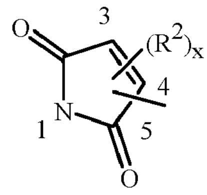 ,

U-77 U-78 U-79 U-80

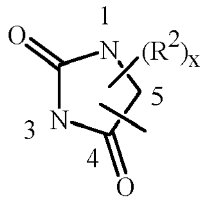 , 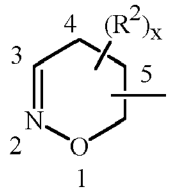 , 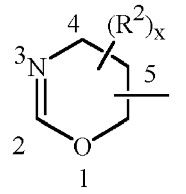 , 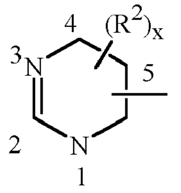 ,

U-81 U-82 U-83 U-84

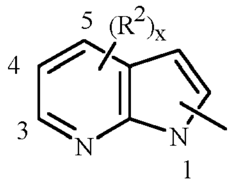 , 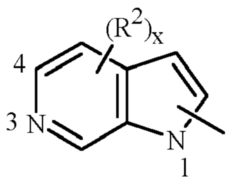 , 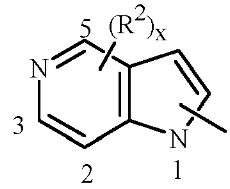 , 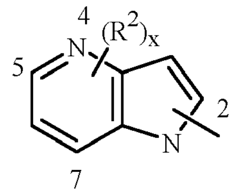 ,

U-85 U-86 U-87 U-88

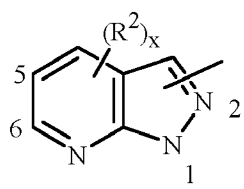 , 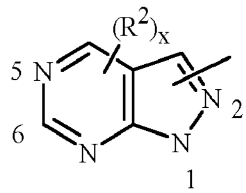 , 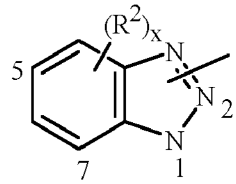 , 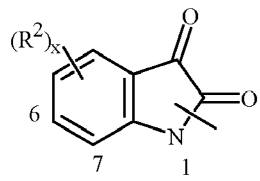 ,

U-89 U-90 U-91 U-92

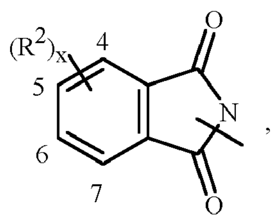 , 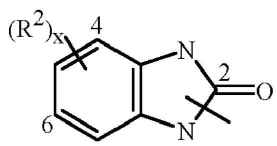 , 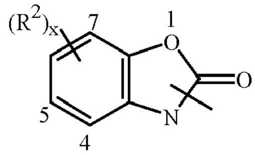 , 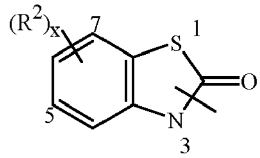 ,

U-93 U-94 U-95 U-96

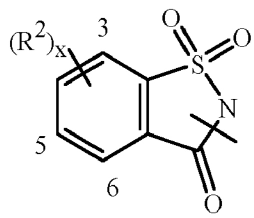 , 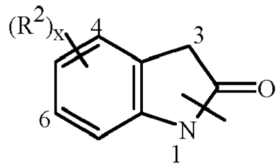 , 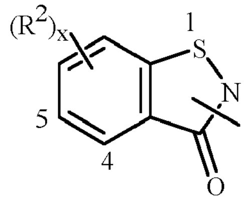 , 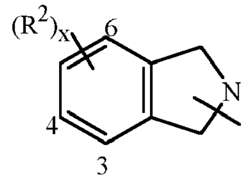 ,

U-97 U-98 U-99 U-100

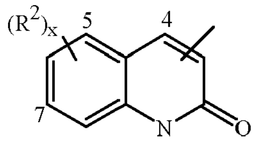 ,
U-101

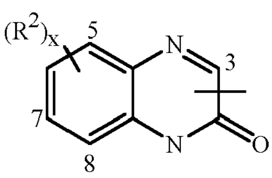 ,
U-102

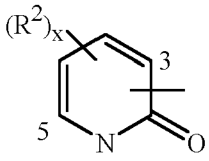 ,
U-103

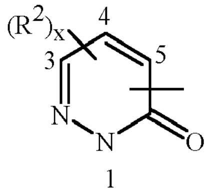 ,
U-104

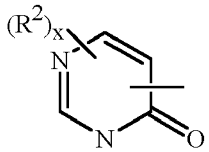 ,
U-105

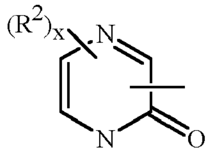 ,
U-106

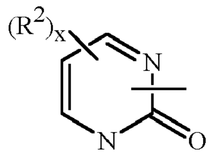 ,
U-107

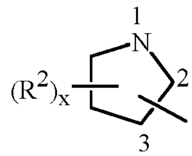 ,
U-108

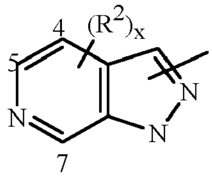 ,
U-109

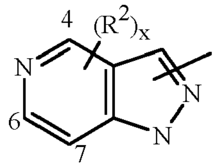 ,
U-110

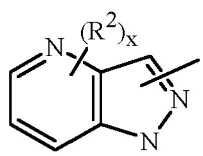 ,
U-111

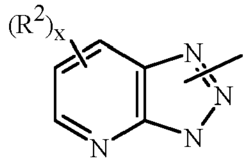 ,
U-112

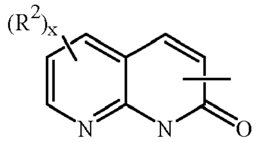 ,
U-113

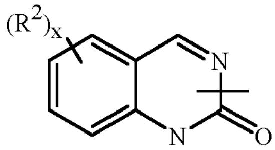 ,
U-114

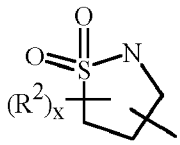 ,
U-115

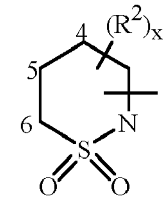 ,
U-116

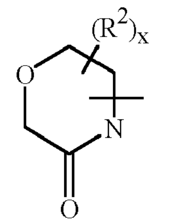
U-117

and

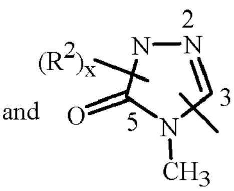 ;
U-118

wherein the floating bond is connected to L in Formula 1 through any available carbon or nitrogen atom of the depicted ring or ring system;

x is 0, 1 or 2;

L is $(CR^{4a}R^{4b})_n$, $OCH_2$, $CH_2O$, $OCH_2CH_2$, $CH_2CH_2O$ or $CH_2OCH_2$, wherein the atom to the left is connected to $R^1$, and the atom to the right is connected to J, each carbon atom is optionally substituted with up to 1 substituent selected from halogen, cyano, hydroxy, methyl, halomethyl or methoxy;

n is 1 or 2;

J is selected from J-1 through J-93

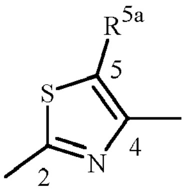 , 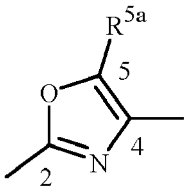 , 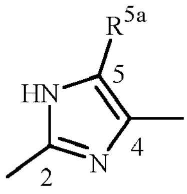 , 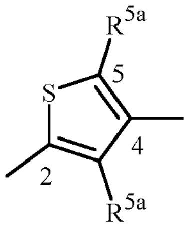 ,

J-1 J-2 J-3 J-4

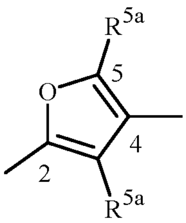 , 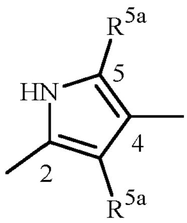 , 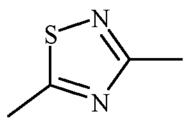 , 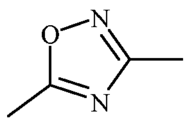 ,

J-5 J-6 J-7 J-8

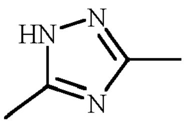 , 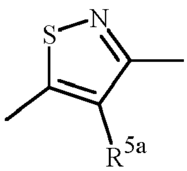 , 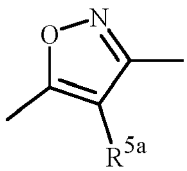 , 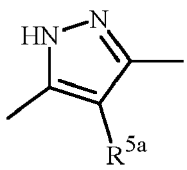 ,

J-9 J-10 J-11 J-12

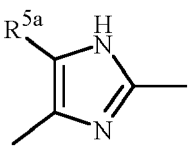 , 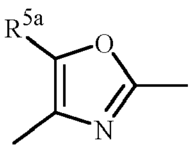 , 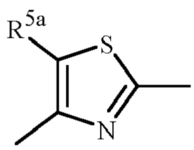 , 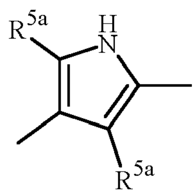 ,

J-13 J-14 J-15 J-16

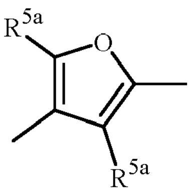 , 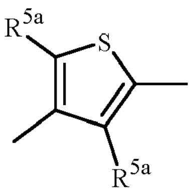 , 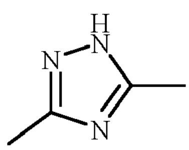 , 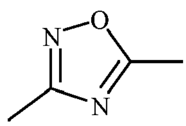 ,

J-17 J-18 J-19 J-20

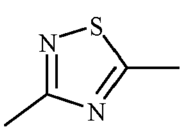 , 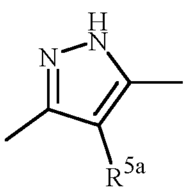 , 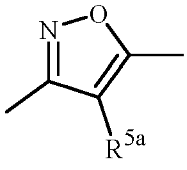 , 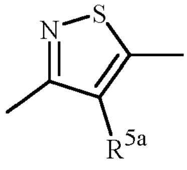 ,

J-21 J-22 J-23 J-24

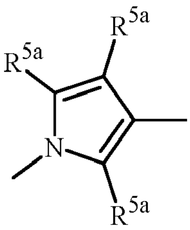
J-25
,
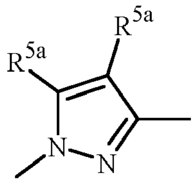
J-26
,
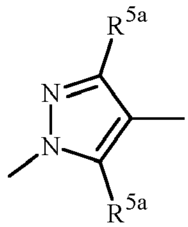
J-27
,
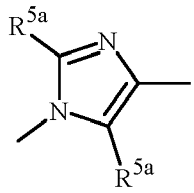
J-28
,

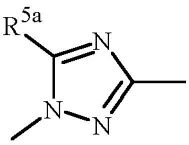
J-29
,
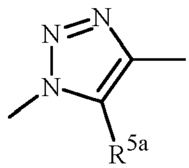
J-30
,
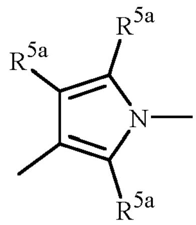
J-31
,
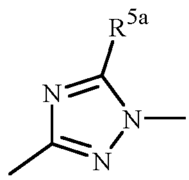
J-32
,

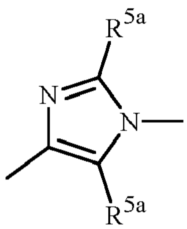
J-33
,
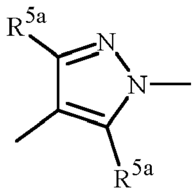
J-34
,
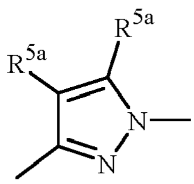
J-35
,
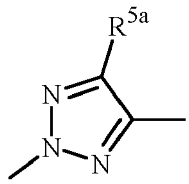
J-36
,

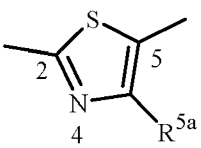
J-37
,
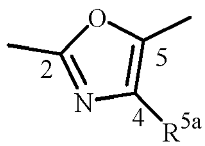
J-38
,
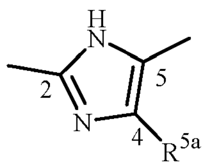
J-39
,
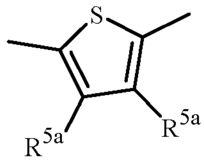
J-40
,

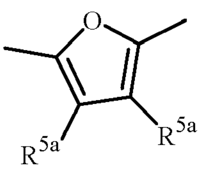
J-41
,
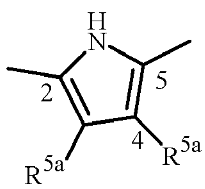
J-42
,
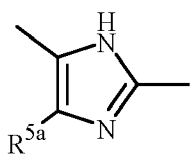
J-43
,
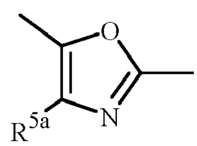
J-44
,

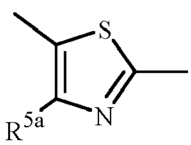
J-45
,
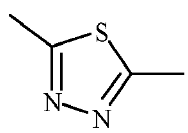
J-46
,
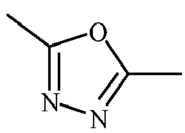
J-47
,
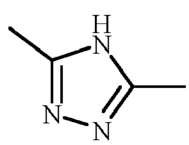
J-48
,

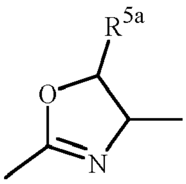 ,
J-49

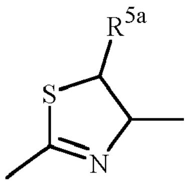 ,
J-50

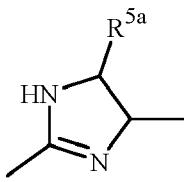 ,
J-51

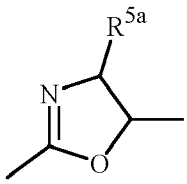 ,
J-52

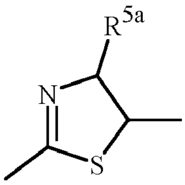 ,
J-53

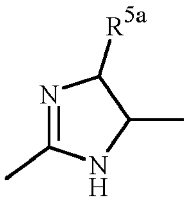 ,
J-54

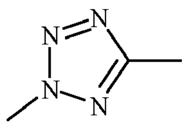 ,
J-55

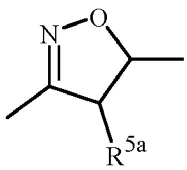 ,
J-56

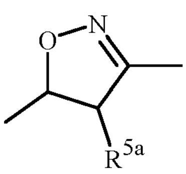 ,
J-57

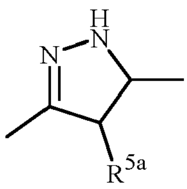 ,
J-58

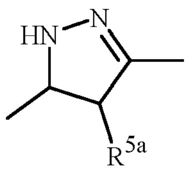 ,
J-59

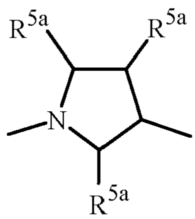 ,
J-60

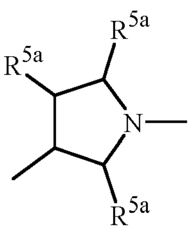 ,
J-61

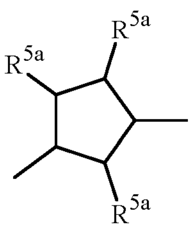 ,
J-62

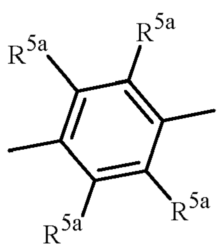 ,
J-63

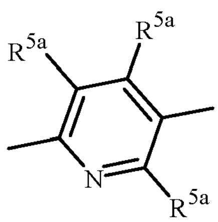 ,
J-64

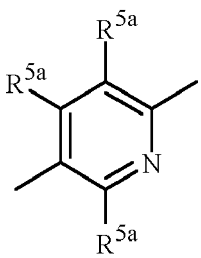 ,
J-65

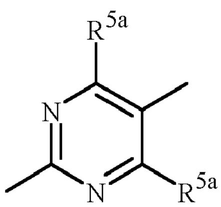 ,
J-66

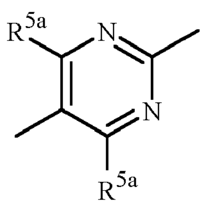 ,
J-67

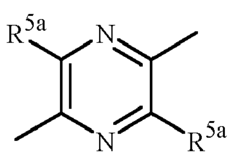 ,
J-68

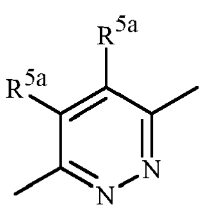 ,
J-69

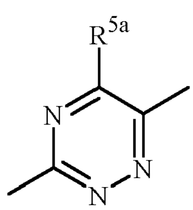 ,
J-70

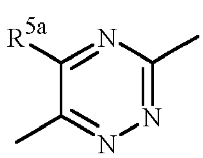 ,
J-71

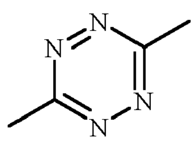 ,
J-72

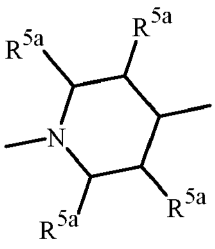 , 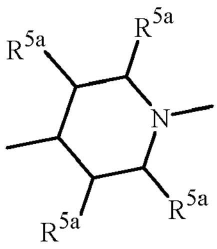 , 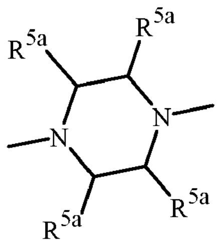 , 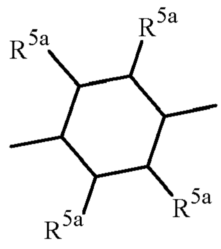 ,

J-73 J-74 J-75 J-76

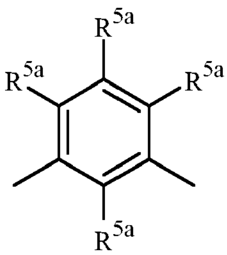 , 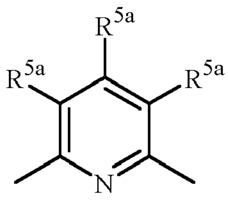 , 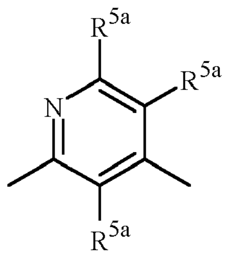 , 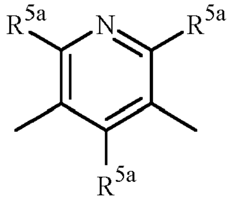 ,

J-77 J-78 J-79 J-80

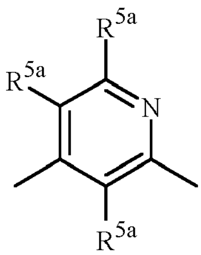 , 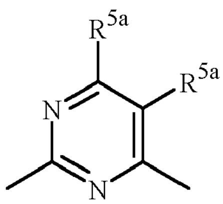 , 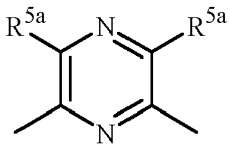 , 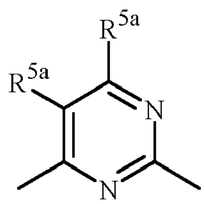 ,

J-81 J-82 J-83 J-84

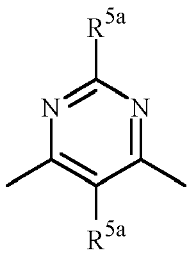 , 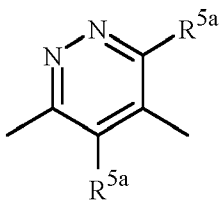 , 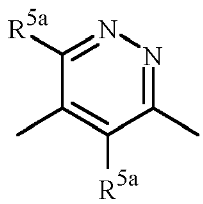 , 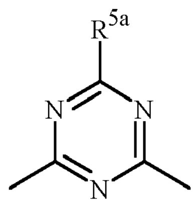 ,

J-85 J-86 J-87 J-88

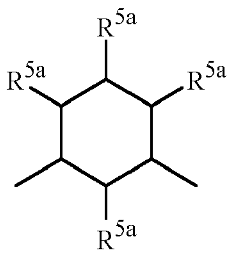 , 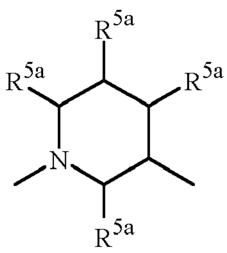 , 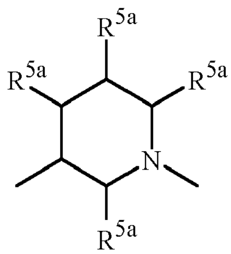 , 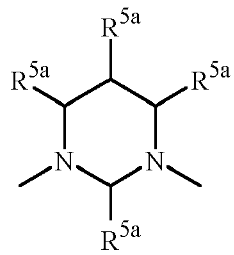

J-89 J-90 J-91 J-92

and

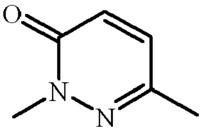

J-93

wherein the bond projecting to the left is bonded to L, and the bond projecting to the right is bonded to the oxadiazole ring in Formula 1;
each $R^{5a}$ is independently H or $R^5$; provided that at most only two $R^{5a}$ substituents are other than H;
each $R^2$ is independently halogen, $C(=O)NR^{3a}R^{3b}$, $C(R^6)=NR^7$ or -U-V-Q; or $C_1$-$C_6$ alkyl, $C_2$-$C_6$ alkenyl, $C_2$-$C_6$ alkynyl, $C_3$-$C_7$ cycloalkyl, $C_1$-$C_6$ alkoxy, $C_2$-$C_6$ alkenyloxy, $C_2$-$C_6$ alkynyloxy, $C_3$-$C_7$ cycloalkoxy, $C_1$-$C_6$ alkylthio, $C_2$-$C_6$ alkoxycarbonyl, $C_3$-$C_6$ alkenyloxycarbonyl, $C_3$-$C_6$ alkynyloxycarbonyl, $C_4$-$C_7$ cycloalkoxycarbonyl, $C_2$-$C_6$ alkylcarbonyloxy or $C_2$-$C_6$ alkylcarbonylamino, each optionally substituted with up to 1 substituent selected from $R^{10}$;
each $R^{3a}$ is independently H, cyano, hydroxy, $C_1$-$C_4$ alkyl, $C_1$-$C_4$ haloalkyl, $C_2$-$C_4$ alkenyl, $C_2$-$C_4$ alkynyl, $C_1$-$C_4$ alkoxy, $C_2$-$C_4$ alkoxyalkyl, $C_2$-$C_4$ alkylcarbonyl, $C_2$-$C_4$ haloalkylcarbonyl or $C_3$-$C_5$ alkoxycarbonylalkyl;
each $R^{3b}$ is independently H, $C_1$-$C_6$ alkyl, $C_1$-$C_6$ haloalkyl, $C_2$-$C_6$ alkenyl, $C_2$-$C_6$ haloalkenyl, $C_2$-$C_6$ alkynyl, $C_2$-$C_6$ haloalkynyl, $C_4$-$C_{10}$ cycloalkylalkyl, $C_4$-$C_{10}$ halocycloalkylalkyl, $C_2$-$C_6$ alkoxyalkyl or $C_2$-$C_6$ haloalkoxyalkyl, each optionally substituted with up to 1 substituent selected from cyano, $C_2$-$C_4$ alkylcarbonyl and $C_2$-$C_4$ alkoxycarbonyl; or
a pair of $R^{3a}$ and $R^{3b}$ substituents attached to the same nitrogen atom are taken together to form a pyrrolidinyl ring optionally substituted with up to 2 substituents independently selected from halogen;
each $R^{4a}$ and $R^{4b}$ is independently H, halogen, hydroxy, methyl or methoxy;
each $R^5$ is independently cyano, halogen, methyl or methoxy;
each $R^6$ is independently H, cyano, halogen methyl or methoxy;
each $R^7$ is independently $C_1$-$C_4$ alkoxy, $C_2$-$C_4$ alkenyloxy or $C_2$-$C_4$ alkynyloxy;
each $R^{10}$ is independently halogen, $C_1$-$C_4$ alkyl, $C_1$-$C_4$ haloalkyl, $C_1$-$C_4$ alkoxy, $C_1$-$C_4$ haloalkoxy, $C_2$-$C_4$ alkylcarbonyl, $C_2$-$C_4$ haloalkylcarbonyl, $C_2$-$C_5$ alkoxycarbonyl or $C(R^{13})=NOR^{14}$;
each U is independently a direct bond, C(=O)O or $C(=O)NR^{17}$;
each V is independently a direct bond; or $C_1$-$C_2$ alkylene optionally substituted with up to 1 substituent selected from $C_1$-$C_2$ alkyl, $C_1$-$C_2$ alkoxy and $C_1$-$C_2$ haloalkoxy;
each Q is independently phenyl optionally substituted with up to 2 substituents independently selected from $R^{12}$; or pyridinyl or pyrazolyl, each ring optionally substituted with up to 2 substituents independently selected from $R^{12}$;
each $R^{12}$ is independently halogen, cyano, $C_1$-$C_3$ alkyl, $C_1$-$C_3$ haloalkyl or $C_1$-$C_3$ alkoxy;
each $R^{13}$ is independently H, halogen, methyl or methoxy;
each $R^{14}$ is independently H, methyl, halomethyl, $C_2$-$C_4$ alkylcarbonyl or $C_2$-$C_4$ alkoxycarbonyl; and
each $R^{17}$ is independently H, cyano, methyl or halomethyl.

Embodiment 3. The composition of Embodiment 2 wherein component (a) comprises a compound of Formula **1** or salt thereof, wherein

$R^1$ is U-1, U-2, U-3, U-4, U-5, U-7, U-8, U-10, U-11, U-12 or U-29;
x is 1 or 2;
L is $(CR^{4a}R^{4b})_n$, $CH_2O$ or $CH_2OCH_2$, wherein the atom to the left is connected to $R^1$, and the atom to the right is connected to J, each carbon atom is optionally substituted with up to 1 substituent selected from halogen, cyano, hydroxy, methyl, halomethyl or methoxy;
J is J-4, J-18, J-27, J-40 or J-63;
each $R^2$ is independently $C(=O)NR^{3a}R^{3b}$ or -U-V-Q; or $C_1$-$C_3$ alkyl, $C_2$-$C_6$ alkoxycarbonyl, $C_3$-$C_6$ alkenyloxycarbonyl or $C_3$-$C_6$ alkynyloxycarbonyl, each optionally substituted with up to 1 substituent selected from $R^{10}$;
each $R^{3a}$ is independently H, $C_1$-$C_4$ alkyl, $C_1$-$C_4$ haloalkyl, $C_2$-$C_4$ alkenyl, $C_2$-$C_4$ alkynyl, $C_2$-$C_4$ alkoxyalkyl or $C_3$-$C_5$ alkoxycarbonylalkyl;
each $R^{3b}$ is independently H, $C_1$-$C_6$ alkyl, $C_1$-$C_6$ haloalkyl, $C_2$-$C_6$ alkoxyalkyl or $C_2$-$C_6$ haloalkoxyalkyl, each optionally substituted with up to 1 substituent selected from cyano, $C_2$-$C_4$ alkylcarbonyl and $C_2$-$C_4$ alkoxycarbonyl; or
a pair of $R^{3a}$ and $R^{3b}$ substituents attached to the same nitrogen atom are taken together to form a pyrrolidinyl ring optionally substituted with up to 2 fluorine atoms;

each $R^{4a}$ and $R^{4b}$ is independently H or methyl;
each $R^5$ is independently methyl or methoxy;
each $R^{10}$ is independently halogen, $C_1$-$C_4$ alkyl, $C_1$-$C_4$ alkoxy, $C_2$-$C_4$ alkylcarbonyl, $C_2$-$C_4$ haloalkylcarbonyl or $C_2$-$C_5$ alkoxycarbonyl;
each U is independently C(=O)O or C(=O)$NR^{17}$;
each V is independently $C_1$-$C_2$ alkylene; and
each $R^{12}$ is independently halogen, methyl or methoxy.

Embodiment 4. The composition of Embodiment 3 wherein component (a) comprises a compound of Formula **1** or salt thereof, wherein

$R^1$ is U-1, U-2, U-8 or U-12;
L is $(CR^{4a}R^{4b})_n$ or $CH_2O$;
n is 1;
J is J-63;
each $R^2$ is independently C(=O)$NR^{3a}R^{3b}$; or $C_1$-$C_2$ alkyl, $C_2$-$C_6$ alkoxycarbonyl, $C_3$-$C_6$ alkenyloxycarbonyl or $C_3$-$C_6$ alkynyloxycarbonyl, each optionally substituted with up to 1 substituent selected from $R^{10}$;
each $R^{3a}$ is independently H, $C_1$-$C_3$ alkyl, $C_1$-$C_3$ haloalkyl or $C_2$-$C_3$ alkoxyalkyl;
each $R^{3b}$ is independently H, $C_1$-$C_3$ alkyl, $C_1$-$C_3$ haloalkyl, $C_2$-$C_3$ alkoxyalkyl or $C_2$-$C_3$ haloalkoxyalkyl, each optionally substituted with up to 1 substituent selected from cyano;
each $R^{4a}$ and $R^{4b}$ is H;
each $R^{5a}$ is H; and
each $R^{10}$ is independently halogen, $C_1$-$C_2$ alkoxy, $C_2$-$C_3$ alkylcarbonyl or $C_2$-$C_3$ haloalkylcarbonyl.

Embodiment 5. The composition of Embodiment 4 wherein component (a) comprises a compound of Formula **1** or salt thereof, wherein

$R^1$ is U-2 or U-12;
x is 1;
$R^2$ is C(=O)$NR^{3a}R^{3b}$ or $C_2$-$C_6$ alkoxycarbonyl;
$R^{3a}$ is H; and
$R^{3b}$ is H, $C_1$-$C_3$ alkyl, $C_1$-$C_3$ cyanoalkyl, $C_1$-$C_3$ haloalkyl, $C_2$-$C_3$ alkoxyalkyl or $C_2$-$C_3$ haloalkoxyalkyl.

Embodiment 6. The composition of Embodiment 5 wherein component (a) comprises a compound of Formula **1** or salt thereof, wherein

$R^1$ is U-2 connected at its 2-position to L and L is $CH_2$; or
$R^1$ is U-12 connected at its 1-position to L and L is $CH_2O$; and
$R^2$ is C(=O)$NR^{3a}R^{3b}$ or $C_2$-$C_3$ alkoxycarbonyl.

Embodiment 7. The composition of Embodiment 1 wherein component (a) comprises a compound selected from the group consisting of

methyl 1-[[4-[5-(trifluoromethyl)-1,2,4-oxadiazol-3-yl]phenyl]methyl]-1*H*-pyrazole-4-carboxylate;
cyanomethyl 1-[[4-[5-(trifluoromethyl)-1,2,4-oxadiazol-3-yl]phenyl]methyl]-1*H*-pyrazole-4-carboxylate;
ethyl 5-methyl-1-[[4-[5-(triffuoromethyl)-1,2,4-oxadiazol-3-yl]phenyl]methyl]-1*H* pyrazole-3 -carboxylate;
*N*-(2-methoxyethyl)-5-methyl-1-[[4-[5-(trifluoromethyl)-1,2,4-oxadiazol-3-yl]phenyl]methyl]-1*H*-pyrazole-3-carboxamide;
propyl 1-[[5-[5-(trifluoromethyl)-1,2,4-oxadiazol-3-yl]-2-thienyl]methyl]-1*H*-pyrazole-4-carboxylate;
*N*-(2-chloroethyl)-2-[[4-[5-(trifluoromethyl)-1,2,4-oxadiazol-3-yl]phenyl]methyl]-4-oxazolecarboxamide;
*N*-(2,2-difluoroethyl)-2-[[4-[5-(trifluoromethyl)-1,2,4-oxadiazol-3-yl]phenyl]methyl]-4-oxazolecarboxamide;
*N*-(2-cyanoethyl)-2-[[4-[5-(trifluoromethyl)-1,2,4-oxadiazol-3-yl]phenyl]methyl]-4-oxazolecarboxamide;
*N*-(2-methoxyethyl)-2-[[4-[5-(trifluoromethyl)-1,2,4-oxadiazol-3-yl]phenyl]methyl]-4-oxazolecarboxamide;
*N*-[2-(1*H*-pyrazol-1-yl)ethyl]-2-[[4-[5-(trifluoromethyl)-1,2,4-oxadiazol-3-yl]phenyl]methyl]-4-oxazolecarboxamide;
*N*-(2,2,2-trifluoroethyl)-2-[[4-[5-(trifluoromethyl)-1,2,4-oxadiazol-3-yl]phenyl]methyl]-4-oxazolecarboxamide;
2-[[4-[5-(trifluoromethyl)-1,2,4-oxadiazol-3-yl]phenyl]methyl]-N (3,3,3-trifluoropropyl)-4-oxazolecarboxamide;
ethyl 1-[[4-[5-(trifluoromethyl)-1,2,4-oxadiazol-3-yl]phenoxy]methyl]-1*H*-pyrazole-4-carboxylate;

*N*-(2-methoxyethyl)-2-[[4-[5-(trifluoromethyl)-1,2,4-oxadiazol-3-yl]phenyl]methyl]-4-thiazolecarboxamide;
*N*-(2-fluoroethyl)-2-[[4-[5-(trifluoromethyl)-1,2,4-oxadiazol-3-yl]phenyl]methyl]-4-Oxazolecarboxamide;
*N*-[2-(trifluoromethoxy)ethyl]-2-[[4-[5-(trifluoromethyl)-1,2,4-oxadiazol-3-yl]phenyl]methyl]-4-oxazolecarboxamide;
(3,3-difluoro-1-pyrrolidinyl)[2-[[4-[5-(trifluoromethyl)-1,2,4-oxadiazol-3-yl]phenyl]methyl]-4-oxazolyl]methanone;
*N*-(2,2,2-trifluoroethyl)-2-[[4-[5-(trifluoromethyl)-l,2,4-oxadiazol-3-yl]phenyl]methyl]-4-thiazolecarboxamide;
*N*-(2,2-difluoroethyl)-2-[[4-[5-(trifluoromethyl)-1,2,4-oxadiazol-3-yl]phenyl]methyl]-4-thiazolecarboxamide;
*N*-(2,2,2-trifluoroethyl)-5-[[4-[5-(trifluoromethyl)-1,2,4-oxadiazol-3-yl]phenyl]methyl]-1,2,4-oxadiazole-3-carboxamide;
3-[4-[(1-cyanomethyl-1*H*-pyrazol-3-yl)methyl]phenyl]-5-(triffuoromethyl)-1,2,4-oxadiazole; *N*-ethyl-1-[[[4-[5-(trifluoromethyl)-1,2,4-oxadiazol-3-yl]phenyl]methoxy]methyl]-1*H*-pyrazole-4-carboxamide;
1-methyl-*N*-(2,2,2-trifluoroethyl)-5-[[4-[5-(trifluoromethyl)-1,2,4-oxadiazol-3-yl]phenyl]methyl]-1*H*-pyrazole-3-carboxamide;
5-[[4-[5-(trifluoromethyl)-1,2,4-oxadiazol-3-yl]phenyl]methyl]-3-isoxazoleacetonitrile; *N*-(2-methoxyethyl)-5-[[4-[5-(triffuoromethyl)-1,2,4-oxadiazol-3-yl]phenyl]methyl]-1,2,4-oxadiazole-3-carboxamide; and
1-methyl-*N*-(2,2,2-trifluoroethyl)-3-[[4-[5-(trifluoromethyl)-1,2,4-oxadiazol-3-yl]phenyl]methyl]-1*H*-pyrazole-5-carboxamide.

Embodiment 8. The composition of any one of Embodiments 1 through 7 wherein component (b) includes at least one fungicidal compound selected from the group consisting of:

(b1) methyl benzimidazole carbamate (MBC) fungicides;
(b2) dicarboximide fungicides;
(b3) demethylation inhibitor (DMI) fungicides;
(b4) phenylamide (PA) fungicides;
(b5) amine/morpholine fungicides;
(b6) phospholipid biosynthesis inhibitor fungicides;
(b7) succinate dehydrogenase inhibitor (SDHI) fungicides;
(b8) hydroxy(2-amino-)pyrimidine fungicides;
(b9) anilinopyrimidine (AP) fungicides;
(b10) *N*-phenyl carbamate fungicides;
(b11) quinone outside inhibitor (QoI) fungicides;
(b12) phenylpyrrole (PP) fungicides;
(b13) azanaphthalene fungicides;
(b14) cell peroxidation inhibitor fungicides;
(b15) melanin biosynthesis inhibitor-reductase (MBI-R) fungicides;
(b16a) melanin biosynthesis inhibitor-dehydratase (MBI-D) fungicides;
(b16b) melanin biosynthesis inhibitor-polyketide synthase (MBI-P) fungicides;
(b17) keto reductase inhibitor (KRI) fungicides;
(b18) squalene-epoxidase inhibitor fungicides;
(b19) polyoxin fungicides;
(b20) phenylurea fungicides;
(b21) quinone inside inhibitor (QiI) fungicides;
(b22) benzamide and thiazole carboxamide fungicides;
(b23) enopyranuronic acid antibiotic fungicides;
(b24) hexopyranosyl antibiotic fungicides;
(b25) glucopyranosyl antibiotic: protein synthesis fungicides;
(b26) glucopyranosyl antibiotic fungicides;
(b27) cyanoacetamideoxime fungicides;
(b28) carbamate fungicides;
(b29) oxidative phosphorylation uncoupling fungicides;
(b30) organo tin fungicides;
(b31) carboxylic acid fungicides;
(b32) heteroaromatic fungicides;
(b33) phosphonate fungicides;
(b34) phthalamic acid fungicides;
(b35) benzotriazine fungicides;

(b36) benzene-sulfonamide fungicides;
(b37) pyridazinone fungicides;
(b38) thiophene-carboxamide fungicides;
(b39) complex I NADH oxido-reductase inhibitor fungicides;
(b40) carboxylic acid amide (CAA) fungicides;
(b41) tetracycline antibiotic fungicides;
(b42) thiocarbamate fungicides;
(b43) benzamide fungicides;
(b44) microbial fungicides;
(b45) quinone outside inhibitor, stigmatellin binding (QoSI) fungicides;
(b46) plant extract fungicides;
(b47) cyanoacrylate fungicides;
(b48) polyene fungicides;
(b49) oxysterol binding protein inhibitor (OSBPI) fungicides;
(b50) aryl-phenyl-ketone fungicides;
(b51) host plant defense induction fungicides;
(b52) multi-site activity fungicides;
(b53) biologicals with multiple modes of action;
(b54) fungicides other than fungicides of component (a) and components (b1) through (b53); and salts of compounds of (b1) through (b54).

Embodiment 9. The composition of Embodiment 8 wherein component (b) comprises at least one fungicidal compound from each of two different groups selected from (b1) through (b54).

Embodiment 10. The composition of any one of Embodiments 1 through 9 wherein component (b) includes at least one compound selected from acibenzolar-S-methyl, aldimorph, ametoctradin, amisulbrom, anilazine, azaconazole, azoxystrobin, benalaxyl, benalaxyl-M, benodanil, benomyl, benthiavalicarb, benthiavalicarb-isopropyl, benzovindiflupyr, bethoxazin, binapacryl, biphenyl, bitertanol, bixafen, blasticidin-S, boscalid, bromuconazole, bupirimate, carboxin, carpropamid, captafol, captan, carbendazim, chloroneb, chlorothalonil, chlozolinate, clotrimazole, copper salts, copper hydroxide, cyazofamid, cyflufenamid, cymoxanil, cyproconazole, cyprodinil, dichlofluanid, diclocymet, diclomezine, dicloran, diethofencarb, difenoconazole, diflumetorim, dimethirimol, dimethomorph, dimoxystrobin, diniconazole, diniconazole-M, dinocap, dithianon, dodemorph, dodine, edifenphos, enestroburin, epoxiconazole, ethaboxam, ethirimol, etridiazole, famoxadone, fenamidone, fenarimol, fenbuconazole, fenfuram, fenhexamid, fenoxanil, fenpiclonil, fenpropidin, fenpropimorph, fenpyrazamine, fentin acetate, fentin chloride, fentin hydroxide, ferbam, ferimzone, fluazinam, fludioxonil, fluindapyr, flumetover, flumorph, fluopicolide, fluopyram, fluoroimide, fluoxastrobin, fluquinconazole, flusilazole, flusulfamide, flutianil, flutolanil, flutriafol, fluxapyroxad, folpet, fosetyl-aluminum, fuberidazole, furalaxyl, furametpyr, hexaconazole, hymexazol, guazatine, imazalil, imibenconazole, iminoctadine, iodocarb, ipconazole, ipfentrifluconazole, iprobenfos, iprodione, iprovalicarb, isoprothiolane, isopyrazam, isotianil, kasugamycin, kresoxim-methyl, mancozeb, mandipropamid, maneb, mepronil, meptyldinocap, metalaxyl, metalaxyl-M, metconazole, methasulfocarb, metiram, metominostrobin, mepanipyrim, metrafenone, myclobutanil, naftifine, neo-asozin (ferric methanearsonate), nuarimol, octhilinone, ofurace, orysastrobin, oxadixyl, oxolinic acid, oxpoconazole, oxycarboxin, oxytetracycline, penconazole, pencycuron, penflufen, penthiopyrad, pefurazoate, phosphorous acid and salts thereof, phthalide, picoxystrobin, piperalin, polyoxin, probenazole, prochloraz, procymidone, propamocarb, propamocarb-hydrochloride, propiconazole, propineb, proquinazid, prothiocarb, prothioconazole, pydiflumetofen, pyraclostrobin, pyrametostrobin, pyraoxystrobin, pyrazophos, pyribencarb, pyributicarb, pyrifenox, pyrimethanil, pyriofenone, pyrisoxazole, pyroquilon, pyrrolnitrin, quinomethionate, quinoxyfen, quintozene, sedaxane, silthiofam, simeconazole, spiroxamine, streptomycin, sulfur, tebuconazole, tebufloquin, tecloftalam, tecnazene, terbinafine, tetraconazole, thiabendazole, thifluzamide, thiophanate, thiophanate-methyl, thiram, tiadinil, tolclofos-methyl, tolylfluanid, tolnifanide, triadimefon, triadimenol, triazoxide, tricyclazole, tridemorph, triflumizole, tricyclazole, trifloxystrobin, triforine, trimorphamide, triticonazole, uniconazole, validamycin, valifenalate, vinclozolin, zineb, ziram, zoxamide, *N'*-[4-[4-chloro-3-(trifluoromethyl)phenoxy]-2,5-dimethylphenyl]-*N*-ethyl-*N*-methylmethanimidamide, 5-chloro-6-(2,4,6-trifluorophenyl)-7-(4-methylpiperidin-1-yl)[1,2,4]triazolo[1,5*a*]pyrimidine (DPX-BAS600F), *N*-[2-[4-[[3-(4-chlorophenyl)-2-propyn-1-yl]oxy]-3-methoxyphenyl]ethyl]-3-methyl-2-[(methylsulfonyl)amino]butanamide, *N*-[2-[4-[[3-(4-chlorophenyl)-2-propyn-1-yl]oxy]-3-methoxyphenyl]ethyl]-3-methyl-2-[(ethylsulfonyl)-amino]butanamide, 4-fluorophenyl *N*-[1-[[[1-(4-cyanophenyl)ethyl]sulfonyl]-methyl]propyl]carbamate, *N*-[[(cyclopropylmethoxy)amino][6-(difluoromethoxy)-2,3-difluorophenyl]methylene]benzeneacetamide, α-(methoxyimino)-*N*-methyl-2-[[[1-[3-(trifluoromethyl)phenyl]ethoxy]imino]methyl]benzeneacetamide, *N*'-[4-[4-chloro-3-(trifluoromethyl)phenoxy]-2,5-dimethylphenyl]-*N*-ethyl-*N*-methylmethanimidamide, 2-[[[[3-(2,6-dichlorophenyl)-1-methyl-2-propen-1-ylidene]amino]oxy]methyl]-α-(methoxyimino)-*N*-methylbenzeneacetamide, 1-[(2-propenylthio)carbonyl]-2-(1-methylethyl)-4-(2-methylphenyl)-5-amino-1*H*-pyrazol-3-one, 5-ethyl-6-octyl-[1,2,4]triazolo[1,5-a]pyrimidin-7-ylamine, methyl *N*-[[5-[1-[2,6-difluoro-4-(1-methylethyl)phenyl]-1*H*-pyrazol-3-yl]-2-methylphenyl]methyl] carbamate, methyl *N*-[[5-[1-(4-cyclopropyl-2,6-difluorophenyl)-1*H*-pyrazol-3-yl]-2-methylphenyl]methyl]carbamate, ethyl 1-[[4-[[(1*Z*)-2-ethoxy-3,3,3-trifluoro-1-propen-1-yl]oxy]phenyl]methyl]-1*H*-pyrazole-4-carboxylate and its (*E*)-isomer and ethyl 1-[[4-[[2-(trifluoromethyl)-1,3-dioxolan-2-yl]methoxy]phenyl]methyl]-1*H*-pyrazole-4-carboxylate.

Embodiment 11. The composition of Embodiment 10 wherein component (b) includes at least one compound selected from azoxystrobin, benzovindiflupyr, bixafen, chlorothalonil, copper hydroxide, cyproconazole, epoxiconazole, fenpropimorph, fluindapyr, fluxapyroxad, mancozeb, picoxystrobin, prothioconazole, pydiflumetofen, tebuconazole, trifloxystrobin, methyl *N*-[[5-[1-[2,6-difluoro-4-(1-methylethyl)phenyl]-1*H*-pyrazol-3-yl]-2-methylphenyl]methyl]carbamate, methyl *N*-[[5-[1-(4-cyclopropyl-2,6-difluorophenyl)-1*H*-pyrazol-3-yl]-2-methylphenyl]methyl] carbamate, ethyl 1-[[4-[[(1*Z*)-2-ethoxy-3,3,3-trifluoro-1-propen-1-yl]oxy]phenyl]methyl]-1*H*-pyrazole-4-carboxylate and its (*E*)-isomer and ethyl 1-[[4-[[2-(trifluoromethyl)-1,3-dioxolan-2-yl]methoxy]phenyl]methyl]-1*H*-pyrazole-4-carboxylate.

Embodiment 12. A composition comprising: (a) at least one compound selected from the compounds of Formula **1** as defined in Embodiment 1, *N*-oxides, and salts thereof; and at least one invertebrate pest control compound or agent.

Embodiment 13. A composition comprising the composition of any one of Embodiments 1 through 12 and at least one additional component selected from the group consisting of surfactants, solid diluents and liquid diluents.

Embodiment 14. A method for protecting a plant or plant seed from diseases caused by fungal pathogens comprising applying a fungicidally effective amount of the composition of any one of Embodiments 1 through 13 to the plant or plant seed.

Embodiment 15. A method for protecting a plant from a rust disease comprising applying to the plant a fungicidally effective amount of the composition of any one of Embodiments 1 through 13 wherein component (b) includes at least one fungicidal compound selected from (b3) demethylation inhibitor fungicides, (b5) amine/morpholine fungicides, (b7) succinate dehydrogenase inhibitor fungicides, (b11) quinone outside inhibitor (QoI) fungicides, (b13) methyl benzimidazole carbamate fungicides and (b52) multi-site activity fungicides.

## Claims

**1.** A compound of Formula 1, an N-oxide, or a salt thereof:

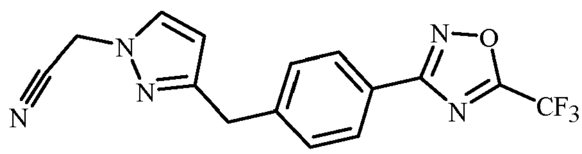

**1**

**2.** A composition comprising a compound of Claim 1 and at least one additional component selected from the group consisting of surfactants, solid diluents and liquid diluents.

**3.** A composition comprising (a) a compound of Claim 1 and (b) at least one additional fungicidal compound.

**4.** The composition of Claim 3 wherein component (b) includes at least one fungicidal compound selected from the group consisting of:

(b1) methyl benzimidazole carbamate (MBC) fungicides;
(b2) dicarboximide fungicides;
(b3) demethylation inhibitor (DMI) fungicides;
(b4) phenylamide (PA) fungicides;

(b5) amine/morpholine fungicides;
(b6) phospholipid biosynthesis inhibitor fungicides;
(b7) succinate dehydrogenase inhibitor (SDHI) fungicides;
(b8) hydroxy(2-amino-)pyrimidine fungicides;
(b9) anilinopyrimidine (AP) fungicides;
(b10) *N*-phenyl carbamate fungicides;
(b11) quinone outside inhibitor (QoI) fungicides;
(b12) phenylpyrrole (PP) fungicides;
(b13) azanaphthalene fungicides;
(b14) cell peroxidation inhibitor fungicides;
(b15) melanin biosynthesis inhibitor-reductase (MBI-R) fungicides;
(b16a) melanin biosynthesis inhibitor-dehydratase (MBI-D) fungicides;
(b16b) melanin biosynthesis inhibitor-polyketide synthase (MBI-P) fungicides;
(b17) keto reductase inhibitor (KRI) fungicides;
(b18) squalene-epoxidase inhibitor fungicides;
(b19) polyoxin fungicides;
(b20) phenylurea fungicides;
(b21) quinone inside inhibitor (QiI) fungicides;
(b22) benzamide and thiazole carboxamide fungicides;
(b23) enopyranuronic acid antibiotic fungicides;
(b24) hexopyranosyl antibiotic fungicides;
(b25) glucopyranosyl antibiotic: protein synthesis fungicides;
(b26) glucopyranosyl antibiotic fungicides;
(b27) cyanoacetamideoxime fungicides;
(b28) carbamate fungicides;
(b29) oxidative phosphorylation uncoupling fungicides;
(b30) organo tin fungicides;
(b31) carboxylic acid fungicides;
(b32) heteroaromatic fungicides;
(b33) phosphonate fungicides;
(b34) phthalamic acid fungicides;
(b35) benzotriazine fungicides;
(b36) benzene-sulfonamide fungicides;
(b37) pyridazinone fungicides;
(b38) thiophene-carboxamide fungicides;
(b39) complex I NADH oxido-reductase inhibitor fungicides;
(b40) carboxylic acid amide (CAA) fungicides;
(b41) tetracycline antibiotic fungicides;
(b42) thiocarbamate fungicides;
(b43) benzamide fungicides;
(b44) microbial fungicides;
(b45) quinone outside inhibitor, stigmatellin binding (QoSI) fungicides;
(b46) plant extract fungicides;
(b47) cyanoacrylate fungicides;
(b48) polyene fungicides;
(b49) oxysterol binding protein inhibitor (OSBPI) fungicides;
(b50) aryl-phenyl-ketone fungicides;
(b51) host plant defense induction fungicides;
(b52) multi-site activity fungicides;
(b53) biologicals with multiple modes of action;
(b54) fungicides other than fungicides of component (a) and components (b1) through (b53); and salts of compounds of (b1) through (b54).

**5.** The composition of Claim 4 wherein component (b) comprises at least one fungicidal compound from each of two different groups selected from (b1) through (b54).

**6.** The composition of Claim 3 wherein component (b) includes at least one compound selected from acibenzolar-S-methyl, aldimorph, ametoctradin, amisulbrom, anilazine, azaconazole, azoxystrobin, benalaxyl, benalaxyl-M, benodanil, benomyl, benthiavalicarb, benthiavalicarb-isopropyl, benzovindiflupyr, bethoxazin, binapacryl, biphenyl, bitertanol, bixafen, blasticidin-S, boscalid, bromuconazole, bupirimate, carboxin, carpropamid, captafol, captan, carbendazim, chloroneb, chlorothalonil, chlozolinate, clotrimazole, copper salts, copper hydroxide, cyazofamid, cyflufenamid, cymoxanil, cyproconazole, cyprodinil, dichlofluanid, diclocymet, diclomezine, dicloran, diethofencarb, difenoconazole, diflumetorim, dimethirimol, dimethomorph, dimoxystrobin, diniconazole, diniconazole-M, dinocap, dithianon, dodemorph, dodine, edifenphos, enestroburin, epoxiconazole, ethaboxam, ethirimol, etridiazole, famoxadone, fenamidone, fenarimol, fenbuconazole, fenfuram, fenhexamid, fenoxanil, fenpiclonil, fenpropidin, fenpropimorph, fenpyrazamine, fentin acetate, fentin chloride, fentin hydroxide, ferbam, ferimzone, fluazinam, fludioxonil, fluindapyr, flumetover, flumorph, fluopicolide, fluopyram, fluoroimide, fluoxastrobin, fluquinconazole, flusilazole, flusulfamide, flutianil, flutolanil, flutriafol, fluxapyroxad, folpet, fosetyl-aluminum, fuberidazole, furalaxyl, furametpyr, hexaconazole, hymexazol, guazatine, imazalil, imibenconazole, iminoctadine, iodocarb, ipconazole, ipfentrifluconazole, iprobenfos, iprodione, iprovalicarb, isoprothiolane, isopyrazam, isotianil, kasugamycin, kresoxim-methyl, mancozeb, mandipropamid, maneb, mepronil, meptyldinocap, metalaxyl, metalaxyl-M, metconazole, methasulfocarb, metiram, metominostrobin, mepanipyrim, metrafenone, myclobutanil, naftifine, neo-asozin (ferric methanearsonate), nuarimol, octhilinone, ofurace, orysastrobin, oxadixyl, oxolinic acid, oxpoconazole, oxycarboxin, oxytetracycline, penconazole, pencycuron, penflufen, penthiopyrad, pefurazoate, phosphorous acid and salts thereof, phthalide, picoxystrobin, piperalin, polyoxin, probenazole, prochloraz, procymidone, propamocarb, propamocarb-hydrochloride, propiconazole, propineb, proquinazid, prothiocarb, prothioconazole, pydiflumetofen, pyraclostrobin, pyrametostrobin, pyraoxystrobin, pyrazophos, pyribencarb, pyributicarb, pyrifenox, pyrimethanil, pyriofenone, pyrisoxazole, pyroquilon, pyrrolnitrin, quinomethionate, quinoxyfen, quintozene, sedaxane, silthiofam, simeconazole, spiroxamine, streptomycin, sulfur, tebuconazole, tebufloquin, tecloftalam, tecnazene, terbinafine, tetraconazole, thiabendazole, thifluzamide, thiophanate, thiophanate-methyl, thiram, tiadinil, tolclofos-methyl, tolylfluanid, tolnifanide, triadimefon, triadimenol, triazoxide, tricyclazole, tridemorph, triflumizole, tricyclazole, trifloxystrobin, triforine, trimorphamide, triticonazole, uniconazole, validamycin, valifenalate, vinclozolin, zineb, ziram, zoxamide, *N*'-[4-[4-chloro-3-(trifluoromethyl)phenoxy]-2,5-dimethylphenyl]-*N*-ethyl-*N*-methylmethanimidamide, 5-chloro-6-(2,4,6-trifluorophenyl)-7-(4-methylpiperidin-1-yl)[1,2,4]triazolo[1,5*a*]pyrimidine (DPX-BAS600F), *N*-[2-[4-[[3-(4-chlorophenyl)-2-propyn-1-yl]oxy]-3-methoxyphenyl]ethyl]-3-methyl-2-[(methylsulfonyl)amino]butanamide, *N*-[2-[4-[[3-(4-chlorophenyl)-2-propyn-1-yl]oxy]-3-methoxyphenyl]ethyl]-3-methyl-2-[(ethylsulfonyl)amino]-butanamide, 4-fluorophenyl *N*-[1-[[[1-(4-cyanophenyl)ethyl]sulfonyl]-methyl]propyl]carbamate, *N*-[[(cyclopropylmethoxy)amino][6-(difluoromethoxy)-2,3-difluorophenyl]methylene]benzeneacetamide, α-(methoxyimino)-*N*-methyl-2-[[[1-[3-(trifluoromethyl)phenyl]ethoxy]imino]methyl]benzeneacetamide, *N*'-[4-[4-chloro-3-(trifluoromethyl)phenoxy]-2,5-dimethylphenyl]-*N*-ethyl-*N*-methylmethanimidamide, 2-[[[[3-(2,6-dichlorophenyl)-1-methyl-2-propen-1-ylidene]amino]oxy]methyl]-α-(methoxyimino)-*N*-methylbenzeneacetamide, 1-[(2-propenylthio)carbonyl]-2-(1-methylethyl)-4-(2-methylphenyl)-5-amino-1*H*-pyrazol-3-one, 5-ethyl-6-octyl-[1,2,4]triazolo[1,5-a]pyrimidin-7-ylamine, methyl *N*-[[5-[1-[2,6-difluoro-4-(1-methylethyl)phenyl]-1*H*-pyrazol-3-yl]-2-methylphenyl]methyl]carbamate, methyl *N*-[[5-[1-(4-cyclopropyl-2,6-difluorophenyl)-1*H*-pyrazol-3-yl]-2-methylphenyl]methyl]carbamate, ethyl 1-[[4-[[(1Z)-2-ethoxy-3,3,3-trifluoro-1-propen-1-yl]oxy]phenyl]methyl]-1*H*-pyrazole-4-carboxylate and its (*E*)-isomer and ethyl 1-[[4-[[2-(trifluoromethyl)-1,3-dioxolan-2-yl]methoxy]phenyl]methyl]-1*H*-pyrazole-4-carboxylate.

**7.** The composition of Claim 6 wherein component (b) includes at least one compound selected from azoxystrobin, benzovindiflupyr, bixafen, chlorothalonil, copper hydroxide, cyproconazole, epoxiconazole, fenpropimorph, fluindapyr, fluxapyroxad, mancozeb, picoxystrobin, prothioconazole, pydiflumetofen, tebuconazole, trifloxystrobin, methyl *N*-[[5-[1-[2,6-difluoro-4-(1-methylethyl)phenyl]-1*H*-pyrazol-3-yl]-2-methylphenyl] methyl]carbamate, methyl *N*-[[5-[1-(4-cyclopropyl-2,6-difluorophenyl)-1*H*-pyrazol-3-yl]-2-methylphenyl]methyl]carbamate, ethyl 1-[[4-[[(1*Z*)-2-ethoxy-3,3,3-trifluoro-1-propen-1-yl]oxy]phenyl]methyl]-1*H*-pyrazole-4-carboxylate and its (*E*)-isomer and ethyl 1-[[4-[[2-(trifluoromethyl)-1,3-dioxolan-2-yl]methoxy]phenyl]methyl]-1*H*-pyrazole-4-carboxylate.

**8.** A composition comprising a compound of Claim 1 and at least one invertebrate pest control compound or agent.

**9.** A composition of any one of Claims 3 through 8 and at least one additional component selected from the group consisting of surfactants, solid diluents and liquid diluents.

**10.** A method for protecting a plant or plant seed from diseases caused by fungal pathogens comprising applying a fungicidally effective amount of the compound of Claim 1 or the composition of any one of Claims 2 through 9 to the plant or plant seed.

**11.** A method for protecting a plant from a rust disease comprising applying to the plant a fungicidally effective amount of the compound of Claim 1 or the composition of any one of Claims 3 through 7 wherein component (b) includes

at least one fungicidal compound selected from (b3) demethylation inhibitor fungicides, (b5) amine/morpholine fungicides, (b7) succinate dehydrogenase inhibitor fungicides, (b11) quinone outside inhibitor (QoI) fungicides, (b13) methyl benzimidazole carbamate fungicides and (b52) multi-site activity fungicides.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- EP 2962568 A
- WO 2008124092 A
- WO 2020097012 A
- WO 2020056090 A
- WO 2020051402 A
- WO 9424861 A
- US 6022552 A
- WO 03024222 A
- US 3060084 A
- WO 9113546 A
- US 4172714 A
- US 4144050 A
- US 3920442 A
- DE 3246493
- US 5180587 A
- US 5232701 A
- US 5208030 A
- GB 2095558 A
- US 3299566 A
- US 3235361 A
- US 3309192 A
- US 2891855 A
- US 6406690 B


### Non-patent literature cited in the description


- **ERNEST L. ELIEL ; SAMUEL H. WILEN.** Stereochemistry of Organic Compounds. John Wiley & Sons, 1994
- **T. L. GILCHRIST.** Comprehensive Organic Synthesis. Pergamon Press, vol. 7, 748-750
- **M. TISLER ; B. STANOVNIK.** Comprehensive Heterocyclic Chemistry. Pergamon Press, vol. 3, 18-20
- **M. R. GRIMMETT ; B. R. T. KEENE ; M. TISLER.** Advances in Heterocyclic Chemistry. Academic Press, vol. 43, 149-161
- **B. STANOVNIK.** Advances in Heterocyclic Chemistry. Academic Press, vol. 9, 285-291
- **G. W. H. CHEESEMAN ; E. S. G. WERSTIUK.** Advances in Heterocyclic Chemistry. Academic Press, vol. 22, 390-392
- Polymorphism in the Pharmaceutical Industry. Wiley-VCH, 2006
- **K. R. MORRIS.** Polymorphism in Pharmaceutical Solids. 1995
- **K. H. KUCK et al.** Modern Selective Fungicides - Properties, Applications and Mechanisms of Action. Gustav Fischer Verlag, 1995, 205-258
- Comprehensive Heterocyclic Chemistry. Pergamon Press, 1984, vol. 6, 365-391
- **MITSUNOBU, O.** Comprehensive Organic Synthesis. Pergamon, 1991, vol. 6, 65-101
- Comprehensive Heterocyclic Chemistry. Pergamon Press, 1984, vol. 4-6
- Comprehensive Heterocyclic Chemistry II. Pergamon Press, 1996, vol. 2-4
- The Chemistry of Heterocyclic Compounds. Wiley
- **D. T. MOWRY.** *Chemical Reviews,* 1948, vol. 42, 236
- **H. GROEGER.** *Chemical Reviews,* 2003, vol. 103, 2795-2827
- **M. NORTH.** Comprehensive Organic Functional Group Transformations. Pergamon, 1995, vol. 3, 615-617
- **HEYDARI et al.** *Journal of Molecular Catalysis A: Chemical,* 2007, vol. 277 (1-2), 142-144
- Comprehensive Organic Functional Group Transformations. Pergamon Press, 1995, vol. 1,2,3,5
- Vogel's Textbook of Practical Organic Chemistry. Longman Group, London, 1989, 470-823
- Advanced Organic Chemistry. Wiley, 1992
- **T. W. GREENE ; P. G. M. WUTS.** Protective Groups in Organic Synthesis. Wiley, 1991
- **MARSDEN.** Solvents Guide. Interscience, 1950
- McCutcheon's Emulsifiers and Detergents. McCutcheon's Division, The Manufacturing Confectioner Publishing Co
- **SISELY ; WOOD.** Encyclopedia of Surface Active Agents. Chemical Publ. Co., Inc, 1964
- **A. S. DAVIDSON ; B. MILWIDSKY.** Synthetic Detergents. John Wiley and Sons, 1987
- **MCCUTCHEON.** Functional Materials. McCutcheon's Division, The Manufacturing Confectioner Publishing Co, vol. 2
- **BROWNING.** Agglomeration. *Chemical Engineering,* 04 December 1967, 147-48
- Perry's Chemical Engineer's Handbook. McGraw-Hill, 1963, 8-57

- **P. KOSTERS et al.** *Seed Treatment: Progress and Prospects,* 1994
- The Formulator's Toolbox - Product Forms for Modern Agriculture. **T. S. WOODS.** Pesticide Chemistry and Bioscience, The Food-Environment Challenge. The Royal Society of Chemistry, 1999, 120-133
- **KLINGMAN.** Weed Control as a Science. John Wiley and Sons, Inc, 1961, 81-96
- **HANCE et al.** Weed Control Handbook. Blackwell Scientific Publications, 1989
- Developments in formulation technology. PJB Publications, 2000
- The Pesticide Manual. British Crop Protection Council, 2003
- The BioPesticide Manual. British Crop Protection Council, 2001
- **COLBY, S. R.** Calculating Synergistic and Antagonistic Responses of Herbicide Combinations. *Weeds,* 1967, vol. 15, 20-22